# EUROPEAN PATENT SPECIFICATION

(11) **EP 3 983 393 B1**
(45) Date of publication and mention of the grant of the patent: **02.04.2025**
(21) Application number: 20734118.1
(22) Date of filing: 11.06.2020
(51) Int. Cl.: C07D 307/94, C07D 473/34, C07D 493/04, C07D 519/00, A61K 31/437, A61K 31/343, A61K 31/357, A61P 35/00

(54) **NOVEL SPIROBICYCLIC INTERMEDIATES**
NEUARTIGE SPIROBICYCLISCHE ZWISCHENPRODUKTE
NOUVEAUX INTERMÉDIAIRES SPIROBICYCLIQUES

(30) Priority: 12.06.2019 IN 201911023295; 27.08.2019 EP 19193707
(43) Date of publication of application: 20.04.2022
(73) Proprietor: Janssen Pharmaceutica NV, 2340 Beerse (BE)
(72) Inventor: VERHOEVEN, Jonas, 1050 Brussel (BE); BRAMBILLA, Marta, 2340 Beerse (BE); CHINTA, Nagaraju, 9000 Gent (BE); HULLAERT, Jan, Julien, A, 9000 Gents (BE); JOUFFROY, Lucile, Marguerite, 9000 Gent (BE); MEERPOEL, Lieven, 2340 Beerse (BE); NEOUCHY, Zeïna, 9000 Gent (BE); THURING, Johannes, Wilhelmus, John, F, 2340 Beerse (BE); VERNIEST, Guido, Alfons, F, 2340 Beerse (BE); WINNE, Johan, Maurits, 9000 Gent (BE)
(74) Representative: Lenaerts, Philip
(86) International application number: PCT/EP2020/066182
(87) International publication number: WO 2020/249663

(56) References cited:
- WO-A1-2017/032840
- WO-A1-2017/153186
- WO-A1-2019/110734

## Description

### Field of the Invention

The present invention relates to novel spirobicyclic intermediates useful in the synthesis of spirobicyclic nucleoside analogues.

### Background of the invention

PRMT5, also described as Hsl7, Jbp1, Skb1, Capsuleen or DartS, is one of the major methyltransferases responsible for mono- and symmetric dimethylation of arginines. Post-translational arginine methylation on histones and non-histone proteins seems to be crucial for a variety of biological processes, like genome organisation, transcription, differentiation, spliceosome function, signal transduction and regulation of cell-cycle progression, stem cells and T-cell fate [Stopa, N. et al., Cell Mol Life Sci, 2015. 72(11): p. 2041-59] [Geoghegan, V. et al., Nat Commun, 2015. 6: p. 6758]. Metazoan PRMTS forms a functional complex with the methylosome protein 50 (MEP50) also named as Wdr77, androgen receptor coactivator p44 and Valois. Both, elevated PRMTS-MEP50 protein level and cytoplasmic accumulation are implicated in cancer tumorigenesis and have recently been correlated with poor clinical outcome [Shilo, K. et al., Diagn Pathol, 2013. 8: p. 201]. Cellular rescue experiments that addressed both the catalytic and scaffold function of the PRMT5-MEP50 complex, beside comprehensive enzymological studies have substantiate the oncogenic link between protein level, localisation and enzymatic function [Gu, Z. et al., Biochem J, 2012. 446(2): p. 235-41] [Di Lorenzo, A. et. al., FEBS Lett, 2011. 585(13): p. 2024-31] [Chan-Penebre, E. et al., Nat Chem Biol, 2015. 11(6): p. 432-7]. This correlation turns PRMTS into an essential small molecule drug target against cancer and other diseases [Stopa, N. et al., Cell Mol Life Sci, 2015. 72(11): p. 2041-59].

PRMTS is a member of the type II PRMT subfamily that utilises S-adenosylmethionine (SAM) to generate symmetric dimethylated arginine on histones and non-histone protein substrates and S-adenosylhomocysteine (SAH). The crystal structure of the human hetereo-octameric complex (PRMT5)₄(MEP50)₄ co-crystalised with SAH and a histone H4 peptide substrate illustrated the mechanism of methylation and substrate recognition [Antonysamy, S. et al., Proc Natl Acad Sci U S A, 2012. 109(44): p. 17960-5]. The regulation of PRMTS activity occurs through a vast number of different binding partners, post-translational modification cross talk, miRNAs and subcellular localisation.

Methylation of histones H2A and H4 on Arg3 and histone H3 on Arg8 regulate chromatin organisation for specific repression of gene transcripts that are involved in differentiation, transformation, cell-cycle progression and tumour suppression [Karkhanis, V. et al., Trends Biochem Sci, 2011. 36(12): p. 633-41]. Furthermore, PRMTS-mediated methylation of histone H4 on Arg3 might recruit the DNA-methyltransferase DNMT3A to couple histone and DNA methylation for long-term gene silencing [Zhao, Q. et al., Nat Struct Mol Biol, 2009. 16(3): p. 304-11].

Non-histone methylation can occur either in the cytoplasm or nucleus dependent on the cellular localisation of PRMT5. The methylation of the Sm proteins D1 and D3, which are required for the assembly of the nuclear splicesome, takes place in the cytoplasm as part of the PRMTS containing "methylosome" [Friesen, W.J. et al., Mol Cell Biol, 2001. 21(24): p. 8289-300]. Further evidence for PRMTS involved in splicing has been provided by the conditional PRMTS knockout in mouse neural stem cells. Cells that lack PRMTS showed a selective retention of introns and skipping of exons with weak 5' donor sites [Bezzi, M. et al., Genes Dev, 2013. 27(17): p. 1903-16].

In addition to a role in splicing, PRMTS influences key pathways involved in cell fate and homeostasis by direct methylation of key signalling nodules like p53 [Jansson, M. et al., Nat Cell Biol, 2008. 10(12): p. 1431-9], EGFR [Hsu, J.M. et al., Nat Cell Biol, 2011. 13(2): p. 174-81], CRAF [Andreu-Perez, P. et al., Sci Signal, 2011. 4(190): p. ra58], PI3K/AKT [Wei, T.Y. et al., Cell Signal, 2014. 26(12): p. 2940-50], NFkB [Wei, H. et al., Proc Natl Acad Sci U S A, 2013. 110(33): p. 13516-21].

Since PRMTS is one of the major sym-Arg methyltransferases and involved in a multitude of cellular processes, an increased protein expression appears to be an important factor in its tumourigenicity. Interestingly, the translation of PRMTS in mantle cell lymphoma (MCL) seems to be regulated by miRNAs. Although MCL cells show less mRNA and a slower transcription rate of PRMTS than normal B lymphocytes, the PRMTS level and the methylation of H3R8 and H4R3 are significantly increased [Pal, S. et al., EMBO J, 2007. 26(15): p. 3558-69]. Re-expression of miRNAs that binds the 3'UTR region of PRMTS decreases PRMTS protein level [Wang, L. et al., Mol Cell Biol, 2008. 28(20): p. 6262-77]. Strikingly, a prmt5 antisense RNA has been found within the human prmt5 gene that supports the hypothesis of a specific translational regulation rather than high mRNA expression level [Stopa, N. et al., Cell Mol Life Sci, 2015. 72(11): p. 2041-59].

Very recently, a novel sub-nanomolar potent PRMTS inhibitor (EPZ015666) with anti-tumour activity in multiple MCL xenograft models has been described to be the first chemical probe suitable for further validation of PRMT5's biology and role in cancer [Chan-Penebre, E. et al., Nat Chem Biol, 2015. 11(6): p. 432-7]. WO2014100695, WO2014100719, WO2015200680, and WO2014100730 disclose compounds useful for inhibiting PRMTS activity.Devkota, K. et al., ACS Med Chem Lett, 2014. 5: p. 293-297, describes the synthesis of a series of analogues of the natural product sinefungin and the ability of these analogues to inhibit EHMT1 and EHMT2. WO2003070739 discloses partial and full agonists of A1 adenosine receptors, their preparation, and their therapeutic use. WO2012082436 discloses compounds and compositions as modulators of histone methyltransferases, and for treating diseases influenced by modulation of histone methyltransferase activity. WO03074083 discloses combination therapies that selectively kill methylthioadenosine phosphorylase deficient cells. Analogs of MTA are described herein as anti-toxicity agents. Kung, P.-P. et al., Bioorg Med Chem Lett, 2005. 15: p. 2829-2833, describes the design, synthesis, and biological evaluation of novel human 5'-deoxy-5'-methylthioadenosine phosphorylase (MTAP) substrates. WO2012075500 discloses 7-deazapurine modulators of histone methyltransferase. WO2014035140 discloses compounds and compositions for modulating histone methyltransferase activity. WO9640686 describes heterocyclic substituted cyclopentane compounds and methods of using such compounds for inhibiting adenosine kinase.

WO2016135582 and US20160244475 describe substituted nucleoside derivatives useful as anticancer agents. Boyer *et al* described diaryl-erythro-furanosyltubercidin analogies as adenosine kinase inhibitors.

Nucleoside analogues are a class of compounds that have interesting therapeutic activities. They are used clinically for the treatment of wide-ranging diseases and disorders such as viral infections and proliferative disorders. For example, WO2019/110734, WO2017/032840, WO2017/153186 and WO2018/065365 disclose nucleoside analogues useful for inhibiting Protein Arginine Methyltransferase 5 (PRMT5) activity and for treating PRMT5-mediated disorders.

It is desirable to develop intermediates and chemical syntheses to allow the preparation of nucleoside analogues having novel substituent patterns.

Brand et al., JOC 2009, 74, 8779-8786 and Ramakrishna et al, RSC Adv., 2015, 5, 8142-8145 describe synthesis of spirocyclic compounds. Nowak et al, JOC 2006, 71, 8876-8883 describes addition of difluorocarbene to 4',5'-unsaturated nucleosides. Redlich et al, Angew. Chem. Int. Ed. Engl. 28 (1989) 777-778 described synthesis of chiral cyclobutanones.

### Summary of the invention

It has been determined that spirobicyclic nucleosides show interesting activity, for example against PRMT5. Novel intermediates that may be used to synthesise spirobicyclic nucleoside analogues have been developed. In particular, these intermediates contain functional groups that can be diversified to give access to a variety of spirobicyclic nucleoside analogues, including cyclopentane containing nucleoside analogues. Accordingly, disclosed herein are compounds which are useful as intermediates for the preparation of spirobicyclic nucleoside analogues that may, for example, possess activity against PRMT5. Also disclosed herein are spirocyclic nucleoside compounds which may serve as intermediates for further functionalisation to provide spirobicyclic nucleoside analogues with interesting activity.

In one aspect the present invention concerns a compound of Formula (A) 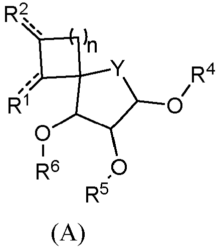 or a salt or solvate thereof, wherein:
Y is -O- or -CH₂-;
n is 0 or 1;
when n is 1, R¹ is hydrogen and R² is -(CH₂)ₘNR^{a}₂, -(CH₂)ₘOR^{a}, -(CH₂)ₘC(O)OR^{a}, - (CH₂)ₘC(O)NR^{a}₂, -(CH₂)ₘC(O)R^{a}, -(CH₂)ₘB(OR^{b})₂, -(CH₂)ₘS(O)ₓR^{c}, a silyl group, C₁-₆ alkyl substituted with a silyl group, =O or =CR^{a}₂; or
when n is 1, R² is hydrogen and R¹ is -(CH₂)ₘNR^{a}₂, -(CH₂)ₘOR^{a}, -(CH₂)ₘOPG, - (CH₂)ₘC(O)OR^{a}, -(CH₂)ₘC(O)NR^{a}₂, -(CH₂)ₘC(O)R^{a}, -(CH₂)ₘB(OR^{b})₂, -(CH₂)ₘS(O)ₓR^{c}, a silyl group, C₁-₆ alkyl substituted with a silyl group, -O-aryl, =O or =CR^{a}₂;
when n is 0, one of R¹ and R² is hydrogen and the other is -(CH₂)ₘNR^{a}₂,-(CH₂)ₘOR^{a}, - (CH₂)ₘOPG, -(CH₂)ₘC(O)OR^{a}, -(CH₂)ₘC(O)NR^{a}₂, -(CH₂)ₘC(O)R^{a}, -(CH₂)ₘB(OR^{b})₂, - (CH₂)ₘS(O)ₓR^{c} or =CH₂;
wherein the bond towards R¹ or R² - - is a single bond when R¹ or R² is hydrogen, - (CH₂)ₘNR^{a}₂, -(CH₂)ₘOR^{a}, -(CH₂)ₘOPG, -(CH₂)ₘC(O)OR^{a}, -(CH₂)ₘC(O)NR^{a}₂, - (CH₂)ₘC(O)R^{a}, -(CH₂)ₘB(OR^{b})₂, -(CH₂)ₘS(O)ₓR^{c}, a silyl group, C₁-₆ alkyl substituted with a silyl group or -O-aryl, or a double bond when R¹ or R² is =O, =CH₂ or =CR^{a}₂;
each R^{a} is, independently, hydrogen or C₁-₆ alkyl;
each R^{b} is hydrogen or both R^{b} are taken together to form a boronic acid protecting group;
R^{c} is C₁₋₆ alkyl or aryl;
PG is a hydroxyl protecting group;
m is 0 or 1;
x is 0, 1 or 2;
R⁴, R⁵ and R⁶ are each independently hydrogen or a hydroxyl protecting group;
or R⁵ and R⁶ taken together are a diol protecting group;
or R⁴ and R⁵ taken together are a diol protecting group;
provided that:
   (a) the compound is not a compound of formula: 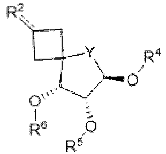 , wherein Y is -O- or -CH₂-; R⁴ is hydrogen or a hydroxyl protecting group such as for example C₁₋₄alkyl, t-butyldimethylsilyl, C₁₋₄alkyl-O-C₁₋₄alkyl, tetrahydropyranyl, allyl, t-butyldiphenylsilyl, benzyl, -C(=O)-C₁₋₄alkyl, or -C(=O)-phenyl; R² is -OH, =O, -CH₂-OH or =CH₂; and R⁵ and R⁶ are each independently -C(=O)-C₁₋₄alkyl, benzoyl, benzyl, or -CH₂-naphthyl, wherein benzyl, -CH₂-naphthyl, and benzoyl are optionally substituted with one or two substituents each independently selected from -CH₃ and -OCH₃; or R⁵ and R⁶ taken together are -C(C₁₋₄alkyl)₂-; or wherein Y is -O- or -CH₂, R⁴ is methyl, R² is =O, or =CH₂, and R⁵ and R⁶ are each independently hydrogen or -C(=O)-C₁₋₄alkyl; and
   (b) the compound is not a compound of formula: 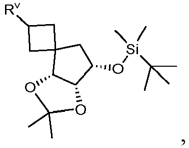 wherein R^{v} is =O, -OH or =CH₂; and
   (c) the compound is not a compound of formula: 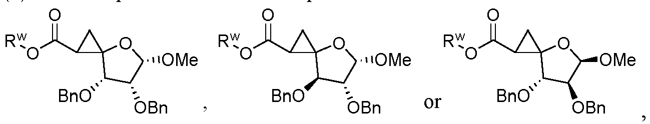 wherein R^{w} is methyl or H; and
   (d) the compound is not a compound of formula: 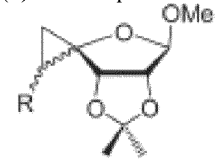 wherein R is CO₂Et or CH₂OH or any stereoisomer thereof.

In another asnect the present invention concerns a comnound of formula (B): 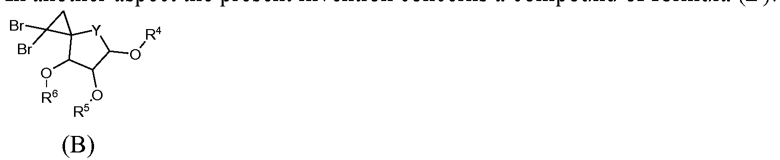 or a salt or solvate thereof, wherein:
Y is -O- or -CH₂-;
R⁴, R⁵ and R⁶ are each, independently, hydrogen or a hydroxyl protecting group;
or R⁵ and R⁶ taken together are a diol protecting group;
or R⁴ and R⁵ taken together are a diol protecting group.

In another aspect the present invention concerns a compound of formula (C): 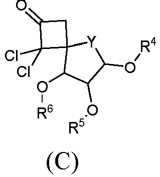 wherein:
Y is -O- or -CH₂-;
R⁴, R⁵ and R⁶ are each, independently, hydrogen or a hydroxyl protecting group; or
R⁴ and R⁵ taken together are a diol protecting group; or
R⁵ and R⁶ taken together are a diol protecting group;
provided that the compound is not:

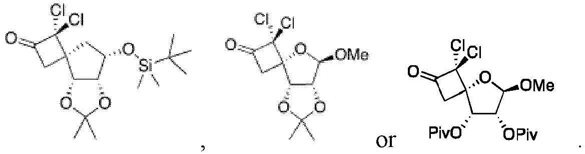

In another aspect the present invention concerns a compound of formula (D): 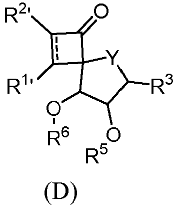 or a salt or solvate thereof, wherein:
Y is -O- or -CH₂-;
wherein - - is a single bond or a double bond; and
when - - is a single bond, one of R¹' and R²' is hydrogen or C₁-₆ alkyl and the other is a silyl group, C₁-₆ alkyl optionally substituted with a silyl group, -O-C₁-₆ alkyl, -B(OR^{b})₂, -S-C₁-₆ alkyl, -S-aryl, -O-aryl or C(O)R^{a};
when - - is a double bond, one of R¹' and R²' is hydrogen or C₁-₆ alkyl and the other is a silyl group, C₁-₆ alkyl optionally substituted with a silyl group, -O-C₁-₆ alkyl, or C(O)R^{a};
   and
R^{a} is hydrogen or C₁-₆ alkyl;
each R^{b} is hydrogen or both R^{b} are taken together to form a boronic acid protecting group;
R³ is OR⁴ or R¹⁰;
R¹⁰ is a nucleobase or nucleobase derivative;
R⁴, R⁵ and R⁶ are each, independently, hydrogen or a hydroxyl protecting group; or
R⁴ and R⁵ taken together are a diol protecting group; or
R⁵ and R⁶ taken together are a diol protecting group.

In another aspect the present invention concerns a compound of formula (E): 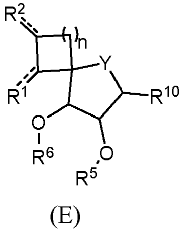 or a salt or solvate thereof, wherein:
Y is -O- or -CH₂-;
n is 0 or 1;
when n is 1, R¹ is hydrogen and R² is -(CH₂)ₘNR^{a}₂, -(CH₂)ₘOR^{a}, -(CH₂)ₘC(O)OR^{a}, - (CH₂)ₘC(O)NR^{a}₂, -(CH₂)ₘC(O)R^{a}, -(CH₂)ₘB(OR^{b})₂, -(CH₂)ₘS(O)ₓR^{c}, a silyl group, C₁-₆ alkyl substituted with a silyl group, =O or =CR^{a}₂; or
when n is 1, R² is hydrogen and R¹ is -(CH₂)ₘNR^{a}₂, -(CH₂)ₘOR^{a}, -(CH₂)ₘOPG, - (CH₂)ₘC(O)OR^{a}, -(CH₂)ₘC(O)NR^{a}₂, -(CH₂)ₘC(O)R^{a}, -(CH₂)ₘB(OR^{b})₂, -(CH₂)ₘS(O)ₓR^{c}, a silyl group, C₁-₆ alkyl substituted with a silyl group, -O-aryl, =O or =CR^{a}₂;
when n is 0, one of R¹ and R² is hydrogen and the other is -(CH₂)ₘNR^{a}₂, -(CH₂)ₘOR^{a}, - (CH₂)ₘOPG, -(CH₂)ₘC(O)OR^{a}, -(CH₂)ₘC(O)NR^{a}₂, -(CH₂)ₘC(O)R^{a}, -(CH₂)ₘB(OR^{b})₂, - (CH₂)ₘS(O)ₓR^{c} or =CH₂;
wherein the bond towards R¹ or R² - - is a single bond when R¹ or R² is hydrogen, - (CH₂)ₘNR^{a}₂, -(CH₂)ₘOR^{a}, -(CH₂)ₘOPG, -(CH₂)ₘC(O)OR^{a}, -(CH₂)ₘC(O)NR^{a}₂, - (CH₂)ₘC(O)R^{a}, -(CH₂)ₘB(OR^{b})₂, -(CH₂)ₘS(O)ₓR^{c}, a silyl group, C₁-₆ alkyl substituted with a silyl group or -O-aryl, or a double bond when R¹ or R² is =O, =CH₂ or =CR^{a}₂;
each R^{a} is, independently, hydrogen or C₁-₆ alkyl;
each R^{b} is hydrogen or both R^{b} are taken together to form a boronic acid protecting group;
R^{c} is C₁-₆ alkyl or aryl;
x is 0, 1 or 2;
m is 0 or 1;
R⁵ and R⁶ are each, independently, hydrogen or a hydroxyl protecting group; or R⁵ and R⁶ taken together are a diol protecting group;
R¹⁰ is a nucleobase or nucleobase derivative;
provided that the compound is not any of: 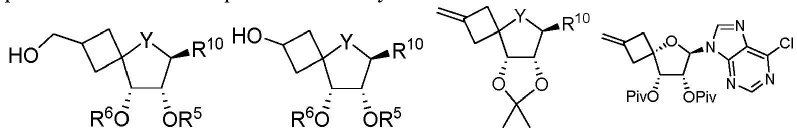 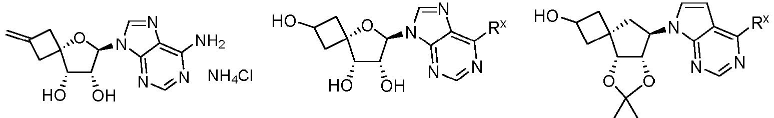
wherein
R⁵ is hydrogen or -C(=O)-C₁₋₄alkyl;
R⁶ is hydrogen or -C(=O)-C₁₋₄alkyl; or
R⁵ and R⁶ taken together are -C(CH₃)₂-
Y is -O- or -CH₂-;
R¹⁰ is 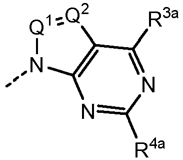
Q¹ is CR^{6a};
Q² is N or CR^{6b};
R^{6a} and R^{6b} each independently represent hydrogen, halogen, C₁₋₄alkyl, -NR^{9a}R^{9b}, or C₁₋₄alkyl substituted with one, two or three halo atoms;
R^{9a} and R^{9b} each independently represent hydrogen or C₁₋₄alkyl;
R^{3a} is hydrogen, halo, -NR^{7a}R⁷⁶, C₁₋₄alkyl, C₂₋₄alkenyl, C₃₋₆cycloalkyl, -OH, or
-O-C₁₋₄alkyl;
R^{7a} is hydrogen;
R^{7b} is hydrogen, C₃₋₆cycloalkyl, or C₁₋₄alkyl;
R^{4a} is hydrogen, halo, -NR^{8a}R^{8b}, or C₁₋₄alkyl; and
R^{x} is 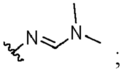
or a salt or solvate thereof.

In another aspect the present invention concerns a compound of formula (H): 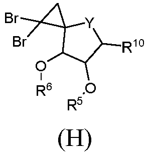 or a salt or solvate thereof, wherein:
R⁵ and R⁶ are each, independently, hydrogen or a hydroxyl protecting group; or R⁵ and R⁶ taken together are a diol protecting group; and
R¹⁰ is a nucleobase or nucleobase derivative.

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

### Detailed description

When describing the compounds of the invention, the terms used are to be construed in accordance with the following definitions, unless a context dictates otherwise.

When any variable occurs more than one time in any constituent or in any formula, its definition in each occurrence is independent of its definition at every other occurrence. Whenever the term "substituted" is used in the present invention, it is meant, unless otherwise is indicated or is clear from the context, to indicate that one or more hydrogens, in particular from 1 to 3 hydrogens, preferably 1 or 2 hydrogens, more preferably 1 hydrogen, on the atom or radical indicated in the expression using "substituted" are replaced with a selection from the indicated group, provided that the normal valency is not exceeded, and that the substitution results in a chemically stable compound, i.e. a compound that is sufficiently robust to survive isolation to a useful degree of purity from a reaction mixture, and formulation into a therapeutic agent.

When two or more substituents are present on a moiety they may, unless otherwise is indicated or is clear from the context, replace hydrogens on the same atom or they may replace hydrogen atoms on different atoms in the moiety.

The prefix "C_{x-y}" (where x and y are integers) as used herein refers to the number of carbon atoms in a given group. Thus, a C₁₋₄alkyl group contains from 1 to 4 carbon atoms, a C₁₋₃alkyl group contains from 1 to 3 carbon atoms and so on.

The term "halo" as a group or part of a group is generic for fluoro, chloro, bromo, iodo unless otherwise is indicated or is clear from the context.

For example the term "C₁₋₆alkyl" as a group or part of a group refers to a hydrocarbyl radical of Formula CₙH₂ₙ₊₁ wherein n is a number ranging from 1 to 6. C₁₋₆alkyl groups comprise from 1 to 6 carbon atoms, preferably from 1 to 4 carbon atoms, preferably from 1 to 3 carbon atoms, more preferably 1 to 2 carbon atoms. C₁₋₆alkyl, C₁₋₄alkyl, and C₁₋₃alkyl groups may be linear or branched and may be substituted as indicated herein. When a subscript is used herein following a carbon atom, the subscript refers to the number of carbon atoms that the named group may contain.

C₁₋₆alkyl includes all linear, or branched alkyl groups with between 1 and 6 carbon atoms, and thus includes methyl, ethyl, n-propyl, i-propyl, 2-methyl-ethyl, butyl and its isomers (e.g. *n-*butyl, isobutyl and tert-butyl), pentyl and its isomers, hexyl and its isomers and the like.

The skilled person will realize that non-limiting examples of suitable -O-C₁₋₄alkyl include methyloxy (also methoxy), ethyloxy (also ethoxy), propyloxy, isopropyloxy, butyloxy, isobutyloxy, sec-butyloxy and tert-butyloxy.

The term "C₂₋₄alkenyl" as used herein as a group or part of a group represents a straight or branched chain hydrocarbon group containing from 2 to 4 carbon atoms and containing a carbon carbon double bond such as, but not limited to, ethenyl, propenyl, butenyl, 1-propen-2-yl, and the like.

The term 'C₃₋₆cycloalkyl' as used herein as a group or part of a group represents cyclic saturated hydrocarbon radicals having from 3 to 6 carbon atoms such as cyclopropyl, cyclobutyl, cyclopentyl or cyclohexyl.

The term "aryl" as used herein as a group or part of a group represents a monocyclic or bicyclic aromatic ring system having from six to fourteen carbon atoms (i.e., C₆₋₁₄aryl). Non-limiting exemplary aryl groups include phenyl, naphthyl, phenanthryl, anthracyl, indenyl, azulenyl, biphenyl, biphenylenyl, and fluorenyl groups. Aryl is preferably chosen from phenyl or naphthyl, more preferably phenyl.

Whenever substituents are represented by chemical structure, "---" represents the bond of attachment to the remainder of the molecule.

Some of the compounds of the invention may exist in their tautomeric form. The term "tautomer" or "tautomeric form" refers to structural isomers of different energies which are interconvertible *via* a low energy barrier. For example, proton tautomers (also known as prototropic tautomers) include interconversions *via* migration of a proton, such as keto-enol and imine-enamine isomerisations. Valence tautomers include interconversions by reorganisation of some of the bonding electrons.

Such forms in so far as they may exist, although not explicitly indicated in the formulae herein, are intended to be included within the scope of the present invention.

As used herein, any chemical formula with bonds shown only as solid lines and not as solid wedged or hashed wedged bonds, or otherwise indicated as having a particular configuration (e.g. *R, S*) around one or more atoms, contemplates each possible stereoisomer, or mixture of two or more stereoisomers. Where the stereochemistry of any particular chiral atom is not specified in the structures shown herein, then all stereoisomers are contemplated and included as the compounds of the invention, either as a pure stereoisomer or as a mixture of two or more stereoisomers.

Compounds described herein include the stereoisomers thereof and the tautomeric forms thereof. However where stereochemistry, as mentioned in the previous paragraph, is specified by bonds which are shown as solid wedged or hashed wedged bonds, or are otherwise indicated as having a particular configuration (e.g. *R*, *S*), then that stereoisomer is so specified and defined. Moreover, when a bond is shown as a wavy bond, unless otherwise defined, this means the stereochemical configuration at the stereocenter is a mixture of stereoisomers. It will be clear this also applies to subgroups of formulae herein.

It follows that a single compound may, where possible, exist in both stereoisomeric and tautomeric form.

The terms "stereoisomers", "stereoisomeric forms" or "stereochemically isomeric forms" hereinbefore or hereinafter are used interchangeably.

Enantiomers are stereoisomers that are non-superimposable mirror images of each other. A 1:1 mixture of a pair of enantiomers is a racemate or racemic mixture.

Atropisomers (or atropoisomers) are stereoisomers which have a particular spatial configuration, resulting from a restricted rotation about a single bond, due to large steric hindrance. All atropisomeric forms of the compounds described herein are intended to be included within the scope of the present invention.

Diastereomers (or diastereoisomers) are stereoisomers that are not enantiomers, i.e. they are not related as mirror images. If a compound contains a double bond, the substituents may be in the E or the Z configuration. Substituents on bivalent cyclic (partially) saturated radicals may have either the cis- or trans-configuration; for example if a compound contains a disubstituted cycloalkyl group, the substituents may be in the cis or trans configuration. Therefore, the invention includes enantiomers, atropisomers, diastereomers, racemates, E isomers, Z isomers, cis isomers, trans isomers and mixtures thereof, whenever chemically possible.

The meaning of all those terms, i.e. enantiomers, atropisomers, diastereomers, racemates, E isomers, Z isomers, cis isomers, trans isomers and mixtures thereof are known to the skilled person.

The absolute configuration is specified according to the Cahn-Ingold-Prelog system. The configuration at an asymmetric atom is specified by either R or S. Resolved stereoisomers whose absolute configuration is not known can be designated by (+) or
(-) depending on the direction in which they rotate plane polarized light. For instance, resolved enantiomers whose absolute configuration is not known can be designated by (+) or (-) depending on the direction in which they rotate plane polarized light.

As an example: 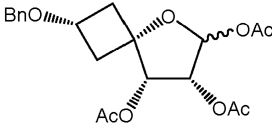 indicates a mixture of 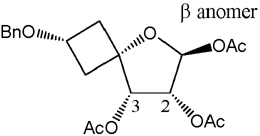 and

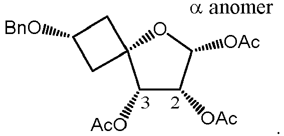

It should be understood that, unless context dictates otherwise, e.g. a Compound of formula (A) 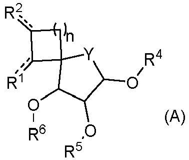 can alternatively be depicted as

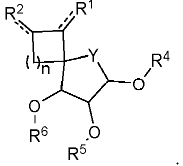

All possible stereochemical configurations are encompassed by these structures.

It should be understood that, unless context dictates otherwise, e.g. a Compound of formula (A) 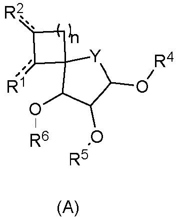 encompasses 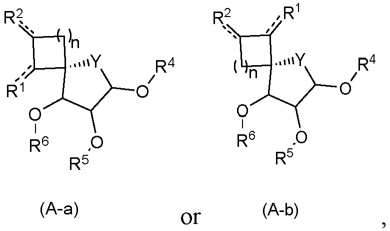 or a mixture thereof.

A skilled person will understand that, unless a context dictates otherwise, this is also true in general for any other general formula of spirobicyclic nucleoside analogues (including cyclopentane containing nucleoside analogues) described in this application. Unless a context dictates otherwise, all such forms are encompassed by a general formula where one possible form is described. All such forms are intended to be included within the scope of the present invention.

It will also be appreciated that any compound described herein containing the structure: 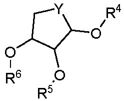 may have any of the following stereochemistries: 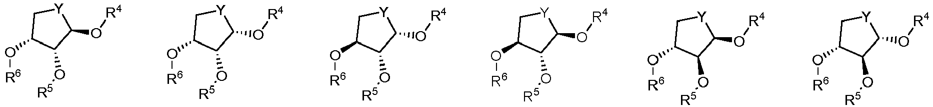 or may be a mixture of any one or more of these stereochemistries. This also applies when OR⁴ is replaced by a nucleobase or nucleobase derivative.

When a specific stereoisomer is identified, this means that said stereoisomer is substantially free, i.e. associated with less than 50%, preferably less than 20%, more preferably less than 10%, even more preferably less than 5%, in particular less than 2% and most preferably less than 1%, of the other stereoisomers. Thus, when a compound is for instance specified as (R), this means that the compound is substantially free of the (S) isomer; when a compound is for instance specified as E, this means that the compound is substantially free of the Z isomer; when a compound is for instance specified as cis, this means that the compound is substantially free of the trans isomer. Stereoisomers may be separated using techniques known in the art, for example chromatographic methods. Functionalisation, for example installation of a suitable protecting group such as a benzoate (conditions eg BzCl, pyridine, dichloromethane) may aid separation of stereoisomers using silica gel chromatography, or reversed phase chromatography, or supercritical fluid (SFC) chromatography.

Salts of the compounds described herein and solvates thereof, may be those wherein the counterion is pharmaceutically acceptable. Salts of acids and bases which are nonpharmaceutically acceptable may also find use, for example, in the preparation or purification of a pharmaceutically acceptable compound. All salts, whether pharmaceutically acceptable or not are included within the ambit of the present invention.

Pharmaceutically-acceptable salts include acid addition salts and base addition salts. Such salts may be formed by conventional means, for example by reaction of a free acid or a free base form with one or more equivalents of an appropriate acid or base, optionally in a solvent, or in a medium in which the salt is insoluble, followed by removal of said solvent, or said medium, using standard techniques (e.g. *in vacuo,* by freeze-drying or by filtration). Salts may also be prepared by exchanging a counter-ion of a compound of the invention in the form of a salt with another counter-ion, for example using a suitable ion exchange resin.

The pharmaceutically acceptable addition salts as mentioned hereinabove or hereinafter are meant to comprise the therapeutically active non-toxic acid and base addition salt forms which the compounds and solvates thereof, are able to form.

Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic, tartaric, citric, methanesulfonic, ethanesulfonic, benzenesulfonic, p-toluenesulfonic, cyclamic, salicylic, p-aminosalicylic, pamoic and the like acids. Conversely said salt forms can be converted by treatment with an appropriate base into the free base form.

The compounds and solvates thereof containing an acidic proton may also be converted into their non-toxic metal or amine addition salt forms by treatment with appropriate organic and inorganic bases.

Appropriate base salt forms comprise, for example, the ammonium salts, the alkali and earth alkaline metal salts, e.g. the lithium, sodium, potassium, magnesium, calcium salts and the like, salts with organic bases, e.g. primary, secondary and tertiary aliphatic and aromatic amines such as methylamine, ethylamine, propylamine, isopropylamine, the four butylamine isomers, dimethylamine, diethylamine, diethanolamine, dipropylamine, diisopropylamine, di-n-butylamine, pyrrolidine, piperidine, morpholine, trimethylamine, triethylamine, tripropylamine, quinuclidine, pyridine, quinoline and isoquinoline; the benzathine, N-methyl-D-glucamine, hydrabamine salts, and salts with amino acids such as, for example, arginine, lysine and the like. Conversely the salt form can be converted by treatment with acid into the free acid form.

The term solvate comprises the hydrates and solvent addition forms which the compounds of the invention are able to form, as well as salts thereof. Examples of such forms are e.g. hydrates, alcoholates and the like.

The compounds of the invention as prepared in the processes described below may be synthesized in the form of mixtures of enantiomers, in particular racemic mixtures of enantiomers, that can be separated from one another following art-known resolution procedures. A manner of separating the enantiomeric forms of the compounds, and salts, and solvates thereof, involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound would be synthesized by stereospecific methods of preparation. These methods will advantageously employ enantiomerically pure starting materials.

The present invention also embraces isotopically-labeled compounds of the present invention which are identical to those recited herein, but for the fact that one or more atoms are replaced by an atom having an atomic mass or mass number different from the atomic mass or mass number usually found in nature (or the most abundant one found in nature).

All isotopes and isotopic mixtures of any particular atom or element as specified herein are contemplated within the scope of the compounds of the invention, either naturally occurring or synthetically produced, either with natural abundance or in an isotopically enriched form. Exemplary isotopes that can be incorporated into compounds of the invention include isotopes of hydrogen, carbon, nitrogen, oxygen, phosphorus, sulfur, fluorine, chlorine and iodine, such as ²H, ³H, ¹¹C, ¹³C, ¹⁴C, ¹³N, ¹⁵O, ¹⁷O, ¹⁸O, ³²P, ³³P, ³⁵S, ¹⁸F, ³⁶Cl, ¹²²I, ¹²³I, ¹²⁵I, ¹³¹I, ⁷⁵Br, ⁷⁶Br, ⁷⁷Br and ⁸²Br. Preferably, the radioactive isotope is selected from the group of ²H, ³H, ¹¹C and ¹⁸F. More preferably, the radioactive isotope is ²H. In particular, deuterated compounds are intended to be included within the scope of the present invention.

Certain isotopically-labeled compounds of the present invention (e.g., those labeled with ³H and ¹⁴C) are useful for substrate tissue distribution assays. Tritiated (³H) and carbon-14 (¹⁴C) isotopes are useful for their ease of preparation and detectability. Further, substitution with heavier isotopes such as deuterium (i.e., ²H may afford certain therapeutic advantages resulting from greater metabolic stability (e.g., increased *in vivo* half-life or reduced dosage requirements) and hence may be preferred in some circumstances. Positron emitting isotopes such as ¹⁵O, ¹³N, ¹¹C and ¹⁸F are useful for positron emission tomography (PET) studies to examine substrate receptor occupancy.

A protecting group as referenced herein may be any suitable hydroxyl protection group. Suitable protecting groups are known in the art, for example from Wuts, P.G.M. and Greene, T.W.: Protective Groups in Organic Synthesis, Fourth Edition, Wiley, New York, 2006. For example, a hydroxyl protecting group may be a silyl group, C₁₋₄alkyl, C₁₋₄alkyl-O-C₁₋₄alkyl, tetrahydropyranyl, allyl, benzyl, -CH₂-naphthyl, or benzoyl, -C(=O)-C₁₋₄alkyl, or -C(=O)-phenyl; wherein benzyl, -CH₂-naphthyl, and benzoyl, are optionally substituted with one or two substituents each independently selected from -CH₃ and -OCH₃.

A silyl group as referenced herein may, for example, be t-butyldimethylsilyl, t-butyldiphenylsilyl, triisopropylsilyl, trimethylsilyl, or triethylsilyl.

A diol protecting group may, for example, be -C(C₁₋₄alkyl)₂- (such as (-C(CH₃)₂)-. It will be appreciated to a person of skill in the art that R⁴ and R⁵ may be taken together to form a diol protecting group when the stereochemistry of the compound is such that -OR⁴ and -OR⁵ are in a *cis* configuration. Likewise, it will be appreciated to a person of skill in the art that R⁵ and R⁶ may be taken together to form a diol protecting group when the stereochemistry of the compound is such that -OR⁵ and -OR⁶ are in a *cis* configuration.

A boronic acid protecting group may, for example, be catechol borane, pinacol borane, N-methyliminodiacetic acid (MIDA) boronate, neopentylglycol borane, pinanediol borane, biscyclohexyldiol borane, or 1-(4-methoxyphenyl)-2-methylpropane-1,2-diol (MPMP-diol) borane.

An amino protecting group may, for example, be Benzoyl (Bz), tert-butyloxycarbonyl (Boc), benzyl (Bn), acetyl (Ac), diphenylcarbamoyl, benzyloxycarbonyl (cbz), fluorenylmethyloxycarbonyl (Fmoc), CF₃C(O)-, trityl (trt), phthaloyl (phth), or =CHN(CH₃)₂.

A nucleobase or nucleobase derivative as referenced herein encompasses both pyrimidine and purine nitrogenous bases, including modified and protected forms thereof. For example, nucleobases as referenced herein include, but are not limited to, adenine, cytosine, guanine, thymine, 6-chloro purine, uracil, adenine, 7-methyl guanine, xanthine, hypoxanthine, purine, 2,6-diaminopurine,5,6-dihydrouracil, 6,8-diaminopurine inosine, 5-methylcytosine, and 5-hydroxymethylcyctosine. A modified or protected nucleobase may, for example, be one in which an amino protecting group (e.g. Benzoyl (Bz), tert-butyloxycarbonyl (Boc), benzyl (Bn), acetyl (Ac), diphenylcarbamoyl, benzyloxycarbonyl (cbz), fluorenylmethyloxycarbonyl (Fmoc), CF₃C(O)-, trityl (trt), phthaloyl (phth), =CHN(CH₃)₂) is present, or an amino (-NH₂) group is replaced by halo (e.g. chloro). Such a modified or protected nucleobase may also be referred to as a nucleobase precursor). By means of example, a nucleobase or nucleobase derivative as referenced herein may include any purine or pyrimidine nitrogenous base, such as any of the nucleobases referenced above, wherein one or more substituents R is present on the heteroaryl core of the nitrogenous base, in place of a hydrogen or another substituent moiety, wherein R is selected from, but not limited, to halo, -NR*R**, C₁₋₄alkyl, C₂₋₄alkenyl, C₃₋₆cycloalkyl, -OH, -O-C₁₋₄alkyl, C₁₋₄alkyl substituted with one, two or three halo atom, wherein R* is hydrogen, C₁₋₄alkyl or -C(O)O-C₁₋₄alkyl and R** is hydrogen, C₃₋₆cycloalkyl, C₁₋₄alkyl, or -C(O)O-C₁₋₄alkyl.

"TBDPS" means tert-butyl diphenyl silyl; "Piv" means pivaloyl; "Bn" means benzyl; "Et" means ethyl; "Me" means methyl; "PMB" means p-methoxybenzyl, "TMS" means trimethylsilyl; "tBu-O" means tert-butoxy; 'Pr" means n-propyl.

In one aspect, the invention relates to a compound of Formula (I) 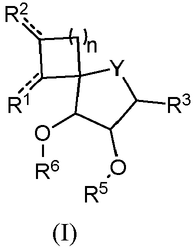 or a salt or solvate thereof, wherein:
Y is -O- or -CH₂-;
n is 0 or 1;
when n is 1, R¹ is hydrogen and R² is -(CH₂)ₘNR^{a}₂, -(CH₂)ₘOR^{a}, -(CH₂)ₘC(O)OR^{a}, - (CH₂)ₘC(O)NR^{a}₂, -(CH₂)ₘC(O)R^{a}, -(CH₂)ₘB(OR^{b})₂, -(CH₂)ₘS(O)ₓR^{c}, a silyl group, C₁-₆ alkyl substituted with a silyl group, =O or =CR^{a}₂; or
when n is 1, R² is hydrogen and R¹ is -(CH₂)ₘNR^{a}₂, -(CH₂)ₘOR^{a}, -(CH₂)ₘOPG, - (CH₂)ₘC(O)OR^{a}, -(CH₂)ₘC(O)NR^{a}₂, -(CH₂)ₘC(O)R^{a}, -(CH₂)ₘB(OR^{b})₂, -(CH₂)ₘS(O)ₓR^{c}, a silyl group, C₁-₆ alkyl substituted with a silyl group, -O-aryl, =O or =CR^{a}₂;
when n is 0, one of R¹ and R² is hydrogen and the other is -(CH₂)ₘNR^{a}₂,-(CH₂)ₘOR^{a}, - (CH₂)ₘOPG, -(CH₂)ₘC(O)OR^{a}, -(CH₂)ₘC(O)NR^{a}₂, -(CH₂)ₘC(O)R^{a}, -(CH₂)ₘB(OR^{b})₂, - (CH₂)ₘS(O)ₓR^{c} or =CH₂;
wherein the bond towards R¹ or R² - - is a single bond when R¹ or R² is hydrogen, - (CH₂)ₘNR^{a}₂, -(CH₂)ₘOR^{a}, -(CH₂)ₘOPG, -(CH₂)ₘC(O)OR^{a}, -(CH₂)ₘC(O)NR^{a}₂, -
(CH₂)ₘC(O)R^{a}, -(CH₂)ₘB(OR^{b})₂, -(CH₂)ₘS(O)ₓR^{c}, a silyl group, C₁-₆ alkyl substituted with a silyl group or -O-aryl, or a double bond when R¹ or R² is =O, =CH₂ or =CR^{a}₂;
each R^{a} is, independently, hydrogen or C₁-₆ alkyl;
each R^{b} is hydrogen or both R^{b} are taken together to form a boronic acid protecting group;
R^{c} is C₁₋₆ alkyl or aryl;
PG is a hydroxyl protecting group;
m is 0 or 1;
x is 0, 1 or 2;
R³ is -OR⁴ or R¹⁰;
R¹⁰ is a nucleobase or nucleobase derivative.
R⁴, R⁵ and R⁶ are each independently hydrogen or a hydroxyl protecting group;
or R⁵ and R⁶ taken together are a diol protecting group;
or R⁴ and R⁵ taken together are a diol protecting group;
provided that:
   (a) the compound is not a compound of formula: 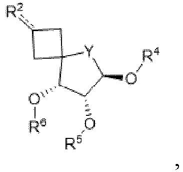 wherein Y is -O- or -CH₂-; R⁴ is hydrogen or a hydroxyl protecting group such as for example C₁₋₄alkyl, t-butyldimethylsilyl, C₁₋₄alkyl-O-C₁₋₄alkyl, tetrahydropyranyl, allyl, t-butyldiphenylsilyl, benzyl, -C(=O)-C₁₋₄alkyl, or -C(=O)-phenyl; R² is -OH, =O, -CH₂-OH or =CH₂; and R⁵ and R⁶ are each independently -C(=O)-C₁₋₄alkyl, benzoyl, benzyl, or -CH₂-naphthyl, wherein benzyl, -CH₂-naphthyl, and benzoyl are optionally substituted with one or two substituents each independently selected from -CH₃ and -OCH₃; or R⁵ and R⁶ taken together are -C(C₁₋₄alkyl)₂-; or wherein Y is -O- or -CH₂, R⁴ is methyl, R² is =O, or =CH₂, and R⁵ and R⁶ are each independently hydrogen or -C(=O)-C₁₋₄alkyl; and
   (b) the compound is not a compound of formula: 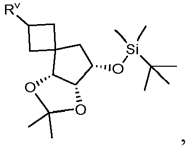 wherein R^{v} is =O, -OH or =CH₂; and
   (c) the compound is not a compound of formula: 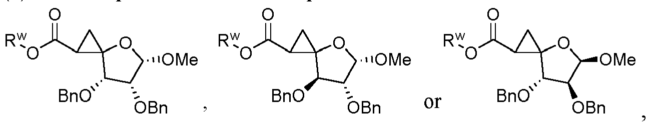 wherein R^{w} is methyl or H; and
   (d) the compound is not a compound of formula: 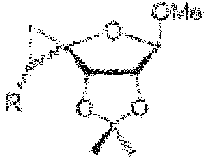 wherein R is CO₂Et or CH₂OH or any stereoisomer thereof; and
   (e) the compound is not any of: 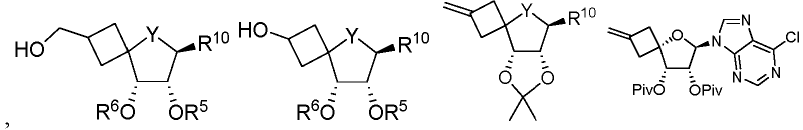 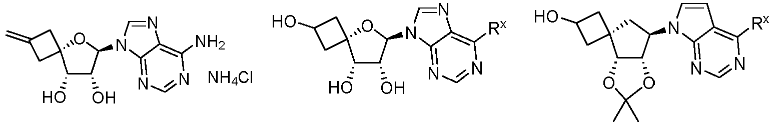 wherein
      R⁵ is hydrogen or -C(=O)-C₁₋₄alkyl;
      R⁶ is hydrogen or -C(=O)-C₁₋₄alkyl; or R⁵ and R⁶ taken together are -C(CH₃)₂-Y is -O- or -CH₂-;
      R¹⁰ is 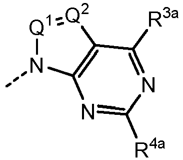
      Q¹ is CR^{6a};
      Q² is N or CR^{6b};
      R^{6a} and R^{6b} each independently represent hydrogen, halogen, C₁₋₄alkyl, -NR^{9a}R^{9b}, or C₁₋₄alkyl substituted with one, two or three halo atoms;
      R^{9a} and R^{9b} each independently represent hydrogen or C₁₋₄alkyl;
      R^{3a} is hydrogen, halo, -NR^{7a}R^{7b}, C₁₋₄alkyl, C₂₋₄alkenyl, C₃₋₆cycloalkyl, -OH, or
      -O-C₁₋₄alkyl;
      R^{7a} is hydrogen;
      R^{7b} is hydrogen, C₃₋₆cycloalkyl, or C₁₋₄alkyl;
      R^{4a} is hydrogen, halo, -NR^{8a}R^{8b}, or C₁₋₄alkyl; and
      R^{x} is 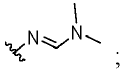
      or a salt or solvate thereof.
In one aspect, the invention relates to a compound of Formula (A) 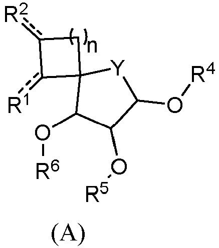 or a salt or solvate thereof, wherein:
   Y is -O- or -CH₂-;
   n is 0 or 1;
   when n is 1, R¹ is hydrogen and R² is -(CH₂)ₘNR^{a}₂, -(CH₂)ₘOR^{a}, -(CH₂)ₘC(O)OR^{a}, - (CH₂)ₘC(O)NR^{a}₂, -(CH₂)ₘC(O)R^{a}, -(CH₂)ₘB(OR^{b})₂, -(CH₂)ₘS(O)ₓR^{c}, a silyl group, C₁-₆ alkyl substituted with a silyl group, =O or =CR^{a}₂; or
   when n is 1, R² is hydrogen and R¹ is -(CH₂)ₘNR^{a}₂, -(CH₂)ₘOR^{a}, -(CH₂)ₘOPG, - (CH₂)ₘC(O)OR^{a}, -(CH₂)ₘC(O)NR^{a}₂, -(CH₂)ₘC(O)R^{a}, -(CH₂)ₘB(OR^{b})₂, -(CH₂)ₘS(O)ₓR^{c}, a silyl group, C₁-₆ alkyl substituted with a silyl group, -O-aryl, =O or =CR^{a}₂;
   when n is 0, one of R¹ and R² is hydrogen and the other is -(CH₂)ₘNR^{a}₂,-(CH₂)ₘOR^{a}, - (CH₂)ₘOPG, -(CH₂)ₘC(O)OR^{a}, -(CH₂)ₘC(O)NR^{a}₂, -(CH₂)ₘC(O)R^{a}, -(CH₂)ₘB(OR^{b})₂, - (CH₂)ₘS(O)ₓR^{c} or =CH₂;
   wherein the bond towards R¹ or R² - - is a single bond when R¹ or R² is hydrogen, - (CH₂)ₘNR^{a}₂, -(CH₂)ₘOR^{a}, -(CH₂)ₘOPG, -(CH₂)ₘC(O)OR^{a}, -(CH₂)ₘC(O)NR^{a}₂, - (CH₂)ₘC(O)R^{a}, -(CH₂)ₘB(OR^{b})₂, -(CH₂)ₘS(O)ₓR^{c}, a silyl group, C₁-₆ alkyl substituted with a silyl group or -O-aryl, or a double bond when R¹ or R² is =O, =CH₂ or =CR^{a}₂;
   each R^{a} is, independently, hydrogen or C₁-₆ alkyl;
   each R^{b} is hydrogen or both R^{b} are taken together to form a boronic acid protecting group;
   R^{c} is C₁₋₆ alkyl or aryl;
   PG is a hydroxyl protecting group;
   m is 0 or 1;
   x is 0, 1 or 2;
   R⁴, R⁵ and R⁶ are each independently hydrogen or a hydroxyl protecting group;
   or R⁵ and R⁶ taken together are a diol protecting group;
   or R⁴ and R⁵ taken together are a diol protecting group;
   provided that:
      (a) the compound is not a compound of formula: 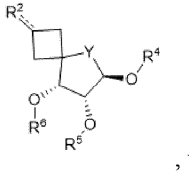 wherein Y is -O- or -CH₂-; R⁴ is hydrogen or a hydroxyl protecting group such as for example C₁₋₄alkyl, t-butyldimethylsilyl, C₁₋₄alkyl-O-C₁₋₄alkyl, tetrahydropyranyl, allyl, t-butyldiphenylsilyl, benzyl, -C(=O)-C₁₋₄alkyl, or -C(=O)-phenyl; R² is -OH, =O, -CH₂-OH or =CH₂; and R⁵ and R⁶ are each independently -C(=O)-C₁₋₄alkyl, benzoyl, benzyl, or -CH₂-naphthyl, wherein benzyl, -CH₂-naphthyl, and benzoyl are optionally substituted with one or two substituents each independently selected from -CH₃ and -OCH₃; or R⁵ and R⁶ taken together are -C(C₁₋₄alkyl)₂-; or wherein Y is -O- or -CH₂, R⁴ is methyl, R² is =O, or =CH₂, and R⁵ and R⁶ are each independently hydrogen or -C(=O)-C₁₋₄alkyl; and
      (b) the compound is not a compound of formula: 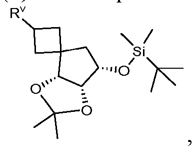 wherein R^{v} is =O, -OH or =CH₂; and
      (c) the compound is not a compound of formula: 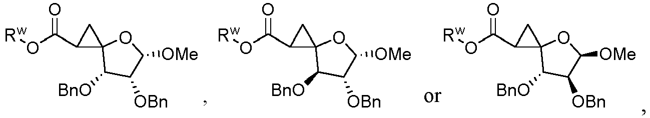 wherein R^{w} is methyl or H; and
      (d) the compound is not a compound of formula: 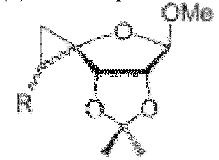 wherein R is CO₂Et or CH₂OH or any stereoisomer thereof.

In an embodiment, the present invention relates to those compounds of Formula (A) and salts, and solvates thereof, or any subgroup thereof as mentioned in any of the other embodiments herein, wherein the compound is not a compound of formula: 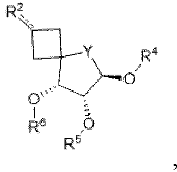 wherein Y is -O- or -CH₂-; R⁴ is any chemical moiety, R² is -OH, =O, - CH₂-OH or =CH₂; and R⁵ and R⁶ are each independently -C(=O)-C₁₋₄alkyl, benzoyl, benzyl, or -CH₂-naphthyl, wherein benzyl, -CH₂-naphthyl, and benzoyl are optionally substituted with one or two substituents each independently selected from -CH₃ and -OCH₃; or R⁵ and R⁶ taken together are -C(C₁₋₄alkyl)₂- or wherein when R⁴ is methyl, R² is =O, or =CH₂, and R⁵ and R⁶ are each independently hydrogen or -C(=O)-C₁₋₄alkyl.

In an embodiment, the present invention relates to those compounds of Formula (A) and salts, and solvates thereof, or any subgroup thereof as mentioned in any of the other embodiments herein, wherein the compound is a compound of formula: 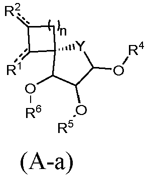 or a salt or solvate thereof.

In an embodiment, the present invention relates to those compounds of Formula (A) and salts, and solvates thereof, or any subgroup thereof as mentioned in any of the other embodiments herein, wherein the compound is a compound of formula: 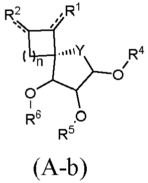 or a salt or solvate thereof.

In an embodiment, the present invention relates to those compounds of Formula (A) and salts, and solvates thereof, or any subgroup thereof as mentioned in any of the other embodiments herein, wherein the compound is a compound of formula (A-1), (A-2), (A-3), (A-4), (A-5) or (A-6): 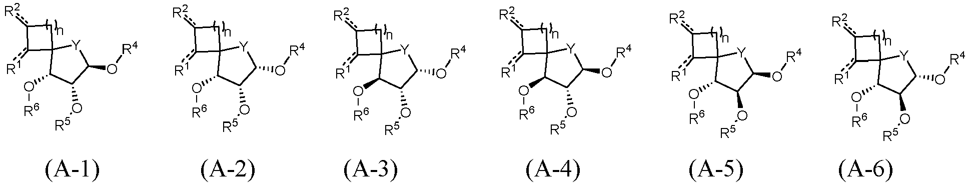 or a salt or solvate thereof.

In an embodiment, the present invention relates to those compounds of Formula (A) and salts, and solvates thereof, or any subgroup thereof as mentioned in any of the other embodiments herein, wherein the compound is a compound of formula (A-1a), (A-2a), (A-3a), (A-4a), (A-5a) or (A-6a): 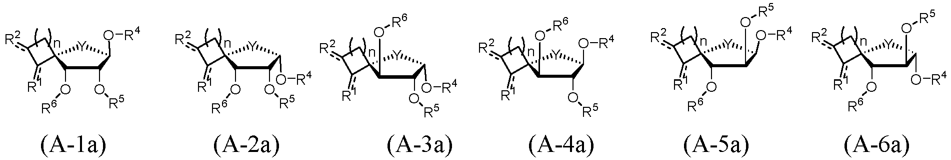 or a salt or solvate thereof.

In an embodiment, the present invention relates to those compounds of Formula (A) and salts, and solvates thereof, or any subgroup thereof as mentioned in any of the other embodiments herein, wherein the compound is a compound of formula (A-1b), (A-2b), (A-3b), (A-4b), (A-5b) or (A-6b): 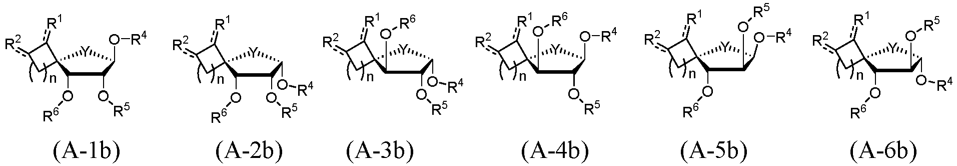 or a salt or solvate thereof.

In an embodiment, the present invention relates to those compounds of Formula (I) and salts, and solvates thereof, wherein the compound has a stereochemistry as depicted for those compounds of any of Formula (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b).

In an embodiment, the present invention relates to those compounds of any of (A-1a), (A-3a), (A-1b), or (A-3b), and salts, and solvates thereof, or any subgroup thereof as mentioned in any of the other embodiments herein.

In an embodiment, the present invention relates to those compounds of any of (A-1a), (A-3a), (A-5a), (A-1b), (A-3b), or (A-5b), and salts, and solvates thereof, or any subgroup thereof as mentioned in any of the other embodiments herein.

In an embodiment, the present invention relates to those compounds of any of Formula (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein:
Y is -O- or -CH₂-;
n is 0 or 1;
when n is 1, R¹ is hydrogen and R² is -(CH₂)ₘNR^{a}₂, -(CH₂)ₘC(O)OR^{a}, -(CH₂)ₘC(O)NR^{a}₂, - (CH₂)ₘC(O)R^{a}, -(CH₂)ₘB(OR^{b})₂, -(CH₂)ₘS(O)ₓR^{c}, a silyl group, C₁-₆ alkyl substituted with a silyl group; or
when n is 1, R² is hydrogen and R¹ is -(CH₂)ₘNR^{a}₂, -(CH₂)ₘOR^{a}, -(CH₂)ₘOPG, - (CH₂)ₘC(O)OR^{a}, -(CH₂)ₘC(O)NR^{a}₂, -(CH₂)ₘC(O)R^{a}, -(CH₂)ₘB(OR^{b})₂, -(CH₂)ₘS(O)ₓR^{c}, a silyl group, C₁-₆ alkyl substituted with a silyl group, -O-aryl, =O or =CR^{a}₂;
when n is 0, one of R¹ and R² is hydrogen and the other is -(CH₂)ₘNR^{a}₂,-(CH₂)ₘOR^{a}, - (CH₂)ₘOPG, -(CH₂)ₘC(O)OR^{a}, -(CH₂)ₘC(O)NR^{a}₂, -(CH₂)ₘC(O)R^{a}, -(CH₂)ₘB(OR^{b})₂, - (CH₂)ₘS(O)ₓR^{c} or =CH₂;
wherein the bond towards R¹ or R² - - is a single bond when R¹ or R² is hydrogen, - (CH₂)ₘNR^{a}₂, -(CH₂)ₘOR^{a}, -(CH₂)ₘOPG, -(CH₂)ₘC(O)OR^{a}, -(CH₂)ₘC(O)NR^{a}₂, - (CH₂)ₘC(O)R^{a}, -(CH₂)ₘB(OR^{b})₂, -(CH₂)ₘS(O)ₓR^{c}, a silyl group, C₁-₆ alkyl substituted with a silyl group or -O-aryl, or a double bond when R¹ or R² is =O, =CH₂ or =CR^{a}₂;
each R^{a} is, independently, hydrogen or C₁-₆ alkyl;
each R^{b} is hydrogen or both R^{b} are taken together to form a boronic acid protecting group;
R^{c} is C₁₋₆ alkyl or aryl;
PG is a hydroxyl protecting group;
m is 0 or 1;
x is 0, 1 or 2;
R⁴, R⁵ and R⁶ are each independently hydrogen or a hydroxyl protecting group;
or R⁵ and R⁶ taken together are a diol protecting group;
or R⁴ and R⁵ taken together are a diol protecting group;
provided that the compound is not a compound of formula: 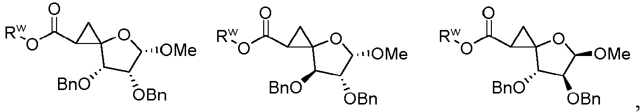 wherein R^{w} is methyl or H; or 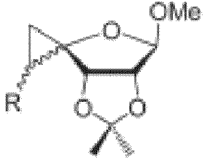 wherein R is CO₂Et or CH₂OH or any stereoisomer thereof.

In an embodiment, the present invention relates to those compounds of any of Formula (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein:
Y is -O- or -CH₂-;
n is 0 or 1;
when n is 1, R¹ is hydrogen and R² is -(CH₂)ₘNR^{a}₂, -(CH₂)ₘOR^{a}, -(CH₂)ₘC(O)OR^{a}, - (CH₂)ₘC(O)NR^{a}₂, -(CH₂)ₘC(O)R^{a}, -(CH₂)ₘB(OR^{b})₂, -(CH₂)ₘS(O)ₓR^{c}, a silyl group, C₁-₆ alkyl substituted with a silyl group, =O or =CR^{a}₂; or
when n is 1, R² is hydrogen and R¹ is -(CH₂)ₘNR^{a}₂, -(CH₂)ₘOR^{a}, -(CH₂)ₘOPG, - (CH₂)ₘC(O)OR^{a}, -(CH₂)ₘC(O)NR^{a}₂, -(CH₂)ₘC(O)R^{a}, -(CH₂)ₘB(OR^{b})₂, -(CH₂)ₘS(O)ₓR^{c}, a silyl group, C₁-₆ alkyl substituted with a silyl group, -O-aryl, =O or =CR^{a}₂;
when n is 0, one of R¹ and R² is hydrogen and the other is -(CH₂)ₘNR^{a}₂, -OR^{a}, -OPG, - (CH₂)C(O)OR^{a}, -(CH₂)ₘC(O)NR^{a}₂, -(CH₂)ₘC(O)R^{a}, -(CH₂)ₘB(OR^{b})₂, -(CH₂)ₘS(O)ₓR^{c} or =CH₂;
wherein the bond towards R¹ or R² - - is a single bond when R¹ or R² is hydrogen, - (CH₂)ₘNR^{a}₂, -(CH₂)ₘOR^{a}, -(CH₂)ₘOPG, -(CH₂)ₘC(O)OR^{a}, -(CH₂)ₘC(O)NR^{a}₂, - (CH₂)ₘC(O)R^{a}, -(CH₂)ₘB(OR^{b})₂, -(CH₂)ₘS(O)ₓR^{c}, a silyl group, C₁-₆ alkyl substituted with a silyl group or -O-aryl, or a double bond when R¹ or R² is =O, =CH₂ or =CR^{a}₂;
each R^{a} is, independently, hydrogen or C₁-₆ alkyl;
each R^{b} is hydrogen or both R^{b} are taken together to form a boronic acid protecting group;
R^{c} is C₁₋₆ alkyl or aryl;
PG is a hydroxyl protecting group;
m is 0 or 1;
x is 0, 1 or 2;
R⁴, R⁵ and R⁶ are each independently hydrogen or a hydroxyl protecting group;
or R⁵ and R⁶ taken together are a diol protecting group;
or R⁴ and R⁵ taken together are a diol protecting group;
provided that:
   (a) the compound is not a compound of formula: 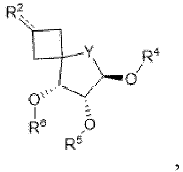 wherein Y is -O- or -CH₂-; R⁴ is hydrogen or a hydroxyl protecting group such as for example C₁₋₄alkyl, t-butyldimethylsilyl, C₁₋₄alkyl-O-C₁₋₄alkyl, tetrahydropyranyl, allyl, t-butyldiphenylsilyl, benzyl, -C(=O)-C₁₋₄alkyl, or -C(=O)-phenyl; R² is -OH, =O, -CH₂-OH or =CH₂; and R⁵ and R⁶ are each independently -C(=O)-C₁₋₄alkyl, benzoyl, benzyl, or -CH₂-naphthyl, wherein benzyl, -CH₂-naphthyl, and benzoyl are optionally substituted with one or two substituents each independently selected from -CH₃ and -OCH₃; or R⁵ and R⁶ taken together are -C(C₁₋₄alkyl)₂-; or wherein Y is -O- or -CH₂, R⁴ is methyl, R² is =O, or =CH₂, and R⁵ and R⁶ are each independently hydrogen or -C(=O)-C₁₋₄alkyl; and
   (b) the compound is not a compound of formula: 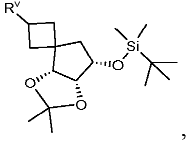 wherein R^{v} is =O, -OH or =CH₂.

In an embodiment, the present invention relates to those compounds of any of Formula (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, wherein:
Y is -O- or -CH₂-;
n is 0 or 1;
when n is 1, R¹ is hydrogen and R² is -(CH₂)ₘNR^{a}₂, -(CH₂)ₘC(O)OR^{a}, -(CH₂)ₘC(O)NR^{a}₂, - (CH₂)ₘC(O)R^{a}, -(CH₂)ₘB(OR^{b})₂, -(CH₂)ₘS(O)ₓR^{c}, a silyl group, C₁-₆ alkyl substituted with a silyl group; or
when n is 1, R² is hydrogen and R¹ is -(CH₂)ₘNR^{a}₂, -(CH₂)ₘOR^{a}, -(CH₂)ₘOPG, - (CH₂)ₘC(O)OR^{a}, -(CH₂)ₘC(O)NR^{a}₂, -(CH₂)ₘC(O)R^{a}, -(CH₂)ₘB(OR^{b})₂, -(CH₂)ₘS(O)ₓR^{c}, a silyl group, C₁-₆ alkyl substituted with a silyl group, -O-aryl, =O or =CR^{a}₂;
when n is 0, one of R¹ and R² is hydrogen and the other is -(CH₂)ₘNR^{a}₂, -OR^{a}, -OPG, -
(CH₂)C(O)OR^{a}, -(CH₂)ₘC(O)NR^{a}₂, -(CH₂)ₘC(O)R^{a}, -(CH₂)ₘB(OR^{b})₂, -(CH₂)ₘS(O)ₓR^{c} or =CH₂;
wherein the bond towards R¹ or R² - - is a single bond when R¹ or R² is hydrogen, - (CH₂)ₘNR^{a}₂, -(CH₂)ₘOR^{a}, -(CH₂)ₘOPG, -(CH₂)ₘC(O)OR^{a}, -(CH₂)ₘC(O)NR^{a}₂, - (CH₂)ₘC(O)R^{a}, -(CH₂)ₘB(OR^{b})₂, -(CH₂)ₘS(O)ₓR^{c}, a silyl group, C₁-₆ alkyl substituted with a silyl group or -O-aryl, or a double bond when R¹ or R² is =O, =CH₂ or =CR^{a}₂;
each R^{a} is, independently, hydrogen or C₁-₆ alkyl;
each R^{b} is hydrogen or both R^{b} are taken together to form a boronic acid protecting group; R^{c} is C₁₋₆ alkyl or aryl;
PG is a hydroxyl protecting group;
m is 0 or 1;
x is 0, 1 or 2; and
R⁴, R⁵ and R⁶ are each independently hydrogen or a hydroxyl protecting group;
or R⁵ and R⁶ taken together are a diol protecting group;
or R⁴ and R⁵ taken together are a diol protecting group.

In an embodiment, the present invention relates to those compounds of any of Formula (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein n is 1.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein n is 1, R¹ is hydrogen and R² -(CH₂)ₘNR^{a}₂, -(CH₂)ₘOR^{a}, -(CH₂)ₘC(O)OR^{a}, -(CH₂)ₘC(O)NR^{a}₂, - (CH₂)ₘC(O)R^{a}, -(CH₂)ₘS(O)ₓR^{c}, a silyl group, C₁-₆ alkyl substituted with a silyl group, =O or =CR^{a}₂; or R² is hydrogen and R¹ is -(CH₂)ₘNR^{a}₂, -(CH₂)ₘOR^{a}, -(CH₂)ₘOPG, - (CH₂)ₘC(O)OR^{a}, -(CH₂)ₘC(O)NR^{a}₂, -(CH₂)ₘC(O)R^{a}, -(CH₂)ₘS(O)ₓR^{c}, a silyl group, C₁-₆ alkyl substituted with a silyl group, -O-aryl, =O or =CR^{a}₂.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein n is 1, R¹ is hydrogen and R² -(CH₂)ₘNR^{a}₂, -(CH₂)ₘOR^{a}, -C(O)OR^{a}, -C(O)NR^{a}₂, -C(O)R^{a}, - (CH₂)ₘS(O)ₓR^{c}, a silyl group, C₁-₆ alkyl substituted with a silyl group, =O or =CR^{a}₂; or R² is hydrogen and R¹ is -(CH₂)ₘNR^{a}₂, -(CH₂)ₘOR^{a}, -(CH₂)ₘOPG, -C(O)OR^{a}, -C(O)NR^{a}₂, - C(O)R^{a}, -(CH₂)ₘS(O)ₓR^{c}, a silyl group, C₁-₆ alkyl substituted with a silyl group, -O-aryl, =O or =CR^{a}₂.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein n is 1, R¹ is -(CH₂)ₘNR^{a}₂, -(CH₂)ₘOR^{a}, -(CH₂)ₘOPG, -C(O)OR^{a}, -C(O)NR^{a}₂, -C(O)R^{a}, =O or =CR^{a}₂; and R² is hydrogen.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein n is 1, R¹ is -NH₂, -CH₂NH₂, -OH, -CH₂OH, =O or =; and R² is hydrogen.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein n is 1, R¹ is -OH, -CH₂OH, =O or =; and R² is hydrogen.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein n is 1, R¹ is hydrogen; and R² is -(CH₂)ₘNR^{a}₂, -(CH₂)ₘOR^{a}, -C(O)OR^{a}, -C(O)NR^{a}₂, -C(O)R^{a}, =O or =CR^{a}₂.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein n is 1, R² is -NH₂, -CH₂NH₂, -OH, -CH₂OH, =O or =; and R¹ is hydrogen.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein n is 1, R² is -OH, -CH₂OH, =O or =; and R¹ is hydrogen.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein n is 1, R¹ is hydrogen; and R² is -(CH₂)ₘNR^{a}₂ or -C(O)NR^{a}₂.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein n is 1, R² is -NH₂ or -CH₂NH₂; and R¹ is hydrogen.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein n is 1, one of R¹ and R² is hydrogen and the other is -NR^{a}₂, -CH₂NR^{a}₂, -OR^{a}, -CH₂OR^{a}, -C(O)OR^{a}, - C(O)R^{a}, S(O)ₓR^{c}, =O or =CR^{a}₂.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein n is 1, one of R¹ and R² is hydrogen and the other is -NR^{a}₂, -CH₂NR^{a}₂, -OR^{a}, -CH₂OR^{a}, -C(O)OR^{a}, =O or =CR^{a}₂.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein n is 1, R¹ is -NR^{a}₂, -CH₂NR^{a}₂, -OR^{a}, -CH₂OR^{a}, -C(O)OR^{a}, -C(O)R^{a}, -B(OR^{b})₂, S(O)ₓR^{c}, =O or =CR^{a}₂; and R² is hydrogen.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein n is 1, R¹ is hydrogen; R² is -NR^{a}₂, -CH₂NR^{a}₂, -CH₂OR^{a}, -C(O)R^{a}, -B(OR^{b})₂, S(O)ₓR^{c}, -C(O)OR^{d}, - OR^{d} or =C(R^{a})(R^{d}); R^{a} is hydrogen or C₁-₆ alkyl; and R^{d} is C₁-₆ alkyl.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein n is 0.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein n is 0, one of R¹ and R² is hydrogen and the other is -(CH₂)ₘNR^{a}₂, -(CH₂)ₘOR^{a}, -(CH₂)ₘOPG, - (CH₂)ₘC(O)OR^{a}, -(CH₂)ₘC(O)NR^{a}₂, -(CH₂)ₘC(O)R^{a}, -(CH₂)ₘB(OR^{b})₂, or =CH₂.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein n is 0, one of R¹ and R² is hydrogen and the other is - -(CH₂)ₘNR^{a}₂, -(CH₂)ₘOR^{a}, -(CH₂)ₘOPG, - C(O)OR^{a}, -C(O)NR^{a}₂ or -B(OR^{b})₂.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein n is 0, one of R¹ and R² is hydrogen and the other is -CH₂NR^{a}₂, -OR^{a}, -CH₂OR^{a}, -C(O)OR^{a}, - C(O)R^{a}, -B(OR^{b})₂ or =CH₂.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein n is 0, one of R¹ and R² is hydrogen and the other is -CH₂NR^{a}₂, -OR^{a}, -CH₂OR^{a}, -C(O)OR^{a} or - B(OR^{b})₂.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein n is 0, one of R¹ and R² is hydrogen and the other is -OH, -CH₂OH, -CH₂NH₂, -C(O)OH or C(O)OEt.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein n is 0, one of R¹ and R² is hydrogen and the other is -OH, or -CH₂NH₂.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein - (CH₂)ₘNR^{a}₂, when present, is -(CH₂)ₘNHC₁-₆alkyl or -(CH₂)ₘNH₂.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein when R¹ or R² is -(CH₂)ₘOPG, PG is a silyl group, -C(=O)-C₁₋₄alkyl, or -C(=O)-phenyl.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein R^{a} is H, methyl, ethyl or propyl.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein -NR^{a}₂, when present, is -NHR^{a}.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein -NR^{a}₂, -CH₂NR^{a}₂, -OR^{a}, -CH₂OR^{a}, -C(O)OR^{a}, -C(O)R^{a}, -S(O)ₓR^{c}, and =CR^{a}₂, when present, are - NH₂, -CH₂NH₂, -OH, -CH₂OH, -C(O)O-C₁-₆alkyl, -C(O)-C₁-₆alkyl, -SR^{c}, and =CH₂, respectively.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein Y is O.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein Y is CH₂.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein R⁴, R⁵ and R⁶ are each, independently, a hydroxyl protecting group, or, if R⁵ and R⁶ are in a *cis* orientation they may optionally be taken together to form a diol protecting group , or if R⁴ and R⁵ are in a *cis* orientation they may optionally taken together to form a diol protecting group.

In an embodiment, the present invention relates to those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), and salts, and solvates thereof, or any subgroup thereof as mentioned in any of the other embodiments herein, wherein a hydroxyl protecting group is selected from a silyl group, C₁₋₄alkyl, C₁₋₄alkyl-O-C₁₋₄alkyl, tetrahydropyranyl, allyl, benzyl, -CH₂-naphthyl, benzoyl, -C(=O)-C₁₋₄alkyl, or -C(=O)-phenyl; wherein benzyl, -CH₂-naphthyl, and benzoyl, are optionally substituted with one or two substituents each independently selected from -CH₃ and -OCH₃; and/or a diol protecting group is -C(C₁₋₄alkyl)₂-.

In an embodiment, the present invention relates to those compounds of any of Formula (I), (A), (A-a), (A-b), (A-1), (A-1a), (A-1b), (A-2), (A-2a) or (A-2b), and salts, and solvates thereof, for example a compound of any of Formula (A-1), (A-1a), (A-1b), (A-2), (A-2a) or (A-2b), or a salt or solvate thereof, or any subgroup thereof as mentioned in any of the other embodiments herein, wherein R⁴ is hydrogen or a hydroxyl protecting group, such as a silyl group, C₁₋₄alkyl, C₁₋₄alkyl-O-C₁₋₄alkyl, tetrahydropyranyl, allyl, benzyl, -C(=O)-C₁₋₄alkyl, or - C(=O)-phenyl, wherein benzyl is optionally substituted with one or two substituents each independently selected from -CH₃ and -OCH₃; and R⁵ and R⁶ are a hydroxyl protecting group such as -C(=O)-C₁₋₄alkyl, benzoyl, benzyl, or -CH₂-naphthyl, wherein benzoyl, benzyl, and - CH₂-naphthyl are optionally substituted with one or two substituents each independently selected from -CH₃ and -OCH₃; or R⁵ and R⁶ taken together are a diol protecting group such as -C(C₁₋₄alkyl)₂-.

In an embodiment, the present invention relates to those compounds of any of Formula (I), (A), (A-a), (A-b), (A-1), (A-1a), (A-1b), (A-2), (A-2a) or (A-2b), and salts, and solvates thereof, for example a compound of any of Formula (A-1), (A-1a), (A-1b), (A-2), (A-2a) or (A-2b), or a salt or solvate thereof, or any subgroup thereof as mentioned in any of the other embodiments herein, wherein R⁴ is a silyl group or C₁₋₄alkyl (preferably Me); and R⁵ and R⁶ are -C(=O)-C₁₋₄alkyl (preferably pivaloyl); or R⁵ and R⁶ taken together are a diol protecting group -C(C₁₋₄alkyl)₂- (preferably -C(CH₃)₂-).

In an embodiment, the present invention relates to those compounds of any of Formula (I), (A), (A-a), (A-b), (A-2), (A-2a), (A-2b), (A-3), (A-3a), (A-3b), (A-5), (A-5a) or (A-5b), and salts, and solvates thereof, for example a compound of any of Formula (A-2), (A-2a), (A-2b), (A-3), (A-3a), (A-3b), (A-5), (A-5a) or (A-5b), or a salt or solvate thereof, or any subgroup thereof as mentioned in any of the other embodiments herein, wherein R⁴ and R⁵ are a hydroxyl protecting group such as a silyl group, C₁₋₄alkyl, -C(=O)-C₁₋₄alkyl, benzoyl, benzyl, or -CH₂-naphthyl, wherein benzoyl, benzyl, and -CH₂-naphthyl are optionally substituted with one or two substituents each independently selected from -CH₃ and -OCH₃; or R⁴ and R⁵ taken together are a diol protecting group such as -C(C₁₋₄alkyl)₂-; and R⁶ is hydrogen or a hydroxyl protecting group such as a silyl group, C₁₋₄alkyl, C₁₋₄alkyl-O-C₁₋₄alkyl, tetrahydropyranyl, allyl, benzyl, -CH₂-naphthyl, -C(=O)-C₁₋₄alkyl, or -C(=O)-phenyl, wherein benzyl is optionally substituted with one or two substituents each independently selected from -CH₃ and -OCH₃.

In an embodiment, the present invention relates to those compounds of any of Formula (I), (A), (A-a), (A-b), (A-2), (A-2a), (A-2b), (A-3), (A-3a), (A-3b), (A-5), (A-5a) or (A-5b), and salts, and solvates thereof, for example a compound of any of Formula (A-3), (A-3a) or (A-3b), or a salt or solvate thereof, or any subgroup thereof as mentioned in any of the other embodiments herein, wherein R⁴ and R⁵ are a hydroxyl protecting group such as -C(=O)-C₁₋₄alkyl, benzoyl, benzyl, or -CH₂-naphthyl, wherein benzoyl, benzyl, and -CH₂-naphthyl are optionally substituted with one or two substituents each independently selected from -CH₃ and -OCH₃; or R⁴ and R⁵ taken together are a diol protecting group such as -C(C₁₋₄alkyl)₂-; and R⁶ is hydrogen or a hydroxyl protecting group such as a silyl group, C₁₋₄alkyl, C₁₋₄alkyl-O-C₁₋₄alkyl, tetrahydropyranyl, allyl, benzyl, -CH₂-naphthyl, -C(=O)-C₁₋₄alkyl, or -C(=O)-phenyl, wherein benzyl is optionally substituted with one or two substituents each independently selected from -CH₃ and -OCH₃.

In an embodiment, the present invention relates to those compounds of any of Formula (I), (A), (A-a), (A-b), (A-3), (A-3a), (A-3b), and salts, and solvates thereof, for example a compound of any of Formula (A-3), (A-3a) or (A-3b), or a salt or solvate thereof, or any subgroup thereof as mentioned in any of the other embodiments herein, wherein R⁴ and R⁵ taken together are a diol protecting group -C(C₁₋₄alkyl)₂- (preferably -C(CH₃)₂-); and R⁶ is a silyl group, benzyl, -CH₂-naphthyl, or -C(=O)-C₁₋₄alkyl, wherein benzyl is optionally substituted with one or two substituents each independently selected from -CH₃ and -OCH₃.

In an embodiment, the present invention relates to those compounds of any of Formula (I), (A), (A-a), (A-b), (A-4), (A-4a), (A-4b), (A-6), (A-6a) or (A-6b), and salts, and solvates thereof, for example a compound of any of Formula (A-4), (A-4a), (A-4b), (A-6), (A-6a) or (A-6b), or a salt or solvate thereof, or any subgroup thereof as mentioned in any of the other embodiments herein, wherein R⁴, R⁵ and R⁶ are each, independently, hydrogen or a hydroxyl protecting group selected from a silyl group, C₁₋₄alkyl, C₁₋₄alkyl-O-C₁₋₄alkyl, tetrahydropyranyl, allyl, benzyl, -CH₂-naphthyl, benzoyl, -C(=O)-C₁₋₄alkyl, or -C(=O)-phenyl; wherein benzyl, -CH₂-naphthyl, and benzoyl, are optionally substituted with one or two substituents each independently selected from -CH₃ and -OCH₃.

In an embodiment, the present invention relates to those compounds of any of Formula (I), (A), (A-a), (A-b), (A-1), (A-1a) or (A-1b), and salts, and solvates thereof, for example a compound of any of Formula (A-1), (A-1a) or (A-1b), or a salt or solvate thereof, or any subgroup thereof as mentioned in any of the other embodiments herein, wherein R⁴ is C₁₋₄ alkyl, such as methyl, and R⁵ and R⁶ taken together are -C(C₁₋₄alkyl)₂-, such as -C(CH₃)₂-.

In an embodiment, the present invention relates to those compounds of any of Formula (I), (A), (A-a), (A-b), (A-3), (A-3a) or (A-3b), and salts, and solvates thereof, for example a compound of any of Formula (A-3), (A-3a) or (A-3b), or a salt or solvate thereof, or any subgroup thereof as mentioned in any of the other embodiments herein, wherein R⁶ is a silyl group, benzyl, -CH₂-naphthyl, benzoyl or -C(=O)-C₁₋₄alkyl, wherein benzyl, -CH₂-naphthyl, and benzoyl, are optionally substituted with one or two substituents each independently selected from -CH₃ and -OCH₃, and R⁵ and R⁶ taken together are -C(C₁₋₄alkyl)₂-, such as - C(CH₃)₂-.

In an embodiment, the present invention relates to a compound selected from: 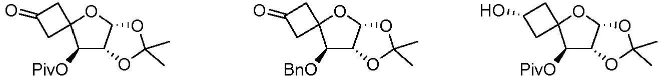 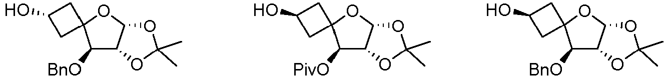 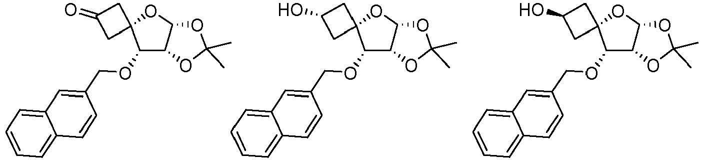 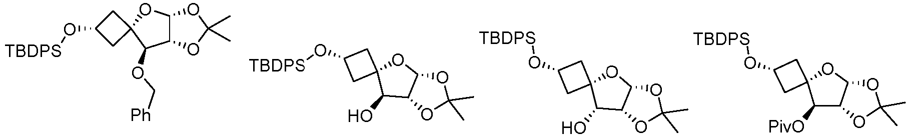 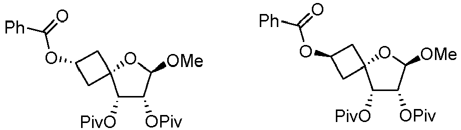 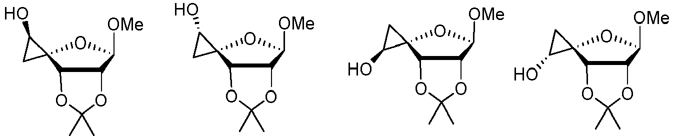 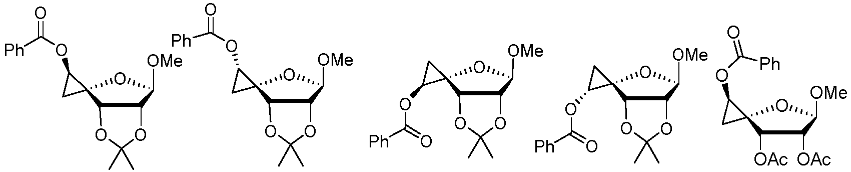 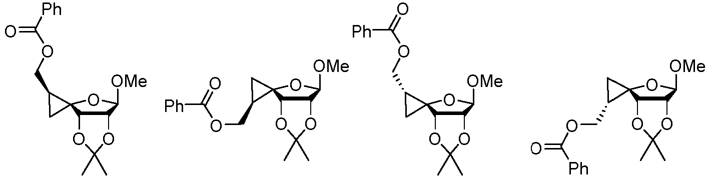 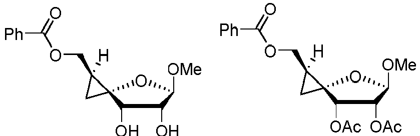 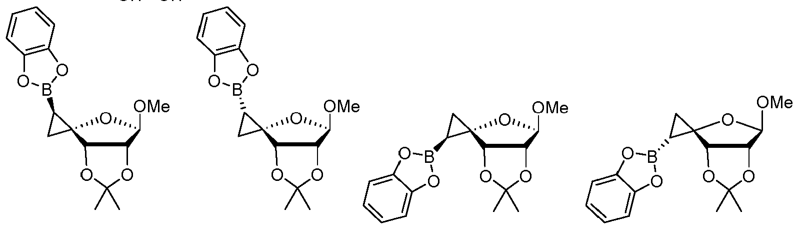 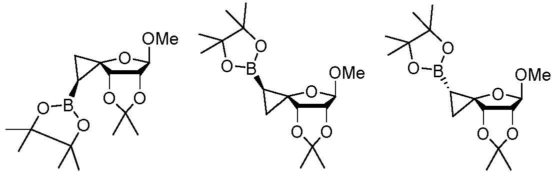 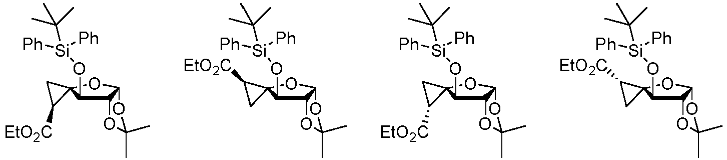 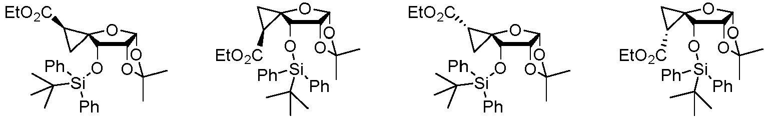 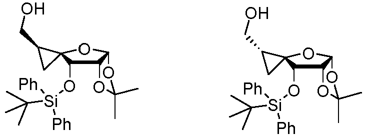 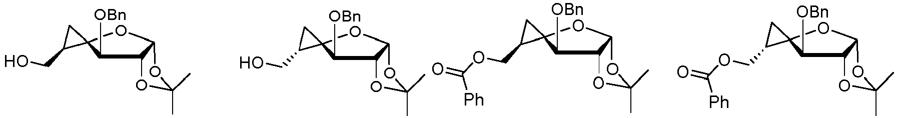 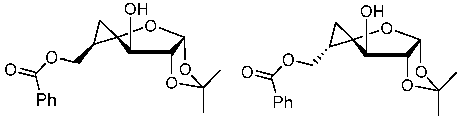 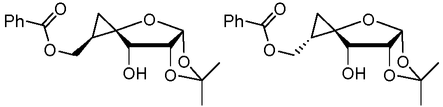 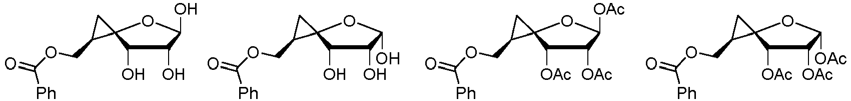 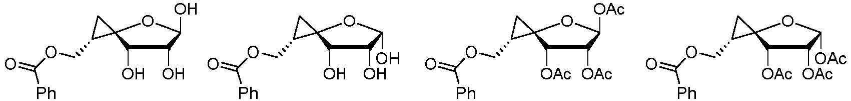 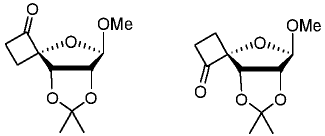 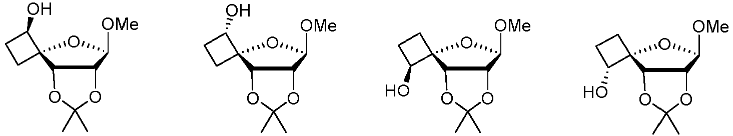 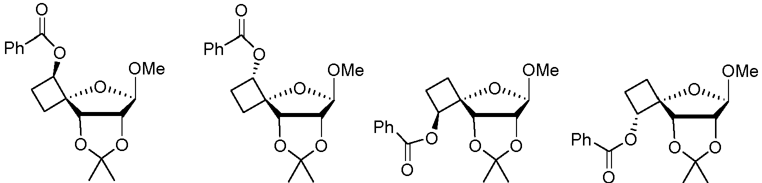 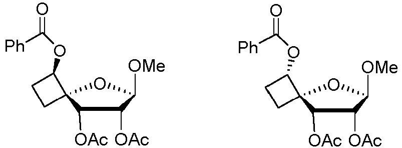 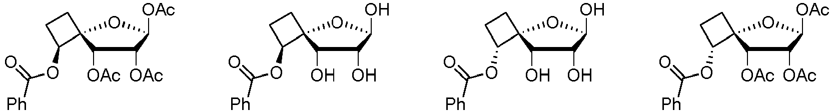 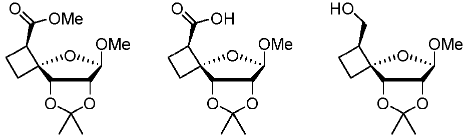 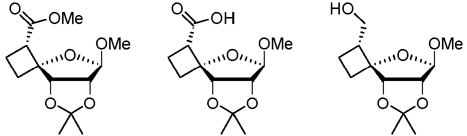 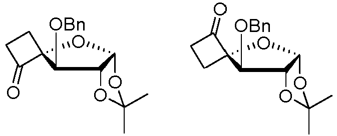 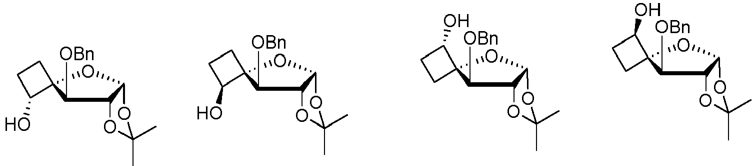 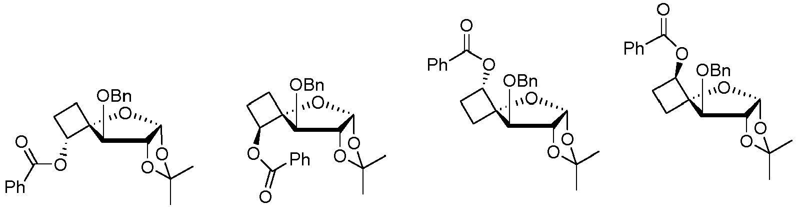 or a salt or solvate thereof.

In another aspect, the invention relates to a compound of Formula (B): 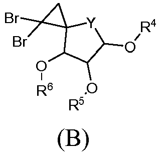 or a salt or solvate thereof, wherein:
Y is -O- or -CH₂-;
R⁴, R⁵ and R⁶ are each, independently, hydrogen or a hydroxyl protecting group;
or R⁵ and R⁶ taken together are a diol protecting group;
or R⁴ and R⁵ taken together are a diol protecting group.

In an embodiment, the present invention relates to those compounds of Formula (B) and salts, and solvates thereof, wherein Y, R⁴, R⁵ and R⁶ are as defined for those compounds of any of Formula (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), or any subgroup thereof as mentioned in any of the other embodiments herein.

In an embodiment, the present invention relates to those compounds of Formula (B) and salts, and solvates thereof, wherein the compound has a stereochemistry as depicted for those compounds of any of Formula (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b).

In an embodiment the present invention relates to a compound selected from: 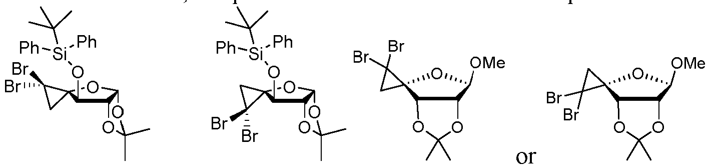 or a salt or solvate thereof.

In another aspect, the invention relates to a compound of Formula (C): 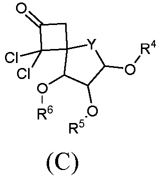 wherein:
Y is -O- or -CH₂-;
R⁴, R⁵ and R⁶ are each, independently, hydrogen or a hydroxyl protecting group; or
R⁴ and R⁵ taken together are a diol protecting group; or
R⁵ and R⁶ taken together are a diol protecting group;
provided that the compound is not:

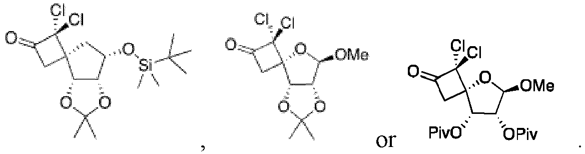

In an embodiment, the present invention relates to those compounds of Formula (B) and salts, and solvates thereof, wherein Y, R⁴, R⁵ and R⁶ are as defined for those compounds of any of Formula (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b), or (A-6b), or any subgroup thereof as mentioned in any of the other embodiments herein.

In an embodiment, the present invention relates to those compounds of Formula (B) and salts, and solvates thereof, wherein the compound has a stereochemistry as depicted for those compounds of any of Formula (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b).

In an embodiment, the present invention relates to those compounds of Formula (C) and salts, and solvates thereof, or any subgroup thereof as mentioned in any of the other embodiments herein, wherein the compound is a compound of formula: 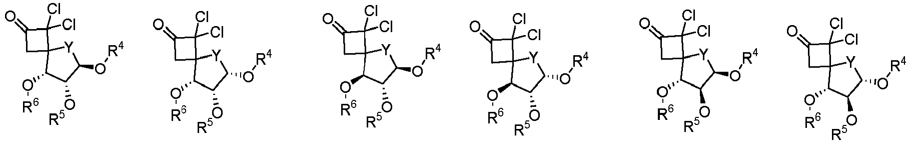 or a salt or solvate thereof.

In an embodiment, the present invention relates to those compounds of Formula (C) and salts, and solvates thereof, wherein the compound is selected from: 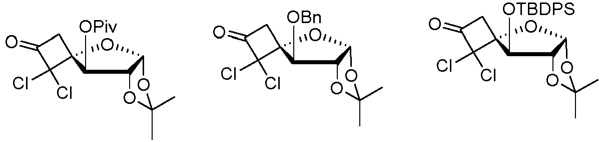 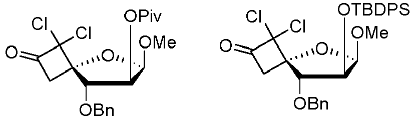 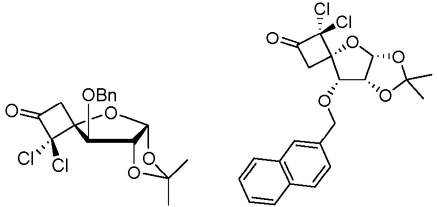 or a salt or solvate thereof.

In another aspect, the invention relates to a compound of Formula (D): 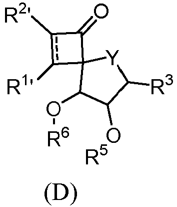 or a salt or solvate thereof, wherein:
Y is -O- or -CH₂-;
wherein - - is a single bond or a double bond; and
when - - is a single bond, one of R¹' and R²' is hydrogen or C₁-₆ alkyl and the other is a silyl group, C₁-₆ alkyl optionally substituted with a silyl group, -O-C₁-₆ alkyl, -B(OR^{b})₂ -S-C₁-₆ alkyl, -S-aryl, -O-aryl or C(O)R^{a};
when - - is a double bond, one of R¹' and R²' is hydrogen or C₁-₆ alkyl and the other is a silyl group, C₁-₆ alkyl optionally substituted with a silyl group, -O-C₁-₆ alkyl, or C(O)R^{a}; and
R^{a} is hydrogen or C₁-₆ alkyl;
each R^{b} is hydrogen or both R^{b} are taken together to form a boronic acid protecting group; R³ is OR⁴ or R¹⁰;
R¹⁰ is a nucleobase or nucleobase derivative;
R⁴, R⁵ and R⁶ are each, independently, hydrogen or a hydroxyl protecting group; or R⁴ and R⁵ taken together are a diol protecting group; or
R⁵ and R⁶ taken together are a diol protecting group.

In an embodiment, the invention relates to those compounds of Formula (D) and salts, and solvates thereof, or any subgroup thereof as mentioned in any of the other embodiments herein, wherein:
Y is -O- or -CH₂-;
wherein - - is a single bond or a double bond; and
when - - is a single bond, one of R¹' and R²' is hydrogen or C₁-₆ alkyl and the other is a silyl group, C₁-₆ alkyl substituted with a silyl group, -O-C₁-₆ alkyl, -B(OR^{b})₂ -S-C₁-₆ alkyl, - S-aryl, -O-aryl or C(O)R^{a};
when - - is a double bond, one of R¹' and R²' is hydrogen or C₁-₆ alkyl and the other is a silyl group, C₁-₆ alkyl substituted with a silyl group, -O-C₁-₆ alkyl, or C(O)R^{a};
   and
R^{a} is hydrogen or C₁-₆ alkyl;
each R^{b} is hydrogen or both R^{b} are taken together to form a boronic acid protecting group;
R³ is OR⁴ or R¹⁰;
R¹⁰ is a nucleobase or nucleobase derivative;
R⁴, R⁵ and R⁶ are each, independently, hydrogen or a hydroxyl protecting group; or
R⁴ and R⁵ taken together are a diol protecting group; or
R⁵ and R⁶ taken together are a diol protecting group.

In an embodiment, the present invention relates to those compounds of Formula (D) and salts, and solvates thereof, or any subgroup thereof as mentioned in any of the other embodiments herein, wherein R³ is OR⁴.

In an embodiment, the present invention relates to those compounds of Formula (D) and salts, and solvates thereof, or any subgroup thereof as mentioned in any of the other embodiments herein, wherein a silyl group, when present, is trimethylsilyl, triethylsilyl, *tert-*butyldiphenylsilyl, triisopropylsilyl or dimethylphenylsilyl.

In an embodiment, the present invention relates to those compounds of Formula (D) and salts, and solvates thereof, or any subgroup thereof as mentioned in any of the other embodiments herein, wherein - - is a single bond.

In an embodiment, the present invention relates to those compounds of Formula (D) and salts, and solvates thereof, or any subgroup thereof as mentioned in any of the other embodiments herein, wherein - - is a double bond.

In an embodiment, the present invention relates to those compounds of Formula (D) and salts, and solvates thereof, or any subgroup thereof as mentioned in any of the other embodiments herein, wherein - - is a single bond, one of R¹' and R²' is hydrogen and the other is a silyl group, C₁alkyl substituted with a silyl group, -O-C₁-₆ alkyl or -S-aryl.

In an embodiment, the present invention relates to those compounds of Formula (D) and salts, and solvates thereof, or any subgroup thereof as mentioned in any of the other embodiments herein, wherein - - is a double bond, one of R¹' and R²' is hydrogen or methyl, and the other is a silyl group, C₁alkyl substituted with a silyl group, -O-C₁-₆ alkyl or -S-aryl.

In an embodiment, the present invention relates to those compounds of Formula (D) and salts, and solvates thereof, or any subgroup thereof as mentioned in any of the other embodiments herein, wherein one of R¹' and R²' is C₁-₆ alkyl and the other of R¹' and R²' is not C₁-₆ alkyl or hydrogen.

In an embodiment, the present invention relates to those compounds of Formula (D) and salts, and solvates thereof, or any subgroup thereof as mentioned in any of the other embodiments herein, wherein one of R¹' and R²' is hydrogen and the other of R¹' and R²' is not C₁-₆ alkyl or hydrogen.

In an embodiment, the present invention relates to those compounds of Formula (D) and salts, and solvates thereof, wherein Y, R⁴, R⁵ and R⁶ are as defined for those compounds of any of Formula (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), or any subgroup thereof as mentioned in any of the other embodiments herein.

In an embodiment, the present invention relates to those compounds of Formula (D) and salts, and solvates thereof, wherein the compound has a stereochemistry as depicted for those compounds of any of Formula (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b).

In an embodiment, the present invention relates to those compounds of Formula (D) and salts, and solvates thereof, wherein the compound is selected from: 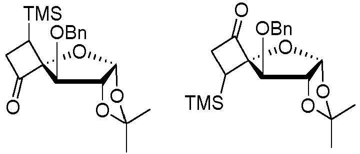 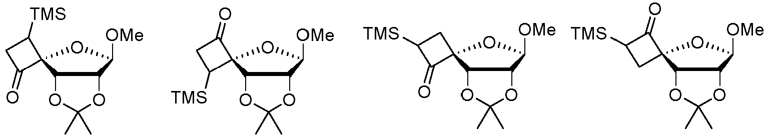 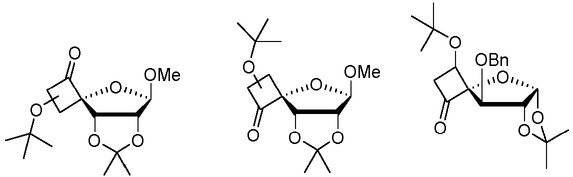 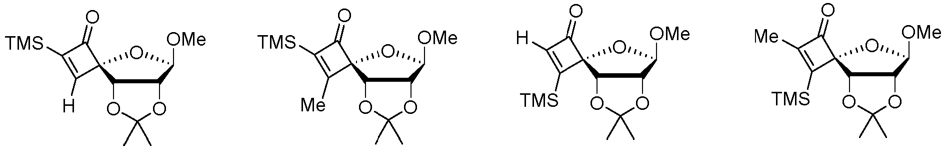 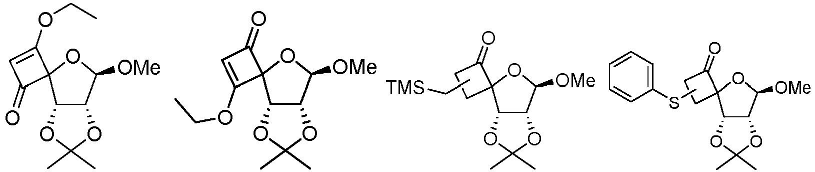 or a salt or solvate thereof.

In another aspect, the invention relates to a compound of Formula (E): 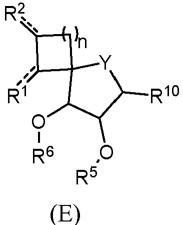 or a salt or solvate thereof, wherein:
Y is -O- or -CH₂-;
n is 0 or 1;
when n is 1, R¹ is hydrogen and R² is -(CH₂)ₘNR^{a}₂, -(CH₂)ₘOR^{a}, -(CH₂)ₘC(O)OR^{a}, - (CH₂)ₘC(O)NR^{a}₂, -(CH₂)ₘC(O)R^{a}, -(CH₂)ₘB(OR^{b})₂, -(CH₂)ₘS(O)ₓR^{c}, a silyl group, C₁-₆ alkyl substituted with a silyl group, =O or =CR^{a}₂; or
when n is 1, R² is hydrogen and R¹ is -(CH₂)ₘNR^{a}₂, -(CH₂)ₘOR^{a}, -(CH₂)ₘOPG, - (CH₂)ₘC(O)OR^{a}, -(CH₂)ₘC(O)NR^{a}₂, -(CH₂)ₘC(O)R^{a}, -(CH₂)ₘB(OR^{b})₂, -(CH₂)ₘS(O)ₓR^{c}, a silyl group, C₁-₆ alkyl substituted with a silyl group, -O-aryl, =O or =CR^{a}₂;
when n is 0, one of R¹ and R² is hydrogen and the other is -(CH₂)ₘNR^{a}₂, -(CH₂)ₘOR^{a}, - (CH₂)ₘOPG, -(CH₂)ₘC(O)OR^{a}, -(CH₂)ₘC(O)NR^{a}₂, -(CH₂)ₘC(O)R^{a}, -(CH₂)ₘB(OR^{b})₂, - (CH₂)ₘS(O)ₓR^{c} or =CH₂;
wherein the bond towards R¹ or R² - - is a single bond when R¹ or R² is hydrogen, - (CH₂)ₘNR^{a}₂, -(CH₂)ₘOR^{a}, -(CH₂)ₘOPG, -(CH₂)ₘC(O)OR^{a}, -(CH₂)ₘC(O)NR^{a}₂, - (CH₂)ₘC(O)R^{a}, -(CH₂)ₘB(OR^{b})₂, -(CH₂)ₘS(O)ₓR^{c}, a silyl group, C₁-₆ alkyl substituted with a silyl group or -O-aryl, or a double bond when R¹ or R² is =O, =CH₂ or =CR^{a}₂ each R^{a} is, independently, hydrogen or C₁-₆ alkyl;
both R^{b} are hydrogen or both R^{b} are taken together to form a boronic acid protecting group;
R^{c} is C₁-₆ alkyl or aryl;
x is 0, 1 or 2;
m is 0 or 1;
R⁵ and R⁶ are each, independently, hydrogen or a hydroxyl protecting group; or R⁵ and R⁶ taken together are a diol protecting group;
R¹⁰ is a nucleobase or nucleobase derivative;
PG is a hydroxyl protecting group;
provided that the compound is not any of: 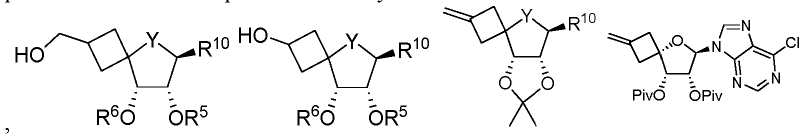 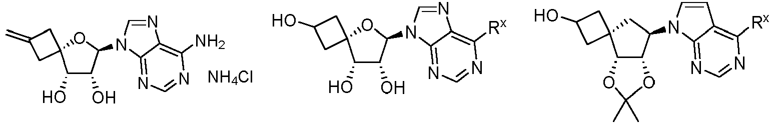
wherein
R⁵ is hydrogen or -C(=O)-C₁₋₄alkyl;
R⁶ is hydrogen or -C(=O)-C₁₋₄alkyl; or R⁵ and R⁶ taken together are -C(CH₃)₂-Y is -O- or -CH₂-;
R¹⁰ is 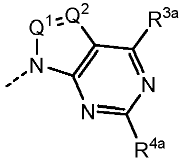
Q¹ is CR^{6a};
Q² is N or CR^{6b};
R^{6a} and R^{6b} each independently represent hydrogen, halogen, C₁₋₄alkyl, -NR^{9a}R^{9b}, or C₁₋₄alkyl substituted with one, two or three halo atoms;
R^{9a} and R^{9b} each independently represent hydrogen or C₁₋₄alkyl;
R^{3a} is hydrogen, halo, -NR^{7a}R^{7b}, C₁₋₄alkyl, C₂₋₄alkenyl, C₃₋₆cycloalkyl, -OH, or -O-C₁₋₄alkyl;
R^{7a} is hydrogen;
R^{7b} is hydrogen, C₃₋₆cycloalkyl, or C₁₋₄alkyl;
R^{4a} is hydrogen, halo, -NR^{8a}R^{8b}, or C₁₋₄alkyl; and
R^{x} is

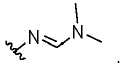

In an embodiment, the present invention relates to those compounds of Formula (E) and salts, and solvates thereof, or any subgroup thereof as mentioned in any of the other embodiments herein, wherein:
Y is -O- or -CH₂-;
n is 0 or 1;
when n is 1, R¹ is hydrogen and R² is -(CH₂)ₘNR^{a}₂, -(CH₂)ₘOR^{a}, -(CH₂)ₘC(O)OR^{a}, - (CH₂)ₘC(O)NR^{a}₂, -(CH₂)ₘC(O)R^{a}, -(CH₂)ₘB(OR^{b})₂, -(CH₂)ₘS(O)ₓR^{c}, a silyl group, C₁-₆ alkyl substituted with a silyl group, =O or =CR^{a}₂; or
when n is 1, R² is hydrogen and R¹ is -(CH₂)ₘNR^{a}₂, -(CH₂)ₘOR^{a}, -(CH₂)ₘOPG, - (CH₂)ₘC(O)OR^{a}, -(CH₂)ₘC(O)NR^{a}₂, -(CH₂)ₘC(O)R^{a}, -(CH₂)ₘB(OR^{b})₂, -(CH₂)ₘS(O)ₓR^{c}, a silyl group, C₁-₆ alkyl substituted with a silyl group, -O-aryl, =O or =CR^{a}₂;
when n is 0, one of R¹ and R² is hydrogen and the other is -(CH₂)ₘNR^{a}₂, -(CH₂)ₘOR^{a}, - (CH₂)ₘOPG, -(CH₂)ₘC(O)OR^{a}, -(CH₂)ₘC(O)NR^{a}₂, -(CH₂)ₘC(O)R^{a}, -(CH₂)ₘB(OR^{b})₂, - (CH₂)ₘS(O)ₓR^{c} or =CH₂;
wherein the bond towards R¹ or R² - - is a single bond when R¹ or R² is hydrogen, - (CH₂)ₘNR^{a}₂, -(CH₂)ₘOR^{a}, -(CH₂)ₘOPG, -(CH₂)ₘC(O)OR^{a}, -(CH₂)ₘC(O)NR^{a}₂, - (CH₂)ₘC(O)R^{a}, -(CH₂)ₘB(OR^{b})₂, -(CH₂)ₘS(O)ₓR^{c}, a silyl group, C₁-₆ alkyl substituted with a silyl group or -O-aryl, or a double bond when R¹ or R² is =O, =CH₂ or =CR^{a}₂ each R^{a} is, independently, hydrogen or C₁-₆ alkyl;
both R^{b} are taken together to form a boronic acid protecting group;
R^{c} is C₁-₆ alkyl or aryl;
x is 0, 1 or 2;
m is 0 or 1;
R⁵ and R⁶ are each, independently, hydrogen or a hydroxyl protecting group; or R⁵ and R⁶ taken together are a diol protecting group;
R¹⁰ is a nucleobase or nucleobase derivative;
PG is a hydroxyl protecting group;
provided that the compound is not any of: 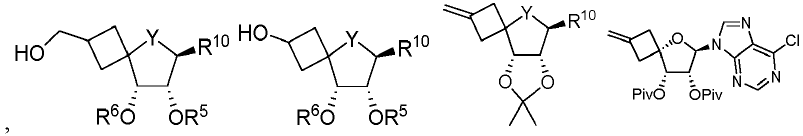 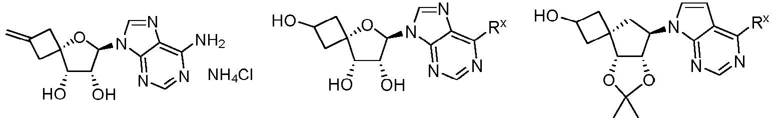
wherein
R⁵ is hydrogen or -C(=O)-C₁₋₄alkyl;
R⁶ is hydrogen or -C(=O)-C₁₋₄alkyl; or R⁵ and R⁶ taken together are -C(CH₃)₂-Y is -O- or -CH₂-;
R¹⁰ is 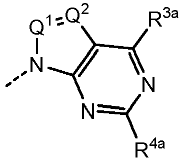
Q¹ is CR^{6a};
Q² is N or CR^{6b};
R^{6a} and R^{6b} each independently represent hydrogen, halogen, C₁₋₄alkyl, -NR^{9a}R^{9b}, or C₁₋₄alkyl substituted with one, two or three halo atoms;
R^{9a} and R^{9b} each independently represent hydrogen or C₁₋₄alkyl;
R^{3a} is hydrogen, halo, -NR^{7a}R^{7b}, C₁₋₄alkyl, C₂₋₄alkenyl, C₃₋₆cycloalkyl, -OH, or -O-C₁₋₄alkyl;
R^{7a} is hydrogen;
R^{7b} is hydrogen, C₃₋₆cycloalkyl, or C₁₋₄alkyl;
R^{4a} is hydrogen, halo, -NR^{8a}R^{8b}, or C₁₋₄alkyl; and
R^{x} is

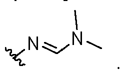

In an embodiment, the present invention relates to those compounds of Formula (E) and salts, and solvates thereof, or any subgroup thereof as mentioned in any of the other embodiments herein, wherein n is 1.

In an embodiment, the present invention relates to those compounds of Formula (E) and salts, and solvates thereof, or any subgroup thereof as mentioned in any of the other embodiments herein, wherein n is 0.

In an embodiment, the present invention relates to those compounds of Formula (E) and salts, and solvates thereof, or any subgroup thereof as mentioned in any of the other embodiments herein, wherein Y is O.
and salts, and solvates thereof, or any subgroup thereof as mentioned in any of the other embodiments herein, wherein Y is CH₂.

In an embodiment, the present invention relates to those compounds of Formula (E) and salts, and solvates thereof, wherein R¹ and R² are as defined for those compounds of any of Formula (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), or any subgroup thereof as mentioned in any of the other embodiments herein.

In an embodiment, the present invention relates to those compounds of Formula (E) and salts, and solvates thereof, wherein R⁴, R⁵ and R⁶ are as defined for those compounds of any of Formula (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), or any subgroup thereof as mentioned in any of the other embodiments herein.

In an embodiment, the present invention relates to those compounds of Formula (E) and salts, and solvates thereof, wherein the compound has a stereochemistry as depicted for those compounds of any of Formula (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), for example as depicted in any of the structures below;

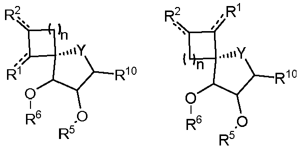

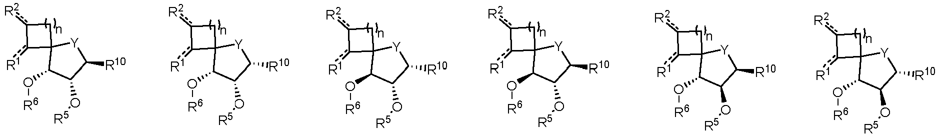

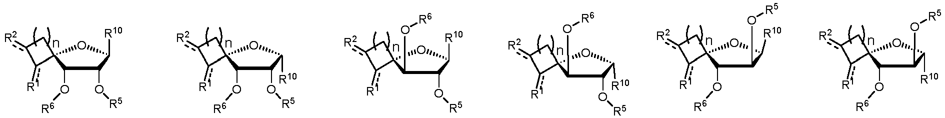

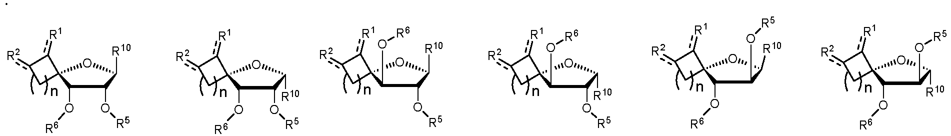

In an embodiment, the present invention relates to those compounds of Formula (E) and salts, and solvates thereof, or any subgroup thereof as mentioned in any of the other embodiments herein, wherein -NR^{a}₂, -CH₂NR^{a}₂, -OR^{a}, -CH₂OR^{a}, -C(O)OR^{a}, -C(O)R^{a}, -S(O)ₓR^{c}, =CR^{a}₂, when present, are -NH₂, -CH₂NH₂, -OH, -CH₂OH, -C(O)O-C₁-₆alkyl, -C(O)-C₁-₆alkyl, -SR^{c}, =CH₂, respectively.

It will be appreciated that all definitions of Y, n, R¹, R², R⁵ and R⁶ as defined for those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), or any subgroup thereof as mentioned in any of the other embodiments herein, may apply to compounds of Formula (E) and salts, and solvates thereof. Accordingly, in an embodiment, the present invention relates to those compounds of Formula (E) and salts, and solvates thereof, wherein any one or more of Y, n, R¹, R², R⁵ and R⁶ as defined for those compounds of any of Formula (I), (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), or any subgroup thereof as mentioned in any of the other embodiments herein.

In an embodiment, the present invention relates to those compounds of Formula (E) and salts, and solvates thereof, or any subgroup thereof as mentioned in any of the other embodiments herein, wherein R⁵ and R⁶ are each, independently, hydrogen or -C(=O)-C₁₋₄alkyl, or R⁵ and R⁶ taken together are -C(C₁₋₄alkyl)₂-, such as -C(CH₃)₂-.

In an embodiment, the present invention relates to those compounds of Formula (E) and salts, and solvates thereof, wherein the compound is selected from: 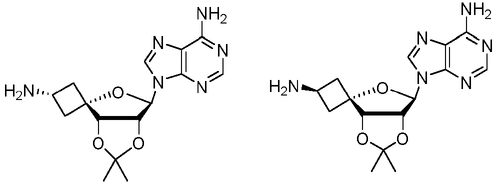 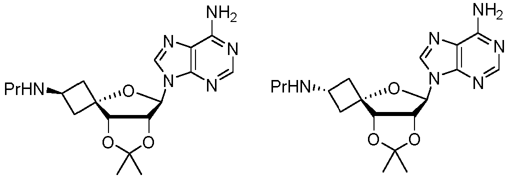 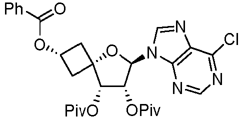 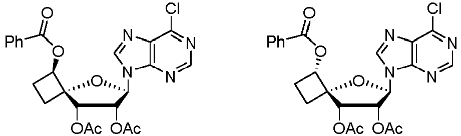 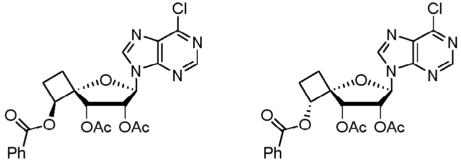 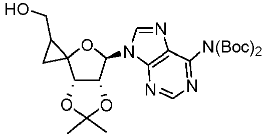 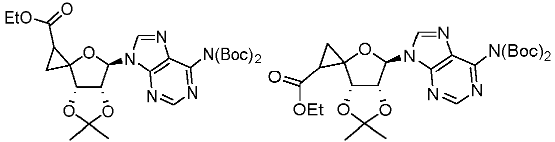 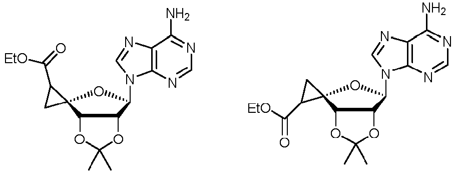 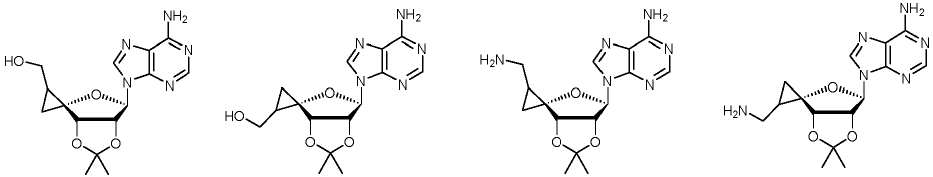 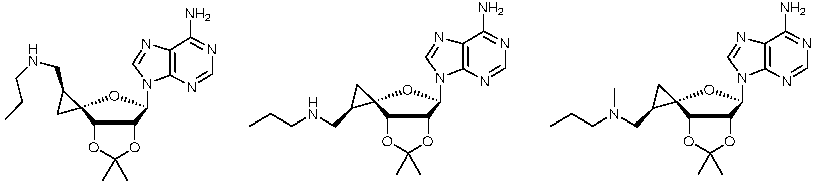 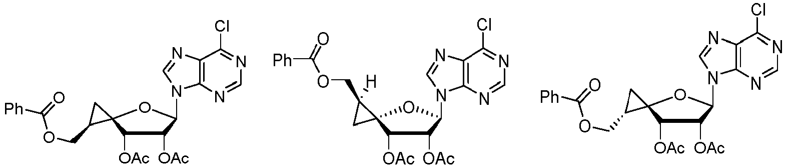 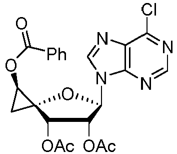 or a salt or solvate thereof.

In an embodiment, the present invention relates to those compounds of Formula (E) and salts, and solvates thereof, wherein the compound is selected from: 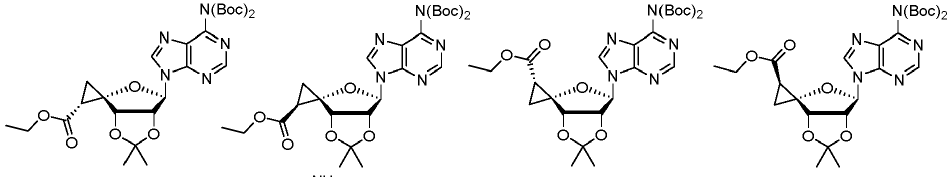 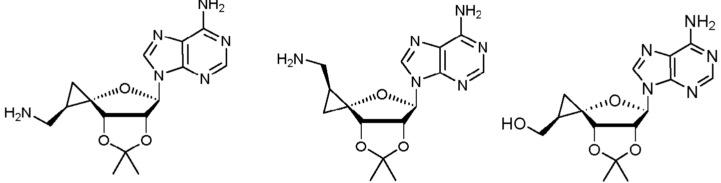 or a salt or solvate thereof.

In an embodiment, the present invention relates to those compounds of Formula (E) and salts, and solvates thereof, wherein the compound is selected from: 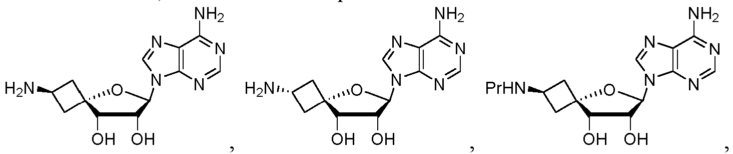 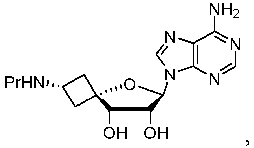 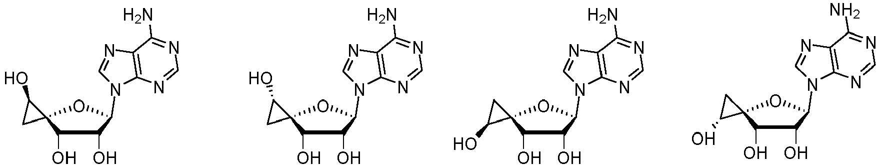 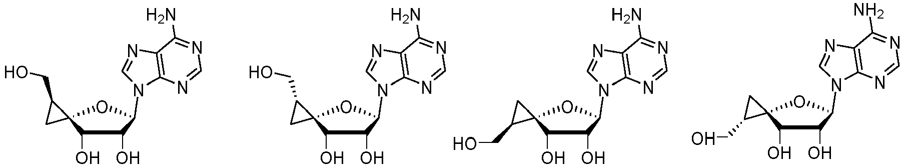 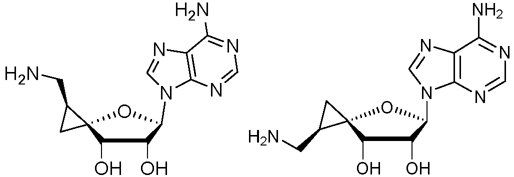 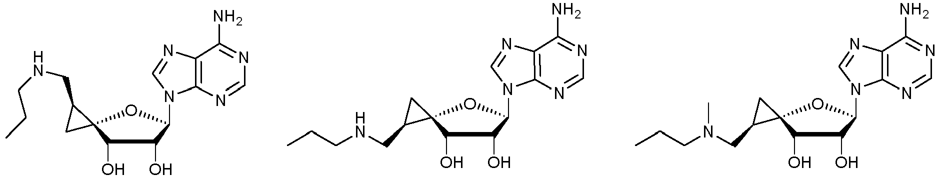 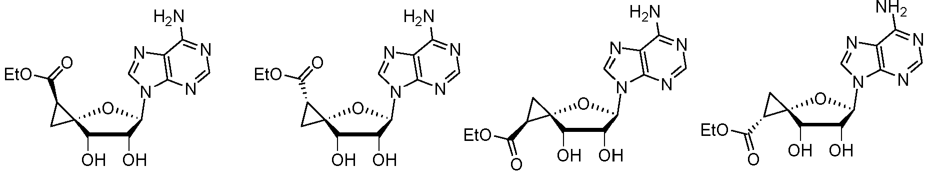 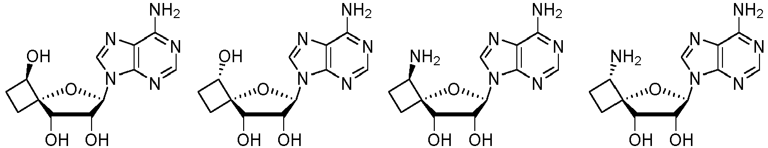 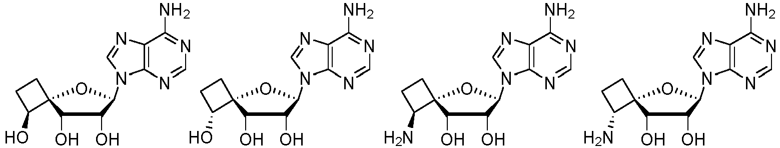 or a salt or solvate thereof.

In another aspect, the invention relates to a compound of Formula (F): 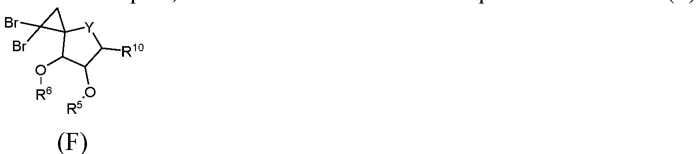 or a salt or solvate thereof, wherein:
R⁵ and R⁶ are each, independently, hydrogen or a hydroxyl protecting group; or R⁵ and R⁶ taken together are a diol protecting group; and
R¹⁰ is a nucleobase or nucleobase derivative.

In an embodiment, the present invention relates to those compounds of Formula (F) and salts, and solvates thereof, wherein R⁴, R⁵ and R⁶ are as defined for those compounds of any of Formula (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b), or any subgroup thereof as mentioned in any of the other embodiments herein.

In an embodiment, the present invention relates to those compounds of Formula (F) and salts, and solvates thereof, wherein the compound has a stereochemistry as depicted for those compounds of any of Formula (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b) or (A-6b).

In an embodiment, the present invention relates to any of those compounds of any of Formula (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b), (A-6b), (B), (C), (D), (E) and (F) and salts, and solvates thereof, or any subgroup thereof as mentioned in any of the other embodiments herein, wherein a silyl group, if present, is t-butyldimethylsilyl, t-butyldiphenylsilyl, triisopropylsilyl, trimethylsilyl, or triethylsilyl.

In an embodiment, the present invention relates to those compounds of any of Formula (A), (A-a), (A-b), (A-1), (A-2), (A-3), (A-4), (A-5), (A-6), (A-1a), (A-2a), (A-3a), (A-4a), (A-5a), (A-6a), (A-1b), (A-2b), (A-3b), (A-4b), (A-5b), (A-6b), (B), (C), (D), (E) and (F) and salts, and solvates thereof, or any subgroup thereof as mentioned in any of the other embodiments herein, wherein -B(OR^{b})₂, if present, is catechol borane, pinacol borane, N-methyliminodiacetic acid (MIDA) boronate, neopentylglycol borane, pinanediol borane, biscyclohexyldiol borane, or 1-(4-methoxyphenyl)-2-methylpropane-1,2-diol (MPMP-diol) borane.

In an embodiment, the present invention relates to those compounds of any of Formula (D), (E) and (F) wherein R¹⁰, when present, is cytosine, thymine, uracil, or a modified or protected form thereof, or a bicyclic aromatic heterocyclic ring system selected from the group consisting of (a-1), (a-2), (a-3), (a-4) and (a-5): 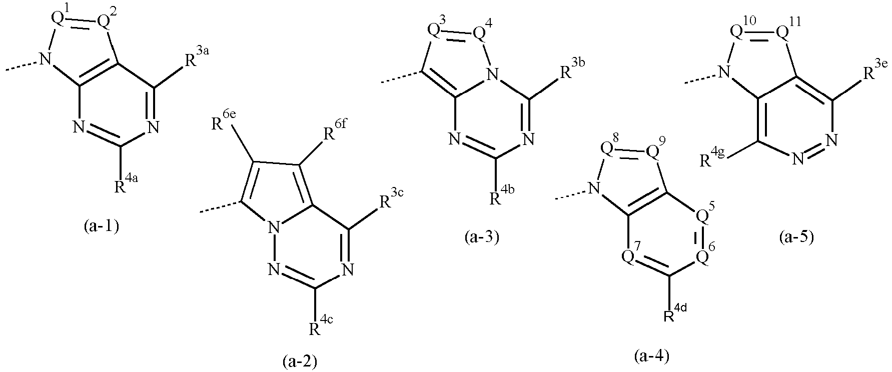
R^{3a}, R^{3b}, R^{3c}, R^{3d} and R^{3e} each independently are hydrogen, halo, -NR^{7a}R^{7b},
C₁₋₄alkyl, C₂₋₄alkenyl, C₃₋₆cycloalkyl, -OH, or -O-C₁₋₄alkyl;
R^{7a} is hydrogen, -C(O)O-C₁₋₄alkyl or 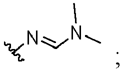
R^{7b} is hydrogen, C₃₋₆cycloalkyl, C₁₋₄alkyl, or -C(O)O-C₁₋₄alkyl;
R^{4a}, R^{4b}, R^{4c}, R^{4d}, R^{4e}, R^{4f} and R^{4g} each independently are hydrogen, halo,
-NR^{8a}R^{8b}, or C₁₋₄alkyl;
R^{8a} and R^{8b} each independently are hydrogen or C₁₋₄alkyl;
Q¹ is N or CR^{6a};
Q² is N or CR^{6b};
Q³ is N or CR^{6c};
Q⁴ is N or CR^{6d};
provided that maximum one of Q³ and Q⁴ is N;
Q⁸ is N or CR^{6g};
Q⁹ is N or CR^{6h};
Q¹⁰ is N or CR⁶ⁱ;
Q¹¹ is N or CR^{6j};
Q⁵ is CR^{3d}; Q⁶ is N; and Q⁷ is CR^{4f}; or
Q⁵ is CR^{3d}; Q⁶ is CR^{4e}; and Q⁷ is N; or
Q⁵ is N; Q⁶ is CR^{4e}; and Q⁷ is CR^{4f}; or
Q⁵ is N; Q⁶ is CR^{4e}; and Q⁷ is N; or
Q⁵ is N; Q⁶ is N; and Q⁷ is CR^{4f}; or
Q⁵ is N; Q⁶ is N; and Q⁷ is N;
R^{6a}, R^{6b}, R^{6c}, R^{6d}, R^{6e}, R^{6f}, R^{6g}, R^{6h}, R⁶ⁱ and R^{6j} each independently are hydrogen, halogen, C₁₋₄alkyl, -NR^{9a}R^{9b}, or C₁₋₄alkyl substituted with one, two or three halo atoms;
R^{9a} and R^{9b} each independently are hydrogen or C₁₋₄alkyl.

In an embodiment, the present invention relates to those compounds of any of Formula (D), (E) and (F) wherein R¹⁰, when present, is cytosine, thymine, uracil, or a modified or protected form thereof, or a bicyclic aromatic heterocyclic ring system selected from the group consisting of (a-1), (a-2), (a-3), (a-4) and (a-5): 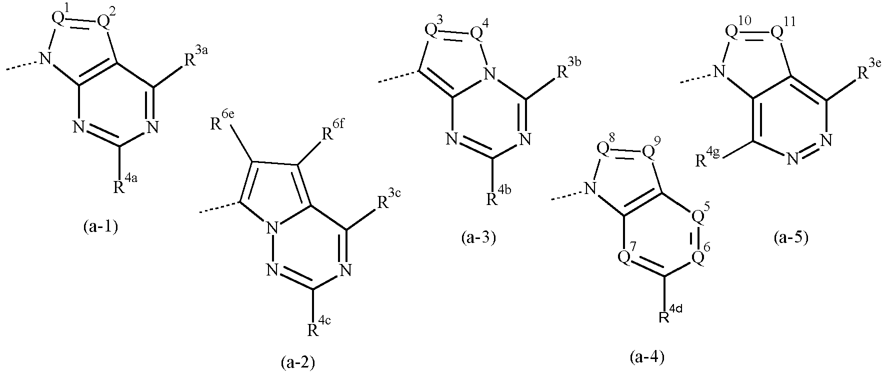
wherein R^{3a}, R^{3b}, R^{3c}, R^{3d} and R^{3e} each independently represent hydrogen, halo, -NR^{7a}R^{7b}, C₁₋₄alkyl, C₂₋₄alkenyl, C₃₋₆cycloalkyl, -OH, or -O-C₁₋₄alkyl;
R^{7a} represents hydrogen;
R^{7b} represents hydrogen, C₃₋₆cycloalkyl, or C₁₋₄alkyl;
R^{4a}, R^{4b}, R^{4c}, R^{4d}, R^{4e}, R^{4f} and R^{4g} each independently represent hydrogen, halo,
-NR^{8a}R^{8b}, or C₁₋₄alkyl;
R^{8a} and R^{8b} each independently represent hydrogen or C₁₋₄alkyl;
Q¹ represents N or CR^{6a};
Q² represents N or CR^{6b};
Q³ represents N or CR^{6c};
Q⁴ represents N or CR^{6d};
provided that maximum one of Q³ and Q⁴ represents N;
Q⁸ represents N or CR^{6g};
Q⁹ represents N or CR^{6h};
Q¹⁰ represents N or CR⁶ⁱ;
Q¹¹ represents N or CR^{6j};
Q⁵ represents CR^{3d}; Q⁶ represents N; and Q⁷ represents CR^{4f}; or
Q⁵ represents CR^{3d}; Q⁶ represents CR^{4e}; and Q⁷ represents N; or
Q⁵ represents N; Q⁶ represents CR^{4e}; and Q⁷ represents CR^{4f}; or
Q⁵ represents N; Q⁶ represents CR^{4e}; and Q⁷ represents N; or
Q⁵ represents N; Q⁶ represents N; and Q⁷ represents CR^{4f}; or
Q⁵ represents N; Q⁶ represents N; and Q⁷ represents N;
R^{6a}, R^{6b}, R^{6c}, R^{6d}, R^{6e}, R^{6f}, R^{6g}, R^{6h}, R⁶ⁱ and R^{6j} each independently represent hydrogen,
halogen, C₁₋₄alkyl, -NR^{9a}R^{9b}, or C₁₋₄alkyl substituted with one, two or three halo atoms;
R^{9a} and R^{9b} each independently represent hydrogen or C₁₋₄alkyl;

In an embodiment, the present invention relates to those compounds of any of Formula (D), (E) and (F) wherein R¹⁰, when present, is cytosine, thymine, uracil, or a modified or protected form thereof, or a bicyclic aromatic heterocyclic ring system selected from the group consisting of (a-1), (a-2), (a-3), (a-4) and (a-5): 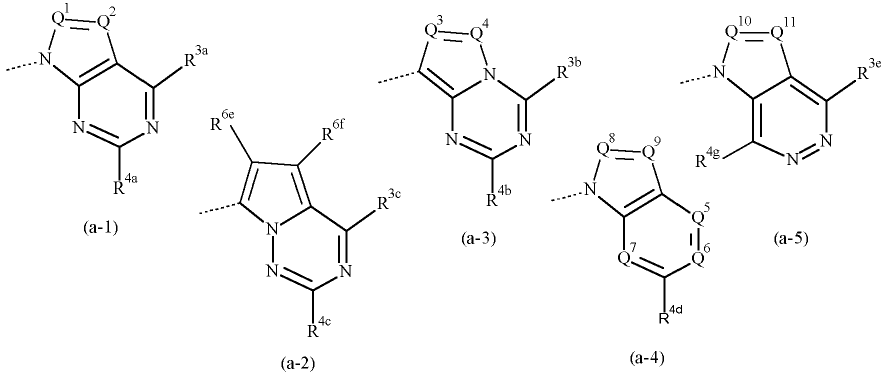
wherein R^{3a}, R^{3b}, R^{3c}, R^{3d} and R^{3e} each independently represent hydrogen, halo, -NR^{7a}R^{7b}, C₁₋₄alkyl, C₂₋₄alkenyl, C₃₋₆cycloalkyl, -OH, or -O-C₁₋₄alkyl;
R^{7a} represents hydrogen;
R^{7b} represents hydrogen, C₃₋₆cycloalkyl, or C₁₋₄alkyl;
R^{4a}, R^{4b}, R^{4c}, R^{4d}, R^{4e}, R^{4f} and R^{4g} each independently represent hydrogen, halo,
-NR^{8a}R^{8b}, or C₁₋₄alkyl;
R^{8a} and R^{8b} each independently represent hydrogen or C₁₋₄alkyl;
Q¹ represents CR^{6a};
Q² represents CR^{6b};
Q³ represents N or CR^{6c};
Q⁴ represents N or CR^{6d};
provided that maximum one of Q³ and Q⁴ represents N;
Q⁸ represents N or CR^{6g};
Q⁹ represents N or CR^{6h};
Q¹⁰ represents N or CR⁶ⁱ;
Q¹¹ represents N or CR^{6j};
Q⁵ represents CR^{3d}; Q⁶ represents N; and Q⁷ represents CR^{4f}; or
Q⁵ represents CR^{3d}; Q⁶ represents CR^{4e}; and Q⁷ represents N; or
Q⁵ represents N; Q⁶ represents CR^{4e}; and Q⁷ represents CR^{4f}; or
Q⁵ represents N; Q⁶ represents CR^{4e}; and Q⁷ represents N; or
Q⁵ represents N; Q⁶ represents N; and Q⁷ represents CR^{4f}; or
Q⁵ represents N; Q⁶ represents N; and Q⁷ represents N;
R^{6a}, R^{6b}, R^{6c}, R^{6d}, R^{6e}, R^{6f}, R^{6g}, R^{6h}, R⁶ⁱ and R^{6j} each independently represent hydrogen,
halogen, C₁₋₄alkyl, -NR^{9a}R^{9b}, or C₁₋₄alkyl substituted with one, two or three halo atoms;
R^{9a} and R^{9b} each independently represent hydrogen or C₁₋₄alkyl;

In an embodiment, the present invention relates to those compounds of any of Formula (D), (E) and (F) wherein R¹⁰, when present, is cytosine, thymine, uracil, or a modified or protected form thereof, or a bicyclic aromatic heterocyclic ring system selected from the group consisting of (a-1), (a-2) and (a-3): 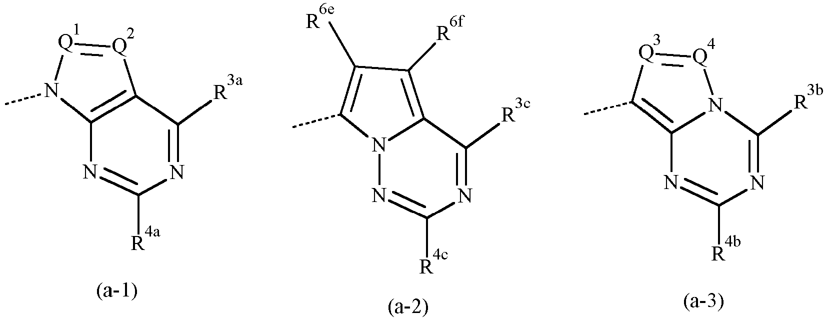
wherein R^{3a}, R^{3b} and R^{3c} each independently represent hydrogen, halo, -NR^{7a}R^{7b}, C₁₋₄alkyl, or
-O-C₁₋₄alkyl;
R^{7a} represents hydrogen;
R^{7b} represents hydrogen or C₁₋₄alkyl;
R^{4a}, R^{4b} and R^{4c} each independently represent hydrogen, halo, -NR^{8a}R^{8b}, or C₁₋₄alkyl;
R^{8a} and R^{8b} each independently represent hydrogen or C₁₋₄alkyl;
Q¹ represents N or CR^{6a};
Q² represents N or CR^{6b};
Q³ represents N or CR^{6c};
Q⁴ represents N or CR^{6d};
provided that maximum one of Q³ and Q⁴ represents N;
R^{6a}, R^{6b}, R^{6c}, R^{6d}, R^{6e} and R^{6f} each independently represent hydrogen, halogen,
C₁₋₄alkyl, -NR^{9a}R^{9b}, or C₁₋₄alkyl substituted with one, two or three halo atoms;
R^{9a} and R^{9b} each independently represent hydrogen or C₁₋₄alkyl.

In an embodiment, the present invention relates to those compounds of any of Formula (D), (E) and (F) wherein R¹⁰, when present, is a bicyclic aromatic heterocyclic ring system (a-1), wherein R^{3a} represents -NH₂ or chloro.

All possible combinations of the above-indicated embodiments are considered to be embraced within the scope of this invention.

### Pharmacology

It has been determined that spirobicyclic nucleosides show interesting activity, for example against PRMT5. Novel intermediates that may be used to synthesise spirobicyclic nucleoside analogues have been developed. In particular, these intermediates contain functional groups that can be diversified to give access to a variety of spirobicyclic nucleoside analogues. Accordingly, disclosed herein are compounds which are useful as intermediates for the preparation of spirobicyclic nucleoside analogues that may, for example, possess activity against PRMT5. Also disclosed herein are spirocyclic nucleoside analogues which may themselves have PRMT5 inhibitory activity or may serve as intermediates for further functionalisation to provide spirobicyclic nucleoside analogues with interesting activity.

It has been found that spirobicyclic nucleoside analogues may inhibit PRMT5 activity.

In particular such compounds may bind to the PRMT5 enzyme, and competitively with natural substrate SAM (S-adenosyl-L-methionine), to inhibit such enzyme.

Such compounds or pharmaceutical compositions thereof may be useful for treating or preventing, in particular treating, of diseases such as a blood disorder, metabolic disorders, autoimmune disorders, cancer, inflammatory diseases, cardiovascular diseases, neurodegenerative diseases, pancreatitis, multiorgan failure, kidney diseases, platelet aggregation, sperm motility, transplantation rejection, graft rejection, lung injuries and the like. Such compounds or pharmaceutical compositions thereof may be useful for treating or preventing, in particular treating, of diseases such as allergy, asthma, hematopoietic cancer, lung cancer, prostate cancer, melanoma, metabolic disorder, diabetes, obesity, blood disorder, sickle cell anemia, and the like.

Such compounds or pharmaceutical compositions thereof may be useful for treating or preventing, in particular treating, of diseases such as a proliferative disorder, such as an autoimmune disease, cancer, a benign neoplasm, or an inflammatory disease.

Such compounds or pharmaceutical compositions thereof may be useful for treating or preventing, in particular treating, of diseases such as a metabolic disorder comprising diabetes, obesity; a proliferative disorder comprising cancer, hematopoietic cancer, lung cancer, prostate cancer, melanoma, or pancreatic cancer; blood disorder; hemoglobinopathy; sickle cell anemia; β -thalessemia, an inflammatory disease, and autoimmune disease e.g. rheumatoid arthritis, systemic lupus erythematosus, Sjogren's syndrome, diarrhea, gastroesophageal reflux disease, and the like.

In some embodiments, the inhibition of PRMT5 may be useful in treating or preventing, in particular treating, the following non-limiting list of cancers: breast cancer, lung cancer, esophageal cancer, bladder cancer, hematopoietic cancer, lymphoma, medulloblastoma, rectum adenocarcinoma, colon adenocarcinoma, gastric cancer, pancreatic cancer, liver cancer, adenoid cystic carcinoma, lung adenocarcinoma, head and neck squamous cell carcinoma, brain tumors, hepatocellular carcinoma, renal cell carcinoma, melanoma, oligodendroglioma, ovarian clear cell carcinoma, and ovarian serous cystadenoma.

Examples of metabolic disorders which may be treated or prevented, in particular treated, include, but are not limited to, diabetes or obesity.

Examples of blood disorders which may be treated or prevented, in particular treated, include, but are not limited to, hemoglobinopathy, such as sickle cell disease or β-thalassemia. Examples of cancers which may be treated or prevented, in particular treated, include, but are not limited to, acoustic neuroma, adenocarcinoma, adrenal gland cancer, anal cancer, angiosarcoma (e.g., lymphangio sarcoma, lymphangioendothelio sarcoma, hemangio sarcoma), appendix cancer, benign monoclonal gammopathy, biliary cancer (e.g., cholangiocarcinoma), bladder cancer, breast cancer (e.g., adenocarcinoma of the breast, papillary carcinoma of the breast, mammary cancer, medullary carcinoma of the breast), brain cancer (e.g., meningioma; glioma, e.g., astrocytoma, oligodendroglioma; medulloblastoma), bronchus cancer, carcinoid tumor, cervical cancer (e.g., cervical adenocarcinoma), chordoma, choriocarcinoma, craniopharyngioma, colorectal cancer (e.g., colon cancer, rectal cancer, colorectal adenocarcinoma), epithelial carcinoma, ependymoma, endothelio sarcoma (e.g., Kaposi's sarcoma, multiple idiopathic hemorrhagic sarcoma), endometrial cancer (e.g. , uterine cancer, uterine sarcoma), esophageal cancer (e.g., adenocarcinoma of the esophagus, Barrett' s adenocarinoma), Ewing sarcoma, eye cancer (e.g., intraocular melanoma, retinoblastoma), familiar hypereosinophilia, gall bladder cancer, gastric cancer (e.g., stomach adenocarcinoma), gastrointestinal stromal tumor (GIST), head and neck cancer (e.g., head and neck squamous cell carcinoma, oral cancer (e.g., oral squamous cell carcinoma (OSCC), throat cancer (e.g., pharyngeal cancer, laryngeal cancer, nasopharyngeal cancer, oropharyngeal cancer)), hematopoietic cancers (e.g., leukemia such as acute lymphocytic leukemia (ALL) (e.g., B-cell ALL, T-cell ALL), acute myelocytic leukemia (AML) (e.g., B-cell AML, T-cell AML), chronic myelocytic leukemia (CML) (e.g., B-cell CML, T-cell CML), and chronic lymphocytic leukemia (CLL) (e.g., B-cell CLL, T- cell CLL); lymphoma such as Hodgkin lymphoma (HL) (e.g., B-cell HL, T-cell HL) and non-Hodgkin lymphoma (NHL) (e.g., B-cell NHL such as diffuse large cell lymphoma (DLCL) (e.g., diffuse large B-cell lymphoma (DLBCL)), follicular lymphoma, chronic lymphocytic leukemia/small lymphocytic lymphoma (CLL/SLL), mantle cell lymphoma (MCL), marginal zone B-cell lymphomas (e.g., mucosa-associated lymphoid tissue (MALT) lymphomas, nodal marginal zone B-cell lymphoma, splenic marginal zone B-cell lymphoma), primary mediastinal B-cell lymphoma, Burkitt lymphoma, lymphoplasmacytic lymphoma (i.e., "Waldenstrom's macro globulinemia"), immunoblastic large cell lymphoma, hairy cell leukemia (HCL), precursor B -lymphoblastic lymphoma and primary central nervous system (CNS) lymphoma; and T-cell NHL such as precursor T-lymphoblastic lymphoma/leukemia, peripheral T-cell lymphoma (PTCL) (e.g., cutaneous T-cell lymphoma (CTCL) (e.g., mycosis fungiodes, Sezary syndrome), angioimmunoblastic T-cell lymphoma, extranodal natural killer T-cell lymphoma, enteropathy type T-cell lymphoma, subcutaneous panniculitis-like T-cell lymphoma, anaplastic large cell lymphoma); a mixture of one or more leukemia/lymphoma as described above; and multiple myeloma (MM)), heavy chain disease (e.g. , alpha chain disease, gamma chain disease, mu chain disease), hemangioblastoma, inflammatory myofibroblastic tumors, immunocytic amyloidosis, kidney cancer (e.g., nephroblastoma a.k.a. Wilms' tumor, renal cell carcinoma), liver cancer (e.g., hepatocellular cancer (HCC), malignant hepatoma), lung cancer (e.g., bronchogenic carcinoma, non-small cell lung cancer (NSCLC), squamous lung cancer (SLC), adenocarcinoma of the lung, *Lewis lung carcinoma,* lung neuroendocrine tumors: typical carcinoid, atypical carcinoid, small cell lung cancer (SCLC), and large cell neuroendocrine carcinoma), leiomyosarcoma (LMS), mastocytosis (e.g., systemic mastocytosis), myelodysplastic syndromes (MDS), mesothelioma, myeloproliferative disorder (MPD) (e.g., polycythemia Vera (PV), essential thrombocytosis (ET), agnogenic myeloid metaplasia (AMM) a.k.a. myelofibrosis (MF), chronic idiopathic myelofibrosis, chronic myelocytic leukemia (CML), chronic neutrophilic leukemia (CNL), hypereosinophilic syndrome (HES)), neuroblastoma, neurofibroma (e.g., neurofibromatosis (NF) type 1 or type 2, schwannomatosis), neuroendocrine cancer (e.g., gastroenteropancreatic neuroendoctrine tumor (GEP-NET), carcinoid tumor), osteosarcoma, ovarian cancer (e.g., cystadenocarcinoma, ovarian embryonal carcinoma, ovarian adenocarcinoma), papillary adenocarcinoma, pancreatic cancer (e.g., pancreatic andenocarcinoma, intraductal papillary mucinous neoplasm (IPMN), Islet cell tumors), penile cancer (e.g., Paget' s disease of the penis and scrotum), pinealoma, primitive neuroectodermal tumor (PNT), prostate cancer (e.g., prostate adenocarcinoma), rectal cancer, rhabdomyosarcoma, salivary gland cancer, skin cancer (e.g., squamous cell carcinoma (SCC), keratoacanthoma (KA), melanoma, basal cell carcinoma (BCC)), small bowel cancer (e.g., appendix cancer), soft tissue sarcoma (e.g., malignant fibrous histiocytoma (MFH), liposarcoma, malignant peripheral nerve sheath tumor (MPNST), chondrosarcoma, fibrosarcoma, myxosarcoma), sebaceous gland carcinoma, sweat gland carcinoma, synovioma, testicular cancer (e.g., seminoma, testicular embryonal carcinoma), thyroid cancer (e.g., papillary carcinoma of the thyroid, papillary thyroid carcinoma (PTC), medullary thyroid cancer), urethral cancer, vaginal cancer, and vulvar cancer (e.g. , Paget' s disease of the vulva).

Examples of neurodegenerative diseases which may be treated or prevented, in particular treated, include, but are not limited to, motor neurone disease, progressive supranuclear palsy, corticobasal degeneration, Pick's disease, Alzheimer's disease, AIDS-related dementia, Parkinson's disease, amyotropic lateral sclerosis, retinitis pigmentosa, spinal muscular atropy, and cerebellar degeneration.

Examples of cardiovascular diseases which may be treated or prevented, in particular treated, include, but are not limited to, cardiac hypertrophy, restenosis, atherosclerosis, and glomerulonephritis.

Examples of inflammatory diseases which may be treated or prevented, in particular treated, include, but are not limited to, inflammation associated with acne, anemia (e.g., aplastic anemia, haemolytic autoimmune anaemia), rhinitis, asthma, arteritis (e.g., polyarteritis, temporal arteritis, periarteritis nodosa, Takayasu's arteritis), arthritis (e.g., crystalline arthritis, osteoarthritis, psoriatic arthritis, gouty arthritis, reactive arthritis, rheumatoid arthritis and Reiter's arthritis), upper respiratory tract disease, ankylosing spondylitis, amylosis, amyotrophic lateral sclerosis, autoimmune diseases, allergies or allergic reactions, atherosclerosis, bronchitis, bursitis, chronic prostatitis, conjunctivitis, Chagas disease, chronic obstructive pulmonary disease, diverticulitis, cermatomyositis, diabetes (e.g., type I diabetes mellitus, type 2 diabetes mellitus), a skin condition (e.g., psoriasis, eczema, eczema hypersensitivity reactions, burns, dermatitis, pruritus (itch)), endometriosis, Guillain-Barre syndrome, infection, ischaemic heart disease, Kawasaki disease, glomerulonephritis, gingivitis, hypersensitivity, headaches (e.g., migraine headaches, tension headaches), ileus (e.g., postoperative ileus and ileus during sepsis), idiopathic thrombocytopenic purpura, interstitial cystitis (painful bladder syndrome), gastrointestinal disorder (e.g., selected from peptic ulcers, regional enteritis, diverticulitis, gastrointestinal bleeding, eosinophilic gastrointestinal disorders (e.g., eosinophilic esophagitis, eosinophilic gastritis, eosinophilic gastroenteritis, eosinophilic colitis), gastritis, diarrhea, gastroesophageal reflux disease (GORD, or its synonym GERD), inflammatory bowel disease (IBD) (e.g., Crohn's disease, ulcerative colitis, collagenous colitis, lymphocytic colitis, ischaemic colitis, diversion colitis, Behcet's syndrome, indeterminate colitis) and inflammatory bowel syndrome (IBS)), lupus, morphea, myeasthenia gravis, myocardial ischemia, multiple sclerosis, nephrotic syndrome, pemphigus vulgaris, pernicious aneaemia, peptic ulcers, polymyositis, primary biliary cirrhosis, neuroinflammation associated with brain disorders (e.g. , Parkinson's disease, Huntington's disease, and Alzheimer's disease), prostatitis, chronic inflammation associated with cranial radiation injury, pelvic inflammatory disease, reperfusion injury, regional enteritis, rheumatic fever, systemic lupus erythematosus, schleroderma, scierodoma, sarcoidosis, spondyloarthopathies, Sjogren's syndrome, thyroiditis, transplantation rejection, tendonitis, trauma or injury (e.g. , frostbite, chemical irritants, toxins, scarring, burns, physical injury), vasculitis, vitiligo and Wegener's granulomatosis.

In particular the inflammatory disease is an acute inflammatory disease (e.g., for example, inflammation resulting from infection). In particular the inflammatory disease is a chronic inflammatory disease (e.g., conditions resulting from asthma, arthritis and inflammatory bowel disease). Such compounds may also be useful in treating inflammation associated with trauma and non-inflammatory myalgia. Such compounds may also be useful in treating inflammation associated with cancer.

Examples of autoimmune diseases which may be treated or prevented, in particular treated, include, but are not limited to, arthritis (including rheumatoid arthritis, spondyloarthopathies, gouty arthritis, degenerative joint diseases such as osteoarthritis, systemic lupus erythematosus, Sjogren's syndrome, ankylosing spondylitis, undifferentiated spondylitis, Behcet's disease, haemolytic autoimmune anaemias, amyotrophic lateral sclerosis, amylosis, multiple sclerosis, acute painful shoulder, psoriatic, and juvenile arthritis), asthma, atherosclerosis, osteoporosis, bronchitis, tendonitis, bursitis, skin condition (e.g., psoriasis, eczema, eczema hypersensitivity reactions, burns, dermatitis, pruritus (itch)), enuresis, eosinophilic disease, gastrointestinal disorder (e.g. , selected from peptic ulcers, regional enteritis, diverticulitis, gastrointestinal bleeding, eosinophilic gastrointestinal disorders (e.g., eosinophilic esophagitis, eosinophilic gastritis, eosinophilic gastroenteritis, eosinophilic colitis), gastritis, diarrhea, gastroesophageal reflux disease (GORD, or its synonym GERD), inflammatory bowel disease (IBD) (e.g., Crohn's disease, ulcerative colitis, collagenous colitis, lymphocytic colitis, ischaemic colitis, diversion colitis, Behcet's syndrome, indeterminate colitis) and inflammatory bowel syndrome (IBS)), and disorders ameliorated by a gastroprokinetic agent (e.g. , ileus, postoperative ileus and ileus during sepsis; gastroesophageal reflux disease (GORD, or its synonym GERD); eosinophilic esophagitis, gastroparesis such as diabetic gastroparesis; food intolerances and food allergies and other functional bowel disorders, such as non-ulcerative dyspepsia (NUD) and non-cardiac chest pain (NCCP, including costo-chondritis)).

An effective therapeutic daily amount would be from about 0.005 mg /kg to 50 mg/kg.

A compound that can be suitable to treat or prevent cancer or cancer-related conditions, may be administered alone or in combination with one or more additional therapeutic agents.

A pharmaceutical composition may comprise a pharmaceutically acceptable carrier and an active ingredient.

The carrier or diluent must be "acceptable" in the sense of being compatible with the other ingredients of the composition and not deleterious to the recipients thereof.

### Methods for the Preparation

In this section, as in all other sections unless the context indicates otherwise, references to Formulae also include all other sub-groups and examples thereof as defined herein.

The general preparation of some typical examples of the compounds of the invention is described hereunder and in the specific examples. Compounds are generally prepared from starting materials which are either commercially available, prepared by standard synthetic processes commonly used by those skilled in the art, or prepared as described in the specific examples. The following schemes are only meant to represent examples of the invention and are in no way meant to be a limit of the invention.

Alternatively, compounds of the present invention may also be prepared by analogous reaction protocols as described in the general schemes below, combined with standard synthetic processes commonly used by those skilled in the art of organic chemistry.

All variables are defined as mentioned hereabove unless otherwise is indicated or is clear from the context.

The skilled person will realize that in the reactions described in the Schemes, it may be necessary to protect reactive functional groups, for example hydroxy, amino, or carboxy groups, where these are desired in the final product, to avoid their unwanted participation in the reactions. Conventional protecting groups can be used in accordance with standard practice. This is illustrated in the specific examples.

The skilled person will realize that in the reactions described in the Schemes, it may be advisable or necessary to perform the reaction under an inert atmosphere, such as for example under N₂-gas atmosphere, for example when NaH is used in the reaction.

It will be apparent for the skilled person that it may be necessary to cool the reaction mixture before reaction work-up (refers to the series of manipulations required to isolate and purify the product(s) of a chemical reaction such as for example quenching, column chromatography, extraction).

The skilled person will realize that heating the reaction mixture under stirring may enhance the reaction outcome. In some reactions microwave heating may be used instead of conventional heating to shorten the overall reaction time.

The skilled person will realize that another sequence of the chemical reactions shown in the Schemes below, may also result in the desired compound of the invention.

The skilled person will realize that intermediates and compounds shown in the schemes below may be further functionalized according to methods well-known by the person skilled in the art. For example, compounds wherein R^{3a}, or any other substituent on a nucleobase or nucleobase derivative, represents Cl, can be converted into compounds wherein R^{3a} (or any other Cl substituent) represents NH₂ by reaction with NH₃ (e.g. 25% in water) in a typical solvent such as for example dioxane, at a typical temperature of about 100 °C.

The skilled person will realize that more compounds of the invention can be prepared by using similar synthetic protocols as described in the Schemes below.

In case one of the starting materials is available as a salt form, the skilled person will realize that it may be necessary to first treat the salt with a base, such as for example N,N-diisopropylethylamine (DIPEA).

All variables are defined as mentioned hereabove unless otherwise is indicated or is clear from the context.

In general, compounds of the invention can be prepared according to the following general schemes.

In the following schemes, unless otherwise defined, R⁴, R⁵ and R⁶ are each independently a hydroxyl protecting group; or R⁵ and R⁶ taken together are a diol protecting group; or R⁴ and R⁵ taken together are a diol protecting group, as defined herein. The skilled person will realise that protecting groups may be utilised, as necessary, in the following schemes and any protecting group may subsequently be removed to provide de-protected compounds. For example, where a compound referenced in the following scheme includes a nucleobase or nucleobase derivative, said nucleobase may be utilised in protected form, for example an NH₂ or OH on a nucleobase may be protected with an amino or hydroxyl protecting group, respectively or an NH₂ on a nucleobase may be replaced by a halo (e.g. chloro).

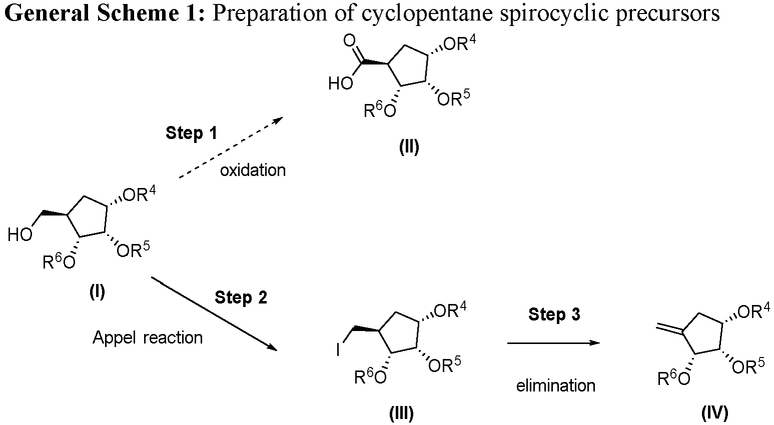

In **General Scheme 1,** R⁴ is limited to a silyl group, eg TBDMS, and R⁵ and R⁶ taken together form a diol protecting group.

In **General Scheme 1,** the following reaction conditions typically apply:
**1: (I)** is reacted is with an oxidant, such as phenyliodine(III) diacetate (PIDA), in the presence of (2,2,6,6-Tetramethyl-piperidin-1-yl)oxyl (TEMPO) in a suitable solvent mixture such as for example acetonitrile and water at a suitable temperature range such as for example room temperature - 50⁰C;
**2: (I)** is reacted with PPh₃, iodine and a base, such as imidazole in a suitable solvent such as for example THF at a suitable temperature range such as for example room temperature;
**3: (III)** is reacted with a base, such as DBU in a suitable solvent such as for example THF at a suitable temperature range such as for example between 60°C and 90°C.

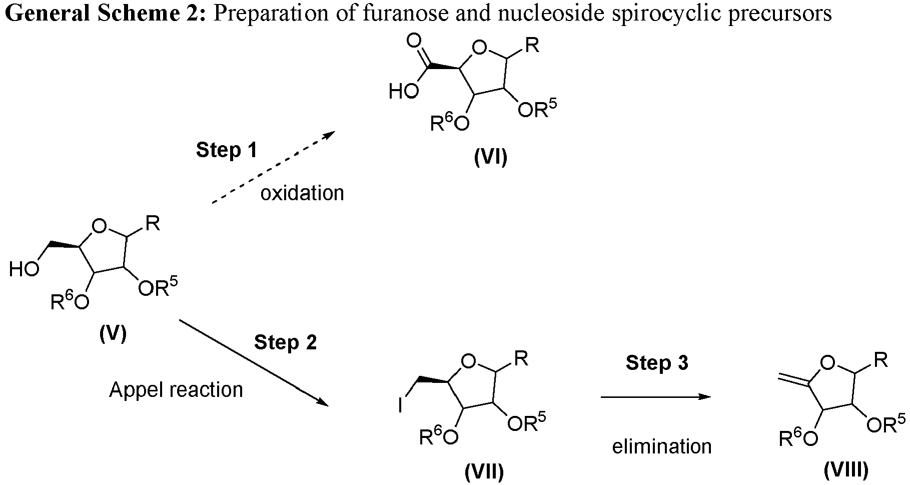

In **General Scheme 2,** R represents R¹⁰ as defined herein or OR⁴, R⁴, R⁵ and R⁶ are each independently a hydroxyl protecting group; or R⁵ and R⁶ taken together are a diol protecting group; or R⁴ and R⁵ taken together are a diol protecting group, as defined herein.

In **General Scheme 2,** the following reaction conditions typically apply:
**1: (V)** is reacted is with an oxidant, such as phenyliodine(III) diacetate (PIDA), in the presence of (2,2,6,6-Tetramethyl-piperidin-1-yl)oxyl (TEMPO) in a suitable solvent mixture such as for example acetonitrile and water at a suitable temperature range such as for example room temperature - 50⁰C;
**2: (V)** is reacted with PPh₃, iodine and a base, such as imidazole in a suitable solvent such as for example THF at a suitable temperature range such as for example room temperature;
**3: (VII)** is reacted with a base, such as DBU in a suitable solvent such as for example THF at a suitable temperature range such as for example between 60°C and 90°C.

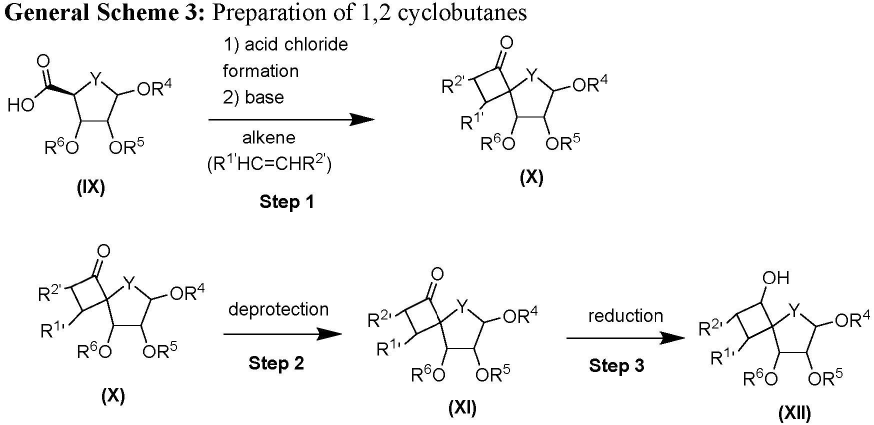

In **General Scheme 3,** one of R¹' and R²' is hydrogen or C₁-₆ alkyl and the other is a silyl group, C₁-₆ alkyl optionally substituted with a silyl group, -O-C₁-₆ alkyl, -S-C₁-₆ alkyl, -S-aryl, -O-aryl, B(OR^{b})₂, or C(O)R^{a}, and R^{a} is hydrogen or C₁-₆ alkyl. A skilled person will understand that after the deprotection step, R¹' and R²' are unprotected if initially a protecting group was present.

It will be appreciated that General Scheme 3 can also be carried out on nucleoside analogues (i.e. where OR⁴ is replaced by R¹⁰).

In **General Scheme 3,** the following reaction conditions typically apply:
**1: (IX)** is reacted with oxalyl chloride in a suitable solvent, such as DCM, optionally in the presence of a catalytic amount of DMF at a suitable temperature range such as for example between 0⁰C and room temperature; The acid chloride intermediate is treated with a base, such as triethyl amine and an alkene (R₁HC=CHR_{2'}), in a suitable solvent such as acetonitrile or toluene, or the like, at a suitable temperature range such as for example between 80 ⁰C and 130 ⁰C. Instead of the activation through an acid chloride, alternative methods can be used, such as reaction of (IX) with 2-chloro-1-methylpyridinium iodide, in a suitable solvent such as acetonitrile at a suitable temperature range such as for example between 80 ⁰C and 110 ⁰C. This reaction can be carried out in the presence of a base, such as diisopropyl ethyl amine or the like and an alkene (R_{1'}HC=CHR_{2'});
**2:** In case R1' or R2' is a silyl group: **(X)** is reacted with a fluoride source, such as TBAF in the presence of a protic solvent, such as HOAc, and an aprotic solvent, such as THF, in a suitable temperature range such as for example between 0 ⁰C and 60 ⁰C; An alternative procedure is to treat **(X)** with trimethylsilyl triflate (TMS-OTf), in a suitable solvent, such as DCM, at a temperature range between 0 ⁰C and 50 ⁰C, eg room temperature. In case R1' or R2' is PhS: the deprotection may be carried out in the presence of a reductant, such as Raney Nickel optionally in the presence of hydrogen, in a solvent such as ethanol or THF, or the like in a temperature range between 60 ⁰C and 120 ⁰C.
**3: (XI)** is reacted with a reductant, such as sodium borohydride or the like, in a suitable solvent such as for example methanol at a suitable temperature for example between -78⁰C and 0⁰C.

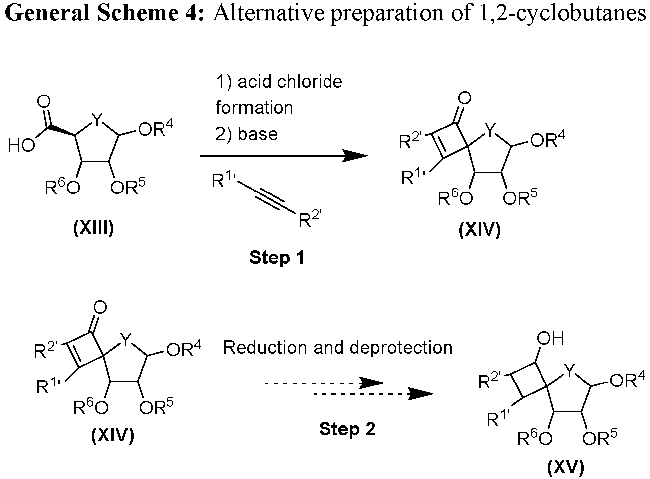

In **General Scheme 4,** one of R¹' and R²' is hydrogen or C₁-₆ alkyl and the other is a silyl group, C₁-₆ alkyl optionally substituted with a silyl group, -O-C₁-₆ alkyl, or C(O)R^{a}, and R^{a} is hydrogen or C₁-₆ alkyl. A skilled person will understand that after the deprotection step, R¹' and R²' are unprotected if initially a protecting group was present.

It will be appreciated that General Scheme 4 can also be carried out on nucleoside analogues (i.e. where OR⁴ is replaced by R¹⁰).

In **General Scheme 4,** similar conditions as in **General Scheme 3** typically apply.

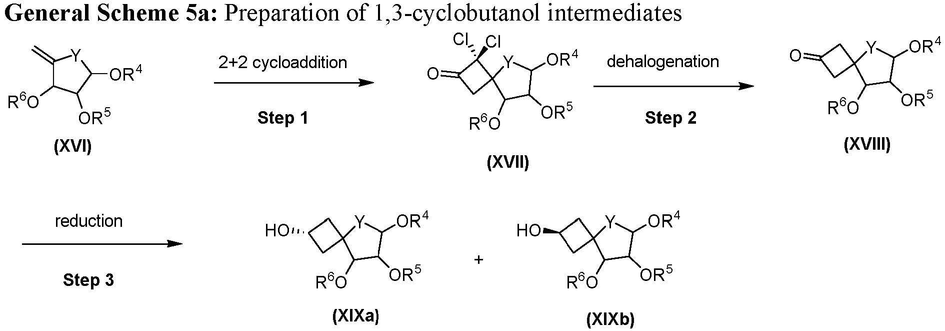

In **General Scheme 5a,** R⁴, R⁵ and R⁶ are each independently a hydroxyl protecting group; or R⁵ and R⁶ taken together are a diol protecting group; or R⁴ and R⁵ taken together are a diol protecting group, as defined herein.

In **General Scheme 5a,** the following reaction conditions typically apply:
**1: (XVI)** is reacted with a zinc source, such as zinc dust or zinc-copper couple, in the presence of dichloro ketene precursor, such as trichloro acetyl chloride in a suitable solvent, such as diethyl ether or THF, or the like, at a suitable temperature range such as for example room temperature and 50 ⁰C. Resulting **(XVII)** may either be isolated or converted via **Step 2,** in the same pot;
**2: (XVII)** is reacted with zinc powder in the presence of a proton source, such as HOAc, in an aprotic solvent, such as THF, in a suitable temperature range such as for example between room temperature and 70 ⁰C;
**3: (XVIII)** is reacted with a reductant, such as sodium borohydride or the like, in a suitable solvent such as for example methanol at a suitable temperature for example between -78⁰C and 0⁰C. Depending on the nature of the protecting groups, either **(XIXa)** or **(XIXb),** might be obtained, or a mixture thereof.

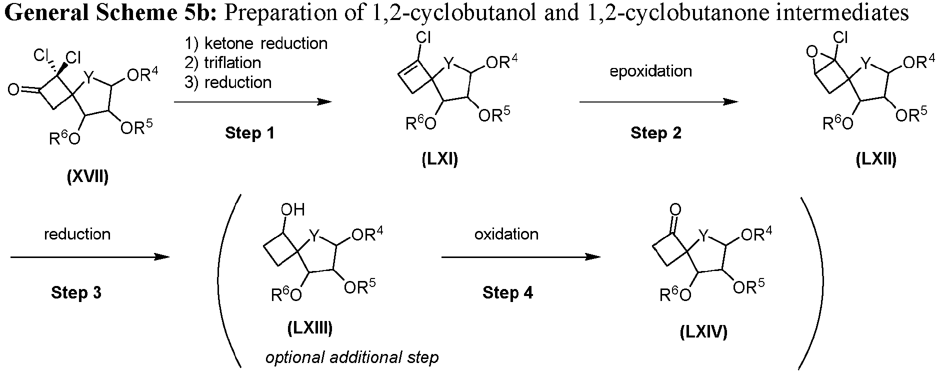

In **General Scheme 5b,** the following reaction conditions typically apply:
**1: (XVII)** is first reacted with a reductant, such as for example sodium borohydride in a suitable solvent such as THF at a suitable temperature such as for example between 0 °C and room temperature. The resulting intermediate is treated with a sulfonating agent such as for example triflic anhydride together with a base such as for example pyridine in an anhydrous solvent such as dichloromethane at a suitable temperature for example at 0 °C. The resulting intermediate is then treated with zinc powder in the presence of a proton source, such as HOAc, in combination with a solvent, such as methanol, at a suitable temperature such as for example 60 °C.
**2: (LXI)** is treated with an oxidant such as for example m-CPBA in a suitable solvent for example dichloromethane in a suitable temperature range such as for example between room temperature and 40 °C.
**3: (LXII)** is reacted with a reducing agent such as for example sodium cyanoborohydride or lithium borohydride in an alcoholic solvent for example iso-propylalcohol in a suitable temperature range between room temperature and 60 °C.
**4: (LXIII)** is treated with an oxidizing agent such as for example Dess-Martin Periodinane (DMP) in a suitable solvent for example dichloromethane at a suitable temperature such as for example room temperature.

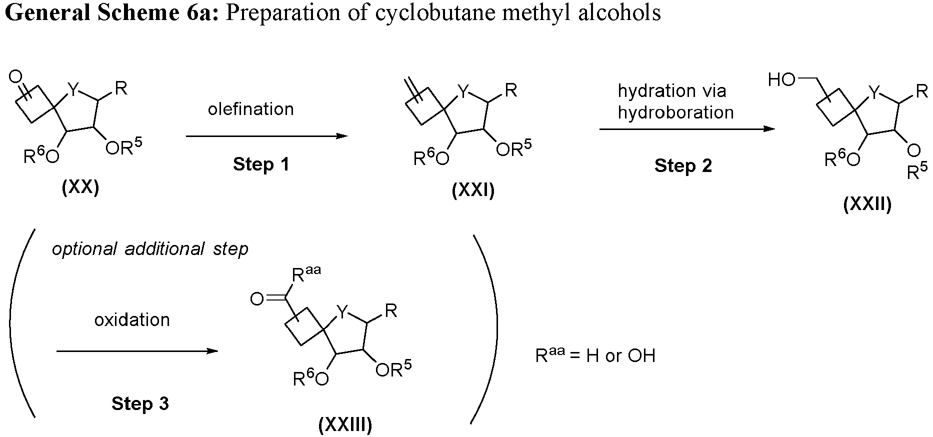

In **General Scheme 6a,** R represents R¹⁰ as defined herein or OR⁴, R⁴, R⁵ and R⁶ are each independently a hydroxyl protecting group; or R⁵ and R⁶ taken together are a diol protecting group; or R⁴ and R⁵ taken together are a diol protecting group, as defined herein.

This chemistry is compatible with 1,2-cyclobutanones and 1,3-cyclobutanones.

It will be appreciated that, where a carboxylic acid-substituted cyclobutyl is prepared, this can be further derivatised by esterification.

In **General Scheme 6a,** the following reaction conditions typically apply:
**1:** In the presence of a reagent such as for example methyltriphenylphosphonium bromide (MePPh3+Br-), in the presence of a base such as for example potassium tert-butoxide (KOtBu), in a solvent such as for example THF, at a suitable temperature such as for example between 0°C and r.t.; Alternatively, using bis(cyclopentadienyl)dimethyltitanium in a suitable solvent system, such as toluene and THF, in a suitable temperature range such as for example between 50 ⁰C and 70 ⁰C;
**2:** In a first step in the presence of an alkene precursor **(XXI)** and a 9-BBN solution in THF typically under nitrogen atmosphere at a temperature between room temperature and reflux. In a second step in the presence of an aqueous base such as for example aqueous NaOH, in the presence of an oxidizing agent such as for example H₂O₂, typically at a temperature between 0 °C to r.t.;
**3:** Using oxidation conditions, well known to the skilled person in the art; eg Dess-Martin periodinane, in a suitable solvent such as dichloro methane, typically at a temperature between 0 °C to room temperature (for the aldehyde, i.e. wherein R^{aa} is H). Alternatively, using an oxidant, such as phenyliodine(III) diacetate (PIDA), in the presence of (2,2,6,6-Tetramethyl-piperidin-1-yl)oxyl (TEMPO) in a suitable solvent mixture such as for example acetonitrile and water at a suitable temperature range such as for example between room temperature and 50⁰C (for the carboxylic acid, i.e. wherein R^{aa} is OH).

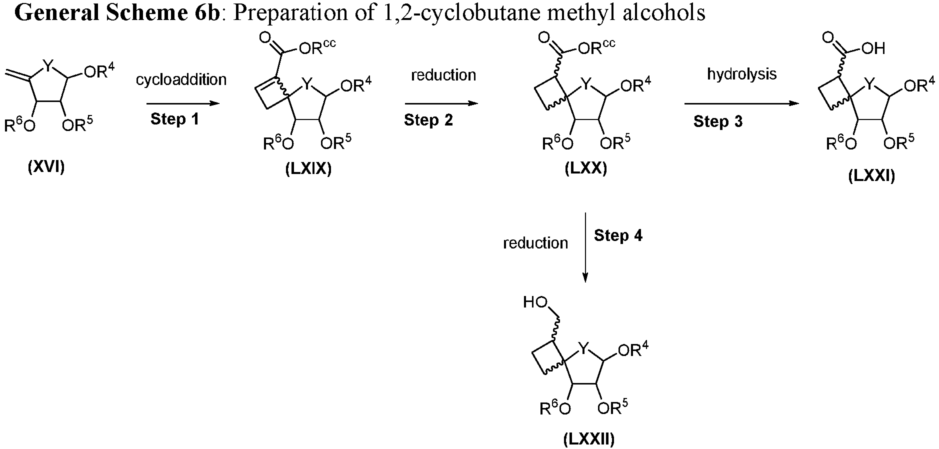

In **General Scheme 6b,** R^{cc} is C₁₋₆ alkyl.

In **General Scheme 6b,** the following reaction conditions typically apply:
**1: (XVI)** under an inert atmosphere is reacted with an unsaturated reagent such as methyl propiolate in the presence of a metal complex such as methylaluminoxane, in a suitable solvent, such as for example DCM, at a suitable temperature such as for example between 0°C and r.t.;
**2: (LXIX)** is treated with a hydrogenation catalyst, such as Pt/C in a suitable solvent, such as ethylacetate, in atmosphere of hydrogen, at a temperature between room temperature and 50 ⁰C.
**3: (LXX)** is reacted with a base such as lithium hydroxyde in an aqueous solvent mixture such as for example aqueous methanol in water. The resulting mixture is reacted for a suitable time such as for example 3 hours at a controlled temperature such as for example between 0°C and r.t.
**4: (LXX)** is reacted with a reductant, such as lithium borohydride or the like, in a suitable solvent such as for example THF at a suitable temperature for example between -78 ⁰C and room temperature.

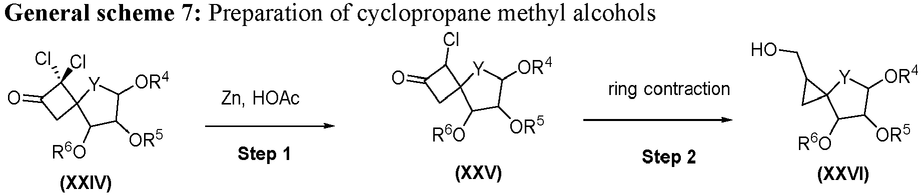

In **General Scheme 7,** R⁴, R⁵ and R⁶ are each independently a hydroxyl protecting group; or R⁵ and R⁶ taken together are a diol protecting group; or R⁴ and R⁵ taken together are a diol protecting group, as defined herein.

In **General Scheme 7,** the following reaction conditions typically apply:
**1: (XXIV)** is reacted with zinc powder in the presence of a proton source, such as 1 equivalent of HOAc, in an aprotic solvent, such as THF, in a suitable temperature of 0 ⁰C, using a short reaction time, eg 20 minutes;
**2: (XXV)** is reacted with a reductant, such as sodium borohydride or the like, in a suitable solvent such as for example THF at a suitable temperature for example between -0⁰C and room temperature. The mixture is then treated with aqueous base, such as NaOH (1 M) or the like at a suitable temperature range between room temperature and 50⁰C.

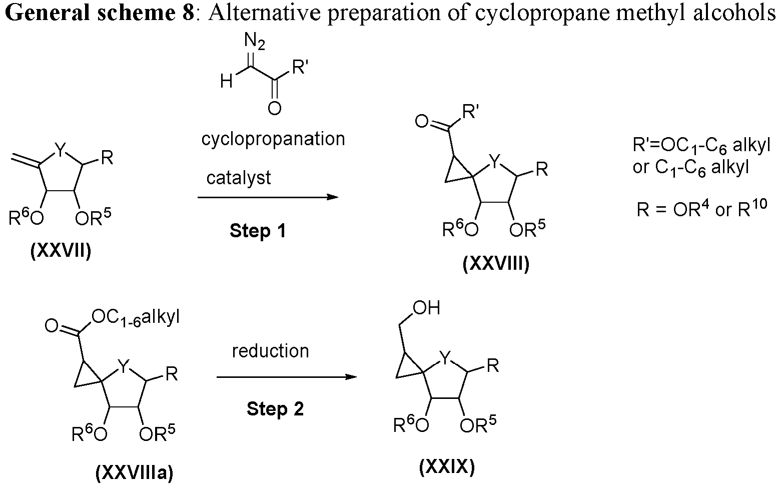

In **General Scheme 8,** R represents R¹⁰ as defined herein or OR⁴, R⁴, R⁵ and R⁶ are each independently a hydroxyl protecting group; or R⁵ and R⁶ taken together are a diol protecting group; or R⁴ and R⁵ taken together are a diol protecting group, as defined herein.

In **General Scheme 8,** the following reaction conditions typically apply:
**1: (XXVII)** is treated under an inert atmosphere with a catalyst such as Rh₂(OAc)₄ or rhodium(II) triphenylacetate dimer with alpha diazo ketone or diazo ester in a suitable solvent, such as dichloromethane at a suitable temperature range between room temperature and 50⁰C. The catalyst may be a chiral catalyst, such as (S)-Ph-Pheox Ru(II) or (R)-Ph-Pheox Ru(II), so that the synthesis is asymmetric;
**2: (XXVIIIa)** is reacted with a reductant, such as lithium borohydride or the like, in a suitable solvent such as for example THF at a suitable temperature for example between -0⁰C and room temperature.

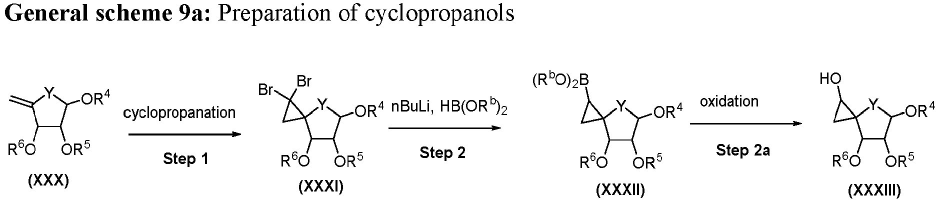

In **General Scheme 9a,** R⁴, R⁵ and R⁶ are each independently a hydroxyl protecting group; or R⁵ and R⁶ taken together are a diol protecting group; or R⁴ and R⁵ taken together are a diol protecting group, as defined herein. R^{b} is a boronic acid protecting group.

In **General Scheme 9a,** the following reaction conditions typically apply:
**1: (XXX)** is treated under an inert atmosphere with bromoform in a biphasic solvent system, consisting of DCM or the like, and water. The reaction requires a phase transfer catalyst, such as benzyltriethylammonium chloride or the like, and a base, such as sodium hydroxide in a suitable solvent, at a suitable temperature such as room temperature;
**2: (XXXI)** is reacted under an inert atmosphere with a strong base, such as nBuLi or the like in a suitable solvent such as for example THF at a suitable temperature for example between - 0⁰C and low temperature, such as -100 ⁰C. The resulting mixture is then treated with a borane, such as catechol borane and the same temperature and allowed to warm to 50 ⁰C, resulting in the *in situ* formation of **(XXXII).**
**2a: (XXXII)** is reacted at room temperature with a mixture of aqueous hydrogen peroxide and a base, such as sodium hydroxide, under an inert atmosphere with a strong base, such as *n*BuLi or the like in a suitable solvent.

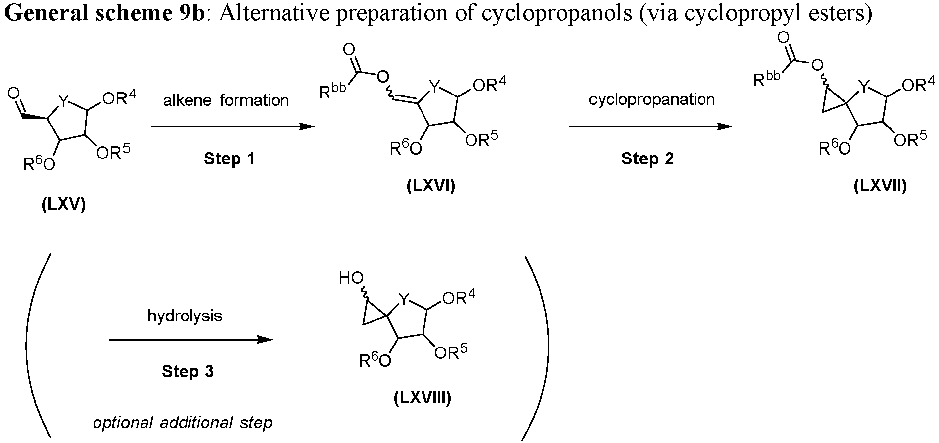

In **General Scheme 9b,** -C(O)R^{bb} is -C(O)C₁₋₆alkyl or a hydroxyl protecting group, such as benzoyl.

In **General Scheme 9b,** the following reaction conditions typically apply:
**1: (LXV)** is treated under an inert atmosphere with a suitable base such as potassium carbonate or the like and reacted with an anhydride such as for example benzoyl anhydride in a suitable anhydrous solvent such as for example acetonitrile at a suitable temperature as for example between room temperature and 60 °C.
**2: (LXVI)** is treated under inert atmosphere with diiodomethane and diethylzinc in an anhydrous solvent such as for example 1,2-dichloroethane at a suitable temperature as for example between room temperature and 50 °C.
**3: (LXVII)** is reacted with a base such as potassium carbonate in an aqueous solvent mixture such as for example aqueous methanol in water. The resulting mixture is reacted for a suitable time such as for example 3 hours at a controlled temperature such as 0 °C.

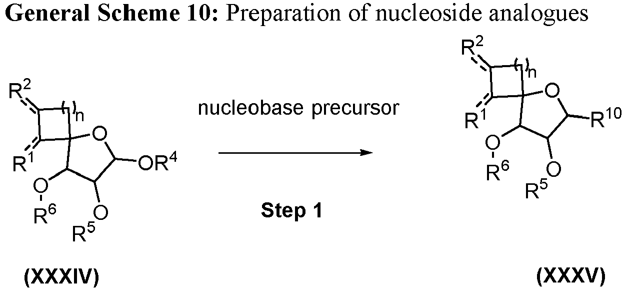

In **General Scheme 10,** R¹, R², R¹⁰ and n are as defined herein, R⁴ is preferably Me or -C(O)C₁₋₆alkyl and R⁵ and R⁶ are preferably -C(O)C₁₋₆alkyl. In **General Scheme 10,** the following reaction conditions typically apply:
**1:** An intermediate of **Formula (XXXIV)** is reacted with a suitable nucleobase precursor, eg 6-chloro purine, typically in the presence of a reagent such as for example bis-(trimethylsilyl)acetamide (BSA), a reagent such as for example trimethylsilyl triflate (TMSOTf), in a solvent such as for example anhydrous CH₃CN; typically at a temperature between room temperature and 100 °C;

It will be appreciated that scheme may be carried out with any suitable nucleobase precursor. The scheme may optionally involve additional step(s) of deprotection of R⁵ or R⁶ and/or conversion of the nucleobases precursor to a nucleobase. For example, after coupling, 6-chloro purine may be converted to adenine.

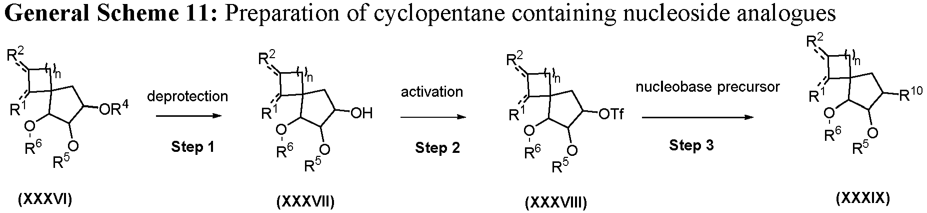

In **General Scheme 11,** R¹, R², R¹⁰ and n are as defined herein, R⁴ is a protecting group, for example a silyl group, and R⁵ and R⁶ taken together are a diol protecting group.

In **General Scheme 11,** the following reaction conditions typically apply:
**1** Typically deprotection in the presence of a reagent such as for example tetrabutylammonium fluoride (TBAF); in a suitable solvent such as for example THF; at a suitable temperature such as for example r.t.;
**2** Reaction with a suitable reagent such as for example Tf₂O (triflic anhydride or trifluoromethanesulfonic anhydride), in a suitable solvent such as for example dichloromethane (DCM) at a suitable temperature such as for example 0 °C;
**3** Reaction with suitable nucleobase precursor in the presence of a base such as for example potassium tert-butoxide, in a suitable solvent such as for example DMF, at a suitable temperature such as for example between -10 °C and 0 °C;

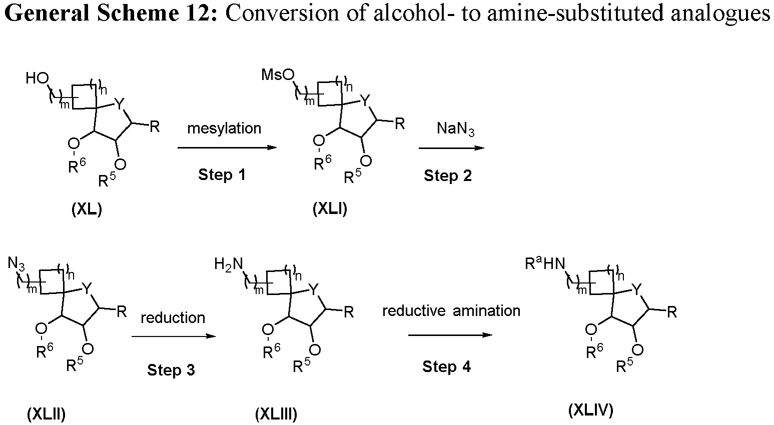

In **General Scheme 12,** R^{a}, m and n are as defined herein, R represents R¹⁰ as defined herein or OR⁴, R⁴, R⁵ and R⁶ are each independently a hydroxyl protecting group; or R⁵ and R⁶ taken together are a diol protecting group; or R⁴ and R⁵ taken together are a diol protecting group, as defined herein.

In **General Scheme 12,** the following reaction conditions typically apply:
**1: (XL)** is reacted with an activating agent, such as mesyl chloride in the presence of a suitable base, such as pyridine, in a suitable solvent such as DCM, typically at a temperature range between 0 ⁰C and 40 °C;
**2: (XLI)** is reacted with an azide, such as sodium azide, in the presence of a catalyst, such as tert butyl ammonium iodide (TBAI), in a suitable solvent such as DMF or DMA, or the like, typically at a temperature range between 0 °C and 120 °C;
**3: (XLII)** is treated with a hydrogenation catalyst, such as Pd/C in a suitable protic solvent, such as methanol or the like, in an atmosphere of hydrogen, at a temperature between room temperature and 50 ⁰C.
**4: (XLIII)** is treated with acetic acid in a suitable solvent, such dichloro methane, and an appropriate aldehyde or ketone. After equilibration at a suitable temperature, such as room temperature, the reduction takes place in the presence of a suitable reductant, such as sodium triacetoxy borohydride (NaBH(OAc)₃) in a suitable temperature range between 0 ⁰C and room temperature.

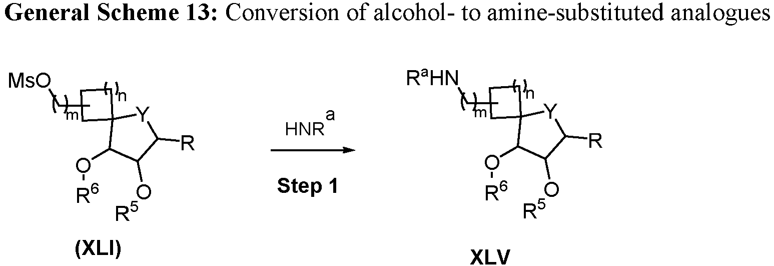

In **General Scheme 13,** R^{a}, m and n are as defined herein, R represents R¹⁰ as defined herein or OR⁴, R⁴, R⁵ and R⁶ are each independently a hydroxyl protecting group; or R⁵ and R⁶ taken together are a diol protecting group; or R⁴ and R⁵ taken together are a diol protecting group, as defined herein.

In **General Scheme 13**, the following reaction conditions typically apply:
**1: (XLI)** is treated with a suitable amine , without an additional solvent in a suitable temperature range between 50 ⁰C and 120 ⁰C.

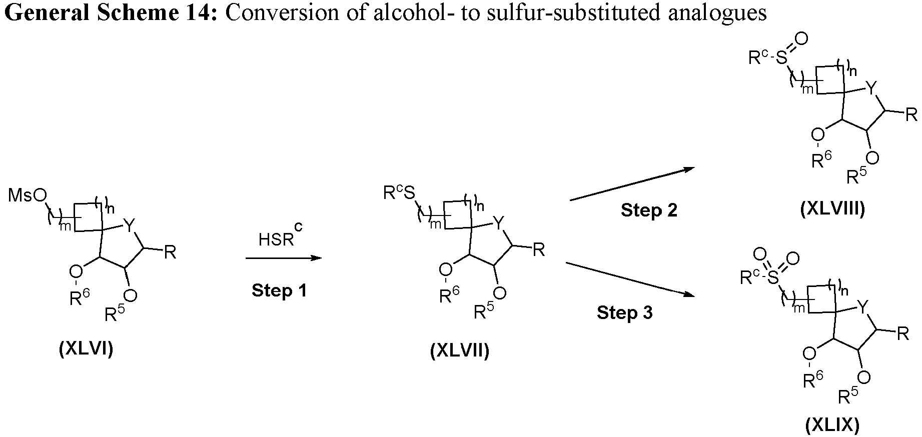

In **General Scheme 14**, R^{c}, m and n are as defined herein, R represents R¹⁰ as defined herein or OR⁴, R⁴, R⁵ and R⁶ are each independently a hydroxyl protecting group; or R⁵ and R⁶ taken together are a diol protecting group; or R⁴ and R⁵ taken together are a diol protecting group, as defined herein.

In **General Scheme 14**, the following reaction conditions typically apply:
**1: (XLVI)** is treated with a suitable thiol, in the presence of a suitable base, such as sodium hydride, in an aprotic polar solvent, such as DMF or NMP, or the like in a suitable temperature range between 20 ⁰C and 120 ⁰C.
**2: (XXLVII)** is treated with a suitable oxidant, such as hydrogen peroxide in a suitable solvent system, such as acetone and water in a suitable temperature range between 20 ⁰C and 60 ⁰C.
**3: (XLVII)** is treated with a suitable oxidant, such as mCPBA in a suitable solvent, such as dichloro methane, in a suitable temperature range between 0 ⁰C and 30 ⁰C.

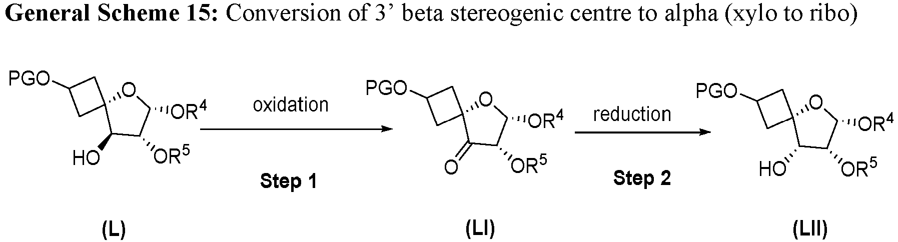

In **General Scheme 15,** the following reaction conditions typically apply:
**1: (L)** is oxidized to the corresponding ketone, using Dess Martin periodinane in a solvent such as dichloro methane at a temperature range between 0 ⁰C and room temperature.
**2: (LI)** can then be reduced by a reagent such as NaBH₄ in a solvent such as methanol or ethanol at a temperature range between 0 ⁰C and room temperature or by using DIBAL (diisobutylaluminum hydride) or the like in a solvent such as dichloro methane at a temperature range between -78 ⁰C and 0 ⁰C.

A skilled person will understand that Scheme 15 might also be applicable for other 1,3-cyclobutane nucleoside analogues, as well as for 1,2-cyclobutanes or cyclopropyl spirobicyclic nucleoside analogues. For example, general scheme 15 could also be represented as: 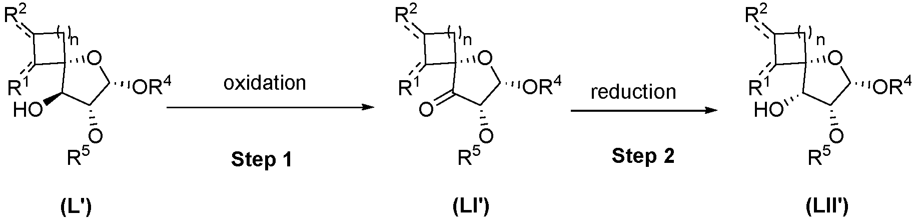 wherein n, R¹ and R² are as defined herein; and wherein the typical reaction conditions described for (L) and (LI) apply to (L') and (LI'), respectively.

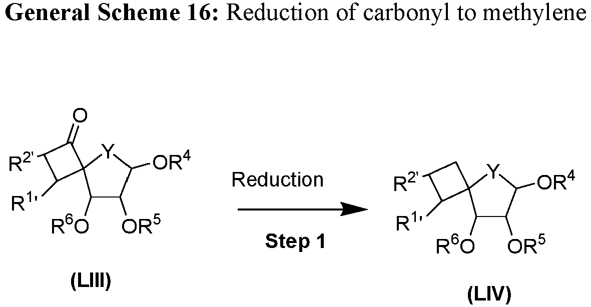

In **General Scheme 16,** one of R¹' and R²' is hydrogen or C₁-₆ alkyl and the other is a silyl group, C₁-₆ alkyl optionally substituted with a silyl group, -O-C₁-₆ alkyl, -S-C₁-₆ alkyl, -S-aryl, or -O-aryl.

In **General Scheme 16,** the following reaction conditions typically apply:
**1: (LIII)** is converted *in situ* to an hydrazone by treatment with hydrazine, optionally in the presence of an acid such as sulfuric acid, in a suitable solvent such as ethanol in a suitable temperature range between 20 ⁰C and 80 ⁰C. The hydrazone can be removed by treatment with a base, such as potassium tert butoxide (KOtBu), or the like, in a suitable solvent such as DMSO at a temperature range between 4 ⁰C and 40 ⁰C Alternatively, **(LIII)** is converted *in situ* to an tosylhydrazone, by reacting with Tosyl-NHNH₂ in a suitable solvent, such as methanol or ethanol or the like in a suitable temperature range between 60 ⁰C and 100 ⁰C, followed by reaction with a reductant such as sodium borohydride or the like in a suitable temperature range between 20 ⁰C and 70 ⁰C.

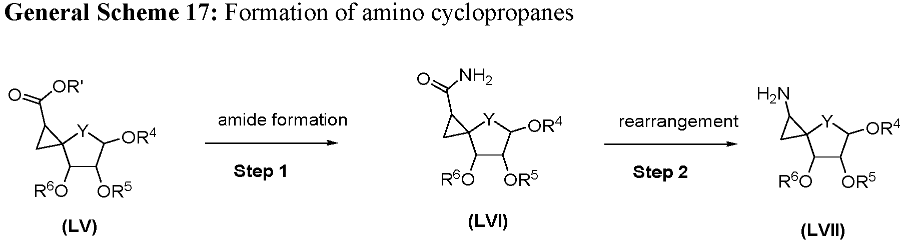

In **General Scheme 17**, R⁴, R⁵ and R⁶ are each independently a hydroxyl protecting group; or R⁵ and R⁶ taken together are a diol protecting group; or R⁴ and R⁵ taken together are a diol protecting group, as defined herein.

In **General Scheme 17**, the following reaction conditions typically apply:
**1**: **(LV)** is hydrolysed in the presence of LiOH or the like in an aqueous solvent system in the presence of methanol or THF or the like at a temperature range between 0 ⁰C and 20 ⁰C. The resulting carboxylic acid is converted in the carboxamide employing ammonia or ammonium chloride or the like in the presence of an activating agent such as HATU or the like, in a solvent such as DMF or DCM or the like at temperature range between 20 ⁰C and 50 ⁰C. Finally, the rearrangement occurs in the presence of NaOCl or NaOBr optionally in the presence of sodium hydroxide in an aqueous solvent at temperature range between 0 ⁰C and 50 ⁰C.

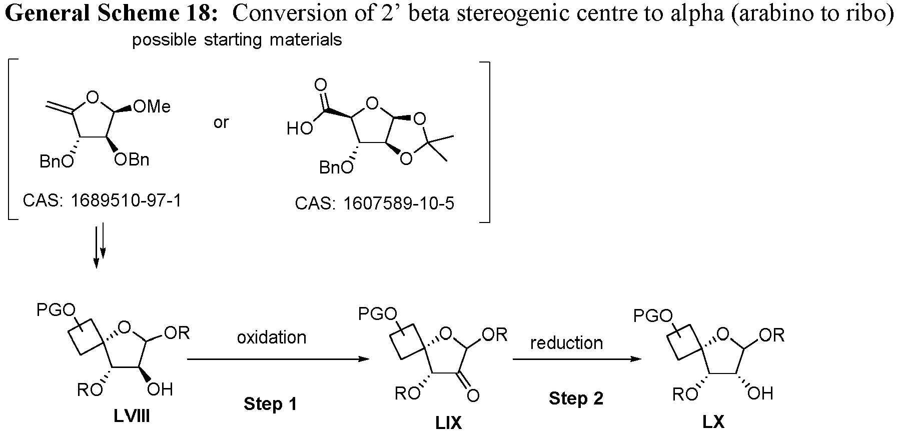

In **General Scheme 18,** R is defined as a suitable hydroxyl protecting group as defined herein, PG is as defined herein.

In **General Scheme 18,** The following reaction conditions typically apply:
**1: (LVIII)** is oxidized to the corresponding ketone, using Dess Martin periodinane in a solvent such as dichloromethane at a temperature range between 0 °C and room temperature.
**2: (LIX)** can then be reduced by a reagent such as NaBH₄ in a solvent such as methanol at a temperature range between 0 ⁰C and room temperature or by using DIBAL or the like in a solvent such as dichloromethane at a temperature range between -78 °C and 0 °C.

A skilled person will understand that Scheme 18 might also be applicable for other 1,3-cyclobutane or 1,2-cyclobutane spirobicyclic nucleoside analogues or for cyclopropyl spirobicyclic nucleoside analogues.

In all these preparations, the reaction products may be isolated from the reaction medium and, if necessary, further purified according to methodologies generally known in the art such as, for example, extraction, crystallization, trituration and chromatography.

The chirally pure forms of the compounds of Formula (I) form a preferred group of compounds. It is therefore that the chirally pure forms of the intermediates and their salt forms are particularly useful in the preparation of chirally pure compounds of Formula (I). Also enantiomeric mixtures of the intermediates are useful in the preparation of compounds of Formula (I) with the corresponding configuration.

### Examples

Hereinafter, the term "rt" or "r.t." means room temperature; "RT" or "Rt" means retention time; "Rf" means retention factor; "Me" means methyl; "MeOH" means methanol; "MeOD" means mono-deuteromethanol; "Et" means ethyl; "EtOH" means ethanol; "EA" or "EtOAc" means ethyl acetate; "Ac" means acetyl; "Ac₂O" means acetic anhydride; "AcOH" means acetic acid; "Et₂O" means di-ethylether; "Int." means intermediate; "DMF" means N,N-dimethyl formamide; "THF" means tetrahydrofuran; "LC" means liquid chromatography; "celite^{®}" means diatomaceous earth; "LCMS" or "LC-MS" means Liquid Chromatography/Mass spectrometry; "GCMS" or "GC-MS" means "Gas Chromatography/Mass spectrometry"; "CV" means column columes; "HPLC" means high-performance liquid chromatography; "TFA" means trifluoroacetic acid; "h" means hour(s); "Me₂S" means dimethyl sulphide; "DMSO" means dimethyl sulfoxide; "DMSO-d6" means deuterated dimethyl sulfoxide; "DIPE" means diisopropyl ether; "DCM" means dichloromethane; "PPh₃" means triphenylphosphine; "TBAF" means tetrabutylammonium fluoride; "DBU" means 1,8-diazabicyclo[5.4.0]undecene-7; "eq." or "equiv" means equivalent(s); "KOtBu"means potassium tert-butoxide; "TBDMSCl" means tert-butyldimethlsilyl chloride; "Bn" means benzyl; "9-BBN" means 9-Borabicyclo[3.3.1]nonane; "Tf₂O" means triflic anhydride; "TBDMS" means tert-butyl dimethylsilyl or t-butyl dimethylsilyl; "aq." means aqueous; "Ts" or "Tos" means tosyl (p-toluenesulfonyl); "DEAD" means diethyl azodicarboxylate; "Bz" means benzoyl; "BnBr" means benzyl bromide; "Bn" means benzyl; "anhyd." means anhydrous; "p-TsOH" means 4-methylbenzenesulfonic acid; "(R)-MonoPhos" means (R)-N,N-dimethyldinaphtho[2,1-D:1',2'-F][1,3,2]dioxaphosphepin-4-amine; "BSA" means N,O-bis(trimethylsilyl)acetamide; "TMSOTf" means trimethylsilyl trifluoromethanesulfonate; "Boc" means tert-butyloxycarbonyl; "Prep SFC" means Preparative Supercritical Fluid Chromatography; "Piv" means pivaloyl; "PivCl" means pivaloyl chloride; "MePPh₃⁺Br⁻" means methyltriphenylphosphonium bromide; "iPrNH₂" means isopropylamine; "iPrOH" means isopropyl alcohol; "n-PrNH₂" means propylamine; "sat." means saturated; "DMA" means dimethyl acetamide; "NMR" means Nuclear Magnetic Resonance; "Chloroform-d" means deuterated chloroform; "DIPEA" means di-isopropyl ethyl amine; "TLC" means Thin Layer Chromatography; "brsm" means based on recovered starting material; "w/w" means weight by weight; "ppm" means parts per million; "TEA" means triethylamine; "PE" means petroleum ether; "DMP" means Dess-Martin periodinane; "DIAD" means diisopropyl azodicarboxylate; "methanol-d4" means deuterated methanol; "TBDPS" means tert-butyl diphenyl silyl; "HMDS" means hexamethyl disilazane; "DMAP" means 4-dimethylamino pyridine; "ESI" means electronspray ionization; "ACN" means acetonitrile; "TBAI" means tetrabutylammonium iodide; "HATU" means N-[(Dimethylamino)-1H-1,2,3-triazolo-[4,5-b]pyridin-1-ylmethylene]-N-methylmethanaminium hexafluorophosphate N-oxide; "brsm" means based on recovered starting material; "HMPA" means hexamethylphosphoramide; "m-CPBA" or "mCPBA" means meta-chloroperoxybenzoic acid; "nBuLi" means n-butyllithium; "EDA" means ethyl diazoacetate; "tBu" means tert-butyl; "DIBAL" or "DIBAl-H" means diisobutylaluminum hydride; "DCE" means 1,2-dichloroethene; "MAO" means methylaluminoxane.

A notation as 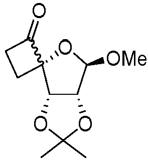 indicates a mixture of:

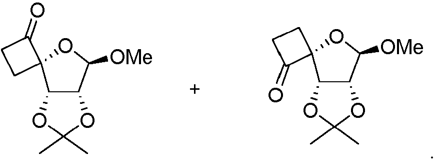

### Preparation of intermediates and nucleosides

### Preparation of cyclobutanes

### Preparation of compound 1

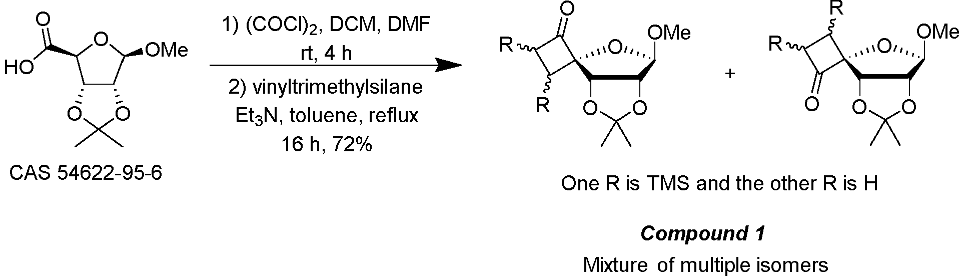

To a stirred solution of CAS 54622-95-6 (200 mg, 0.92 mmol, 1 eq) in DCM (2 mL) and one drop of DMF was added oxalyl chloride (0.12 mL, 1.37 mmol, 1.5 eq) and the resulting reaction mixture was then stirred at room temperature for 4 hours. Excess solvent and oxalyl chloride were removed *in vacuo* for 1 hour and proceeded to next step.

To the above obtained acid chloride in a sealed tube in toluene (2 mL) was added vinyltrimethylsilane (1.35 mL, 9.16 mmol, 10 eq) followed by Et₃N (0.15 mL, 1.10 mmol, 1.2 eq) and the resulting reaction mixture was heated at 120°C overnight (16 h) before quenching with HCl (1M, 10.00 mL). The aqueous phase was extracted with EtOAc (20 × 2 mL), and the combined organic layers were dried over Na₂SO₄ and concentrated under reduced pressure to provide the crude product. The residue was then purified by silica gel column chromatography (petroleum ether / EtOAc 90:10, Rf = 0.5), to give ***compound 1*** (198 mg, 0.66 mmol, 72%) as a colorless oil (which is a mixture of diastereomers as suggested by NMR spectroscopy).

### Data for the major isomer:

**¹H NMR (400 MHz, Chloroform-d)** *δ* 4.91 (s, 1H), 4.68 (d, *J* = 5.9 Hz, 1H), 4.57 (d, *J* = 5.9 Hz, 1H), 3.35 (s, 3H), 3.01 (dd, *J* = 17.5, 13.2 Hz, 1H), 2.56 (dd, *J* = 17.5, 6.6 Hz, 1H), 2.21 (dd, *J =* 13.2, 6.6 Hz, 1H), 1.46 (s, 4H), 1.32 (s, 3H), 0.08 ppm (s, 9H).

**¹³C NMR (101 MHz, Chloroform-*d*)** *δ* 206.40, 112.65, 106.92, 103.08, 84.66, 79.29, 54.68, 40.91, 26.26, 24.95, 18.64, -2.31 ppm.

GC-MS: [M - 86] 214, 13.67 min (Method 4) (No molecular ion peak found)

### Preparation of compound 2

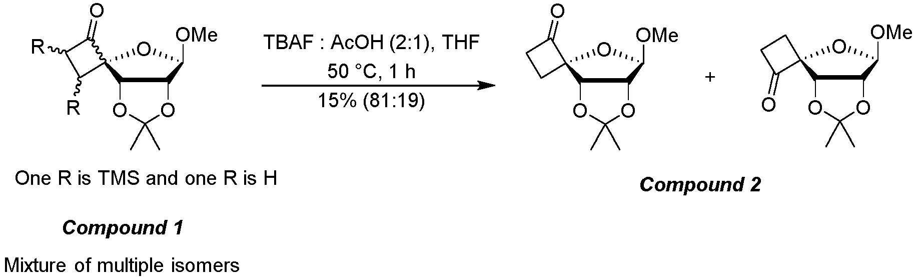

To a stirred solution of ***compound 1*** (250 mg, 0.83 mmol, 1 eq) in dry THF (4 mL) was added acetic acid (0.05 mL, 0.83 mmol, 1 eq) and TBAF (1.66 mL, 1.0 M in THF, 2 eq) dropwise. The resulting reaction mixture was heated at 50 °C for 1 hour. After completion of the starting material (TLC), water (20 mL) was added and extracted with EtOAc (30 mL). Combined organic layers were dried over Na2SO4 and concentrated under reduced pressure to provide the crude product. The residue was then purified by silica gel column chromatography (petroleum ether / EtOAc 90:10, R_{f} = 0.2) to afford ***compound 2*** (28 mg, 0.12 mmol, 15%) as a colorless oil (which is a mixture of two (81:19) diastereomers as suggested by NMR spectroscopy). This experiment was repeated 3 more times to provide (30 mg, 13%), (50 mg, 13%), (125 mg, 14%, 20 % (brsm)), all these were combined to get ***compound 2*** (232 mg).

### Data for major isomer:

**¹H NMR (300 MHz, Chloroform-*d*)** *δ* 5.01 (s, 1H), 4.87 (d, *J* = 5.7 Hz, 1H), 4.64 (d, *J* = 5.7 Hz, 1H), 3.35 (s, 3H), 3.03 - 2.96 (m, 1H), 2.83 (dd, *J* = 10.0, 4.2 Hz, 1H), 2.20 (td, *J* = 11.3, 4.2 Hz, 1H), 2.04 (td, *J* = 11.2, 10.1 Hz, 1H), 1.49 (s, 3H), 1.30 ppm (s, 3H).

**¹³C NMR (101 MHz, Chloroform-d)** *δ* 200.03, 113.88, 108.56, 99.60, 86.67, 84.74, 55.12, 40.90, 27.01, 26.07, 25.27 ppm.

GC-MS: [M+1] 229, 13.60 min (Method 4).

### Preparation of compound 3

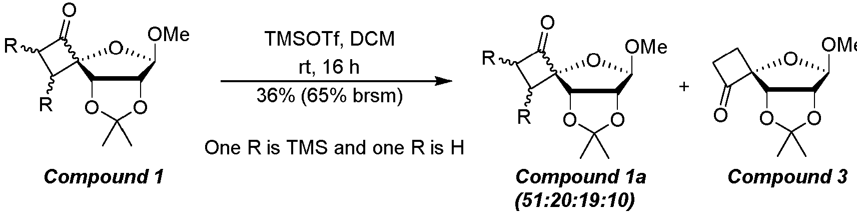

To a stirred solution of ***compound 1*** (113 mg, 0.38 mmol, 1 eq) (wavy bond in cyclobutane of compound 1 refers to mixture of multiple isomers as obtained before) in dry DCM (1.5 mL) at room temperature was added TMSOTf (0.06 mL, 0.34 mmol, 0.9 eq) and the resulting reaction mixture was stirred for 16 hours at same temperature. Sat. NaHCO₃ (5 ml) was added cautiously and extracted with EtOAc. Organic layer was dried over Na₂SO₄ and concentrated under reduced pressure to provide the crude product, consisting of starting material as well as product (TLC). The residue was then purified by silica gel column chromatography (petroleum ether / EtOAc 90:10, R_{f} 0.5) to afford ***compound 1a*** (40 mg, 0.13 mmol) as a colorless oil (which is a mixture of four (51:20:19:10) diastereomers as suggested by NMR spectroscopy) and ***compound 3*** (31 mg, 0.14 mmol, 36% (65% brsm)) as colorless crystals, as a single diastereomer as suggested by NMR spectroscopy.

**Data for major isomer of *compound 1a:* ¹H NMR (400 MHz, Chloroform-d)** δ 4.89 (s, 1H), 4.64 (d, *J =* 5.7 Hz, 1H), 4.60 (d, *J =* 5.8 Hz, 1H), 3.31 (s, 3H), 3.21 (dd, *J =* 17.5, 4.6 Hz, 1H), 2.70 (dd, *J =* 17.2, 10.1 Hz, 1H), 1.85 (dd, *J =* 12.9, 10.1 Hz, 1H), 1.47 (d, *J =* 0.7 Hz, 3H), 1.30 (d, *J =* 0.7 Hz, 3H), 0.12 ppm (s, 9H).

**Data for *compound 3:* ¹H NMR (400 MHz, Chloroform-*d*)** δ 5.01 (s, 1H), 4.87 (d, *J* = 5.7 Hz, 1H), 4.64 (d, *J =* 5.7 Hz, 1H), 3.35 (s, 3H), 3.07 - 2.92 (m, 1H), 2.81 (ddd, *J* = 17.1, 10.0, 4.0 Hz, 1H), 2.20 (td, *J =* 11.2, 4.0 Hz, 1H), 2.04 (dd, *J =* 11.5, 10.9 Hz, 1H), 1.49 (s, 3H), 1.30 ppm (s, 3H).

### Preparation of compounds 4 and 5

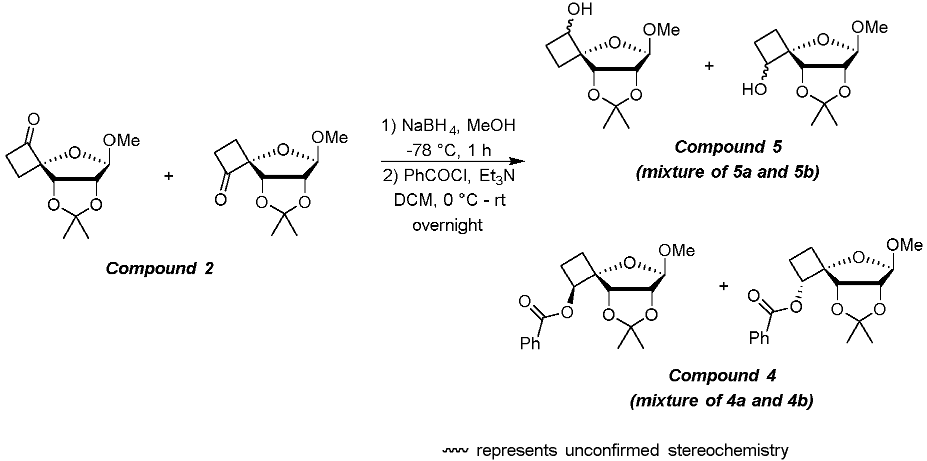

To ***compound 2*** (205 mg, 0.90 mmol, 1.00 eq) in MeOH (2 mL) was added NaBH₄ (44 mg, 1.17 mmol, 1.30 eq) at - 78 °C and the resulting reaction mixture was stirred for 1 hour at -78 °C. The reaction was then quenched by the addition of saturated NH₄Cl (10 mL) and extracted with ethyl acetate (30 mL). The organic layer was dried over anhydrous sodium sulfate, filtered, concentrated *in vacuo* and proceeded to next step.

To the above obtained alcohol in DCM (2 mL) was added Et₃N (0.26 mL, 1.80 mmol, 2 equiv), benzoyl chloride (0.16 mL, 1.35 mmol, 1.5 equiv) at 0 °C and the resulting reaction mixture was stirred at rt overnight. The reaction was then quenched by the addition of saturated NaHCO₃ (20 mL) and extracted with ethyl acetate (30 mL). The organic layer was dried over anhydrous sodium sulfate, filtered, concentrated under vacuum. The residue was then purified by silica gel column chromatography (petroleum ether / EtOAc 80:20, R_{f} = 0.6 and R_{f} = 0.4) to afford ***compound 4*** (110 mg, 0.33 mmol, 37%, as a mixture of two (62:38) diastereomers as suggested by NMR spectroscopy) as a colorless oil and ***compound 5*** (100 mg, 0.43 mmol, 48%, as a mixture of two (62:38) diastereomers as suggested by NMR spectroscopy).

A purification was performed on ***compound* 4** by Prep SFC (Stationary phase: Chiralcel Diacel OJ 20 x 250 m, Mobile phase: CO₂, iPrOH) to yield ***compound 4a*** (63.4 mg, 0.19 mmol, 58% yield, major isomer) and ***compound 4b*** (31 mg, 0.09 mmol, 28% yield, minor isomer).

### Data for compound 4a:

**LCMS: (ESI⁺):** [M+1]⁺ = 335.0, RT 2.18 min (Method 11)

### Data for compound 4b:

**LCMS: (ESI⁺):** [M+1]⁺ = 335.1, RT 2.18 min (Method 11)

**Data for major isomer *compound 4a:* ¹H NMR (300 MHz, Chloroform-*d*)** *δ* 8.13 - 8.05 (m, 2H), 7.58 - 7.49 (m, 1H), 7.47 - 7.36 (m, 2H), 5.60 (t, *J =* 8.7 Hz, 1H), 4.88 (s, 1H), 4.86 (d, *J* = 5.6 Hz, 1H), 4.61 (d, *J* = 5.6 Hz, 1H), 3.35 (s, 3H), 2.42 - 2.15 (m, 2H), 2.05 - 1.85 (m, 1H), 1.79 - 1.65 (m, 1H), 1.20 (d, *J* = 0.7 Hz, 3H), 1.16 ppm (d, *J =* 0.7 Hz, 3H).

**Data for major isomer of *compound 5:* ¹H NMR (300 MHz, Chloroform-d)** *δ* 4.94 (d, *J* = 5.8 Hz, 1H), 4.87 (s, 1H), 4.77 - 4.70 (m, 1H), 4.64 (d, *J* = 5.8 Hz, 1H), 4.40 (bs, 1H), 3.32 (s, 3H), 2.68 - 2.53 (m, 1H), 2.29 - 2.20 (m, 1H), 1.74 (ddd, *J =* 9.8, 8.4, 1.4 Hz, 1H), 1.62 (dd, *J* = 18.8, 8.7 Hz, 1H), 1.50 (d, *J = 0.7* Hz, 3H), 1.33 ppm (d, *J =* 0.7 Hz, 3H).

Alternative preparation of ***compound 1*** (larger scale)

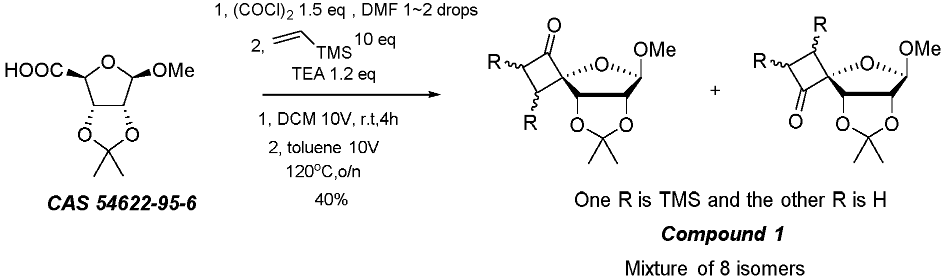

To a solution of CAS 54622-95-6 (64.00g, 293.58 mmol, 1 eq) in toluene (640 mL), one drop of DMF was added, followed by oxalyl chloride (55.92 g, 440.37 mmol, 1.5 eq) and the resulting reaction mixture was then stirred at room temperature for 1 h. Excess of solvent and oxalyl chloride were removed using vacuum pump for 1 h. To the above obtained acid chloride in a sealed tube in toluene (600 mL) was added vinyltrimethylsilane (293.61 g, 2935.80 mmol, 10 eq) followed by Et3N (35.58 g, 352.296 mmol, 1.2 eq) and the resulting reaction mixture was heated at 120 °C overnight (16 h) before quenching with HCl (1M aq, 800.00 mL). The aqueous phase was extracted with EtOAc (2 × 600 mL), and the combined organic layers were dried over Na2SO4 and concentrated under reduced pressure to provide the crude product. The residue was then purified by silica gel column chromatography (petroleum ether / EtOAc 90:10, Rf = 0.5), to afford ***compound 1.***

**26%** (23 g, 76.33mmol, crude) yellow oil. **LCMS (ESI+) m/z:** calcd. for C₁₄H₂₄O₅Si [M+H₂O] + = 318.14, found 318.20, RT: 1.678 min, Method 1.

Alternative preparation of ***compound 5*** (larger scale)

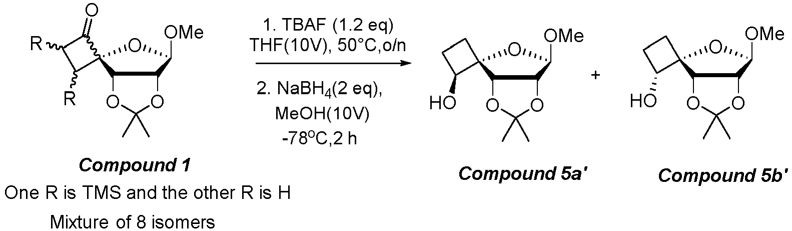

To ***compound 1*** (23 g, 76.33 mmol, 1.00 eq) in dry THF (230 mL) was added acetic acid (4.57 g, 76.33 mmol, 1.00 eq) and TBAF (152.66 mL, 1.0 M in THF, 2.00 eq) dropwise. The resulting reaction mixture was heated at 50 °C for overnight. After completion of the starting material (TLC), MeOH (200 mL) was added and NaBH₄ (2.9 g, 76.33mmol, 2.00 eq) was added at 0°C. The resulting reaction mixture was stirred for 2h at 0°C. The reaction was then quenched by the addition of saturated NH₄Cl (200 mL) and extracted with ethyl acetate (300 mL). The organic layer was dried over anhydrous sodium sulfate, filtered, and concentrated under vacuum. The residue was then purified by silica gel column chromatography (petroleum ether / EtOAc 90:10, R_{f} = 0.2), the front peak was ***compound 5a'**,* the back peak was ***compound 5b'*** (obtained as a mixture of ***compound 5b'*** and ***compounds 95a*** and ***95b*** described below).

**3.3% yield** (580 mg, 2.52 mmol, crude) yellow oil of ***compound 5a'**.* **GCMS (ESI+) m/z:** calcd. for C₁₁H₁₈O₅ [M-Me]- = 215.09, found 215.09, RT: 6.387 min, Method 1.

**2.5% yield** (30%, 1.5 g, 6.52 mmol, crude) yellow oil of ***compound 5b'*** (obtained as a mixture of ***compound 5b'*** and ***compounds 95a*** and ***95b*** described below). **GCMS (ESI+) m/z**: calcd. for C₁₁H₁₈O₅ [M-Me]- = 215.09, found 215.09, RT: 6.377 min, Method 1.

### Preparation of compound 4a'

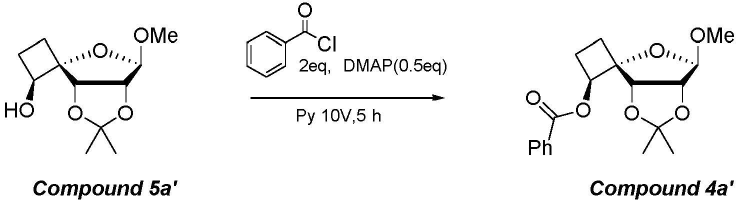

***Compound 5a'*** (1.03 g, 4.47 mmol, 1.00 eq), DMAP (272.67 mg, 2.23 mmol, 0.50 eq) was dissolved in pyridine (10 mL), then benzoyl chloride (1.25 g, 8.94 mmol, 2.00 eq) was added dropwise at 0°C and the resulting solution was stirred for 1 hour at 0°C. To the reaction was then added HCl (1M, aqueous, 20mL) until pH<7 was reached. The product was extracted with EtOAc (3 x 20 mL) and combined organic layers were dried (Na₂SO₄), filtered and the filtrate was concentrated *in vacuo.* The residue was purified by C18 Column (Mobile Phase A: Water (0.1mmol/L NH4HCO3), Mobile Phase B: ACN; Flow rate: 80 mL/min; Gradient: 40B to 70 B in 30 min; 210/254 nm). The fractions containing the product were collected and the solvent was evaporated to afford ***compound 4a'.***

**29% yield** (430 mg, 1.28 mmol), colorless oil. **¹H NMR (300 MHz, Chloroform-*d*)** δ 8.12 - 8.05(m, 2H), 7.57 - 7.49 (m, 1H), 7.46 - 7.36 (m, 2H), 5.61 (t, J = 8.9 Hz, 1H), 4.93 - 4.83 (m, 2H), 4.66 - 4.58 (m, 1H), 3.36 (s, 3H), 2.45 - 2.30 (m, 1H), 2.11 - 1.85 (m, 2H), 1.80 - 1.66 (m, 1H), 1.21 (s, 3H), 1.16 ppm (s, 3H). **¹³C NMR (75 MHz, CDCl₃)** δ 165.92, 132.62, 130.56, 129.90, 128.06, 112.68, 108.27, 87.81, 85.43, 83.93, 70.89, 55.04,27.78, 26.04, 25.09, 21.67 ppm. **LCMS (ESI+)** m/z: calcd. for C₁₈H₂₂O₆ [M+H]+ = 335.14, found 335.14, RT: 1.811 min, Method 1.

### Preparation of compound 138a

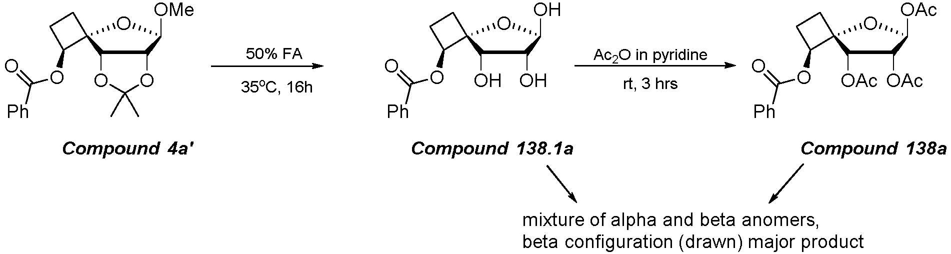

***Compound 4a'*** (430 mg, 1.28 mmol, 1.00eq) was dissolved in H₂O (5 mL) and formic acid (5 mL) was added. The mixture was heated to 35°C for 16 hours. Subsequently, the solution was cooled to room temperature, Subsequently, the solution was cooled to room temperature and concentrated in vacuo. This resulted in 440 mg of crude ***compound 138.1a.***

***Compound 138.1*** (440 mg, crude; mixture of alpha and beta anomers, beta anomer being major product) was dissolved in dry pyridine (5 mL) and stirred for 30 minutes. Acetic anhydride (195.84 mg, 1.92 mmol, 1.50 eq) was added at 0°C, was added to the stirring solution at room temperature. The mixture was stirred at room temperature for 3 hours. Subsequently, the mixture was poured into ice-cold water (10 ml) and stirred for 30 minutes at room temperature. The crude mixture was extracted with CH₂Cl₂ (3x 10 ml) and combined organic layers were washed with brine (3x 10 ml), dried (Na₂SO₄), filtered and the filtrate was concentrated in vacuo. The residue was purified by silica gel chromatography (gradient elution: PE / EA from 100:1 to 10:1). Fractions containing the product were combined and the solvent was removed in vacuo to afford ***compound 138a.***

**80% yield for 2 steps** (420 mg, 1.03mmol, crude; mixture of alpha and beta anomers, beta anomer being major product), colorless oil. **LCMS (ESI+) m/z:** calcd. for C₂₀H₂₂O₉ [M+H₂O]+ = 424.13, found 424.13, RT: 1.523 min, Method 1.

### Preparation of compound 139a

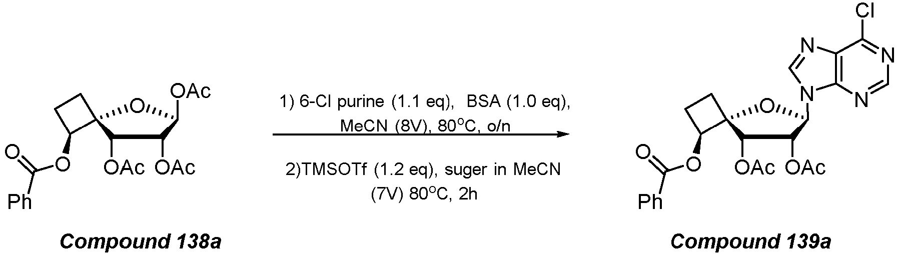

6-Chloropurine (121.79 mg,0.81 mmol, 1.10 eq) was dissolved in MeCN (25 mL), and N,O-bis(trimethylsilyl)acetamide (150.22 mg, 0.74 mmol, 1.00 eq) was added dropwise. The mixture was heated to 80°C for 16 hours. After the mixture was cooled to room temperature, ***compound 138a*** (300 mg, 0.74 mmol, 1.00 eq) in MeCN (2.2 mL) was added, followed by trimethylsilyltrifluoromethanesulfonate (194.94 mg, 0.88 mmol, 1.20 eq) and the mixture was heated to 80°C for 2 hours. The mixture was cooled to room temperature and diluted with EtOAc (20 mL), washed with saturated NaHCO₃ (3x20 mL) and saturated aq. NaCl (3 x 20 mL). The organic layer was dried (Na₂SO₄), filtered and the filtrate was concentrated *in vacuo.* The residue was purified by silica gel column chromatography with PE/EtOAc (gradient elution: PE/EtOAc from 99:1 to 4:1). The fractions containing the product were collected and the solvent was removed *in vacuo* to afford ***compound 139a.***

**59% yield** (220 mg, 0.44mmol, crude), colorless oil. **LCMS (ESI+) m/z:** calcd. for C₂₃H₂₁ClN₄O₇ [M+H]+ = 501.11, found 501.11, RT: 1.534 min, Method 1.

### Preparation of compound 140a

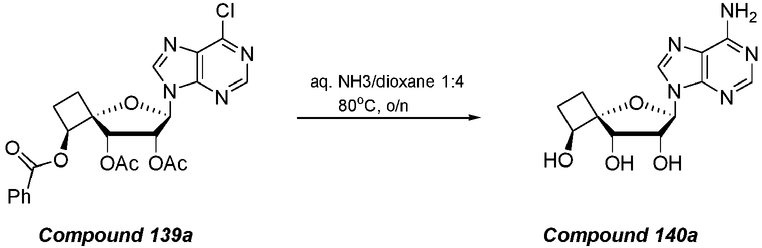

***Compound 139a*** (280 mg, 0.56 mmol, 1.00 eq) was dissolved in 1,4-dioxane (2.4 mL), and NH₃ (aq. 0.6 mL) was added. The mixture was heated to 80°C for 16 hours. After cooling the mixture to room temperature, solvents were removed *in vacuo* and the residue was purified via silica gel column chromatography with dichloromethane/ methanol (gradient elution: DCM/MeOH from 99:1 to 5:1). The fractions containing the product were collected and the solvent was removed in vacuo to afford ***compound 140a.***

**44% yield** (75 mg, 0.25 mmol), off-white solid. **¹H NMR (300 MHz, MeOD)** δ 8.21 (d, J = 6.9 Hz, 2H), 6.16 (d, J = 6.8 Hz, 1H), 5.08 - 5.01(m, 1H), 4.61 (d, J = 3.9 Hz, 1H), 4.40 - 4.31 (m, 1H), 2.17 - 2.00 (m, 2H), 1.78 - 1.59 ppm (m, 2H). **¹³C NMR** (75 **MHz, MeOD)** δ 155.90, 152.43, 149.60, 140.12, 119.31, 88.43, 87.24, 76.52, 73.87, 25.86, 22.51 ppm. **LCMS (ESI+)** m/z: calcd. for C₁₂H₁₅N₅O₄ [M+H]+ = 294.11, found 294.11, RT: 0.446 min, Method 1.

### Preparation of compound 4b'

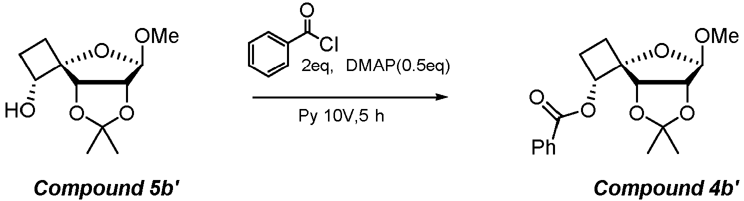

***Compound 5b'*** (2.6 g, used as the mixture obtained in the "Alternative preparation of ***compound 5"*** procedure above, containing 30% of pure ***compound 5b',*** 800 mg, 3.47 mmol, 1.00 eq), DMAP (211.67 mg, 1.73 mmol, 0.50 eq) was dissolved in pyridine (10 mL), then benzoyl chloride (971.60 mg, 6.94 mmol, 2.00 eq) was added dropwise at 0°C and the resulting solution was stirred for 1 hour at 0°C. To the reaction was then added HCl (1M, aqueous, 20mL) until pH<7 was reached. The product was extracted with EtOAc (3 x 20 mL) and combined organic layers were dried (Na₂SO₄), filtered and the filtrate was concentrated *in*

*vacuo.* The residue was purified by C18 Column (Mobile Phase A: Water (0.1mmol/L NH4HCO3), Mobile Phase B: ACN; Flow rate: 80 mL/min; Gradient: 40B to 70 B in 30 min; 210/254 nm). The fractions containing the product were collected and the solvent was evaporated to afford ***compound 4b'.***

**34% yield** (400 mg, 1.19 mmol), colorless oil. **¹H NMR (300 MHz, Chloroform-*d*)** δ 8.15 - 8.06 (m, 2H), 7.59 - 7.51 (m, 1H), 7.48 - 7.39 (m, 2H), 5.64 - 5.52 (m, 1H), 5.04 (s, 1H), 4.63 (d, J = 0.8 Hz, 2H), 3.42 (s, 3H), 2.44 - 2.17 (m, 3H), 2.05 - 1.88 (m, 1H), 1.35 (s, 3H), 1.30 ppm (s, 3H). **¹³C NMR (75 MHz, CDCl₃)** δ 165.77, 132.84, 130.41, 129.81, 128.28, 112.84, 108.18, 89.65, 85.06, 84.26, 70.78, 54.95, 30.64, 26.22, 25.75, 25.25 ppm. **LCMS (ESI+) m/z:** calcd. for C₁₈H₂₂O₆ [M+H]+ = 335.14, found 335.14, RT: 1.675 min, Method 1.

### Preparation of compound 138b

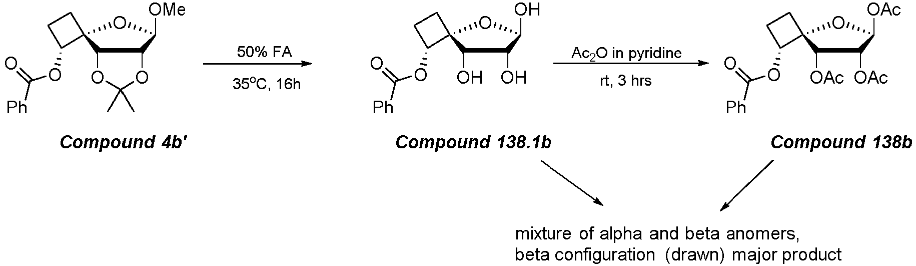

***Compound 4b'*** (400 mg, 1.19 mmol, 1.00eq) was dissolved in H₂O (5 mL) and formic acid (5 mL) was added. The mixture was heated to 35°C for 16 hours. Subsequently, the solution was cooled to room temperature and concentrated in vacuo. This result in 420 mg of crude ***compound 138.1b*** (mixture of alpha and beta anomers, beta anomer being major product). ***Compound 138.1b*** (420 mg, crude) was dissolved in dry pyridine (5 mL) and stirred for 30 minutes. Acetic anhydride (181.56 mg, 1.78 mmol, 1.50 eq) was added at 0°C, was added to the stirring solution at room temperature. The mixture was stirred at room temperature for 3 hours. Subsequently, the mixture was poured into ice-cold water (10 ml) and stirred for 30 minutes at room temperature. The crude mixture was extracted with CH₂Cl₂ (3x 10 ml) and combined organic layers were washed with brine (3x 10 ml), dried (Na₂SO₄), filtered and the filtrate was concentrated in vacuo. The residue was purified by silica gel chromatography (gradient elution: PE / EA from 100:1 to 10:1). Fractions containing the product were combined and the solvent was removed in vacuo to afford ***compound 138b.***

**74% yield for** 2 **steps** (361mg, 0.88mmol, crude; mixture of alpha and beta anomers, beta anomer being major product), colorless oil. **LCMS (ESI+) m/z:** calcd. for C₂₀H₂₂O₉ [M+H₂O] + = 424.13, found 424.13, RT: 1.545 min, Method 1.

### Preparation of compound 139b

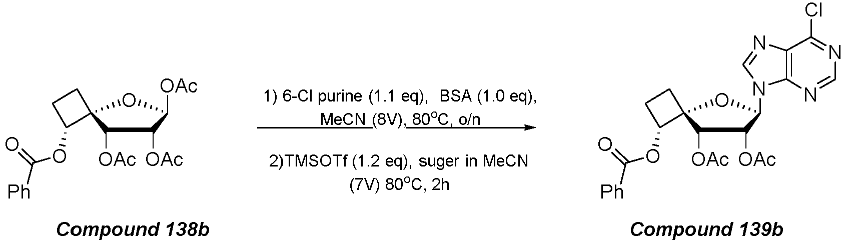

6-Chloropurine (100.10 mg, 0.65 mmol, 1.10 eq) was dissolved in MeCN (25 mL), and N,O-bis(trimethylsilyl)acetamide (131.95 mg, 0.65 mmol, 1.00 eq) was added dropwise. The mixture was heated to 80°C for 16 hours. After the mixture was cooled to room temperature, ***compound 138b*** (241 mg, 0.59 mmol, 1.00 eq) in MeCN (2.2 mL) was added, followed by trimethylsilyltrifluoromethanesulfonate (157.21 mg, 0.71 mmol, 1.20 eq) and the mixture was heated to 80°C for 2 hours. The mixture was cooled to room temperature and diluted with EtOAc (20 mL, washed with saturated NaHCO₃ (3x20 mL) and saturated aq. NaCl (3 x 20 mL. The organic layer was dried (Na₂SO₄), filtered and the filtrate was concentrated *in vacuo.* The residue was purified by silica gel column chromatography with PE/EtOAc (gradient elution: PE/EtOAc from 99:1 to 4:1). The fractions containing the product were collected and the solvent was removed *in vacuo* to afford ***compound 139b.***

**71% yield** (210 mg, 0.42mmol, crude), colorless oil. **LCMS (ESI+) m/z:** calcd. for C₂₃H₂₁ClN₄O₇ [M+H]+ = 501.11, found 501.11, RT: 1.597 min, Method 1.

### Preparation of compound 140b

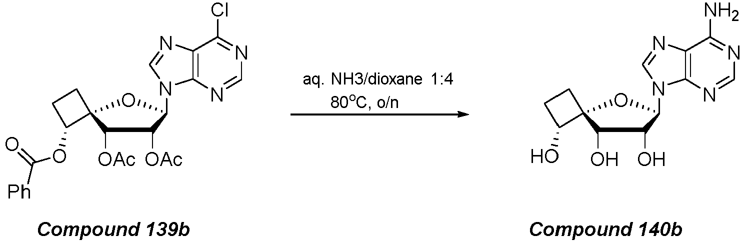

***Compound 139b*** (295 mg, 0.59 mmol, 1.00 eq) was dissolved in 1,4-dioxane (3.0 mL), and NH₃ (aq. 0.8 mL) was added. The mixture was heated to 80°C for 16 hours. After cooling the mixture to room temperature, solvents were removed *in vacuo* and the residue was purified via silica gel column chromatography with dichloromethane/ methanol (gradient elution: DCM/MeOH from 99:1 to 5:1). The fractions containing the product were collected and the solvent was removed in vacuo to afford ***compound 140b.***

**53% yield** (92 mg, 0.31 mmol), off-white solid. **¹H NMR (300 MHz, Methanol-d4)** δ 8.23 (d, J = 9.8 Hz, 2H), 6.07 (d, J = 5.2 Hz, 1H), 4.83 - 4.77 (m, 1H), 4.52 - 4.44 (m, 1H), 4.23 (d, J = 5.1 Hz, 1H), 2.21 - 2.08 (m, 2H), 2.04 - 1.92 ppm (m, 2H).**¹³C NMR (75 MHz, CDCl₃)** waiting for data. **¹³C NMR (75 MHz, MeOD)** δ 155.74, 152.18, 149.31, 140.06, 119.30, 90.77, 88.47, 73.75, 73.71, 67.29, 26.56, 26.01 ppm. **LCMS (ESI+)** m/z: calcd. for C₁₂H₁₅N₅O₄ [M+H]+ = 294.11, found 294.11, RT: 0.412 min, Method 1.

### Preparation of compounds 6a and 6b

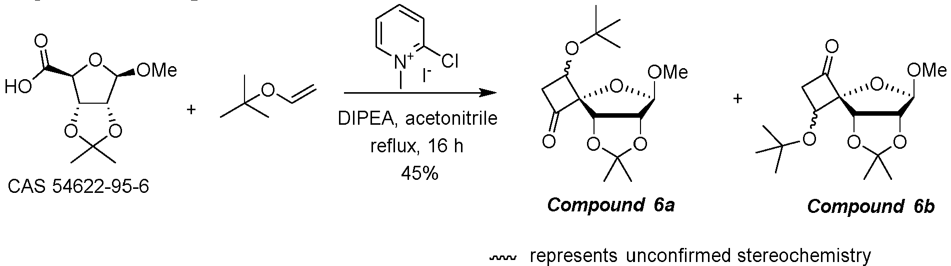

To a stirred solution of CAS 54622-95-6 (400 mg, 1.83 mmol, 1 eq) in acetonitrile (6 mL) was added 2-Chloro-1-methylpyridinium iodide (702 mg, 2.75 mmol, 1.5 eq), tert-Butyl vinyl ether (2.42 mL, 18.33 mmol, 10 eq) and finally DIPEA (0.96 mL, 5.49 mmol, 3 eq). The resulting reaction mixture was then refluxed for 16 hours. Reaction was then cooled to room temperature, quenched with HCl (20 mL, 1M), extracted with ethyl acetate, the combined organics were dried over sodium sulfate, filtered and concentrated to give crude product ***compound 6.*** The crude product consisting of 2 isomers, was then purified by silica gel column chromatography (petroleum ether / EtOAc 80:20 to provide two isomers ***compound 6a*** as a colorless oil and ***compound 6b*** as colorless crystals in a pure form).

***Compound 6a* (20% yield, 110 mg), R_{f} 0.6 (petroleum ether** / **EtOAc 80:20): ¹H NMR (400 MHz, Chloroform-*d*)** *δ* 5.48 (d, *J =* 5.8 Hz, 1H), 5.02 (s, 1H), 4.55 (d, *J =* 5.8 Hz, 1H), 4.11 (t, *J* = 7.9 Hz, 1H), 3.32 (s, 3H), 2.98 - 2.82 (m, 2H), 1.46 (s, 3H), 1.29 (s, 3H), 1.19 ppm (s, 9H). **¹³C NMR (101 MHz, Chloroform-*d*)** *δ* 200.48, 113.42, 109.05, 102.24, 84.87, 79.55, 74.50, 66.55, 55.17, 50.53, 27.99, 26.03, 25.30 ppm.

***Compound 6b* (25% yield, 140 mg), R_{f} 0.4 (petroleum ether** / **EtOAc 80:20): ¹H NMR (400 MHz, Chloroform-*d*)** *δ* 5.04 (s, 1H), 4.84 (d, *J* = 5.7 Hz, 1H), 4.60 (d, *J* = 5.8 Hz, 1H), 4.15 (dd, *J =* 6.1, 1.8 Hz, 1H), 3.43 (s, 3H), 3.22 (dd, *J =* 17.4, 6.1 Hz, 1H), 2.65 (dd, *J =* 17.3, 1.8 Hz, 1H), 1.48 (s, 3H), 1.29 (s, 3H), 1.22 ppm (s, 9H).

**¹³C NMR (101 MHz, Chloroform-*d*)** *δ* 199.49, 114.03, 109.00, 100.54, 86.17, 84.47, 75.33, 67.26, 56.19, 51.16, 28.37, 26.03, 25.48 ppm.

GC-MS: [M⁺] 301, 15.14 min (Method 4).

### Preparation of compound 7

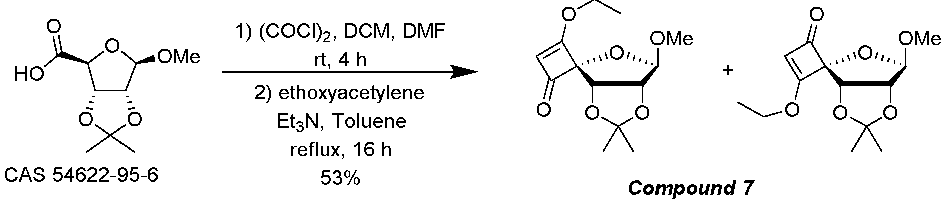

To a stirred solution of CAS 54622-95-6 (2.00 g, 9.16 mmol, 1 eq) in DCM (8 mL), one drop of DMF was added oxalyl chloride (1.18 mL, 13.75 mmol, 1.5 eq) and the resulting reaction mixture was then stirred at room temperature for 4 h. Excess solvent and oxalyl chloride were removed *in vacuo* for 1 hour and proceeded to next step.

To the above obtained carbonyl chloride in DCM (25 ml) was added ethoxyacetylene (18.33 mmol 40%w/w in hexanes, 2 eq). The stirred solution was then treated dropwise at rt with triethylamine (1.38 mL, 10.08 mmol, 1.1 eq). After 30 min the suspension was heated to reflux and stirred for a further 16 hours. The resulting turbid mixture was allowed to cool prior to removal of triethylammonium chloride by filtration and the filtrate was concentrated in vacuo to give a brown oil. The residue was then purified by silica gel column chromatography (petroleum ether / EtOAc 70:30), to give ***compound 7*** (1.32 g, 4.88 mmol, 53%) as a brown colored oil (which is a mixture of two (74:26) diastereomers as suggested by NMR spectroscopy).

**Data for major isomer: ¹H NMR (400 MHz, Chloroform-*d*)** *δ* 5.35 (d, *J* = 0.9 Hz, 1H), 5.02 (d, *J* = 1.2 Hz, 1H), 4.88 (dd, *J* = 5.8, 1.2 Hz, 1H), 4.61 (dt, *J* = 5.8, 0.8 Hz, 1H), 4.30 - 4.16 (m, 2H), 3.35 (s, 3H), 1.51 (s, 3H), 1.47 (td, *J =* 7.1, 1.1 Hz, 3H), 1.30 ppm (s, 3H).

**¹³C NMR (101 MHz, Chloroform-*d*)** *δ* 186.52, 182.08, 113.98, 113.40, 106.51, 101.02, 84.32, 79.97, 69.94, 54.84, 25.88, 24.91, 14.00 ppm.

### Preparation of compound 8

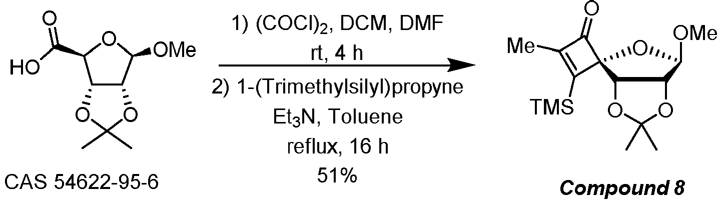

To a stirred solution of CAS 54622-95-6 (200 mg, 0.92 mmol, 1 eq) in DCM (2 mL), one drop of DMF was added oxalyl chloride (0.12 mL, 1.37 mmol, 1.5 eq) and the resulting reaction mixture was then stirred at room temperature for 4 hours. Excess solvent and oxalyl chloride were removed *in vacuo* for 1 hour and proceeded to next step.

To the above obtained acid chloride in a sealed tube in toluene (2 mL) was added 1-(Trimethylsilyl)propyne (1.03 mL, 9.16 mmol, 10 eq) followed by Et₃N (0.15 mL, 1.10 mmol, 1.2 eq) and the resulting reaction mixture was heated at 120°C overnight (16 hours) before quenching with HCl (1M, 10.00 mL). The aqueous phase was extracted with EtOAc (20 × 2 mL), and the combined organic layers were dried over Na₂SO₄ and concentrated under reduced pressure to provide the crude product. The residue was then purified by silica gel column chromatography (petroleum ether / EtOAc 90:10), to give ***compound 8*** (146 mg, 0.47 mmol, 51%) as a colorless oil (which is a mixture of two (82:18) diastereomers as suggested by NMR spectroscopy).

**Data for major isomer: ¹H NMR (400 MHz, Chloroform-d)** *δ* 4.89 (s, 1H), 4.62 (d, *J* = 6.0 Hz, 1H), 4.59 (d, *J* = 6.0 Hz, 1H), 3.28 (s, 3H), 1.87 (s, 3H), 1.50 (s, 3H), 1.26 (s, 3H), 0.28 ppm (s, 9H).

**¹³C NMR (101 MHz, Chloroform-d)** *δ* 190.85, 179.55, 164.97, 112.15, 107.42, 107.24, 85.15, 80.81, 54.60, 25.55, 23.60, 10.45, -1.09 ppm.

**LCMS** : **(ESI⁺):** [M+1]⁺ = 313.3, RT 1.23 min (Method 10)

### Preparation of compound 9

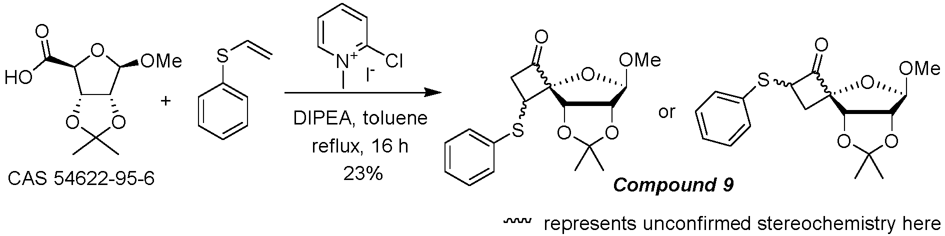

To a stirred solution of CAS 54622-95-6 (400 mg, 1.83 mmol, 1 eq) in toluene (6 mL) was added 2-Chloro-1-methylpyridinium iodide (702 mg, 2.75 mmol, 1.5 eq), Phenyl vinyl sulfide (0.60 mL, 4.58 mmol, 2.5 eq) and DIPEA (0.96 mL, 5.49 mmol, 3 eq). The resulting reaction mixture was then refluxed for 16 hours. Reaction was then cooled to room temperature, quenched with HCl (20 mL, 1M), extracted with ethyl acetate, the combined organics were dried over sodium sulfate, filtered and concentrated. The crude product was then subjected to silica gel chromatography (petroleum ether / EtOAc 90:10) to afford ***compound 9*** (140 mg, 0.42 mmol, 23%) as a light yellow color oil (which is a mixture of two (82:18) diastereomers as suggested by NMR spectroscopy).

**Data for major isomer: ¹H NMR (400 MHz, Chloroform-*d*)** *δ* 7.32 (s, 5H), 5.20 - 5.12 (m, 2H), 5.06 (s, 1H), 4.44 (d, *J* = 6.1 Hz, 1H), 3.39 (d, *J* = 8.5 Hz, 1H), 3.36 (d, *J* = 4.8 Hz, 1H), 3.33 (s, 3H), 1.39 ppm (s, 3H), 1.30 (s, 3H).

**¹³C NMR (101 MHz, Chloroform-*d*)** *δ* 195.94, 158.20, 134.45, 129.25, 129.08, 127.89, 113.36, 107.43, 95.72, 82.32, 77.32, 77.00, 76.68, 76.42, 55.57, 40.93, 26.76, 25.90 ppm.

### Preparation of compound 10

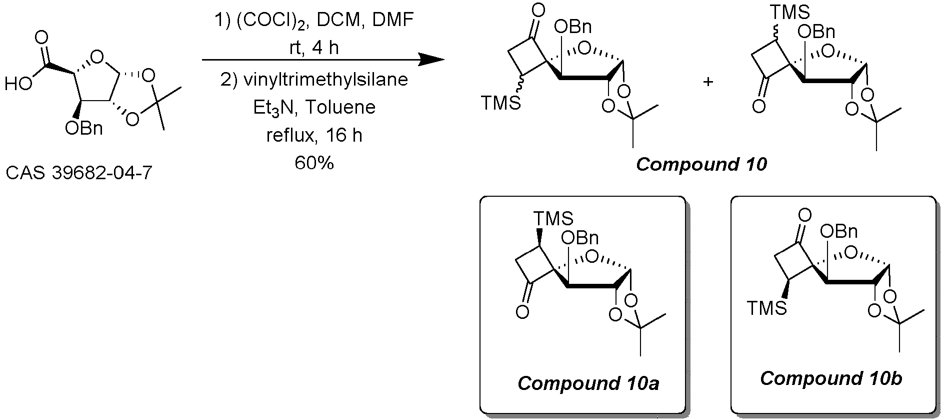

To a stirred solution of CAS 39682-04-7 (3.10 g, 10.53 mmol, 1 eq) in DCM (15 mL), one drop of DMF was added oxalyl chloride (1.35 mL, 15.80 mmol, 1.5 eq) and the resulting reaction mixture was then stirred at room temperature for 4 hours. Excess solvent and oxalyl chloride were removed *in vacuo* for 1 hour and proceeded to next step.

To a stirred solution of Et₃N (2.28 mL, 15.80 mmol, 1.5 eq) and vinyltrimethylsilane (12.42 mL, 84.29 mmol, 8 eq) in toluene (20 mL) at 80 °C was added above obtained acid chloride in toluene (10 mL) using syringe pump over 2 hours. The reaction mixture was then refluxed at 120 °C overnight (16 hours) before quenching with HCl (1M, 40.00 mL). The aqueous phase was extracted with EtOAc (40 × 2 mL), and the combined organic layers were dried over Na₂SO₄ and concentrated under reduced pressure to provide the crude product. The residue was then purified by silica gel column chromatography (petroleum ether / EtOAc 90:10), to give ***compound 10*** (2.40 g, 6.37 mmol, 60%) as a colorless oil (which is amixture of four (56:24:11:9) diastereomers as suggested by NMR spectroscopy).

A purification was performed on ***compound 10*** via Prep HPLC (Stationary phase: RP XBridge Prep C18 OBD-10µm,50×150mm, Mobile phase: 0.25% NH₄HCO₃ solution in water, CH₃CN) to yield two fractions of ***compound 10a*** (11.5 mg, 0.03 mmol, 5% yield) (50.6 mg, 0.13 mmol, 20% yield) and ***compound 10b*** (12 mg, 0.03 mmol, 5% yield).

### Compound 10a

**¹H NMR (400 MHz, Chloroform-*d*)** : *δ* 7.31-7.49 (m, 5H), 5.89 (d, *J =* 4.0 Hz, 1H), 4.59-4.72 (m, 3H), 4.12 (s, 1H), 3.02 (dd, *J* = 16.3, 11.9 Hz, 1H), 2.65 (dd, *J* = 16.1, 12.1 Hz, 1H), 1.69 (s, 3H), 1.61-1.67 (m, 1H), 1.30 (s, 3H), 0.00 ppm (s, 9H).

**¹³C NMR (101 MHz, Chloroform-*d*:** δ 202.8, 137.8, 129.7, 129.4, 129.3, 114.2, 105.9, 104.9, 84.2, 82.9, 73.1, 44.6, 27.4, 26.9, 25.0, 0.0 ppm.

**LCMS: (ESI⁺):** [M+1]⁺ = 377.4, RT 1.31 min (Method 10)

### Compound 10b

**¹H NMR (600 MHz, Chloroform-*d*):** δ 7.33-7.37 (m, 2H), 7.29-7.33 (m, 1H), 7.26-7.29 (m, 2H), 6.12 (d, *J = 4.3* Hz, 1H), 4.75 (dd, *J =* 4.3, 2.1 Hz, 1H), 4.73 (d, *J =* 11.4 Hz, 1H), 4.57 (d, *J =* 11.4 Hz, 1H), 4.31 (d, *J =* 2.0 Hz, 1H), 2.79 (d, *J =* 12.2 Hz, 2H), 1.84 (t, *J =* 12.2 Hz, 1H), 1.57 (s, 3H), 1.43 (s, 3H), 0.14 ppm (s, 9H).

**¹³C NMR (151 MHz, Chloroform-*d*:** δ 205.7, 136.6, 128.3, 127.7, 127.0, 115.3, 103.8, 100.3, 85.2, 85.1, 71.8, 43.1, 28.2, 27.4, 22.8, -2.0 ppm.

**LCMS: (ESI⁺):** [M+18]⁺ = 394.3, RT 1.35 min (Method 10)

### Preparation of Compound 11

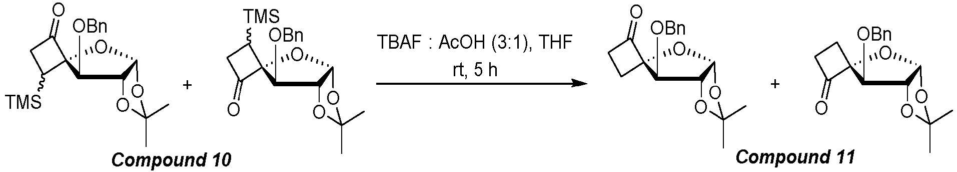

To a stirred solution of ***compound 10*** (1.60 g, 4.25 mmol, 1 eq) in dry THF (5 mL) was added acetic acid (0.24 mL, 4.25 mmol, 1 eq) and TBAF (12.75 mL, 1.0 M in THF, 3 eq) dropwise. The resulting reaction mixture was stirred at rt for 5 hours, quenched by the addition of water (20 mL) and extracted with EtOAc (40 mL). Combined organic layers were dried over Na₂SO₄ and concentrated under reduced pressure to provide the crude product. The residue was then purified by silica gel column chromatography (petroleum ether / EtOAc 80:20, R_{f} = 0.5) to afford ***compound 11*** (185 mg, 0.61 mmol, 14% (28% brsm)) as a colorless oil (which is a mixture of two (65:35) diastereomers as suggested by NMR spectroscopy). This experiment was repeated one more time to provide 180 mg, 14% and these two products were combined to get ***compound 11*** (365 mg).

**Data for major isomer of *compound 11:* ¹H NMR (Chloroform-d, 600 MHz):** δ = 7.34-7.38 (m, 2H), 7.31-7.33 (m, 1H), 7.30 (br d, J = 6.9 Hz, 2H), 6.05 (d, J = 3.6 Hz, 1H), 4.69 (d, J = 12.2 Hz, 1H), 4.66 (d, J = 3.5 Hz, 1H), 4.49 (d, J = 12.2 Hz, 1H), 4.20 (s, 1H), 2.64-2.77 (m, 2H), 2.36 (td, J = 11.3, 4.9 Hz, 1H), 2.14-2.22 (m, 1H), 1.50 (s, 3H), 1.34 ppm (s, 3H). **¹³C NMR (Chloroform-d, 151 MHz):** δ = 203.1, 136.8, 128.6, 128.2, 128.0, 113.4, 106.6, 99.7, 86.6, 83.6, 72.0, 40.7, 27.1, 26.5, 26.0 ppm.

### Preparation of Compound 12

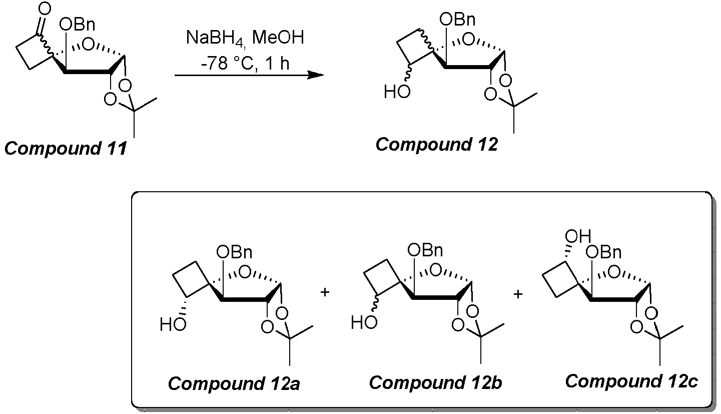

To ***compound 11*** (350 mg, 1.15 mmol, 1.00 eq) (wavy bond in cyclobutane of compound 11 refers to mixture of multiple isomers as obtained before) in MeOH (4 mL) was added NaBH₄ (57 mg, 1.50 mmol, 1.30 eq) at -78°C and the resulting reaction mixture was stirred for 1 hour at -78°C. The reaction was then quenched by the addition of saturated NH₄Cl (10 mL) and extracted with ethyl acetate (30 mL). The organic layer was dried over anhydrous sodium sulfate, filtered, concentrated *in vacuoto* give crude ***compound 12.*** The crude product consisting of 3 isomers (TLC), was then purified by silica gel column chromatography (petroleum ether / EtOAc 90:10 to 80:20) to provide three isomers ***compound 12a*** (Rf 0.4, 165 mg, 47% as a mixture of two diastereomers (91:9 as suggested by NMR spectroscopy)) as a colorless oil, ***compound 12b*** (Rf 0.3, 80 mg, 23% as mixture of 3 diastereomers (68:26:6) as suggested by NMR spectroscopy) as colorless oil and ***compound 12c*** (Rf 0.2, 80 mg, 23% as a single pure isomer as suggested by NMR spectroscopy) as a colorless oil.

A purification on ***compound 12a*** was performed via Prep HPLC (Stationary phase: RP XBridge Prep C18 OBD-10µm,50×150mm, Mobile phase: 0.25% NH₄HCO₃ solution in water, MeOH) to yield 86 mg, 0.28 mmol, 53% yield.

### Data for compound 12a

**¹H NMR (300 MHz, Chloroform-*d*)** *δ* 7.39 - 7.25 (m, 5H), 5.92 (d, *J =* 4.2 Hz, 1H), 4.74 (d, *J =* 12.0 Hz, 1H), 4.64 (d, *J = 4.2* Hz, 1H), 4.52 (d, *J =* 12.1 Hz, 1H), 4.15 - 4.05 (m, 1H), 3.84 (s, 1H), 2.92 (d, *J =* 3.4 Hz, 1H), 2.29 (t, *J = 8.7* Hz, 2H), 1.89 - 1.68 (m, 2H), 1.48 (d, *J = 0.7* Hz, 3H), 1.28 ppm (d, *J* = 0.7 Hz, 3H).

**LCMS: (ESI⁺):** [M+18]⁺ = 324.3, RT 1.01 min (Method 10)

**Data for major isomer of *compound 12b*: ¹H NMR (300 MHz, Chloroform-*d*)** *δ* 7.52 - 7.35 (m, 5H), 5.93 (d, *J = 3.4* Hz, 1H), 4.88 - 4.81 (m, 1H), 4.71 (d, *J = 3.8* Hz, 1H), 4.48 (s, 1H), 4.44 - 4.37 (m, 1H), 4.33 (dd, *J* = 8.9, 7.8 Hz, 1H), 3.04 (d, *J* = 1.1 Hz, 1H), 2.36 (dddd, *J* = 12.3, 10.0, 2.5, 1.2 Hz, 1H), 2.14 (dddd, *J* = 11.0, 9.8, 8.3, 2.5 Hz, 1H), 1.90 - 1.74 (m, 1H), 1.65 (s, 3H), 1.42 (d, *J = 0.7* Hz, 3H), 1.36 - 1.30 ppm (m, 1H).

### Data for compound 12c

**¹H NMR (300 MHz, Chloroform-*d*)** δ 7.36 - 7.20 (m, 5H), 5.91 (d, *J= 3.9* Hz, 1H), 4.69 (d, *J* = 12.1 Hz, 1H), 4.56 (dd, *J =* 4.0, 0.8 Hz, 1H), 4.46 (d, *J* = 12.1 Hz, 1H), 4.35 - 4.24 (m, 1H), 3.77 (t, *J =* 0.6 Hz, 1H), 2.50 (d, *J =* 8.7 Hz, 1H), 2.15 - 2.00 (m, 2H), 2.00 - 1.84 (m, 1H), 1.81 - 1.68 (m, 1H), 1.45 - 1.37 (m, 3H), 1.25 ppm (d, *J =* 0.7 Hz, 3H).

**LCMS: (ESI⁺):** [M+18]⁺ = 324.4, RT 0.94 min (Method 10)

### Preparation of Compound 13

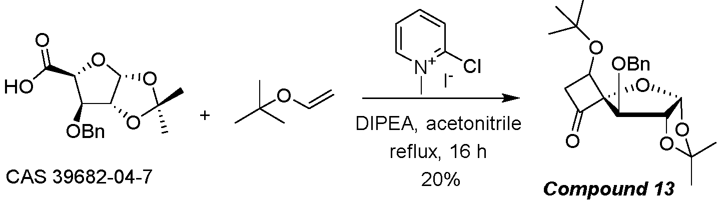

To a stirred solution of CAS 39682-04-7 (400 mg, 1.36 mmol, 1 eq) in acetonitrile (6 mL) was added 2-Chloro-1-methylpyridinium iodide (521 mg, 2.04 mmol, 1.5 eq), *tert*-butyl vinyl ether (1.79 mL, 13.59 mmol, 10 eq) and finally DIPEA (0.71 mL, 4.08 mmol, 3 eq). The resulting reaction mixture was then refluxed for 16 hours. Reaction was then cooled to room temperature, quenched with HCl (20 mL, 1M), extracted with ethyl acetate, the combined organics were dried over sodium sulfate, filtered and concentrated. The crude product was then subjected to silica gel chromatography (petroleum ether / EtOAc 90:10) to afford ***compound 13*** (100 mg, 0.27 mmol, 20%, as a single diastereomer but unknown diastereomer) as a colorless oil.

**¹H NMR (400 MHz, Chloroform-*d*)** *δ* 7.39 - 7.28 (m, 5H), 6.07 (d, *J* = 4.3 Hz, 1H), 4.77 (d, *J =* 12.0 Hz, 1H), 4.73 (dd, *J =* 4.4, 2.1 Hz, 1H), 4.68 (d, *J = 2.2* Hz, 1H), 4.60 (d, *J* = 12.0 Hz, 1H), 4.32 (t, *J* = 8.3 Hz, 1H), 2.87 - 2.74 (m, 2H), 1.49 (s, 3H), 1.39 (s, 3H), 1.18 ppm (s, 9H). **¹³C NMR (101 MHz, Chloroform-*d*)** *δ* 205.64, 137.11, 128.34, 127.78, 127.76, 114.63, 105.73, 101.55, 85.76, 81.73, 74.70, 72.33, 63.74, 50.16, 28.21, 27.86, 27.44 ppm

### Preparation of Compound 14

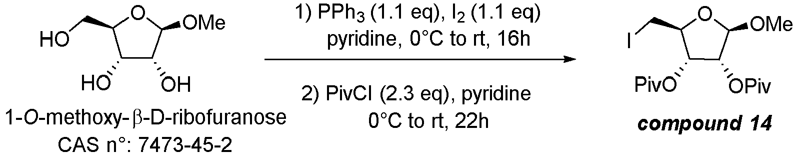

1-*O*-methoxy-β-D-ribofuranose (25.3 g, 154 mmol, 1.00 eq) was dissolved in pyridine (253 ml) and triphenylphosphine (44.4 g, 169 mmol, 1.10 eq) was added portionwise. The solution was cooled to 0°C and iodine (42.9 g, 169 mmol, 1.10 eq) was added portionwise over a period of 40 minutes. The solution was cooled at 0°C for an additional 20 minutes after addition of the reagents before it was stirred at room temperature for 24 hours. Subsequently, the reaction mixture was cooled to 0°C and pivaloyl chloride (43.5 ml, 354 mmol, 2.30 eq) was added dropwise via a pressure equalized dropping funnel over a period of 1.5 hours to the stirring mixture. After addition of the reagent, the mixture was warmed to room temperature and stirred for 22 hours. The mixture was concentrated to a minimal volume *in vacuo* and coevaporated with toluene (2x 300 ml). To the remaining brown slurry was added n-heptane (1L) upon which triphenylphosphine-oxides precipitated. The mixture was sonicated for 1 hour and the solid material was filtrated and washed with n-heptane (300 ml). The filtrate was concentrated *in vacuo* to a minimal volume to yield a yellow syrup. Subsequently, the syrup was redissolved in EtOAc (500 ml) and washed with a solution of sodiumthiosulfate (aq. sat. 1× 250 ml) and brine (1× 250 ml). The organic phase was dried (MgSO₄), filtrated and the filtrate was concentrated *in vacuo* to yield ***compound 14*** (49.0 g, 72% crude yield) which solidified upon standing at room temperature.
**¹H NMR (400 MHz, Chloroform-*d*** δ 5.27 (d, *J*=4.8 Hz, 1H), 5.20 (dd, *J*=6.3, 4.8 Hz, 1H), 4.85 (s, 1H), 4.24 (q, *J*=6.6 Hz, 1H), 3.41 (s, 3H), 3.33 (dd, *J*=6.5, 4.9 Hz, 2H), 1.22 (s, 9H), 1.21 ppm (s, 9H)
**¹³C NMR (101 MHz, Chloroform-*d*** δ 177.0, 176.8, 106.1, 80.3, 75.2, 74.9, 55.3, 38.8, 38.6, 27.1, 6.7 ppm

### Preparation of Compound 15

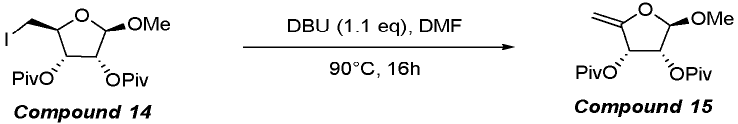

***Compound 14*** (47.8 g, 108 mmol, 1.00 eq) was dissolved in DMF (500 ml) and DBU (17.8 ml, 119 mmol, 1.10 eq) was added at once to the stirring mixture which was heated to 90°C for 18 hours. The mixture was cooled to room temperature and concentrated *in vacuo* to approximately 250 ml. Subsequently, the brown solution was diluted in n-heptane (1.5 1) and washed with brine (3x 750 ml). The resulting organic phase was dried (MgSO₄), filtered and the filtrate was concentrated *in vacuo.* The residue was purified by column chromatography over silica gel (gradient elution: *n*-heptane/EtOAc from 99:1 to 9:1). The fractions containing the product were collected and the solvent was evaporated to give the desired ***compound 15*** (30.2 g, 96.1 mmol, 89% yield) as a colorless liquid.
**¹H NMR (360 MHz, Chloroform-*d*)** δ 5.76 (dt, *J*=5.1, 1.9 Hz, 1H), 5.16 (d, *J=5.1* Hz, 1H), 5.02 (s, 1H), 4.51 (t, *J*=1.8 Hz, 1H), 4.08 (t, *J*=2.0 Hz, 1H), 3.46 (s, 3H), 1.23 (s, 9H), 1.21 ppm (s, 9H)
**¹³C NMR (101 MHz, Chloroform-*d*)** δ 177.3, 177.2, 157.2, 106.2, 84.6, 73.2, 69.7, 56.3, 39.1, 39.0, 27.4, 27.3 ppm

### Preparation of Compound 16

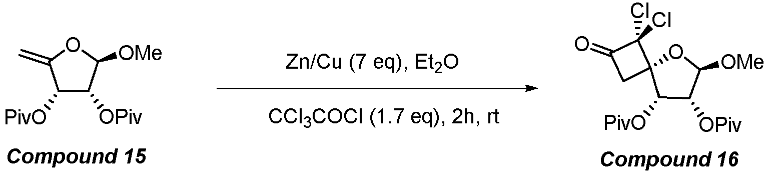

Zinc powder (25.0 g, 0.38 mol, 1.00 eq) was added to a two-necked round bottomed flask (500 ml) containing demineralized water (100 ml) and the solution was degassed with nitrogen during 15 minutes. Subsequently, copper(II)sulfate (1.85 g, 11.5 mmol, 0.03 eq) was added and the stirring solution was degassed and stirred for 45 minutes. The mixture was filtered and the black solids were washed with degassed water (250 ml) and degassed acetone (250 ml), respectively. The zinc-copper couple was dried *in vacuo* for 12 hours. ***Compound 15*** (10.0 g, 31.8 mmol, 1.00 eq) was weighed in an oven dried flask and dissolved in anhydrous diethylether (300 ml, dried over 4Å molecular sieves). Subsequently, zinc-copper couple (14.6 g, 223 mmol, 7.00 eq) was added at once to the stirring solution in diethylether. An oven dried pressure equalized dropping funnel was installed and charged with anhydrous diethylether (100 ml) and trichloroacetyl chloride (6.10 ml, 54.1 mmol, 1.70 eq). The reagent was added dropwise over a period of 2.5 hours and the temperature was monitored carefully in order not to exceed 25°C. After addition, zinc-copper couple was decanted, rinsed with diethylether (100 ml) and the organic layer was diluted with n-heptane (500 ml) before it was washed with NaHCO₃ (aq. sat. 3x 300 ml) and brine (2x 250 ml). The organic phase was dried (MgSO₄), filtered and the filtrate was concentrated *in vacuo* at 40°C to give ***compound 16*** (13.2 g, crude).
**¹H NMR (360 MHz, Chloroform-*d*)** δ 5.84 (d, *J=4.0* Hz, 1H), 5.35 (d, *J=4.4* Hz, 1H), 4.98 (s, 1H), 3.92 (d, *J=18.7* Hz, 1H), 3.51 (s, 1H), 3.40 (d, *J=18.7* Hz, 1H), 1.19 ppm (s, 18H)
**¹³C NMR** (91 **MHz, Chloroform-*d*)** δ 191.0, 176.3, 175.4, 106.0, 91.4, 83.2, 74.6, 71.2, 55.9, 52.0, 38.5, 26.8 ppm

### Preparation of Compound 17

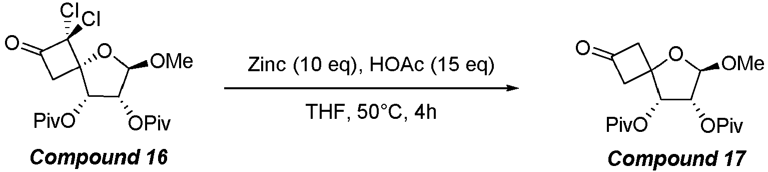

***Compound 16*** (2.64 g, 6.21 mmol, 1.00 eq) was dissolved in THF (45.0 ml) and acetic acid (5.33 ml, 93.1 mmol, 15.0 eq) was added followed by the portionwise addition of zinc powder (4.06 g, 62.1 mmol, 10.0 eq) and the mixture was heated to 50°C for 5 hours. Subsequently, the solution was cooled to room temperature and filtered over celite. The filtrate was concentrated to a minimal volume *in vacuo,* redissolved in EtOAc (200 ml), washed with brine (2x 75 ml), dried (MgSO₄), filtrated and concentrated *in vacuo.* The residue was purified by column chromatography over silica gel (gradient elution: n-heptane/EtOAc from 99:1 to 1:1). The fractions containing the product were collected and the solvent was evaporated to give the desired ***compound 17*** (1.48 g, 4.16 mmol, 67% yield) as a colorless oil.
**¹H NMR (360 MHz, Chloroform-*d*)** δ 5.56 (d, *J=4.4* Hz, 1H), 5.26 (dd, *J=4.4,* 0.7 Hz, 1H), 4.91 (s, 1H), 3.43-3.51 (m, 2H), 3.42 (s, 3H), 3.33-3.41 (m, 1H), 3.14-3.25 (m, 1H), 1.22 (s, 9H), 1.21 ppm (s, 9H)
**¹³C NMR (101 MHz, Chloroform-*d*)** δ 203.2, 177.2, 176.8, 105.7, 75.7, 75.1, 74.0, 58.3, 55.7, 55.5, 39.0, 38.8, 27.1 ppm

### Preparation of Compound 18a and Compound 18b

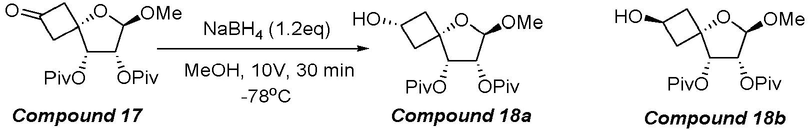

***Compound 17*** (500 mg, 1.40 mmol, 1.0 eq) was dissolved in MeOH (5 mL), NaBH₄ (64.0 mg, 1.68 mmol, 1.20 equiv) was added portionwise at -78°C, the resulting solution was stirred for 30 min at -78°C. The reaction was then quenched by the addition of 20 mL of saturated NH₄Cl aqueous. The resulting solution was extracted with 2x20 mL of ethyl acetate and the organic layers combined. The mixture was dried over anhydrous sodium sulfate and concentrated *in vacuo.* The residue was applied onto a silica gel column with ethyl acetate/petroleum ether (gradient elution: PE/EtOAc from 99:1 to 4:1). The fractions containing the product were collected and the solvent was evaporated to afford ***compound 18a*** (mixed with 14% ***compound 18b***).

**75% yield** (375 mg, 11.4 mmol), colorless oil. **¹H NMR (400 MHz, Chloroform-*d*)** δ 5.27 (d, J = 4.5 Hz, 1H), 5.16 (dd, J = 4.6, 1.9 Hz, 1H), 4.83 (d, J = 1.9 Hz, 1H), 3.92 (p, J = 7.1 Hz, 1H), 3.39 (s, 3H), 2.78-2.86 (m, 1H), 2.74 - 2.65 (m, 1H), 2.41 - 2.33 (m, 1H), 2.15-2.21 (m, 1H), 1.23 (s, 9H), 1.20 ppm (s, 9H). **GCMS** (ESI⁺) *m*/*z:* calcd. For C₁₈H₃₀O₇ [M-CH₃O]⁺ = 327.18, found 327.14, RT: 9.317 min, (Method 1).

### Preparation of Compound 19a and Compound 19b

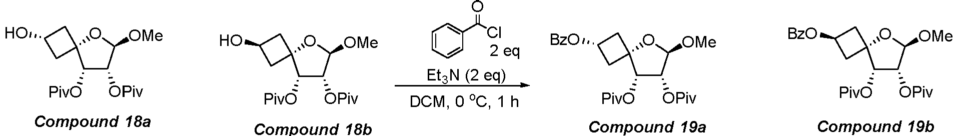

***Compound 18a*** (mixture with ***compound 18b*)** (7.00 g, 19.5 mmol, 1.0 eq), TEA (3.94 g, 39.1 mmol, 2.0 equiv) was dissolved in DCM (70 mL), then benzoyl chloride (5.47 g, 39.1 mmol, 2.0 equiv) was added dropwise at 0°C, the resulting solution was stirred for 1 hour at 0°C. The reaction was then quenched with 1M HCl aqueous until pH<7. The resulting solution was added 130 mL DCM and extracted with 1x200 mL of H₂O and the organic layers combined. The mixture was dried over anhydrous sodium sulfate and concentrated *in vacuo.* The residue was applied onto a silica gel column with ethyl acetate/ petroleum ether (gradient elution: PE/ EtOAc from 99:1 to 20:1). The fractions containing the product were collected and the solvent was evaporated to afford ***compound 19a*** (mixture with 13% ***compound 19b*)*.***

**87% yield** (7.90 g, 17.10 mmol), colorless oil. **¹H NMR (400 MHz, Chloroform-*d*)** δ 8.07 - 8.01 (m, 2H), 7.60 - 7.52 (m, 1H), 7.44 (dd, J = 8.4, 7.1 Hz, 2H), 5.38 (d, J = 4.5 Hz, 1H), 5.21 (dd, J = 4.5, 1.3 Hz, 1H), 4.86 - 4.78 (m, 2H), 3.40 (s, 3H), 3.05-3.14 (m, 1H), 2.94 - 2.84 (m, 1H), 2.73 (dd, J = 12.9, 7.3 Hz, 1H), 2.44 (dd, J = 12.9, 7.3 Hz, 1H), 1.29 (s, 9H), 1.22 ppm (s, 9H). **LCMS** (ESI⁺) *m*/*z:* calcd. For C₂₅H₃₄O₈ [M+NH_{4]}⁺= 480.26, found 480.25, RT: 2.151 min (Method 1).

### Preparation of Compound 19b

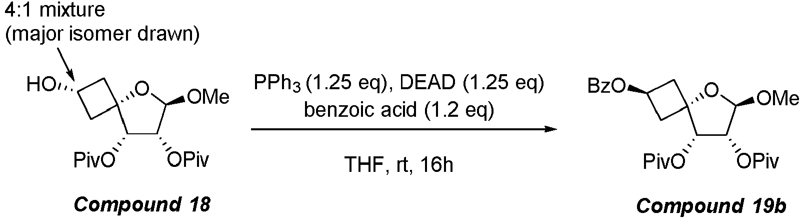

***Compound 18*** (2.28 g, 6.36 mmol, 1.00 eq) was dissolved in THF (60 ml) and benzoic acid (932 mg, 7.63 mmol, 1.20 eq) was added followed by triphenylphosphine (2.01 g, 7.63 mmol, 1.20 eq). The mixture was cooled to 0°C and diethyl azodicarboxylate (1.15 ml, 7.31 mmol, 1.20 eq) was added. The mixture was warmed to room temperature after the addition and stirred for 16 hours followed by adding pentane (120 ml) to precipitate triphenylphosphine-oxides. The solids were filtered and rinsed with heptane (30 ml). To the filtrate was added brine (50 ml) and the product was extracted in heptane (1× 150 ml, 2x 100 ml). Combined organic layers were dried (MgSO₄), filtered and the filtrate was concentrated *in vacuo.* The residue was purified by column chromatography over silica gel (gradient elution: n-heptane/EtOAc from 99:1 to 4:1). The fractions containing intermediate 30 were collected and the solvent was evaporated to give ***Compound 19b*** (1.79 g, 3.88 mmol, 61% yield) as a colorless oil.
**¹H NMR (400 MHz, Chloroform-*d*)** δ 7.98-8.04 (m, 2H), 7.52-7.60 (m, 1H), 7.39-7.47 (m, 2H), 5.44 (m, 1H), 5.37 (d, *J=4.4* Hz, 1H), 5.18 (dd, *J=4.4,* 1.5 Hz, 1H), 4.88 (d, *J*=1.5 Hz, 1H), 3.42 (s, 3H), 2.81 (ddd, *J*=13.4, 7.5, 4.4 Hz, 1H), 2.62-2.72 (m, 2H), 2.52-2.62 (m, 1H), 1.21 (s, 9H), 1.20 ppm (s, 9H)
**¹³C NMR (101 MHz, Chloroform-*d*)** δ 177.2, 177.0, 166.1, 133.0, 130.0, 129.6, 128.4, 105.7, 81.1, 75.1, 74.3, 65.5, 55.4, 41.3, 39.3, 39.0, 38.8, 27.2, 27.1 ppm

### Preparation of Compound 20

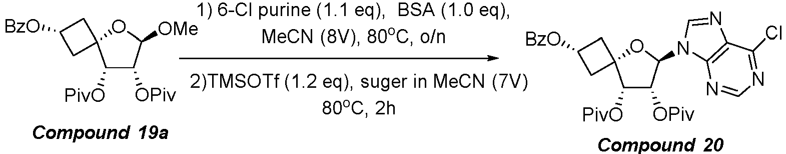

6-Cl Purine CAS 87-42-3 (1.69 g, 10.9 mmol, 1.1 equiv) was dissolved in MeCN (37 mL), and N,O-bis(trimethylsilyl)acetamide (2.02 g, 9.95 mmol, 1 equiv) was added dropwise. The mixture was heated to 80°C for overnight. After the mixture had cooled, ***compound 19a*** (mixture with 13% ***compound 19b).*** (4.6 g, 9.95 mmol, 1.00 equiv) in MeCN (31 mL) was added, trimethylsilyltrifluoromethanesulfonate (2.65 g, 11.94 mmol, 1.2 equiv) was added, and the mixture was heated to 80°C for 2 hours. Upon cooling to rt, the mixture was extracted to EtOAc twice with saturated NaHCO₃ and once with saturated aq. NaCl. The organic layer was dried over Na₂SO₄. Solvents were removed *in vacuo.* The residue was applied onto a silica gel column with dichlormethane/ methanol (gradient elution:DCM /MeOH from 99:1 to 30:1). The fractions containing the product were collected and the solvent was evaporated to afford ***compound 20.* 86% yield** (5.00 g, 8.56 mmol), foamed solid. **¹H NMR (300 MHz, Chloroform-*d*)** δ 8.81 (s, 1H), 8.21 (s, 1H), 8.09 - 8.02 (m, 2H), 7.64 - 7.57 (m, 1H), 7.49 (dd, J = 8.4, 6.9 Hz, 2H), 6.23 (t, J = 5.2 Hz,, 1H), 6.08 (d, J = 5.5 Hz, 1H), 5.83 (d, J = 4.8 Hz, 1H), 5.44 - 5.36 (m, 1H), 3.08 - 2.99 (m, 1H), 2.83 - 2.63 (m, 3H), 1.32 (s, 9H), 1.16 ppm (s, 9H). **LCMS** (ESI⁺) *m*/*z:* calcd. For C₂₉H₃₃ClN₄O₇ [M+H]⁺= 585.20, found 585.30, RT: 2.226 min (Method 6).

### Preparation of Compound 21

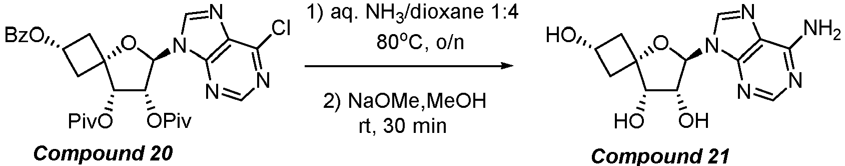

***Compound 20*** (4.0 g, 6.8 mmol, 1.0 equiv) was dissolved in 1,4-dioxane (40 mL), and aq.NH₃ (10 mL). The mixture was heated to 80°C overnight. Upon cooling to rt, then solvents were removed *in vacuo.* The product was dissolved in MeOH (40 mL) and sodium methoxide (0.37 g, 6.84 mmol, 1.00 equiv). The reaction was stirred at rt for 30 min, then stirred with H exchange resin to pH=7. Then filtered, and the filtrate was concentrated under reduced pressure. The residue was applied onto a silica gel column with dichloromethane/ methanol (gradient elution: DCM/ MeOH from 99:1 to 90:10). The fractions containing the product were collected and the solvent was evaporated to afford ***compound 21.***

**95% yield** (1.90 g, 6.48 mmol), white solid. **¹H NMR (400 MHz, D₂O)** δ8.24 (s, 1H), 8.16 (s, 1H), 5.97 (d, J = 6.6 Hz, 1H), 4.91 (dd, J = 6.7, 4.5 Hz, 1H), 4.40 (p, J = 6.5 Hz, 1H), 4.28 (d, J = 4.5 Hz, 1H), 2.64 - 2.42 (m, 4H), 2.29 - 2.22 ppm (m, 1H).**¹³C NMR (101 MHz, DMSO)** δ 156.06, 153.09, 150.02, 140.48, 119.36, 86.86, 78.91, 75.14, 73.28, 57.38, 45.97, 41.03 ppm. **LCMS** (ESI⁺) *m*/*z:* calcd. for C₁₂H₁₅N₅O₄ [M+H]⁺=294.11, found 294.12, RT: 1.301 min (Method 8).

### Preparation of Compound 22

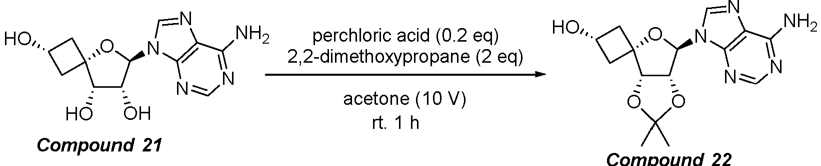

***Compound 21*** (1.80 g, 6.14 mmol, 1.00 equiv) was dissolved in acetone (18 mL), 2,2-dimethoxypropane (1.3 g, 12.3 mmol, 2.00 equiv) and perchloric acid (0.12 g, 1.22 mmol, 0.20 equiv) was added at 0°C. The reaction was stirred at rt for 1 hour. To the reaction was then added 1M NaOH aqueous until pH=7, followed by addition of 50 mL DCM and extracted with 1x60 mL of H₂O and the organic layers were combined. The mixture was dried over anhydrous sodium sulfate and concentrated *in vacuo.* The residue was applied onto a silica gel column with dichlormethane/ methanol (gradient elution: DCM/MeOH from 99:1 to 85:15). The fractions containing the product were collected and the solvent was evaporated to afford ***compound 22.***

**77% yield** (1.58 g, 4.50 mmol), light yellow oil. **¹H NMR (400 MHz, DMSO-d6)** δ 8.00 (s, 1H), 7.96 (s, 1H), 5.81 (s, 1H), 5.41 (d, J = 5.7 Hz, 1H), 4.68 (d, J = 5.7 Hz, 1H), 3.56 (p, J = 7.3 Hz, 1H), 2.51 (dd, J = 12.1, 6.1 Hz, 1H), 2.24 (dt, J = 12.2, 6.4 Hz, 1H), 1.85 (dd, J = 11.9, 7.9 Hz, 1H), 1.23 (s, 3H), 1.21 - 1.16 (m, 1H), 1.13 ppm (s, 3H). **¹³C NMR (101 MHz, DMSO)** δ 156.04, 153.16, 149.37, 140.31, 118.89, 112.93, 88.54, 84.95, 83.96, 80.02, 57.56, 48.94, 45.32, 26.62, 25.34 ppm. **LCMS** (ESI⁺) *m*/*z:* calcd. for C₁₅H₁₉N₅O₄ [M+H]⁺= 334.14, found 334.10, RT: 0.894 min (Method 3).

### Preparation of Compound 23

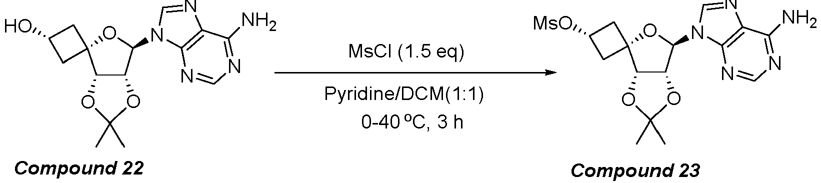

***Compound 22*** (1.5 g, 4.5 mmol, 1.0 equiv) was dissolved in dichloromethane (15 mL), pyridine (15 mL) and MsCl (0.77 g, 6.75 mmol, 1.5 equiv) was added at 0°C. The reaction was stirred at 40°C for 3 hours. Then the reaction mixture was poured into 30 mL ice water and extracted with 30 mL dichloromethane, and concentrated under reduced pressure giving ***compound 23.* 97% yield** (1.8 g, 4.38 mmol), light yellow oil. **¹H NMR (300 MHz, Methanol-*d4*)** δ 8.23 (s, 1H), 8.19 (s, 1H), 6.14 (d, J = 1.1 Hz, 1H), 5.77 (dd, J = 5.8, 1.1 Hz, 1H), 5.08 (d, J = 5.8 Hz, 1H), 4.74 (q, J = 7.1 Hz, 1H), 3.13 (dt, J = 12.8, 6.4 Hz, 1H), 3.01 (s, 3H), 2.83 (dt, J = 12.7, 6.4 Hz, 1H), 2.54 (dd, J = 12.8, 7.3 Hz, 1H), 1.96 (dd, J = 12.5, 7.1 Hz, 1H), 1.52 (s, 3H), 1.42 ppm (s, 3H). **¹³C NMR (75 MHz, Methanol-*d4*)** δ 155.85, 152.30, 148.83, 140.71, 119.06, 113.22, 89.77, 85.02, 84.26, 81.16, 66.42, 43.35, 38.31, 36.62, 25.40, 24.05 ppm. **LCMS** (ESI⁺) *m*/*z:* calcd. for C₁₆H₂₁N₅O₆S [M+H]⁺= 412.12, found 412.15, RT: 0.987 min (Method 1).

### Preparation of Compound 24

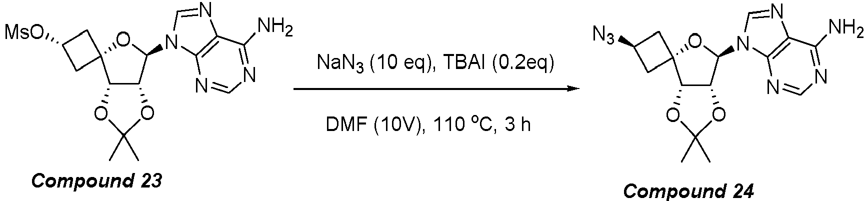

***Compound 23*** (1.80 g, 4.38 mmol, 1.00 equiv) was dissolved in DMF (30 mL), tetrabutylammonium iodide (0.16 g, 0.43 mmol, 0.10 equiv) and NaN₃ (2.8 g, 43.8 mmol, 10.00 equiv) was added at 0°C. The reaction was stirred at 110°C for 3 hours. Then the reaction mixture was poured into ice water 45mL and extracted with 2x50 mL dichloromethane and concentrated under reduced pressure. The residue was applied onto a silica gel column with dichlormethane/methanol (gradient elution: DCM/MeOH from 99:1 to 90:10). The fractions containing the product were collected and the solvent was evaporated to afford ***Compound 24.***

**70% yield** (1.10 g, 8.56 mmol), white solid. **¹H NMR (300 MHz, Methanol-*d4*)** δ 8.22 (s, 1H), 8.19 (s, 1H),6.15 (s, 1H), 5.69 (dd, J = 5.9, 0.9 Hz, 1H), 5.18 (d, J = 5.9 Hz, 1H), 4.04 - 3.96 (m, 1H), 2.67 - 2.49 (m, 2H), 2.26 (ddd, J = 13.0, 5.1, 3.2 Hz, 1H), 1.88 (ddd, J = 13.2, 7.8, 3.4 Hz, 1H), 1.51 (s, 3H), 1.43 ppm (s, 3H). **¹³C NMR (75 MHz, Methanol-*d4*)** δ 155.79, 152.55, 148.94, 140.72, 119.00, 112.94, 89.48, 86.17, 85.95, 84.09, 50.02, 41.14, 36.21, 25.36, 24.01 ppm. **LCMS** (ESI⁺) *m*/*z:* calcd. for C₁₅H₁₈N₈O₃ [M+H]⁺= 359.15, found 359.15, RT: 1.216 min, (Method 6).

### Preparation of Compound 25

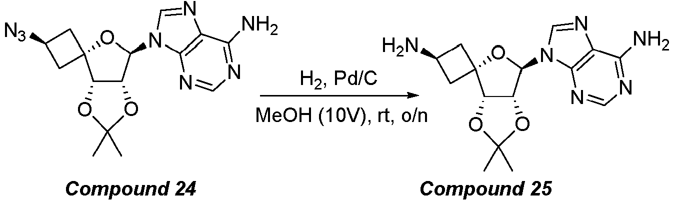

***Compound 24*** (1.0 g, 2.80 mmol, 1.00 equiv) was dissolved in methanol (10 mL) and Pd/C (0.2 g) was added. The reaction was stirred at r.t overnight under a hydrogen atmosphere. Following completion, the solution was then filtered. The filter cake was washed with MeOH, and the filtrate was concentrated under reduced pressure. The residue was applied onto a silica gel column with dichlormethane/ methanol (gradient elution: DCM/MeOH from 99:1 to 80:20). The fractions containing the product were collected and the solvent was evaporated to afford ***Compound 25***

**75% yield** (0.75 g, 2.25 mmol), white solid. **¹H NMR (300 MHz, Methanol-*d4*)** δ8.23 (s, 1H), 8.22 (s, 1H), 6.20 (s, 1H), 5.68 (dd, J = 5.9, 0.8 Hz, 1H), 5.22 (d, J = 5.9 Hz, 1H), 3.61 (t, J = 7.9 Hz, 1H), 2.64 - 2.57 (m, 1H), 2.52 (ddd, J = 13.6, 8.2, 5.0 Hz, 1H), 2.25 (dd, J = 13.3, 7.5 Hz, 1H), 1.80 (ddd, J = 13.2, 8.2, 4.9 Hz, 1H), 1.51 (s, 3H), 1.43 ppm (s, 3H). **¹³C NMR (101 MHz, Methanol-*d4*)** δ 156.02, 152.54, 148.94, 141.03, 119.01, 112.86, 89.46, 86.35, 85.82, 84.14, 41.40, 40.93, 36.98, 25.35, 23.98 ppm. **LCMS** (ESI⁺) *m*/*z:* calcd. for C₁₅H₂₀N₆O₃ [M+H]⁺= 333.16, found 333.10, RT: 0.902 min, (Method 3).

### Preparation of Compound 26

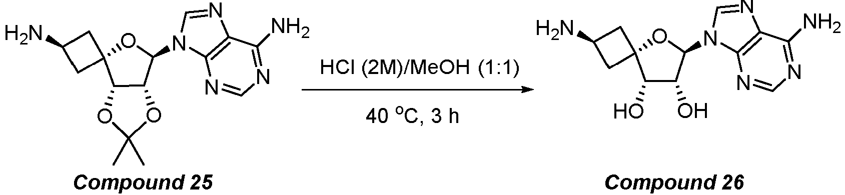

To ***compound 25*** (0.12 g, 0.36 mmol, 1.00 eq) in MeOH (2 mL) was added 2M HCl (2 mL) and the mixture was heated to 40°C for 3 hours. Subsequently, the solution was cooled to room temperature and carefully quenched with NaHCO₃ to pH=7. The mixture was extracted with CH₂Cl₂ (3x 2 mL) and combined organic layers were dried (Na₂SO₄), filtrated and the filtrate was concentrated in vacuo. The residue was purified by Pre-HPLC (Column: Atlantis Prep T3 OBD Column, 19*250 mm 10 u; Mobile Phase A: Water (10 mM NH₄HCO₃), Mobile Phase B: ACN; Flow rate: 25 mL/min; Gradient: 8 % B to 12 % B in 7 min). The fractions containing the product were collected and the solvent was evaporated to afford ***compound 26.***

**76% yield** (0.08 g, 0.27 mmol), white solid. **¹H NMR (300 MHz, Methanol*-d4*)** δ 8.25 (s, 1H), 8.17 (s, 1H), 6.01 (d, J = 6.4 Hz, 1H), 4.90 (dd, J = 6.3, 4.6 Hz, 1H), 4.27 (d, J = 4.6 Hz, 1H), 3.56 (p, J = 7.5 Hz, 1H), 2.60 (ddd, J = 12.9, 8.2, 4.5 Hz, 1H), 2.45 (ddd, J = 12.8, 8.1, 4.6 Hz, 1H), 2.31 (dd, J = 13.5, 7.0 Hz, 1H), 2.13 ppm (dd, J = 13.1, 6.8 Hz, 1H). **¹³C NMR (75 MHz, D₂O)** δ 155.68, 152.99, 149.17, 140.14, 119.00, 87.04, 85.03, 75.67, 73.84, 42.36, 40.93, 38.77 ppm. **LCMS** (ESI⁺) *m*/*z:* calcd. for C₁₂H₁₆N₆O₃ [M+H]⁺= 293.13, found 293.2, RT: 1.346 min, (Method 8)

### Preparation of Compound 27

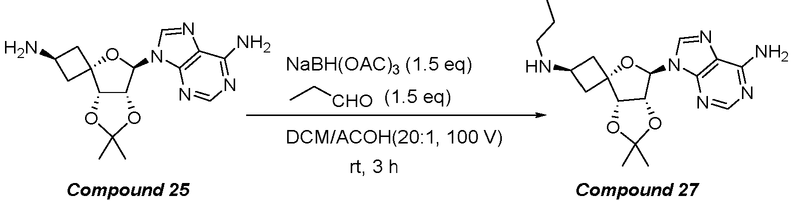

To ***compound 25*** (0.10 g, 0.30 mmol, 1.00 eq) in DCM (10 mL) was added AcOH (0.5 mL), followed by addition of propanal (0.026 g, 0.44 mmol, 1.50 eq). The reaction was stirred at r.t for 30min. the solution was cooled to 0°C. Slowly add NaBH(OAc)₃ (0.13 g, 0.62 mmol, 2.00 eq), and the mixture was stirred at r.t for 3 hours, and concentrated in vacuo. The residue was applied onto a silica gel column with dichlormethane/methanol (gradient elution: DCM/MeOH from 99:1 to 90:10). The fractions containing the product were collected and the solvent was evaporated to afford ***compound 27.***

**62% yield** (0.07 g, 0.18 mmol), white solid. **¹H NMR (300 MHz, D₂O)** δ 8.16 (d, J = 17.6 Hz, 2H), 6.23 (s, 1H), 5.66 (d, J = 5.9 Hz, 1H), 5.22 (d, J = 5.9 Hz, 1H), 3.84 - 3.65 (m, 1H), 2.87 - 2.67 (m, 3H), 2.59 (dd, J = 13.9, 7.0 Hz, 1H), 2.41 (dd, J = 13.7, 8.1 Hz, 1H), 1.89 (s, 3H), 1.75 (t, J = 7.1 Hz, 1H), 1.53 (d, J = 15.6 Hz, 4H), 1.42 (s, 3H), 0.86 ppm (t, J = 7.4 Hz, 3H). **¹³C NMR (101 MHz, D₂O)** δ 155.60, 152.93, 148.72, 140.76, 114.02, 88.29, 85.20, 85.11, 83.277, 47.02, 46.58, 37.52, 33.78, 25.23, 24.10, 22.71, 19.04, 10.12 ppm. **LCMS** (ESI⁺) *m*/*z:* calcd. for C₁₈H₂₆N₆O₃ [M+H]⁺= 375.21 found 375.10, RT: 1.285 min, (Method 3).

### Preparation of Compound 28

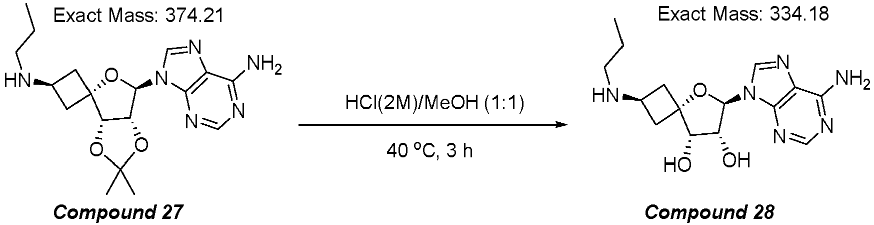

To ***compound 27*** (0.10 g, 0.27 mmol, 1.00 eq) in MeOH (2 mL) was added 2M HCl (2 mL) and the mixture was heated to 40°C for 3 hours. Subsequently, the solution was cooled to room temperature and carefully quenched with NaHCO₃ to pH= 7. The mixture was extracted with CH₂Cl₂ (3x 2 mL) and combined organic layers were dried (Na₂SO₄), filtrated and the filtrate was concentrated in vacuo. The residue was purified by Pre-HPLC (Column: Atlantis Prep T3 OBD Column, 19*250 mm 10u; Mobile Phase A: Water (10mM NH₄HCO₃), Mobile Phase B: ACN; Flow rate: 25 mL/min; Gradient: 8 % B to 12 % B in 7 min; 210/254 nm). The fractions containing the product were collected and the solvent was evaporated to afford ***compound 28.***

**45% yield** (0.040 g, 0.12 mmol), white solid.**¹H NMR (300 MHz, DMSO-*d6*)** δ 8.25 (s, 1H), 8.13 (s, 1H), 7.22 (s, 2H), 5.85 (d, J = 5.6 Hz, 1H), 5.26 (s, 1H), 4.81 (t, J = 5.0 Hz, 1H), 4.07 (d, J = 4.4 Hz, 1H), 3.37-3.24 (s, 2H) 3.19 - 3.09 (m, 1H), 2.41 (d, J = 9.0 Hz, 1H), 2.35 (t, J = 7.0 Hz, 2H), 2.26 (d, J = 12.8 Hz, 1H), 2.17 - 2.04 (m, 1H), 1.97 (d, J = 12.5 Hz, 1H), 1.39 (h, J = 7.3 Hz, 2H), 0.86 ppm (t, J = 7.4 Hz, 3H).**¹³C NMR (75 MHz, DMSO)** δ 156.52, 153.01, 149.98, 140.36, 119.79, 87.94, 84.94, 75.91, 73.70, 49.18, 47.14, 41.00, 37.38, 23.18, 12.33 ppm. **LCMS** (ESI⁺) *m*/*z:* calcd. for C₁₅H₂₂N₆O₃ [M+H]⁺= 335.18, found 335.25, RT: 0.908 min, (Method 3).

### Preparation of Compound 29

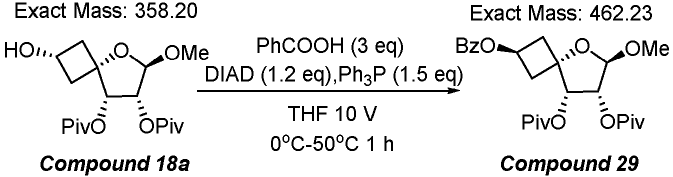

***Compound 18a*** (mixture with ***compound 18b*)** (6.00 g, 16.8 mmol, 1.00 equiv) was dissolved in THF (60 mL), and triphenyl phosphine (8.80 g, 33.6 mmol, 2.00 equiv), benzoic acid (6.20 g, 50.4 mmol, 3.00 equiv) were added. Next, DIAD (10.0 g, 50.4 mmol, 3.00 equiv) was added slowly at 0°C. The mixture was allowed to stir at rt for 1 h, and then heated to 50°C for 2 hours, followed by an additional 0.5 eq of triphenyl phosphine and 1 eq DIAD at 0°C, and then heated to 50°C for 1 hour. The reaction was cooled to RT and added to saturated NaHCO₃ aqueous (200 mL), and the aqueous phase was extracted with EA (200 mL), the organic phase was dried over anhydrous sodium sulfate and concentrated *in vacuo.* The residue was purified by column chromatography over silica gel (gradient elution: PE/EtOAc from 99:1 to 5:1). The fractions containing the product were collected and the solvent was evaporated to afford ***compound 29.***

**69% yield** (12.0 g, 35.92 mmol), yellow oil. **¹HNMR (300 MHz, Chloroform-*d*)** 8.03 - 7.98 (m, 2H), 7.59 - 7.53 (m, 1H), 7.43 (t, J = 7.8 Hz, 2H), 5.47 - 5.39 (m, 1H), 5.37 (d, J = 4.5 Hz, 1H), 5.18 (dd, J = 4.5, 1.5 Hz, 1H), 4.88 (d, J = 1.6 Hz, 1H), 3.42 (s, 3H), 2.81 (m, 1H), 2.71 - 2.61 (m, 2H), 2.60 - 2.50 (m, 1H), 1.21 ppm (s, 9H), 1.20 (s, 9H). **LCMS** (ESI⁺) *m*/*z:* calcd. for C₂₅H₃₄O₈ [M+NH₄]⁺= 480.26, found 480.25, RT: 1.557 min, Method 7.

### Preparation of Compound 30

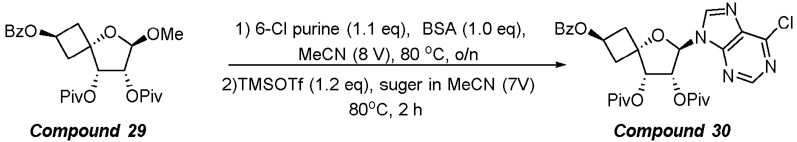

6-Cl Purine CAS 87-42-3 (1.65 g, 10.7 mmol, 1.1 equiv) was dissolved in MeCN (35 mL), and N,O-bis(trimethylsilyl)acetamide (2.02 g, 9.73 mmol, 1.0 equiv) was added dropwise. The mixture was heated to 80 °C for overnight. After the mixture had cooled, ***compound 29*** (4.5 g, 9.73 mmol, 1.00 equiv) in MeCN (30 mL) was added, trimethylsilyltrifluoromethanesulfonate (2.59 g, 11.68 mmol, 1.2 equiv) was added, and the mixture was heated to 80 °C for 2 h. Upon cooling to r.t, the mixture was extracted with EtOAc twice, washed with saturated NaHCO₃ and once with saturated aq. NaCl. The organic layer was dried over Na₂SO₄. Solvents were removed *in vacuo.* The residue was applied onto a silica gel column with dichloromethane/methanol (gradient elution: DCM/MeOH from 99:1 to 30:1). The fractions containing the product were collected and the solvent was evaporated to afford ***compound 30.***

**76% yield** (4.30 g, 7.36 mmol), foamed solid.**¹H NMR (300 MHz, Chloroform-*d*)** δ 8.78 (s, 1H), 8.19 (s, 1H), 8.08 - 7.98 (m, 2H), 7.63 - 7.53 (m, 1H), 7.46 (dd, J = 8.4, 6.9 Hz, 2H), 6.25 - 6.18 (m, 1H), 6.06 (d, J = 5.5 Hz, 1H), 5.81 (d, J = 4.8 Hz, 1H), 5.43 - 5.32 (m, 1H), 3.02 (m, 1H), 2.86 - 2.60 (m, 3H), 1.29 (s, 9H), 1.13 ppm (s, 9H). **LCMS** (ESI⁺) *m*/*z:* calcd. For C₂₉H₃₃ClN₄O₇ [M+H]⁺= 585.20, found 585.30, RT: 2.227 min, Method 5.

### Preparation of Compound 31

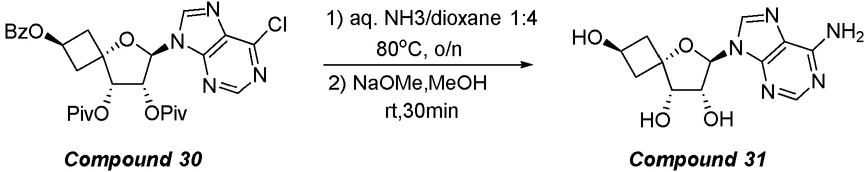

***Compound 30*** (4.30 g, 7.35 mmol, 1.00 equiv) was dissolved in 1,4-dioxane (45 mL), and concentrated aq.NH₃ (11 mL). The mixture was heated to 80 °C for overnight. Upon cooling to rt, then solvents were removed *in vacuo.* The product was dissolved in MeOH (45 mL) and sodium methoxide (0.40 g, 7.35 mmol, 1.00 equiv) was added. The reaction was stirred at RT for 30 min, then stirred with H exchange resin to pH=7. Then filtered, and the filtrate was concentrated under reduced pressure. The residue was applied onto a silica gel column with dichloromethane/ methanol (gradient elution: DCM/MeOH from 99:1 to 90:10). The fractions containing the product were collected and the solvent was evaporated to afford ***compound 31.* 95% yield** (2.05g, 7.00 mmol), white solid. **¹H NMR (300 MHz, Methanol-*d4*)** δ 8.24 (s, 1H), 8.16 (s, 1H), 6.00 (d, J = 6.6 Hz, 1H), 4.93 (dd, J = 6.7, 4.5 Hz, 1H), 4.44 (m, 1H), 4.31 (d, J = 4.5 Hz, 1H), 2.57 - 2.66 (m, 1H), 2.53 - 2.40 (m, 2H), 2.29 ppm (dd, J = 13.0, 5.7 Hz, 1H). **LCMS**(ESI⁺) *m*/*z:* calcd. for C₁₂H₁₅N₅O₄ [M+H]⁺= 294.11, found 294.20, RT: 1.409 min, Method 8.

### Preparation of Compound 32

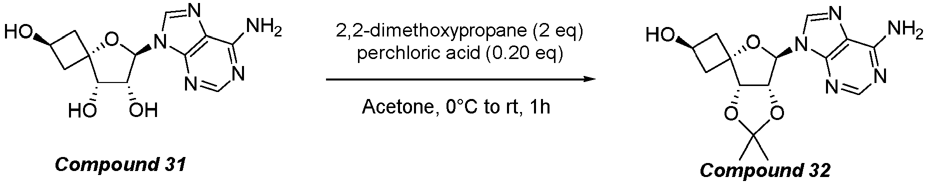

***Compound 31*** (2.00 g, 6.82 mmol, 1.00 equiv) was dissolved in acetone (20 mL), 2,2-dimethoxypropane (1.40 g, 13.6 mmol, 2.00 equiv) and perchloric acid (0.13 g, 1.36 mmol, 0.20 equiv) were added at 0 °C. The reaction was stirred at RT for 1 h. The reaction was then quenched with 1M NaOH aqueous until pH=7. To the resulting solution was added 60 mL DCM and extracted with 1x60 mL of H₂O and the organic layers were combined. The mixture was dried over anhydrous sodium sulfate and concentrated *in vacuo.* The residue was applied onto a silica gel column with dichlormethane/methanol (gradient elution: DCM/MeOH from 99:1 to 85:15). The fractions containing the product were collected and the solvent was evaporated to afford ***compound 32.***

**79% yield** (1.80 g, 5.40 mmol), white solid. **¹H NMR (400 MHz, Methanol-*d4*)** δ 8.23 (d, J = 5.3 Hz, 2H), 6.15 (s, 1H), 5.67 (dd, J = 5.9, 0.9 Hz, 1H), 5.13 (d, J = 5.9 Hz, 1H), 4.26 - 4.15 (m, 1H), 2.46 (m, 2H), 2.07 (ddd, J = 12.7, 5.5, 1.9 Hz, 1H), 1.75 (ddd, J = 13.2, 7.2, 3.1 Hz, 1H), 1.49 (s, 3H), 1.41 ppm (s, 3H). **¹³C NMR (101 MHz, Methanol-d4** δ 155.27, 151.41, 148.96, 141.07, 118.94, 89.43, 86.57, 85.45, 83.97, 61.30, 53.40, 44.13, 39.85, 25.40, 24.04 ppm. **LCMS** (ESI⁺) *m*/*z:* calcd. for C₁₅H₂₂N₆O₃ [M+H]⁺= 334.15, found 334.10, RT: 0.903 min, Method 3.

### Preparation of Compound 33

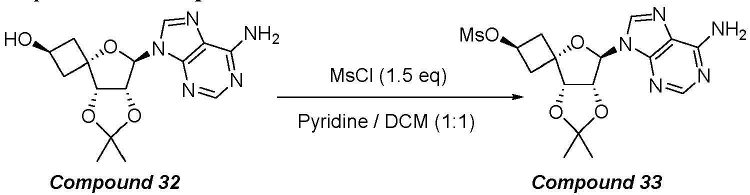

***Compound 32*** (1.00 g, 3.00 mmol, 1.00 equiv) was dissolved in dichloromethane (10 mL), pyridine (10 mL) and MsCl (0.51 g, 4.50 mmol, 1.5 equiv) was added at 0 °C. The reaction was stirred at 40 °C for 3 h. Then poured the reaction into 30 mL of ice water and extracted with 1x20 mL dichloromethane, and concentrated under reduced pressure giving ***compound 33.***

**81% yield** (1.00 g, 2.43 mmol), white solid. **¹H NMR (400 MHz, Methanol-*d4*)** δ 8.20 (d, J = 7.4 Hz, 2H), 6.17 (s, 1H), 5.67 (d, J = 5.9 Hz, 1H), 5.23 (d, J = 5.9 Hz, 1H), 4.98 (p, J = 6.3 Hz, 1H), 3.01 (s, 3H), 2.84 (dd, J = 11.6, 7.4 Hz, 1H), 2.65 (dt, J = 13.8, 6.3 Hz, 1H), 2.51 (dd, J = 11.4, 6.9 Hz, 1H), 2.04 - 1.93 (m, 1H), 1.50 (s, 3H), 1.42 ppm (s, 3H). **LCMS** (ESI⁺) *m*/*z:* calcd. for C₁₆H₂₁N₅O₆S [M+H]⁺= 412.12, found 412.15, RT: 1.190 min, Method 3.

### Preparation of Compound 34

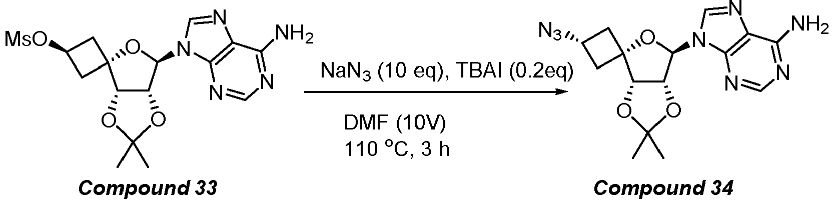

***Compound 33*** (300 mg, 0.73 mmol, 1.00 equiv) was dissolved in DMF (3 mL), tetrabutylammonium iodide (27 mg, 0.07 mmol, 0.10 equiv) and NaN₃ (474 mg, 7.3 mmol, 10.00 equiv) were added at 0 °C. The reaction was stirred at 110 °C for 3 h. Then poured the reaction into ice water 4 mL, extracted with 2x5 mL dichloromethane and concentrated under reduced pressure. The residue was applied onto a silica gel column with dichlormethane/methanol (gradient elution: DCM/MeOH from 99:1 to 90:10). The fractions containing the product were collected and the solvent was evaporated to afford ***compound 34.***

**76% yield** (200 mg, 0.56 mmol), white solid. **¹H NMR (300 MHz, Methanol-*d4*)** δ 8.20 (d, J = 8.1 Hz, 2H), 6.12 (s, 1H), 5.78 (dd, J = 5.8, 1.1 Hz, 1H), 5.07 (d, J = 5.8 Hz, 1H), 3.65 (p, J = 7.7 Hz, 1H), 2.64 (dt, J = 12.6, 6.5 Hz, 1H), 2.27 (dd, J = 12.4, 8.3 Hz, 1H), 1.68 (dd, J = 12.2, 8.0 Hz, 1H), 1.52 (s, 3H), 1.42 (s, 3H), 1.02 ppm (t, J = 7.3 Hz, 1H). **¹³C NMR (75 MHz, Methanol-*d4*)** δ 156.01, 152.52, 148.89, 140.57, 119.03, 113.13, 89.70, 85.01, 84.15, 81.69, 46.52, 41.64, 36.43, 25.39, 24.04, 12.46 ppm. **LCMS** (ESI⁺) *m*/*z:* calcd. for C₁₅H₁₈N₈O₃ [M+H]⁺= 359.15, found 359.20, RT: 1.364 min, Method 22.

### Preparation of Compound 35

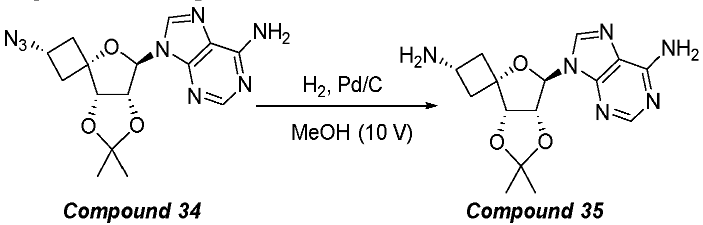

***Compound 34*** (200 mg, 0.56 mmol, 1.00 equiv) was dissolved in methanol (2 mL) and Pd/C (40 mg) was added. The reaction was stirred at r.t for overnight. Following completion, the solution was then filtered. The filter cake was washed with MeOH, and the filtrate was then concentrated under reduced pressure. The residue was applied onto a silica gel column with dichloromethane/methanol (gradient elution: DCM/MeOH from 99:1 to 80:20). The fractions containing the product were collected and the solvent was evaporated to afford ***compound 35.* 70% yield** (130 mg, 0.39 mmol), white solid. **¹H NMR (300 MHz, Methanol-*d4*)** δ 8.20 (d, J = 1.9 Hz, 2H), 6.11 (s, 1H), 5.78 (dd, J = 5.8, 1.0 Hz, 1H), 5.03 (d, J = 5.8 Hz, 1H), 3.14 (p, J = 7.9 Hz, 1H), 2.91 (dt, J = 12.6, 6.4 Hz, 1H), 2.54 (dt, J = 12.4, 6.5 Hz, 1H), 2.11 (dd, J = 12.1, 8.6 Hz, 1H), 1.55-1.47 (m, 4H), 1.42 ppm (s, 3H). **¹³C NMR** (75 **MHz, Methanol-*d4*)** δ 156.01, 152.50, 148.98, 140.38, 118.94, 112.98, 89.51, 85.02, 84.05, 81.51, 43.70, 38.56, 38.44, 25.40, 24.05 ppm. **LCMS** (ESI⁺) *m*/*z:* calcd. for C₁₅H₂₀N₆O₃ [M+H]⁺= 333.16, found 333.05, RT: 0.966 min, Method 3.

### Preparation of Compound 36

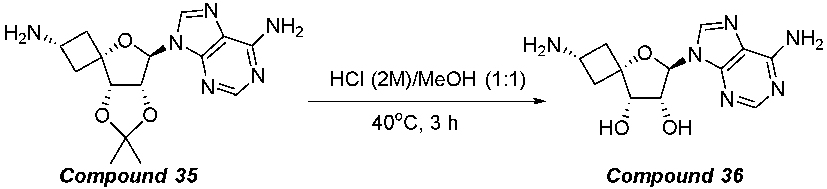

***Compound 35*** (155 mg, 0.470 mmol, 1.00 eq) in MeOH (2 mL) was added 2M HCl (2 mL) and the mixture was heated to 40 °C for 3 h. Subsequently, the solution was cooled to room temperature and carefully quenched with NaHCO₃ to pH= 7. The mixture was extracted with CH₂Cl₂ (3x 2 mL) and the combined organic layers were dried (Na₂SO₄), filtrated and the filtrate was concentrated in vacuo. The residue was purified by Pre-HPLC (Column: Atlantis Prep T3 OBD Column, 19*250 mm 10u; Mobile Phase A: Water (10 mM NH₄HCO₃), Mobile Phase B: ACN; Flow rate: 25 mL/min; Gradient: 8 % B to 12 % B in 7 min; 210/254 nm;). The fractions containing the product were collected and the solvent was evaporated to afford ***compound 36.***

**37% yield** (50 mg, 0.17 mmol), white solid. **¹H NMR (400 MHz, DMSO-d6)** δ 8.26 (d, J = 4.8 Hz, 1H), 8.13 (s, 1H), 7.24 (s, 2H), 5.81 (d, J = 7.2 Hz, 1H), 5.33 (s, 2H), 4.98 (dd, J = 7.1, 4.1 Hz, 1H), 3.86 (d, J = 4.0 Hz, 1H), 3.17 (m, 3H), 2.91 - 2.69 (m, 2H), 1.85 (q, J = 10.4 Hz, 1H), 1.67 ppm (t, J = 9.9 Hz, 1H). **¹³C NMR (101 MHz, DMSO)** δ 156.50, 153.01, 150.25, 140.36, 119.77, 86.96, 79.77, 75.28, 73.34, 49.06, 46.93, 41.66 ppm. **LCMS** (ESI⁺) *m*/*z:* calcd. for C₁₂H₁₆N₆O₃ [M+H]⁺= 293.13, found 293.20, RT: 0.948 min, Method 2.

### Preparation of Compound 37

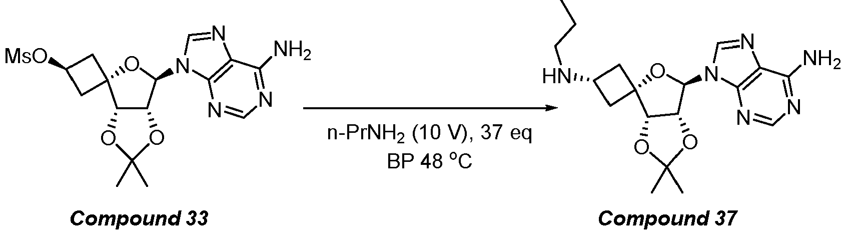

***Compound 33*** (190 mg, 0.46mmol, 1.00 eq) was dissolved in n-PrNH₂ (2 mL) and the mixture was heated to 90 °C for 48 h. Subsequently, the solution was cooled to room temperature and purified by Prep-HPLC (Column: Atlantis Prep T3 OBD Column, 19*250 mm 10u; Mobile Phase A: Water (10 mM NH₄HCO₃), Mobile Phase B: ACN; Flow rate: 25 mL/min; Gradient: 8 % B to 12 % B in 7 min; 210/254 nm). The fractions containing the product were collected and the solvent was evaporated to afford ***compound 37.***

**68% yield** (130 mg, 0.35 mmol), white solid. **¹H NMR (300 MHz, Methanol-d4)** δ 8.21 (d, J = 2.1 Hz, 2H), 6.14 (s, 1H), 5.80 (dd, J = 5.9, 1.0 Hz, 1H), 5.13 (d, J = 5.8 Hz, 1H), 2.99 (dt, J = 13.0, 6.3 Hz, 1H), 2.92 - 2.83 (m, 2H), 2.66 (dd, J = 7.7, 3.1 Hz, 2H), 2.30 (dd, J = 12.4, 8.7 Hz, 1H), 1.69 (dt, J = 15.1, 7.6 Hz, 3H), 1.52 (s, 3H), 1.43 (s, 3H), 1.01 ppm (t, J = 7.5 Hz, 3H). **¹³C NMR (75 MHz, Methanol-d4)** δ 165.19, 152.54, 148.85, 140.60, 113.16, 84.96, 84.09, 81.85, 43.93, 40.27, 38.06, 35.06, 23.99, 20.58, 20.25, 20.25, 10.02, 9.70 ppm. **LCMS** (ESI+) m/z: calcd. for C₁₂H₁₆N₆O₃ [M+H]+ = 375.21, found 375.20, RT: 1.263 min, Method: 3.

### Preparation of Compound 38

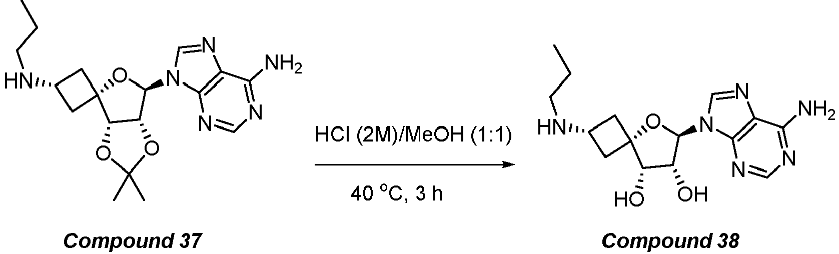

***Compound 37*** (130 mg, 0.35 mmol, 1.00 eq) in MeOH (2 mL) was added 2M HCl (2 mL) and the mixture was heated to 40 °C for 3 h. Subsequently, the solution was cooled to room temperature and carefully quenched with NaHCO₃ to pH= 7. The mixture was extracted with CH₂Cl₂ (3x 2 mL) and the combined organic layers were dried (Na₂SO₄), filtrated and the filtrate was concentrated in vacuo. The residue was purified by Pre-HPLC (Column: Atlantis Prep T3 OBD Column, 19*250 mm 10u; Mobile Phase A: Water (10 mM NH₄HCO₃), Mobile Phase B: ACN; Flow rate: 25 mL/min; Gradient: 8% B to 12% B in 7 min; 210/254 nm). The fractions containing the product were collected and the solvent was evaporated to afford ***compound 38.***

**34** % **yield** (40 mg, 0.12 mmol), white solid. **¹H NMR (400 MHz, Methanol-d4)** δ 8.22 (d, J = 5.5 Hz, 2H), 5.98 (d, J = 6.7 Hz, 1H), 5.07 (dd, J = 6.6, 4.3 Hz, 1H), 4.12 (d, J = 4.3 Hz, 1H), 2.94 (tq, J = 15.0, 7.5, 7.0 Hz, 2H), 2.66 (dt, J = 12.2, 6.2 Hz, 1H), 2.51 (t, J = 7.5 Hz, 2H), 2.14 (dd, J = 11.8, 7.5 Hz, 1H), 2.05 - 1.90 (m, 1H), 1.52 (h, J = 7.4 Hz, 2H), 0.95 ppm (t, J = 7.4 Hz, 3H). **¹³C NMR (101 MHz, Methanol-d4)** δ 155.91, 152.42, 149.56, 140.10, 119.29, 88.01, 80.72, 75.31, 73.77, 48.59, 44.12, 42.60, 37.53, 22.26, 10.69 ppm. **LCMS** (ESI⁺) *m*/*z:* calcd. for C₁₂H₁₆N₆O₃ [M+H]⁺= 335.18, found 335.15, RT: 1.937 min, Method 3.

### Preparation of Compound 67.1

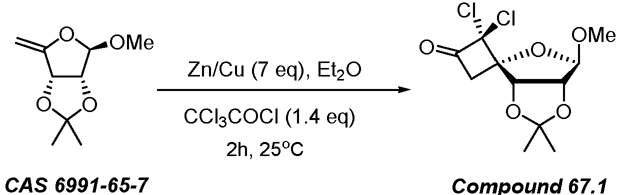

Zinc powder (25.0 g, 0.380 mol) was added to a two-necked round bottomed flask (500 ml) containing demineralized water (100 ml) and the solution was degassed with nitrogen for 15 minutes. Subsequently, copper(II)sulfate (1.85 g, 11.5 mmol) was added and the stirring solution was degassed for 45 minutes. The mixture was filtered and the solids were washed with degassed water (250 ml) and degassed acetone (250 ml), respectively. The zinc-copper couple was dried *in vacuo* for 12 hours. A solution of CAS 6991-65-7 (5.00 g, 26.9 mmol, 1.00 eq) in anhydrous Et₂O (150 ml, dried over 4Å molecular sieves) was added to the zinc-copper couple (12.2 g, 186 mmol, 7.00 eq) in a flame-dried flask under inert argon atmosphere. Subsequently, a solution of trichloroacetylchloride (4.29 ml, 37.7 mmol, 1.40 eq) in anhydrous Et₂O (30 ml) was added dropwise to the stirring mixture over a period of 3 hours at 25°C. After complete addition, stirring was stopped and the organic layer was decanted from precipitated zinc salts and washed with pentane/Et₂O (100 ml). The organic phase was washed with NaHCO₃ (aq. sat. 3x 150 ml) and brine (3x 100 ml), dried (MgSO₄), filtered and the filtrate was concentrated *in vacuo* to give ***compound 67.1*** (7.20 g, crude).

**¹H NMR (250 MHz, CDCl₃)** δ 5.10 (d, *J*=5.7 Hz, 1H), 5.07 (s, 1H), 4.68 (d, *J*=5.7 Hz), 3.61 (dd, *J*= 28.2, 18.7, 2H), 3.52 (s, 1H), 1.43 (s, 3H), 1.34 ppm (s, 3H). **¹³C NMR (63 MHz, CDCl₃)** δ 191.6, 113.5, 110.0, 91.1, 87.6, 85.3, 81.2, 57.3, 50.0, 26.5, 25.5 ppm.

### Preparation of Compound 93

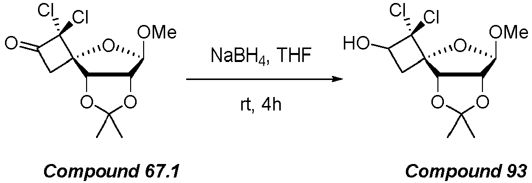

***Compound 67.1*** (63 g, 212.83 mmol, 1.00 eq) was dissolved in THF (630 mL), then NaBH₄ (16.2 g, 426 mmol, 2.00 eq) was added at 0°C. The mixture was stirred at 0°C for 30 min. and subsequently added to saturated NH₄Cl aqueous (1000 mL) at 0°C. The product was extracted with EtOAc (3 x 1000 mL) and combined organic layers were dried (Na₂SO₄), filtered and the filtrate was concentrated *in vacuo.* The residue was purified via silica gel column with PE/EtOAc (gradient elution: PE/EtOAc from 99:1 to 70:30). The fractions containing the product were collected and the solvent was evaporated to afford ***compound 93.***

**72% yield** (45 g, 151.00 mmol), light yellow oil. **¹H NMR** (300 MHz, Chloroform-d) δ 4.99 (d, J = 5.7 Hz, 1H), 4.95 (s, 1H), 4.68 (d, J = 5.8 Hz, 1H), 4.21 (dd, J = 9.7, 8.0 Hz, 1H), 3.53 (s, 3H), 2.89 (dd, J = 11.8, 8.0 Hz, 1H), 2.16 (dd, J = 11.8, 9.7 Hz, 1H), 1.44 (s, 3H), 1.36 ppm (s, 3H). **¹³C NMR** (75 MHz, DMSO) δ 112.5, 108.9, 95.9, 86.9, 84.7, 82.0, 71.0, 56.3, 35.4, 26.7, 25.5 ppm.

### Preparation of Compound 94

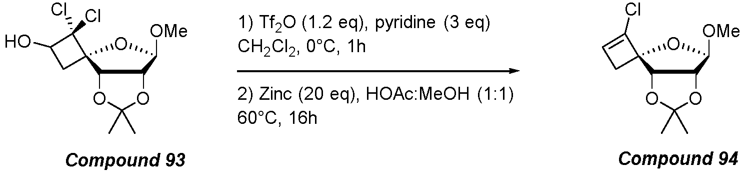

***Compound 93*** (45 g, 151 mmol, 1.00 eq) was dissolved in CH₂Cl₂ (450 mL), then pyridine (35.8 g, 453.16 mmol, 3.00 eq) and Tf₂O (51.1 g, 181 mmol, 1.20 eq) were added at 0°C. The mixture was stirred at 0°C for 1 hour. The mixture was added to saturated NaHCO₃ aqueous (500 mL) at 0°C, and then extracted with EtOAc (3 x 500 mL). The organic phase was dried with Na₂SO₄. Filtered and the filtrate was concentrated *in vacuo.* The residue (45 g, crude) was dissolved in AcOH:MeOH (1:1, 20V) and zinc (136 g, 2092.30 mmol, 20.0 eq) was added. The mixture was stirred at 60°C for 16 hours. The residue was added to saturated NaHCO₃ aqueous (500 mL) at 0°C, the solids were filtered out, and the aqueous was extracted with ethylacetate (500 mL), the organic phase was dried over anhydrous sodium sulfate and concentrated *in vacuo.* The residue was purified by column chromatography over silica gel (gradient elution:PE/EtOAc from 99:1 to 20: 1). The fractions containing the product were collected and the solvent was evaporated to afford ***compound 94.***

**69% yield** (25.5 g, 217 mmol, 2 steps). **¹H NMR** (300 MHz, Chloroform-d) δ 6.17 (t, J = 1.1 Hz, 1H), 4.97 (s, 1H), 4.79 (d, J = 5.9 Hz, 1H), 4.60 (d, J = 5.9 Hz, 1H), 3.36 (s, 3H), 2.99 (dd, J = 12.1, 1.2 Hz, 1H), 2.58 (dd, J = 12.2, 1.1 Hz, 1H), 1.45 (s, 3H), 1.33 ppm (s, 3H). **¹³C NMR** (75 MHz, CDCl₃) δ 133.0, 132.3, 112.5, 107.5, 93.6, 85.1, 81.2, 54.5, 36.4, 26.4, 25.3 ppm.

### Preparation of Compound 95a

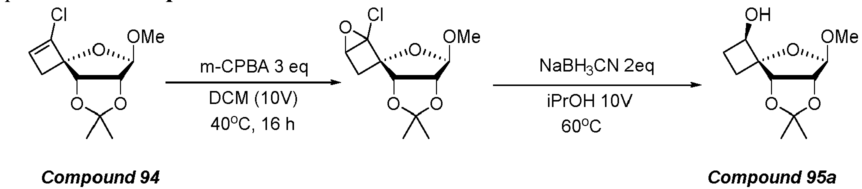

***Compound 94*** (25 g, 102 mmol, 1.00 eq) was dissolved in DCM (250mL) and *m*-CPBA (52.0 g, 302 mmol, 3.00 eq) was added at 0°C. The mixture was stirred at 40°C for 16 hours. After the mixture had cooled, the solids were removed via filtration and *i*PrOH (250 mL), followed by NaBH₃CN (12.8 g, 203 mmol, 2.00 eq) was added at 0°C. The mixture was stirred at 60 °C for 2 hours. After the mixture had cooled to room temperature, the residue was added to saturated aqueous NaHCO₃, and the aqueous layer was extracted with EtOAc (3 x 300 mL). Combined organic fractions were dried over anhydrous sodium sulfate and concentrated *in vacuo.* The residue was purified by column chromatography over silica gel (gradient elution:PE/EtOAc from 99:1 to 95:5). The fractions containing the product were collected and the solvent was evaporated to afford ***compound 95a.***

**52% yield** (12.1 g, 52.6 mmol, 2 steps), colorless oil. **¹H NMR** (400 MHz, Chloroform-d) δ 5.00 (d, J = 5.8 Hz, 1H), 4.83 (s, 1H), 4.52 (d, J = 5.8 Hz, 1H), 4.03 - 3.97 (m, 1H), 3.41 (s, 3H), 2.21 - 2.09 (m, 2H), 1.74 (td, J = 11.3, 8.9 Hz, 1H), 1.67 - 1.56 (m, 1H), 1.43 (s, 3H), 1.35 ppm (s, 3H). **¹³C NMR** (75 MHz, DMSO) δ 111.5, 107.3, 91.0, 85.1, 78.1, 73.9, 54.5, 26.7, 25.5, 23.4, 20.0 ppm.

### Preparation of Compound 96

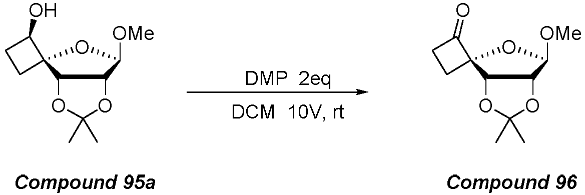

***Compound 95a*** (300 mg, 1.30 mmol, 1.00 eq) was dissolved in DCM (3 mL), then DMP (1.10 g, 2.60 mmol, 2.00 eq) was added at 0°C. The mixture was stirred at room temperature for 2 hours. The reaction was quenched by the addition of saturated aqueous Na₂S₂O₃ (20 mL) and saturated aqueous NaHCO₃ (20 mL), followed by extraction with EtOAc (3 x 20 mL). The organic phase was dried with Na₂SO₄, filtered and the filtrate was concentrated *in vacuo.* The residue was purified via silica gel column with PE/EtOAc (gradient elution: PE/EtOAc from 99:1 to 90:10). The fractions containing the product were collected and the solvent was removed *in vacuo* to afford ***compound 96.***

**63% yield** (187 mg, 0.82 mmol), white solid. **¹H NMR** (300 MHz, Chloroform-d) δ 4.97 (s, 1H), 4.76 (d, J = 5.9 Hz, 1H), 4.63 (d, J = 5.9 Hz, 1H), 3.39 (s, 3H), 2.95 - 2.81 (m, 2H), 2.64 (ddd, J = 12.2, 10.6, 6.0 Hz, 1H), 2.15 (dt, J = 12.1, 10.4 Hz, 1H), 1.37 (s, 3H), 1.34 ppm (s, 3H). **¹³C NMR** (75 MHz, CDCl₃) δ 206.2, 113.1, 107.1, 101.6, 85.0, 79.0, 54.9, 39.5, 26.3, 25.4, 20.0 ppm.

### Preparation of Compound 97a

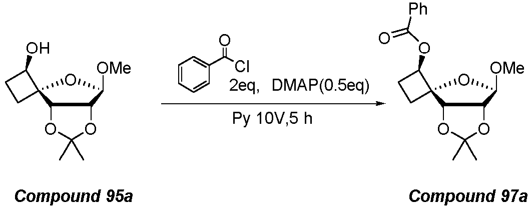

***Compound 95a*** (12.0 g, 52.2 mmol, 1.00 eq), DMAP (5.79g, 26.1 mmol, 0.50 eq) was dissolved in pyridine (120 mL), then benzoyl chloride (14.6 g, 104 mmol, 2.00 eq) was added dropwise at 0°C and the resulting solution was stirred for 1 hour at 0°C. To the reaction was then added HCl (1M, aqueous, 20 ml) until pH<7 was reached. The product was extracted with EtOAc (3 x 200 mL) and combined organic layers were dried (Na₂SO₄), filtered and the filtrate was concentrated *in vacuo.* The residue was purified via silica gel column with PE/ EtOAc (gradient elution: PE/EtOAc from 99:1 to 80:20). The fractions containing the product were collected and the solvent was removed *in vacuo* to afford ***compound 97a.***

**79% yield** (13.7 g, 41.0 mmol), colorless oil. **¹H NMR** (300 MHz, Chloroform-d) δ 8.04 - 7.97 (m, 2H), 7.63 - 7.56 (m, 1H), 7.50 - 7.44 (m, 2H), 5.37 - 5.31 (m, 1H), 5.21 (d, J = 5.8 Hz, 1H), 4.86 (s, 1H), 4.62 (d, J = 5.9 Hz, 1H), 3.28 (s, 3H), 2.48 - 2.34 (m, 2H), 2.01 - 1.89 (m, 1H), 1.86 - 1.73 (m, 1H), 1.49 (s, 3H), 1.44 ppm (s, 3H). **¹³C NMR** (75 MHz, CDCl3) δ 165.1, 130.2, 132.9, 129.5, 128.4, 112.4, 107.7, 89.0, 85.2, 78.7, 74.3, 55.0, 26.4, 25.6, 22.0, 20.8 ppm.

### Preparation of Compound 98a

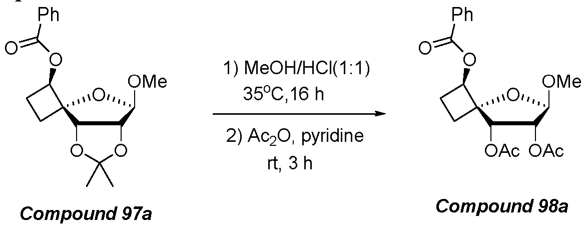

***Compound 97a*** (7.00 g, 21.0 mmol, 1.00 eq) was dissolved in MeOH (70 mL) and HCl (2M, aq. 70 mL) was added. The mixture was heated to 35°C for 16 hours. Subsequently, the solution was cooled to room temperature and concentrated *in vacuo.* This resulted in 12 g of crude ***compound 98a.***

Half the amount of the residue (6 g, crude) was dissolved in dry pyridine (60 mL) and stirred for 30 minutes. Acetic anhydride (3.10 g, 29.8 mmol, 1.50 eq) was added at 0°C. The mixture was stirred at room temperature for 3 hours. Subsequently, the mixture was poured into ice-cold water (200 ml) and stirred for 30 minutes at room temperature. The crude mixture was extracted with CH₂Cl₂ (3x 200 ml) and combined organic layers were washed with brine (3x 200 ml), dried (Na₂SO₄), filtered and the filtrate was concentrated *in vacuo.* The residue was purified by silica gel chromatography (gradient elution: PE / EA from 100:1 to 10:1). Fractions containing the product were combined and the solvent was removed *in vacuo* to afford ***compound 98a.***

**40% yield** (3.10 g, 8.20 mmol, 2 steps), yellow oil. **¹H NMR** (300 MHz, Methanol-d4) δ 8.06 - 8.00 (m, 2H), 7.63 - 7.59 (m, 1H), 7.49 (dt, J = 4.7, 1.9 Hz, 2H), 5.92 (d, J = 4.7 Hz, 1H), 5.35 - 5.29 (m, 1H), 5.25 (dd, J = 4.8, 3.0 Hz, 1H), 4.98 (d, J = 3.0 Hz, 1H), 3.35 (s, 3H), 2.33 - 2.24 (m, 2H), 2.14 (s, 3H), 2.09 (s, 3H), 1.96 - 1.86 (m, 1H), 1.66 - 1.57 ppm (m, 1H). **¹³C NMR** (101 MHz, CDCl3) δ 174.2, 174.0, 168.9, 137.1, 136.6, 132.3, 132.0, 109.3, 90.4, 80.0, 78.3, 74.4, 58.7, 57.3, 26.2, 25.0, 23.0 ppm.

### Preparation of Compound 99a

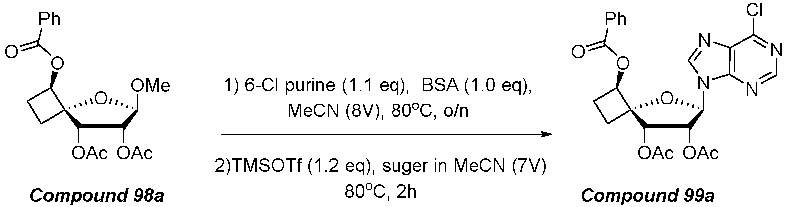

6-Chloropurine (1.40 g, 8.99 mmol, 1.10 eq) was dissolved in MeCN (25 mL), and N,O-bis(trimethylsilyl)acetamide (1.65 g, 8.13 mmol, 1.00 eq) was added dropwise. The mixture was heated to 80°C for 16 hours. After the mixture was cooled to room temperature, ***compound 98a*** (3.10 g, 8.20 mmol, 1.00 eq) in MeCN (22 mL) was added, followed by trimethylsilyltrifluoromethanesulfonate (2.15 g, 9.68 mmol, 1.20 eq) and the mixture was heated to 80°C for 2 hours. The mixture was cooled to room temperature and diluted with EtOAc (50 mL), washed with saturated NaHCO₃ (3x50 mL) and saturated aq. NaCl (3 x 50 mL). The organic layer was dried (Na₂SO₄), filtered and the filtrate was concentrated *in vacuo.* The residue was purified by silica gel column chromatography with PE/EtOAc (gradient elution: PE/EtOAc from 99:1 to 4:1). The fractions containing the product were collected and the solvent was removed *in vacuo* to afford ***compound 99a.***

**57% yield** (2.30 g, 4.60 mmol), white solid. **¹H NMR** (400 MHz, Methanol-d4) δ 8.66 (s, 1H), 8.33 - 8.28 (m, 2H), 8.12 (s, 1H), 7.70 - 7.64 (m, 1H), 7.55 (t, J = 7.7 Hz, 2H), 6.55 (dd, J = 7.4, 4.2 Hz, 1H), 6.39 (d, J = 7.5 Hz, 1H), 6.26 (d, J = 4.2 Hz, 1H), 5.55 (dd, J = 9.1, 7.6 Hz, 1H), 2.39 - 2.32 (m, 2H), 2.29 (s, 3H), 2.04 (s, 3H), 1.98 (dd, J = 12.4, 9.9 Hz, 1H), 1.80 - 1.73 ppm (m, 1H). **¹³C NMR** (75 MHz, MeOD) δ 170.2, 169.8, 165.6, 151.3, 151.1, 150.4, 146.6, 133.3, 132.1, 129.7, 129.5, 128.4, 88.4, 86.4, 73.9, 72.6, 70.8, 22.0, 20.3, 19.0, 18.8 ppm. **LCMS (ESI+)** m/z: calcd. for C23H21ClN4O7 [M+H]+=501.11, found 501.20, RT:1.806 min, (Method 3).

### Preparation of Compound 100a

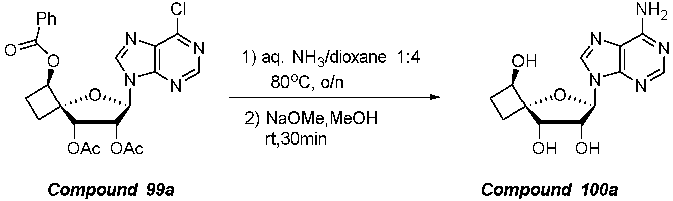

***Compound 99a*** (110 mg, 0.22 mmol, 1.00 eq) was dissolved in 1,4-dioxane (1.2 mL), and NH₃ (aq. 0.3 mL) was added. The mixture was heated to 80°C for 16 hours. After cooling the mixture to room temperature, solvents were removed *in vacuo* and the product was dissolved in MeOH (1.2 ml). Sodium methoxide (11.8 g, 0.22 mmol, 1.00 eq) was added and the mixture was stirred at room temperature for 30 minutes, then stirred with hydrogen exchange resin (CAS : 78922-04-0) for 30 min to PH=7. The resulting mixture was filtered and the filtrate was concentrated *in vacuo.* The residue was purified via silica gel column chromatography with dichloromethane/ methanol (gradient elution: DCM/MeOH from 99:1 to 5:1). The fractions containing the product were collected and the solvent was removed in vacuo to afford ***compound 100a.***

**43% yield** (30 mg, 0.10 mmol), white solid. **¹H NMR** (400 MHz, Methanol-d4) δ 8.27 (s, 1H), 8.22 (s, 1H), 5.98 (d, J = 7.1 Hz, 1H), 5.02 (dd, J = 7.1, 4.4 Hz, 1H), 4.42 (d, J = 4.4 Hz, 1H), 4.17 (t, J = 8.7 Hz, 1H), 2.37 - 2.29 (m, 1H), 2.10 - 2.01 (m, 1H), 1.62 - 1.44 (m, 2H). **¹³C NMR** (75 MHz, MeOD) δ 156.2, 152.3, 148.9, 140.6, 119.7, 91.4, 88.5, 74.8, 73.5, 70.0, 20.7, 20.1 ppm. **LCMS (ESI⁺)** m/z: calcd. for C₁₂H₁₅N₅O₄ [M+H]+ =294.11 found 294.00, RT: 0.799 min (Method 3).

### Preparation of Compound 95

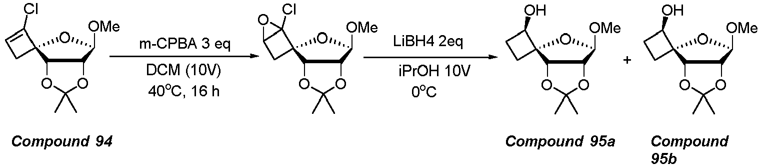

***Compound 94*** (5.00 g, 20.3 mmol, 1.00 eq) was dissolved in DCM (50 mL), then m-CPBA (10.4 g, 60.5 mmol, 3.00 eq) was added at 0°C. The mixture was stirred at 40°C for 16 hours. After the mixture had cooled to room temperature, the solids were removed via filtration, and iPrOH (50 mL) and NaBH₃CN (2.6 g, 40.63 mmol, 2.00 eq) were added at 0°C. The mixture was stirred at 60°C for 2 hours. After the mixture had cooled to room temperature, the residue was added to aqueous saturated NaHCO₃ solution and the aqueous layer was extracted with EtOAc (3 x 300 mL). Combined organic fractions were dried with anhydrous sodium sulphate, filtered and the filtrate was concentrated *in vacuo.* The residue was purified by column chromatography over silica gel (gradient elution: PE/EtOAc from 99:1 to 95:5). Fractions containing the product were collected and the solvent was removed *in vacuo* to afford ***compound 95a*** and ***compound 95b*** as a 44:56 mixture, respectively.

45% yield (2.1 g, 9.13 mmol, 2 steps), colorless oil.

### Preparation of Compound 97

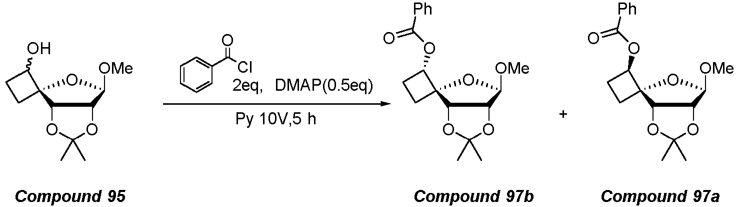

*Compound 95* (2.1 g, 9.13 mmol, 1.00 eq) and DMAP (1.01g, 4.54 mmol, 0.5 eq) were dissolved in pyridine (20 mL), then benzoyl chloride (2.5mg, 17.9 mmol, 2.00 eq) was added dropwise at 0°C and the resulting solution was stirred for 1 hour at 0°C. To the reaction mixture was then added HCl (aqueous, 1M) until PH<7. To the resulting solution was added DCM (200 mL) and H₂O (200 ml). The product was extracted with DCM (3x 200 ml) and combined organic layers were dried (MgSO₄), filtered and the filtrate was concentrated *in vacuo.* The residue was purified by Pre-HPLC (Column: C18 Column, 20-30um; Mobile Phase A: Water (10 mmol/L NH₄HCO₃), Mobile Phase B: ACN; Flow rate: 100 mL/min; Gradient: 30B to 70B in 30 min; 210/254 nm;). The fractions containing the product were collected and the solvent was evaporated to afford *compound **97a*** and *compound 97b.*

***Compound 97b:*** 50% yield (1.5 g, 3.96 mmol), colorless oil. ¹H NMR (300 MHz, Chloroform-d) δ 8.08 (dt, J = 7.1, 1.4 Hz, 2H), 7.59 - 7.53 (m, 1H), 7.48 - 7.42 (m, 2H), 5.14 - 5.06 (m, 1H), 4.92 (d, J = 6.0 Hz, 1H), 4.87 (s, 1H), 4.58 (d, J = 6.0 Hz, 1H), 2.42 - 2.14 (m, 4H), 1.44 (s, 3H), 1.35 (s, 3H) ppm. ¹³C NMR (75 MHz, CDCl₃) δ 166.2, 132.8, 130.3, 129.6, 128.2, 112.2, 109.4, 92.5, 85.4, 84.2,72.9, 55.8, 26.4, 25.3, 24.6, 23.8 ppm.

***Compound 97a:*** 25% yield (1.0 g, 2.64 mmol), colorless oil.

### Preparation of Compound 98b

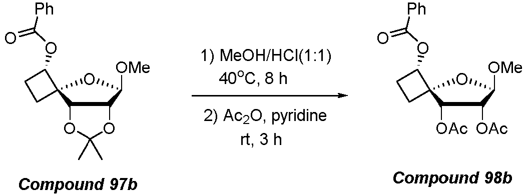

***Compound 97b*** (800 mg, 2.11 mmol, 1.00 eq) was dissolved in MeOH (8 mL) and HCl (2M, 8 mL) was added. The mixture was heated to 35°C for 16 hours. Subsequently, the solution was cooled to room temperature and the filtrate was concentrated *in vacuo.* The residue (700 mg) was dissolved in anhydrous pyridine (7 mL) and stirred for 30 minutes. Acetic anhydride (364 mg, 3.56 mmol, 1.5 eq) was added to the stirring solution at room temperature. The mixture was stirred at room temperature for 3 hours. Subsequently, the mixture was poured into ice-cold water (30 ml) and stirred for 30 min at room temperature. The crude mixture was extracted with CH₂Cl₂ (3 x 30 ml) and the combined organic layers were washed with brine (3 x 200 ml), dried over Na₂SO₄ and concentrated and purified by silica chromatography (gradient elution: PE / EA from 100:1 to 10:1). Fractions containing the product were combined and the solvent was removed *in vacuo* to afford ***compound 98b.***

**43% yield** (2 steps, 390 mg, 1.03 mmol), yellow oil. **¹H NMR** (300 MHz, Chloroform-d) δ 8.13 - 8.09 (m, 2H), 7.58 - 7.53 (m, 1H), 7.47 - 7.41 (m, 2H), 5.64 (d, J = 4.9 Hz, 1H), 5.31 (d, J = 2.0 Hz, 1H), 5.24 (dd, J = 4.9, 2.2 Hz, 1H), 4.96 (d, J = 2.2 Hz, 1H), 3.14 (s, 3H), 2.43 - 2.28 (m, 2H), 2.22 - 2.15 (m, 2H), 2.13 (s, 3H), 2.06 ppm (s, 3H). **¹³C NMR** (75 MHz, CDCl₃) δ 169.7, 169.4, 165.8, 132.9, 130.0, 129.7, 128.3, 105.9, 88.9, 75.5, 74.1, 71.5, 55.7, 25.4, 24.1, 22.8, 20.5 ppm.

### Preparation of Compound 99b

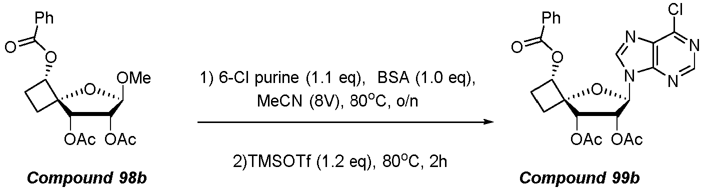

6-Chloro purine (177 mg, 1.13 mmol, 1.10 eq) was dissolved in MeCN (3.2 mL), and N,O-bis(trimethylsilyl)acetamide (208 mg, 1.02 mmol, 1.00 eq) was added dropwise. The mixture was heated to 80°C for 16 hours. After the mixture had cooled, ***compound 98b*** (390 mg, 1.03 mmol, 1.00 eq) in MeCN (2.8 mL) was added, Trimethylsilyltrifluoromethanesulfonate (272 mg, 1.22 mmol, 1.20 eq) was added and the mixture was heated to 80°C for 2 hours. Upon cooling to room temperature, the mixture was diluted with EtOAc, washed with saturated NaHCO₃ and saturated aq. NaCl. The organic layer was dried over Na₂SO₄, filtered and the filtrate was concentrated *in vacuo.* The residue was purified via silica gel column chromatography (gradient elution: PE/EA from 99:1 to 4:1). The fractions containing the product were collected and the solvent was evaporated to afford ***compound 99b.***

**57% yield** (294 mg, 0.58 mmol), white solid. **¹H NMR** (300 MHz, Chloroform-d) δ 8.55 (s, 1H), 8.38 (s, 1H), 7.96 - 7.88 (m, 2H), 7.58 - 7.51 (m, 1H), 7.40 (t, J = 7.7 Hz, 2H), 6.37 (d, J = 7.5 Hz, 1H), 6.17 (dt, J = 7.6, 4.2 Hz, 1H), 5.91 (d, J = 5.0 Hz, 1H), 5.38 (td, J = 5.6, 2.7 Hz, 1H), 2.49 - 2.31 (m, 4H), 2.25 (s, 3H), 2.02 ppm (s, 3H). **¹³C NMR** (101 MHz, CDCl₃) δ 169.7, 169.1, 166.8, 152.2, 152.0, 151.2, 143.6, 133.7, 131.8, 129.6, 128.7, 128.6, 87.2, 84.2, 74.2, 73.3, 72.1, 27.2, 21.8, 20.6, 20.3 ppm. **LCMS (ESI+)** m/z: calcd. for C₂₃H₂₁ClN₄O₇ [M+H]+ =501.11, found 501.10, RT:1.767 min (Method 3).

### Preparation of Compound 100b

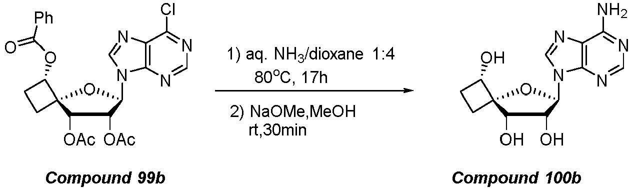

***Compound 99b*** (130 mg, 0.26 mmol, 1.00 eq) was dissolved in 1,4-dioxane (1.2 mL), and ammonia (aq. 0.3 mL) was added. The mixture was heated to 80°C for 16 hours. Upon cooling to room temperature solvents were removed *in vacuo.* The product was dissolved in MeOH (1.2 ml) and sodium methoxide (12 mg, 0.22 mmol, 1.00 eq) was added. The reaction was stirred at room temperature for 30 minutes, followed by stirring with Hydrogen exchange resin (CAS: 78922-04-0) to PH=7 for 30 minutes. The solids were removed by filtration and the filtrate was concentrated under reduced pressure. The residue was purified via silica gel column chromatography (gradient elution: DCM/MeOH from 99:1 to 5:1). The fractions containing the product were collected and the solvent was evaporated to afford ***compound 100b.***

**30% yield** (23 mg, 0.07 mmol), white solid. **¹H NMR** (400 MHz, Methanol-d4) δ 8.34 (s, 1H), 8.21 (s, 1H), 6.07 (d, J = 7.3 Hz, 1H), 4.67 (dd, J = 7.4, 4.1 Hz, 1H), 4.25 (ddt, J = 7.1, 4.7, 1.9 Hz, 1H), 4.07 (d, J = 4.1 Hz, 1H), 2.39 - 2.31 (m, 1H), 2.14 - 2.03 (m, 2H), 1.94 - 1.83 ppm (m, 1H). **¹³C NMR** (101 MHz, MeOD) δ 156.2, 152.2, 148.7, 140.7, 119.6, 89.6, 89.1, 74.6, 74.1, 72.1, 24.4, 24.3 ppm. **LCMS (ESI⁺)** m/z: calcd. for C₁₂H₁₅N₅O₄ [M+H]+ =294.11 found 294.00, RT: 0.660 min (Method 3).

### Preparation of Compound 39

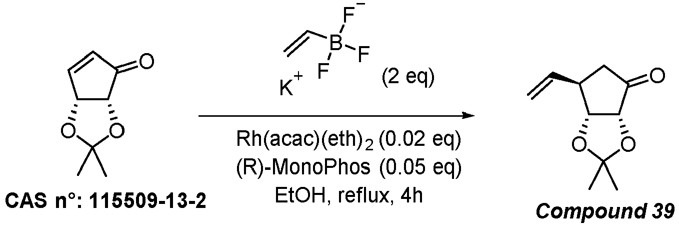

Acetylacetonatobis(ethylene)rhodium(I) (837 mg, 3.24 mmol, 0.02 eq) and (R)-N,N-dimethyldinaphtho[2,1-D:1',2'-F][1,3,2]dioxaphosphepin-4-amine (2.91 g, 8.11 mmol, 0.05 eq) were dissolved in EtOH (625 ml) under nitrogen atmosphere. The mixture was stirred at room temperature and flushed through with nitrogen gas for 15 minutes. Then (-)-(3AR,6AR)-3A,6A-dihydro-2,2-dimethyl-4H-cyclopenta-1,3-dioxol-4-one (25.0 g, 162.16 mmol, 1.00 eq) and potassium vinyltrifluoroborate (45.7 g, 324 mmol, 2.00 eq) were added and the reaction mixture was stirred and refluxed for 4 hours. The reaction mixture (suspension) was cooled down to room temperature. The precipitate was filtered off over a pad of Celite and washed with ethanol. The solvents of the filtrate were evaporated. n-Heptane was added to the residue and the resulting suspension was filtered off over a pad of Celite and washed with heptanes resulting in a dark brown solid residue. The filtrate was washed with NH₄OH (3x 300 ml), washed with brine, dried with MgSO₄, filtered and the filtrate evaporated yielding ***compound 39*** (16.2 g, 51% crude yield).

### Preparation of Compound 40

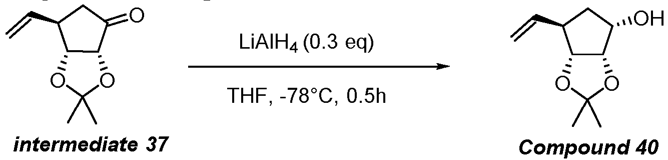

A solution of ***compound 39*** (16.2 g, 82.6 mmol, 1.00 eq) in THF (200 ml) was added dropwise to a stirring solution of lithium aluminum hydride (24.8 ml, 1M in THF, 24.8 mmol, 0.30 eq) in THF (400 ml) at -78°C under nitrogen atmosphere. The reaction mixture was stirred at -78°C under nitrogen atmosphere for 30 minutes. The reaction was quenched by the dropwise addition of acetone (6.1 mL) followed by water (50 ml) at -78°C. After addition, the reaction mixture was warmed to room temperature and EtOAc (400 ml) was added. The mixture was shaken vigorously. The organic layer was separated, washed three times with water, washed with brine, dried (MgSO₄), filtered and the filtrate was evaporated. The residue was purified by column chromatography over silica gel (gradient elution: n-heptane/EtOAc from 1:0 to 1:1). The fractions containing the product were collected and the solvent was evaporated to give the desired ***compound 40*** (10.7 g, 71% yield).

### Preparation of Compound 41

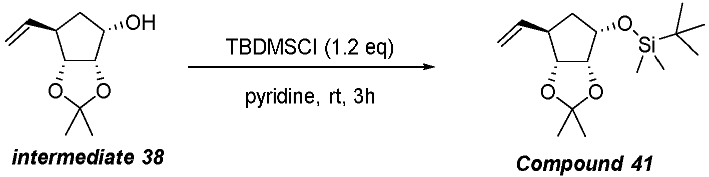

***Compound 40*** (3.10 g, 16.6 mmol, 1.00 eq) was dissolved in pyridine (10.3 ml) and *tert*butyldimethylsilyl chloride (2.88 g, 19.1 mmol, 1.15 eq) was added portionwise at room temperature. The mixture was stirred for 17 hours at room temperature and diluted in EtOAc (250 ml). The organic layer was washed with brine (4x 80 ml), dried (MgSO₄), filtered and the filtrate was concentrated *in vacuo.* The residue was purified by column chromatography over silica gel (gradient elution: n-heptane/EtOAc from 1:0 to 7:3). The fractions containing the product were collected and the solvent was evaporated to give the desired ***compound 41*** (3.15 g, 63% yield) as a slightly yellow oil.

### Preparation of Compound 42

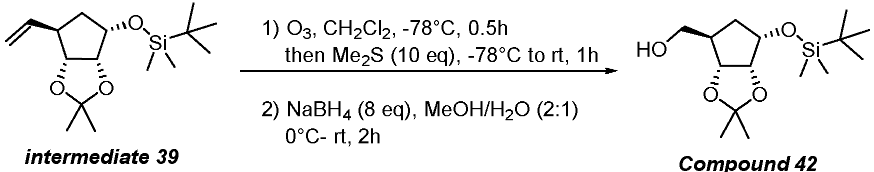

***Compound 41*** (20.1 g, 43.8 mmol, 1.00 eq) was dissolved in CH₂Cl₂ (400 ml) and the mixture was cooled to -78°C. Ozone was generated from oxygen gas with an ozone generater (Fischer OZ500/5) and bubbled in the cooled solution through a glass pipet. A blue color was observed after 1.5 hours and ozone was added for an additional 20 minutes at -78°C. Subsequently, the mixture was flushed with nitrogen for 5 minutes (disappearance of the blue color) and dimethyl sulfide (25.7 ml, 350 mmol, 8.00 eq) was added at -78°C. The flow of nitrogen gas was stopped and the mixture was stirred for 1 hour while the temperature was allowed to increase to -40°C. The mixture was concentrated *in vacuo* at 30°C to a minimal volume and the resulting yellow oil was redissolved in methanol (220 ml) and water (110 ml). The solution was cooled to 0°C and sodium borohydride (19.8 g, 526 mmol, 12.0 eq) was added portionwise. The ice bath was removed after 1.5 hours and stirring was continued at room temperature. After 4 hours stirring the mixture was diluted in CH₂Cl₂ (350 ml) and NH4Cl (aq. sat. 150 ml) was added. The product was extracted in CH₂Cl₂ (3x 350 ml) and combined organic layers were dried (MgSO₄), filtered and the filtrate was concentrated *in vacuo.* The residue was purified by column chromatography over silica gel (gradient elution: n-heptane/EtOAc from 1:0 to 0:1). The fractions containing the product were collected and the solvent was evaporated to give the desired ***compound 42*** (8.30 g, 63% yield) as a slightly yellow oil.
**¹H NMR (400 MHz, Chloroform-*d*):** δ = 4.37-4.42 (m, 2H), 4.10-4.15 (m, 1H), 3.55-3.62 (m, 1H), 3.45-3.53 (m, 1H), 2.16-2.26 (m, 1H), 2.01 (dt, *J*=12.7, 8.2 Hz, 1H), 1.86 (br s, 1H), 1.57-1.66 (m, 1H), 1.49 (s, 3H), 1.31 (s, 3H), 0.91 (s, 9H), 0.09 ppm (d, *J*=2.9 Hz, 6H)
**¹³C NMR (101 MHz, Chloroform-*d*):** δ = 111.7, 82.0, 80.9, 72.7, 64.3, 44.7, 34.4, 26.5, 26.0, 24.9, 18.4, -4.5 ppm

### Preparation of Compound 43

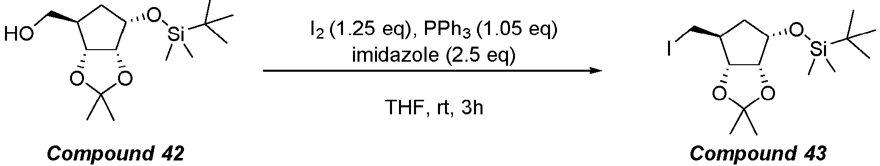

***Compound 42*** [CAS: 514206-18-9] (8.30 g, 27.4 mmol, 1.00 eq) was dissolved in THF (140 ml). Imidazole (4.67 g, 68.6 mmol, 2.50 eq) and triphenylphosphine (8.03 g, 29.1 mmol, 1.05 eq) were added followed by the portionwise addition of iodine (8.79 g, 34.3 mmol, 1.25 eq) at room temperature. After one hour, additional amounts of triphenylphosphine (2.29 g, 8.31 mmol, 0.30 eq) and iodine (2.46 g, 9.60 mmol, 0.35 eq) were added. Reaction was continued for 2 hours, then, the mixture was concentrated to a minimal volume in vacuo and n-heptane (400 ml) was added. Triphenylphosphine-oxides were precipitated and the mixture was sonicated for 30 minutes. The organic layer was separated by filtration and the solids were rinsed with n-heptane (100 ml). To the filtrate was added sodiumthiosulfite (aq. sat. 150 ml) and the product was extracted in n-heptane (3x 400 ml). Combined organic fractions were dried (MgSO₄), filtered and the filtrate was concentrated in vacuo to give ***compound 43*** (9.84 g, 87% calculated yield on HNMR) as a colorless oil.

**¹H NMR (400 MHz, Chloroform-d):** δ = 4.29-4.39 (m, 1H), 4.21 (dd, J=6.1, 2.4 Hz, 1H), 4.05 (dt, J=7.8, 5.0 Hz, 1H), 2.97-3.15 (m, 2H), 2.22-2.38 (m, 1H), 1.96 (dt, J=13.0, 7.7 Hz, 1H), 1.56 (dt, J=13.0, 5.3 Hz, 1H), 1.40 (s, 3H), 1.23 (s, 3H), 0.82 (s, 9H), 0.00 ppm (d, J=2.4 Hz, 6H)

### Preparation of Compound 44

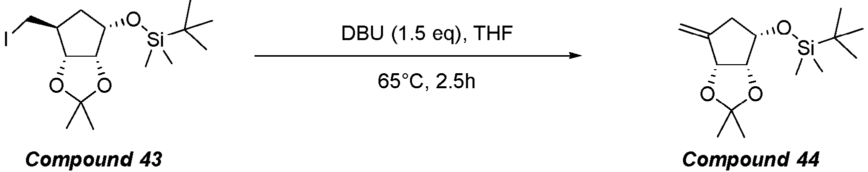

***Compound 43*** (9.84 g, 23.9 mmol, 1.00 eq) was dissolved in THF (168 ml) and 1,8-diazabicyclo [5.4.0]undec-7-ene (5.35 ml, 35.8 mmol, 1.50 eq) was added. The mixture was heated to 65°C for 2.5 hours, then cooled to room temperature. Precipitated DBU-salts were filtered and rinsed with THF and the filtrate was concentrate to a minimal volume in vacuo. Subsequently, n-heptane (400 ml) and brine (100 ml) were added and the product was extracted in n-heptane (3x 400 ml). Combined organic layers were dried (MgSO₄), filtered and the filtrate was concentrated in vacuo. The residue was purified by column chromatography over silica gel (gradient elution: n-heptane/EtOAc from 1:0 to 1:1). The fractions containing the product were collected and the solvent was evaporated to yield the desired ***compound 44*** (6.00 g, 77% yield over 2 steps) as a colorless liquid.
**¹H NMR (400 MHz, Chloroform-*d*):** δ = 5.14-5.20 (m, 1H), 5.11 (dd, J=2.6, 1.0 Hz, 1H), 4.62 (d, J=5.7 Hz, 1H), 4.46 (t, J=5.1 Hz, 1H), 3.90 (ddd, J=11.2, 6.5, 4.7 Hz, 1H), 2.67 (ddtd, J=13.9, 11.1, 2.7, 1.2 Hz, 1H), 2.29-2.35 (m, 1H), 1.50 (s, 3H), 1.35 (s, 3H), 0.92 (s, 9H), 0.11 ppm (d, J=2.8 Hz, 6H)
**¹³C NMR (101 MHz, Chloroform-*d*):** δ = 145.6, 113.6, 111.2, 80.8, 80.4, 72.3, 37.5, 26.4, 26.0, 24.7, 18.4, -4.5 ppm

### Preparation of Compound 45

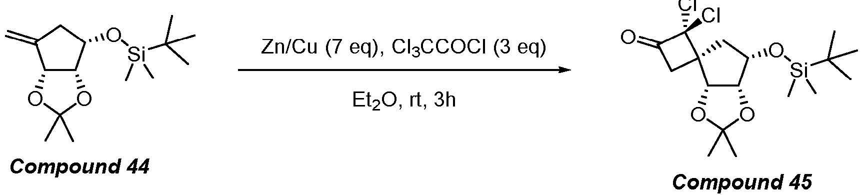

Zinc powder (25.0 g, 0.38 mol, 1.00 eq) was added to a two-necked round bottomed flask (500 ml) containing demineralized water (100 ml) and the solution was degassed with nitrogen during 15 minutes. Subsequently, copper(II)sulfate (1.85 g, 11.5 mmol, 0.03 eq) was added and the stirring solution was degassed and stirred for 45 minutes. The mixture was filtered and the black solids were washed with degassed water (250 ml) and degassed acetone (250 ml), respectively. The zinc-copper couple was dried in vacuo for 12 hours. ***Compound 44*** (2.50 g, 8.79 mmol, 1.00 eq) was dissolved in anhydrous Et₂O (70 ml, dried over 4Å molecular sieves and zinc-copper couple (7.93 g, 61.5 mmol, 7.00 eq) was added. Trichloroacetyl chloride (2.94 ml, 26.4 mmol, 3.00 eq) was dissolved in anhydrous Et₂O (20 ml), loaded in a glass syringe and added dropwise at room temperature with a rate of 6.5 ml/h. After 3 hours, the zinc-copper couple was removed via decantation and the organic layer was diluted in Et2O (500 ml) and washed with NaHCO₃ (aq. sat. 3x 150 ml) and brine (3x 150 ml), dried (MgSO₄), filtered and the filtrate was concentrated in vacuo to yield ***compound 45*** (3.36 g, crude).
**¹H NMR (400 MHz, Chloroform-*d*):** δ = 4.79 (dd, J=5.8, 1.0 Hz, 1H), 4.55 (t, J=5.3 Hz, 1H), 4.11 (dt, J=9.8, 5.1 Hz, 1H), 3.65 (d, J=18.3 Hz, 1H), 3.12 (d, J=18.3 Hz, 1H), 2.36 (dd, J=12.9, 5.4 Hz, 1H), 2.15 (dd, J=12.9, 9.8 Hz, 1H), 1.48 (s, 3H), 1.37 (s, 3H), 0.91-0.93 (m, 9H), 0.12 ppm (d, J=2.2 Hz, 6H)
**¹³C NMR (101MHz, Chloroform-*d*):** δ = 191.8, 112.3, 80.7, 80.1, 71.5, 51.7, 26.1, 25.9, 18.4, -4.6, -4.9 ppm

### Preparation of Compound 46

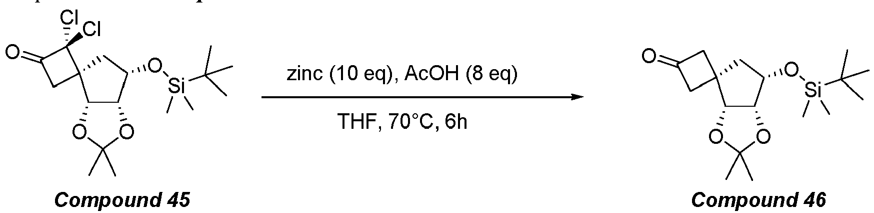

***Compound 45*** (1.25 g, 3.16 mmol, 1.00 eq) was dissolved in THF (30 ml) and zinc (2.07 g, 31.6 mmol, 10.0 eq) and acetic acid (1.45 ml, 25.3 mmol, 8.00 eq) were added. The mixture was heated to 70°C for 6 hours and then cooled to room temperature. The mixture was filtered over celite, the solids were rinsed with THF and the filtrate was concentrated to a minimal volume in vacuo. Subsequently, the oil was redissolved in CH₂Cl₂ (100 ml) and brine (50 ml) was added. The product was extraced in CH₂Cl₂ (3x 100ml) and combined organic fractions were dried (MgSO₄), filtered and the filtrate was concentrated in vacuo. The residue was purified by column chromatography over silica gel (gradient elution: n-heptane/EtOAc from 1:0 to 2:3). The fractions containing the product were collected and the solvent was evaporated to yield the desired ***compound 46*** (0.648 mg, 63% yield over 2 steps) as a colorless oil.
**¹H NMR (400 MHz, Chloroform-*d*):** δ = 4.49 (t, J=5.1 Hz, 1H), 4.29-4.34 (m, 1H), 3.88 (dt, J=10.9, 5.3 Hz, 1H), 3.36 (ddd, J=18.3, 4.1, 2.4 Hz, 1H), 2.89-2.99 (m, 1H), 2.79-2.87 (m, 1H), 2.68-2.75 (m, 1H), 2.19 (t, J=11.4 Hz, 1H), 1.82 (dd, J=11.8, 5.7 Hz, 1H), 1.58 (s, 1H), 1.48 (s, 3H), 1.34 (s, 3H), 0.90-0.94 (m, 9H), 0.11 ppm (d, J=2.8 Hz, 6H)
**¹³C NMR (101 MHz, Chloroform-*d*):** δ = 206.0, 111.1, 85.2, 80.2, 72.3, 56.7, 52.8, 41.3, 34.4, 26.0, 26.0, 24.5, 18.4, -4.4, -4.7 ppm

### Preparation of Compound 47

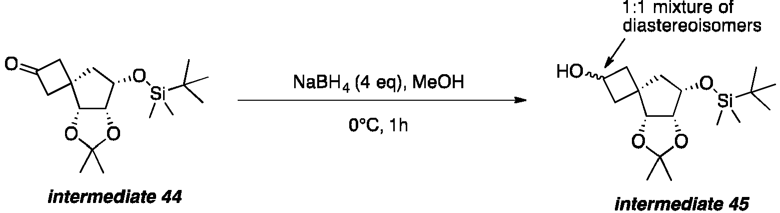

***Compound 46,*** also labelled ***intermediate* 44** (600 mg, 1.84 mmol, 1.00 eq) was dissolved in methanol (20.0 ml) and cooled to 0°C. Sodium borohydride (282 mg, 7.35 mmol, 4.00 eq) was added portionwise and the mixture was stirred for 1 hour at 0°C. The solution was concentrated to a minimal volume *in vacuo* and dissolved in CH₂Cl₂ (100 ml) and NH₄Cl (sat. aq. 50 ml) was added. The product was extracted in CH₂Cl₂ (3x 100 ml) and combined organic layers were dried (MgSO₄), filtered and the filtrate was concentrated in vacuo to yield ***compound 47,*** also labelled ***intermediate 45*** (515 mg, 85 % yield) in a 1:1 mixture.

### Preparation of Compound 48

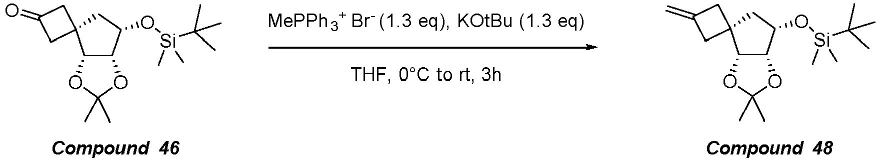

Methyltriphenylphosphonium bromide (1.45 g, 3.98 mmol, 1.30 eq) was weighed in an oven dried vial and THF (12.0 ml) was added. The heterogeneous solution was cooled to 0°C and potassium tert-butoxide (3.98 ml, 1M in THF, 3.98 mmol, 1.30 eq) was added dropwise. The mixture was stirred at 0°C for 20 minutes. The freshly prepared wittig reagent was added dropwise via syringe to ***compound 46*** (1.00 g, 3.06 mmol, 1.00 eq) dissolved in THF (12.0 ml) at 0°C. The yellow mixture was stirred for 1.5 hours at 0°C and then 1.5 hours at room temperature. The mixture was concentrated to a minimal volume in vacuo and redissolved in n-heptane (300 ml). Triphenylphosphine-oxides were precipitated and the mixture was sonicated for 5 minutes, filtered and the filtrate was washed with NH4Cl (aq. sat. 2x 50 ml) and brine (2x 50 ml). The organic layer was dried (MgSO₄), filtered and the filtrate was concentrated in vacuo. The residue was purified by column chromatography over silica gel (gradient elution: n-heptane/EtOAc from 1:0 to 7:3). The fractions containing the product were collected and the solvent was evaporated to yield the desired ***compound 48*** (931 mg, 94% yield).
**¹H NMR (400 MHz, Chloroform-*d*):** δ = 4.81 (quin, J=2.4 Hz, 1H), 4.78 (quin, J=2.4 Hz, 1H), 4.40 (t, J=5.1 Hz, 1H), 4.26 (dd, J=5.5, 0.9 Hz, 1H), 3.80 (dt, J=11.2, 5.5 Hz, 1H), 2.87 (dd, J=16.1, 2.2 Hz, 1H), 2.54 (dq, J=15.9, 2.4 Hz, 1H), 2.31-2.46 (m, 2H), 1.95 (t, J=11.4 Hz, 1H), 1.76 (dd, J=11.7, 5.7 Hz, 1H), 1.45 (s, 3H), 1.32 (s, 3H), 0.91 (s, 9H), 0.10 ppm (d, J=2.2 Hz, 6H)
**¹³C NMR (101 MHz, Chloroform-*d*):** δ = 144.7, 110.4, 106.8, 85.3, 79.9, 72.0, 42.5, 41.2, 39.2, 36.8, 26.0, 26.0, 24.5, 18.4, -4.4, -4.6 ppm

### Preparation of Compound 49

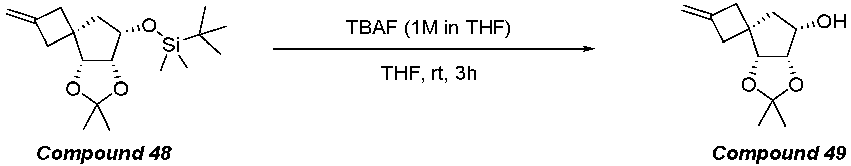

***Compound 48*** (931 mg, 2.87 mmol, 1.00 eq) was dissolved in THF (2.00 ml) and tetrabutylammonium fluoride (10.0 ml, 1M in THF, 10.0 mmol, 3.50 eq) was added. The mixture was stirred at room temperature for 3 hours. The mixture was concentrated to a minimal volume in vacuo, dissolved in EtOAc (250 ml) and washed with NH₄Cl (aq. sat. 3x 50ml) and brine (3x 50 ml). The organic layer was dried (MgSO₄), filtered and the filtrate was concentrated in vacuo. The residue was purified by column chromatography over silica gel (gradient elution: n-heptane/EtOAc from 1:0 to 0:1). The fractions containing the product were collected and the solvent was evaporated to yield the desired ***compound 49*** (556 mg, 92% yield)
**¹H NMR (400 MHz, Chloroform-*d*):** δ = 4.83 (quin, J=2.3 Hz, 1H), 4.80 (quin, J=2.4 Hz, 1H), 4.46-4.49 (m, 1H), 4.36-4.39 (m, 1H), 3.81 (br s, 1H), 2.82-2.88 (m, 1H), 2.60 (dq, J=16.1, 2.4 Hz, 1H), 2.38-2.46 (m, 2H), 2.27-2.38 (m, 1H), 1.97 (dd, J=12.0, 5.9 Hz, 1H), 1.74 (t, J=11.4 Hz, 1H), 1.47 (s, 3H), 1.36 ppm (s, 3H)
**¹³C NMR (101 MHz, Chloroform-*d*):** δ = 144.1, 110.6, 107.2, 85.4, 78.7, 70.8, 41.7, 41.2, 39.5, 36.6, 25.9, 24.3 ppm

### Preparation of Compound 50

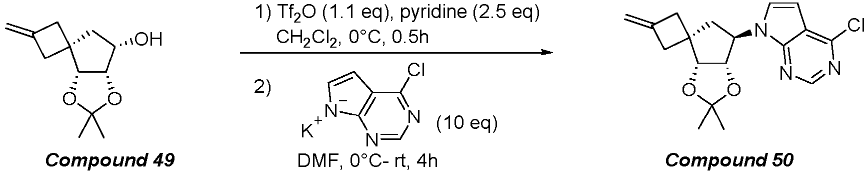

Potassium 6-chloro-7-deazapurine was prepared as follows. A mixture of 4-chloro-7H-pyrrolo[2,3-D]pyrimidine, CAS 3680-69-1 (100 g, 651 mmol, 1.00 eq) and KOtBu (73.1 g, 651 mmol, 1.00 eq) in THF (1l) was stirred at room temperature for 45 minutes until a clear solution was obtained. The solvents were evaporated. The residue was triturated in DIPE. The white solids were filtered off and dried *in vacuo* at 30°C yielding potassium 6-chloro-7-deazapurine (113 g, 90% yield).

***Compound 49*** (647 mg, 3.08 mmol, 1.00 eq) was dissolved in anhydrous CH₂Cl₂ (20.0 ml) and pyridine (0.62 ml, 7.69 mmol, 2.50 eq) was added. The mixture was cooled to 0°C and trifluoromethanesulfonic anhydride (0.57 ml, 3.39 mmol, 1.10 eq) was added dropwise. The mixture was stirred for 30 minutes at 0°C, diluted in CH₂Cl₂ (100 ml) and NaHCO₃ (aq. sat. 40 ml) was added. The product was extracted in CH₂Cl₂ (3x 100 ml) and combined organic layers were dried (MgSO₄), filtered and the filtrate was concentrated in vacuo and used immediately in the part of the procedure.

Potassium 6-chloro-7-deazapurine (5.90 g, 30.8 mmol, 10.0 eq) was dissolved in anhydrous DMF (35.0 ml) and stirred for 30 minutes at 0°C. This was followed by the dropwise addition of the crude triflate (1.05 g, 3.08 mmol, 1.00 eq) dissolved in anhydrous DMF (8.00 ml) over 15 min at 0°C. The mixture was stirred for 2 hours at 0°C and then warmed to room temperature and stirred for an additional 2 hours. The mixture was poured in NH4Cl (aq. sat. 50 ml) and the product was extracted in EtOAc (3x 100 ml). Combined organic layers were washed with brine (3x 100 ml), dried (MgSO₄), filtered and the filtrate was concentrated in vacuo to a minimal volume. To the resulting powder, n-heptane (100 ml) was added and the mixture was sonicated for 10 minutes. The solids were filtered, rinsed with n-heptane and the filtrate was concentrated to a minimal volume in vacuo. The residue was purified by column chromatography over silica gel (gradient elution: n-heptane/EtOAc from 1:0 to 0:1). The fractions containing the product were collected and the solvent was evaporated to yield the desired ***compound 50*** (856 mg, 80% over 2 steps).
**¹H NMR (400 MHz, Chloroform-*d*):** δ = 8.64-8.65 (m, 1H), 7.18 (d, J=3.7 Hz, 1H), 6.61 (d, J=3.7 Hz, 1H), 5.10 (dd, J=6.3, 3.1 Hz, 1H), 4.96 (td, J=6.8, 3.1 Hz, 1H), 4.80 (dquin, J=18.2, 2.4 Hz, 2H), 4.69 (d, J=6.5 Hz, 1H), 3.18 (dd, J=15.5, 2.4 Hz, 1H), 2.75 (dd, J=15.3, 2.6 Hz, 1H), 2.48-2.58 (m, 2H), 2.33-2.44 (m, 2H), 1.55 (s, 3H), 1.35 ppm (s, 3H)
**¹³C NMR (101 MHz, Chloroform-*d*):** δ = 152.3, 150.6, 143.4, 127.5, 117.9, 112.7, 107.0, 99.8, 85.7, 84.9, 61.5, 43.0, 42.3, 42.2, 38.3, 26.6, 24.7 ppm

### Preparation of Compound 51

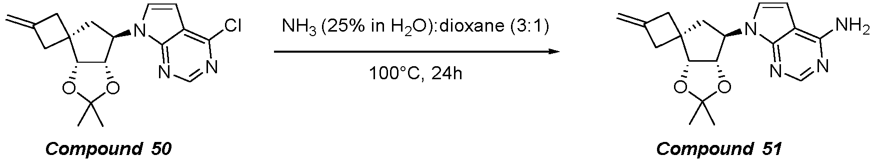

***Compound 50*** (850 mg, 2.46 mmol, 1.00 eq) was dissolved in 1,4-dioxane (20.0 ml) NH3 (60.0 ml, 25% in H₂O) was added. The solution was heated to 100°C for 24 hours in a pressure reactor. The mixture was concentrated to a minimal volume in vacuo and coevaporated twice with toluene. The residue was purified by column chromatography over silica gel (gradient elution: CH₂Cl₂/MeOH from 1:0 to 7:3). The fractions containing the product were collected and the solvent was evaporated to yield the desired ***compound 51*** (790 mg, 98% yield).
**¹H NMR (400 MHz, Chloroform-*d*):** δ = 8.33 (s, 1H), 6.89 (d, J=3.7 Hz, 1H), 6.35 (d, J=3.5 Hz, 1H), 5.18 (br s, 2H), 5.09 (dd, J=6.4, 2.9 Hz, 1H), 4.93 (td, J=6.7, 2.9 Hz, 1H), 4.81 (quin, J=2.4 Hz, 1H), 4.76 (quin, J=2.4 Hz, 1H), 4.67 (d, J=6.4 Hz, 1H), 3.16 (dd, J=15.6, 2.4 Hz, 1H), 2.74 (dd, J=15.2, 2.4 Hz, 1H), 2.45-2.55 (m, 2H), 2.30-2.45 (m, 2H), 1.97 (br s, 1H), 1.55 (s, 3H), 1.35 ppm (s, 3H)
**¹³C NMR (101 MHz, Chloroform-*d*):** δ = 156.6, 151.7, 150.5, 143.8, 123.0, 112.4, 106.8, 103.5, 97.6, 85.9, 85.1, 60.8, 43.1, 42.5, 42.3, 38.3, 26.6, 24.7 ppm

### Preparation of Compounds 52, 52a and 52b

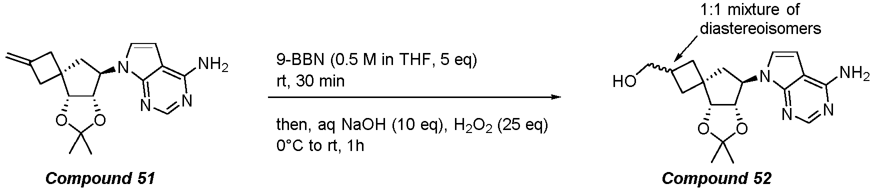

To ***compound 51*** (255 mg, 0.78 mmol, 1.00 eq) was added 9-borabicyclo[3.3.1]nonane (7.81 ml, 0.5M in THF, 3.91 mmol, 5.00 eq) and the mixture was stirred for 30 minutes at room temperature. The solution was cooled to 0°C and NaOH (7.81 ml, 1M in H₂O, 7.81 mmol, 10.0 eq) was added followed by the dropwise addition of hydrogenperoxide (1.99 ml, 30% in H₂O, 19.5 mmol, 25.0 eq). The mixture was stirred for 1 hour at room temperature, then diluted in CH₂Cl₂ (250 ml) and washed with NaHCO₃ (aq. sat. 3x 50 ml) and brine (1x 50 ml). The organic layer was dried (MgSO₄), filtered and the filtrate was concentrated in vacuo. The residue was purified by column chromatography over silica gel (gradient elution: CH₂Cl₂/MeOH from 1:0 to 7:3). The fractions containing the product were collected and the solvent was evaporated to yield ***compound 52*** (213 mg, 79% yield) as a 1:1 mixture of diastereoisomers. A purification was performed on a sample of intermediate 72 via prep SFC (stationary phase: Chiralcel Diacel OJ 20 x 250 mm, mobile phase: CO2, EtOH + 0.4% iPrNH2) to yield ***compound 52a*** (15 mg) and ***compound 52b*** (18 mg). 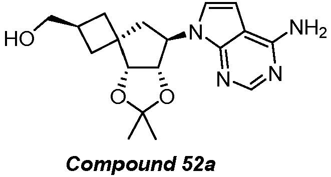
**¹H NMR (400MHz, Chloroform-*d*):** δ = 8.31 (s, 1H), 6.89 (d, J=3.5 Hz, 1H), 6.34 (d, J=3.5 Hz, 1H), 5.24 (br s, 2H), 5.02 (dd, J=6.8, 3.5 Hz, 1H), 4.55 (d, J=6.8 Hz, 1H), 2.35-2.52 (m, 3H), 2.27 (dd, J=11.8, 8.3 Hz, 1H), 1.76-1.95 (m, 3H), 1.53 (s, 3H), 1.33 ppm (s, 3H)
**¹³C NMR (101MHz. Chloroform-*d*):** δ = 156.7, 151.7, 150.4, 123.1, 112.7, 97.7, 86.2, 84.6, 66.9, 60.3, 43.4, 43.3, 34.1, 31.6, 30.4, 26.5, 24.8 ppm 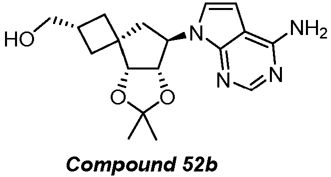
**¹H NMR (400MHz, Chloroform-*d*):** δ = 8.31 (s, 1H), 6.88 (d, J=3.7 Hz, 1H), 6.32 (d, J=3.7 Hz, 1H), 5.29 (br s, 1H), 5.00 (dd, J=6.3, 2.8 Hz, 1H), 4.86-4.94 (m, 1H), 4.66 (d, J=6.4 Hz, 1H), 3.50-3.64 (m, 2H), 2.46-2.58 (m, 1H), 2.36-2.46 (m, 2H), 2.20 (dd, J=13.6, 5.9 Hz, 1H), 2.05-2.15 (m, 2H), 1.64-1.74 (m, 2H), 1.54 (s, 3H), 1.35 ppm (s, 3H)
**¹³C NMR (101MHz, Chloroform-*d*):** δ = 156.7, 151.7, 150.4, 122.7, 112.3, 97.7, 86.6, 85.4, 66.9, 60.5, 43.7, 43.5, 35.4, 31.5, 30.2, 26.6, 24.7 ppm

### Preparation of Compound 53

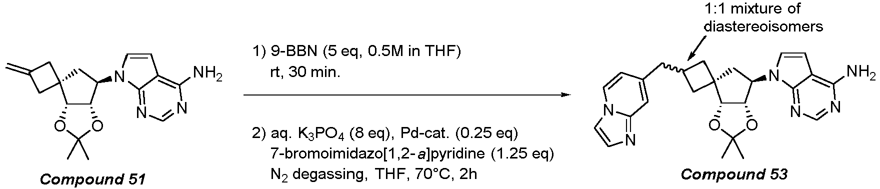

To ***compound 51*** (60 mg, 0.18 mmol, 1.00 eq) was added 9-borabicyclo[3.3.1]nonane (0.5M in THF, 1.84 ml, 0.92 mmol, 5.00 eq) at room temperature. The mixture was stirred for 30 minutes. Subsequently, potassium phosphate (312 mg, 1.47 mmol, 8.00 eq) dissolved in water (0.58 ml, 32.0 mmol, 174 eq) was degassed with nitrogen for 10 minutes and added to the reaction mixture. The solution was stirred for 10 minutes at room temperature with degassing and 7-bromoimidazo[1,2-*a*]pyridine [CAS: 808744-34-5] (54.3 mg, 0.28 mmol, 1.50 eq) and 1,1'-bis(di-tert-butylphosphino)ferrocene palladium dichloride [CAS: 95408-45-0] (30.2 mg, 0.05 mmol, 0.25 eq) dissolved in THF (2.4 ml) was added to the mixture. Degassing with nitrogen was continued for 15 minutes before the mixture was heated to 70°C. After 2 hours, the dark brown solution was cooled to room temperature, diluted with EtOAc (90 ml), washed with NH4OH (25% in H₂O, 2x 30 ml) and brine (2x 30 ml). The organic layer was dried (MgSO₄), filtered and the filtrate was concentrated in vacuo to yield ***compound 53*** (219 mg, crude) as a 1:1 mixture of diastereoisomers used without purification in the next step.

### Preparation of Compounds 54a and 54b

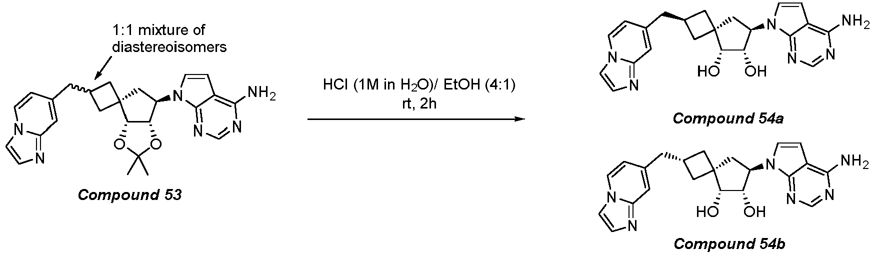

***Compound 53*** (crude from previous step) was dissolved in EtOH (4.00 ml) and HCl (16.0 ml, 1M in H₂O) was added and the mixture was stirred at room temperature for 2 hours. The solution was diluted with water (20 ml), frozen and lyophilized to give a solid residue. A purification was performed via prep SFC (stationary phase: Chiralcel Diacel OJ 20 x 250 mm, mobile phase: CO₂, EtOH + 0.4% iPrNH₂) to yield ***compound 54a*** (14.5 mg, 0.03 mmol, 19% yield over 2 steps) and ***compound 54b*** (15.0 mg, 0.04 mmol, 20% yield over 2 steps).

### Compound 54a:

**¹H NMR (400MHz, DMSO-d6):** δ = 8.42 (d, J=6.9 Hz, 1H), 8.01 (s, 1H), 7.84 (s, 1H), 7.49 (s, 1H), 7.30 (s, 1H), 7.16 (d, J=3.3 Hz, 1H), 6.88 (s, 2H), 6.73 (d, J=6.9 Hz, 1H), 6.53 (d, J=3.3 Hz, 1H), 4.71-4.88 (m, 3H), 4.17-4.25 (m, 1H), 3.71 (br t, J=4.3 Hz, 1H), 2.71 (br d, J=7.7 Hz, 2H), 2.52-2.59 (m, 1H), 2.35 (dd, J=13.0, 9.8 Hz, 1H), 2.01-2.17 (m, 2H), 1.92 (dd, J=13.0, 8.5 Hz, 1H), 1.77-1.86 (m, 1H), 1.68-1.77 ppm (m, 1H).

**¹³C NMR (101MHz, DMSO-d6):** δ = 157.4, 151.2, 149.8, 144.8, 137.8, 132.8, 126.2, 122.3, 114.6, 113.7, 112.3, 102.7, 98.6, 77.9, 75.5, 59.4, 42.2, 41.6, 41.2, 38.0, 34.1, 29.7 ppm. **LCMS** (ESI+): [M+1]+= 405.3, RT 1.15 min (Method 9)

### Compound 54b:

**¹H NMR (400MHz, DMSO-d6):** δ = 8.42 (d, J=6.9 Hz, 1H), 8.01 (s, 1H), 7.84 (s, 1H), 7.49 (s, 1H), 7.29 (s, 1H), 7.12 (d, J=3.3 Hz, 1H), 6.88 (s, 2H), 6.73 (d, J=6.9 Hz, 1H), 6.54 (d, J=3.7 Hz, 1H), 4.77-4.89 (m, 3H), 4.24-4.33 (m, 1H), 3.79 (t, J=3.9 Hz, 1H), 3.18 (d, J=4.5 Hz, 1H), 2.70 (br d, J=6.5 Hz, 2H), 2.47 (br d, J=4.1 Hz, 1H), 2.29 (dd, J=13.6, 10.4 Hz, 1H), 2.08-2.19 (m, 1H), 1.74-1.88 (m, 2H), 1.45-1.56 ppm (m, 1H).

**¹³C NMR (101MHz, DMSO-d6):** δ = 157.4, 151.2, 150.0, 144.9, 137.8, 132.8, 126.2, 121.9, 114.6, 113.8, 112.3, 102.7, 98.7, 77.4, 75.7, 58.7, 42.6, 41.9, 40.3, 33.9, 29.6 ppm.

**LCMS** (ESI+): [M+1]+= 405.2, RT 1.13 min (Method 9)

### Preparation of Compounds 55a and 55b

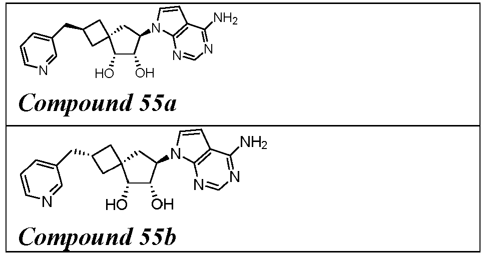

***Compounds 55a and 55b*** were prepared by a process analogous to that used for the preparation of ***Compounds 54a*** and ***54b.***

### Compound 55a

**¹H NMR** (DMSO-d₆, 400MHz): δ = 8.45 (d, *J*=4.0 Hz, 1H), 8.01 (s, 1H), 7.66 (td, *J*=7.6, 1.8 Hz, 1H), 7.11-7.23 (m, 3H), 6.87 (br s, 2H), 6.53 (d, *J*=3.5 Hz, 1H), 4.66-4.88 (m, 3H), 4.14-4.24 (m, 1H), 3.67-3.73 (m, 1H), 3.17 (d, *J*=3.5 Hz, 1H), 2.81 (d, *J*=7.7 Hz, 2H), 2.54-2.69 (m, 1H), 2.33 (dd, *J*=13.0, 9.7 Hz, 1H), 2.10 (dd, *J=11.2,* 8.8 Hz, 1H), 1.98-2.05 (m, 1H), 1.90 (dd, *J=13.0,* 8.6 Hz, 1H), 1.81 (br dd, *J=10.7,* 8.7 Hz, 1H), 1.71 ppm (ddd, *J=11.3,* 7.9, 3.5 Hz, 1H)
¹³C NMR (DMSO-d₆, 101MHz): δ = 160.9, 157.9, 151.7, 150.4, 149.4, 136.7, 123.1, 122.7, 121.6, 103.2, 99.1, 78.4, 76.0, 59.9, 45.1, 42.8, 41.7, 38.6, 34.7, 29.8 ppm **LCMS** (ESI+): [M+1]+= 366.3, RT 1.20 min (Method 9)

### Compound 55b

**¹H NMR** (DMSO-d₆, 400MHz): δ = 8.45 (d, *J*=4.0 Hz, 1H), 8.01 (s, 1H), 7.66 (td, *J*=7.6, 1.8 Hz, 1H), 7.14-7.24 (m, 2H), 7.10 (d, *J*=3.5 Hz, 1H), 6.86 (s, 2H), 6.53 (d, *J*=3.5 Hz, 1H), 4.73-4.90 (m, 3H), 4.24-4.33 (m, 1H), 3.77 (t, *J*=4.0 Hz, 1H), 2.80 (d, *J*=7.5 Hz, 2H), 2.51-2.60 (m, 1H), 2.43 (tt, *J*=7.4, 4.0 Hz, 1H), 2.26 (dd, *J*=13.6*,* 10.3 Hz, 1H), 2.11 (ddd, J=11.2, 7.6, 4.1 Hz, 1H), 1.73-1.85 (m, 2H), 1.50 ppm (dd, *J*=11.0*,* 8.6 Hz, 1H)
¹³C NMR (DMSO-d₆, 101MHz): δ = 160.9, 157.9, 151.7, 150.5, 149.4, 136.7, 123.2, 122.4, 121.6, 103.2, 99.2, 77.9, 76.2, 59.3, 55.4, 45.4, 43.1, 42.4, 40.9, 34.4, 29.7 ppm **LCMS** (ESI+): [M+1]+= 366.3, RT 1.20 min (Method 9)

### Preparation of Compound 56

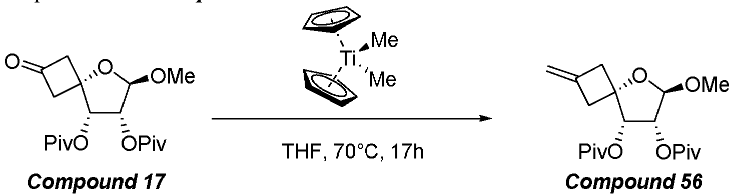

***Compound 17*** (2.00 g, 5.50 mmol, 1.00 eq) was weighed in a three neck 100 ml flask equipped with a reflux condenser, thermometer and a CaCl₂ tube. To the substrate was added a solution (5 wt% in toluene) of bis(cyclopentadienyl)dimethyltitanium (39.4 mL, 7.97 mmol, 1.45 eq, CAS: 1271-66-5). The flask was covered from light with aluminium foil and heated to 70°C. [Note: upon heating, the active Petasis reagent is generated and 1 equivalent of methane gas relative to the titanocene is liberated. Therefore, closed systems should be avoided for reaction setup in glassware. Additionally, reaction in metal pressurized reactors did show only low conversions, as the titanocene reagent sticks to the reactor walls.] The reaction was stirred for 17 hours after which full conversion was observed. The mixture was concentrated to a minimal volume *in vacuo* and to the residue was added *n*-heptane (100 ml). The solids were sonicated for 5 minutes and removed via filtration over Celite (rinsed with n-heptane). The organic layer was concentrated to a minimal volume *in vacuo.* The residue was purified by column chromatography over silica gel (gradient elution: n-heptane /EtOAc from 1:0 to 3:7 in 15 column volumes). The fractions containing the product were collected and the solvent was evaporated to afford ***compound 56*** (58% yield, 1.14 g; 3.19 mmol, colorless oil).
**¹H NMR (500 MHz, Chloroform-*d*):** δ 5.42 (d, *J=* 4.5 Hz, 1H), 5.17, (dd, *J=* 4.5, 1.7 Hz, 1H), 4.85-4.83 (m, 3H), 3.39 (s, 3H), 3.09-3.05 (m, 2H), 2.94-2.85 (m, 2H), 1.21 (s, 9H), 1.20 ppm (s, 9H).
**¹³C NMR (125 MHz, Chloroform-*d*):** δ 177.4, 177.2, 139.7, 107.6, 105.7, 80.6, 75.6, 74.5, 55.6, 44.8, 41.5, 39.2, 39.0, 27.4, 27.3 ppm.

### Preparation of Compound 57

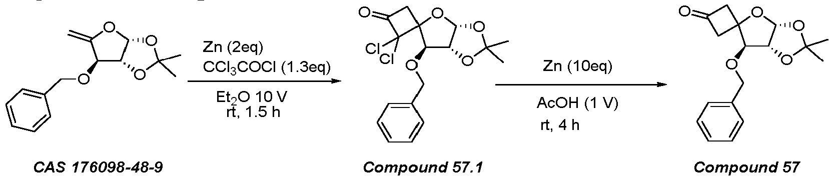

CAS 176098-48-9 (1.8 g, 6.87 mmol, 1 eq) was dissolved in Et₂O (18 mL), zinc (0.89 g, 13.7 mmol, 2.00 eq) was added. This was followed by the addition of a solution of trichloroacetyl chloride (1.6 g, 8.9 mmol, 1.3 eq) in Et₂O (6 mL) dropwise with stirring at 0 °C. The resulting solution was stirred for 2 h at r.t. Then zinc (4.47 g, 68.7 mmol, 10.0 eq), AcOH (1.8 mL) was added at 0 °C, the mixture was stirred at r.t for 3 h. The residue was added to saturated NaHCO₃ aqueous (50 mL) at 0 °C, the solids were filtered out, and the aqueous phase was extracted with EA (50 mL), the organic phase was dried over anhydrous sodium sulfate and concentrated *in vacuo.* The residue was purified by column chromatography over silica gel (gradient elution: PE/EtOAc from 99:1 to 5:1). The fractions containing the product were collected and the solvent was evaporated to afford the title ***compound 57.***

**55% yield** (2.0 g, 7.63 mmol), yellow oil. **¹H NMR (400 MHz Chloroform-*d*)** δ 7.39 - 7.31 (m, 5H), 5.99 (d, J = 4.0 Hz, 1H), 4.79 - 4.73 (m, 2H), 4.56 (d, J = 11.8 Hz, 1H), 4.00 (s, 1H), 3.50 - 3.35 (m, 3H), 3.28 - 3.19 (m, 1H), 1.45 (s, 3H), 1.33 ppm (s, 3H). **¹³C NMR (101 MHz, Chloroform-*d*)** δ 204.58, 136.96, 128.69, 128.26, 127.83, 111.90, 105.70, 85.25, 82.92, 78.60, 71.91, 59.13, 54.53, 26.26, 25.75 ppm. **LCMS** (ESI+) m/z: calcd. for C₁₅H₁₈O₄ [M+H]⁺ = 305.13, found 305.15, RT: 0.887 min, Method 1.

### Preparation of Compound 58

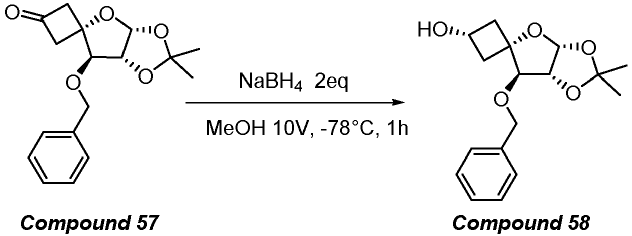

***Compound 57*** (900 mg, 2.96 mmol, 1 eq) was dissolved in MeOH (9 mL), then NaBH₄ (225 mg, 5.92 mmol, 2 eq) was added at -78 °C, The resulting solution was stirred for 1 h at -78 °C. The mixture was added to saturated NH₄Cl aqueous (50 mL) at 0 °C, and then extracted with EA (50 mL), the organic phase was dried over anhydrous sodium sulfate and concentrated *in vacuo.* The residue was purified by column chromatography over silica gel (gradient elution: PE/EtOAc from 99:1 to 70:30). The fractions containing the product were collected and the solvent was evaporated to afford the title ***compound 58.***

**55% yield** (550 mg, 1.63 mmol), colorless oil. **¹H NMR (300 MHz, Chloroform-*d*)** δ 7.41 - 7.31 (m, 5H), 5.86 (d, J = 4.0 Hz, 1H), 4.76 (d, J = 12.2 Hz, 1H), 4.64 (d, J = 4.0 Hz, 1H), 4.52 (d, J = 12.2 Hz, 1H), 3.87 - 3.74 (m, 2H), 2.92 (dt, J = 12.6, 6.4 Hz, 1H), 2.74 (dt, J = 12.3, 6.3 Hz, 1H), 2.24 (ddd, J = 11.4, 7.5, 3.4 Hz, 2H), 1.80 (s, 1H), 1.47 (s, 3H), 1.31 ppm (s, 3H). **¹³C NMR (101 MHz, Chloroform-*d*)** δ 137.37, 128.58, 128.06, 127.80, 111.78, 104.90, 84.72, 83.18, 79.04,, 71.68, 59.85, 46.63, 40.53, 26.52, 25.94 ppm, **LCMS** (ESI+) m/z: calcd. for C₁₇H₂₂O₅ [M+H]⁺ = 307.15, found 307.20, RT: 0.734 min, Method 1.

### Preparation of Compound 59

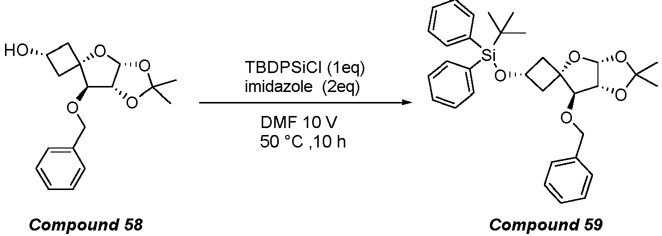

***Compound 58*** (1.96 mmol, 600 mg, 1 eq.) was dissolved in DMF (6 mL), imidazole (267 mg, 3.92 mmol, 2 eq) and TBDPSiCl (537 mg, 1.96 mmol, 1 eq) was added, the mixture was stirred at 50 °C for 10 h. The mixture was cooled down to room temperature and poured in EtOAc (10 mL) followed by washing with brine (3 x 10 mL). The organic phase was dried over MgSO₄, filtered and evaporated to a minimum. An additional washing step after dissolving the obtained residue in EtOAc (10 mL) with brine (5 x 10 mL) was required to remove the remaining DMF. The residue was purified by column chromatography over silica gel (PE/EtOAc from 99:1 to 90: 10). The fractions containing the product were collected and the solvent was evaporated to afford ***compound 59.***

**56% yield** (600 mg, 1.10 mmol), colorless oil. **¹H NMR (400 MHz, Chloroform-*d*)** δ 7.74 - 7.59 (m, 6H), 7.44 - 7.31 (m, 9H), 5.81 (d, J = 4.0 Hz, 1H), 4.64 - 4.53 (m, 2H), 4.36 (d, J = 12.2 Hz, 1H), 3.76 (p, J = 7.1 Hz, 1H), 3.54 (s, 1H), 2.69 (dt, J = 12.5, 6.4 Hz, 1H), 2.50 (dt, J = 12.4, 6.4 Hz, 1H), 2.36 (td, J = 11.2, 7.6 Hz, 2H), 1.42 (s, 3H), 1.26 (s, 3H), 1.01 ppm (s, 9H). **¹³C NMR (101 MHz, Chloroform-*d*)** δ 135.52, 135.50, 134.83, 129.67, 129.65, 129.61, 128.45, 127.89, 127.74, 127.64, 127.62, 127.59, 111.69, 104.81, 84.56, 83.26, 79.36, 71.55, 60.47, 46.68, 40.66, 26.71, 26.59, 26.56 ppm, **LCMS** (ESI+) m/z: calcd. for C₃₃H₄₀O₅Si [M+NH₄]⁺ = 562.26, found 562.25, RT: 2.657 min, Method 1.

### Preparation of Compound 60

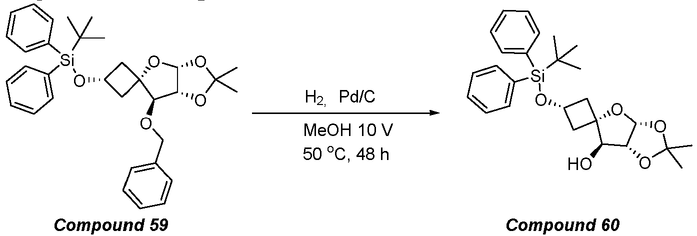

***Compound 59*** (530 mg, 0.97 mmol, 1.0 equiv) was dissolved in methanol and Pd/C (106 mg) was added. The reaction was stirred at 50 °C for 48 h. Following completion, the solution was then filtered. The filter cake was washed with MeOH, and the filtrate was then concentrated under reduced pressure. The residue was applied onto a silica gel column with dichloromethane/methanol (gradient elution: PE/ EtOAc from 99:1 to 80:20). The fractions containing the product were collected and the solvent was evaporated to afford ***compound 60.* 33% yield** (150 mg, 0.33 mmol), colorless oil. **¹H NMR (300 MHz, Chloroform-*d*)** δ 7.64 (dt, J = 7.9, 1.8 Hz, 4H), 7.43 - 7.34 (m, 6H), 5.80 (d, J = 3.9 Hz, 1H), 4.48 (d, J = 3.9 Hz, 1H), 3.96 (p, J = 7.1 Hz, 1H), 3.81 (s, 1H), 2.64 - 2.53 (m, 2H), 2.40 (dt, J = 12.6, 6.7 Hz, 2H), 1.43 (s, 3H), 1.25 (s, 3H), 1.02 ppm (s, 9H). **¹³C NMR (101 MHz, Chloroform-*d*)** δ 135.49, 133.87, 129.69, 127.68, 127.66, 111.73, 104.45, 85.99, 79.87, 78.10, 60.33, 53.43, 46.09, 39.80, 26.69, 26.31, 25.78, 18.95 ppm. **LCMS** (ESI+) m/z: calcd. For C₂₆H₃₄O₅Si [M+NH4]⁺ = 472.22, found 472.25, RT: 1.835 min, Method 1.

### Synthesis of Compound 60.1

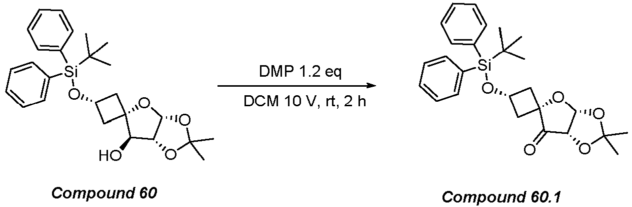

***Compound 60*** (200 mg, 0.44 mmol, 1 eq) was dissolved in DCM (2 mL), then DMP (224 mg, 0.52 mmol, 1.2 eq) was added at 0 °C, The mixture was stirred at RT for 2 h. The reaction was quenched by addition of 2 mL of saturated aqueous solution of Na₂S₂O₃ and 2 mL of saturated aqueous solution of NaHCO₃, then extracted with EtOAc (3 x 20 mL). The organic phase was dried with Na₂SO₄. The residue was purified via silica gel chromatography with PE/EA (gradient elution: PE/EtOAc from 99:1 to 90:10). The fractions containing the product were collected and the solvent was evaporated to afford ***Compound 60.1.***

75% **yield** (150 mg, 0.33 mmol), light yellow oil. **¹H NMR** (300 MHz, Chloroform-d) δ 7.64-7.62 (m, 4H), 7.44 - 7.36 (m, 6H), 5.96 (d, J = 4.4 Hz, 1H), 4.39 - 4.21 (m, 2H), 2.76 - 2.66 (m, 1H), 2.59 - 2.49 (m, 2H), 2.44 - 2.37 (m, 1H), 1.43 (s, 3H), 1.38 (s, 3H), 1.05 (s, 9H). **¹³C NMR** (75 MHz, CDCl₃) δ 211.71, 135.42, 133.70,133.64, 129.72, 127.68, 114.02, 102.18, 76.21, 75.97, 59.28, 47.73, 44.56, 27.31, 26.72, 22.69, 18.94, **LCMS** (ESI+) m/z: calcd, for C₂₆H₃₂O₅Si [M+NH₄]⁺ = 470.20, found 470.30, RT: 2.228 min, Method 1.

### Synthesis of Compound 60.2

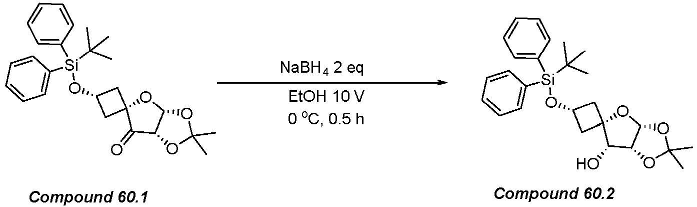

***Compound 60.1*** (130 mg, 0.28 mmol, 1 eq) was dissolved in EtOH (1.3 mL), then NaBH₄ (20 mg, 0.56 mmol, 2 eq) was added at 0 °C, The mixture was stirred at 0 °C for 30 min. The mixture was added to saturated NH₄Cl aqueous (30 mL) at 0°C, and then extracted with EtOAc (3 x 30 mL). The organic phase was dried with Na₂SO₄. The residue was purified via silica gel chromatography with PE/EA (gradient elution: PE/EtOAc from 99:1 to 80:20). The fractions containing the product were collected and the solvent was evaporated to afford ***Compound 60.2.***

**69% yield** (90 mg, 0.19 mmol), light yellow oil. **¹H NMR** (300 MHz, Chloroform-d) δ 7.66 - 7.60 (m, 4H), 7.42 - 7.32 (m, 6H), 5.68 (d, 1H), 4.44 (dd, J = 5.3, 4.1 Hz, 1H), 4.03 (q, J = 7.1 Hz, 1H), 3.72 (dd, J = 10.9, 5.3 Hz, 1H), 2.71-2.62 (m, 1H), 2.57-2.49 (m, 1H), 2.40-2.32 (m, 2H), 1.46 (s, 3H), 1.27 (s, 3H), 1.03 (s, 9H). **¹³C NMR** (75 MHz, Chloroform-d) δ 135.49, 134.14,134.06, 129.55, 127.57, 112.41, 103.70, 78.80, 77.08, 75.66, 60.30, 44.63, 42.54, 26.75, 26.37, 26.11, 18.95, **LCMS** (ESI+) m/z: calcd, for C₂₆H₃₄O₅Si [M+NH₄]⁺ = 472.22, found 472.20, RT: 2.062 min, Method 1.

### Preparation of Compound 61

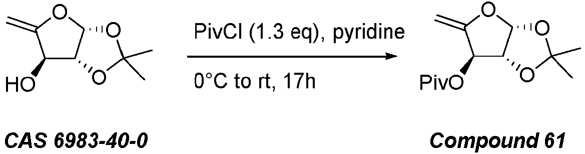

CAS 6983-40-0 (2.00 g, 11.6 mmol, 1.0 eq) was dissolved in pyridine (20 mL), cooled to 0°C and PivCl (1.81 g, 15.08 mmol, 1.3 eq) was added. The mixture was stirred at 0 °C to rt for 17 h, Subsequently, the mixture was slowly poured into ice-cold NH₄Cl (aq. 30 mL) and the product was extracted with n-heptane (3 x 50 mL). Combined organic layers were washed with brine, dried (MgSO₄), filtered and the filtrate was concentrated in vacuo. The residue was purified via silica gel chromatography (gradient: PE/ EtOAc from 99:1 to 90:10). The fractions containing the product were collected and the solvent was evaporated to afford ***compound 61.***

**67% yield** (2.0 g, 7.81 mmol), colorless oil. **¹H NMR (400 MHz, Chloroform-*d*):** δ 6.05-6.11 (m, 1H), 5.48 (s, 1H), 4.56-4.60 (m, 1H), 4.55 (d, *J=* 3.7 Hz, 1H), 4.34 (d, *J=* 2.0 Hz, 1H), 1.48 (s, 3H), 1.39-1.41 (m, 3H), 1.19-1.21 ppm (m, 9H). **¹³C NMR (101 MHz, Chloroform-*d*):** δ 176.9, 158.5, 114.1, 106.4, 88.8, 83.0, 75.3, 38.6, 28.0, 27.2, 26.9 ppm. **LCMS** (ESI+) m/z:calcd. for C₁₃H₂₀O₅ [M+H]⁺ = 257.13, found 257.10, RT: 1.113 min, Method 6.

### Preparation of Compound 62

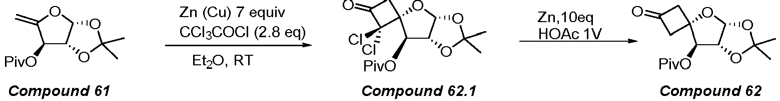

***Compound 61*** (2.2 g, 8.5 mmol, 1.0 eq) was dissolved in Et₂O (22 mL), Zn(Cu) (10.1 g, 59.5 mmol, 7.0 eq) was added. This was followed by the addition of a solution of trichloroacetyl chloride (4.4 g, 24.5 mmol, 2.8 eq) in Et₂O (6 mL) dropwise with stirring at 0 °C. The resulting solution was stirred for 2 h at r.t. Then zinc (5.58 g, 85 mmol, 10.00 eq), AcOH (2.2 mL) was added at 0 °C, the mixture was stirred at r.t for 3 h. The residue was added to saturated NaHCO₃ aqueous (50 mL) at 0 °C, the solids were filtered out, and the aqueous phase was extracted with EA (50 mL), the organic phase was dried over anhydrous sodium sulfate and concentrated *in vacuo.* The residue was purified by column chromatography over silica gel (gradient elution: PE/EtOAc from 99:1 to 80:20). The fractions containing the product were collected and the solvent was evaporated to afford ***compound 62.***

**60% yield** (1.5 g, 5.03 mmol). **¹H NMR (400 MHz, Chloroform-*d*)** δ 5.99 (d, J = 3.9 Hz, 1H), 5.35 (s, 1H), 4.60 (d, J = 3.9 Hz, 1H), 3.44 (t, J = 4.0 Hz, 2H), 3.37 (dd, J = 13.2, 4.6 Hz, 1H), 3.27 - 3.20 (m, 1H), 1.33 (s, 3H), 1.24 ppm (s, 9H), **¹³C NMR (75 MHz, Chloroform-*d*)** δ 202.93, 177.27, 112.18, 105.40, 84.26, 79.34, 78.02,, 59.05, 54.40, 38.97, 27.02, 26.07, 25.61 ppm, **LCMS** (ESI+) m/z:calcd. for C₁₅H₂₂O₆ [M+H]⁺ = 299.14, found 299.10, RT: 0.991 min, Method 6.

### Preparation of Compound 63

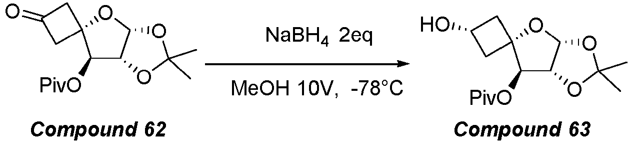

***Compound 62*** (1.54 g, 5.16 mmol, 1 eq) was dissolved in MeOH (15 mL), then NaBH₄ (0.37 g, 10.3 mmol, 2 eq) was added at -78 °C, The resulting solution was stirred for 1 h at -78 °C. The mixture was added to saturated NH₄Cl aqueous (50 mL) at 0 °C, and then extracted with EA (50 mL), the organic phase was dried over anhydrous sodium sulfate and concentrated *in vacuo.* The residue was purified by column chromatography over silica gel (gradient elution: PE/EtOAc from 99:1 to 70:30). The fractions containing the product were collected and the solvent was evaporated to afford ***compound 63.***

**52% yield** (780 mg, 2.6 mmol). **¹H NMR** (400 **MHz, Chloroform-*d*)** δ 5.84 (d, J = 3.9 Hz, 1H), 5.13 (s, 1H), 4.48 (d, J = 3.9 Hz, 1H), 2.89 (dt, J = 12.5, 6.4 Hz, 1H), 2.49 (dt, J = 12.6, 6.3 Hz, 1H), 2.30 - 2.23 (m, 2H), 1.49 (s, 3H), 1.28 (s, 3H), 1.22 ppm (s, 9H). **¹³C NMR (101 MHz, Chloroform-*d*)** δ 177.50, 112.10, 104.66, 84.38, 79.04, 78.60, 59.54, 46.10, 40.32, 38.95, 27.12, 26.40, 25.85 ppm. **LCMS** (ESI+) m/z: calcd. For C₁₅H₂₄O₆ [M+NH4]⁺ = 318.20, found 318.16, RT: 1.228 min, Method 1.

### Preparation of Compound 64

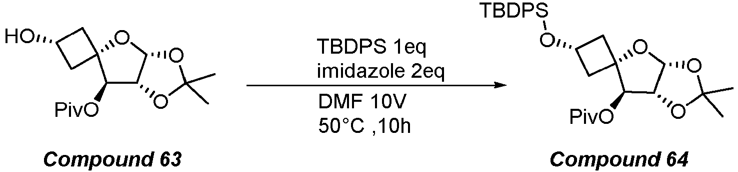

***Compound 63*** (750 mg, 2.50 mmol, 1.0 eq) was dissolved in DMF (8 mL), imidazole (340 mg, 5.00 mmol, 2.0 eq) and TBDPS (688 mg, 2.50 mmol, 1.0 eq) was added, The mixture was stirred at 50 °C for 10 h. The mixture was cooled down to room temperature and poured in EtOAc (15 mL) followed by washing with brine (3 x 15 mL). The organic phase was dried over MgSO₄, filtered and evaporated to a minimum. An additional washing step after dissolving the obtained residue in EtOAc (15 mL) with brine (5 x 15 mL) was required to remove the remaining DMF. The residue was purified by column chromatography over silica gel (PE / EtOAc from 99:1 to 95:5). The fractions containing the product were collected and the solvent was evaporated to afford ***compound 64.***

52% yield (700 mg, 1.30 mmol), white solid. **¹H NMR (300 MHz, Chloroform-*d*)** δ 7.62 (ddd, J = 7.8, 2.8, 1.7 Hz, 4H), 7.36 (qt, J = 8.7, 2.7 Hz, 6H), 5.78 (d, J = 3.9 Hz, 1H), 4.91 (s, 1H), 4.38 (d, J = 3.9 Hz, 1H), 3.98 (p, J = 7.1 Hz, 1H), 2.67 (dt, J = 12.3, 6.3 Hz, 1H), 2.35 (td, J = 12.2, 7.5 Hz, 2H), 2.21 (dt, J = 12.4, 6.3 Hz, 1H), 1.45 (s, 3H), 1.25 (d, J = 1.9 Hz, 3H), 1.12 (s, 9H), 1.03 ppm (s, 9H). **¹³C NMR (75 MHz, Chloroform-*d*)** δ 135.49, 135.45, 133.93, 133.84, 129.63, 129.61, 127.61, 127.59, 112.03, 104.54, 84.38, 79.02, 78.77, 65.54, 60.25, 46.30, 40.56, 38.82, 30.58, 27.02, 26.72, 26.43, 25.88 ppm, **LCMS** (ESI+) m/z: calcd, for C₃₁H₄₂O₆Si [M+NH₄]⁺ = 556.28, found 556.20, RT: 2.697 min, Method 3.

### Alternative preparation of Compound 60

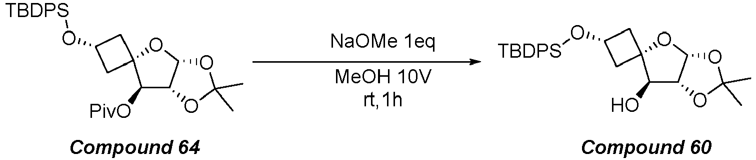

***Compound* 64** (1.37 mmol, 740 mg, 1 eq.) was dissolved in MeOH (8 mL) and sodium methoxide (74.0 mg, 1.37 mmol, 1.00 equiv) was added. The reaction was stirred at RT for 30 min, then stirred with H exchange resin to pH = 7. Then filtered, and the filtrate was concentrated under reduced pressure. The residue was applied onto a silica gel column with PE / EtOAc (gradient elution: PE / EtOAc from 99:1 to 80:20). The fractions containing the product were collected and the solvent was evaporated to afford ***compound 60* 36% yield** (230 mg, 0.50 mmol), colorless oil. **¹H NMR (300 MHz, Chloroform-*d*)** δ 7.64 (dt, J = 7.9, 1.8 Hz, 4H), 7.43 - 7.34 (m, 6H), 5.80 (d, J = 3.9 Hz, 1H), 4.48 (d, J = 3.9 Hz, 1H), 3.96 (p, J = 7.1 Hz, 1H), 3.81 (s, 1H), 2.64 - 2.53 (m, 2H), 2.40 (dt, J = 12.6, 6.7 Hz, 2H), 1.43 (s, 3H), 1.25 (s, 3H), 1.02 ppm (s, 9H). **¹³C NMR (101 MHz, Chloroform-*d*)** δ 135.49, 133.87, 129.69, 127.68, 127.66, 111.73, 104.45, 85.99, 79.87, 78.10, 60.33, 53.43, 46.09, 39.80, 26.69, 26.31, 25.78, 18.95 ppm. **LCMS** (ESI+) m/z: calcd. For C₂₆H₃₄O₅Si [M+NH4]⁺ = 472.22, found 472.25, RT: 1.835 min, Method 4.

### Preparation of Compounds 66a and 66b

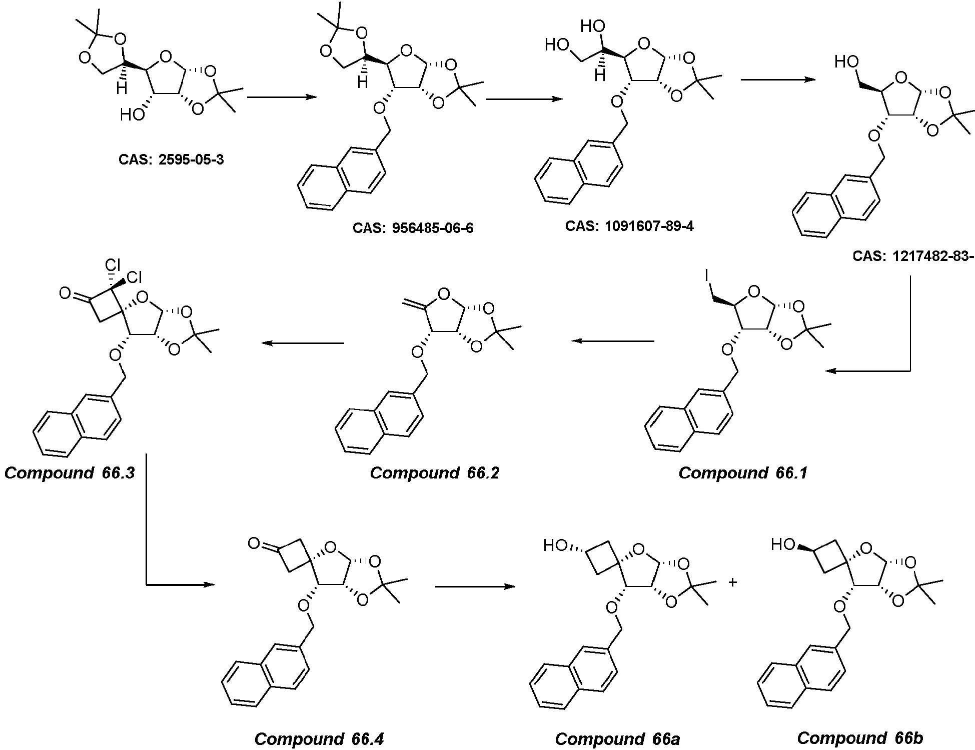

### Preparation of Compound 66.1

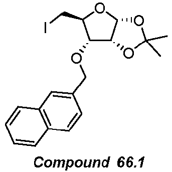

((3aR,5R,6R,6aR)-2,2-Dimethyl-6-(naphthalen-2-ylmethoxy)tetrahydrofuro[2,3-d][1,3]dioxol-5-yl)methanol (4.71 g, 14.3 mmol, 1.00 eq, CAS: 1217482-83-1) was dissolved in THF (87 ml). Triphenylphosphine (8.98 g, 34.2 mmol, 2.4 eq) and imidazole (2.91 g, 42.79 mmol, 3.3 eq) were added, followed by the portion wise addition of iodine (8.80 g, 34.6 mmol, 2.4 eq). The reaction mixture was stirred for 2 hours at reflux under nitrogen atmosphere. A saturated aqueous solution of Na₂SO₃ was added, followed by 5 minutes stirring. Next, the reaction mixture was extracted with EtOAc (4 x 100 ml) and combined organic layers were washed with saturated aqueous Na₂SO₃ (1 x 200 ml), saturated aqueous NaHCO₃ (1 x 200 ml) and brine (1 x 200 ml), then dried with MgSO₄, filtered and the filtrate was concentrated *in vacuo.* n-Heptane was added to the residue and the mixture was stirred for 5 minutes. The solids were removed by filtration and rinsed with n-heptane after which the filtrate was concentrated *in vacuo.* The residue was purified by silicagel column chromatography (gradient of *n-*heptane/EtOAc, from 95:5 to 80:20). Tubes containing the product were combined and concentrated *in vacuo* to yield ***compound 66.1.***

**87% yield** (26.3 g, 12.4 mmol), white solid. **¹H NMR (400 MHz, Chloroform-*d*):** δ 7.94 - 7.83 (m, 4H), 7.54 (dd, *J*=8.54, 1.63 Hz, 1H), 7.53 - 7.48 (m, 2H), 5.75 (d, *J*=3.74 Hz, 1H), 4.95 (d, *J*=12.10 Hz, 1H), 4.76 (d, *J*=12.10 Hz, 1H), 4.59 (t, *J*=3.96 Hz, 1H), 3.78 (m, 1H), 3.66 (dd, *J*=8.47, 4.29 Hz, 1H), 3.55 (dd, *J=*11.00*,* 3.08 Hz, 1H), 3.34 (dd, *J=*11.22*,* 4.40 Hz, 1H), 1.62 (s, 3H), 1.38 ppm (s, 3H). **¹³C NMR (100 MHz, Chloroform-*d*):** δ 134.72, 133.23, 133.20, 128.49, 127.90, 127.75, 127.05, 126.28, 126.16, 125.80, 113.24, 103.95, 81.71, 77.47, 76.32, 72.46, 26.84, 26.57, 7.30 ppm. **ESI-MS:** *m*/*z* 458.0 [M + NH₄]⁺ **R_{f}** (n-heptane/ EtOAc; 1:1) = 0.77

**LCMS:** [M+1]⁺= 458.0 [M+1] ⁺, 2.29 min (Method 13)

### Preparation of Compound 66.2

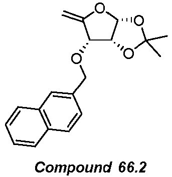

***Compound 66.1*** (9.35 g, 19.3 mmol, 1.00 eq) was dissolved in DMF (71 ml) and sodium iodide (17.4 g, 115.8 mmol, 6.00 eq) was added. The reaction mixture was stirred at 80-85°C for 2 hours and DBU (3.46 mL, 23.2 mmol, 1.2 eq) was added. The reaction mixture was stirred for 1.5 hours at 85°C. A second portion of DBU (0.87 mL, 5.80 mmol, 0.3 eq) was added and stirring was continued at 85°C for 1.5 hours. Next, the reaction mixture was cooled to room temperature and diluted with EtOAc and deionized water. The aqueous layer was separated and extracted with EtOAc (1x) after which combined organic layers were washed with a citric acid/sodium hydroxide (pH=4) buffer solution (3x) and brine (1x). The organic layer was dried (MgSO₄), filtered and the filtrate was concentrated *in vacuo.* The residue was purified by silica gel column chromatography (gradient of n-heptane/EtOAc, from 90:10 to 80:20). Fractions containing the product were combined and the solvent was removed *in vacuo* to afford ***compound 66.2.***

**65% yield** (3.94 g, 12.5 mmol), white solid. **¹H NMR (400 MHz, Chloroform-*d*):** δ 7.89 - 7.83 (m, 4H), 7.57 (dd, *J*=8.3, 1.61 Hz, 1H,), 7.53 - 7.47 (m, 2H), 5.83 (d, *J*=3.26 Hz, 1H), 5.02 (d, *J*=12.42 Hz, 1H), 4.88 (d, *J*=12.42 Hz, 1H), 4.59 (m, 1H), 4.52 (m, 1H), 4.36 (m, 1H), 4.34 (dd, *J=4.50,* 2.15 Hz, 1H), 1.57 (s, 3H), 1.43 ppm (s, 3H). **¹³C NMR (100 MHz, Chloroform-*d*):** δ 158.79, 134.77, 133.20, 133.17, 128.45, 127.67, 127.75, 126.92, 126.28, 126.15, 125.74, 114.78, 104.27, 83.94, 77.42, 72.57, 27.91, 27.28 ppm. **R_{f}** (n-heptane/ EtOAc; 1:1) = 0.77. **MP:** 102.01°C.

**LCMS:** [M+1]⁺= 313.1 [M+1] ⁺, 2.17 min (Method 13)

### Preparation of Compound 66.3

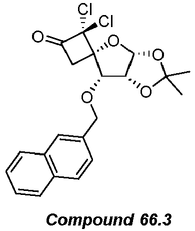

***Compound 66.2*** (3.88 g, 12.4 mmol, 1.00 eq) was dissolved in anhydrous THF (48 mL) and activated zinc* (5.69 g, 87.0 mmol, 7.00 eq) - prepared as described below - was introduced. A solution of trichloroacetylchloride (2.28 mL, 20.2 mmol, 1.80 eq) dissolved in anhydrous THF (16 ml) was added dropwise over 1 hour at room temperature. Subsequently, zinc salts were removed via filtration over Celite^{®} and rinsed with EtOAc. The filtrate was washed with saturated aqueous NaHCO₃ (3x) and brine (2x). The organic layer was dried (MgSO₄), filtered and the filtrate was concentrated *in vacuo* to afford ***compound 66.3*** as an orange viscous oil. The product was used immediately as such in the next step.

(5.85 g, crude), **¹H NMR (400 MHz, Chloroform-*d*):** δ 7.92 - 7.80 (m, 4H), 7.54 - 7.48 (m, 3H), 5.84 (d, *J*=3.30 Hz, 1H), 4.94 (d, *J*=11.67 Hz, 1H), 4.86 (d, *J*=11.66 Hz, 1H), 4.62 (t, *J*=3.96 Hz, 1H), 4.30 (d, *J*=4.40 Hz, 1H), 4.05 (d, *J*=18.27 Hz, 1H), 3.55 (d, *J*=18.27 Hz, 1H), 1.54 (s, 3H), 1.33 ppm (s, 3H). **¹³C NMR (100MHz, Chloroform-*d*** δ 191.48, 134.14, 133.25, 133.11, 128.24, 127.94, 127.77, 127.02, 126.34, 126.27, 125.91, 120.23, 113.94, 104.28, 83.22, 79.22, 78.08, 72.75, 51.45, 26.78, 26.03 ppm.

* Zinc powder (100 g, 1.54 mol) was added to deionized water (500 ml). The mixture was stirred and nitrogen gas was bubbled through the solution for 10 min after which anhydrous CuSO₄ (7.36 g, 46.1 mmol) was added. The suspension was stirred and bubbled for 45 minutes and then filtered. Zinc powder was rinsed with both degassed deionized water (1.25 L) and degassed acetone (550 mL), then dried overnight *in vacuo.* The resulting black powder (100 g) was stored under inert atmosphere.

### Preparation of Compound 66.4

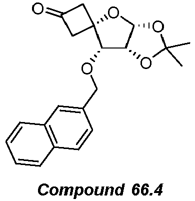

***Compound 66.3*** (5.26 g, 12.4 mmol, 1.00 eq) was dissolved in THF (49 ml) and zinc (8.12 g, 124 mmol, 10.0 eq) was added. The reaction mixture was cooled to 0°C and glacial acetic acid (3.56 ml, 62.1 mmol, 5.00 eq) was carefully added. The reaction mixture was stirred for 20 hours at room temperature and zinc was removed via filtration over Celite^{®} and rinsed with EtOAc. The filtrate was washed with saturated NaHCO₃ (3x) and brine (3x). The aqueous phase was extracted with EtOAc (1x). Combined organic layers were dried (MgSO₄), filtered and the filtrate was concentrated *in vacuo.* The residue was purified via silicagel column chromatography (gradient of *n*-heptane/EtOAc, from 95:5 to 75:25). Fractions containing the product were combined and the solvent was removed *in vacuo* to afford ***compound 66.4.***

**44% yield** (2 steps, 1.94 g, 5.46 mmol), white solid. **¹H NMR (400 MHz, Chloroform-*d*):** δ 7.89 - 7.77 (m, 4H), 7.54 - 7.48 (m, 3H), 5.77 (d, *J*=4.07 Hz, 1H), 5.04 (d, *J*=12.21 Hz, 1H), 4.78 (d, *J*=12.21 Hz, 1H), 4.68 (t, *J*=4.07 Hz, 1H), 3.92 (d, *J*=4.48 Hz, 1H), 3.80 (ddd, *J*=17.70, 6.92, 2.24 Hz, 1H), 3.31 (ddd, *J*=17.50, 6.11, 2.04 Hz, 1H), 3.24 (ddd, *J*=17.91, 6.10, 2.44 Hz, 1H), 2.99 (ddd, *J*=17.80, 6.82, 2.24 Hz, 1H), 1.55 (s, 3H), 1.35 ppm (s, 3H). **¹³C NMR (100 MHz, Chloroform-*d*):** δ 204.66, 134.54, 133.22, 133.11, 128.61,_127.86, 127.79, 126.92, 126.99, 126.41, 125.61, 112.93, 104.16, 79.91, 76.83, 75.25, 72.46, 56.10, 55.01, 26.32, 25.80 ppm. **R_{f}** (n-heptane/ EtOAc; 1:1) = 0.67. **MP:** 101.79°C.

**LCMS:** [M+1]⁺= 355.3 [M+1] ⁺, 2.08 min (Method 9)

### Preparation of Compounds 66a and 66b

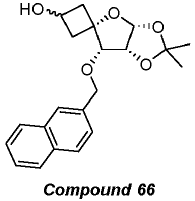

***Compound 66.4*** (598 mg, 1.69 mmol, 1.00 eq) was dissolved in a mixture of THF, deionized water and methanol (6:2:5, 13 ml) then cooled to 0°C. Sodium borohydride (140 mg, 3.71 mmol, 2.20 eq) was added followed by a stirring of 30 minutes. Subsequently, 10 equivalents of acetic acid (0.97 ml) were added in order to quench the reaction. The mixture was diluted with EtOAc (20 ml) and neutralized with saturated aqueous Na₂CO₃. The aqueous layer was separated and extracted with EtOAc (2 x 20 mL), the combined organic phases were washed with saturated aqueous Na₂CO₃ (2 x 20 mL) and brine (3 x 20 mL), dried with MgSO₄, filtered and the filtrate was concentrated *in vacuo* to afford ***compound 66*** (619.9 mg, crude, white solid) as a mixture of *cis*/*trans* isomers (0.9:1 ratio, respectively).

**LCMS:** [M+1]⁺= 357.2 [M+1] ⁺, 1.89 min (Method 13)

To obtain an analytical pure sample of both isomers ***compound 66b*** and
**(*****compound 66a**,* the mixture of alcohols was protected (t-butyldiphenyl silyl), the silylated intermediates were separated via silica gel chromatography and subsequently deprotected with TBAF to obtain the isolated products below.

### Compound 66b

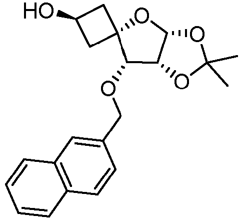

**¹H NMR (400 MHz, Chloroform-d):** δ 7.96 - 7.82 (m, 4H), 7.59 (dd, *J*=8.47, 1.65 Hz, 1H), 7.55 - 7.48 (m, 2H), 5.69 (d, *J*=3.96 Hz, 1H), 5.13 (d, *J*=11.88 Hz, 1H), 4.85 (d, *J*=11.88 Hz, 1H), 4.61 (t, *J*=4.18 Hz, 1H), 4.28 (tt, *J*=7.10, 3.47 Hz, 1H), 3.63 (d, *J*=4.40 Hz, 1H), 2.76 - 2.57 (m, 4H), 2.20 - 1.93 (m, 1H), 1.57 (s, 3H), 1.33 - 1.31 ppm (m, 3H). **¹³C NMR (100 MHz, Chloroform-*d*):** δ 134.20, 133.22, 133.14, 128.63, 127.88, 127.74, 127.22, 126.34, 126.26, 125.74, 112.69, 104.08, 81.55, 80.89, 76.51, 72.45, 62.79, 42.92, 41.91, 26.39, 25.85 ppm. **ESI-MS:** *m*/*z* 379.2 [M + Na]⁺
**R_{f}** (n-heptane/ EtOAc; 1:1) = 0.45
**LCMS:** [M+1]⁺= 379.2 [M+Na] ⁺, 1.89 min (Method 9)

### Compound 66a

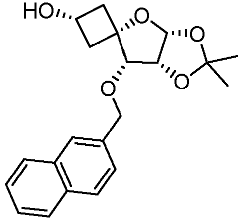

**¹H NMR (400 MHz, Chloroform-*d*):** δ 7.89 - 7.82 (m, 4H), 7.59 - 7.47 (m, 3H), 5.65 (d, *J*=3.87 Hz, 1H), 5.02 (d, *J*=12.23 Hz, 1H), 4.76 (d, *J*=12.33 Hz, 1H), 4.57 (t, *J*=4.13 Hz, 1H), 4.20 - 4.09 (m, 1H), 3.63 (d, *J*=4.29 Hz, 1H), 3.27 (ddd, *J*=12.23, 6.85, 5.28 Hz, 1H), 2.54 - 2.40 (m, 1H), 2.40 - 2.26 (m, 3H), 2.18 (dd, *J*=12.12, 7.00 Hz, 1H), 1.57 (s, 3H), 1.32 ppm (s, 3H). **¹³C NMR (100 MHz, Chloroform-d):** δ 134.98, 133.10, 128.39, 127.81, 127.72, 126.68, 126.28, 126.11, 125.53, 112.84, 103.86, 81.21, 77.19, 76.17, 72.30, 59.91, 44.13, 43.50, 26.55, 25.97 ppm. **R_{f}** (n-heptane/ EtOAc; 1:1) = 0.45
**LCMS:** [M+1]⁺= 357.3 [M+1] ⁺, 1.88 min (Method 9)

### Preparation of Compound 141

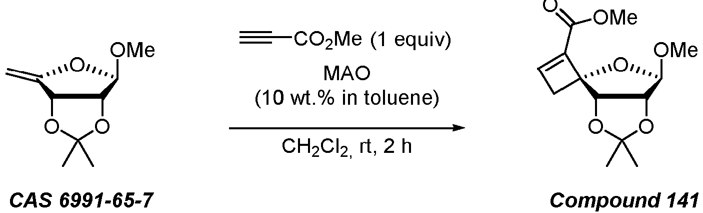

Methylaluminoxane (MAO, 10 wt. % in toluene, 3.06 mL) was slowly added at room temperature under an argon atmosphere to a solution containing methylpropiolate (45.15 mg, 0.537 mmol, 1 equiv) in 6 mL of dry CH₂Cl₂. The reaction mixture was stirred at room temperature for 15 min and CAS 6991-65-7 (100 mg, 0.537 mmol, 1 equiv) in solution in dry CH₂Cl₂ (1 mL) was then added. After 2 h stirring, H₂O (10 mL) was carefully added and the aqueous phase was washed with EtOAc (3x20 mL). The combined organic phases were dried over anhydrous Na₂SO₄, filtered, and concentrated *in vacuo* to give a residue that was purified by silica gel column chromatography (gradient elution: PE/EtOAc from 100:1 to 80:20) to afford the desired ***compound 141*** as a yellow oil (20% yield; 0.272 mmol; 73.6 mg).
**R*_{f}*:** 0.25 (Petroleum ether/EtOAc 90:10 *v*/*v*, UV)
**¹H NMR (CDCl₃, 400 MHz):** δ = 6.91 (t, 1 H, *J =* 1.2 Hz), 4.96 (s, 1 H), 4.96 (d, 1 H, *J =* 5.8 Hz), 4.74 (d, 1 H, *J =* 5.8 Hz), 3.72 (s, 3 H), 3.28 (s, 3 H), 3.08 (dd, 1 H, *J =* 15.6 Hz, 1.2 Hz), 2.68 (dd, 1 H, *J =* 15.6 Hz, 1.2 Hz), 1.47 (s, 3 H), 1.34 (s, 3 H) ppm. **¹³C NMR (CDCl₃, 125 MHz):** δ = 161.8, 145.8, 142.9, 112.3, 108.9, 89.2, 85.7, 82.0, 54.8, 51.2, 37.1, 26.3, 25.2 ppm. **LCMS (ESI+) m/z:** calcd. for C₁₂H₁₅O₅ [M-OMe]+ = 239.1, found 239.1, RT: 1.341 min, Method 2.

### Preparation of Compound 142

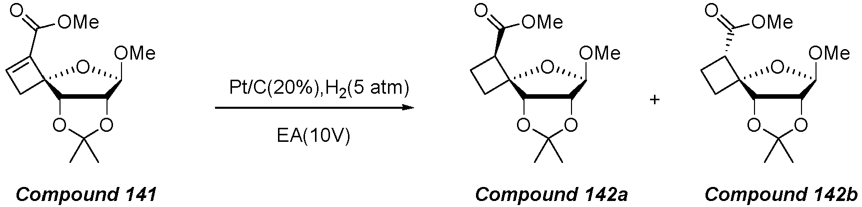

***Compound 141*** (1.20 g, 4.44 mmol, 1.00 eq), EA (12 ml) and Pt/C(300 mg, 20%) were added bubbling with H2 (5 atm) for o/n. Then the catalyst was filtered over a celite pad and the filtrate was concentrated under reduced pressure. The crude was purified over silica gel column chromatography (gradient elution: PE/EtOAc from 100:1 to 90:10), the front peak was ***compound 142a,*** the back peak was ***compound 142b.***

**33%** (400 mg, 1.47 mmol) yellow oil of ***compound 142a.* ¹H NMR (400 MHz, Chloroform-d)** δ 5.38 (d, J = 5.8 Hz, 1H), 4.81 (s, 1H), 4.50 (d, J = 5.8 Hz, 1H), 3.64 (s, 3H), 3.32 (s, 3H), 3.16 - 3.09(m, 1H), 2.46 - 2.39 (m, 1H), 2.23 - 2.13 (m, 1H), 2.07 - 1.86 (m, 2H), 1.42 (s, 3H), 1.36 ppm (s, 3H). **¹³C NMR (101 MHz, CDCl3)** δ 172.21, 111.97, 108.13, 87.37, 84.75, 78.91, 54.84, 51.30, 50.09 ,27.11, 26.33, 25.28, 17.79 ppm. **GCMS (ESI+) m/z:** calcd. for C₁₃H₂₀O₆ [M-Me]⁻ = 257.1, found 257.1, RT: 6.909 min, Method 1.

**50%** (600 mg, 1.47 mmol) yellow oil of ***compound 142b.* ¹H NMR (400 MHz, Chloroform-d)** δ 4.84 (s, 1H), 4.68 - 4.62 (m, 2H), 3.73 (s, 3H), 3.26 (s, 3H), 3.06 - 3.00 (m, 1H), 2.77 - 2.66 (m, 1H), 2.53 - 2.44 (m, 1H), 2.15 - 2.02 (m, 1H), 1.90 - 1.79 (m, 1H), 1.42 (s, 4H), 1.36 ppm (s, 3H). **¹³C NMR (101 MHz, CDCl3)** δ 172.68, 112.76, 109.27, 86.67, 85.17, 85.01, 56.06, 51.69, 50.79, 28.87, 26.43, 25.58, 17.29 ppm. **GCMS (ESI+) m/z:** calcd. for C₁₃H₂₀O₆ [M-Me]⁻ = 257.1, found 257.1, RT: 7.303 min, Method 1.

### Preparation of Compound 143a

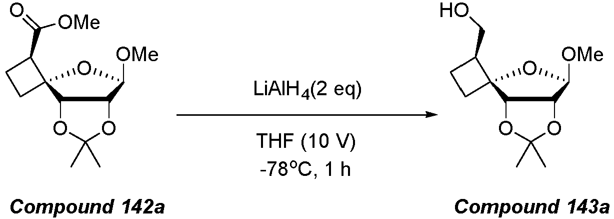

***Compound 142a*** (150 mg, 0.55 mmol, 1.00 eq) was dissolved in dry THF (2 mL). The solution was cooled to - 78 °C and LiAlH₄ (42.82 mg, 1.10 mmol, 2.00 eq) was added. The resulting mixture was stirred for 3 h at - 78 °C. The reaction was then quenched by the addition of 5 ml H₂O, 15 ml aq. NaOH (3 M) and 5ml H₂O. The solution was filtered over a celite pad and the filtrate was concentrated under reduced pressure. The crude was purified over silica gel column chromatography (gradient elution: PE/EtOAc from 100:1 to 90:10) to afford ***compound 143a.***

**73%** (98 mg, 0.40 mmol) off-white solid.**¹H NMR (300** MHz, **Chloroform-d)** δ 4.94 (d, J = 5.8 Hz, 1H), 4.85 (s, 1H), 4.68 (d, J = 5.8 Hz, 1H), 3.72 - 3.53 (m, 2H), 3.42 (s, 3H), 2.74 - 2.58 (m, 1H), 2.45 - 2.33 (m, 1H), 2.23 - 2.08 (m, 1H), 1.96 - 1.81 (m, 1H), 1.43 (s, 3H), 1.36 (s, 3H), 1.31 - 1.23 ppm (m, 1H). **¹³C NMR** (75 **MHz, CDCl3)** δ 112.23, 108.30, 87.74, 85.57, 79.09, 62.97, 55.26, 48.58, 26.30, 25.73, 25.20, 15.92 ppm. **GCMS (ESI+) m/z:** calcd. for C₁₂H₂₀O₅ [M-Me]⁻ = 229.1, found 229.1, RT: 6.944 min, Method 1.

### Preparation of Compound 144a

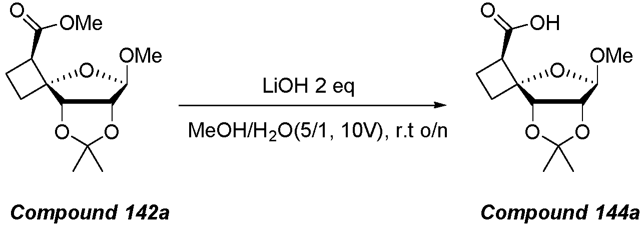

***Compound 142a*** (100 mg, 0.36 mmol, 1.00 eq) was dissolved in MeOH/H₂O (5/1, 2 mL). The solution was cooled to 0 °C and LiOH (17.28 mg, 0.72 mmol, 2.00 eq) was added. The resulting mixture was stirred for overnight at r.t. The reaction was then quenched by the addition of 10 ml aq.CA (1 M) extracted with dichloromethane (3 x 20 mL), dried over anhydrous Na₂SO₄, filtered, and concentrated under reduced pressure. The solution was filtered, and the filtrate was concentrated under reduced pressure. The crude was purified over silica gel column chromatography (gradient elution: PE/EtOAc from 100:1 to 90:10) to afford ***compound 144a.***

**53%** (51 mg, 0.19 mmol) light yellow solid.**¹H NMR (400 MHz, Chloroform-d) δ** 4.93 (s, 1H), 4.86 (d, J = 5.6 Hz, 1H), 4.68 (d, J = 5.7 Hz, 1H), 3.52 (s, 3H), 3.19 (t, J = 9.6 Hz, 1H), 2.46 - 2.37 (m, 1H), 2.29 - 2.17 (m, 1H), 2.13 - 2.02 (m, 2H), 1.42 (s, 3H), 1.33 ppm (s, 3H). **¹³C NMR (101 MHz, CDCl3)** δ 171.59, 111.74, 107.61, 87.26, 83.64, 79.17, 54.50, 49.95, 25.12, 24.28, 23.99, 14.92 ppm. **GCMS (ESI+) m/z:** calcd. for C₁₂H₁₈O₆ [M-Me]⁻ = 243.0, found 243.0, RT: 7.490 min, Method 1.

### Preparation of Compound 143b

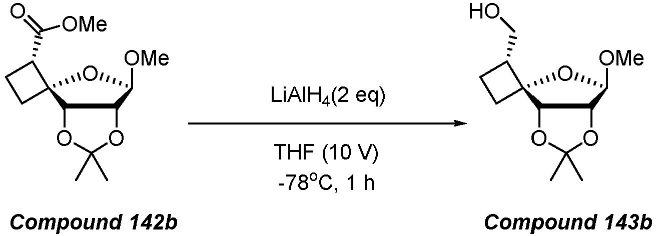

***Compound 142b*** (150 mg, 0.55 mmol, 1.00 eq) was dissolved in dry THF (2 mL). The solution was cooled to - 78 °C and LiAlH₄ (42.82 mg, 1.10 mmol, 2.00 eq) was added. The resulting mixture was stirred for 3 h at - 78 °C. The reaction was then quenched by the addition of 5 ml H₂O, 15 ml aq. NaOH (3 M) and 5ml H₂O. The solution was filtered over a celite pad and the filtrate was concentrated under reduced pressure. The crude was purified over silica gel column chromatography (gradient elution: PE/EtOAc from 100:1 to 90:10) to afford ***compound 143b.***

**29%** (40 mg, 0.16 mmol) yellow oil.**¹H NMR (300 MHz, Chloroform-d)** δ 4.91 (s, 1H), 4.69 - 4.58 (m, 2H), 3.89 - 3.79 (m, 1H), 3.59 - 3.50(m, 1H), 3.47 (s, 3H), 2.55 - 2.39 (m, 2H), 2.28 - 2.09 (m, 1H), 1.85 - 1.70 (m, 1H), 1.61 - 1.48 (m, 1H), 1.44 (s, 3H), 1.35 (m, 3H), 1.26 ppm (s, 1H). **¹³C NMR (101 MHz, CDCl3)** δ 112.62, 110.33, 88.92, 85.71, 85.15, 62.21, 56.49, 47.67, 27.82, 26.54, 25.41, 16.19 ppm. **GCMS (ESI+) m/z:** calcd. for C₁₂H₂₀O₅ [M-Me]- = 229.1, found 229.1, RT: 7.008 min, Method 1.

### Preparation of Compound 144b

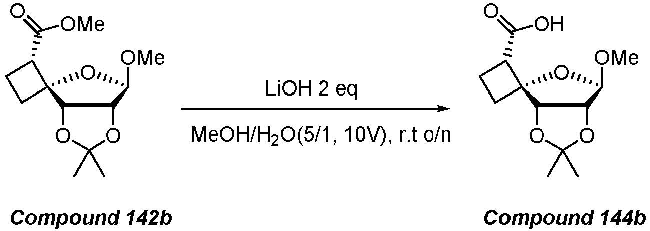

***Compound 142b*** (220 mg, 0.81 mmol, 1.00 eq) was dissolved in MeOH/H₂O (5/1, 2.5 mL). The solution was cooled to 0 °C and LiOH (38.88 mg, 1.62 mmol, 2.00 eq) was added. The resulting mixture was stirred for overnight at r.t. The reaction was then quenched by the addition of 10 ml aq.CA (1 M) extracted with dichloromethane (3 x 20 mL), dried over anhydrous Na₂SO₄, filtered, and concentrated under reduced pressure. The solution was filtered, and the filtrate was concentrated under reduced pressure. The crude (175 mg, containing ***compound 144a*** and ***compound 144b,*** ratio 1:2) was purified over silica gel column chromatography (gradient elution: PE/EtOAc from 100:1 to 90:10), the front peak was ***compound 144a,*** the back peak was ***compound 144b.***

17% (35 mg, 1.47 mmol, containing ***compound 144a*** and ***compound 144b,*** ratio 1:15) off-white solid. **¹H NMR (300 MHz, Chloroform-d)** δ 4.87 (s, 1H), 4.72 - 4.65 (m, 2H), 3.33 (s, 3H), 3.06 - 2.98 (m, 1H), 2.76 - 2.62 (m, 1H), 2.56 - 2.44 (m, 1H), 2.19 - 2.05 (m, 1H), 1.93 - 1.79 (m, 1H), 1.42 (s, 3H), 1.36 ppm (s, 3H). **¹³C NMR (101 MHz, CDCl3)** δ 176.29, 111.92, 108.28, 85.39, 84.00, 83.72, 55.09, 49.96, 27.83, 25.38, 24.53, 15.89 ppm. GCMS (ESI+) m/z: calcd. for C₁₂H₁₈O₆ [M-Me]⁻ = 243.0, found 243.0, RT: 7.634 min, Method 1.

### Preparation of Compound 149

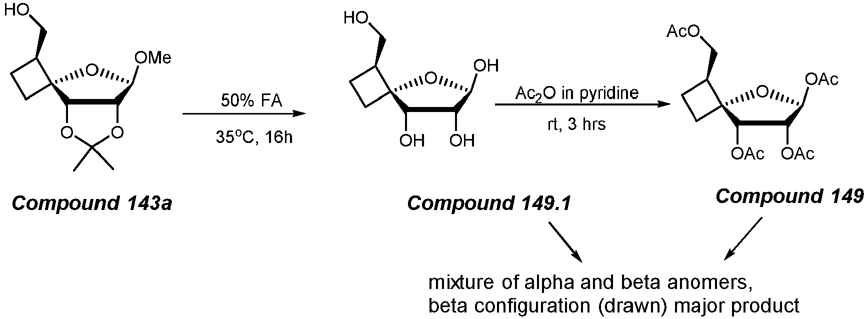

***Compound 143a*** (138 mg, 0.56 mmol, 1.00eq) was dissolved in H₂O (1.5 mL), and formic acid (FA) (1.5 mL) was added. The mixture was heated to 50°C for 16 hours. Subsequently, the solution was cooled to room temperature and concentrated in vacuo. This resulted in 141 mg of ***compound 149.1.***

***Compound 149.1*** (141 mg, crude; mixture of alpha and beta anomers, beta anomer being major product) was dissolved in dry pyridine (1.5 mL) and stirred for 30 minutes. Acetic anhydride (85.68 mg, 0.84 mmol, 1.50 eq) was added at 0°C to the stirring solution. The mixture was stirred at room temperature for 3 hours. Subsequently, the mixture was poured into ice-cold water (5 ml) and stirred for 30 minutes at room temperature. The crude mixture was extracted with CH₂Cl₂ (3x 5 ml) and the combined organic layers were washed with brine (3x 5 ml), dried (Na₂SO₄), filtered and the filtrate was concentrated in vacuo. The residue was purified by silica gel chromatography (gradient elution: PE / EA from 100:1 to 10: 1). Fractions containing the product were combined and the solvent was removed in vacuo to afford ***compound 149.***

**74% yield for 2 steps** (150 mg, 0.41mmol), colorless oil. **LCMS (ESI+) m/z:** calcd. for C₁₆H₂₂O₉ [M+H₂O] + = 376.20, found 376.20, RT: 1.290 min, Method 1.

### Preparation of Compound 150

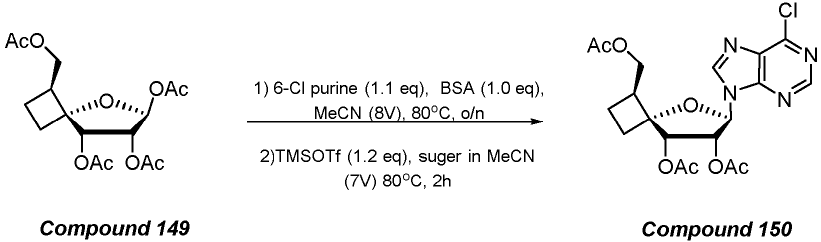

6-Chloropurine (69.45 mg,0.45 mmol, 1.10 eq) was dissolved in MeCN (1.1 mL), and N,O-bis(trimethylsilyl)acetamide (BSA) (83.23 mg, 0.41 mmol, 1.00 eq) was added dropwise. The mixture was heated to 80°C for 16 hours. After the mixture was cooled to room temperature, **compound 149** (150 mg, 0.41 mmol, 1.00 eq) in MeCN (1.2 mL) was added, followed by trimethylsilyltrifluoromethanesulfonate (109.22 mg, 0.49 mmol, 1.20 eq) and the mixture was heated to 80°C for 2 hours. The mixture was cooled to room temperature and diluted with EtOAc (10 mL) , washed with saturated NaHCO₃ (3x10 mL) and saturated aq. NaCl (3 x 10 mL) . The organic layer was dried (Na₂SO₄), filtered and the filtrate was concentrated in vacuo. The residue was purified by silica gel column chromatography with PE/EtOAc (gradient elution: PE/EtOAc from 99:1 to 4:1). The fractions containing the product were collected and the solvent was removed in vacuo to afford the title ***compound 150.***

**60% yield** (113 mg, 0.25mmol, crude), off-white solid. **LCMS (ESI+) m/z:** calcd. for C₁₉H₂₁ClN₄O₇ [M+H]+ = 453.2, found 453.2, RT: 1.324 min, Method 1.

### Preparation of compound 151

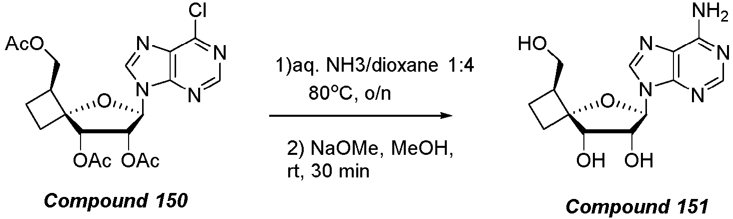

**Compound 150** (113 mg, 0.25 mmol, 1.00 eq) was dissolved in 1,4-dioxane (1.2 mL), and NH₃ (aq. 0.3 mL) was added. The mixture was heated to 80°C for 16 hours. After cooling the mixture to room temperature, solvents were removed *in vacuo* and the product was dissolved in MeOH (1.2 ml). Sodium methoxide (13.5 mg, 0.25 mmol, 1.00 eq) was added and the mixture was stirred at room temperature for 30 minutes, then stirred with hydrogen exchange resin (CAS : 78922-04-0) 30 min to PH=7. The resulting mixture was filtered and the filtrate was concentrated *in vacuo.* The residue was purified via silica gel column chromatography with dichloromethane/ methanol (gradient elution: DCM/MeOH from 99:1 to 5:1). The fractions containing the product were collected and the solvent was removed in vacuo to afford ***compound 151.***

**65% yield** (49 mg, 0.15 mmol), off-white solid. **¹H NMR** (300 MHz, Methanol-d4) δ 8.46 (s, 1H), 8.18 (s, 1H), 6.03 (d, J = 6.1 Hz, 1H), 4.95 - 4.89 (m, 1H), 4.49 (d, J = 4.5 Hz, 1H), 3.84 - 3.58 (m, 2H), 2.86 - 2.69 (m, 1H), 2.64 - 2.50 (m, 1H), 2.09 - 1.89 (m, 1H), 1.88 - 1.68 (m, 1H), 1.29 - 1.20 (m, 1H). **¹³C NMR (75 MHz, MeOD)** δ 155.89, 152.37, 149.24, 140.26, 118.94, 88.06, 87.50, 75.43, 70.22, 60.87, 27.50, 14.50, 7.83 ppm. **LCMS (ESI+)** m/z: calcd. for C₁₃H₁₇N₅O₄ [M+H]+ =308.13 found 308.13, RT: 0.524 min, Method 1.

### Preparation of Compound 152

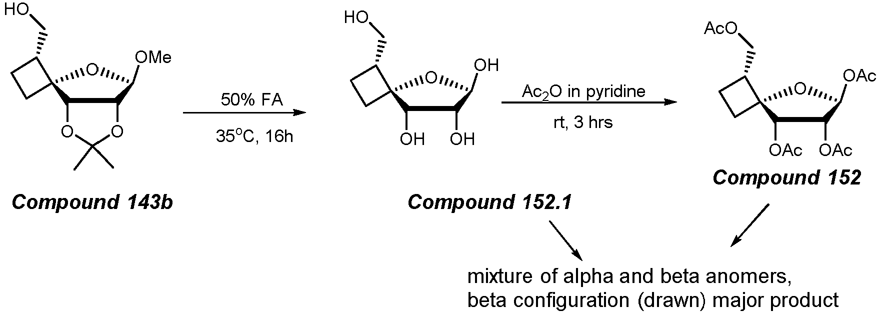

**Compound 143b** (186 mg, 0.76 mmol, 1.00eq) was dissolved in H₂O (2.0 mL) and formic acid (FA) (2.0 mL) was added. The mixture was heated to 50°C for 16 hours. Subsequently, the solution was cooled to room temperature, Subsequently, the solution was cooled to room temperature and concentrated in vacuo. This result in 194 mg of crude **compound 152.1.**

**Compound 152.1** (194 mg, crude) was dissolved in dry pyridine (2.0 mL) and stirred for 30 minutes. Acetic anhydride (116.28 mg, 1.14 mmol, 1.50 eq) was added at 0°C, was added to the stirring solution at room temperature. The mixture was stirred at room temperature for 3 hours. Subsequently, the mixture was poured into ice-cold water (5 ml) and stirred for 30 minutes at room temperature. The crude mixture was extracted with CH₂Cl₂ (3x 5 ml) and combined organic layers were washed with brine (3x 5 ml), dried (Na₂SO₄), filtered and the filtrate was concentrated *in vacuo.* The residue was purified by silica gel chromatography (gradient elution: PE / EA from 100:1 to 10:1). Fractions containing the product were combined and the solvent was removed in vacuo to afford the title ***compound 152.***

73% yield for 2 steps (198 mg, 0.55mmol, crude), colorless oil. LCMS (ESI+) m/z: calcd. for C₁₆H₂₂O₉ [M+H₂O] + = 376.30, found 376.30, RT: 1.283 min, Method 1.

### Preparation of compound 153

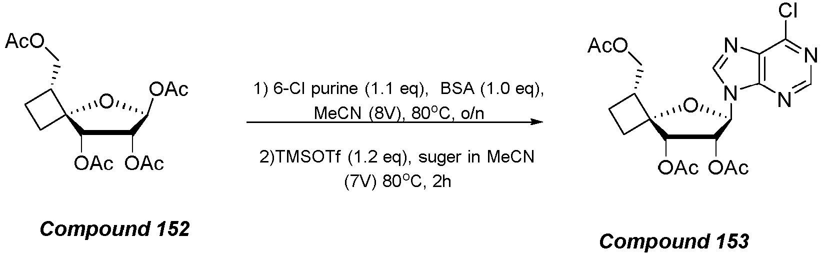

6-Chloropurine (93.17 mg,0.60 mmol, 1.10 eq) was dissolved in MeCN (1.4 mL), and N,O-bis(trimethylsilyl)acetamide (111.65 mg, 0.55 mmol, 1.00 eq) was added dropwise. The mixture was heated to 80°C for 16 hours. After the mixture was cooled to room temperature, **compound 152** (198 mg, 0.55 mmol, 1.00 eq) in MeCN (1.5 mL) was added, followed by trimethylsilyltrifluoromethanesulfonate (146.52 mg, 0.66 mmol, 1.20 eq) and the mixture was heated to 80°C for 2 hours. The mixture was cooled to room temperature and diluted with EtOAc (10 mL) , washed with saturated NaHCO₃ (3x10 mL) and saturated aq. NaCl (3 x 10 mL) . The organic layer was dried (Na₂SO₄), filtered and the filtrate was concentrated in vacuo. The residue was purified by silica gel column chromatography with PE/EtOAc (gradient elution: PE/EtOAc from 99:1 to 4:1). The fractions containing the product were collected and the solvent was removed in vacuo to afford the title ***compound** 153.*

57% yield (142 mg, 0.31mmol, crude), off-white solid. LCMS (ESI+) m/z: calcd. for C₁₉H₂₁ClN₄O₇ [M+H]+ = 453.2, found 453.2, RT: 1.311 min, Method 1.

### Preparation of compound 154

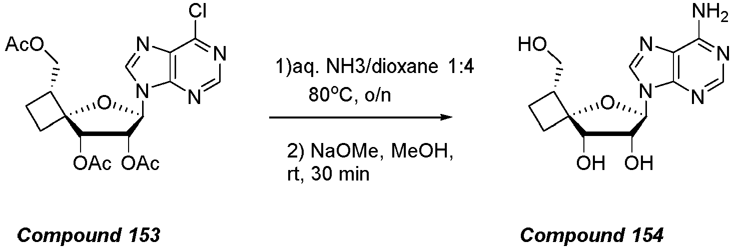

**Compound 153** (142 mg, 0.31 mmol, 1.00 eq) was dissolved in 1,4-dioxane (1.5 mL), and NH₃ (aq. 0.37 mL) was added. The mixture was heated to 80°C for 16 hours. After cooling the mixture to room temperature, solvents were removed *in vacuo* and the product was dissolved in MeOH (1.5 ml). Sodium methoxide (16.74 mg, 0.31 mmol, 1.00 eq) was added and the mixture was stirred at room temperature for 30 minutes, then stirred with hydrogen exchange resin (CAS: 78922-04-0) 30 min to PH=7. The resulting mixture was filtered and the filtrate was concentrated *in vacuo.* The residue was purified via silica gel column chromatography with dichloromethane/ methanol (gradient elution: DCM/MeOH from 99:1 to 5:1). The fractions containing the product were collected and the solvent was removed in vacuo to afford ***compound 154.***

**57% yield** (55 mg, 0.18 mmol), brown solid. **1H NMR (300 MHz, Methanol-d4)** δ 8.44 (s, 1H), 8.18 (s, 1H), 6.02 (d, J = 7.3 Hz, 1H), 4.94 (dd, J = 7.3, 4.5 Hz, 1H), 4.22 - 4.16 (m, 1H), 3.91 - 3.75 (m, 1H), 3.59 - 3.51 (m, 1H), 2.71 - 2.47 (m, 2H), 2.15 - 2.01 (m, 1H), 1.86 - 1.67 (m, 1H), 1.61 - 1.45 ppm (m, 1H). **13C NMR (75 MHz, Methanol-d4)** δ 156.03, 152.32, 149.28, 140.79, 119.28, 87.65, 87.36, 75.58, 72.74, 60.88, 27.70, 15.81, 7.85 ppm . LCMS (ESI+) m/z: calcd. for C₁₃H₁₇N₅O₄ [M+H]+ =308.13 found 308.13, RT: 0.502 min, Method 1.

### Preparation of cyclopropanes

### Preparation of Compound 67

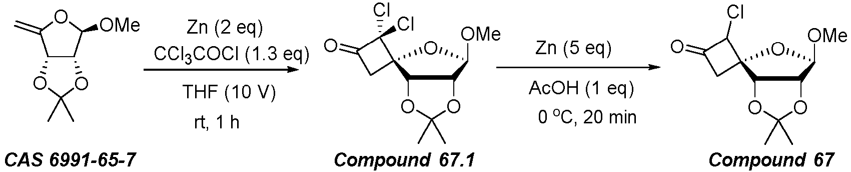

CAS 6991-65-7 (30 g, 161 mmol, 1.00 eq) was dissolved in THF (210 mL), zinc (21.1 g, 322.4 mmol, 2.00 equiv) was added. This was followed by the addition of a solution of trichloroacetyl chloride (38.1 g, 209.6 mmol, 1.30 equiv) in tetrahydrofuran (90 mL) dropwise with stirring at 0 °C. The resulting solution was stirred for 30 min at r.t. Then zinc (52.7 g, 806 mmol, 5.00 equiv), AcOH (9.7 g, 161.2 mmol, 1.00 equiv) was added at 0°C, the mixture was stirred at 0 °C for 20 minutes. The residue was added to saturated NaHCO₃ aqueous (500 mL) at 0 °C, the solids were filtered out, and the aqueous phase was extracted with EA (500 mL), the organic phase was dried over anhydrous sodium sulfate and concentrated *in vacuo.* The residue was purified by column chromatography over silica gel (gradient elution: PE/EtOAc from 99:1 to 5:1). The fractions containing the product were collected and the solvent was evaporated to afford ***compound 67.***

**73% yield** (2 steps from 1.5, 31.0 g, 114.5 mmol), colorless oil. **¹H NMR (300 MHz, Chloroform-*d*)** δ 5.01 (s, 1H), 4.97 (d, J = 5.7, 1H), 4.83 (dd, J = 3.9, 1.7 Hz, 1H), 4.68 (d, J = 5.7 Hz, 1H), 3.53 (dd, J = 18.7, 1.7 Hz, 1H), 3.47 - 3.42 (m, 1H), 3.41 (s, 3H), 1.44 (s, 3H), 1.35 ppm (s, 3H).**¹³C NMR (75 MHz, Chloroform-*d*)** δ197.20, 112.96, 108.57, 85.20, 81.86, 79.46, 69.65, 55.21, 49.85, 26.27, 25.12 ppm. **GCMS** (ESI⁺) *m*/*z*: for C₁₁H₁₅ClO₅ [M-CH₃O]⁺ = 231.04, found 231.01, RT: 7.277 min, Method: 1.

### Preparation of Compound 68.

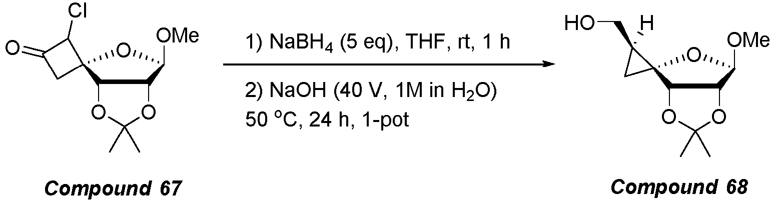

***Compound 67*** (27.0 g, 103 mmol, 1.00 eq) was dissolved in anhydrous THF (270 mL), cooled to 0 °C and NaBH₄ (19.6 g, 515 mmol, 5.00 eq) was added portionwise. The mixture was stirred at room temperature for 1 hour. Subsequently, NaOH (aq. 1M, 1080 mL) was added and stirring was continued for 24 hours at 50 °C. The product was extracted with EtOAc (3x500 mL) and combined the organic phase, dried (MgSO₄), filtered and concentrated *in vacuo.* The residue was purified by column chromatography over silica gel (gradient elution: n-heptane/EtOAc from 99:1 to 1:1). The fractions containing the product were collected and the solvent was evaporated to afford ***compound 68.***

**59% yield** (14 g, 60.84 mmol), colorless oil (storage in the fridge). **¹H NMR (300 MHz, Chloroform-d)** δ 4.94 (s, 1H), 4.68 (d, J = 5.9 Hz, 1H), 4.60 (d, J = 5.9 Hz, 1H), 3.88 (dd, J = 11.6, 5.8 Hz, 1H), 3.34 (s, 3H), 3.25 (dd, J = 11.6, 9.5 Hz, 1H), 1.51 (s, 3H), 1.46 - 1.39 (m, 1H), 1.35 (s, 3H), 1.23 (dd, J = 10.0, 6.9 Hz, 1H), 0.79 ppm (t, J = 6.9 Hz, 1H). GCMS (ESI+): RT: 6.798 min, for C₁₁H₁₈O₅ [M-CH₃O]⁺ =199.10, found 199.09, Method:1.

### Preparation of Compound 69

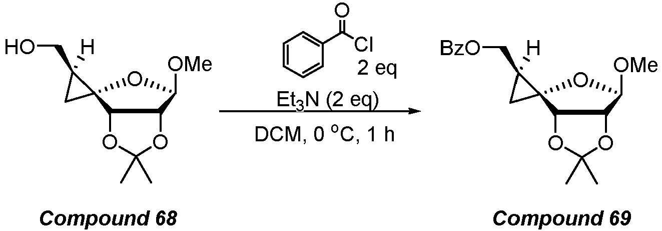

***Compound 68*** (12.0 g, 52.2 mmol, 1.00 eq), TEA (10.5 g, 104 mmol, 2.0 equiv) was dissolved in DCM (120 mL), then benzoyl chloride (14.6 g, 104.34 mmol, 2.00 equiv) was added dropwise at 0°C, the resulting solution was stirred for 1 h at 0°C. The reaction was then quenched with 1M HCl aqueous until pH<7. The resulting solution was diluted with 250 mL DCM and washed with 1x400 mL of H₂O and the organic layers combined. The mixture was dried over anhydrous sodium sulfate and concentrated *in vacuo.* The residue was applied onto a silica gel column with ethyl acetate/petroleum ether (gradient elution: PE/EtOAc from 99:1 to 20:1). The fractions containing the product were collected and the solvent was evaporated to afford ***compound 69.***

**68% yield** (14.0 g, 41.91 mmol), yellow oil. **¹H NMR (400 MHz, Methanol-d4)** δ 8.06 - 8.00 (m, 2H), 7.62 - 7.57 (m, 1H), 7.50 - 7.44 (m, 2H), 4.85 (s, 1H), 4.77 - 4.66 (m, 2H), 4.59 (d, J = 5.9 Hz, 1H), 3.86 (dd, J = 12.1, 10.2 Hz, 1H), 3.18 (s, 3H), 1.65 (m, 1H), 1.46 (s, 3H), 1.32 (s, 3H), 1.24 (dd, J = 10.1, 6.9 Hz, 1H), 0.89 ppm (t, J = 7.0 Hz, 1H). **¹³C NMR (101 MHz, Methanol-d4)** δ 167.83, 134.17, 131.60, 130.57, 129.50, 113.61, 108.63, 86.85, 81.47, 70.72, 66.02, 54.85, 26.78, 25.66, 24.09, 10.55 ppm. **LCMS** (ESI+) m/z: calcd. for C₁₈H₂₂O₆ [M+H]⁺ = 335.14, found 335.15, RT: 1.565 min, Method: 2.

### Preparation of Compound 70

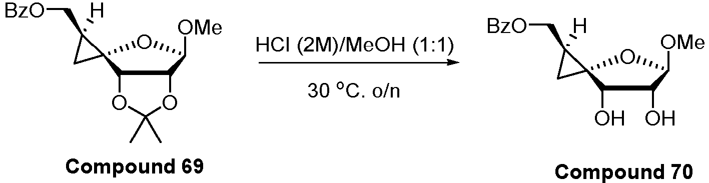

To ***compound 69*** (14.0 g, 41.9 mmol, 1.00 eq) in MeOH (140 mL) 2M HCl (140 mL) was added and the mixture was heated to 30 °C for overnight. Subsequently, the solution was cooled to room temperature and carefully quenched with NaHCO₃ to pH= 7. The mixture was extracted with CH₂Cl₂ (3x140 mL) and combined organic layers were dried (NaSO₄), filtrated and the filtrate was concentrated in vacuo. The residue was purified by column chromatography over silica gel (gradient elution: n-heptane/ EtOAc from 95:5 to 80:20). The fractions containing the product were collected and the solvent was evaporated to afford ***compound 70.***

**80% yield** (9.80 g, 33.33 mmol), brown solid. **¹H NMR (400 MHz, Methanol-d4)** δ 8.05 - 7.99 (m, 2H), 7.63 - 7.57 (m, 1H), 7.50 - 7.45 (m, 2H), 4.80 (d, J = 1.5 Hz, 1H), 4.60 (dd, J = 11.9, 6.3 Hz, 1H), 4.32 (d, J = 5.2 Hz, 1H), 4.08 (dd, J = 5.2, 1.5 Hz, 1H), 3.89 (dd, *J =* 11.9, 9.9 Hz, 1H), 3.27 (s, 3H), 1.68 - 1.60 (m, 1H), 1.06 (dd, J = 10.2, 6.6 Hz, 1H), 0.90 ppm (t, J = 6.7 Hz, 1H). **¹³C NMR (101 MHz, Methanol-d4)** δ 166.56, 132.82, 130.16, 129.13, 128.17, 107.73, 75.83, 69.88, 68.80, 64.62, 53.86, 20.03, 10.05 ppm, **LCMS** (ESI+) m/z: calcd. For C₁₅H₁₈O₆ [M-H]⁻ = 393.11, found 393.15, RT: 1.145 min, Method: 4.

### Preparation of Compound 71

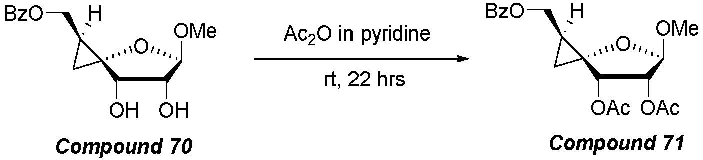

***Compound 70*** (9.80 g, 33.3 mmol, 1.0 eq) was dissolved in pyridine (100 mL) followed by the addition of acetic anhydride (30 mL) and stirred at room temperature for 3 h. The mixture was diluted in CH₂Cl₂ (200 mL), washed with brine (2x200 mL) and the organic layer was dried (Na₂SO₄), filtrated and the filtrate was concentrated in vacuo. The residue was purified by column chromatography over silica gel (gradient elution: n-heptane/ EtOAc from 95:5 to 80:20). The fractions containing the product were collected and the solvent was evaporated to afford ***compound 71.***

**66% yield** (8.0 g, 21.16 mmol), white solid. **¹H NMR (300 MHz, Methanol-d4)** δ 8.11 - 8.03 (m, 2H), 7.64 - 7.55 (m, 1H), 7.47 (ddd, J = 8.3, 6.7, 1.4 Hz, 2H), 5.54 (d, J = 5.6 Hz, 1H), 5.27 (dd, J = 5.6, 2.0 Hz, 1H), 5.00 (d, J = 2.0 Hz, 1H), 4.81 - 4.72 (m, 1H), 3.77 (dd, J = 12.2, 10.3 Hz, 1H), 3.26 (s, 3H), 2.07 (s, 6H), 1.74 (ddt, J = 10.3, 6.9, 4.8 Hz, 1H), 1.23 (dd, J = 10.2, 6.9 Hz, 1H), 0.89 ppm (t, J = 6.9 Hz, 1H). **¹³C NMR** (75 **MHz, Methanol-d4)** δ 170.41, 169.94, 166.39, 132.84, 129.97, 129.22, 128.17, 105.78, 76.90, 71.61, 67.88, 64.45, 54.15, 22.05, 19.11, 18.93, 10.14 ppm. **LCMS** (ESI+) m/z: calcd. for C₁₉H₂₂O₈ [M+Na]⁺ = 401.12, found 401.10, RT: 0.919 min, Method: 4.

### Preparation of Compound 72

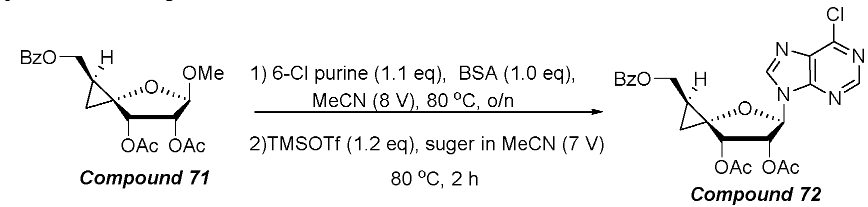

6-Cl Purine (3.50 g, 22.6 mmol, 1.1 equiv) was dissolved in MeCN (62.4 mL), and N,O-bis(trimethylsilyl)acetamide (4.19 g, 20.6 mmol, 1.00 equiv) was added dropwise. The mixture was heated to 80 °C for overnight. After the mixture had cooled, ***compound* 71** (7.8 g, 20.63 mmol, 1.0 equiv) in MeCN (54.6 mL) was added, Trimethylsilyltrifluoromethanesulfonate (5.49 g, 24.7 mmol, 1.20 equiv) was added, and the mixture was heated to 80 °C for 2 hours. Upon cooling to r.t, the mixture dissolved in EtOAc (100 mL) and washed twice with saturated NaHCO₃ (100 mL) and once with saturated aq. NaCl (100 mL). The organic layer was dried over Na₂SO₄. Solvents were removed *in vacuo.* The residue was applied onto a silica gel column with dichlormethane/methanol (gradient elution: DCM/MeOH from 99:1 to 30: 1). The fractions containing the product were collected and the solvent was evaporated to afford ***compound 72.***

78% yield (8.0 g, 16 mmol), white solid. **¹H NMR (300 MHz, Methanol-d4)** δ 8.38 (s, 1H), 8.21 (s, 1H), 6.08 (d, J = 6.0 Hz, 1H), 5.08 (dd, J = 6.0, 5.0 Hz, 1H), 4.25 (d, J = 5.1 Hz, 1H), 3.88 (dd, J = 11.5, 5.9 Hz, 1H), 3.25 (dd, J = 11.5, 9.7 Hz, 1H), 1.59 - 1.46 (m, 1H), 1.05 (dd, J = 10.3, 6.6 Hz, 1H), 0.89 ppm (t, J = 6.8 Hz, 1H). **LCMS** (ESI+) m/z: calcd. for C₂₃H₂₁ClN₄O₇ [M+H]⁺ = 501.11, found 501.20, RT: 0.920 min, Method: 4.

### Preparation of Compound 73

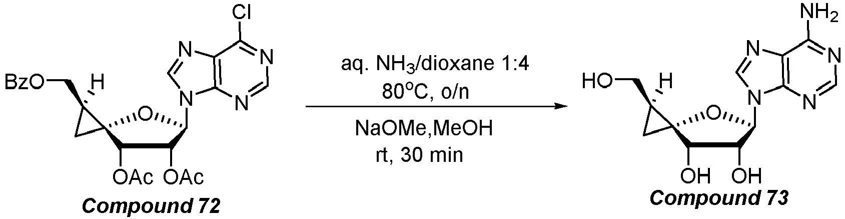

***Compound* 72** (8.0 g, 16 mmol, 1.0 equiv) was dissolved in 1,4-dioxane(80 mL), and aq.NH₃ (20 mL) was added. The mixture was heated to 80 °C for overnight. Upon cooling to r.t, then solvents was removed *in vacuo.* The product was dissolved in MeOH (80 mL), and sodium methoxide (0.86 g, 16 mmol, 1.00 equiv) was added. The reaction was stirred at RT for 30 min, then stirred with H exchange resin to pH=7. Then filtered, and the filtrate was concentrated under reduced pressure. The residue was applied onto a silica gel column with DCM/ MeOH (gradient elution: DCM/MeOH from 99:1 to 90:10). The fractions containing the product were collected and the solvent was evaporated to afford ***compound 73.***

**90% yield** (4.20 g, 14.33 mmol), white solid. **¹H NMR (300 MHz, DMSO-d6)** δ 8.39 (d, J = 2.6 Hz, 1H), 8.17 (d, J = 2.7 Hz, 1H), 7.32 (s, 2H), 5.97 (dd, J = 6.3, 2.6 Hz, 1H), 5.58 (dd, J = 6.7, 2.4 Hz, 1H), 5.19 (dd, J = 5.3, 2.4 Hz, 1H), 4.99 - 4.88 (m, 1H), 4.86 - 4.77 (m, 1H), 4.10 (td, J = 5.1, 2.5 Hz, 1H), 3.75 - 3.60 (m, 1H), 3.22 - 3.08 (m, 1H), 1.32 (d, J = 7.7 Hz, 1H), 0.88 (td, J = 7.9, 7.0, 3.6 Hz, 1H), 0.75 ppm (td, J = 6.7, 2.6 Hz, 1H). **¹³C NMR** (75 **MHz, DMSO-d6)** δ 155.97, 152.61, 149.54, 139.16, 118.77, 86.20, 75.24, 70.77, 69.91, 60.66, 24.41, 10.85 ppm. **LCMS** (ESI+) m/z: calcd. for C₁₂H₁₅N₅O₄ [M+H]⁺ = 294.11 found 294.12, RT: 0.477 min, Method: 3.

### Preparation of Compound 74

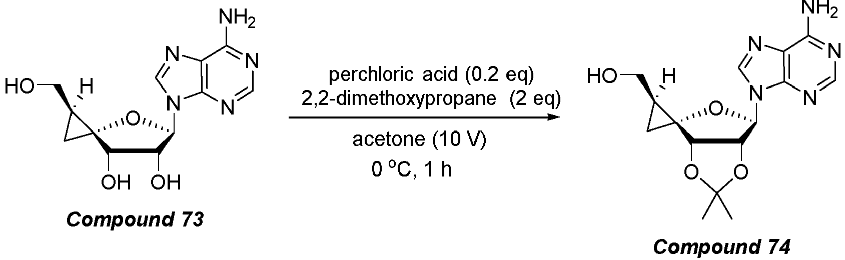

***Compound 73*** (2.0 g, 6.8 mmol, 1.00 equiv) was dissolved in acetone (20 mL), 2,2-dimethoxypropane (1.40 g, 13.5 mmol, 2.00 equiv) and perchloric acid (0.68 g, 6.8 mmol, 0.20 equiv) was added at 0 °C. The reaction was stirred at RT for 1 hour. The reaction was then added 1M NaOH aqueous until pH=7. The resulting solution was added 50 mL DCM and extracted with 1x60 mL of H₂O and the organic layers combined. The mixture was dried over anhydrous sodium sulfate and concentrated *in vacuo.* The residue was applied onto a silica gel column with dichlormethane/methanol (gradient elution: DCM/MeOH from 99:1 to 85:15). The fractions containing the product were collected and the solvent was evaporated to afford ***compound 74.***

**83% yield** (1.4 g, 4.20 mmol), light yellow oil. **¹H NMR (400 MHz, DMSO-d6)** δ 8.44 (s, 1H), 8.17 (s, 1H), 7.32 (s, 2H), 6.18 (s, 1H), 5.45 (d, J = 5.9 Hz, 1H), 5.02 (d, J = 6.0 Hz, 1H), 4.91 (t, J = 4.9 Hz, 1H), 3.63 (dt, J = 11.0, 5.2 Hz, 1H), 3.24 - 3.13 (m, 1H), 1.53 (s, 3H), 1.37 (s, 3H), 1.14 - 0.97 (m, 2H), 0.74 ppm (t, J = 5.7 Hz, 1H). **¹³C NMR (101 MHz, DMSO)** δ 156.54, 153.27, 149.57, 140.05, 118.80, 112.74, 87.38, 85.51, 80.55, 72.01, 61.07, 26.85, 26.50, 25.71, 10.37 ppm. **LCMS** (ESI+) m/z: calcd. for C₁₅H₁₉N₅O₄ [M+H]⁺ = 334.14, found 334.10, RT: 0.958 min, Method: 3.

### Preparation of Compound 75

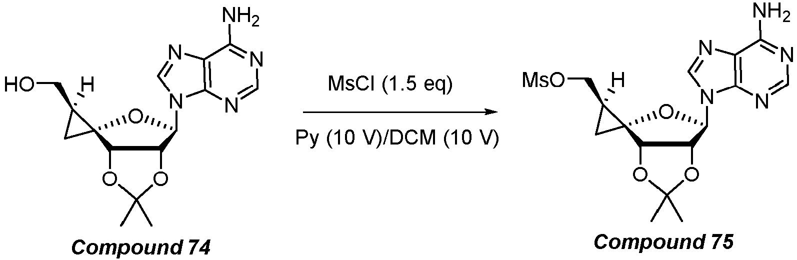

***Compound* 74** (300 mg, 0.90 mmol, 1.00 equiv) was dissolved in dichloromethane (3 mL), pyridine (3 mL) and MsCl (154 mg, 1.35 mmol, 1.50 equiv) was added at 0 °C. The reaction was stirred at 40 °C for 3 h. Then poured the reaction into 30 mL of ice water and extracted with 1x30 mL dichloromethane, and concentrated under reduced pressure giving crude ***compound 75*** which could be directly used in the next step without further purification. (300 mg, 0.73 mmol), light yellow oil. **LCMS** (ESI⁺) *m*/*z:* calcd. for C₁₆H₂₁N₅O₆S [M+H]⁺ =412.12, found 412.10, RT: 0.743 min, Method: 5.

### Preparation of Compound 76

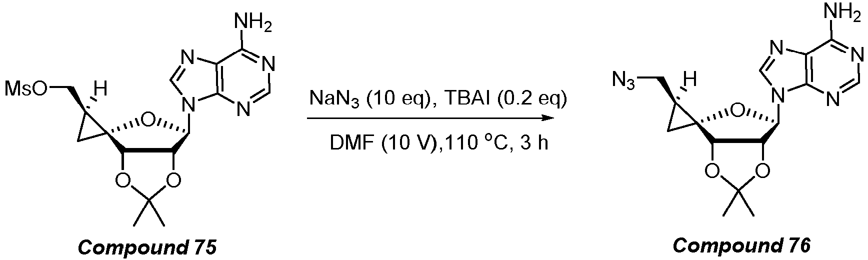

***Compound 75*** (300 mg, 0.73 mmol, 1.00 equiv) was dissolved in DMF (3 mL), tetrabutylammonium iodide (54 mg, 0.15 mmol, 0.20 equiv) and NaN₃ (475 mg, 7.30 mmol, 10.0 equiv) was added at 0 °C. The reaction was stirred at 110 °C for 3 h. Then poured the reaction into ice water 10 mL and extracted with 2x10 mL dichloromethane and concentrated under reduced pressure. The residue was applied onto a silica gel column with dichlormethane/methanol (gradient elution: DCM/MeOH from 99:1 to 90:10). The fractions containing the product were collected and the solvent was evaporated to afford ***compound 76.* 37% yield for 2 steps** (120 mg, 0.34 mmol), white solid. **¹H NMR (400 MHz, Methanol-d4)** δ 8.28 (s, 1H), 8.22 (s, 1H), 6.24 (s, 1H), 5.63 (d, J = 5.9 Hz, 1H), 5.06 (d, J = 6.0 Hz, 1H), 3.49 - 3.39 (m, 1H), 3.27 - 3.18 (m, 1H), 1.58 (s, 3H), 1.43 (s, 3H), 1.21 - 1.14 (m, 2H), 0.90 ppm (q, J = 4.4, 3.2 Hz, 1H). **¹³C NMR (101 MHz, Methanol-d4)** δ 157.44, 154.13, 150.58, 141.68, 120.21, 114.72, 90.12, 86.52, 81.67, 73.34, 52.56, 26.91, 25.83, 24.29, 11.96 ppm. **LCMS** (ESI⁺) *m*/*z:* calcd. for C₁₅H₁₈N₈O₃ [M+H]⁺ =359.15, found 359.15, RT: 1.272 min, Method: 2.

### Preparation of Compound 77

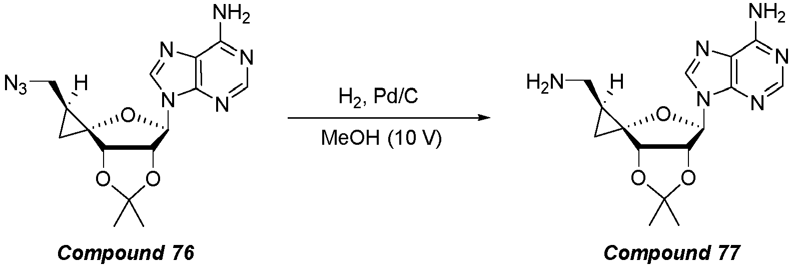

***Compound 76*** (90 mg, 0.25 mmol, 1.00 equiv) was dissolved in methanol (0.9 mL) and Pd/C (20 mg) was added. The reaction was stirred at r.t for overnight. Following completion, the solution was then filtered. The filter cake was washed with MeOH, and the filtrate was then concentrated under reduced pressure. The residue was applied onto a silica gel column with dichlormethane/methanol (gradient elution: DCM/MeOH from 99:1 to 80:20). The fractions containing the product were collected and the solvent was evaporated to afford ***compound 77.* 74% yield** (62 mg, 0.19 mmol), white solid. **¹H NMR (400 MHz, Methanol-d4)** δ 8.22 (d, J = 2.3 Hz, 2H), 6.21 (s, 1H), 5.66 (d, J = 5.6 Hz, 1H), 5.12 (dd, J = 5.6 Hz, 1H), 2.91 (dd, J = 12.5, 5.5 Hz, 1H), 2.60 - 2.47 (m, 1H), 1.58 (s, 3H), 1.42 (s, 3H), 1.13 (m, 1H), 1.04 (m, 1H), 0.87 - 0.78 ppm (m, 1H). **¹³C NMR (101 MHz, Methanol-d4)** δ 157.48, 154.10, 150.40, 142.33, 120.45, 114.65, 90.59, 86.43, 81.91, 74.10, 42.46, 26.93, 26.12, 25.86, 12.17 ppm. **LCMS** (ESI⁺) *m*/*z:* calcd. for C₁₅H₂₀N₆O₃ [M+H]⁺ =333.16, found 333.15, RT: 0.575 min, Method: 5.

### Preparation of Compound 78

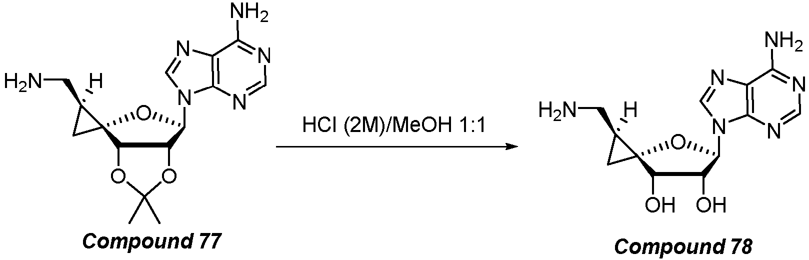

***Compound* 77** (62 mg, 0.19 mmol, 1.00 eq) in MeOH (0.6 mL) 2M HCl (0.6 mL) was added and the mixture was heated to 30°C for overnight. Subsequently, the solution was cooled to room temperature and carefully quenched with NaHCO₃ to pH= 7. The mixture was extracted with CH₂Cl₂ (3x 10 mL) and combined organic layers were dried (NaSO₄), filtrated and the filtrate was concentrated in vacuo. The residue was purified by column chromatography over silica gel (gradient elution: DCM/MeOH from 95:5 to 80:20). The fractions containing the product were collected and the solvent was evaporated to afford ***compound 78.***

**55% yield** (30 mg, 0.10 mmol), white solid. **¹H NMR (300 MHz, Methanol-d4)** δ 8.27 (s, 1H), 8.21 (s, 1H), 5.99 (d, J = 5.9 Hz, 1H), 5.14 (t, J = 5.6 Hz, 1H), 4.28 (d, J = 5.3 Hz, 1H), 2.82 - 2.68 (m, 2H), 1.50 - 1.38 (m, 1H), 1.01 (dd, J = 10.2, 6.5 Hz, 1H), 0.86 ppm (t, J = 6.8 Hz, 1H). **¹³C NMR (75 MHz, Methanol-d4)** δ 156.02, 152.48, 149.28, 140.46, 119.41, 88.36, 74.68, 71.13, 69.78, 40.58, 23.52, 11.15 ppm. **LCMS** (ESI⁺) *m*/*z:* calcd. for C₁₂H₁₆N₆O₃ [M+H]⁺ =293.13, found 293.05, RT: 0.558 min, Method 6.

### Preparation of Compound 101

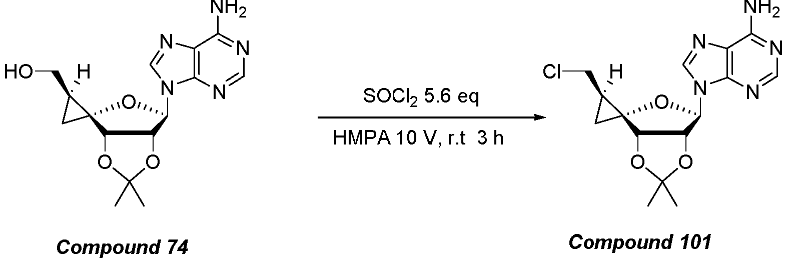

***Compound* 74** (200 mg, 0.60 mmol, 1.00 eq) was dissolved in HMPA (2.00 mL). SOCl₂ (397 mg, 3.36 mmol, 5.60 eq) was added. The mixture was allowed to stir at RT for 3h. Then pour the reaction into 5 ml of ice water and extract with 1x5 ml dichloromethane, and concentrated under reduced pressure giving crude ***compound 101.***

**LCMS(ESI+)** m/z:calcd. for C₁₅H₁₈CiN₅O₃[M+H]+ =352.11, found 371.15, RT:1.276min, Method 12.

### Preparation of Compound 102

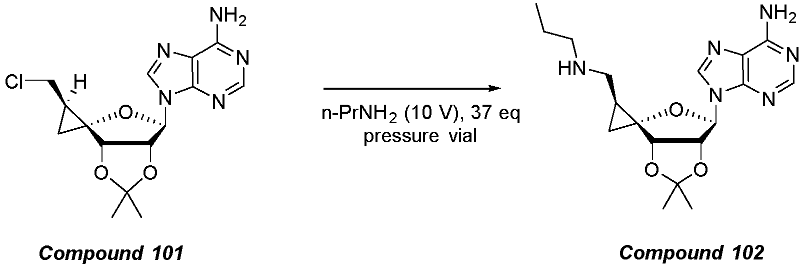

***Compound 101*** (180 mg,0.51 mmol, 1.00 eq) was dissolved in n-PrNH₂ (2 ml) and the mixture was heated to 90°C for 12 h. Subsequently, the solution was cooled to room temperature. The mixture was extracted with CH₂Cl₂ (3x 2 ml) and combined organic layers were dried (Na₂SO₄), filtrated and the filtrate was concentrated in vacuo. The residue was applied onto a silica gel column with dichloromethane/ methanol (gradient elution: DCM/MeOH from 99:1 to 67:33). The fractions containing the product were collected and the solvent was evaporated to afford ***compound 102.***

**52% yield** (100 mg, 0.26mmol), white solid. **1H NMR** (400 MHz, Methanol-d4) δ 8.30 (s, 1H), 8.06 (s, 1H), 6.21 (s, 1H), 5.64 (d, J = 6.0 Hz, 1H), 4.96 (d, J = 6.0 Hz, 1H), 3.81 (d, J = 13.1 Hz, 1H), 3.69 (d, J = 13.1 Hz, 1H), 3.37 (d, J = 1.3 Hz, 3H), 2.63 (dd, J = 12.7, 5.9 Hz, 1H), 2.49 (dd, J = 12.5, 6.8 Hz, 1H), 1.59 (s, 3H), 1.41 (s, 3H), 1.32 (d, J = 8.8 Hz, 2H), 1.13 - 1.08 (m, 2H), 0.77 ppm (d, J = 4.5 Hz, 1H). **13C NMR** (75 MHz, MeOD)δ 156.03, 152.65, 149.05, 140.75, 118.92, 113.15, 88.94, 84.96, 80.39, 72.26, 50.98, 49.25, 25.50, 24.46, 23.44, 22.08, 11.01, 10.62 ppm. **LCMS(ESI+)**m/z:calcd. for C₁₂H₁₆N₆O₃[M+H]+ =375.21, found 375.25, RT: 1.009 min, Method 12.

### Preparation of Compound 103

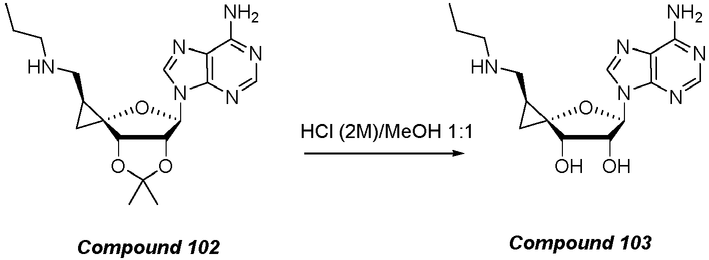

***Compound 102*** (100 mg, 0.26 mmol, 1.00 eq) in MeOH (1.0 ml) 2M HCl (1.0ml) was added and the mixture was heated to 40°C for 3h. Subsequently, the solution was cooled to room temperature and carefully quenched with NaHCO₃ to PH= 7. The filtrate was concentrated in vacuo. The residue was purified by Pre-HPLC (Column: Atlantis Prep T3 OBD Column, 19*250 mm 10u; Mobile Phase A: Water(10MMOL/L NH₄HCO₃), Mobile Phase B: ACN; Flow rate: 25 mL/min; Gradient: 2B to 8B in 7 min; 210/254 nm;). The fractions containing the product were collected and the solvent was evaporated to afford ***compound 103.***

**16% yield** (15mg, 0.04mmol), white solid. **1H NMR** (300 MHz, Methanol-d4) δ 8.28 (s, 1H), 8.22 (s, 1H), 6.00 (d, J = 5.7 Hz, 1H), 5.14 (t, J = 5.5 Hz, 1H), 4.28 (d, J = 5.4 Hz, 1H), 2.76 (dd, J = 12.5, 7.2 Hz, 1H), 2.70 - 2.59 (m, 3H), 1.60 (h, J = 7.2 Hz, 2H), 1.44 (dd, J = 10.2, 7.2 Hz, 1H), 1.08 - 1.02 (m, 1H), 0.98 (t, J = 7.5 Hz, 3H), 0.87 ppm (t, J = 6.9 Hz, 1H). **13C NMR** (75 MHz, MeOD) δ 156.03, 152.65, 149.05, 140.75, 118.92, 113.15. 88.30, 74.66, 70.97, 69.73, 50.97, 21.98, 21.22, 11.43, 10.58 ppm. LCMS(ESI+)m/z:calcd. for C₁₅H₂₀N₆O₃[M+H]+ =335.18, found 335.20, RT: 0.585 min, Method 3.

### Preparation of Compound 80

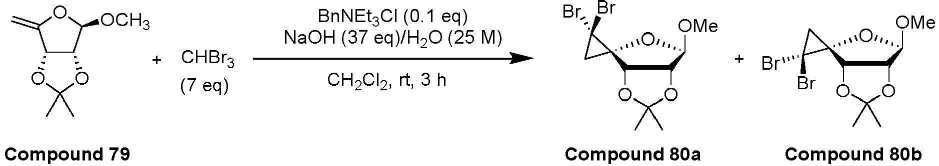

***Compound* 79 (**CAS 6991-65-7**)** (10 g, 53.70 mmol, 1 eq) and benzyltriethylammonium chloride (1.22 g, 5.37 mmol, 0.1 eq) were dissolved in a mixture of bromoform (32.87 mL, 375.93 mmol, 7 eq) and dichloromethane (72 mL) and a solution of NaOH (79.77 g, 1994.47 mmol, 25 M in H₂O, 27 eq) was added dropwise at room temperature and under argon atmosphere. The mixture was stirred at room temperature for 3 h. The product was extracted with Et₂O (3 x 100 mL) (ignore polymeric tar). The combined organic phase was washed with brine (100 mL), dried over Na₂SO₄, filtered and concentrated under reduced pressure.

The crude was purified over silica gel column chromatography (elution with Petroleum Ether (PE) 100%, then with PE/EtOAc = 95:5 and 90:10). The fractions corresponding to both diastereoisomers were collected and the solvent was evaporated to afford ***compound 80a***(13.65 g, 38.13 mmol, 71%) as an orange oil and ***compound 80b*** (1.10 g, 3.07 mmol, 6%) as a dark orange oil.

### Compound 80a:

**¹H NMR (400 MHz, Chloroform-d):** δ 5.13 (s, 1 H), 5.05 (d, *J =* 5.7 Hz, 1H), 4.71 (d, *J =* 5.7 Hz, 1 H), 3.56 (s, 3 H), 2.00 (d, *J =* 9.5 Hz, 1 H), 1.96 (d, *J* = 9.5 Hz, 1 H), 1.46 (s, 3 H), 1.37 ppm (s, 3 H).

**¹³C** NMR (100 **MHz,** Chloroform-d): δ 113.4, 108.9, 85.5, 82.0, 73.6, 57.2, 33.4, 29.6, 26.7, 26.1 ppm.

### Compound 80b:

**¹H** NMR (400 MHz, Chloroform-d): δ 5.08 (s, 1 H), 4.73 (d, *J =* 5.7 Hz, 1 H), 4.65 (d, *J =* 5.7 Hz, 1 H), 3.33 (s, 3 H), 1.98 (d, *J =* 9.5 Hz, 1 H), 1.83 (d, *J =* 9.5 Hz, 1H), 1.58 (s, 3 H), 1.38 ppm (s, 3 H).

**¹³C** NMR (100 **MHz,** Chloroform-d): δ 113.7, 109.0, 85.9, 84.9, 71.1, 55.4, 35.7, 26.6, 26.0, 22.9 ppm.

GCMS: [M]⁺· = 355, 19.85 min (Method 4)

### Preparation of Compound 81

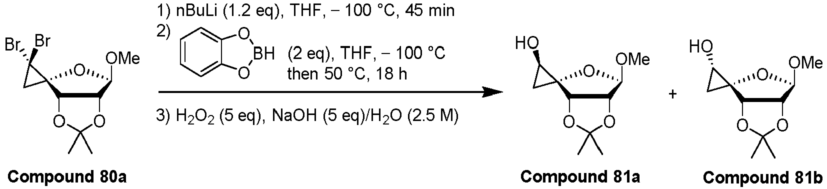

In a three-necked round bottom flask under argon atmosphere, ***compound 80a*** (5 g, 13.97 mmol, 1 eq) was dissolved in anhydrous THF (140 mL) under argon atmosphere. The solution was cooled to - 100 °C (ethanol/liquid nitrogen bath) and a solution of nBuLi (6.7 mL, 16.76 mmol, 2.5 M in hexane, 1.2 eq) was added dropwise and the reaction was stirred for 1 h. Then, a solution of catecholborane (27.9 mL, 27.9 mmol, 1 M in THF, 2 eq) was added and the resulting mixture was warmed to room temperature. Then, the reaction mixture was stirred for 16 h at 50 °C then was cooled to room temperature. A mixture of hydrogen peroxide (8 mL, 69.83 mmol, 30% in water, 5 eq) and sodium hydroxide (27.9 mL, 69.83 mmol, 2.5 M in water, 5 eq) was added and the reaction mixture was stirred at room temperature for 3 h. The reaction was quenched by adding 95 mL of a saturated aqueous solution of Na₂S₂O₃ and 65 mL of a saturated aqueous solution of NaHCO₃. The product was extracted with EtOAc (3 x 50 mL). The combined organic phases were washed with an aqueous saturated solution of Na₂S₂O₃ (50 mL) and brine (50 mL). The combined organic phase was washed, dried over Na₂SO₄, filtered and concentrated under reduced pressure.

The crude was purified over silica gel column chromatography (elution with PE/EtOAc = 90:10, 80:20 and then 70:30) to afford ***compound 81*** (1.22 g, 5.64 mmol, 40%) as a yellowish oil and as a mixture of ***compound 81a*** (major) and ***compound 81b*** (minor) with a ratio major/minor = 70:30. Both diastereoisomers were characterized by the NMR analysis of the mixture.

### Compound 81a:

**¹H** NMR (400 MHz, Chloroform-d): δ 4.94 (s, 1 H), 4.82 (d, *J =* 6.0 Hz, 1 H), 4.63 (d, *J =* 6.0 Hz, 1 H), 3.49 (dd, *J* = 7.7, 4.4 Hz, 1 H), 3.32 (s, 3 H), 2.58 (br s, 1 H), 1.51 (s, 3 H), 1.36 (s, 3 H), 1.21 (d, *J =* 7.8 Hz, 1 H), 1.04 ppm (dd, *J =* 8.2, 4.4 Hz, 1 H).

**¹³C** NMR (100 **MHz,** Chloroform-d): δ 112.5, 107.9, 85.2, 78.3, 69.5, 55.1, 54.4, 26.6, 25.6, 13.9 ppm.

### Compound 81b:

**¹H** NMR (400 MHz, **Chloroform-d):** δ 4.98 (s, 1 H), 4.65 (d, *J =* 6.0 Hz, 1 H), 4.28 (d, *J =* 5.9 Hz, 1 H), 3.46 (m, 1 H), 3.45 (s, 3 H), 2.17 (br s, 1 H), 1.51 (s, 3 H), 1.33 (s, 3 H), 1.21 (m, 1 H), 1.09 ppm (d, *J* = 4.3 Hz, 1 H).

**¹³C** NMR (100 **MHz,** Chloroform-d): δ 112.8, 108.9, 85.1, 83.5, 69.4, 56.4, 52.8, 26.5, 25.5, 14.0 ppm.

GCMS: [M-2H]⁺· = 214, 12.85 min (Method 4)

### Preparation of Compound 82

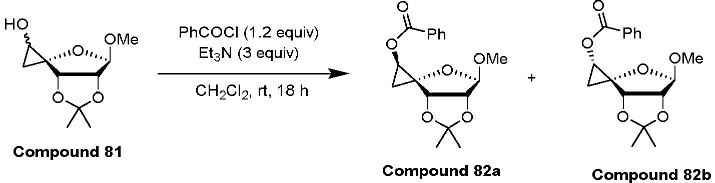

In a round bottom flask under argon atmosphere, *compound 81* (1 g, 4.62 mmol, 1 eq) was dissolved in dry dichloromethane (80 mL) and benzoyl chloride (0.64 mL, 5.55 mmol, 1.2 eq) and triethylamine (1.93 mL, 13.87 mmol, 3 eq) were added dropwise at 0 °C. The resulting reaction mixture was warmed at room temperature and stirred for 18 h. The volatile compounds were evaporated under reduced pressure.

The crude was purified over silica gel column chromatography (elution with PE/EtOAc = 95:5 then 90:10) to afford the desired compounds *compound **82a*** (1 g, 3.12 mmol, 67%) as a colorless oil and *compound 82b* (120 mg, 0.37 mmol, 8%) as a colorless oil. The two diastereoisomers were partially separated and the characterization was done for both isolated fractions. The stereochemistry was assigned by NMR analysis.

### Compound 82a:

**¹H** NMR (400 MHz, Chloroform-d): δ 7.91 (m, 2 H), 7.45 (m, 1 H), 7.32 (m, 2 H), 4.93 (s, 1 H), 4.72 (d, *J =* 5.9 Hz, 1 H), 4.60 (d, *J =* 5.9 Hz, 1 H), 4.39 (dd, *J* = 8.0, 4.7 Hz, 1 H), 3.28 (s, 3 H), 1.47 (d, *J =* 8.5 Hz, 1 H), 1.44 (s, 3 H), 1.28 (s, 3 H), 1.23 ppm (dd, *J* = 8.7, 4.7 Hz, 1 H).
**¹³C** NMR (100 **MHz,** Chloroform-d): δ 166.3, 133.7, 130.2, 129.7 (2C), 128.5 (2C), 112.8, 107.6, 85.3, 78.7, 68.4, 56.1, 54.9, 26.7, 25.9, 12.8 ppm.
LCMS **(ESI⁺):** [M+1]⁺ = 321.2, RT 1.11 min (Method 10)
GCMS: [M] ^{+.} = 320, 16.38 min (Method 4)

### Compound 82b:

**¹H** NMR (400 MHz, Chloroform-d): δ 8.05 (m, 2 H), 7.55 (m, 1 H), 7.43 (m, 2 H), 4.99 (s, 1 H), 4.70 (d, *J =* 5.9 Hz, 1 H), 4.50 (d, *J =* 5.9 Hz, 1 H), 4.42 (dd, *J =* 7.8, 4.8 Hz, 1 H), 3.14 (s, 3 H), 1.52 (s, 3 H), 1.49 (d, *J =* 8.7 Hz, 1 H), 1.40 (dd, *J =* 8.7, 4.9 Hz, 1 H), 1.35 ppm (s, 3 H). **¹³C** NMR (100 **MHz,** Chloroform-d): δ 166.6, 133.7, 133.3, 129.7 (2C), 128.6 (2C), 112.9, 108.6, 85.4, 83.2, 69.0, 55.0, 55.6, 26.5, 25.8, 11.4 ppm.
LCMS **(ESI⁺)**: [M+1]⁺ = 321.4, RT 1.07 min (Method 10)
GCMS: [M] ^{+.} = 320, 16.77 min (Method 4)

### Preparation of Compound 83

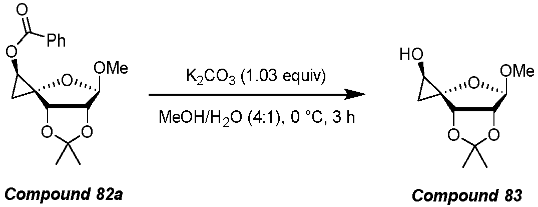

*Compound **82a*** (40 mg, 0.12 mmol, 1 eq) was dissolved in methanol (2.4 mL) then distilled water (0.48 mL) was added. The reaction mixture was cooled to 0°C and K₂CO₃ (17.7 mg, 0.12 mmol) was added at once. The flask was sonicated for 30 seconds at 0 °C to ensure a homogeneous mixture. The reaction mixture was stirred for 3 h at 0°C. The mixture was quenched with an aqueous buffer solution pH = 4 (24 ml) (Fluka, cat. n° 33665). The product was extracted with EtOAc (3 x 10 mL). The combined organic phases were washed with brine (2 x 10 mL). The combined organic phase was dried over Na₂SO₄, filtered and concentrated under reduced pressure.

The crude was purified over silica gel column chromatography (elution with PE/EtOAc = 85:15 then 70:30) to afford **compound 83** (11 mg, 0.05 mmol, 41%) as a colorless oil.

**¹H NMR (300 MHz, Chloroform-d):** δ 4.94 (s, 1 H), 4.83 (d, *J* = 6.0 Hz, 1 H), 4.63 (d, *J =* 6.0 Hz, 1H), 3.52 (dd, *J* = 7.7, 4.4 Hz, 1 H), 3.32 (s, 3 H), 1.51 (s, 3 H), 1.36 (s, 3 H), 1.21 (d, *J =* 7.9 Hz, 1 H), 1.05 ppm (dd, J = 8.2, 4.4 Hz, 1 H). **¹³C NMR (100 MHz, CDCl₃):** δ 112.5, 107.9, 85.2, 78.3, 69.5, 55.1, 54.4, 26.6, 25.6, 14.0 ppm. GCMS: [M-2H] ^{+.} = 214, 12.85 min (Method 4).

### Preparation of Compound 134

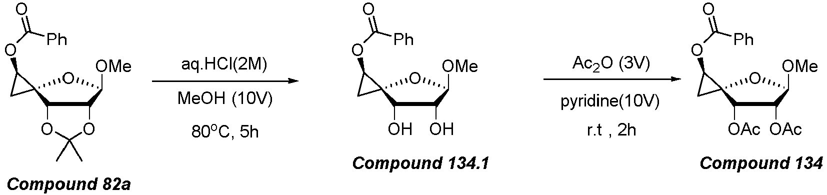

***Compound 82a*** (0.6 g, 1.93mmol, 1.00 eq) was dissolved in MeOH (6 mL), 2M aq. HCl (6 mL) was added and the mixture was heated to 40°C for 5 hours. Subsequently, the solution was cooled to room temperature and carefully quenched with NaHCO₃ to pH=7. The mixture was extracted with CH₂Cl₂ (3x 6 mL) and combined organic layers were dried (Na₂SO₄), filtrated and the filtrate concentrated under reduced pressure giving ***compound 134.1*** (0.47g, crude) which was used in next step. The ***compound 134.1*** residue (0.47 g,1.74 mmol, 1.00 eq) was dissolved in pyridine (4.7mL) followed by the addition of acetic anhydride (1.5 mL) and stirred at room temperature for 2h. The mixture was diluted in CH₂Cl₂ (10 mL), washed with brine (2x 10 mL) and the organic layer was dried (Na₂SO₄), filtrated and the filtrate was concentrated in vacuo. The residue was purified by column chromatography over silica gel (gradient elution: n-heptane/EtOAc from 95:5 to 80:20). The fractions containing the product were collected and the solvent was evaporated to afford ***compound** 134* as a yellow solid (0.35 g, 0.96 mmol, 51% for 2 steps).

**¹H** NMR (300 MHz, Chloroform-d) δ 8.05 - 7.98 (m, 2H), 7.62 - 7.53 (m, 1H), 7.50 - 7.41 (m, 2H), 5.70 (d, J = 5.6 Hz, 1H), 5.38 (dd, J = 5.6, 1.7 Hz, 1H), 5.08 (d, J = 1.7 Hz, 1H), 4.51 (dd, J = 8.1, 4.6 Hz, 1H), 3.43 (s, 3H), 2.12 (s, 3H), 2.08 (s, 3H), 1.56 (t, J = 8.5 Hz, 1H), 1.24 ppm (dd, J = 8.8, 4.6 Hz, 1H). **¹³C** NMR (75 MHz, CDCl₃) δ 169.54, 166.36, 133.38, 129.69, 129.19, 128.49, 106.27, 98.38, 76.00, 69.91, 66.20, 55.42, 54.61, 20.48, 13.78, 13.49 ppm. **LCMS(ESI+)** m/z:calcd. For C₁₈H₂₀O₈[M+NH4]+ =382.12 found 382.150 RT:1.493min, Method: 1.

### Preparation of Compound 135

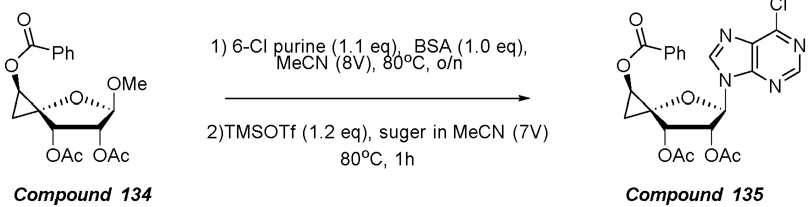

6-Cl Purine (0.15 g, 1.0mmol, 1.10equiv) was dissolved in MeCN (2.3mL), and N,O-bis(trimethylsilyl)acetamide (0.18 g, 0.91 mmol, 1.00 equiv) was added dropwise. The mixture was heated to 80°C for overnight. After the mixture had cooled, ***compound 134*** (0.33 g, 0.91 mmol, 1.00 equiv) in MeCN (2.6 mL) was added, trimethylsilyltrifluoromethanesulfonate (0.24 g, 1.1 mmol, 1.20 equiv) was added, and the mixture was heated to 80°C for 1 h. Upon cooling to rt, the mixture was extracted with EtOAc twice, once with saturated NaHCO₃ and once with saturated aq. NaCl. The organic layer was dried over Na₂SO₄. Solvents were removed under vacuum. The residue was applied onto a silica gel column with n-heptane/ EtOAc (gradient elution: n-heptane/EtOAc from 99:1 to 80:20). The fractions containing the product were collected and the solvent was evaporated to afford ***compound 135*** as a white solid (0.27 g, 0.55 mmol, **57%).**

**¹H NMR** (300 MHz, Chloroform-d) δ 9.01 (s, 1H), 8.75 (s, 1H), 8.12 - 8.05 (m, 2H), 7.68 - 7.60 (m, 1H), 7.54 - 7.46 (m, 2H), 6.58 (d, J = 6.8 Hz, 1H), 6.21 (dd, J = 6.8, 5.0 Hz, 1H), 5.52 (d, J = 4.9 Hz, 1H), 4.37 (dd, J = 8.2, 5.3 Hz, 1H), 2.22 (s, 3H), 2.01 (s, 3H), 1.62 - 1.56 ppm (m, 2H). **¹³C NMR** (75 MHz, CDCl3) δ 169.84, 169.06, 167.45, 152.33, 152.05, 151.46, 143.91, 134.00, 131.88, 129.97, 128.70, 128.33, 85.07, 75.49, 71.39, 68.83, 55.53, 20.66, 20.18, 13.85 ppm. LCMS(ESI+)m/z:calcd. For C₂₂H₁₉ClN₄O₇ [M+H]+ =486.09 found 487.10, RT: 1.507min, Method 1.

### Preparation of Compound 136

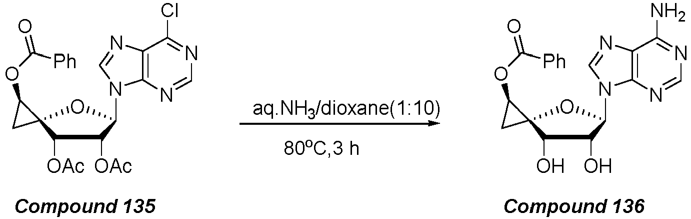

***Compound 135*** (0.13 g, 0.27 mmol, 1.00 equiv) was dissolved in 1,4-dioxane(1.3 mL), and aq. NH3 (0.13 mL) was added. The mixture was heated to 80°C for 3 hours. Upon cooling to r.t, then solvents were removed under vacuum. The residue was applied onto a silica gel column with DCM / MeOH (gradient elution: DCM / MeOH from 99:1 to 92:08). The fractions containing the product were collected and the solvent was evaporated to afford ***compound 136*** as a yellow oil (65 mg, 0.17 mmol, **64%**).

**¹H NMR** (300 MHz, DMSO-d6) δ 8.50 (s, 1H), 8.14 (s, 1H), 8.06 - 7.95 (m, 2H), 7.71 (t, J = 7.4 Hz, 1H), 7.57 (t, J = 7.6 Hz, 2H), 6.10 (d, J = 6.7 Hz, 1H), 5.67 (d, J = 6.7 Hz, 1H), 5.52 (d, J = 5.0 Hz, 1H), 4.88 (q, J = 6.2 Hz, 1H), 4.45 (dd, J = 7.6, 5.4 Hz, 1H), 4.10 (q, J = 5.3 Hz, 1H), 1.48 - 1.39 (m, 2H) ppm. **¹³C NMR** (75 MHz, DMSO) δ 166.98, 156.53, 153.32, 150.35, 139.27, 134.32, 129.79, 129.37, 129.26, 119.29, 86.84, 75.83, 69.99, 55.75, 49.06, 14.13 ppm. **LCMS(ESI+)m/z:calcd.** For C₁₈H₁₇N₅O₅[M+H]+ =384.12 found 384.15, RT:0.904min, Method 1.

### Preparation of Compound 137

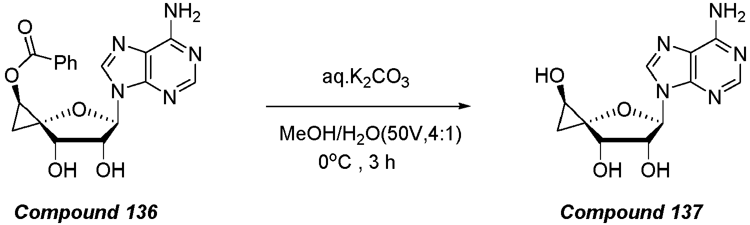

***Compound 136*** (50 mg, 0.13 mmol, 1.00 equiv) was dissolved in MeOH(2.5 mL) and water(0.5mL), then aq.K₂CO₃ (0.1 mL , 0.3 mol/L) was added dropwise at 0°C and stirred at 0°C for 3h . After completion, the reaction was carefully quenched with citric acid aqueous solution (0.2 mol/L) to PH=7. Then, the solvent was removed under vacuum. The residue was applied onto a silica gel column with DCM / MeOH (gradient elution: DCM / MeOH from 99:1 to 90:10). The fractions containing the product were collected and the solvent was evaporated to afford ***compound 137*** as a white solid (16 mg, 0.05 mmol, **44%**).

**¹H NMR** (300 MHz, Methanol-d4) δ 8.28 (s, 1H), 8.23 (s, 1H), 6.11 (d, J = 6.8 Hz, 1H), 5.10 (dd, J = 6.7, 5.0 Hz, 1H), 4.31 (d, J = 5.0 Hz, 1H), 3.54 (dd, J = 7.6, 5.0 Hz, 1H), 1.14 - 1.06 ppm (m, 2H). **¹³C NMR** (75 MHz, MeOD) δ 155.93, 152.53, 149.55, 139.87, 119.17, 87.92, 75.20, 70.97, 69.45, 52.35, 14.63 ppm. **LCMS (ESI+)** m/z:calcd. For C₁₁H₁₃N₅O₄ [M+H]+ =280.10 found 280.10, RT:0.346min, Method 1.

### Preparation of Compound 104

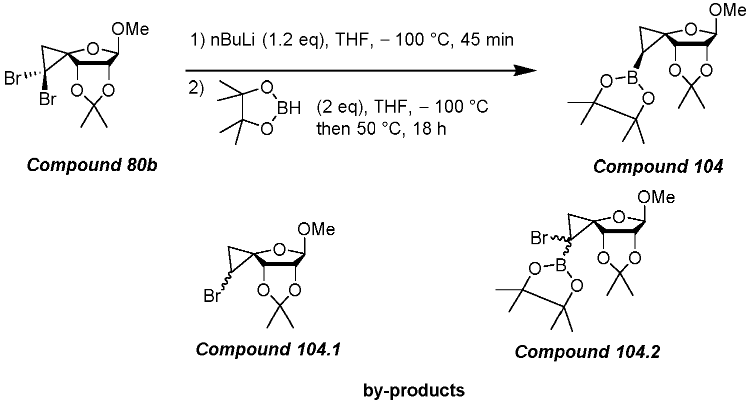

In a two-necked round bottom flask under argon atmosphere, ***compound 80b*** (320.8 mg, 0.9 mmol, 1 eq) was dissolved in anhydrous THF (9 mL). The solution was cooled to - 100 °C (ethanol/liquid nitrogen bath) and a solution of nBuLi (0.43 mL, 1.07 mmol, 2.5 M in hexane, 1.2 eq) was added dropwise and the reaction was stirred for 45 min. Then, a solution of pinacolborane (1.8 mL, 1.8 mmol, 1 M in THF, 2 eq) was added dropwise and the resulting mixture was warmed to room temperature. Then, the reaction mixture was stirred for 18 h at 50 °C. The reaction was quenched by adding 20 mL of a saturated aqueous solution of NaHCO₃. The product was extracted with EtOAc (3 × 10 mL). The combined organic phases were washed with water (20 mL) and brine (20 mL). The combined organic phase was dried over Na₂SO₄, filtered and concentrated under reduced pressure. The analysis of the ¹H NMR spectrum of the crude mixture indicated that one isomer of the product was obtained accompanied by at least two main by-products.

The crude was purified over silica gel column chromatography (elution with PE/EtOAc = 98:2, 95:5 and 90:10) to afford: fraction 1 (42 mg, 0.15 mmol, 17%) as a yellowish oil and as two isomers of the mono-brominated by-product ***compound 104.1*** (major/minor = 82:18) accompanied of traces of the completely debrominated by-product; fraction 2 (40.5 mg, 0.12 mmol, 14%) as a colorless oil and as one isomer of the desired product, ***compound 104;*** fraction 3 (26.3 mg) as a yellowish oil and as a mixture of a by-product likely to be the *gem*-Br,Bpin-cyclopropane ***compound 104.2*** (16.8 mg, 0.04 mmol, 5%) and the desired product ***compound 104*** (9.5mg, 0.03 mmol, 3%) (by-product/product = 51:49); and fraction 4 (16.2 mg, 0.05 mmol, 5.5%) as yellowish oil and as the desired product ***compound 104,*** the same as in fraction 2. The total isolated yield of ***compound 104*** is 19% and the overall yield is 22.5%. The characterisation of the product was done by analyzing fractions 2 and 4. The stereochemistry of the by-products was not determined.

**¹H NMR (400 MHz, CDCl₃):** δ 4.88 (s, 1 H), 4.60 (d, *J =* 5.9 Hz, 1 H), 4.52 (d, *J* = 5.9 Hz, 1 H), 3.28 (s, 3 H), 1.46 (s, 3 H), 1.27 (s, 3 H), 1.23 (s, 6 H), 1.21 (s, 6 H), 1.02-0.95 (m, 2 H), 0.54 (dd, *J =* 10.9, 8.2 Hz, 1 H) ppm. **¹³C NMR (100 MHz, CDCl₃):** δ 112.0, 108.1, 85.5, 83.5 (2 C), 83.1, 72.2, 54.8, 26.6, 25.8, 24.92 (2 C), 24.86 (2 C), 19.9 (2 C) ppm. **GCMS:** [M] ^{+•} =326, 14.61 min (Method 4).

### Preparation of Compound 105

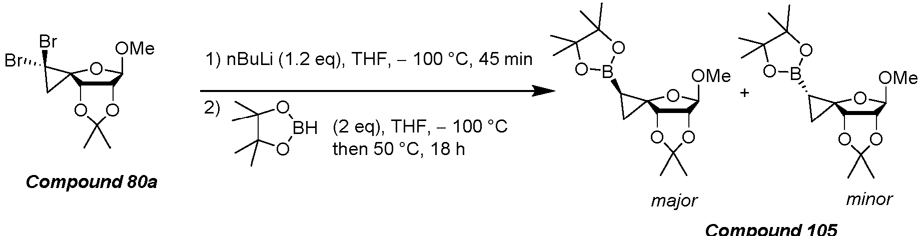

In a two-necked round bottom flask under argon atmosphere, ***compound 80a*** (332.2 mg, 0.9 mmol, 1 eq) was dissolved in anhydrous THF (9 mL). The solution was cooled to - 100 °C (ethanol/liquid nitrogen bath) and a solution of *n*BuLi (0.43 mL, 1.08 mmol, 2.5 M in hexane, 1.2 eq) was added dropwise and the reaction was stirred for 45 min. Then, a solution of pinacolborane (1.8 mL, 1.8 mmol, 1 M in THF, 2 eq) was added dropwise and the resulting mixture was warmed to room temperature. Then, the reaction mixture was stirred for 18 h at 50 °C. The reaction was quenched by adding 20 mL of a saturated aqueous solution of NaHCO₃. The product was extracted with EtOAc (3 × 10 mL). The combined organic phases were washed with water (20 mL) and brine (20 mL). The combined organic phase was dried over Na₂SO₄, filtered and concentrated under reduced pressure. The analysis of the ¹H NMR spectrum of the crude mixture indicated that both diastereoisomers were obtained with a ratio *major*/*minor =* 66:34. The crude was purified over silica gel column chromatography (elution with PE/EtOAc = 95:5, 90:10 and 80:20) to afford a fraction containing both isomers of the product but contaminated with pinacolborane and an unknown impurity. This fraction was purified over silica gel column chromatography (elution with PE/EtOAc = 95:5, 90:10, 93:7 and 91:9) to afford ***compound 105*** (153.7 mg, 0.47 mmol, 52%) as a colorless oil and as a mixture of two isomers (ratio *major*/*minor =* 63:37).

**GCMS:** [M]^{+•} = 326, 15.26 min (Method 4).

**Major isomer: ¹H NMR (400 MHz, CDCl₃):** δ 4.97 (s, 1 H), 4.65 (d, *J =* 5.9 Hz, 1 H), 4.45 (d, *J =* 5.9 Hz, 1 H), 3.39 (s, 3 H), 1.49 (s, 3 H), 1.33 (s, 3 H), 1.25 (s, 6 H), 1.23 (m, 1H), 1.22 (s, 6 H), 1.18 (m, 1 H), 0.31 ppm (dd, *J =* 11.1, 8.5 Hz, 1 H). **¹³C NMR (100 MHz, CDCl₃):** δ 112.7, 109.4, 85.7, 85.4, 83.4 (2 C), 73.1, 56.1, 29.8, 26.7, 26.2, 25.5 (2 C), 24.5 (2 C), 10.8 ppm. **GCMS:** [M]^{+•} = 326, 15.24 min (Method 4).

**Minor isomer: ¹H NMR (400 MHz, CDCl₃):** δ 4.91 (s, 1 H), 4.79 (d, *J =* 5.9 Hz, 1 H), 4.61 (d, *J=* 5.9 Hz, 1 H), 3.28 (s, 3 H), 1.51 (s, 3 H), 1.34 (s, 3 H), 1.28 (m, 1 H), 1.24 (s, 12 H), 1.04 (dd, **J** = 8.7, 5.6 Hz, 1 H), 0.34 ppm (dd, *J =* 11.3, 8.7 Hz, 1 H). **¹³C NMR (100 MHz, CDCl₃):** δ 112.3, 108.2, 85.4, 83.4 (2 C), 82.0, 72.1, 55.0, 29.5, 26.6, 25.7, 25.0 (2 C), 24.7 (2 C), 10.1 ppm. **GCMS:** [M]^{+•} = 326, 14.39 min (Method 4).

### Preparation of Compound 84

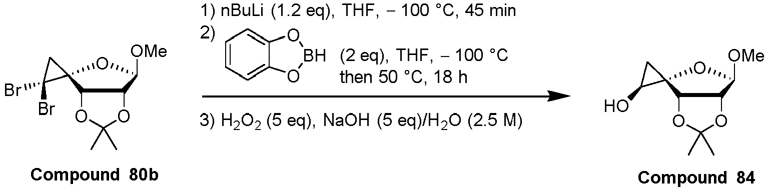

In a three-necked round bottom flask under argon atmosphere, ***compound 80b*** (1 g, 2.79 mmol, 1 eq) was dissolved in anhydrous THF (28 mL) under argon atmosphere. The solution was cooled to - 100 °C (ethanol/liquid nitrogen bath) and a solution of nBuLi (1.34 mL, 3.35 mmol, 2.5 M in hexane, 1.2 eq) was added dropwise and the reaction was stirred for 45 min. Then, a solution of catecholborane (5.59 mL, 5.59 mmol, 1 M in THF, 2 eq) was added and the resulting mixture was warmed to room temperature. Then, the reaction mixture was stirred for 18 h at 50 °C then was cooled to room temperature. A mixture of hydrogen peroxide (1.6 mL, 13.97 mmol, 30% in water, 5 eq) and sodium hydroxide (5.59 mL, 13.97 mmol, 2.5 M in water, 5 eq) was added and the reaction mixture was stirred at room temperature for 3 h. The reaction was quenched by adding 19 mL of a saturated aqueous solution of Na₂S₂O₃ and 13 mL of a saturated aqueous solution of NaHCO₃. The product was extracted with EtOAc (3 × 20 mL). The combined organic phases were washed with an aqueous saturated solution of Na₂S₂O₃ (20 mL) and brine (20 mL). The combined organic phase was washed, dried over Na₂SO₄, filtered and concentrated under reduced pressure.

The crude was purified over silica gel column chromatography (elution with PE/EtOAc = 90:10, 80:20 and then 70:30) to afford ***compound 84*** (338.9 mg, 1.57 mmol, 56%) as a single diastereoisomer and as an orange oil.

### Compound 84:

**¹H NMR (400 MHz, Chloroform-*d*):** δ 4.95 (s, 1 H), 4.78 (d, *J =* 5.9 Hz, 1 H), 4.70 (d, *J =* 5.9 Hz, 1 H), 3.83 (dd, *J =* 7.5, 4.1 Hz, 1 H), 3.27 (s, 3 H), 3.03 (br s, 1 H), 1.54 (s, 3 H), 1.34 (s, 3 H), 1.09 (dd, *J =* 7.3, 4.1 Hz, 1 H), 1.04 ppm (m, 1 H).
**¹³C NMR (100 MHz, Chloroform-*d*):** δ 113.1, 108.3, 85.4, 84.3, 69.3, 54.9, 50.2, 26.5, 25.3, 22.8 ppm.

### Preparation of Compound 85

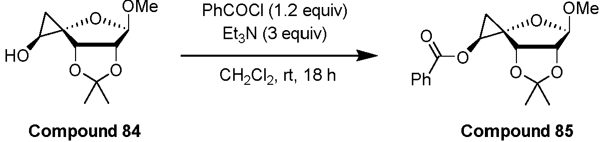

In a round bottom flask under argon atmosphere, ***compound 84*** (25 mg, 0.12 mmol, 1 eq) was dissolved in dry dichloromethane (1.6 mL) and benzoyl chloride (0.016 mL, 0.14 mmol, 1.2 eq) and triethylamine (0.048 mL, 0.35 mmol, 3 eq) were added dropwise at 0 °C. The resulting reaction mixture was warmed at room temperature and stirred for 18 h. The volatile compounds were evaporated under reduced pressure.

The crude was purified over silica gel column chromatography (elution with PE/EtOAc = 95:5 then 90:10) to afford ***compound 85*** (31.7 mg, 0.099 mmol, 85%) as a colorless oil.

### Compound 85:

**¹H NMR (400 MHz, Chloroform-*d*):** δ 8.03-8.00 (m, 2 H), 7.56 (m, 1 H), 7.45-7.41 (m, 2 H), 5.01 (s, 1 H), 4.83 (dd, *J =* 8.2, 4.5 Hz, 1 H), 4.74 (d, *J =* 5.9 Hz, 1 H), 4.71 (d, *J =* 5.9 Hz, 1 H), 3.32 (s, 3 H), 1.46 (s, 3 H), 1.36 (t, *J =* 8.0 Hz, 1 H), 1.26 (s, 3 H), 1.23 ppm (m, 1 H).
**¹³C NMR (100 MHz, Chloroform-*d*):** δ 167.0, 133.1, 130.2, 129.8 (2 C), 128.4 (2 C), 113.1, 108.9, 85.4, 84.0, 68.0, 55.1, 52.0, 26.5, 25.6, 20.7 ppm.
**LCMS** : **(ESI⁺):** [M+18]⁺ = 338.3, RT 1.09 min (Method 10)

### Preparation of Compound 106

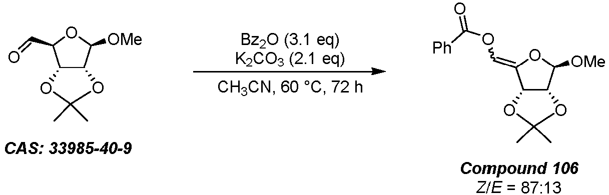

In a round bottom flask under argon atmosphere, to a solution of CAS 33985-40-9 (200 mg, 0.99 mmol, 1 eq) in dry acetonitrile (6 mL), potassium carbonate (287 mg, 2.08 mmol, 2.1 eq) and benzoic anhydride (708 mg, 3.07 mmol, 3.1 eq) were added and the mixture was heated at 60 °C for 72 h (the conversion of the substrate didn't go further). The reaction mixture was cooled to room temperature then the solid was filtered and washed well with acetonitrile. The filtrate was concentrated under reduced pressure and the residue was diluted with EtOAc (30 mL). The solution was washed brine (2 × 10 mL) and the organic phase was dried over Na₂SO₄ and concentrated under reduced pressure. The residue was purified over silica gel column chromatography (elution with PE 100% then PE/EtOAc = 95:5) to afford ***compound 106*** (123 mg, 0.40 mmol, 40%) as a pale pink oil and as a mixture of two inseparable isomers Z and E with a ratio *Z*/*E* = 87:13.
**¹H NMR (400 MHz, CDCl₃): (*Z* isomer)** δ 8.15-8.12 (m, 2 H), 7.59 (m, 1 H), 7.49-7.45 (m, 2 H), 7.18 (s, 1 H), 5.26 (s, 1 H), 5.19 (d, *J =* 6.2 Hz, 1 H), 4.59 (d, *J =* 6.0 Hz, 1 H), 3.47 (s, 3 H), 1.49 (s, 3 H), 1.37 ppm (s, 3 H). **¹³C NMR (100 MHz, CDCl₃): (*Z* isomer)** δ 163.2, 143.2, 133.6, 130.2 (2 C), 129.2, 128.7 (2 C), 117.7, 113.6, 109.5, 82.8, 78.2, 55.9, 26.8, 25.7 ppm.
**GCMS:** [M] ^{+•} =306, 16.60 min (Method 4),

### Preparation of Compound 107

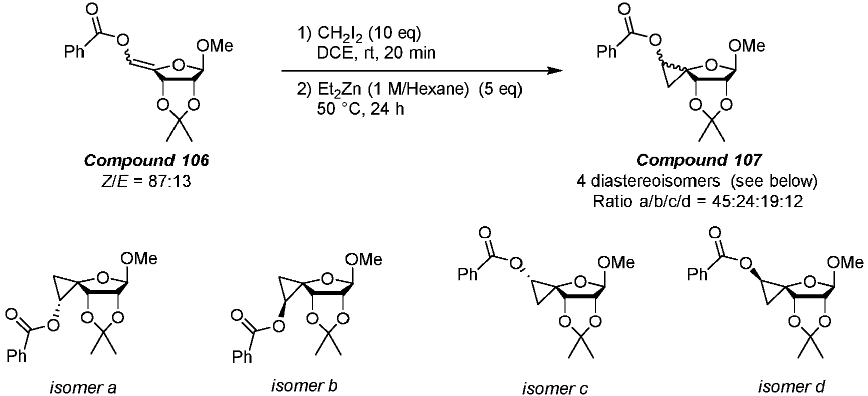

In an oven dried round bottom flask under argon atmosphere, ***compound 106*** (112.5 mg, 0.37 mmol, 1 eq) was dissolved in dry 1,2-dichloroethane (3.7 mL) and diiodomethane (0.29 mL, 3.67 mmol, 10 eq). The resulting mixture was stirred at room temperature for 15 min then diethylzinc (1.84 mL, 1.84 mmol, 1M/hexane, 5 eq) was added dropwise. The reaction mixture was heated for 24 h at 50 °C. The reaction mixture was quenched with a saturated aqueous solution of NaHCO₃ (6 mL) and diluted with dichloromethane (20 mL) and some ethyl acetate. After separation of both layers, the aqueous phase was extracted with dichloromethane (2 × 10 mL) and the combined organic layers were washed with brine (20 mL). The organic phase was dried over Na₂SO₄, filtrated and concentrated under reduced pressure.

The crude was purified over silica gel column chromatography (elution with PE/EtOAc = 95:5, 90:10 then 80:20) to afford ***compound 107*** (42 mg, 0.13 mmol, 36%) as a yellowish oil and as a mixture of 4 diastereoisomers **a, b, c, d** (named arbitrarily) with a ratio **a/b/c/d** = 45:24:19:12. An analytical sample of each isomer was purified via Prep SFC (Stationary phase: Chiralpak Daicel IG 20 × 250 mm, Mobile phase: CO₂, *i*PrOH + 0.4 *i*PrNH₂).

*Isomer* ***a:* ¹H NMR (400 MHz, CDCl₃):** δ 8.08-8.06 (m, 2 H), 7.55 (m, 1 H), 7.46-7.42 (m, 2 H), 5.08 (s, 1 H), 4.71 (d, *J =* 5.9 Hz, 1 H), 4.49 (dd, *J* = 7.9, 4.7 Hz, 1 H), 4.44 (d, *J =* 5.9 Hz, 1 H), 3.36 (s, 3 H), 1.55 (s, 3 H), 1.35 (s, 3 H), 1.32 (t, *J =* 7.9 Hz, 1 H), 1.18 ppm (dd, *J =* 7.8, 4.7 Hz, 1 H). **¹³C NMR (100 MHz, CDCl₃):** δ 167.0, 133.2, 130.01, 129.97 (2 C), 128.5 (2 C), 113.4, 108.1, 85.5, 83.7, 67.9, 55.1, 50.6, 26.6, 25.9, 19.2 ppm. **LCMS (of mixture 64_1):** [M+1]⁺ = 321.3, 1.07 min (Method 10).

*Isomer* ***b:* ¹H NMR (400 MHz, CDCl₃):** δ 8.03-8.00 (m, 2 H), 7.56 (m, 1 H), 7.45-7.41 (m, 2 H), 5.01 (s, 1 H), 4.83 (dd, *J* = 8.2, 4.5 Hz, 1 H), 4.74 (d, *J =* 5.9 Hz, 1 H), 4.71 (d, *J =* 5.9 Hz, 1 H), 3.32 (s, 3 H), 1.46 (s, 3 H), 1.36 (t, *J =* 8.0 Hz, 1 H), 1.26 (s, 3 H), 1.23 ppm (m, 1 H). **¹³C NMR (100 MHz, CDCl₃):** δ 167.0, 133.1, 130.2, 129.8 (2 C), 128.4 (2 C), 113.1, 108.9, 85.4, 84.0, 68.0, 55.1, 52.0, 26.5, 25.6, 20.7 ppm. **LCMS:** [M+1]⁺ = 321.3, 1.09 min (Method 10). **GCMS:** [M]^{+•} = 320, 16.40 min (Method 4).

*Isomer c:* **¹H NMR (400 MHz, CDCl₃):** δ 8.06-8.03 (m, 2 H), 7.55 (m, 1 H), 7.45-7.40 (m, 2 H), 4.99 (s, 1 H), 4.70 (d, *J=* 5.9 Hz, 1 H), 4.50 (d, *J=* 5.9 Hz, 1 H), 4.42 (dd, *J =* 7.8, 4.8 Hz, 1 H), 3.14 (s, 3 H), 1.52 (s, 3 H), 1.49 (d, *J* = 8.7 Hz, 1H), 1.40 (dd, *J* = 8.7, 4.9 Hz, 1 H), 1.35 ppm (s, 3 H). **¹³C NMR (100 MHz, CDCl₃):** δ 166.6, 133.7, 133.3, 129.7 (2C), 128.6 (2C), 112.9, 108.6, 85.4, 83.2, 69.0, 55.0, 55.6, 26.5, 25.8, 11.4 ppm. **LCMS:** [M+1]⁺= 321.2, 1.07 min (Method 10). **GCMS:** [M]^{+•} = 320, 16.77 min (Method 4).

*Isomer* ***d (4.3 mg):* ¹H NMR (400 MHz, CDCl₃):** δ 7.93-7.90 (m, 2 H), 7.45 (m, 1 H), 7.36-7.31 (m, 2 H), 4.93 (s, 1 H), 4.72 (d, *J =* 5.9 Hz, 1 H), 4.60 (d, *J =* 5.9 Hz, 1 H), 4.39 (dd, *J =* 8.0, 4.7 Hz, 1 H), 3.28 (s, 3 H), 1.47 (d, *J =* 8.5 Hz, 1 H), 1.44 (s, 3 H), 1.28 (s, 3 H), 1.23 ppm (dd, *J =* 8.7, 4.7 Hz, 1 H). **¹³C NMR (100 MHz, CDCl₃):** δ 166.3, 133.7, 130.2, 129.7 (2C), 128.5 (2C), 112.8, 107.6, 85.3, 78.7, 68.4, 56.1, 54.9, 26.7, 25.9, 12.8 ppm. **LCMS:** [M+1]⁺= 321.4, 1.11 min (Method 10). **GCMS:** [M]^{+•} = 320, 16.38 min (Method 4).

### Preparation of Compounds 86a and 86b

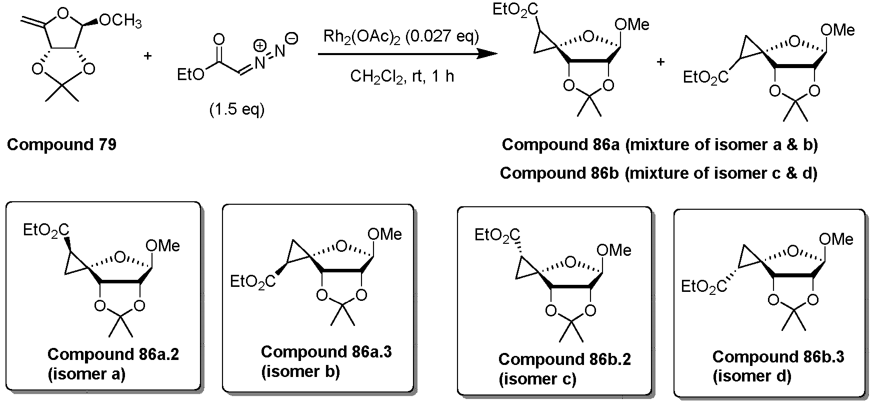

In an oven dried round bottom flask under argon atmosphere, ***compound 79 (***CAS 6991-65-7**)** (287 mg, 1.54 mmol, 1 eq) was dissolved in dry dichloromethane (3 mL) and catalyst Rh₂(OAc)₄ (18.4 mg, 0.042 mmol, 0.027 eq) was added. A solution of ethyl diazoacetate (0.28 mL, 2.31 mmol, 1.5 eq) in dry dichloromethane (5 mL) was added dropwise. The resulting mixture was stirred for 1 h at room temperature. The catalyst was filtered over a celite pad and the filtrate was concentrated under reduced pressure.

The crude was purified over silica gel column chromatography (elution with PE 100% then PE/EtOAc = 95:5 and 90:10) to afford ***compound 86a*** (194 mg, 0.71 mmol, 46%) as a yellow oil and as a mixture of two diastereoisomers **a** and **b** (named arbitrarily) and ***compound 86b*** (225 mg, 0.83 mmol, 54%) as a yellow oil and as a mixture of two diastereoisomers **c** and **d** (named arbitrarily). A mixture ***compound 86*** containing all 4 diasteromers was also collected with a ratio: **c/a/b/d** = 51:27:12:10 as determined by LC-MS analysis and 1H-NMR.

A purification was performed on ***compound 86b,*** 90mg (= ***compound 86b.1***) via Prep HPLC (Stationary phase: RP XBridge Prep C18 OBD-10µm,50×150mm, Mobile phase: 0.25% NH4HCO3 solution in water, CH3CN) to yield ***compound 86b.2*** (24.6 mg, 0.09 mmol, 27% yield) and ***compound 86b.3*** (12.5 mg, 0.05 mmol, 14% yield). The stereochemistry of isomer *a* (***compound 86a.2***) and isomer b (***compound 86a.3***) has been identified by comparing NMR data reported in the literature [Werz, B. D. et al. J. Org. Chem. 2009, 74, 8779-8786.].

### Compound 86a

**GCMS:** [M]^{+•} = 272, 13.35 min (Method 4)

### Compound 86b

**GCMS:** [M]^{+•} = 272, 13.59 min (Method 4)

### Compound 86

LCMS (ESI⁺) : [M+1]+= 273.3, RT 0.85 min (Method 10), **isomer c *= compound 86b.2***
LCMS (ESI⁺) : [M+18]+= 290.3, RT 0.89 min (Method 10), **isomer d *= compound 86b.3***
LCMS (ESI⁺) : [M+18]+= 290.4, RT 0.95 min (Method 10), **isomer a *= compound 86a.2***
LCMS (ESI⁺) : [M+18]+= 290.4, RT 0.98 min (Method 10), **isomer b *= compound 86a.3***

### Preparation of Compounds 86a and 86b (larger scale)

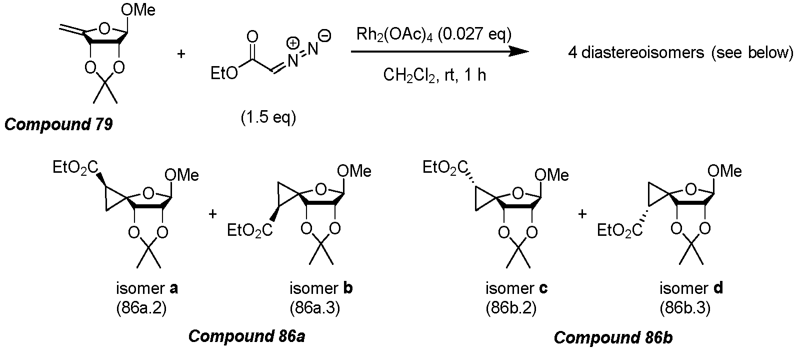

In an oven dried round bottom flask under argon atmosphere, ***compound 79 (***CAS 6991-65-7**)** (1 g, 5.37 mmol, 1 eq) was dissolved in dry dichloromethane (10 mL) and catalyst Rh₂(OAc)₄ (64.1 mg, 0.14 mmol, 0.027 eq) was added. A solution of ethyl diazoacetate (0.98 mL, 8.06 mmol, 1.5 eq) in dry dichloromethane (18 mL) was added dropwise. The resulting mixture was stirred for 1 h at room temperature. The catalyst was filtered over a celite pad and the filtrate was concentrated under reduced pressure. The analysis of the ¹H NMR spectrum of the crude mixture indicated that the 4 diastereoisomers were obtained with a ratio: **c/a/b/d** = 39:32:16:14.

The crude was purified over silica gel column chromatography (elution with PE 100% then PE/EtOAc = 95:5 and 90:10) to afford ***compound 86a*** (627 mg, 2.30 mmol, 43% isolated) as a colorless oil and as a mixture of two diastereoisomers **a** and **b** (named arbitrarily, ratio **a/b** = 70:30); a mixture of ***compounds 86a and 86b*** (293.5 mg, ratio **c/d/a/b** = 41:40:10:9); and ***compound 86b*** (494.8 mg, 1.82 mmol, 34% isolated) as a colorless oil and as a mixture of two diastereoisomers **c** and **d** (named arbitrarily, ratio **c/d** = 85:15).

The calculated total masses and the calculated yields of the mixture of isomers **a** and **b** are 682.8 mg, 2.51 mmol, 47% and of the mixture of isomers **c** and **d are** 732.5 mg, 2.69 mmol, 50%. The total masses and yields below were calculated from the ¹H NMR spectra of the purified fractions:
*Isomer* ***a* (*compound 86a.2*)** = 468.4 mg (32%).
*Isomer* ***b* (*compound 86.3*)** = 214.5 mg (15%).

The stereochemistry of isomers **a** and **b** has been identified by comparing NMR data reported in the literature (Werz, B. D. et al. J. Org. Chem. 2009, 74, 8779-8786).

*Isomer **c*** (***compound 86b.2***) *(540.9 mg, 37% yield):* **¹H NMR (400 MHz, CDCl₃):** δ 4.97 (s, 1 H), 4.65 (d, *J =* 5.8 Hz, 1 H), 4.42 (d, *J =* 5.8 Hz, 1 H), 4.20 (m, 1 H), 4.09 (m, 1 H), 3.22 (s, 3 H), 1.90-1.81 (m, 2 H), 1.49 (s, 3 H), 1.39 (dd, *J =* 8.6, 6.6 Hz, 1 H), 1.32 (s, 3 H), 1.27 ppm (t, *J =* 7.1 Hz, 3 H).

**¹³C NMR (100 MHz, CDCl₃):** δ 169.4, 113.1, 109.0, 84.9, 84.5, 73.1, 60.8, 55.8, 27.3, 26.5, 25.9, 14.4, 11.7 ppm.

*Isomer **d** (**compound 86b.3**) (191.6 mg, 13% yield):* **¹H NMR (400 MHz, CDCl₃):** δ 5.02 (s, 1 H), 4.64 (d, *J =* 5.8 Hz, 1 H), 4.40 (d, *J = 5.8* Hz, 1 H), 4.23-4.16 (m, 2 H), 3.36 (s, 3 H), 2.11 (dd, *J =* 9.2, 7.1 Hz, 1 H), 1.68 (dd, *J =* 7.0, 6.1 Hz, 1 H), 1.48 (s, 3 H), 1.34 (s, 3 H), 1.26 (t, *J* = 7.1 Hz, 3 H), 1.18 ppm (dd, *J =* 9.2, 5.9 Hz, 1 H). **¹³C NMR (100 MHz, CDCl₃):** δ 169.5, 113.3, 107.6, 84.9, 83.9, 71.7, 60.9, 55.0, 26.7, 26.2, 20.6, 19.2, 14.4 ppm.

### Preparation of Compound 108

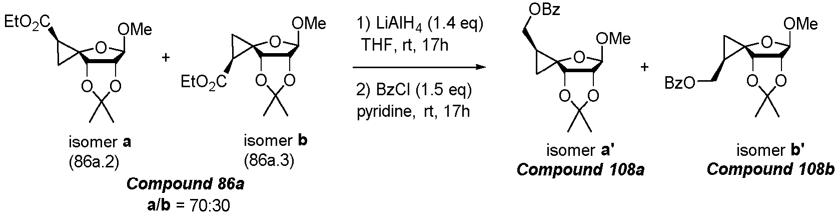

In an oven dried round bottom flask under argon atmosphere, ***compound 86a*** (40 mg, 0.15 mmol, 1 eq) was dissolved in anhydrous THF (2.1 mL). The solution was cooled at 0 °C, and LiAlH₄ (8.05 mg, 0.21 mmol, 1.4 eq) was added at once. The mixture was stirred for 1h at 0 °C, then room temperature for 17h. The reaction was carefully quenched by a saturated aqueous solution of NH₄Cl (2.5 mL). The product was extracted with EtOAc (3x 6 mL) and combined organic layers were dried (Na₂SO₄), filtered and the filtrate was concentrated under reduced pressure to afford a crude mixture which was directly used for next step. To the crude mixture was added anhydrous pyridine (0.80 mL) followed by the dropwise addition benzoyl chloride (0.026 mL, 0.22 mmol, 1.5 eq) at room temperature. The mixture was stirred for 17h at room temperature. The reaction was quenched with a saturated aqueous solution of NH₄Cl (5 mL). The product was extracted with EtOAc (3x 7 mL) and washed with 1M HCl (10 mL) to remove most of the pyridine then washed with brine (10 mL). The combined organic layers were dried (Na₂SO₄), filtered and the filtrate was concentrated under reduced pressure to afford a crude mixture.

The crude was purified over silica gel column chromatography (elution with PE/EtOAc = 95:5) to afford ***compound 108*** (43 mg, 0.13 mmol, 87%) as a turbid white oil and as a mixture of two diastereoisomers **a' (*compound 108a*)** and **b' (*compound 108b*)** with a ratio **a'/b'** = 71:29. Separation by preparative LC afforded a good separation. The separation of both isomers has been achieved by preparative SFC.

***Compound 108a:*** (15.8 mg, 37% yield, colorless oil): **¹H NMR (400 MHz, CDCl₃):** δ 8.07-8.05 (m, 2 H), 7.56 (m, 1 H), 7.46-7.43 (m, 2 H), 4.92 (s, 1 H), 4.71 (dd, *J =* 12.1, 6.1 Hz, 1 H), 4.66 (d, *J* = 5.9 Hz, 1 H), 4.63 (d, *J =* 5.9 Hz, 1 H), 3.84 (dd, *J =* 12.0, 10.7 Hz, 1 H), 3.23 (s, 3 H), 1.66 (m, 1 H), 1.51 (s, 3 H), 1.35 (s, 3 H), 1.32 (dd, *J =* 10.1, 7.1 Hz, 1 H), 0.90 ppm (t, *J =* 7.0 Hz, 1 H). **¹³C NMR (100 MHz, CDCl₃):** δ 166.5, 133.1, 130.4, 129.7 (2 C), 128.5 (2 C), 112.7, 107.4, 85.7, 80.3, 69.6, 65.1, 54.7, 26.6, 25.6, 22.9, 10.3 ppm.

***Compound 108b:*** (7.8 mg, 18% yield, white solid): **¹H NMR (400 MHz, CDCl₃):** δ 8.08-8.06 (m, 2 H), 7.56 (m, 1 H), 7.45-7.43 (m, 2 H), 4.93 (s, 1 H), 4.69 (d, *J* = 5.9 Hz, 1 H), 4.63 (dd, *J =* 11.9, 6.0 Hz, 1 H), 4.60 (d, *J =* 5.9 Hz, 1 H), 4.22 (dd, *J =* 11.9, 8.8 Hz, 1 H), 3.30 (s, 3 H), 1.87 (m, 1 H), 1.45 (s, 3 H), 1.28 (s, 3 H), 1.07 (dd, *J =* 10.0, 6.0 Hz, 1 H), 0.85 ppm (t, *J =* 6.4 Hz, 1 H). **¹³C NMR (100 MHz, CDCl₃):** δ 166.7, 133.0, 130.5, 129.8 (2 C), 128.4 (2 C), 112.7, 108.1, 85.6, 83.4, 69.6, 65.3, 54.9, 26.4, 25.2, 18.1, 17.3 ppm.

### Preparation of Compound 109

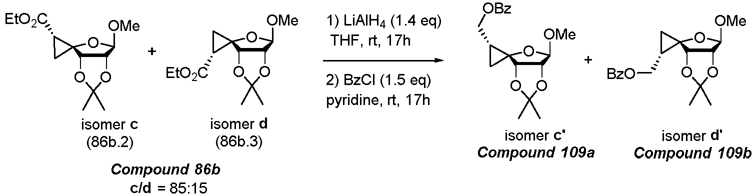

In an oven dried round bottom flask under argon atmosphere, ***compound 86b*** (40 mg, 0.15 mmol, 1 eq) was dissolved in anhydrous THF (2.1 mL). The solution was cooled at 0 °C, and LiAlH₄ (8.05 mg, 0.21 mmol, 1.4 eq) was added at once. The mixture was stirred for 1h at 0 °C, then room temperature for 17h. The reaction was carefully quenched by a saturated aqueous solution of NH₄Cl (2.5 mL). The product was extracted with EtOAc (3x 6 mL) and combined organic layers were dried (Na₂SO₄), filtered and the filtrate was concentrated under reduced pressure to afford the crude mixture which was directly used for next step.

To the crude mixture was added anhydrous pyridine (0.80 mL) followed by the dropwise addition benzoyl chloride (0.026 mL, 0.22 mmol, 1.5 eq) at room temperature. The mixture was stirred for 17h at room temperature. The reaction was quenched with a saturated aqueous solution of NH₄Cl (5 mL). The product was extracted with EtOAc (3x 7 mL) and washed with 1M HCl (10 mL) to remove most of the pyridine then washed with brine (10 mL). The combined organic layers were dried (Na₂SO₄), filtered and the filtrate was concentrated under reduced pressure to afford the crude mixture.

The crude was purified over silica gel column chromatography (elution with PE/EtOAc = 95:5) to afford ***compound 109*** (40 mg, 0.12 mmol, 81% calculated) as a cloudy oil (93% purity, contains DCM) and as a mixture of two diastereoisomers **c' (*compound 109a*)** and **d' (*compound 109b*)** with a ratio **c'/d'** = 84:16. The separation of both isomers has been achieved by preparative SFC.

***Compound 109a:*** (20.8 mg, 48% yield, colorless oil) **¹H NMR (400 MHz, CDCl₃):** δ 8.07-8.06 (m, 2 H), 7.56 (m, 1 H), 7.46-7.43 (m, 2 H), 4.97 (s, 1 H), 4.67 (d, *J =* 5.9 Hz, 1 H), 4.45 (d, *J =* 5.9 Hz, 1 H), 4.39 (dd, *J =* 11.6, 6.9 Hz, 1 H), 4.27 (dd, *J =* 11.6, 7.7 Hz, 1 H), 3.31 (s, 3 H), 1.52 (s, 3 H), 1.47 (m, 1 H), 1.34 (s, 3 H), 1.21(dd, *J =* 9.6, 7.1 Hz, 1 H), 1.00 ppm (t, *J* = 6.9 Hz, 1 H). **¹³C NMR (100 MHz, CDCl₃):** δ 167.0, 133.0, 130.5, 129.7 (2 C), 128.5 (2 C), 112.6, 109.1, 85.3, 84.7, 70.6, 64.3, 56.1, 26.5, 25.6, 22.8, 9.9 ppm.

***Compound 109b:*** (3.5 mg, 8% yield, white solid) **¹H NMR (400 MHz, CDCl₃):** δ 8.09-8.07 (m, 2 H), 7.55 (m, 1 H), 7.44-7.41 (m, 2 H), 4.99 (s, 1 H), 4.68 (d, *J* = 5.9 Hz, 1 H), 4.61 (dd, *J =* 11.5, 7.0 Hz, 1 H), 4.44 (d, *J =* 5.9 Hz, 1 H), 4.35 (dd, *J =* 11.5, 8.0 Hz, 1 H), 3.33 (s, 3 H), 1.69 (m, 1 H), 1.47 (s, 3 H), 1.33 (s, 3 H), 1.02(dd, *J =* 9.8, 6.1 Hz, 1 H), 0.84 ppm (t, *J* = 6.5 Hz, 1 H). **¹³C NMR (100 MHz, CDCl₃):** δ 166.8, 133.0, 130.6, 129.8 (2 C), 128.4 (2 C), 112.8, 108.0, 85.5, 84.2, 70.0, 64.5, 55.0, 26.8, 25.9, 17.6, 15.5 ppm.

### Asymmetric preparation 1 of Compound 86 (selective for isomer a)

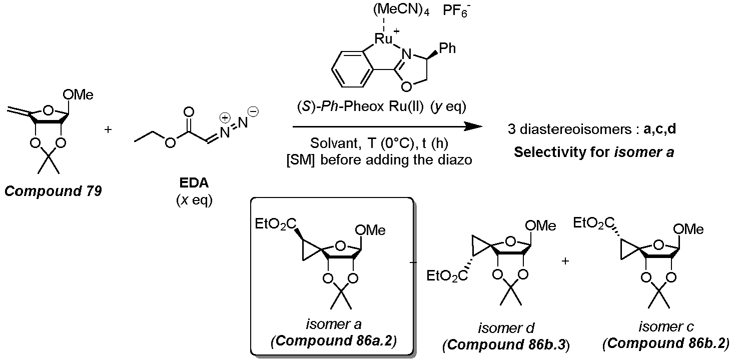

To an oven dried round bottom flask under argon atmosphere, ***compound 79*** (93 mg, 0.5 mmol, 1 eq) was dissolved in anhydrous CH₂Cl₂ (concentration solution 0.5 M) and catalyst (S)-Ph-Pheox Ru(II) (0.06 eq) was added. A solution of ethyl diazoacetate (EDA) (3 eq, to a concentration of 0.44 M) in anhydrous CH₂Cl₂ was added dropwise at -10°C and the solution was stirred for 2 hours. The catalyst was filtered over a celite pad and the filtrate was concentrated under reduced pressure. The analysis of the ¹H NMR spectrum of the crude mixture containing trimethoxybenzene as an internal standard allowed to determine the conversion of the substrate as well as the yield of the product. Isomer **a (*compound 86.2*)*,*** isomer **d** *(**compound 86b.3**)* and isomer **c** (***compound 86b.2***) were obtained in a **89:9:2 ratio,** respectively in a **combined yield of 81%.**

### Asymmetric preparation 2 of Compound 86 (selective for isomer c)

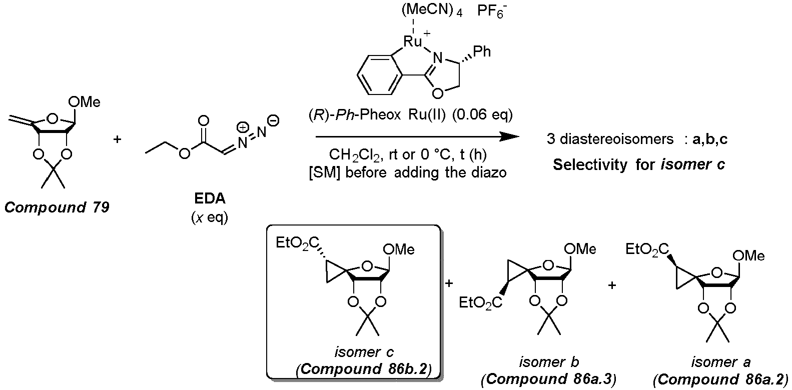

In an oven dried round bottom flask under argon atmosphere, ***compound 79*** (93 mg, 0.5 mmol, 1 eq) was dissolved in dry dichloromethane (concentration of 0.4M) and catalyst (*R*)-*Ph*-Pheox Ru(II) (***compound 147***) (21 mg, 0.03 mmol, 0.06 eq, ~ 90% purity) was added. A solution of ethyl diazoacetate (EDA) in dry dichloromethane (3 eq, concentration of 0.33M) was added dropwise at room temperature. The resulting mixture was stirred at room temperature for 3 hours. The catalyst was filtered over a celite pad and the filtrate was concentrated under reduced pressure. The analysis of the ¹H NMR spectrum of the crude mixture containing trimethoxybenzene as an internal standard allowed to determine the conversion of the substrate as well as the yield of the product. Isomer **c** (***compound 86b.2***), isomer **b** (***compound 86a.3***) and isomer **a** (***compound 86.2***) were obtained in a **92:7:1 ratio,** respectively in a **combined yield of 88%.**

### Preparation of Compound 110

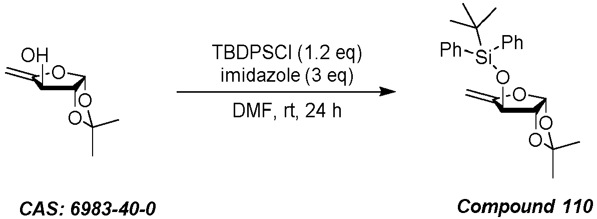

In an oven-dried round bottom flask under argon atmosphere, CAS 6983-40-0 (500 mg, 2.9 mmol, 1 eq) was dissolved in anhydrous DMF (12.5 mL), then imidazole (593 mg, 8.71 mmol, 3 eq) and TBDPSCl (0.92 mL, 3.48 mmol, 1.2 eq) were added at room temperature. The reaction mixture was stirred at room temperature for 24 h. The reaction was quenched with water (100 mL). The product was extracted with EtOAc (3x 40 mL) then washed with a saturated solution of NaHCO₃ (30 mL) and with water (30 mL). The combined organic layers were dried (Na₂SO₄), filtered and the filtrate was concentrated under reduced pressure to afford the crude mixture. The crude was purified over silica gel column chromatography (elution with PE/EtOAc = 95:5) to afford ***compound 110*** (1.07 g, 2.61 mmol, 90%) as a colorless oil.

**¹H NMR (400 MHz, CDCl₃):** δ 7.67-7.65 (m, 4 H), 7.48-7.36 (m, 6 H), 6.15 (d, *J =* 3.0 Hz, 1 H), 4.48-4.47 (m, 2 H), 4.32 (d, *J =* 1.8 Hz, 1 H), 3.66 (d, *J =* 1.9 Hz, 1 H), 1.37 (s, 3 H), 1.34 (s, 3 H), 1.06 ppm (s, 9 H). **¹³C NMR (100 MHz, CDCl₃):** δ 161.4, 136.1 (2 C), 136.0 (2 C), 133.1, 133.0, 130.2, 130.1, 128.0 (2 C), 127.8 (2 C), 113.9, 106.8, 87.7, 85.0, 76.0, 28.1, 27.4, 27.0 (3 C), 19.4 ppm. **GCMS:** [M]⁺˙ = 410, 18.41 min (Method 5).

### Preparation of Compound 111

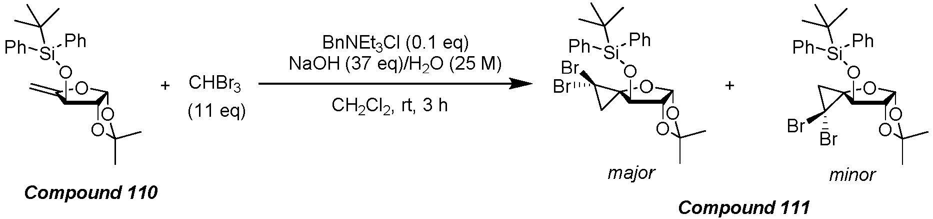

***Compound 110*** (500 mg, 1.22 mmol, 1 eq) and benzyltriethylammonium chloride (27.8 mg, 0.12 mmol, 0.1 eq) were dissolved in a mixture of bromoform (1.17 mL, 13.4 mmol, 11 eq) and dichloromethane (1.6 mL) and a solution of NaOH (1.81 g, 45.2 mmol, 25 M in H₂O, 37 eq) was added dropwise at room temperature and under argon atmosphere. The mixture was stirred at room temperature for 3h. The product was extracted with Et₂O (3 × 100 mL) (ignore polymeric tar). The combined organic phase was washed with brine (100 mL), dried over Na₂SO₄, filtered and concentrated under reduced pressure. The crude was purified over silica gel column chromatography (elution with PE/EtOAc = 98:2 and 95:5) to afford ***compound 111*** (224.2 mg, 0.38 mmol, 32%) as an orange oil and as an inseparable mixture of 2 diastereoisomers with a ratio *major*/*minor =* 81:19 (determined by ¹H NMR analysis).

Major isomer: **¹H NMR (400 MHz, CDCl₃):** δ 7.66-7.63 (m, 4 H), 7.45-7.37 (m, 6 H), 6.16 (d, *J =* 3.7 Hz, 1 H), 4.68 (d, *J =* 3.7 Hz, 1 H), 4.64 (s, 1 H), 1.71 (d, *J* = 9.7 Hz, 1 H), 1.64 (s, 3 H), 1.42 (d, *J* = 9.7 Hz, 1 H), 1.31 (s, 3 H), 1.08 ppm (s, 9 H). **¹³C NMR (100 MHz, CDCl₃):** δ 136.0 (2 C), 135.9 (2 C), 132.9, 132.7, 130.4, 130.3, 128.2 (2 C), 128.0 (2 C), 113.2, 106.4, 86.2, 79.6, 74.4, 30.9, 29.0, 27.0 (3 C), 26.6, 26.4, 19.6 ppm.

### Preparation of Compound 112

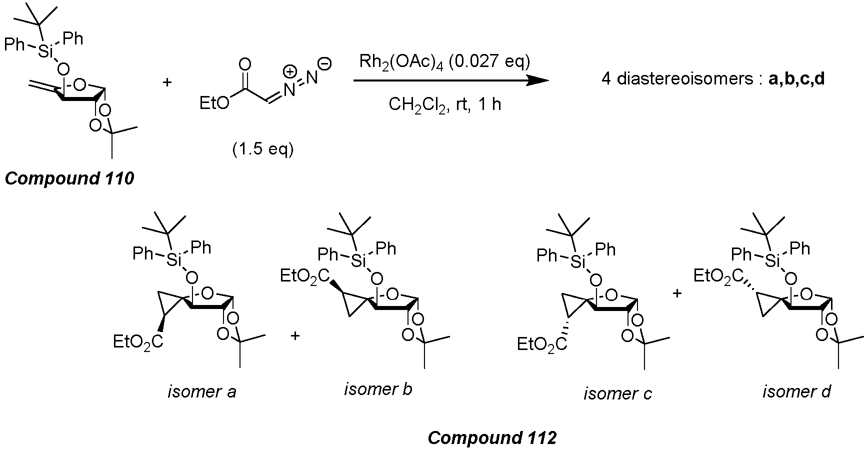

In an oven dried round bottom flask under argon atmosphere, ***compound 110*** (205.3 mg, 0.5 mmol, 1 eq) was dissolved in dry dichloromethane (1 mL) and catalyst Rh₂(OAc)₄ (6 mg, 0.014 mmol, 0.027 eq) was added. A solution of ethyl diazoacetate (0.091 mL, 0.75 mmol, 1.5 eq) in dry dichloromethane (1.7 mL) was added dropwise. The resulting mixture was stirred for 1 h at room temperature. The catalyst was filtered over a celite pad and the filtrate was concentrated under reduced pressure. The analysis of the ¹H NMR spectrum of the crude mixture indicated that the conversion of ***compound 110*** was incomplete and that the 4 possible diastereoisomers were obtained with a ratio: **a/c/d/b** = 53:32:8:7. The crude was purified over silica gel column chromatography (elution with PE/EtOAc = 98:2 to 95:5 then 90:10) to afford fraction 1 corresponding to the recovered starting material ***compound 110*** (105.5 mg, 49% conversion), fraction 2 (56.2 mg, 0.11 mmol, 23% isolated) as a colorless oil and as a mixture of two diastereoisomers **a** and **b** of ***compound 112*** (named arbitrarily, ratio **a/b** = 90:10), and fraction 3 (51.1 mg, 0.10 mmol, 20% isolated) as a colorless turbid oil and as a mixture of two diastereoisomers **c** and **d** of ***compound 112*** (named arbitrarily, ratio **c/d** = 60:40) accompanied by some unknown impurities. Only the major isomer, *isomer a,* has been characterized by NMR spectroscopy from fraction 2 and only the major isomer, *isomer c,* has been characterized by NMR spectroscopy from fraction 3

The total masses and yields below were calculated from the ¹H NMR spectra of the purified fractions:
- *isomer a:* m = 50.6 mg (20%)
- *isomer b:* m = 5.6 mg (2%)
- *isomer c:* m = 30.7 mg (12%)
- *isomer d:* m = 20.4 mg (8%)

Fractions 2 and 3 were purified via Preparative SFC separation affording:
*I****somer a:* ¹H NMR (400 MHz, CDCl₃):** δ 7.71-7.67 (m, 4 H), 7.44-7.37 (m, 6 H), 5.93 (d, *J* = 3.7 Hz, 1 H), 4.51 (s, 1 H), 4.44 (d, *J =* 3.7 Hz, 1 H), 3.95 (dd, *J =* 10.8, 7.1 Hz, 1 H), 4.03 (dd, *J =* 10.8, 7.1 Hz, 1 H), 1.92 (dd, *J =* 9.5, 7.3 Hz, 1 H), 1.48 (s, 3 H), 1.32 (m, 1 H), 1.26 (s, 3 H), 1.20 (s, 3 H), 1.14 (m, 1 H), 1.09 ppm (s, 9 H). **¹³C NMR (100 MHz, CDCl₃):** δ 171.2, 136.0 (2 C), 135.9 (2 C), 133.7, 133.5, 130.03, 130.01, 127.9 (2 C), 127.8 (2 C), 112.5, 105.3, 85.6, 75.9, 73.9, 60.4, 29.8, 27.5, 27.1 (3 C), 26.3, 19.6, 14.4, 12.2 ppm. **GCMS:** [M-H]^{+•} = 495, 20.62 min (Method 5).
*I****somer b:* GCMS:** [M-*t*Bu]^{+•} = 439, 20.84 min (Method 5).
*I****somer c:* ¹H NMR (400 MHz, C₆D₆):** δ 7.66-7.58 (m, 4 H), 7.29-7.24 (m, 6 H), 5.95 (d, *J =* 4.1 Hz, 1 H), 4.61 (d, *J =* 4.0 Hz, 1 H), 4.09 (m, 1 H), 3.94 (m, 2 H), 1.60 (tₐₚₚ, *J =* 6.6 Hz, 1 H), 1.50 (dd, *J* = 9.0, 6.8 Hz, 1 H), 1.42 (s, 3 H), 1.11 (s, 3 H), 1.083 (m, 3 H), 1.080 (s, 9 H), 0.64 ppm (dd, *J =* 9.0, 6.9 Hz, 1 H). **¹³C NMR (100 MHz, CDCl₃):** δ 169.4, 135.9 (2 C), 135.8 (2 C), 133.2, 133.0, 130.3, 130.2, 128.1 (2 C), 128.0 (2 C), 111.7, 105.7, 85.9, 81.9, 73.9, 60.5, 27.0 (3 C), 26.2, 25.94, 25.90, 19.5, 14.3, 11.5 ppm. **GCMS:** [M]⁺˙ = 496, 20.98 min (Method 5).
*I****somer d:* ¹H NMR (400 MHz, CDCl₃):** δ 7.69-7.67 (m, 2 H), 7.65-7.63 (m, 2 H), 7.46-7.44 (m, 2 H), 7.41-7.38 (m, 4 H), 5.93 (d, *J =* 3.8 Hz, 1 H), 4.55 (d, *J =* 3.8 Hz, 1 H), 4.19-4.10 (m, 2 H), 3.93 (s, 1 H), 1.66 (dd, *J* = 8.9, 7.2 Hz, 1 H), 1.61 (dd, *J =* 7.0, 5.8 Hz, 1 H), 1.43 (s, 3 H), 1.27 (s, 3 H), 1.24 (t, *J =* 7.1 Hz, 3 H), 1.10 (s, 9 H), 0.88 ppm (dd, *J* = 9.0, 5.7 Hz, 1 H).
**GCMS:** [M]^{+•} = 496, 21.61 min (Method 5).

Asymmetric preparation 1 of ***Compound 112*** (selective for isomer c)

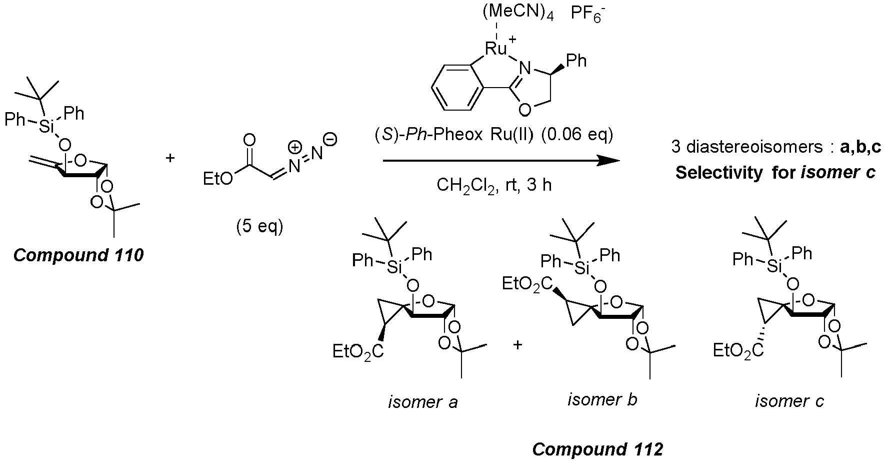

In an oven dried round bottom flask under argon atmosphere, ***compound 110*** (68.5 mg, 0.17 mmol, 1 eq) was dissolved in dry dichloromethane (0.6 mL) and catalyst (*S*)-*Ph*-Pheox Ru(II) (6.3 mg, 0.01 mmol, 0.06 eq) was added. A solution of ethyl diazoacetate (0.1 mL, 0.83 mmol, 5 eq) in dry dichloromethane (3.3 mL) was added dropwise. The resulting mixture was stirred for 3 h at room temperature. The catalyst was filtered over a celite pad and the filtrate was concentrated under reduced pressure. The analysis of the ¹H NMR spectrum of the crude mixture indicated that the conversion of ***compound 110*** was incomplete and that three diastereoisomers were obtained with a ratio: **c/a/b** = 79:17:4. The crude was purified over silica gel column chromatography (elution with PE/EtOAc = 98:2 to 95:5 then 90:10) to afford fraction 1 corresponding to the recovered starting material ***compound 110*** (19 mg, 72% conversion), fraction 2 (15 mg, 0.02 mmol, 55% purity, 10%) as a colorless oil and as a mixture of two diastereoisomers **a** and **b** (named arbitrarily, ratio **a/b** = 85:15) of ***compound 112*** contaminated with DCM and diethyl but-2-enedioate (from EDA dimerization) and fraction 3 (53 mg, 0.10 mmol, 97% purity, 62%) as a colorless oil and as a single diastereoisomer of ***compound 112,** isomer c,* contaminated with DCM. The major isomer, *isomer c,* was characterised from fraction 3.

***isomer c:* ¹H NMR (400 MHz, CDCl₃):** δ 7.69-7.61 (m, 4 H), 7.47-7.36 (m, 6 H), 6.03 (d, *J* = 4.0 Hz, 1 H), 4.62 (d, *J =* 4.0 Hz, 1 H), 4.21-4.12 (m, 1 H), 4.06-3.99 (m, 1H), 3.90 (s, 1 H), 1.56-1.52 (m, 2 H), 1.44 (s, 3 H), 1.25 (s, 3 H), 1.20 (t, *J =* 7.1 Hz, 3 H), 1.07 (s, 9 H), 0.70 (qₐₚₚ, *J =* 9.6 Hz, 1H) ppm. **¹³C NMR (100 MHz, CDCl₃):** δ 169.3, 135.84 (2 C), 135.80 (2 C), 133.2, 132.9, 130.3, 130.1, 128.1 (2 C), 127.9 (2 C), 111.6, 105.7, 85.9, 81.6, 73.8, 60.5, 27.0 (3 C), 26.2, 25.92, 25.87, 19.5, 14.3, 11.4 ppm. **GCMS:** [M]^{+•} = 496, 21.57 min (Method 5).

Asymmetric preparation 2 of ***Compound 112*** (selective for isomer a)

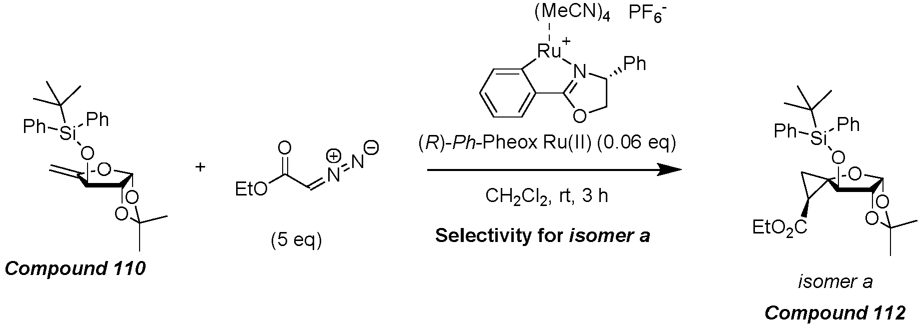

In an oven dried round bottom flask under argon atmosphere, ***compound 110*** (50.9 mg, 0.12 mmol, 1 eq) was dissolved in dry dichloromethane (0.44 mL) and catalyst (*R*)-*Ph*-Pheox Ru(II) 5.1 mg, 0.007 mmol, 0.06 eq, ~ 93% purity) was added. A solution of ethyl diazoacetate (0.07 mL, 0.62 mmol, 5 eq) in dry dichloromethane (2.5 mL) was added dropwise. The resulting mixture was stirred for 3 h at room temperature. The catalyst was filtered over a celite pad and the filtrate was concentrated under reduced pressure. The analysis of the ¹H NMR spectrum of the crude mixture indicated that the conversion of ***compound 110*** is incomplete and that one single diastereoisomer was obtained which corresponds to *isomer a.* The crude was purified over silica gel column chromatography (elution with PE/EtOAc = 98:2 to 95:5) to afford fraction 1 corresponding to the recovered starting material ***compound 110*** (23.9 mg, 93% purity, 57% conversion) contaminated with (*Z*)- diethyl but-2-enedioate (from EDA dimerization) and DCM, and fraction 2 (52 mg, 0.07 mmol, 68% purity, 57%) as a colorless oil and a mixture of the desired product, *isomer **a*** of ***compound 112,*** contaminated (*E*)- and (*Z*)-diethyl but-2-enedioate and DCM. Characterisation of *isomer **a*** of ***compound 112*** is provided in the procedure for "Preparation of ***Compound 112"*** above.

### Preparation of Compound 113.1

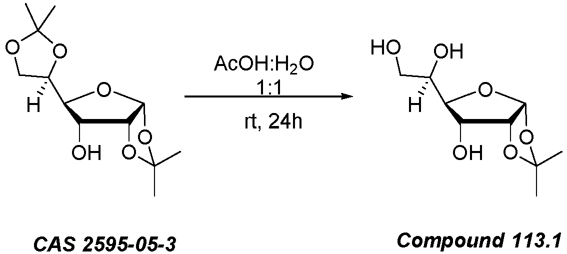

CAS 2595-05-3 (10g, 38.46 mmol, 1.00 eq) was dissolved in AcOH (100 mL), then H₂O (100 ml) was added. The mixture was stirred at rt for 24 hour and subsequently added to saturated NaHCO₃ aqueous (300 mL) at 0°C, then evaporated to dryness. The residue was purified via silica gel column with DCM/MEOH (gradient elution: DCM/MEOH from 99:1 to 95:5). The fractions containing the product were collected and the solvent was evaporated to afford ***compound 113.1.***

**73% yield** (6.2 g, 28.18 mmol), white solid. **¹H NMR** (400 MHz, Methanol-d4) δ 5.75 (d, J = 3.6 Hz, 1H), 4.58 (t, J = 4.2 Hz, 1H), 4.13 (dd, J = 8.6, 4.7 Hz, 1H), 3.95 - 3.89 (m, 2H), 3.71 (dd, J = 11.6, 4.1 Hz, 1H), 3.64 (dd, J = 11.6, 6.3 Hz, 1H), 1.55 (s, 3H), 1.36 ppm (s, 3H). **¹³C NMR** (101 MHz, MeOD) δ 112.32, 103.91, 80.21, 79.82, 71.11, 70.37, 62.55, 25.60, 25.31 ppm.

### Preparation of Compound 113.2

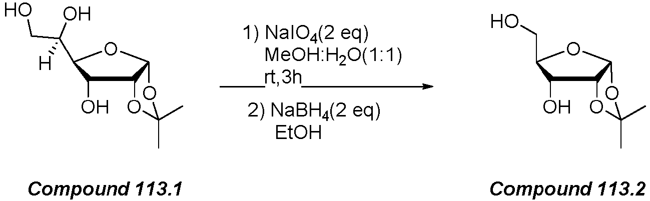

***Compound 113.1*** (6.2 g, 28.18 mmol, 1.00 eq) was dissolved in MeOH:H₂O (1:1, 20 V), then NaIO₄ (12.1 g, 56.54 mmol, 2.00 eq) were added at 0°C. The mixture was stirred at rt for 3 hours. The mixture was evaporated to dryness. The residue (6.2 g, crude) was dissolved in EtOH (620 ml) and NaBH₄ (2.5 g, 65.79 mmol, 2.00 eq) was added at 0°C. The mixture was stirred at rt for 16 hours. The residue was added to saturated NH₄Cl aqueous (300 mL) at 0°C, the mixture was evaporated to dryness. The residue was purified by column chromatography over silica gel (gradient elution: DCM/MEOH from 99:1 to 93:7). The fractions containing the product were collected and the solvent was evaporated to afford ***compound 113.2.***

**79% yield** (4.2 g, 22.11 mmol, 2 steps) white solid. **¹H NMR** (400 MHz, Chloroform-d) δ 5.81 (d, J = 3.8 Hz, 1H), 4.58 (t, J = 4.4 Hz, 1H), 4.00 (dd, J = 8.9, 5.1 Hz, 1H), 3.94 (dd, J = 12.3, 2.7 Hz, 1H), 3.85 (dt, J = 9.0, 3.2 Hz, 1H), 3.74 (dd, J = 12.3, 3.7 Hz, 1H), 1.57 (s, 3H), 1.37 ppm (s, 3H). **¹³C NMR** (101 MHz, CDCl3) δ 112.78, 103.99, 80.64, 78.80, 70.85, 60.76, 53.45, 26.50 ppm.

### Preparation of Compound 113.3

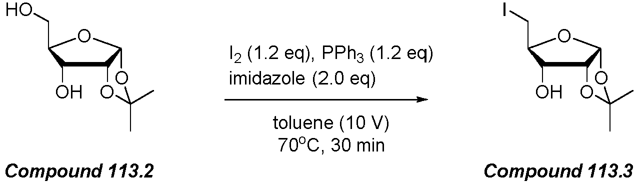

***Compound 113.2*** (4.2 g, 22.11 mmol, 1.00 eq) was dissolved in toluene (420mL) and PPH₃ (6.95 g, 26.53 mmol, 1.20 eq) was added. I₂ (6.74 g, 26.54 mmol, 1.20 eq) was added at 0 °C. The mixture was stirred at 70 °C for 2 hours. After the mixture had cooled to 0°C, the residue was added to saturated aqueous Na₂S₂O₃ (300 ml), and the aqueous was extracted with EtOAc (2 × 300 mL), the organic phase was dried over anhydrous sodium sulfate and concentrated *in vacuo.* The residue was purified by column chromatography over silica gel (gradient elution:PE/EtOAc from 99:1 to 90: 10). The fractions containing the product were collected and the solvent was evaporated to afford ***compound 113.3.***

**74% yield** (4.9 g, 16.33 mmol), white solid. **¹H NMR** (300 MHz, Methanol-d4) δ 5.73 (d, J = 3.6 Hz, 1H), 4.58 (t, J = 4.1 Hz, 1H), 3.72 (dd, J = 8.5, 4.5 Hz, 1H), 3.65 - 3.51 (m, 2H), 3.36 - 3.28 (m, 2H), 1.52 (s, 3H), 1.34 ppm (s, 3H). **¹³C NMR** (75 MHz, MeOD) δ 112.49, 103.58, 79.68, 78.16, 75.54, 25.52, 25.25, 5.01 ppm.

### Preparation of Compound 113

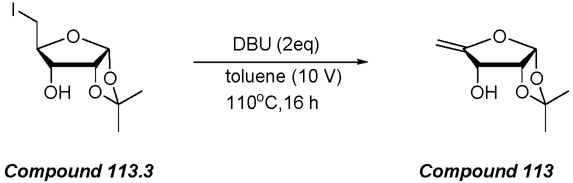

***Compound 113.3*** (4.5 g, 15.01 mmol, 1.00 eq) was dissolved in toluene (450 mL), then DBU (4.56 g, 30.01 mmol, 2.00 eq) was added. The mixture was stirred at 110 °C for 16 hours. The mixture was cooled down to room temperature and poured in EtOAc (3 × 300 ml) followed by washing with brine (400 ml). The organic phase was dried with Na₂SO₄, filtered and the filtrate was concentrated *in vacuo.* The residue was purified via silica gel column with PE/EtOAc (gradient elution: PE/EtOAc from 99:1 to 90:10). The fractions containing the product were collected and the solvent was removed *in vacuo* to afford ***compound 113.***

**57% yield** (1.43 g, 8.31 mmol), white solid. **¹H NMR** (300 MHz, Methanol-d4) δ 5.87 (d, J = 2.8 Hz, 1H), 4.56 (td, J = 4.4, 3.9, 2.5 Hz, 2H), 4.34 (t, J = 1.9 Hz, 1H), 4.17 (t, J = 1.8 Hz, 1H), 1.47 (s, 3H), 1.39 ppm (s, 3H). **¹³C NMR** (75 MHz, MeOD) δ 161.97, 113.92, 104.03, 81.31, 79.29, 71.22, 26.80, 26.07 ppm.

### Preparation of Compound 114

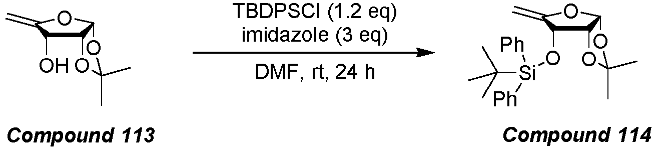

In an oven-dried round bottom flask under argon atmosphere, ***compound 113*** (200 mg, 1.16 mmol, 1 eq) was dissolved in anhydrous DMF (5 mL), then imidazole (237.2 mg, 3.48 mmol, 3 eq) and TBDPSCl (0.37 mL, 1.39 mmol, 1.2 eq) were added at room temperature. The reaction mixture was stirred at room temperature for 24 h. The reaction was quenched with water (40 mL). The product was extracted with EtOAc (3x 10 mL) then washed with a saturated solution of NaHCO₃ (15 mL) and with water (15 mL). The combined organic layers were dried (Na₂SO₄), filtered and the filtrate was concentrated under reduced pressure to afford the crude mixture. The crude was purified over silica gel column chromatography (elution with PE/EtOAc = 98:2) to afford ***compound 114*** (319 mg, 0.75 mmol, 96% purity, 64%) as a turbid colorless oil contaminated with DCM.

**¹H NMR (400 MHz, CD₂Cl₂):** δ 7.81-7.71 (m, 4 H), 7.46-7.38 (m, 6 H), 5.62 (d, *J* = 3.2 Hz, 1 H), 4.51 (m, 1 H), 4.42 (m, 1 H), 4.32 (m, 1 H), 4.04 (m, 1 H), 1.52 (d, *J* = 0.8 Hz, 3 H), 1.34 (d, *J* = 0.4 Hz, 3 H), 1.11 (m, 9 H) ppm. **¹³C NMR (100 MHz, CD₂Cl₂):** δ 161.6, 136.5 (2 C), 136.2 (2 C), 133.9, 133.4, 130.60, 130.56, 128.4 (2 C), 128.2 (2 C), 114.7, 104.5, 83.4, 79.3, 73.4, 28.2, 27.4, 27.1 (3 C), 19.8 ppm. **GCMS:** [M]^{+•} = 410, 18.60 min (Method 5).

### Preparation of Compound 114 (larger scale)

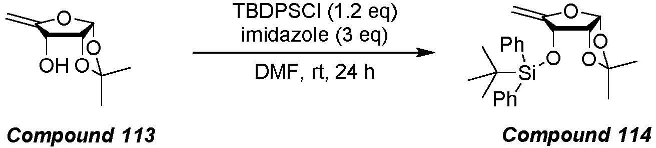

***Compound 113*** (5.00 g, 29.07 mmol, 1.00 eq) was dissolved in anhydrous DMF (100 mL), then imidazole (5.90 g, 87.21 mmol, 3.00 eq) and TBDPSCl (9.06 mL, 34.88 mmol, 1.20 eq) were added at room temperature. The reaction mixture was stirred at room temperature for 24 h. The reaction was quenched with water (500 mL). The product was extracted with EtOAc (3x 200 mL) then washed with a saturated solution of NaHCO₃ (200 mL) and with water (200 mL). The combined organic layers were dried (Na₂SO₄), filtered and the filtrate was concentrated under reduced pressure to afford the crude mixture. The crude was purified over silica gel column chromatography (elution with Petroleum ether/EtOAc = 98:2) to afford ***compound 114.***

**64% yield** (7.7 g, 18.78 mmol) colorless oil. **¹H NMR (400 MHz, CD₂Cl₂):** δ 7.81~7.71 (m, 4 H), 7.46~7.38 (m, 6 H), 5.62 (d, J = 3.2 Hz, 1 H), 4.51 (m, 1 H), 4.42 (m, 1 H), 4.32 (m, 1 H), 4.04 (m, 1 H), 1.52 (d, J = 0.8 Hz, 3 H), 1.34 (d, J = 0.4 Hz, 3 H), 1.11 ppm (m, 9 H). **¹³C NMR (100 MHz, CD₂Cl₂):** δ 161.6, 136.5, 136.2, 133.9, 133.4, 130.60, 130.56, 128.4, 128.2, 114.7, 104.5, 83.4, 79.3, 73.4, 28.2, 27.4, 27.1, 19.8 ppm. **LCMS (ESI+) m/z:** calcd. for C₂₄H₃₀O₄Si [M+H]+ = 411.19, found 411.19, RT: 1.993 min, Method 2.

### Preparation of Compound 115

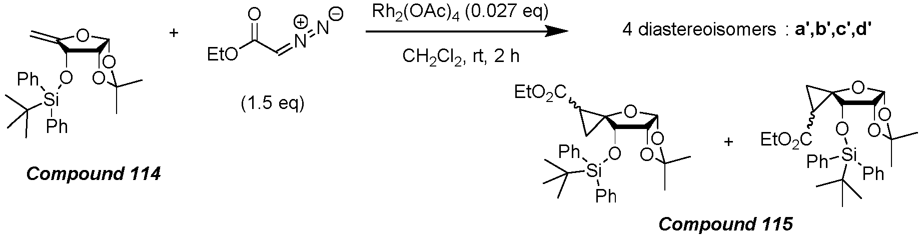

In an oven dried round bottom flask under argon atmosphere, ***compound 114*** (88 mg, 0.206 mmol, 1 eq) was dissolved in dry dichloromethane (0.41 mL) and catalyst Rh₂(OAc)₄ (2.5 mg, 0.006 mmol, 0.027 eq) was added. A solution of ethyl diazoacetate (0.037 mL, 0.309 mmol, 1.5 eq) in dry dichloromethane (0.67 mL) was added dropwise. The resulting mixture was stirred for 2 h at room temperature. The catalyst was filtered over a celite pad and the filtrate was concentrated under reduced pressure. The analysis of the ¹H NMR spectrum of the crude mixture indicated that the conversion of ***compound 114*** was incomplete and that the 4 possible diastereoisomers were obtained with a ratio: **c'/a'/b'/d'** = 39:34:14:13. The crude was purified over silica gel column chromatography (elution with PE/EtOAc = 98:2 to 95:5 then 90:10 and 80:20) to afford fraction 1 corresponding to the recovered starting material ***compound 114*** (54mg, 0.120 mmol, 91% purity, 42% conversion) contaminated with (*Z*)-diethyl but-2-enedioate (from EDA dimerization), fraction 2 (22 mg, 0.04 mmol, 90% purity, 19%) as a colorless oil and as a mixture of two isomers of the product, ***compound 115,*** with a ratio **a'/b'** = 75:25 (named arbitrarily) contaminated with (*E*)- and (*Z*)-diethyl but-2-enedioate (from EDA dimerization), and fraction 3 (22 mg, 0.044 mmol, 21.5%) as a colorless oil and a mixture of two isomers of ***compound 115*** with a ratio **c'/d'** = 79:21 (named arbitrarily) contaminated with a few unknown impurities. Only the major isomer, *isomer a,* was described from fraction 2 and only the major isomer, *isomer c,* was described from fraction 3.

***isomer a':* ¹H NMR (400 MHz, CD₂Cl₂):** δ 7.77-7.75 (m, 2 H), 7.69-7.67 (m, 2 H), 7.45-7.33 (m, 6 H), 5.44 (d, *J* = 3.8 Hz, 1 H), 4.47 (d, *J =* 4.8 Hz, 1 H), 4.04-3.96 (m, 2 H), 3.89 (dd, *J* = 4.6, 4.0 Hz, 1 H), 1.87 (dd, *J =* 10.0, 8.1 Hz, 1 H), 1.64 (dd, *J* = 7.8, 6.0 Hz, 1 H), 1.52 (s, 3 H), 1.31-1.28 (m, 4 H), 1.12 (s, 3 H), 1.09 (s, 9 H) ppm. **¹³C NMR (126 MHz, CD₂Cl₂):** δ 170.0, 137.0 (2 C), 136.7 (2 C), 134.5, 134.4, 130.24, 130.21, 127.9 (2 C), 127.8 (2 C), 114.4, 103.7, 80.7, 72.1, 71.3, 61.1, 27.5, 27.4 (3 C), 26.9, 22.7, 19.9, 14.6, 14.2 ppm.

**GCMS:** [M-(CH₂CHCO₂Et)]⁺˙ = 396, 21.93 min (Method 4).

***isomer c':* ¹H NMR (400 MHz, CD₂Cl₂):** δ 7.77-7.74 (m, 2 H), 7.66-7.64 (m, 2 H), 7.48-7.38 (m, 6 H), 5.55 (d, *J* = 3.8 Hz, 1 H), 4.24 (d, J = 4.4 Hz, 1 H), 4.11-4.05 (m, 2 H), 4.06 (d, *J* = 4.1 Hz, 1 H), 1.59-1.58 (m, 5 H), 1.52 (m, 1 H), 1.25 (s, 3 H), 1.22 (t, *J =* 7.1 Hz, 3 H), 1.08 (s, 9 H) ppm. **¹³C NMR (126 MHz, CD₂Cl₂):** δ 170.3, 136.6 (2 C), 136.3 (2 C), 133.7, 133.4, 130.7, 130.6, 128.5 (2 C), 128.1 (2 C), 114.0, 104.2, 79.2, 73.2, 69.9, 61.2, 27.24, 27.21 (3 C), 27.0, 19.7, 19.1, 14.6, 14.4 ppm. **GCMS:** [M-((3xCH₃)-tBu)]^{+•} = 381, 21.21 min (Method 4).

Alternative preparation of ***Compound 115*** (larger scale)

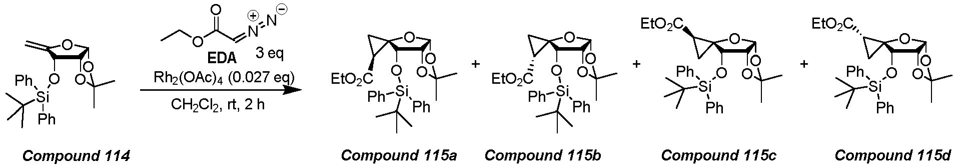

***Compound 114*** (2.00g, 4.87 mmol, 1.00 eq ) was dissolved in dry dichloromethane (20 mL) and Rh₂(OAc)₄(36.81 mg, 0.13 mmol, 0.027 eq) was added. The solution was cooled to - 10 °C and a solution of ethyl diazoacetate (1667.00 mg, 14.61 mmol, 3.00 eq) in dry dichloromethane (5 mL) was added dropwise. The resulting mixture was stirred for 2 h at - 10°C. The catalyst was filtered over a celite pad and the filtrate was concentrated under reduced pressure. The residue was purified by C18 Column (Mobile Phase A: Water (0.1mmol/L NH₄HCO₃), Mobile Phase B: ACN; Flow rate: 80 mL/min; Gradient: 60B to 90 B in 30 min; 210/254 nm). The fractions containing the product were collected and the solvent was evaporated to afford the crude, this residue was purified by prep-SFC using the following conditions: Column: Lux 3um Cellulose-4 4.6*100m m,3um, Co-solvent: MeOH (0.1%DEA), Gradient (B%) : 5% to 20% in 2m in hold 1min at 20% ,Flow rate = 4 ml/min, Temperature: 35°C, the first fractions (500 mg, crude) contained ***compound 115c*** and ***compound 115a,*** the second fractions (650 mg, crude) contained ***compound 115d*** and ***compound 115b.***

The first fraction (500 mg, crude) was re-purified by prep-SFC using the following conditions: Column: Lux 3um Cellulose-4 4.6*100mm,3um, Co-solvent: IPA:Hex=1:4(0.1%DEA), Gradient (B%) : 5% to 20% in 2min,hold 1min at 20% ,Flow rate = 4 ml/min, Temperature: 35°C, the front peak was ***compound 115a,*** the back peak was ***compound 115c.***

The second fraction (650 mg, crude) was re-purified by prep-SFC using the following conditions: Column: Lux 3um Cellulose-4 4.6* 100mm,3um, Co-solvent: MeOH (0.1%DEA), Gradient (B%): 5% to 20% in 2min, hold 1min at 20%, Flow rate = 4 ml/min, Temperature: 35°C, the front was ***compound 115b,*** the back peak was ***compound 115d.***

**2.0% yield** (50 mg, 0.10 mmol) off-white solid of ***compound 115a.* ¹H NMR (300 MHz, Chloroform-d)** δ 7.83 - 7.77 (m, 2H), 7.75 - 7.68 (m, 2H), 7.49 - 7.32 (m, 6H), 5.37 (d, J = 4.0 Hz, 1H), 4.37 (d, J = 4.4 Hz, 1H), 4.23 - 4.13 (m, 2H), 3.60 (t, J = 4.2 Hz, 1H), 2.03 - 1.95 (m, 1H), 1.73 (s, 3H), 1.59 - 1.50 (m, 1H), 1.30 (t, J = 7.1 Hz, 3H), 1.22 (d, J = 3.1 Hz, 1H), 1.16 (s, 3H), 1.08 (s, 9H) ppm. **¹³C NMR (75 MHz, CDCl₃)** δ 168.83, 136.22, 135.96, 133.60, 132.95, 129.93, 127.71, 127.39, 113.08, 102.62, 78.62, 73.75, 68.98, 60.44, 26.88, 26.29, 26.01, 25.72, 19.10, 14.25, 8.35 ppm. **LCMS (ESI+) m/z:** calcd. for C₂₈H₃₆O₆Si [M+H] ⁺ = 497.23, found 497.23, RT: 2.013 min, Method 2.

**2.4% yield** (60 mg, 0.12 mmol) yellow oil of ***compound 115b.* ¹H NMR (300 MHz, Chloroform-d)**δ 7.80 - 7.73 (m, 2H), 7.66 - 7.57 (m, 2H), 7.50 - 7.33 (m, 6H), 5.61 (d, J = 4.1 Hz, 1H), 4.29 - 4.07 (m, 3H), 4.01 (t, J = 4.6 Hz, 1H), 2.37 - 2.29 (m, 1H), 1.64 (t, J = 6.6 Hz, 1H), 1.58(m, 3 H), 1.29 (t, J = 7.1 Hz, 3H), 1.20 (s, 3H), 1.09 (s, 9H), 0.93 - 0.85 (m, 1H) ppm. **¹³C NMR (75 MHz, CDCl₃)** δ 170.53, 136.14, 135.77, 133.13, 132.85, 130.17, 130.04, 127.90, 127.55, 112.46, 104.05, 72.25, 69.64, 60.39, 26.93, 25.98, 25.94, 20.63, 19.34, 14.31, 10.04 ppm. **LCMS (ESI+) m/z:** calcd. for C₂₈H₃₆O₆Si [M+H] ⁺ = 497.23, found 497.23, RT: 2.068 min, Method 2.

**6.2% yield** (150 mg, 0.30 mmol) off-white solid of ***compound 115c.***

**12.4% yield** (300 mg, 0.60 mmol) yellow oil of ***compound 115d.***

Asymmetric preparation of ***Compound 115c***

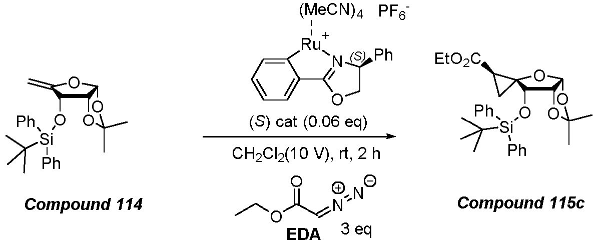

***Compound 114*** (500 mg, 1.21 mmol, 1.00 eq) was dissolved in dry dichloromethane (2.5 mL) and (S)catalyst CAS 1259070-80-8 (20.71 mg, 0.03 mmol, 0.027 eq) was added. The solution was cooled to - 10 °C and a solution of ethyl diazoacetate (206.9 mg, 1.82 mmol, 1.50 eq) in dry dichloromethane (1.5 mL) was added dropwise. The resulting mixture was stirred for 2 h at -10°C. The catalyst was filtered over a celite pad and the filtrate was concentrated under reduced pressure. The residue was purified by C18 Column (Mobile Phase A: Water (0.1mmol/L NH₄HCO₃), Mobile Phase B: ACN; Flow rate: 80 mL/min; Gradient: 60B to 90 B in 30 min; 210/254 nm). The fractions containing the product were collected and the solvent was evaporated to afford ***compound 115c.***

**50% yield** (302 mg, 0.60 mmol) white solid. **¹H NMR (300 MHz, DMSO-d6):** δ 7.71 - 7.64 (m, 2H), 7.62 - 7.56 (m, 2H), 7.51 - 7.33 (m, 6H), 5.50 (d, J = 3.8 Hz, 1H), 4.47 (d, J = 4.7 Hz, 1H), 3.98 - 3.92 (m, 1H), 3.93 - 3.81 (m, 2H), 1.94 - 1.85 (m, 1H), 1.53 - 1.44 (m, 1H), 1.43 (s, 3H), 1.29 - 1.24 (m, 1H), 1.08 (s, 3H), 1.06 (s, 3H), 1.02 ppm (s, 9H). **¹³C NMR (75 MHz, DMSO):** δ 169.32, 136.39, 136.08, 133.86, 133.62, 130.27, 127.95, 127.76, 113.28, 103.03, 79.96, 71.42, 70.56, 60.60, 55.37, 27.41, 27.31, 26.87, 21.73, 19.48, 14.50, 13.44 ppm. **LCMS (ESI+) m/z:** calcd. for C₂₈H₃₆O₆Si [M+H]+ = 497.23, found 497.23, RT: 2.076 min, Method 2.

### Preparation of Compound 145

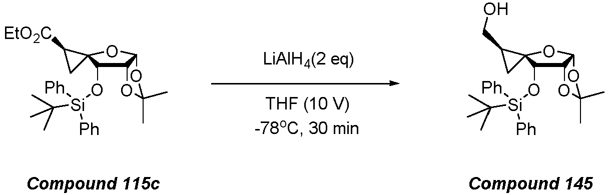

***Compound 115c*** (140 mg, 0.28 mmol, 1.00 eq) was dissolved in dry THF (2 mL). The solution was cooled to - 78 °C and LiAlH₄ (21.8 mg, 0.56 mmol, 2.00 eq) was added. The resulting mixture was stirred for 3 h at - 78 °C. The reaction was then quenched by the addition of 5 ml H₂O, 15 ml aq.NaOH(3 M) and 5ml H₂O. The solution was filtered over a celite pad and the filtrate was concentrated under reduced pressure. The residue was purified by C18 Column (Mobile Phase A: Water (0.1mmol/L NH₄HCO₃), Mobile Phase B: ACN; Flow rate: 80 mL/min; Gradient: 40B to 70 B in 30 min; 210/254 nm). The fractions containing the product were collected and the solvent was evaporated to afford ***compound 145.***

**78% yield** (101 mg, 0.21 mmol) white solid. **1H NMR (300 MHz, DMSO-d6)** δ 7.75 - 7.68 (m, 2H), 7.67 - 7.60 (m, 2H), 7.51 - 7.35 (m, 6H), 5.46 (d, J = 4.0 Hz, 1H), 4.49 - 4.41 (m, 2H), 3.92 (t, J = 4.3 Hz, 1H), 3.48 - 3.38 (m, 1H), 3.19 - 3.08 (m, 1H), 1.45 (s, 3H), 1.21 - 1.13 (m, 1H), 1.06 (s, 4H), 1.04 (s, 9H), 0.87 - 0.75 ppm (m, 2H). **13C NMR (75 MHz, DMSO)** δ 136.34, 135.99, 133.67, 133.51, 130.40, 128.20, 127.89, 112.53, 102.90, 79.80, 71.45, 67.37, 59.66, 27.35, 27.16, 26.83, 19.75, 19.53, 11.95 ppm. **LCMS (ESI+) m/z:** calcd. for C₂₆H₃₄O₅Si [M+H₂O]+ = 472.22, found 472.22, RT: 1.858 min, Method 2.

### Preparation of Compound 146

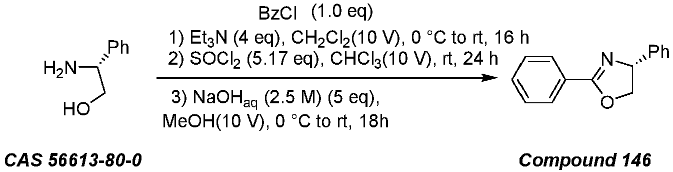

CAS 56613-80-0 (5 g, 36.49 mmol, 1.10 eq) and triethylamine (19.14 mL, 132.69 mmol, 4.00 eq) in dichloromethane (50 mL) was added a solution of benzoylchloride (4.64 g, 33.17 mmol, 1.00 eq) in dichloromethane (20mL) at 0 °C. After stirring at room temperature for 16 h, the reaction mixture was concentrated under reduce pressure. The residue was dissolved in CHCl₃ (50 mL) and was treated with SOCl₂ (20.40 g, 171.49 mmol, 5.17 eq) at 0 °C. After stirring at room temperature for 24 h, the solvent and SOCl₂ were removed under reduce pressure. Saturated aqueous solution of NaHCO₃ (100 mL) was added to the residue with stirring for 5 min. The organic product was extracted with dichloromethane (3 × 100 mL), dried over anhydrous Na₂SO₄, filtered, and concentrated under reduced pressure. Subsequently, to a solution of the previous residue in methanol (50 mL) was added an aqueous solution of NaOH (66 mL, 165.85 mmol, 2.5 M, 5 eq) at 0 °C. After stirring at room temperature for 18 h, the solvent was removed under vacuo, followed by the addition of water (60 mL) and the resulting aqueous mixture was extracted with dichloromethane (3 × 50 mL). The organic layer dried over anhydrous Na₂SO₄, filtered, and evaporated under vacuo to afford the crude mixture. The crude was purified by a silica gel column chromatography (elution with petroleum ether/EtOAc = 90:10) to afford ***compound 146.***

**83%** (6.2 g, 27.53 mmol) orange oil that turned solid after being stored in the freezer. **1H NMR (400 MHz, CDCl₃):** 8.07-8.04 (m, 2 H), 7.52 (m, 1 H), 7.47-7.42 (m, 2 H), 7.39-7.27 (m, 5 H), 5.40 (dd, J = 10.1, 8.2 Hz, 1 H), 4.81 (dd, J = 10.1, 8.4 Hz, 1 H), 4.29 ppm (t, J = 8.3 Hz, 1 H). **13C NMR (100 MHz, CDCl₃):** 164.9, 142.5, 131.7, 128.9, 128.6, 128.5, 127.8, 127.7, 126.9, 75.0, 70.2 ppm. **LCMS (ESI+) m/z:** calcd. for C₁₅H₁₃NO [M+H]+ = 224.15, found 224.15, RT: 1.389 min, Method 2.

### Preparation of (R)-Ph-Pheox Ru(II) (Compound 147)

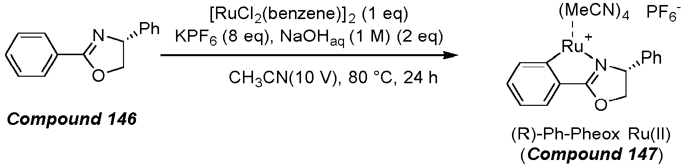

***Compound 146*** (200 mg, 0.89 mmol, 2.00 eq), [RuCl₂(benzene)]₂ (231 mg, 0.44 mmol, 1.00 eq), and KPF₆ (678 mg, 3.52 mmol, 8.00 eq). The reaction flask was evacuated and backfilled with argon. Through the side arm degassed ACN (10 mL) and an aqueous solution of NaOH (0.9 mL, 0.89 mmol, 1 M, 2 eq) were injected. The suspension was refluxed at 80 °C for 24 h. The solvent was removed under reduced pressure to afford the crude mixture. The crude was purified by a silica gel column chromatography (elution with CH₂Cl₂/ACN = 99:1 then 95:5) to afford (*R*)-*Ph*-Pheox Ru(II) ***(compound 147).***

**70%** (400 mg, 0.31mmol) as a yellow solid. **1H NMR (400 MHz, CDCl₃):** 7.79 (m, 1 H), 7.55 (d, J = 7.5 Hz, 1H), 7.35-7.32 (m, 5 H), 7.20 (t, J = 7.3 Hz, 1 H), 6.96 (m, 1 H), 5.28 (m, 1 H), 5.10 (dd, J = 9.8, 8.9 Hz, 1 H), 4.55 (dd, J = 8.6, 7.4 Hz, 1 H), 2.50 (s, 3 H), 2.24 (s, 3 H), 2.20 (s, 3 H), 2.04 ppm (s, 3 H). **LCMS (ESI+) m/z:** calcd. for C₂₃H₂₄N₅ORu+ [M]+ = 488.10, found 488.10, RT: 1.615 min, Method 6.

### Asymmetric preparation of Compound 115d

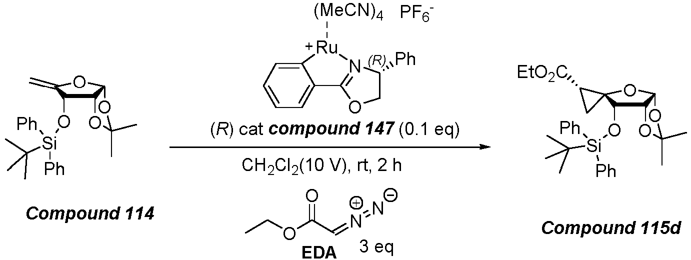

***Compound 114*** (500 mg, 1.21 mmol, 1.00 eq) was dissolved in dry dichloromethane (2.5 mL) and (R) catalyst, ***compound 147,*** (82.86 mg, 0.12 mmol, 0.1 eq) was added. The solution was cooled to - 10 °C and a solution of ethyl diazoacetate (468.27 mg, 3.63 mmol, 3.00 eq) in dry dichloromethane (1.5 mL) was added dropwise. The resulting mixture was stirred for 2 h at - 10°C. The catalyst was filtered over a celite pad and the filtrate was concentrated under reduced pressure. The residue was purified by C18 Column (Mobile Phase A: Water (0.1mmol/L NH₄HCO₃), Mobile Phase B: ACN; Flow rate: 80 mL/min; Gradient: 60B to 90 B in 30 min; 210/254 nm). The fractions containing the product were collected and the solvent was evaporated to afford ***compound 115d.***

**30% yield** (254 mg, 0.36 mmol) white solid. **1H NMR (400 MHz, Chloroform-d)** δ 7.81 - 7.76 (m, 2H), 7.70 - 7.63 (m, 2H), 7.52 - 7.39 (m, 6H), 5.60 (d, J = 3.8 Hz, 1H), 4.25 (d, J = 4.5 Hz, 1H), 4.21 - 4.10 (m, 2H), 4.03 (t, J = 4.2 Hz, 1H), 1.68 - 1.60 (m, 6H), 1.31 - 1.24 (m, 6H), 1.10 ppm (s, 9H). **13C NMR (101 MHz, CDCl3)** δ 170.09, 136.10, 135.75, 133.13, 132.87, 130.16, 130.04, 127.92, 127.57, 113.41, 103.71, 78.55, 72.68, 69.56, 60.67, 26.94, 26.88, 26.79, 19.35, 18.76, 14.28, 14.20 ppm. **LCMS (ESI+) m/z:** calcd. for C₂₈H₃₆O₆Si [M+H]+ = 497.23, found 497.23, RT: 2.019 min, Method 2.

### Preparation of Compound 148

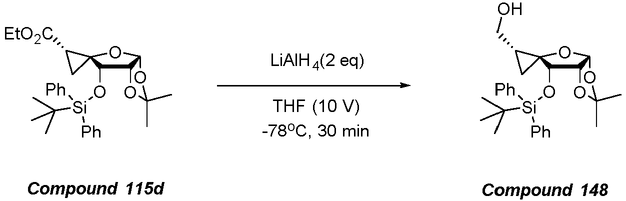

***Compound 115d*** (200 mg, 0.40 mmol, 1.00 eq) was dissolved in dry THF (2 mL). The solution was cooled to - 78 °C and LiAlH₄ (31 mg, 0.80 mmol, 2.00 eq) was added. The resulting mixture was stirred for 3 h at - 78 °C. The reaction was then quenched by the addition of 5 ml H₂O, 15 ml aq.NaOH(3 M) and 5ml H₂O. The solution was filtered over a celite pad and the filtrate was concentrated under reduced pressure. The residue was purified by C18 Column (Mobile Phase A: Water (0.1mmol/L. NH₄HCO₃), Mobile Phase B: ACN; Flow rate: 80 mL/min; Gradient: 40B to 70 B in 30 min; 210/254 nm). The fractions containing the product were collected and the solvent was evaporated to afford ***compound 148.***

**73% yield** (132 mg, 0.29 mmol) white solid. **1H NMR (300 MHz, Chloroform-d)** δ 7.83 - 7.74 (m, 2H), 7.68 - 7.60 (m, 2H), 7.52 - 7.34 (m, 6H), 5.57 (d, J = 3.9 Hz, 1H), 4.20 (d, J = 4.5 Hz, 1H), 4.00 (t, J = 4.2 Hz, 1H), 3.97 - 3.87 (m, 1H), 3.66 - 3.55 (m, 1H), 1.16 (s, 3H), 1.41 - 1.32 (m, 1H), 1.25 (s, 3H), 1.08 (s, 9H), 0.93 - 0.82 (m, 1H), 0.77 - 0.70 ppm (m, 1H). **13C NMR (75 MHz, CDCl₃)** δ 136.10, 135.75, 133.45, 133.04, 130.01, 129.91, 127.80, 127.47, 113.21, 103.55, 78.98,72.59, 67.52, 62.73, 26.86, 26.74, 26.62, 19.34, 16.30, 11.58 ppm. **LCMS (ESI+) m/z:** calcd. for C₂₆H₃₄O₅Si [M+H₂O]+ = 472.22, found 472.22, RT: 1.861 min, Method 2.

### Preparation of CAS: 29834-94-4

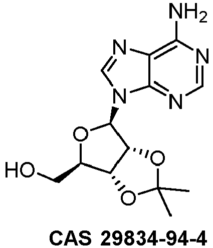

Adenosine (52.9 g, 197.9 mmol, CAS: 58-61-7) andp-toluenesulfonic acid monohydrate (43.3 g, 227.6 mmol) were dissolved in acetone (1 L). The reaction mixture was stirred for 30 min, after which anhydrous triethyl orthoformate (151 mL, 906.6 mmol) was added. The reaction mixture was stirred until complete conversion. The mixture was neutralized by addition of Na₂CO₃ (aq. sat. 1 L), after which DCM:MeOH (9:1, v/v, 1 L) was added, this resulted in formation of a white precipitate. The precipitate was collected by filtration. The aqueous phase was separated, followed by the addition of DCM:MeOH (9:1, v/v, 1 L), once more yielding a white precipitate. The combined precipitates were washed with DCM and water, and dried in a vacuum oven (60 °C) to afford CAS 29834-94-4*.* The product was used in the next step without further purification.

**56% yield** (34.8 g, 110.4 mmol), white powder. **1H NMR (400 MHz, Chloroform-*d*)** δ 8.32 (s, 1H) 7.82 (s, 1H) 6.35 (dd, *J*=11.7, 1.8 Hz, 1H) 5.85 (d, *J*=4.9 Hz, 1H) 5.57 (br s, 2H) 5.21 (t, *J*=5.4 Hz, 1H) 5.12 (dd, *J*=5.9, 1.1 Hz, 1H) 4.53 (s, 1H) 3.97 (dt, *J*=12.8, 1.7 Hz, 1H) 3.75 - 3.82 (m, 1H) 1.65 (s, 3H) 1.38 ppm (s, 3H).

### Preparation of CAS: 139301-93-2

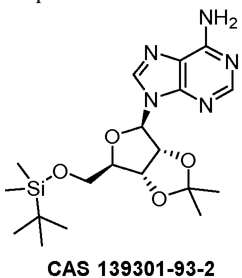

CAS 29834-94-4 (11.3 g, 34.8 mmol) and imidazole (4.74 g, 69.6 mmol) were dissolved in THF:DMF (5:1, v/v, 150 mL). TBDMSCl (7.87 g, 52.2 mmol) was added slowly. After 18 hours, water was added to the reaction mixture and the solvents of the reaction were evaporated *in vacuo* to yield a white precipitate. The precipitate was collected, washed with water, and dried in a vacuum oven (60 °C) to afford CAS 139301-93-2***.*** The product was used in the next step without further purification.

**85% yield** (12.7 g, 29.5 mmol), white powder. **1H NMR (400 MHz, Chloroform-*d*)** δ 8.38 (s, 1H), 8.05 (s, 1H), 6.17 (d, *J*=2.4 Hz, 1H), 5.54 (br s, 2H), 5.28 (dd, *J*=2.6, 6.2 Hz, 1H), 4.96 (dd, *J*=2.40, 6.2 Hz, 1H), 4.39-4.46 (m, 1H), 3.85-3.91 (m, 1H), 3.74-3.79 (m, 1H), 1.63 (s, 3H), 1.41 (s, 3H), 0.85 (s, 9H), 0.02 (s, 3H), 0.01 ppm (s, 3H). **LC/MS:** *m*/*z* 422.2 [M+H]⁺, RT 2.09 min, purity: 98%, method 13.

### Preparation of CAS: 1152172-19-4

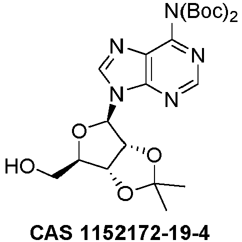

CAS: 139301-93-2 (12.5 g, 26.7 mmol) and DMAP (0.362 g, 2.97 mmol) were dissolved in THF (100 mL). Di-*tert*-butyl carbonate (20.6 g, 94.3 mmol) in THF (50 mL) was added dropwise. After 4 hours, the reaction mixture was diluted with water, extracted with EtOAc, and washed with brine and NaHCO₃ (aq. sat.). The organic layer was dried (MgSO₄), filtered and the filtrate was concentrated *in vacuo* to obtain CAS 1152172-19-4***.*** The product was used in the next step without further purification.

**89% yield** (16.2 g, 26,5 mmol), yellow oil. **1H NMR (400 MHz, Chloroform-*d*)** δ 8.85 (s, 1H), 8.14 (s, 1H), 5.96 (d, *J*=4.7 Hz, 1H), 5.39 (br d, *J*=10.9 Hz, 1H), 5.20-5.25 (m, 1H), 5.13 (dd, *J*=1.4, 6.0 Hz, 1H), 4.56 (d, *J*=1.5 Hz, 1H), 4.00 (d, *J*=12.8 Hz, 1H), 3.79-3.87 (m, 1H), 1.66 (s, 3H), 1.48 (s, 18H), 1.40 ppm (s, 3H).

### Preparation of Compound 87

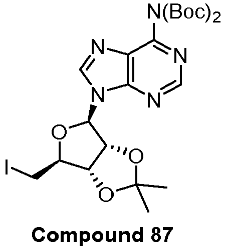

CAS: 1152172-19-4 (15.2 g, 24.9 mmol), triphenylphosphine (7.18 g, 27.4 mmol), and imidazole (3.39 g, 49.8 mmol) were dissolved in dry THF (285 mL). Iodine (9.47 g, 37.3 mmol) in dry THF (95.0 mL). was added dropwise. After 1 hour, the reaction mixture was quenched with Na₂S₂O₃ (aq. sat.) and the product was extracted with EtOAc. The organic layer was washed with water and brine, dried (MgSO₄), filtered, and the filtrate was concentrated *in vacuo.* The resulting oil was triturated in DIPE (300 mL), filtered, washed with DIPE, and the filtrate was concentrated *in vacuo.* The residue was purified by column chromatography over silica gel (isocratic elution: *n*-heptane:EtOAc 1: 1) to obtain ***compound 87.***

**90% yield** (16.2 g, 22.3 mmol), yellow oil. **1H NMR (400 MHz, Chloroform-*d*)** δ 8.88 (s, 1H), 8.24 (s, 1H), 6.21 (d, *J*=2.2 Hz, 1H), 5.50 (dd, *J*=2.4, 6.4 Hz, 1H), 5.09 (dd, *J*=2.9, 6.4 Hz, 1H), 4.44 (ddd, *J*=3.1, 5.2, 8.0 Hz, 1H), 3.44 (dd, *J*=7.9, 10.3 Hz, 1H), 3.29 (dd, *J*=5.3, 10.3 Hz, 1H), 1.64 (s, 3H), 1.47-1.48 (s, 18H), 1.42 ppm (s, 3H).

### Preparation of Compound 88

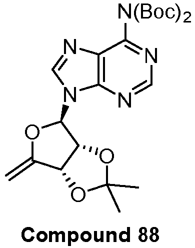

***Compound 87*** (16.4 g, 22.6 mmol) was dissolved in THF (120 mL). The reaction mixture was heated to 65 °C and DBU (5.06 mL, 33.9 mmol) was added. After one hour, the reaction mixture was allowed to cool to room temperature. The mixture was filtered and washed with EtOAc . The filtrate was washed with water and brine. The organic layer was dried (MgSO₄), filtered, and the filtrate was concentrated *in vacuo.* The residue was purified by recrystallization in DIPE (650 mL) to give ***compound 88.*** The mother liquor was concentrated *in vacuo* and purified via reversed phase preparative HPLC to give an extra fraction of pure ***compound 88.***

**74% combined yield** (10.3 g, 47.6 mmol), white solid. **¹H NMR (400 MHz, Chloroform-*d*)** δ 8.84 (s, 1H), 8.08 (s, 1H), 6.32 (d, *J*=0.7 Hz, 1H), 5.54 (d, *J*=6.1 Hz, 1H), 5.32 (dd, *J*=0.8, 6.1 Hz, 1H), 4.66 (dd, *J*=1.0, 2.6 Hz, 1H), 4.53 (dd, *J*=0.6, 2.6 Hz, 1H), 1.61 (s, 3H), 1.49 (s, 18H), 1.45 ppm (s, 3H). **¹³C NMR (101 MHz, Chloroform-*d*)** δ 161.4, 152.4, 150.5, 142.9, 114.4, 90.7, 89.1, 83.9, 82.7, 79.5, 27.8, 26.8, 25.7 ppm. **LC/MS:** *m*/*z* 490.2 [M+H]⁺, RT 2.18 min, purity 99%, method 13.

### Preparation of Compounds 89a, 89b, 89c and 89d

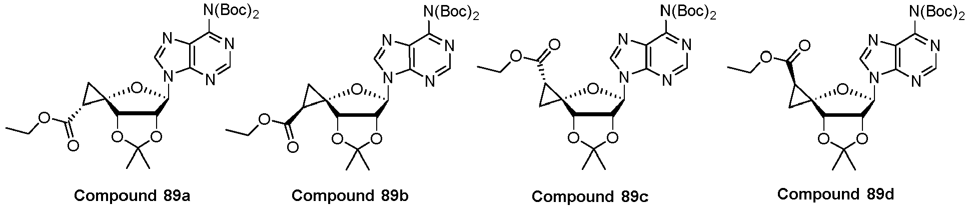

***Compound 88*** (0.40 g, 0.76 mmol) and Rhodium(II) triphenylacetate dimer (0.052 g, 0.038 mmol) were dissolved in dry DCM (8 mL) and heated to reflux (45 °C). Ethyldiazoacetate (0.20 mL, 1.9 mmol) in dry DCM (2.5 mL) was added to the reaction mixture via a syringe pump over a duration of 5 hours, after which the reaction mixture was allowed to cool to room temperature. The reaction mixture was concentrated *in vacuo* and purified via column chromatography over silica gel (gradient: n-heptane/ EtOAc from 95:5 to 60:40 in 12 CV; *n-*heptane/ EtOAc 60:40 in 10 CV) to give a fraction containing ***compounds 89b, 89c*** and ***89d*** as an inseparable mixture (first eluting fraction) and a fraction containing ***compound 89a*** as a single isomer. Both fractions were further purified by preparative supercritical fluid chromatography to give pure ***compounds 89b, 89c*** and ***89d.***

***Compound 89a:* 24% yield** (0.11 g, 0.18 mmol), colorless oil. **¹H NMR (400 MHz, Chloroform-*d*)** δ 8.85 (s, 1H), 8.14 (s, 1H), 6.19 (s, 1H), 5.61 (d, *J*=5.94 Hz, 1H), 5.04 (d, *J*=5.94 Hz, 1H), 4.14-4.25 (m, 2H), 2.29 (dd, *J*=7.3, 9.24 Hz, 1H), 1.63 (s, 3H), 1.46 (s, 18H), 1.45 (s, 3H), 1.42-1.48 (m, 1H), 1.27 (t, *J*=7.2 Hz, 2H), 1.21-1.30 ppm (m, 2H). **LC/MS:** *m*/*z* 576.3 [M+H]⁺, RT 2.28 min, purity 99%, method 9. **SFC/MS:** *m*/*z* 576.331 [M+H]⁺, RT 2.40 min, purity: 100%, method 1.

***Compound 89b:* 16% yield** (0.075 g, 0.13 mmol), colorless oil. **¹H NMR (400 MHz, Chloroform-*d*)** δ 8.88 (s, 1H), 8.08 (s, 1H), 6.17 (s, 1H), 5.72 (d, *J*=5.94 Hz, 1H), 5.26 (d, *J*=5.94 Hz, 1H), 4.22 (ddd, *J*=7.2, 10.8, 33.8 Hz, 2H), 2.22 (dd, *J*=7.04, 9.7 Hz, 1H), 1.58 (s, 3H), 1.51 (dd, *J*=6.2, 6.8 Hz, 1H), 1.46 (s, 18H), 1.41 (s, 3H), 1.30 (t, *J*=7.2 Hz, 3H), 0.79 ppm (dd, *J*=6.05, 9.57 Hz, 1H). **LC/MS:** *m*/*z* 576.3 [M+H]⁺, RT 2.32 min, purity 96%, method 9. **SFC/MS:** *m*/*z* 576.331 [M+H]⁺, RT 2.16 min, purity: 100%, method 1.

***Compound 89c:* 6% yield** (0.033 g, 0.048 mmol), colorless oil. **¹H NMR (400 MHz, Chloroform-*d*)** δ 8.86 (s, 1H), 8.05 (s, 1H), 6.18 (s, 1H), 5.73 (d, *J*=5.94 Hz, 1H), 5.10 (d, *J*=5.94 Hz, 1H), 3.67-3.79 (m, 1H), 3.26-3.36 (m, 1H), 1.91-1.96 (m, 1H), 1.82-1.86 (m, 1H), 1.63 (s, 3H), 1.53-1.54 (m, 1H), 1.50 (s, 18H), 1.46 (s, 3H), 0.88 ppm (t, *J*=7.2 Hz, 3H). **LC/MS:** *m*/*z* 576.3 [M+H]⁺, RT 2.28 min, purity 83%, method 9. **SFC/MS:** *m*/*z* 576.331 [M+H]⁺, RT 2.83 min, purity: 98%, method 1.

***Compound 89d:* 8% yield** (0.037 g, 0.057 mmol), colorless oil. **¹H NMR (400 MHz, Chloroform-*d*)** δ 8.75 (s, 1H) 8.08 (s, 1H) 6.17 (s, 1H) 5.68 (d, *J*=5.9 Hz, 1H) 5.51 (d, *J*=5.9 Hz, 1H) 4.22 (m, *J*=10.9, 7.2 Hz, 1H) 3.98 - 4.04 (m, 1H) 1.62 - 1.66 (m, 1H) 1.64 (s, 3H) 1.56 (s, 3H) 1.52 - 1.54 (m, 1H) 1.46 (s, 18H) 1.42 (dd, *J*=9.5, 7.3 Hz, 1H) 1.20 ppm (t, *J*=7.2 Hz, 3H) **LC/MS:** *m*/*z* 576.3 [M+H]⁺, RT 2.30 min, purity 89%, method 9. **SFC/MS:** *m*/*z 576.331* [M+H]⁺, RT 1.87 min, purity: 100%, method 1.

### Preparation of Compound 90b

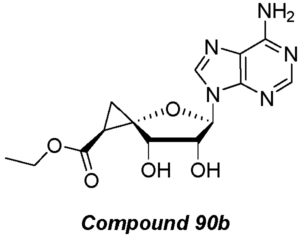

***Compound 89b*** (0.075 g, 0.13 mmol) was dissolved in DCM (1 mL). TFA (0.24 mL, 3.1 mmol) was added dropwise. After 7 days, the reaction mixture was concentrated *in vacuo* and purified via reversed phase preparative HPLC followed by lyophilization to afford ***Compound 90b. 9%* yield** (4.3 mg, 0.012 mmol), white powder. **¹H NMR** (400 MHz, METHANOL-*d*₄) δ 8.29 (s, 1H) 8.21 (s, 1H) 6.13 (d, *J*=7.5 Hz, 1H) 5.29 (dd, *J*=7.5, 4.4 Hz, 1H) 4.26 (d, *J*=4.3 Hz, 1H) 4.12 - 4.24 (m, 2H) 2.07 (dd, *J*=9.6, 6.9 Hz, 1H) 1.58 (dd, *J*=6.7, 6.1 Hz, 1H) 1.42 (dd, *J*=9.7, 6.0 Hz, 1H) 1.29 ppm (t, *J*=7.2 Hz, 3H). **¹³C NMR** (101 MHz, METHANOL-*d*₄) δ 172.89, 157.51, 154.13, 151.27, 141.87, 120.82, 89.26, 76.11, 75.01, 74.79, 62.19, 23.59, 20.00, 14.59 ppm. **LC/MS:** *m*/*z* 336.0 [M+H]⁺, RT 1.08 min, purity 96%, method 13.

### Preparation of Compound 91a

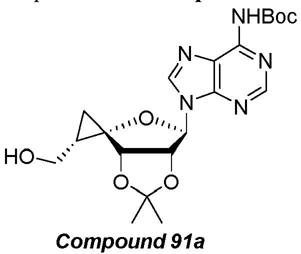

***Compound 89a*** (0.060 g, 0.10 mmol) was dissolved in dry THF (1 mL). Lithium borohydride (2M in THF) (0.13 mL, 0.26 mmol) was added dropwise. After 24 hours, the reaction mixture was quenched with NH₄Cl (aq. sat. 2 mL) and the product was extracted with EtOAc. The organic layer was washed with brine, dried (MgSO₄), filtered, and concentrated *in vacuo.* The residue was purified via reversed phase preparative HPLC to afford ***compound 91a.***

**8% yield** (0.004 g; 0.0078 mmol), brown oil. **¹H NMR (400 MHz, Chloroform-*d*)** δ 8.75 (s, 1H), 8.03 (br s, 1H), 7.97 (s, 1H), 6.18 (s, 1H), 5.74 (d, *J*=6.1 Hz, 1H), 4.94 (d, *J*=6.1 Hz, 1H), 3.90 (dd, *J*=5.5, 11.6 Hz, 1H), 3.50-3.60 (m, 1H), 1.66-1.68 (m, 1H), 1.64-1.66 (m, 1H), 1.62 (s, 3H), 1.58 (s, 9H), 1.43 (s, 3H), 0.91 (dd, *J*=6.3, 10.0 Hz, 1H), 0.39 ppm (t, *J*=6.7 Hz, 1H) **¹³C NMR (101 MHz, Chloroform-*d*)** δ 153.1, 150.7, 150.1, 149.6, 142.0, 121.9, 113.5, 90.2, 84.9, 84.3, 82.4, 72.9, 62.0, 28.1, 26.7, 25.4, 20.0, 17.5 ppm.

### Preparation of Compound 92a

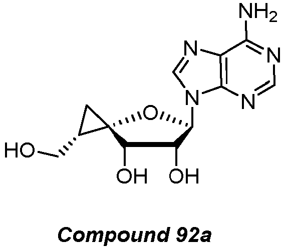

***Compound 91a*** was deprotected to give ***Compound 92a*** using the method as described for the deprotection of ***Compound 89b.***

**30% yield** (0.045 g, 0.141 mmol), white solid. ¹H NMR (400 MHz, CD₃OD) δ 8.18 (s, 1 H), 8.11 (s, 1 H), 6.03 (d, J=7.0 Hz, 1 H), 5.17 (dd, J=7.2, 4.3 Hz, 1 H), 4.09 (d, J=4.4 Hz, 1 H), 3.67 (dd, J=11.9, 6.8 Hz, 1 H), 3.40 (dd, J=11.9, 8.6 Hz, 1 H), 1.42 - 1.54 (m, 1 H), 0.99 (dd, J=10.1, 6.2 Hz, 1 H), 0.75 ppm (t, J=6.5 Hz, 1 H),. 13C NMR (101 MHz, CD3OD) δ 157.30, 153.92, 151.07, 141.51, 120.26 (derived from HMBC), 88.93, 76.72, 75.76, 72.54, 63.74, 23.28, 16.89 ppm. **LC/MS** *m*/*z* 294.0 [M+H]⁺, RT 0.79 min, method 13.

### Alternative preparation of Compound 92a

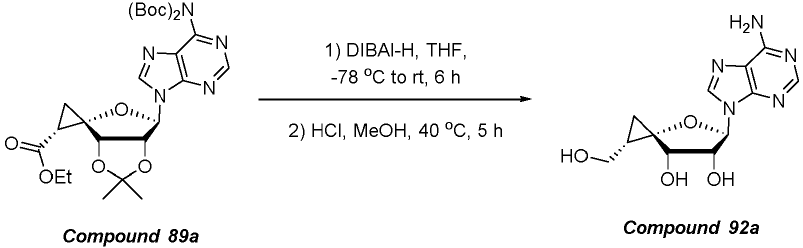

***Compound 89a*** (0.300 g, 0.469 mmol) was dissolved in dry THF (6 mL). Subsequently, the mixture was cooled to -78 °C before dropwise addition of DIBAl-H (1M in THF, 1.88 mL). After six hours, the reaction mixture was quenched by addition of Rochelle's Salt (aq. sat.), diluted with H₂O, and extracted with EtOAc. The organic layer was washed with brine, dried (MgSO₄), filtered, and concentrated *in vacuo* to afford the partly deprotected alcohol. The crude alcohol was dissolved in methanol (10.0 mL). HCl (aq. 2M, 10.0 mL) was added slowly. The reaction mixture was heated to 40 °C. After five hours, the reaction mixture was neutralized by addition of Na₂CO₃ (1.07 g, 10.1 mmol). The reaction mixture was concentrated *in vacuo* and purified via reversed phase preparative HPLC to afford ***compound 92a.***

**15% yield** (0.022 g, 0.0683 mmol), white solid. **¹H NMR** (400 MHz, CD₃OD) δ 8.28 (s, 1 H), 8.21 (s, 1 H), 6.23 (d, J=25.7 Hz, 1 H), 6.07 (d, J=5.9 Hz, 1 H), 5.23 - 5.34 (m, 1 H), 5.06 (t, J=5.5 Hz, 1 H), 4.48 - 4.51 (m, 1 H), 4.12 (d, J=5.1 Hz, 1 H), 3.55 - 3.71 (m, 2 H), 1.50 - 1.59 (m, 1 H), 1.02 (dd, J=9.9, 6.2 Hz, 1 H), 0.80 ppm (t, J=6.6 Hz, 1 H),. **¹³C NMR** (101 MHz, CD₃OD) δ 155.96, 152.55, 149.50, 139.96, 119.15, 88.24, 74.58, 73.52, 70.82, 60.18, 19.39, 13.45 ppm. **LC/MS:** *m*/*z* 294.0 [M+H]⁺, RT 0.87 min, purity 97%, Method 13.

### Preparation of Compound 92b

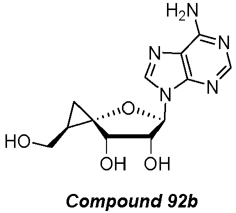

***Compound 92b*** was prepared from ***compound 89b*** using the method described for preparation of ***compound 92a*** from ***compound 89a.***

**30% yield** (0.045 g, 0.141 mmol), white solid. **¹H NMR** (400 MHz, CD₃OD) δ 8.18 (s, 1 H), 8.11 (s, 1 H), 6.03 (d, *J*=7.0 Hz, 1 H), 5.17 (dd, *J*=7.2, 4.3 Hz, 1 H), 4.09 (d, *J*=4.4 Hz, 1 H), 3.67 (dd, *J=*11.9*,* 6.8 Hz, 1 H), 3.40 (dd, *J*=11.9*,* 8.6 Hz, 1 H), 1.42 - 1.54 (m, 1 H), 0.99 (dd, *J=10.1,* 6.2 Hz, 1 H), 0.75 ppm (t, J=6.5 Hz, 1 H),. **¹³C NMR** (101 MHz, CD₃OD) δ 157.30, 153.92, 151.07, 141.51, 120.26 (confirmed by HMBC), 88.93, 76.72, 75.76, 72.54, 63.74, 23.28, 16.89 ppm. **LC/MS:** *m*/*z* 294.2 [M+H]⁺, RT 0.78 min, purity 100%, Method 13.

### Preparation of Compound 92c

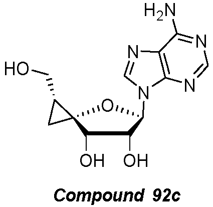

***Compound 92c*** was prepared from ***compound 89c*** using the method described for preparation of ***compound 92a*** from ***compound 89a.***

**28% yield** (0.040 g, 0.131 mmol), white solid. **¹H NMR** (400 MHz, CD₃OD) δ 8.39 (s, 1 H), 8.18 (s, 1 H), 6.13 (d, J=7.7 Hz, 1 H), 5.09 (dd, *J*=7.7, 4.4 Hz, 1 H), 3.97 (d, *J*=4.6 Hz, 1 H), 3.83 (dd, *J*=11.7, 4.8 Hz, 1 H), 3.34 - 3.41 (m, 1 H), 1.38 - 1.47 (m, 1 H), 1.16 - 1.22 (m, 1 H), 0.69 ppm (t, *J*=6.8 Hz, 1 H),. **¹³C NMR** (101 MHz, CD₃OD) δ 155.99, 152.20, 148.62, 140.94, 119.42, 88.35, 75.11, 73.70, 71.02, 59.93, 24.10, 9.21 ppm. **LC/MS:** *m*/*z* 294.2 [M+H]⁺, RT 0.82 min, purity 100%, Method 13.

### Preparation of Compound 92d

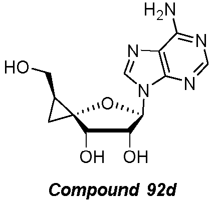

***Compound 92d*** was prepared from ***compound 89d*** using the method described for preparation of ***compound 92a*** from ***compound 89a.***

**14% yield** (0.029 g, 0.0797 mmol), white solid. **¹H NMR** (400 MHz, CD₃OD) δ 8.37 (s, 1 H), 8.19 (s, 1 H), 6.09 (d, *J*=5.9 Hz, 1 H), 5.10 (t, *J*=5.5 Hz, 1 H), 4.27 (d, *J*=5.1 Hz, 1 H), 3.88 (dd, *J=11.4,* 5.9 Hz, 1 H), 3.22 - 3.29 (m, 1 H), 1.48 - 1.58 (m, 1 H), 1.06 (dd, *J*=10.2, 6.7 Hz, 1 H), 0.89 ppm (t, *J*=6.8 Hz, 1 H). **¹³C NMR** (101 MHz, CD₃OD) δ 155.98, 152.51, 149.19, 140.12, 119.06, 87.83, 75.82, 71.37, 70.75, 61.52, 24.38, 10.60 ppm. **LC/MS:** *m*/*z* 294.0 [M+H]⁺, RT 0.79 min, purity 100%, Method 13.

***Compound 92d*** corresponds to ***compound 73.*** Therefore, this represents an alternative preparation of ***compound 73.***

### Preparation of Compound 116

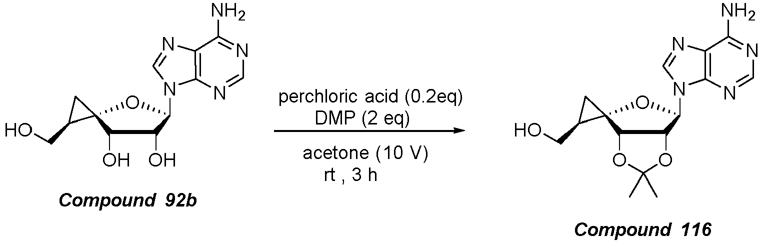

***Compound 92b*** (3.21 g, 10.95 mmol, 1.00 equiv) was dissolved in acetone (35 mL), DMP (2.27 g, 21.82 mmol, 2.00 equiv) and perchloric acid (0.22 g, 2.20 mmol, 0.20 equiv) was added at 0 °C. The reaction was stirred at RT for 3 h. The reaction was then added 1M NaOH aqueous until PH=7. The resulting solution was added 100 mL DCM and extracted with 1x 100 mL of H₂O and the organic layers combined. The mixture was dried over anhydrous sodium sulfate and concentrated under vacuum. The residue was applied onto a silica gel column with dichlormethane/methanol (gradient elution: DCM/MeOH from 99:1 to 85:15).

The fractions containing the product were collected and the solvent was evaporated to afford ***compound 116.***

**71% yield** (2.6 g, 7.80 mmol), light yellow solid. **¹H NMR** (300 MHz, Methanol-d4) δ 8.20 (d, J = 6.0 Hz, 2H), 6.22 (s, 1H), 5.72 (d, J = 5.8 Hz, 1H), 5.12 (d, J = 5.8 Hz, 1H), 3.79 (dd, J = 11.9, 6.6 Hz, 1H), 3.48 (dd, J = 11.9, 7.8 Hz, 1H), 1.59 (d, J = 2.8 Hz, 3H), 1.41 (s, 3H), 1.28 (s, 1H), 0.77 (t, J = 6.4 Hz, 1H), 0.44 ppm (dd, J = 10.2, 5.9 Hz, 1H). **¹³C NMR** (101 MHz, MeOD) δ 155.95, 152.60, 149.35, 140.55, 118.62, 112.89, 88.78, 84.48, 84.12, 71.14, 62.02, 25.25, 23.89, 20.91, 17.46 ppm. **LCMS (ESI+)** m/z: calcd. for C₁₅H₁₉N₅O₄ [M+H]+ =334.14 found 334.05, RT: 1.026 min, Method 3.

### Preparation of Compound 117

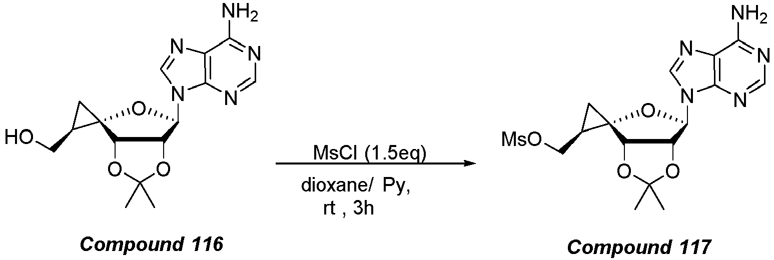

***Compound 116*** (500 mg, 1.50 mmol, 1.00 equiv) was dissolved in dioxane (5 ml), pyridine (355 mg, 4.50 mmol, 3.00 equiv) and MsCl ( 256 mg, 2.25 mmol, 1.50 equiv) was added at 0 °C. The reaction was stirred at 0 °C for 3 h. Then pour the reaction into 10 ml of ice water and extract with 1x20 ml dichloromethane, and concentrated under reduced pressure giving crude ***compound 117.***

(500 mg crude, 1.21 mmol), light yellow oil. LCMS(ESI+)m/z:calcd. for C₁₆H₂₁N₅O₆S [M+H]+ =412.12, found 412.10, RT: 0.600 min, Method 6.

### Preparation of Compound 118

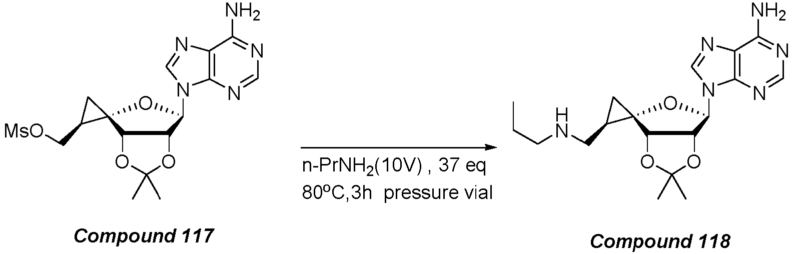

***Compound 117*** (200 mg crude) was dissolved in n-PrNH2 (2 ml) and the mixture was heated to 80°C for 3 h. Subsequently, the solution was cooled to room temperature. The mixture was extracted with CH₂Cl₂ (3x 10 ml) and combined organic layers were dried (Na₂SO₄), filtrated and the filtrate was concentrated in vacuo. The residue was applied onto a silica gel column with dichlormethane/ methanol (gradient elution: DCM/MeOH from 99:1 to 60:40). The fractions containing the product were collected and the solvent was evaporated to afford ***compound 118.***

**69% yield** (154 mg, 0.26mmol, 2 steps), brown solid. **¹H NMR** (300 MHz, Methanol-d4) δ 8.22 (d, J = 0.7 Hz, 2H), 6.26 (s, 1H), 5.80 (d, J = 5.8 Hz, 1H), 5.24 (d, J = 5.8 Hz, 1H), 3.03 - 2.97 (m, 2H), 2.95 - 2.86 (m, 2H), 2.71 (s, 3H), 1.73 - 1.68 (m, 2H), 1.62 (s, 3H), 1.45 (s, 3H), 1.03 (d, J = 3.2 Hz, 2H), 0.64 ppm (dd, J = 10.1, 6.3 Hz, 1H). m/z:calcd. for C₁₈H₂₆N₆O₃[M+H]+ =375.21, found 375.25, RT: 1.517 min, Method 3.

### Preparation of Compound 119

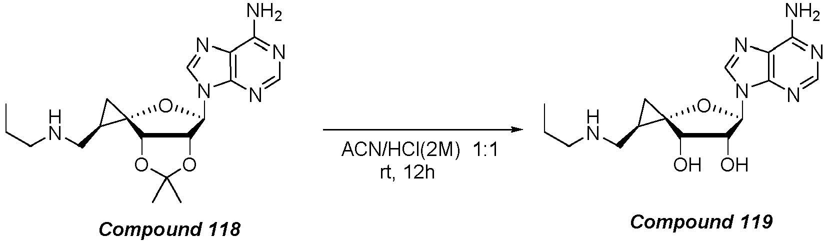

***Compound 118*** (110 mg, 0.29 mmol, 1.00eq) in ACN (1.0 ml) then was added 2M HCl (1.0ml), the mixture was heated to rt for 12h. Subsequently carefully quenched with NaHCO₃ to PH= 7. The filtrate was concentrated in vacuo. The residue was purified by Pre-HPLC (Column: Atlantis Prep T3 OBD Column, 19*250 mm 10u; Mobile Phase A: Water(10MMOL/L NH4HCO3), Mobile Phase B: ACN; Flow rate: 25 mL/min; Gradient: 5B to 15B in 7 min; 210/254 nm;). The fractions containing the product were collected and the solvent was evaporated to afford ***compound 119.***

**37% yield** (37 mg, 0.11mmol), white solid. **¹H NMR** (300 MHz, Methanol-d4) δ 8.29 (s, 1H), 8.21 (s, 1H), 6.16 (d, J = 6.7 Hz, 1H), 5.22 (dd, J = 6.7, 4.3 Hz, 1H), 4.25 (d, J = 4.3 Hz, 1H), 3.11 (dd, J = 12.6, 5.8 Hz, 1H), 2.74 (dt, J = 11.5, 7.2 Hz, 1H), 2.67 - 2.57 (m, 1H), 2.40 (dd, J = 12.6, 10.5 Hz, 1H), 1.65 - 1.49 (m, 3H), 1.15 (dd, J = 10.2, 6.1 Hz, 1H), 0.99 (t, J = 7.4 Hz, 3H), 0.86 ppm (t, J = 6.4 Hz, 1H). **¹³C NMR** (75 MHz, MeOD) δ 155.93, 152.53, 149.66, 140.04, 119.18, 87.92, 75.34, 73.95, 71.07, 50.25, 49.68, 21.70, 18.85, 16.17, 10.53 ppm. **LCMS(ESI+)**m/z:calcd. for C₁₅H₂₂N₆O₃[M+H]+ =335.18, found 335.25, RT: 0.498min, Method 1.

### Preparation of Compound 120

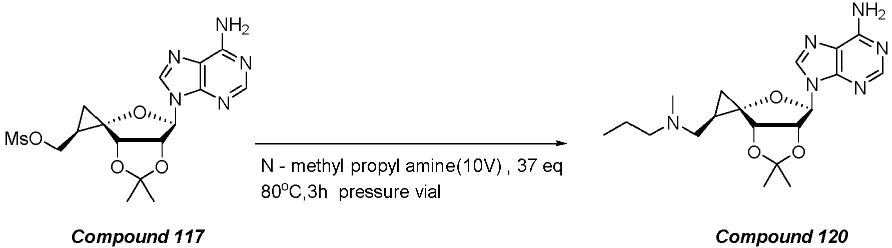

***Compound 117*** (200 mg crude) was dissolved in N - methyl propyl amine (2 ml) and the mixture was heated to 80°C for 3 h. Subsequently, the solution was cooled to room temperature. The mixture was extracted with CH₂Cl₂ (3x 10 ml) and combined organic layers were dried (Na₂SO₄), filtrated and the filtrate was concentrated in vacuo. The residue was applied onto a silica gel column with dichloromethane/ methanol (gradient elution: DCM/MeOH from 99:1 to 70:30). The fractions containing the product were collected and the solvent was evaporated to afford ***compound 120.***

**71% yield** (182 mg, 0.46mmol, 2 steps), white solid. **¹H NMR** (300 MHz, Methanol-d4) δ 8.21 (d, J = 2.1 Hz, 2H), 6.24 (s, 1H), 5.77 (d, J = 5.8 Hz, 1H), 5.18 (d, J = 5.8 Hz, 1H), 2.99 - 2.91 (m, 2H), 2.90 - 2.82 (m, 2H), 2.71 (s, 3H), 2.70 (s, 3H), 2.67 (s, 2H), 1.60 (s, 3H), 1.43 (s, 3H), 1.00 (d, J = 2.0 Hz, 2H), 0.65 ppm (dd, J = 9.9, 6.1 Hz, 1H). **LCMS(ESI+)**m/z:calcd. for C₁₉H₂₈N₆O₃[M+H]+ =389.22, found 389.15, RT: 1.558 min, Method 3.

### Preparation of Compound 121

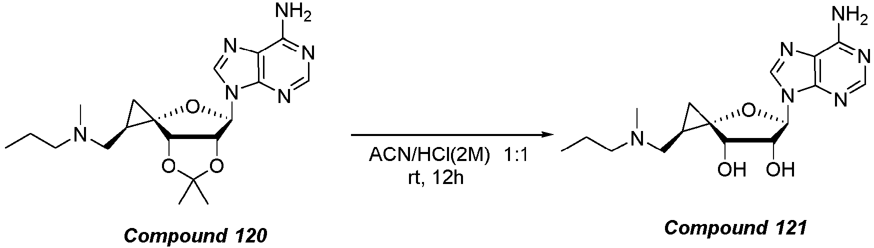

***Compound 120*** (110 mg, 0.28 mmol, 1.00eq) in ACN (1.0 ml), then was added 2M HCl (1.0ml), the mixture was heated to rt for 12h. Subsequently carefully quenched with NaHCO₃ to PH= 7. The filtrate was concentrated in vacuo. The residue was purified by Pre-HPLC (Column: Atlantis Prep T3 OBD Column, 19*250 mm 10u; Mobile Phase A: Water(10MMOL/L NH4HCO3), Mobile Phase B: ACN; Flow rate: 25 mL/min; Gradient: 5B to 35B in 7 min; 210/254 nm;). The fractions containing the product were collected and the solvent was evaporated to afford ***compound 121.***

**25% yield** (25 mg, 0.071mmol), white solid. **¹H NMR** (300 MHz, Methanol-d4) δ 8.30 (s, 1H), 8.21 (s, 1H), 6.20 (d, J = 7.2 Hz, 1H), 5.27 (dd, J = 7.2, 4.3 Hz, 1H), 4.13 (d, J = 4.3 Hz, 1H), 2.66 (dd, J = 13.0, 6.0 Hz, 1H), 2.57 - 2.47 (m, 1H), 2.37 (d, J = 7.8 Hz, 1H), 2.33 (s, 3H), 2.28 - 2.21 (m, 1H), 1.57 (dq, J = 10.5, 7.1 Hz, 3H), 1.16 (dd, J = 10.1, 6.0 Hz, 1H), 0.95 (t, J = 7.4 Hz, 3H), 0.89 ppm (d, J = 6.2 Hz, 1H). **¹³C NMR** (75 MHz, MeOD) δ 155.92, 152.51, 149.74, 140.16, 119.20, 87.98, 75.37, 74.05, 72.03, 58.81, 58.23, 39.89, 19.43, 17.84, 17.75, 10.75 ppm. **LCMS(ESI+)**m/z:calcd. for C₁₆H₂₄N₆O₃[M+H]+ =349.19, found 349.30, RT: 0.760min, Method 1.

### Preparation of Compound 122

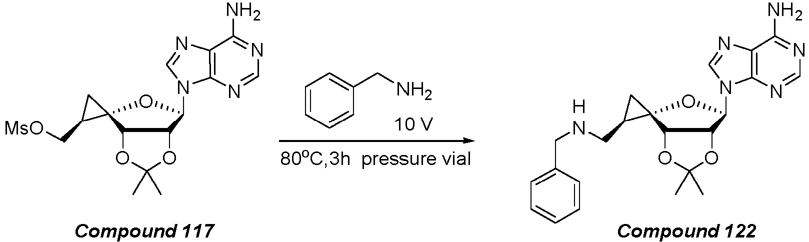

***Compound 117*** (200 mg crude) was dissolved in phenylmethanamine (2 ml) and the mixture was heated to 80°C for 3 h. Subsequently, the solution was cooled to room temperature. The mixture was extracted with CH₂Cl₂ (3x 10 ml) and combined organic layers were dried (Na₂SO₄), filtrated and the filtrate was concentrated in vacuo. The residue was applied onto a silica gel column with dichlormethane/ methanol (gradient elution: DCM/MeOH from 99:1 to 60:40). The fractions containing the product were collected and the solvent was evaporated to afford ***compound 122.***

**71% yield** (179 mg, 0.42mmol, 2 steps), white solid. **¹H NMR** (300 MHz, Methanol-d4) δ 8.20 (s, 1H), 8.16 (s, 1H), 7.32 (d, J = 2.3 Hz, 5H), 6.20 (s, 1H), 5.72 (d, J = 5.8 Hz, 1H), 5.06 (d, J = 5.8 Hz, 1H), 3.81 (s, 1H), 3.66 (d, J = 12.7 Hz, 1H), 2.82 (dd, J = 12.6, 5.8 Hz, 1H), 2.51 (dd, J = 12.6, 9.4 Hz, 1H), 1.62 (ddd, J = 9.9, 6.4, 3.5 Hz, 1H), 1.50 (s, 3H), 1.39 (s, 3H), 0.67 (t, J = 6.4 Hz, 1H), 0.40 ppm (dd, J = 10.3, 5.9 Hz, 1H). **¹³C NMR** (75 MHz, MeOD) δ 155.96, 152.60, 149.37, 140.53, 139.05, 128.13, 127.17, 118.65, 112.88, 88.95, 84.41, 83.93, 71.54, 53.08, 50.12, 45.06, 25.37, 23.86, 18.15, 17.62 ppm. **LCMS(ESI+)**m/z:calcd. for C₂₂H₂₆N₆O₃[M+H]+ =423.21, found423.15, RT: 1.476 min, Method 3.

### Preparation of Compound 123

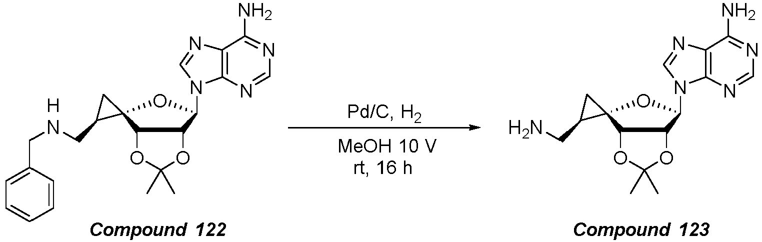

***Compound 122*** (160 mg, 0.37 mmol, 1.00 equiv) was dissolved in methanol and Pd/C (32 mg) was added. The reaction was stirred at rt 24 h Following completion, the solution was then filtered. The filter cake was washed with MeOH, and the filtrate was then concentrated under reduced pressure. The residue was applied onto a silica gel column with PE/EA (gradient elution: PE/EtOAc from 99:1 to 5:1). The fractions containing the product were collected and the solvent was evaporated to afford ***compound 123.***

**73% yield** (91 mg, 0.27 mmol), white solid. **¹H NMR** (300 MHz, Methanol-d4) δ 8.24 (dd, J = 4.7, 1.4 Hz, 2H), 6.27 (d, J = 1.6 Hz, 1H), 5.80 (dd, J = 5.9, 1.7 Hz, 1H), 5.26 (d, J = 5.5 Hz, 1H), 3.16 - 2.98 (m, 2H), 1.76 - 1.66 (m, 1H), 1.64 (s, 3H), 1.46 (s, 3H), 0.97 (dd, J = 8.0, 5.3 Hz, 1H), 0.66 - 0.57 ppm (m, 1H). **¹³C NMR** (75 MHz, MeOD) δ 155.98, 152.61, 149.31, 140.68, 118.71, 113.32, 89.07, 84.47, 83.86, 71.53, 40.84, 25.15, 23.71, 18.27, 16.57 ppm. **LCMS** (ESI+) m/z: calcd. For C₁₅H₂₀O₆ [M+NH4]⁺ = 333.16 found 333.25, RT: 0.687 min, Method 1.

### Preparation of Compound 124

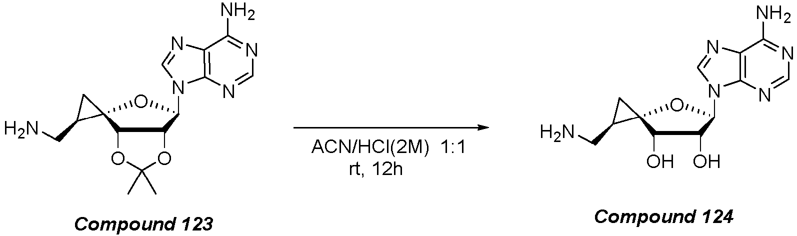

***Compound 123*** (90 mg, 0.27 mmol, 1.00eq) in ACN (1.0 ml) Then was added 2M HCl (1.0ml), the mixture was heated to 40°C for 3h. Subsequently, the solution was cooled to room temperature and carefully quenched with NaHCO3 to PH= 7. The filtrate was concentrated in vacuo. The residue was purified by Pre-HPLC (Column: Atlantis Prep T3 OBD Column, 19*250 mm 10u; Mobile Phase A: Water(10MMOL/L NH4HCO3), Mobile Phase B: ACN; Flow rate: 25 mL/min; Gradient: 2B to 20B in 7 min; 210/254 nm;). The fractions containing the product were collected and the solvent was evaporated to afford ***compound 124.***

**25% yield** (20 mg, 0.068 mmol), white solid. **¹H NMR** (300 MHz, Methanol-d4) δ 8.27 (s, 1H), 8.20 (s, 1H), 6.12 (d, J = 6.7 Hz, 1H), 5.22 (dd, J = 6.7, 4.4 Hz, 1H), 4.25 (d, J = 4.4 Hz, 1H), 3.10 (dd, J = 13.2, 6.0 Hz, 1H), 2.57 (dd, J = 13.2, 10.0 Hz, 1H), 1.49 (ddd, J = 10.1, 6.4, 3.7 Hz, 1H), 1.12 (dd, J = 10.2, 6.2 Hz, 1H), 0.83 ppm (t, J = 6.5 Hz, 1H). **¹³C NMR** (75 MHz, CDCl3) δ159.69, 156.53,153.33, 143.93, 123.14, 91.66, 78.96, 77.78, 74.24, 44.94, 23.29, 19.36 ppm. LCMS(ESI+) m/z:calcd. for C₁₂H₁₆N₆O₃[M +H]+ = 293.13, found 371.05, RT: 0.960min, Method 2.

### Preparation of Compound 125

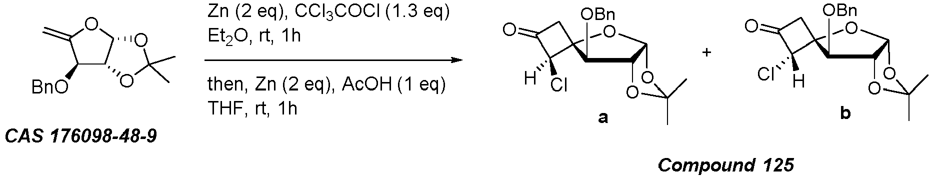

CAS 176098-48-9 (75.0 g, 286 mmol, 1.00 eq) was dissolved in Et₂O (750 ml) and zinc powder (37.0 g, 569 mmol, 2.00 eq) was added. This was followed by the addition of a solution of trichloroacetyl chloride (70.0 g, 389 mmol, 1.30 eq.) in Et₂O (200 ml) dropwise with stirring at 0°C. The resulting solution was stirred for 1 hour at room temperature, then zinc (92 g, 1.42 mol, 5.00 eq) was added in several portions, followed by AcOH (17.0 g, 283 mmol, 1.00 eq) which was added dropwise while the solution was cooled with an ice bath to control the internal temperature below 10°C. After addition, the mixture was stirred at room temperature for 10 minutes. The residue was added to an aqueous saturated solution of NaHCO₃ (1000 ml) at 0°C. The solids were removed via filtration and the aqueous phase was extracted with ethylacetate (1000 ml), then dried over anhydrous sodium sulfate and concentrated *in vacuo.* The residue was purified by column chromatography over silica gel (gradient elution: petroleum ether/EtOAc from 99:1 to 5:1). The fractions containing the product were collected and the solvent was removed *in vacuo* to afford ***compound 125*** as a mixture of isomers **a:b** in a 86:14 ratio, respectively.

**54% yield** (52.0 g, 153 mmol), colorless oil. ***Compound 125a:* ¹H NMR** (300 MHz, CDCl₃) δ 7.41 - 7.33 (m, 5H), 5.98 (d, *J=* 4.0 Hz, 1H), 5.13 - 5.03 (m, 1H), 4.82 - 4.75 (m, 2H), 4.62 (d, *J=* 11.5 Hz, 1H), 4.45 (s, 1H), 3.69 - 3.60 (m, 1H), 3.32 - 3.24 (m, 1H), 1.59 (s, 3H) 1.40 ppm (s, 3H). **¹³C NMR** (75 MHz, CDCl₃) δ 197.6, 136.7, 128.6, 128.2, 127.8, 113.2, 105.7, 84.2, 83.3, 81.9, 72.6, 69.1, 53.4, 26.7, 26.3 ppm.

### Preparation of Compound 126

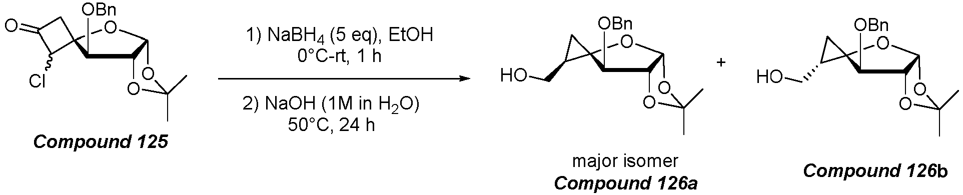

***Compound 125*** (2.2 g, 6.50 mmol, 1.00 eq) was dissolved in EtOH (22 ml), cooled to 0°C, and NaBH₄ (1.23 g, 32.3 mmol, 5.00 eq) was added at 0°C. The mixture was stirred at room temperature for 1 hour. Subsequently, NaOH (aq. 1M, 88 ml) was added and stirring was continued for 24 hours at 50°C. The product was extracted with EtOAc (3x50 ml) and combined organic layers were dried (MgSO₄), filtered and the filtrate was concentrated *in vacuo* to afford ***compound 126*** as a 82:18 mixture of isomers **a** and **b,** respectively. The residue was purified by Pre-HPLC (Column: WelFlash C18-I Column, 50*250 mm 20u; Mobile Phase A: Water (10 mmol/L NH₄HCO₃), Mobile Phase B: CH₃CN; Flow rate: 100 ml/min; Gradient: 40B to 70B in 30 min; collection at 210/254 nm). The fractions containing the product were collected and the solvent was removed *in vacuo* to afford ***compound 126a*** and ***compound 126b.***

**30% yield** (600 mg, 1.96 mmol), colorless oil.

***Compound 126a:* ¹H NMR** (400 MHz, CDCl₃) δ=7.41 - 7.35 (m, 5H), 5.97 (d, *J=* 3.6 Hz, 1H), 4.78 (d, *J=* 3.7 Hz, 1H), 4.74 (d, *J=* 12.1 Hz, 1H), 4.63 (d, *J=* 12.1 Hz, 1H), 3.90 - 3.83 (m, 1H), 3.82 (s, 1H), 2.83 (dd, *J=* 12.3, 10.5 Hz, 1H), 1.63 (s, 3H), 1.39 (s, 3H), 1.30 - 1.21 (m, 2H), 0.65 (t, *J=* 6.8 Hz, 1H). **¹³C NMR** (75 MHz, CDCl₃) δ=137.29, 128.59, 128.14, 127.82, 112.49, 104.16, 83.14, 80.97, 71.55, 69.70, 63.28, 26.16, 25.95, 25.53, 8.82 ppm. **LC-MS** (ESI⁺) m/z: [M+NH₄]⁺ Calcd. for C₁₇H₂₂O₅ 324.15, found 324.25 (Method 1), RT: 1.22 min.

***Compound 126b:* 4% yield** (80 mg, 0.26 mmol), colorless oil. **¹H NMR** (DMSO-d6, 300 MHz) δ=5.91 (d, *J=* 4.3 Hz, 1H), 4.78 (d, *J=* 4.1 Hz, 1H), 4.61-4.69 (m, 1H), 4.44-4.54 (m, 1H), 4.22 (br s, 1H), 3.60-3.68 (m, 1H), 3.10-3.24 (m, 1H), 1.43 (s, 3H), 1.27 (s, 3H), 1.14-1.24 (m, 1H), 0.89-0.98 (m, 1H), 0.63-0.71 ppm (m, 1H). **¹³C NMR** (DMSO-d6, 75 MHz): δ=138.6, 128.7, 128.1, 128.0, 111.0, 105.1, 86.5, 83.0, 70.7, 70.7, 60.6, 26.6, 26.2, 25.3, 9.4 ppm.

### Preparation of Compound 126 (larger scale)

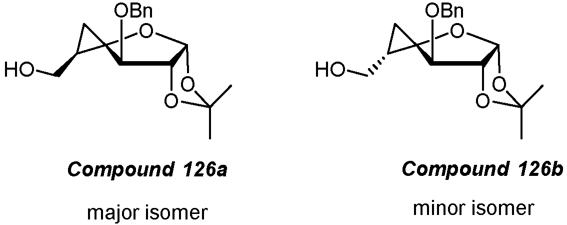

***Compound 125*** (52.0 g, 153 mmol, 1.00 eq) was dissolved in EtOH (520 ml), cooled to 0°C and NaBH₄ (29.2 g, 768 mmol, 5.00 eq) was added at 0°C. The mixture was stirred at room temperature for 1 hour. Subsequently, NaOH (aq. 1M, 2.08 l) was added and stirring was continued for 24 hours at 50°C. The product was extracted with EtOAc (3x 2 l) and combined organic phases were dried (MgSO₄), filtered and the filtrate was concentrated *in vacuo.* The residue was purified by Pre-HPLC (Column: WelFlash C18-I Column, 50*250 mm 20u; Mobile Phase A: Water (10 mmol/l NH₄HCO₃), Mobile Phase B: ACN; Flow rate: 100 mL/min; Gradient: 40B to 70B in 30 min; 210/254 nm). The fractions containing the product were collected and the solvent was evaporated to afford ***compound 126a* (42% yield,** 20 g, 65.4 mmol) and ***compound 126b* (8.5% yield,** 4.0 g, 12.9 mmol) as colorless oils.

### Preparation of Compound 127a

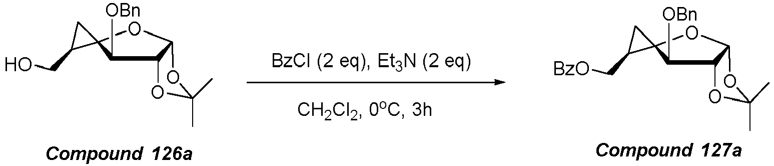

***Compound 126a*** (20.0 g, 65.4 mmol, 1.00 eq) and triethylamine (13.2 g, 131 mmol, 2.00 eq) was dissolved in CH₂Cl₂ (200 ml), followed by the dropwise addition of benzoyl chloride (18.3 mg, 131 mmol, 2.00 eq) at 0°C. The resulting solution was stirred for 3 hours at 0°C. To the reaction was then added 1M aqueous HCl until a pH <7 was obtained. To the resulting solution was added CH₂Cl₂ (300 ml) and water (300 ml), and the product was extracted in CH₂Cl₂ (3x 300 ml). Combined organic layers were dried Na₂SO₄), filtered and the filtrate was concentrated *in vacuo.* The residue was purified via silica gel column chromatography (gradient elution: petroleum ether/EtOAc from 99:1 to 90:10). Fractions containing the product were collected and the solvent was removed *in vacuo* to afford ***compound 127a.* 76% yield** (20.5 g, 50.0 mmol), colorless oil. **¹H NMR** (300 MHz, CDCl3) δ=8.08 - 8.02 (m, 2H), 7.59 - 7.53 (m, 1H), 7.45 (dd, *J=* 8.3, 6.9 Hz, 2H), 7.35 (d, *J=* 17.0 Hz, 5H), 5.94 (d, *J=* 3.9 Hz, 1H), 4.78 - 4.70 (m, 2H), 4.60 (d, *J=* 11.8 Hz, 1H), 4.25 (dd, *J=* 11.9, 6.9 Hz, 1H), 4.11 (dd, *J=* 11.9, 7.9 Hz, 1H), 3.96 (s, 1H), 1.78 - 1.69 (m, 1H), 1.48 (s, 3H), 1.34 (s, 3H), 1.28 - 1.23 (m, 1H), 0.88 ppm (t, *J=* 6.9 Hz, 1H). **¹³C NMR** (75 MHz, CDCl₃) δ=166.5, 137.4, 132.9, 130.2, 129.7, 128.5, 128.3, 128.0, 127.9, 112.6, 104.5, 84.2, 81.7, 71.6, 69.5, 64.8, 26.9, 26.6, 22.0, 10.4 ppm. **LC-MS** (ESI+) m/z: [M+NH₄]⁺ Calcd. for C₂₄H₂₆O₆ 428.17, found 428.20 (Method 1), RT: 1.57 min.

### Preparation of Compound 128a

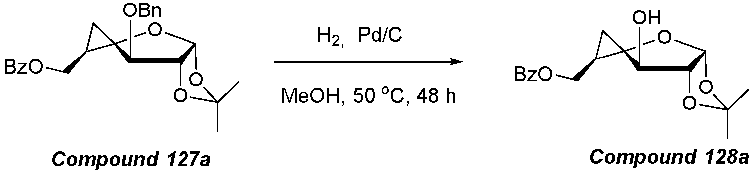

***Compound 127a*** (20.5 g, 50.0 mmol, 1.00 eq) was dissolved in methanol and Pd/C (10 wt%, 4.10 g) was added. The reaction was stirred at room temperature for 24 hours. Following completion, the solution was filtered, the filter cake was washed with MeOH and the filtrate was concentrated *in vacuo.* The residue was purified via silica gel column chromatography (gradient elution: petroleum ether/EtOAc from 99:1 to 70:30). The fractions containing the product were collected and the solvent was evaporated to afford ***compound 128a.***

**73% yield** (11.8 g, 36.9 mmol), white solid. **¹H NMR** (300 MHz, CDCl₃) δ 8.09 - 8.04 (m, 2H), 7.60 - 7.54 (m, 1H), 7.45 (dd, *J=* 7.5, 6.2 Hz, 2H), 5.95 (d, *J=* 3.7 Hz, 1H), 4.69 (d, *J=* 3.7 Hz, 1H), 4.48 (dd, *J=* 11.9, 6.2 Hz, 1H), 4.21 - 4.11 (m, 2H), 1.85 - 1.78 (m, 1H), 1.49 (s, 3H), 1.32 (s, 3H), 1.27 - 1.22 (m, 1H), 0.87 ppm (d, *J=* 6.9 Hz, 1H). **¹³C NMR** (75 MHz, CDCl₃) δ 166.7, 133.0, 130.2, 129.7, 128.3, 112.5, 104.5, 86.0, 75.3, 71.6, 64.7, 26.7, 26.4, 23.5, 9.31 ppm. **LC-MS** (ESI⁺) m/z: [M+NH₄]⁺ Calcd. for C₁₇H₂₀O₆ 338.13, found 338.25 (Method 1), RT: 1.26 min.

### Preparation of Compound 129a

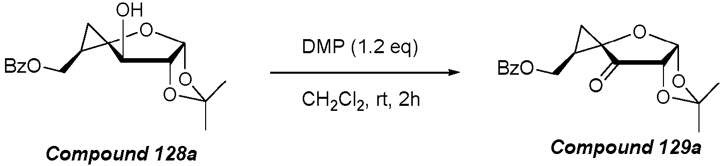

***Compound 128a*** (11.8 g, 36.9 mmol, 1.00 eq) was dissolved in CH₂Cl₂ (120 ml), followed by the addition of Dess-Martin periodinane (18.8 g, 44.3 mmol, 1.20 eq) at 0 °C. The mixture was stirred at room temperature for 2 hours. The reaction was quenched by the addition of a saturated aqueous solution of Na₂S₂O₃ (100 ml) and a saturated aqueous solution of NaHCO₃ (100 ml) was added. The product was extracted with EtOAc (3 × 200 ml) and combined organic layers were dried (Na₂SO₄), filtered and the filtrate was concentrated *in vacuo.* The residue was purified via silica gel column chromatography (gradient elution: petroleum ether/EtOAc from 99:1 to 80:20). The fractions containing the product were collected and the solvent was evaporated to afford ***compound 129a.***

**88% yield** (10.2 g, 31.9 mmol), white solid. **¹H NMR** (300 MHz, CDCl₃) δ 8.03 (dt, *J=* 7.1, 1.4 Hz, 2H), 7.60 - 7.53 (m, 1H), 7.43 (dd, *J=* 8.4, 7.0 Hz, 2H), 6.07 (d, *J=* 4.5 Hz, 1H), 4.62 - 4.52 (m, 2H), 4.35 (dd, *J=* 12.0, 8.3 Hz, 1H), 2.31 (dtd, *J=* 9.9, 8.3, 6.7 Hz, 1H), 1.63 (dd, *J=* 10.0, 6.0 Hz, 1H), 1.45 (s, 3H), 1.42 (s, 3H), 1.39 - 1.34 ppm (m, 1H). **¹³C NMR** (75 MHz, CDCl₃) δ 207.8, 166.4, 133.0, 130.0, 129.7, 128.2, 114.6, 102.2, 77.8, 67.1, 61.0, 29.7, 27.7, 27.1, 20.0 ppm. **LC-MS** (ESI⁺) m/z: [M+NH₄]⁺ Calcd. for C₁₇H₁₈O₆ 336.11, found 336.20 (Method 1), RT: 1.49 min.

### Preparation of Compound 130a

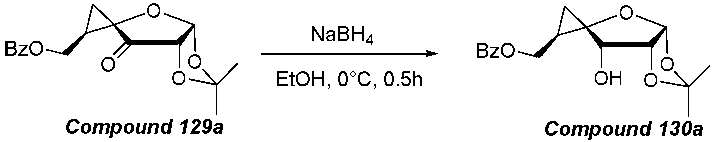

***Compound 129a*** (10.2 g, 31.9 mmol, 1.00 eq) was dissolved in EtOH (87 ml), then NaBH₄ (2.40 g, 63.2 mmol, 2.00 eq) was added at 0°C. The mixture was stirred at 0°C for 30 minutes, then NH₄Cl (sat. aq. 100 mL) was added at 0°C. The product was extracted with EtOAc (3 × 100 ml) and combined organic layers were dried (Na₂SO₄), filtered and the filtrate was concentrated *in vacuo.* The residue was purified via silica gel column chromatography (gradient elution: PE/EtOAc from 99:1 to 70:30). Fractions containing the product were collected and the solvent was evaporated to afford ***compound 130a.***

**80% yield** (8.70 g, 27.2 mmol), white solid. **¹H NMR** (400 MHz, CDCl₃) δ 8.11 - 8.04 (m, 2H), 7.60 - 7.54 (m, 1H), 7.45 (dd, *J=* 8.4, 7.1 Hz, 2H), 5.73 (d, *J=* 4.0 Hz, 1H), 4.72 (dd, *J=* 5.1, 4.0 Hz, 1H), 4.57 (qd, *J=* 11.8, 7.7 Hz, 2H), 4.44 (dd, *J=* 10.0, 5.1 Hz, 1H), 3.08 (d, *J*= 10.1 Hz, 1H), 1.77 - 1.70 (m, 1H), 1.47 (s, 3H), 1.35 (s, 3H), 1.17 (m, 1H), 0.74 (t, *J=* 6.8 Hz, 1H). **¹³C NMR** (101 MHz, CDCl₃) δ 166.8, 133.0, 130.3, 129.8, 128.4, 114.0, 103.1, 80.2, 72.9, 67.6, 64.8, 26.8, 26.8, 22.0, 11.4 ppm. **LC-MS** (ESI⁺) m/z: [M+NH₄]⁺ Calcd. for C₁₇H₂₀O₆ 338.13, found 338.20 (Method 1), RT: 1.34 min.

### Preparation of Compound 131a

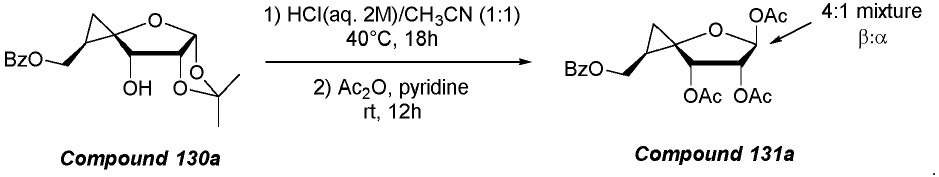

***Compound 130a*** (8.70 g, 27.2 mmol, 1.00 eq) was dissolved in CH₃CN (80 ml), and HCl (aq. 2M, 80 ml) was added. The mixture was heated to 40°C for 18 hours and the solution was cooled to room temperature and carefully quenched with NaHCO₃ (aq. sat.) until a pH of 7 was obtained. The mixture was filtered, and the filtrate was concentrated *in vacuo.* The residue was dissolved in anhydrous pyridine (80 ml) and stirred for 30 minutes at room temperature, then acetic anhydride (3.00 g, 29.4 mmol, 1.20 eq) was added dropwise to the stirring solution at room temperature. The mixture was stirred at room temperature for 12 hours. Subsequently, the mixture was poured into ice-cold water (500 ml) and stirred for 30 minutes at room temperature. The product was extracted with CH₂Cl₂ (3x 200 ml) and the combined organic layers were washed with brine (3 × 200 ml), dried (Na₂SO₄), concentrated *in vacuo* and the residue was purified by silica chromatography (gradient elution: petroleum ether/EtOAc from 100:1 to 10:1). Fractions containing the product were combined and the solvent was removed *in vacuo* to afford ***compound 131a.***

**71% yield** (2 steps from ***compound 130a,*** 7.80 g, 19.2 mmol), white solid. **¹H NMR** (300 MHz, CDCl₃) δ 8.07 - 8.02 (m, 2H), 7.61 - 7.55 (m, 1H), 7.46 (dd, *J=* 8.2, 6.8 Hz, 2H), 6.25 (d, *J=* 3.8 Hz, 1H), 5.65 (dd, *J=* 5.1, 3.8 Hz, 1H), 5.43 (dd, *J=* 7.5, 5.1 Hz, 1H), 4.27 (dd, *J=* 7.1, 2.1 Hz, 2H), 2.12 (s, 3H), 2.06 (s, 3H), 2.00 (s, 3H), 1.30 - 1.24 (m, 2H), 0.94 - 0.88 ppm (m, 1H). **¹³C NMR** (75 MHz, CDCl₃) δ = 169.9, 169.8, 169.2, 166.2, 133.1, 129.8, 129.6, 128.4, 98.5, 76.2, 74.3, 68.8, 63.5, 21.0, 20.4, 20.3, 18.1, 16.1 ppm. **LC-MS** (ESI⁺) m/z [M+H]⁺ Calcd. for C₂₀H₂₂O₉ 424.13, found 424.25 (Method 1), RT: 1.49 min.

### Preparation of Compound 132a

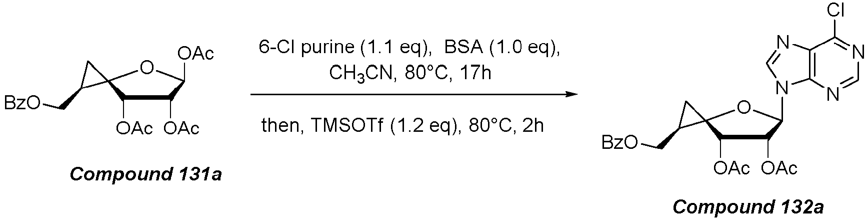

6-Chloropurine (3.25 g, 21.1 mmol, 1.10 eq) was dissolved in anhydrous CH₃CN (1.6 ml), and N,O-bis(trimethylsilyl)acetamide (3.90 g, 19.2 mmol, 1.00 eq) was added. The mixture was heated to 80°C for 17 hours. After the mixture was cooled, ***compound 131a*** (7.80 g, 19.2 mmol, 1.00 eq) dissolved in anhydrous CH₃CN (1.4 mL) was added, followed by trimethylsilyltrifluoromethane sulfonate (5.11 g, 23.0 mmol, 1.20 eq). The mixture was heated to 80°C for 2 hours. Upon cooling to room temperature, the mixture was diluted in EtOAc, washed with NaHCO₃ (aq. sat. 3x) and brine. The organic layer was dried (Na₂SO₄), filtered and the filtrate was concentrated *in vacuo.* The residue was purified via silica gel column chromatography (gradient elution: petroleum ether/EtOAc from 99:1 to 4:1). The fractions containing the product were collected and the solvent was evaporated to afford ***compound 132a.***

**82% yield** (7.90 g, 15.8 mmol), white solid. **¹H NMR** (400 MHz, CDCl₃) δ 8.82 (s, 1H), 8.24 (s, 1H), 8.10 - 8.05 (m, 2H), 7.64 - 7.59 (m, 1H), 7.50 (dd, *J=* 8.3, 7.1 Hz, 2H), 6.61 (dd, *J=* 7.0, 4.5 Hz, 1H), 6.32 (d, *J=* 7.0 Hz, 1H), 5.68 (d, *J=* 4.6 Hz, 1H), 4.38 - 4.29 (m, 2H), 2.12 (s, 3H), 2.03 (s, 3H), 2.00 - 1.95 (m, 1H), 1.35 (dd, *J=* 10.6, 6.7 Hz, 1H), 1.15 (t, *J=* 7.0 Hz, 1H) ppm. **¹³C NMR** (75 MHz, CDCl₃) δ 169.6, 169.1, 166.2, 152.3, 151.7, 151.5, 144.0, 133.3, 132.5, 129.7, 129.6, 128.5, 86.0, 74.5, 73.7, 69.5, 63.2, 20.5, 20.2, 19.3, 16.6 ppm. **LC-MS** (ESI⁺) m/z: [M+H]⁺ Calcd. for C₂₃H₂₁ClN₄O₇ 501.11, found 501.20 (Method 1), RT: 1.50 min.

### Preparation of Compound 133a

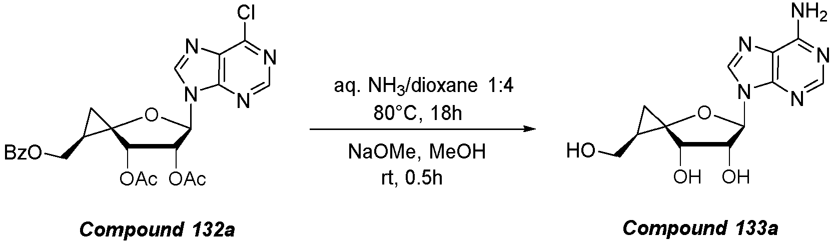

***Compound 132a*** (7.90 g, 15.8 mmol, 1.00 eq) was dissolved in 1,4-dioxane (80 ml) and ammonia (aq. 25%, 20 ml) was added. The mixture was heated to 80°C for 18 hours. Upon cooling to room temperature, the solvents were removed *in vacuo.* The residue was dissolved in MeOH (80 ml) and sodium methoxide (0.85 g, 15.7 mmol, 1.00 eq) was added. The reaction was stirred at room temperature for 30 minutes, then stirred with hydrogen exchange resin to pH 7. The solution was filtered, and the filtrate was concentrated *in vacuo.* The residue was purified via silica gel column chromatography (gradient elution: CH₂Cl₂/MeOH from 99:1 to 5:1). The fractions containing the product were collected and the solvent was evaporated to afford ***compound 133a.***

**69% yield** (3.2 g, 10.90 mmol), white solid. **¹H NMR** (400 MHz, Methanol-d₄) δ 8.29 (s, 1H), 8.20 (s, 1H), 6.13 (d, *J=* 7.2 Hz, 1H), 5.27 (dd, *J=* 7.1, 4.4 Hz, 1H), 4.18 (d, *J=* 4.3 Hz, 1H), 3.78 (dd, *J=* 11.9, 6.9 Hz, 1H), 3.48 (dd, *J=* 11.9, 8.7 Hz, 1H), 1.57 (ddt, *J=* 10.2, 8.8, 6.8 Hz, 1H), 1.08 (dd, *J=* 10.1, 6.1 Hz, 1H), 0.85 ppm (t, *J=* 6.4 Hz, 1H). **¹³C NMR** (101 MHz, Methanol-d₄) δ 155.9, 152.5, 149.7, 140.1, 119.2, 87.5, 75.3, 74.4, 71.2, 62.4, 21.9, 15.5 ppm. **LC-MS** (ESI⁺) m/z: [M+H]⁺ Calcd. for C₁₂H₁₅N₅O₄ 294.11, found 294.05 (Method 3), RT: 0.64 min.

***Compound 133a*** corresponds to ***compound 92b.*** Therefore, this represents an alternative preparation of ***compound 92b.***

### Preparation of Compound 127b

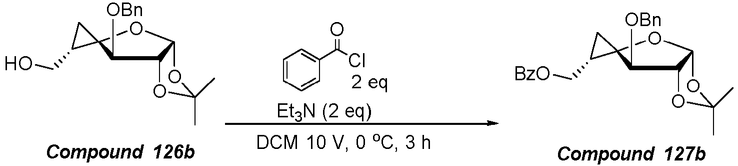

***Compound 126b*** (4.0 g, 13.07 mmol, 1.00 eq) and triethylamine (2.6 g, 26.14 mmol, 2.00 equiv) was dissolved in DCM (40 mL), then benzoyl chloride (3.7 g, 26.14 mmol, 2.00 equiv) was added dropwise at 0 °C, the resulting solution was stirred for 3 h at 0°C. To the reaction was then added 1M HCl aqueous until pH<7. To the resulting solution was added 50 mL DCM and extracted with 1x50 mL of H₂O and the organic layers were combined. The mixture was dried over anhydrous sodium sulfate and concentrated *in vacuo.* The residue was applied onto a silica gel column with ethyl acetate/petroleum ether (gradient elution: PE/EtOAc from 99:1 to 90:10). The fractions containing the product were collected and the solvent was evaporated to afford ***compound 127b.***

**73% yield** (3.9 g, 9.5 mmol), colorless oil **¹H NMR** (400 MHz, Chloroform-d) δ 8.10 - 8.06 (m, 2H), 7.60 - 7.55 (m, 1H), 7.46 (dd, J = 8.4, 7.1 Hz, 2H), 7.39 - 7.32 (m, 5H), 6.02 (d, J = 4.3 Hz, 1H), 4.80 - 4.72 (m, 3H), 4.54 (d, J = 12.1 Hz, 1H), 4.08 (dd, J = 12.0, 9.2 Hz, 1H), 3.64 (s, 1H), 1.57 (s, 3H), 1.52 (m, 1H), 1.37 (s, 3H), 1.15 - 1.04 ppm (m, 2H). **¹³C NMR** (101 MHz, CDCl₃) δ 166.85, 137.47, 132.81, 130.53, 129.62, 128.55, 128.29, 127.99, 127.74, 111.74, 105.28, 86.15, 83.51, 71.27, 70.12, 64.40, 26.67, 26.08, 21.43, 9.52 ppm. **LCMS(ESI+)** m/z:calcd. For C₂₄H₂₆O₆[M+NH4]+ =428.17 found 428.20, RT:1.890min, Method 1.

### Preparation of Compound 128b

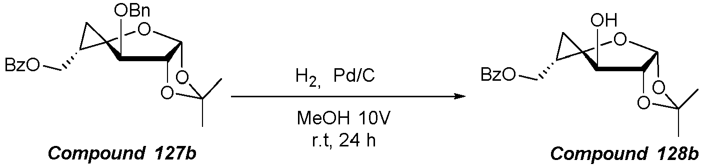

***Compound 127b*** (3.8 g, 9.26 mmol, 1.00 equiv) was dissolved in methanol (38 ml) and Pd/C (0.8 g) was added. The reaction was stirred at room temperature for 24 h. Following completion, the solution was then filtered. The filter cake was washed with MeOH, and the filtrate was then concentrated under reduced pressure. The residue was applied onto a silica gel column with PE/EA (gradient elution: PE/EtOAc from 99:1 to 70:30). The fractions containing the product were collected and the solvent was evaporated to afford ***compound 128b.***

**84% yield** (2.5 g, 7.81 mmol), white solid. **¹H NMR** (300 MHz, Chloroform-d) δ 8.08 - 8.03 (m, 2H), 7.59 - 7.53 (m, 1H), 7.47 - 7.41 (m, 2H), 5.99 (d, J = 4.3 Hz, 1H), 4.82 - 4.63 (m, 2H), 4.12 - 4.01 (m, 1H), 3.82 (d, J = 7.1 Hz, 1H), 1.65 (m, 1H), 1.54 (s, 3H) 1.33 (s, 3H), 1.13 - 0.92 ppm (m, 2H). **¹³C NMR** (75 MHz, CDCl₃) δ 166.88, 132.85, 129.60, 128.30, 111.63, 105.25, 85.74, 79.82, 79.10, 72.02, 64.22, 26.51, 25.86, 21.95, 8.53 ppm. **LCMS** (ESI+) m/z: calcd. For C₁₇H₂₀O₆ [M+NH4]⁺ = 338.13 found 338.25, RT: 1.299 min, Method 1.

### Preparation of Compound 129b

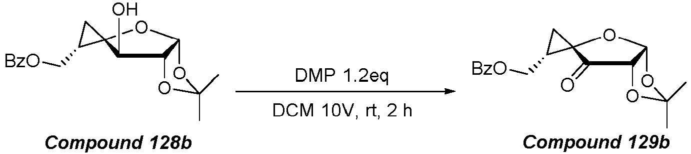

***Compound 128b*** (2.4 g, 7.50 mmol, 1 eq) was dissolved in DCM (24 mL), then Dess-Martin Periodinane (DMP) (3.8 g, 9.00 mmol, 1.2 eq) was added at 0°C, The mixture was stirred at room temperature for 2 h. The reaction was quenched by the addition of 40 mL of saturated aqueous solution of Na₂S₂O₃ and 40 mL of saturated aqueous solution of NaHCO₃. then extracted with EtOAc (3 x 50 mL). The organic phase was dried with Na₂SO₄. The residue was applied onto a silica gel column with PE /EA (gradient elution: PE/EtOAc from 99:1 to 80:20). The fractions containing the product were collected and the solvent was evaporated to afford ***compound 129b.***

**50% yield** (1.2 g, 3.77 mmol), white solid. **¹H NMR** (300 MHz, Chloroform-d) δ 8.08 - 8.02 (m, 2H), 7.61 - 7.55 (m, 1H), 7.48 - 7.41 (m, 2H), 6.14 (d, J = 4.7 Hz, 1H), 4.62 (d, J = 4.7 Hz, 1H), 4.53 (dd, J = 12.0, 6.5 Hz, 1H), 4.48 - 4.40 (m, 1H), 2.11 (m, 1H), 1.55 (dd, J = 10.1, 6.0 Hz, 1H), 1.42 (s, 6H), 1.34 ppm (dd, J = 7.8, 6.0 Hz, **1H).¹³C NMR** (75 MHz, CDCl₃) δ 209.44, 166.40, 133.02, 130.07, 129.70, 128.30, 114.11, 102.28, 77.20, 68.06, 62.34, 27.62, 27.39, 27.24, 18.66 ppm. **LCMS (ESI+)** m/z: calcd, for C₁₇H₁₈O₆ [M+NH4]+ = 336.11, found 336.20, RT: 1.493 min, Method 1.

### Preparation of Compound 130b

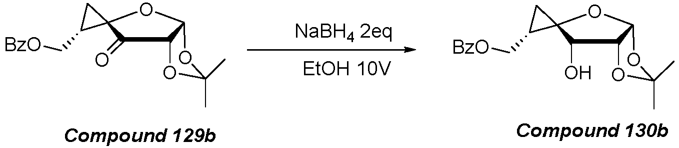

***Compound 129b*** (1.0 g, 3.14 mmol, 1 eq) was dissolved in EtOH (10 mL), then NaBH₄ (0.24 g, 6.28 mmol, 2 eq) was added at 0 °C, The mixture was stirred at 0°C for 30 min. The mixture was added to a saturated NH₄Cl aqueous solution (10 mL) at 0°C, and then extracted with EtOAc (3 x 10 mL). The organic phase was dried with Na₂SO₄, the solvent was evaporated to afford the 510 mg crude ***compound 130b.*** The residue was used as such in subsequent steps.

### Preparation of Compound 131.1b

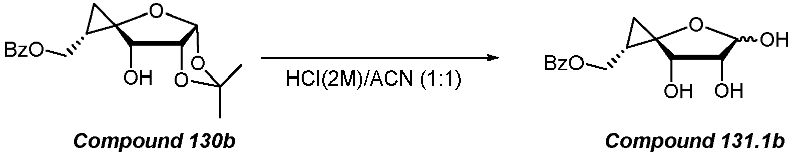

***Compound 130b*** (450 mg, 1.40 mmol, 1.00 equiv) was dissolved in acetonitrile (4.5 mL), and aqueous HCl (2M, 4.5 mL) was added. The mixture was heated to 40°C for 16 h. Subsequently, the solution was cooled to room temperature and carefully quenched with NaHCO₃ to pH= 7, then filtrated and the filtrate was concentrated *in vacuo.* This resulted in 373 mg of crude ***compound 131.1b,*** and the residue was used as such in subsequent steps.

### Preparation of Compound 131b

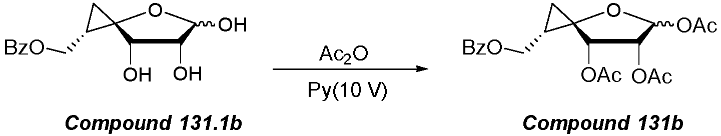

***Compound 131.1b*** (373 mg, crude) was dissolved in dry pyridine (4 mL) and stirred for 30 minutes. Acetic anhydride (162 mg, 1.59 mmol, 1.2 eq), which was cooled on ice before use, was added to the stirring solution at room temperature. The mixture was stirred at r.t for 12 hours. Subsequently, the mixture was poured into ice-cold water (5 ml) and stirred for 30 min at room temperature. The crude mixture was extracted with CH₂Cl₂ (3 x 5 ml) and the combined organic layers were washed with brine (3 x 5 ml), dried over Na₂SO₄ and concentrated and purified by silica chromatography (gradient elution: PE / EA from 100:1 to 10:1). This result in 280 mg of ***compound 131b.***

**22% yield** (3 steps from ***compound 129b,*** 280 mg, 0.69 mmol), white solid. **¹H NMR** (300 MHz, Chloroform-d) δ 8.10 - 8.06 (m, 2H), 7.58 - 7.54 (m, 1H), 7.48 - 7.42 (m, 2H), 6.27 (d, J = 2.0 Hz, 1H), 5.45 (dd, J = 5.2, 2.0 Hz, 1H), 5.40 (d, J = 5.2 Hz, 1H), 4.58 (dd, J = 11.6, 7.0 Hz, 1H), 4.33 - 4.26 (m, 1H), 2.12 (s, 3H), 2.03 (s, 3H), 1.97 (s, 3H), 1.69 - 1.64 (m, 1H), 1.10 - 1.01 ppm (m, 2H). **¹³C NMR** (75 MHz, CDCl₃) δ 169.82, 169.47, 169.29, 166.48, 132.91, 130.41, 129.62, 128.31, 98.15, 75.24, 72.26, 68.31, 63.32, 21.01, 20.42, 20.22, 16.48, 13.95 ppm. **LCMS (ESI+)** m/z: calcd. for C₂₀H₂₂O₉ [M+H]+ = 424.13, found 424.25, RT: 1.502 min, Method 1.

### Preparation of Compound 132b

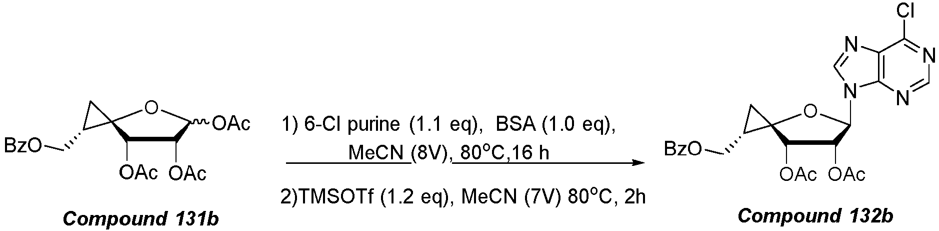

6-Cl Purine (103.7 g, 0.67 mmol, 1.10 eq.) was dissolved in MeCN (2 mL), and N,O-bis(trimethylsilyl)acetamide (123.8 mg, 0.61 mmol, 1.00 equiv) was added dropwise. The mixture was heated to 80°C for 16 h. After the mixture had cooled to room temperature, ***compound 131b*** (250 mg, 0.61 mmol, 1.00 equiv) in MeCN (2 mL) was added, Trimethylsilyltrifluoromethanesulfonate (162.5 mg, 0.73 mmol, 1.20 equiv) was added, and the mixture was heated to 80°C for 2 h. Upon cooling to rt, the mixture was dilute to EtOAc, washed with saturated NaHCO₃ and saturated aq. NaCl. The organic layer was dried over Na₂SO₄. Solvents were removed *in vacuo.* The residue was applied onto a silica gel column with PE/EA (gradient elution: PE/EA from 99:1 to 4:1). The fractions containing the product were collected and the solvent was evaporated to afford ***Compound 132b.***

**62% yield** (210 mg, 0.42 mmol), white solid. **¹H NMR** (300 MHz, Chloroform-d) δ 8.79 (s, 1H), 8.23 (s, 1H), 8.12 - 8.08 (m, 2H), 7.63 - 7.57 (m, 1H), 7.47 (dd, J = 8.3, 7.1 Hz, 2H), 6.25 (d, J = 4.0 Hz, 2H), 5.64 (d, J = 4.2 Hz, 1H), 4.66 (dd, J = 11.6, 6.2 Hz, 1H), 4.20 (dd, J = 11.6, 9.0 Hz, 1H), 2.10 (s, 3H), 1.93 (s, 3H), 1.85 - 1.77 (m, 1H), 1.32 - 1.26 (m, 1H), 1.09 ppm (t, J = 7.1 Hz, 1H). **¹³C NMR** (75 MHz, CDCl₃) δ 169.70, 169.33, 166.30, 152.35, 151.66, 151.41, 143.96, 133.13, 132.43, 130.21, 129.58, 128.44, 86.64, 73.65, 72.83, 69.23, 63.01, 20.46, 20.11, 17.51, 14.52 ppm. **LCMS (ESI+)** m/z: calcd. for C₂₃H₂₁ClN₄O₇ [M+H]⁺ = 501.11, found 501.20, RT:1.508 min, Method 1.

### Preparation of Compound 133b

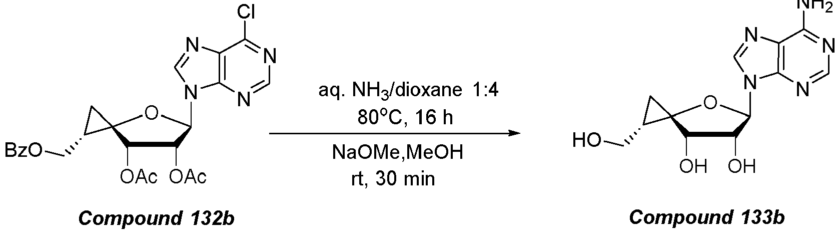

***Compound 132b*** (180 mg, 0.36 mmol, 1.00 equiv) was dissolved in 1,4-dioxane (2 mL), and aq.NH₃ (0.5 mL) was added. The mixture was heated to 80°C for 16 h. Upon cooling to r.t, the solvent was removed *in vacuo.* The product was dissolved in MeOH (2 ml), and sodium methoxide (20 mg, 0.36 mmol, 1.00 equiv) was added. The reaction was stirred at RT for 30 min, then stirred with Hydrogen exchange resin to pH=7, then filtered, and the filtrate was concentrated *in vacuo.* The residue was applied onto a silica gel column with dichlormethane/ methanol (gradient elution: DCM/MeOH from 99:1 to 5:1). The fractions containing the product were collected and the solvent was evaporated to afford ***compound 133b.***

**36% yield** (40 mg, 0.13 mmol), white solid. **¹H NMR** (300 MHz,DMSO-d6) δ 8.34 (s, 1H), 8.15 (s, 1H), 7.27 (s, 2H), 5.91 (d, J = 6.1 Hz, 1H), 4.99 - 4.94 (m, 1H), 3.98 (d, J = 5.1 Hz, 1H), 3.52 - 3.46 (m, 1H), 3.33 (dd, J = 11.3, 7.4 Hz, 1H), 1.36 (dd, J = 9.7, 6.9 Hz, 1H), 0.88 (dd, J = 9.7, 5.8 Hz, 1H), 0.62 ppm (t, J = 6.4 Hz, 1H). **¹³C NMR** (75 MHz, DMSO-d6) δ 156.53, 153.16, 150.10, 140.38, 119.63, 87.75, 74.15, 73.38, 70.98, 59.99, 20.17, 14.47 ppm. **LCMS (ESI+)** m/z: calcd. for C₁₂H₁₅N₅O₄ [M+H]+ =294.11 found 294.05, RT: 0.631 min, Method 3.

***Compound 133b*** corresponds to ***compound 92a.*** Therefore, this represents an alternative preparation of ***compound 92a.***

### Analytical Methods

### LCMS (Liquid chromatography/Mass spectrometry)

The High Performance Liquid Chromatography (HPLC) measurement was performed using a LC pump, a diode-array (DAD) or a UV detector and a column as specified in the respective methods. If necessary, additional detectors were included (see table of methods below).

Flow from the column was brought to the Mass Spectrometer (MS) which was configured with an atmospheric pressure ion source. It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software.

Compounds are described by their experimental retention times (Rt) and ions. If not specified differently in the table of data, the reported molecular ion corresponds to the [M+H]⁺ (protonated molecule) and/or [M-H]⁻ (deprotonated molecule). In case the compound was not directly ionizable the type of adduct is specified (i.e. [M+NH₄]⁺, [M+HCOO]⁻, etc...). For molecules with multiple isotopic patterns (Br, Cl), the reported value is the one obtained for the lowest isotope mass. All results were obtained with experimental uncertainties that are commonly associated with the method used.

Hereinafter, "SQD" means Single Quadrupole Detector, "MSD" Mass Selective Detector, "BEH" bridged ethylsiloxane/silica hybrid, "DAD" Diode Array Detector, "HSS" High Strength silica, "ELSD" Evaporative Light Scanning Detector, "ACN" means acetonitrile, "TFA" means trifluoroacetic acid.

**Table 1: LCMS Method codes (Flow expressed in mL/min; column temperature (T) in °C; Run time in minutes).**

| **Method code** | **Instrument** | **Column** | **Mobile phase** | **Gradient** | **Flow-Col T** | **Run time** |
|---|---|---|---|---|---|---|
| 1 | Agilent LC-30AD LCMS-2020 Shimadzu UV 220, 254nm | EVO C18 (2.6µm,3.0*50mm) | A: Water+ 5mM NH₄HCO₃ | From 90% A to 5% A in 2.0min hold 0.6 min, to 90% A in 0.15min, hold 0.25min | 1.2-40 | 3 |
| | | | B:ACN | | | |
| 2 | Agilent LC-20ADXR LCMS-2020 Shimadzu UV 220, 254nm | Titank C18 (3µm,3.0*50mm) | A: Water+5mM NH₄HCO₃ | From 90% A to 5% A in 1.8min, hold 0.8 min, to 90% A in 0.15min, hold 0.25min | 1.5-40 | 3.0 |
| | | | B:ACN | | | |
| 3 | Agilent LC-20ADXR LCMS-2020 Shimadzu UV 220, 254nm | XBridge BEH C18 (2.5µm,3.0*50mm) | A: Water+ 0.04%NH₃.H₂O | From 90% A to 5% A in 2.0min hold 0.79 min, to 90% A in 0.06min, hold 0.16min | 1.2-40 | 3 |
| | | | B:ACN | | | |
| 4 | Agilent LC-20ADXR LCMS-2020 Shimadzu UV 220, 254nm | Kinetex XB C18 A100 (1.6µm,2.0*50mm) | A: Water/0.05%TFA | From 95% A to 0% A in 0.8min hold 0.5 min, to 95% A in 0.05min, hold 0.15min | 0.9-40 | 1.5 |
| | | | B: ACN/0.05%TFA | | | |
| 5 | Agilent LC-20ADXR LCMS-2020 Shimadzu UV 220, 254nm | Kinetex XB C18 (1.7µm,2.1*30mm) | A: Water/0.05%TFA | From 95% A to 0% A in 1.5min hold 0.8 min, to 95% A in 0.03min, hold 0.17min | 0.9-40 | 2.5 |
| | | | B: ACN/0.05%TFA | | | |
| 6 | Agilent LC-20ADXR LCMS-2020 Shimadzu UV 220, 254nm | Shim-pack-XR-ODS (1.6µm,2.0*50mm) | A: Water/0.05%TFA | From 95% A to 5% A in 2.0min hold 0.7 min, to 95% A in 0.05min, hold 0.25min | 1.2-40 | 3.0 |
| | | | B: ACN/0.05%TFA | | | |
| 7 | Agilent LC-20ADXR LCMS-2020 Shimadzu UV 220, 254nm | Shim-pack-XR-ODS (1.6µm,2.0*50mm) | A: Water/0.05%TFA | From 95% A to 5% A in 1.1min hold 0.6 min, to 95% A in 0.05min, hold 0.25min | 1.2-40 | 2.0 |
| | | | B: ACN/0.05%TFA | | | |
| 8 | Agilent LC-20ADXR LCMS-2020 Shimadzu UV 220, 254nm | Titank C18 (3µm,3.0*50mm) | A: Water+5m MNH4HCO3 | From 100% A to 65% A in 2.25min, to 5% A in 0.55min, hold 0.5 min, to 90% A in 0.02min, hold 0.18min | 1.2-40 | 3.5 |
| | | | B:ACN | | | |
| 9 | Waters: Acquity^{®} UPLC^{®} - DAD and SQD | Waters: BEH (1.8µm, 2.1*100mm) | A: 10mM CH₃COONH₄ in 95% H₂O + 5% CH₃CN | From 100% A to 5% A in 2.10min, to 0% A in 0.90min, to 5% A in 0.5min | 0.7 55 | 3.5 |
| | | | B: CH₃CN | | | |
| 10 | Waters: Acquity^{®} UPLC^{®} - DAD and SQD | Waters: BEH C18 (1.7µm, 2.1*50mm) | A: 10mM CH₃COONH₄ in 95% H₂O + 5% CH₃CN | From 95% A to 5% A in 1.3min, held for 0.7min | 0.8-55 | 2 |
| | | | B: CH₃CN | | | |
| 11 | Waters: Acquity^{®} UPLC^{®}-DAD, SQD and ELSD | Waters: HSS T3 (1.8µm, 2.1*100mm) | A: 10mM CH₃COONH₄ in 95% H₂O + 5% CH₃CN, B: CH₃CN | From 100% A to 5% A in 2.10min, to 0% A in 0.90min, to 5% A in 0.5min | 0.6-55 | 3.5 |
| 12 | Agilent LC-20ADXR LCMS-2020 Shimadzu UV 220, 254nm | XBridge C18 (2.5µm,3.0*50mm) | A: Water/5mM NH₄HCO₃ | From 90% A to 5% A in 2.0min hold 0.6 min, to 90% A in 0.2min, hold 0.2min | 1.2-40 | 3 |
| | | | B:ACN | | | |
| 13 | Waters: Acquity^{®} UPLC^{®}-DAD and SQD | Waters:BEH (1.8µm, 2.1*100mm) | A: 10mM CH₃COONH₄ in 95% H₂O + 5% CH₃CN | From 100% A to 5% A in 2.10min, to 0% A in 0.90min, to 5% A in 0.5min | 0.7-55 | 3.5 |
| | | | B: CH₃CN | | | |

### GCMS (Gas chromatography-mass spectrometry)

**Table 2: GCMS Method codes (Flow expressed in mL/min; Run time in minutes) ("EI" means electron ionization).**

| Method code | Instrument | Ionization mode | Column | Oven program | Carrier gas / Injection. mode Flow | Run time |
|---|---|---|---|---|---|---|
| 1 | Agilent: 5977B MSD | EI | Agilent: J&W HP-5MS column (0.25 µm, 250 µm x30m) | 60°C, hold for 0.5 min, then 25°C/min 9.2 min until 290°C, hold for 5.0 min | He/Split, 1:100-1.5 | 14.7 |
| 2 | Agilent: 5975C | EI | Agilent: DB-5MS column (0.33 µm, 0.2 mm x12 m) | 50°C, hold for 1 min, then 40°C/min 6.25 min until 300°C, hold for 1.75 min | He/Split, 1:20-1.0742 | 9.0 |
| 3 | Agilent: GC6890-MSD5973 N | EI | Agilent: J&W HP-5MS column (0.25 µm, 0.25mmx20m) | 50°C, hold for 0.1 min, then 24°C/min 9.583 min until 280°C, hold for 5.0 min | He/Split, 1:10-2.3 | 14.6 83 |
| 4 | Agilent : GC 6890 MSD5973 | EI | Agilent J&W Scientific DB-5ms column (0.25 µm, 0.25mmx60m) (Method mass range 10-400) | 3 min 70°C, 17.5°C/min to 320°C, 12.71 min 320°C | He/2 min splitless-1.3 mL/min | 30 |
| 5 | Agilent : GC 6890 MSD5973 | EI | Agilent J&W Scientific DB-5ms column (0.25 µm, 0.25mmx60m) (Method mass range 10-800) | 3 min 70°C, 17.5°C/min to 320°C, 12.71 min 320°C | He/2 min splitless-1.3 mL/min | 30 |

### SFC-MS (Supercritical Fluid Chromatography Mass Spectrometry)

**Table 3: SFC-MS method codes ("BPR" means backpressure; "iPrNH₂" means isopropylamine)**

| **Method code** | **Column** | **mobile phase** | **gradient** | **Flow Col T** | **Run time BPR** |
|---|---|---|---|---|---|
| 1 | Daicel Chiralpak^{®} IC-3 column (3.0 µm, 150 x 4.6 mm) | A:CO₂ | 10%-50% B in 6 min, hold 3.5 min | 2.5-40 | 9.5-110 |
| | | B: EtOH+0.2% iPrNH₂ | | | |

### Inhibition Studies

The test compound, reference compound, or water (control) were incubated for 120 min at 22°C with about 200 ng human PRMT5 complex enzyme, 600 nM [³H]SAM and 250 nM Histone H4 full length in a buffer containing 45 mM Tris-HCl (pH 9), 45 mM NaCl, 4.5 mM MgCl₂ and 3.6 mM DTT (Dithiothreitol).

For control basal measurements, the enzyme was omitted from the reaction mixture. Following incubation, the reaction was stopped by adding 33 mM citric acid and the samples were filtered rapidly *in vacuothrough* glass fiber filters (GF/B, Packard) presoaked with 33 mM citric acid and rinsed several times with ice-cold 33 mM citric acid using a 96-sample cell harvester (Unifilter, Packard). The filters were dried then counted for radioactivity in a scintillation counter (Topcount, Packard) using a scintillation cocktail (Microscint 0, Packard).

The results were expressed as a percent inhibition of the control enzyme activity.

**Table 4: PRMT5 % inhibition**

| **Compound** | **% PRMT5 inhibition at 30µM** |
|---|---|
| 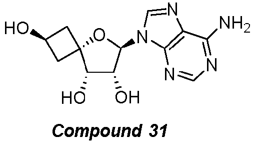 | 37.7 |
| 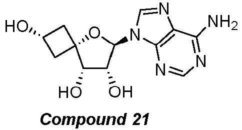 | 70.3 |
| 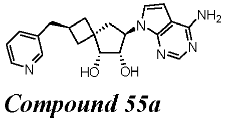 | 100.3 |
| 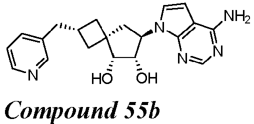 | 99.8 |
| 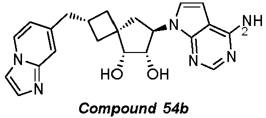 | 102.4 |
| 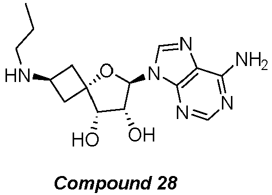 | 69.8 |
| 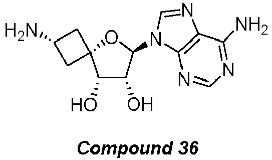 | 46.3 |
| 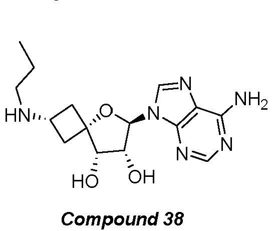 | 33.8 |
| 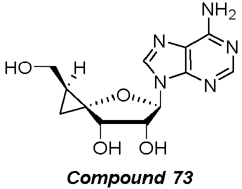 | 36.4 |
| 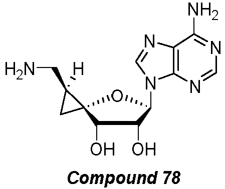 | 55.9 |
| 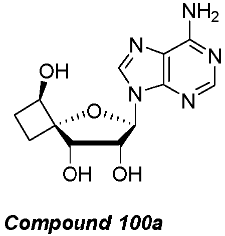 | 98.0 |
| 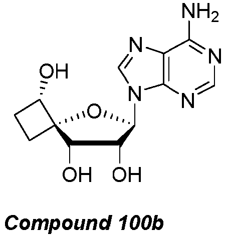 | 42.0 |
| 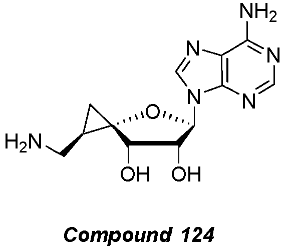 | 79.0 |
| 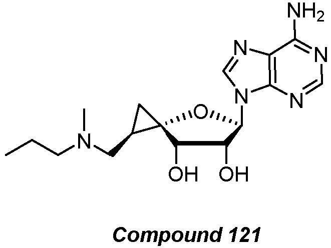 | 16.0 |
| 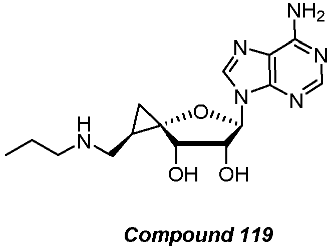 | 39.0 |
| 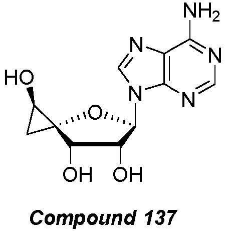 | 72.0 |
| 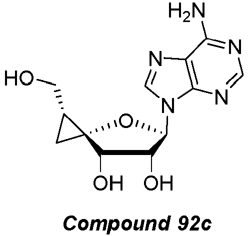 | 29.0 |
| 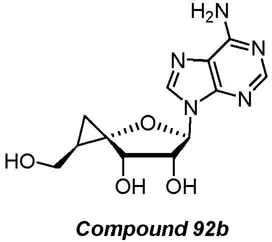 | 84.0 |
| 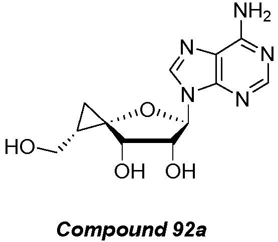 | 31.0 |

## Claims

1. A compound of Formula (A) 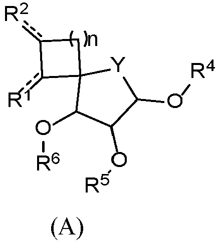 or a salt or solvate thereof, wherein:
Y is -O- or -CH₂-;
n is 0 or 1;
when n is 1, R¹ is hydrogen and R² is -(CH₂)ₘNR^{a}₂, -(CH₂)ₘOR^{a}, -(CH₂)ₘC(O)OR ^{a}, - (CH₂)ₘC(O)NR^{a}₂, -(CH₂)ₘC(O)R^{a}, -(CH₂)ₘB(OR^{b})₂, -(CH₂)ₘS(O)ₓR^{c}, a silyl group, C₁-₆ alkyl substituted with a silyl group, =O or =CR^{a}₂; or
when n is 1, R² is hydrogen and R¹ is -(CH₂)ₘNR^{a}₂, -(CH₂)ₘOR^{a}, -(CH₂)ₘOPG, - (CH₂)ₘC(O)OR^{a}, -(CH₂)ₘC(O)NR^{a}₂, -(CH₂)ₘC(O)R^{a}, -(CH₂)ₘB(OR^{b})₂, -(CH₂)ₘS(O)ₓR^{c}, a silyl group, C₁-₆ alkyl substituted with a silyl group, -O-aryl, =O or =CR^{a}₂;
when n is 0, one of R¹ and R² is hydrogen and the other is -(CH₂)ₘNR^{a}₂,-(CH₂)ₘOR^{a}, - (CH₂)ₘOPG, -(CH₂)ₘC(O)OR^{a}, -(CH₂)ₘC(O)NR^{a}₂, -(CH₂)ₘC(O)R^{a}, -(CH₂)ₘB(OR^{b})₂, - (CH₂)ₘS(O)ₓR^{c} or =CH₂;
wherein the bond towards R¹ or R² - - is a single bond when R¹ or R² is hydrogen, - (CH₂)ₘNR^{a}₂, -(CH₂)ₘOR^{a}, -(CH₂)ₘOPG, -(CH₂)ₘC(O)OR^{a}, -(CH₂)ₘC(O)NR^{a}₂, - (CH₂)ₘC(O)R^{a}, -(CH₂)ₘB(OR^{b})₂, -(CH₂)ₘS(O)ₓR^{c}, a silyl group, C₁-₆ alkyl substituted with a silyl group or -O-aryl, or a double bond when R¹ or R² is =O, =CH₂ or =CR^{a}₂;
each R^{a} is, independently, hydrogen or C₁-₆ alkyl;
each R^{b} is hydrogen or both R^{b} are taken together to form a boronic acid protecting group that is catechol borane, pinacol borane, N-methyliminodiacetic acid boronate, neopentylglycol borane, pinanediol borane, biscyclohexyldiol borane, or 1-(4-methoxyphenyl)-2-methylpropane-1,2-diol borane;
R^{c} is C₁₋₆ alkyl or aryl;
PG is a hydroxyl protecting group that is a silyl group, C₁₋₄alkyl, C₁₋₄alkyl-O-C₁₋₄alkyl, tetrahydropyranyl, allyl, benzyl, -CH₂-naphthyl, benzoyl, -C(=O)-C₁₋₄alkyl, or -C(=O)-phenyl; wherein benzyl, -CH₂-naphthyl, and benzoyl, are optionally substituted with one or two substituents each independently selected from -CH₃ and -OCH₃;
m is 0 or 1;
x is 0, 1 or 2;
R⁴, R⁵ and R⁶ are each independently hydrogen or a hydroxyl protecting group that is a silyl group, C₁₋₄alkyl, C₁₋₄alkyl-O-C₁₋₄alkyl, tetrahydropyranyl, allyl, benzyl, -CH₂-naphthyl, benzoyl, -C(=O)-C₁₋₄alkyl, or -C(=O)-phenyl; wherein benzyl, -CH₂-naphthyl, and benzoyl, are optionally substituted with one or two substituents each independently selected from -CH₃ and -OCH₃;
or R⁵ and R⁶ taken together are a diol protecting group -C(C₁₋₄alkyl)₂-;
or R⁴ and R⁵ taken together are a diol protecting group -C(C₁₋₄alkyl)₂-;
provided that:
(a) the compound is not a compound of formula: 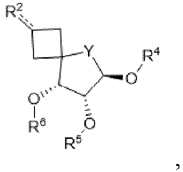 wherein Y is -O- or -CH₂-; R⁴ is hydrogen or a hydroxyl protecting group that is C₁₋₄alkyl, t-butyldimethylsilyl, C₁₋₄alkyl-O-C₁₋₄alkyl, tetrahydropyranyl, allyl, t-butyldiphenylsilyl, benzyl, -C(=O)-C₁₋₄alkyl, or -C(=O)-phenyl; R² is -OH, =O, -CH₂-OH or =CH₂; and R⁵ and R⁶ are each independently -C(=O)-C₁₋₄alkyl, benzoyl, benzyl, or -CH₂-naphthyl, wherein benzyl, -CH₂-naphthyl, and benzoyl are optionally substituted with one or two substituents each independently selected from -CH₃ and -OCH₃; or R⁵ and R⁶ taken together are -C(C₁₋₄alkyl)₂-; or wherein Y is -O- or -CH₂, R⁴ is methyl, R² is =O, or =CH₂, and R⁵ and R⁶ are each independently hydrogen or -C(=O)-C₁₋₄alkyl; and
(b) the compound is not a compound of formula: 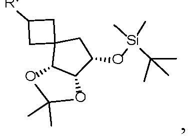 wherein R^{v} is =O, -OH or =CH₂; and
(c) the compound is not a compound of formula: 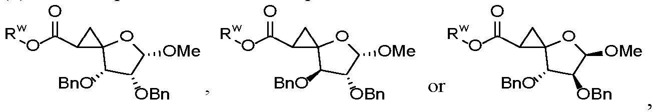 wherein R^{w} is methyl or H; and
(d) the compound is not a compound of formula: 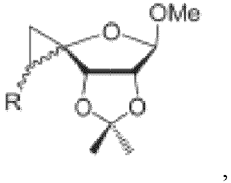 wherein R is CO₂Et or CH₂OH or any stereoisomer thereof.

2. The compound of claim 1, wherein the compound is a compound of formula (A-1a), (A-3a), (A-1b) or (A-3b): 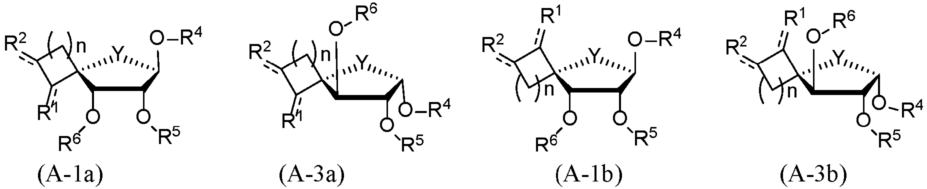 or a salt or solvate thereof.

3. The compound of any one of claims 1 to 2 , wherein Y is O.

4. The compound of any one of claims 1 to 2 , wherein Y is CH₂.

5. The compound of any one of claims 1 to 4 , wherein n is 1.

6. The compound of claim 5, wherein:
R¹ is hydrogen and R² -(CH₂)ₘNR^{a}₂, -(CH₂)ₘOR^{a}, -(CH₂)ₘC(O)OR^{a}, -(CH₂)ₘC(O)NR^{a}₂, - (CH₂)ₘC(O)R^{a}, -(CH₂)ₘS(O)ₓR^{c}, a silyl group, C₁-₆ alkyl substituted with a silyl group, =O or =CR^{a}₂; or
R² is hydrogen and R¹ is -(CH₂)ₘNR^{a}₂, -(CH₂)ₘOR^{a}, -(CH₂)ₘOPG, -(CH₂)ₘC(O)OR^{a}, - (CH₂)ₘC(O)NR^{a}₂, -(CH₂)ₘC(O)R^{a}, -(CH₂)ₘS(O)ₓR^{c}, a silyl group, C₁-₆ alkyl substituted with a silyl group, -O-aryl, =O or =CR^{a}₂.

7. The compound of any one of claims 1 to 4, , wherein n is 0.

8. The compound of claim 7, wherein one of R¹ and R² is hydrogen and the other is - (CH₂)ₘNR^{a}₂, -(CH₂)ₘOR^{a}, -(CH₂)ₘOPG, -C(O)OR^{a}, -C(O)NR^{a}₂ or -B(OR^{b})₂.

9. The compound of claim 1, wherein the compound is selected from: 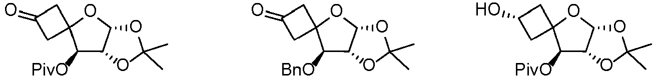 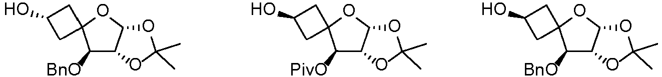 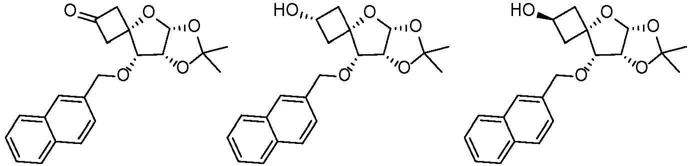 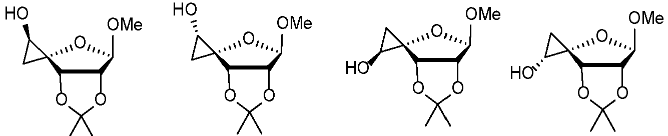 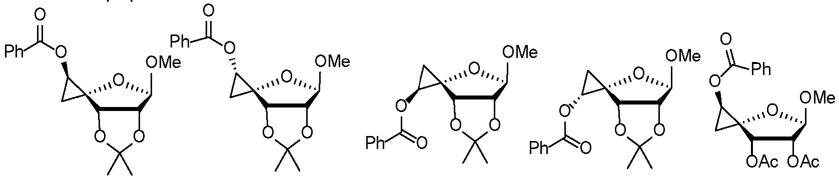 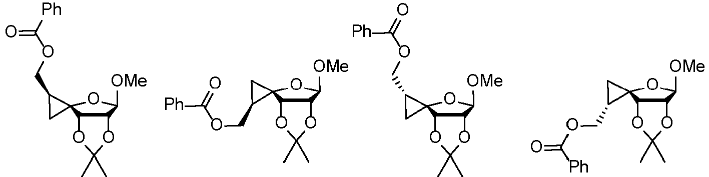 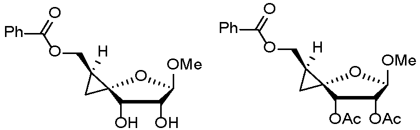 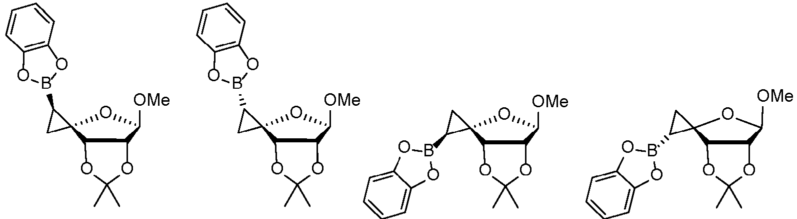 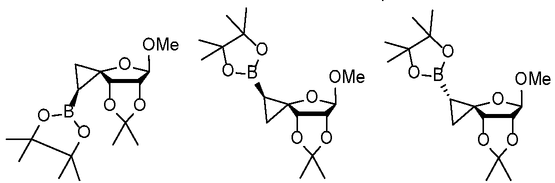 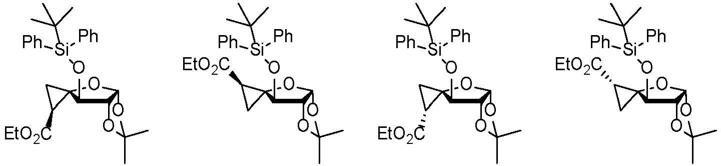 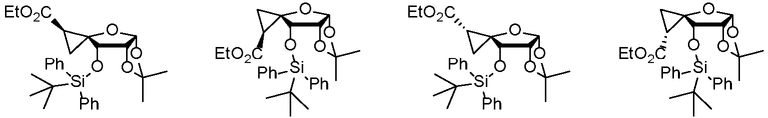 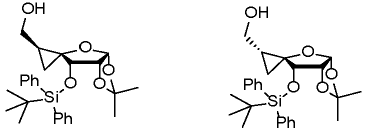 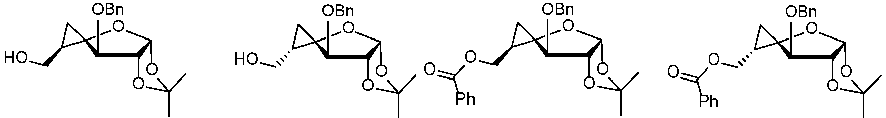 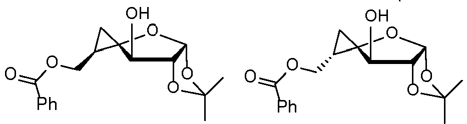 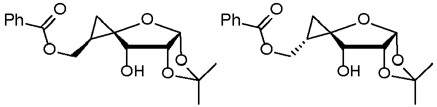 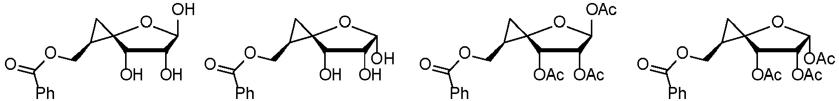 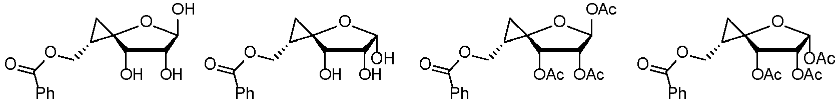 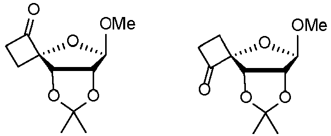 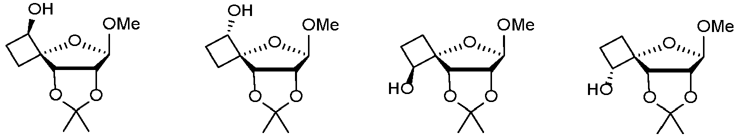 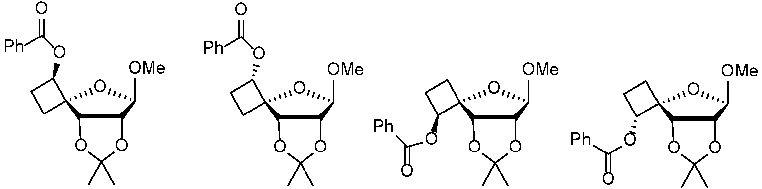 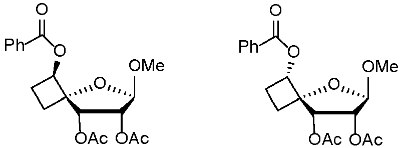 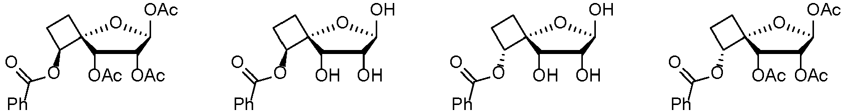 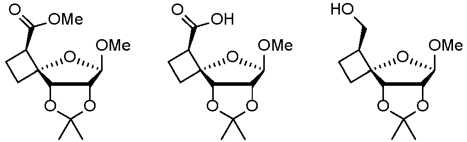 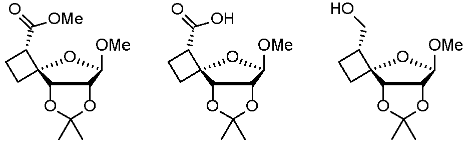 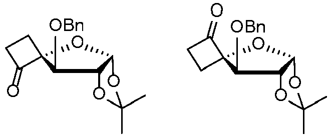 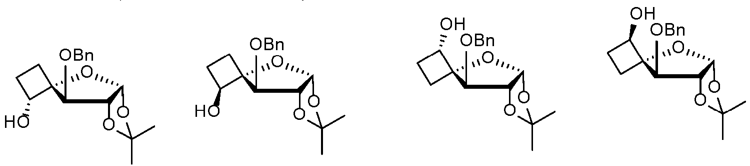 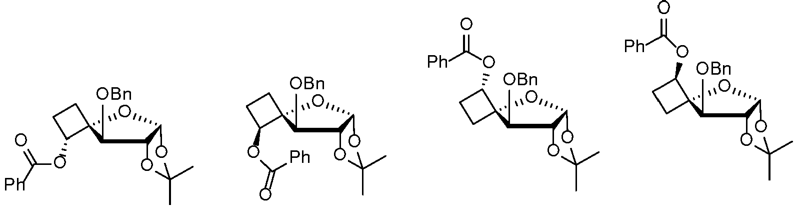 or a salt or solvate thereof.

10. A compound of formula (B): 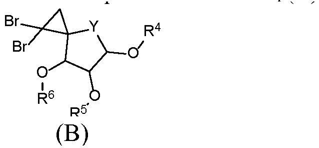 or a salt or solvate thereof, wherein:
Y is -O- or -CH₂-;
R⁴, R⁵ and R⁶ are each, independently, hydrogen or a hydroxyl protecting group that is a silyl group, C₁₋₄alkyl, C₁₋₄alkyl-O-C₁₋₄alkyl, tetrahydropyranyl, allyl, benzyl, -CH₂-naphthyl, benzoyl, -C(=O)-C₁₋₄alkyl, or -C(=O)-phenyl; wherein benzyl, -CH₂-naphthyl, and benzoyl, are optionally substituted with one or two substituents each independently selected from -CH₃ and -OCH₃;
or R⁵ and R⁶ taken together are a diol protecting group -C(C₁₋₄alkyl)₂-;
or R⁴ and R⁵ taken together are a diol protecting group -C(C₁₋₄alkyl)₂-.

11. The compound of claim 10, wherein the compound is selected from: 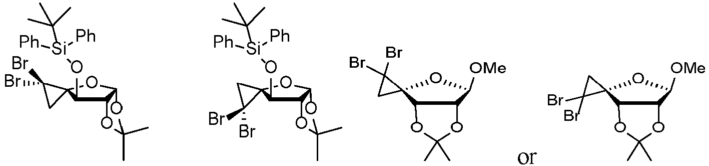 or a salt or solvate thereof.

12. A compound of formula (C): 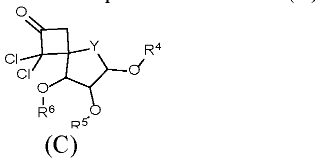 wherein:
Y is -O- or -CH₂-;
R⁴, R⁵ and R⁶ are each, independently, hydrogen or a hydroxyl protecting group that is a silyl group, C₁₋₄alkyl, C₁₋₄alkyl-O-C₁₋₄alkyl, tetrahydropyranyl, allyl, benzyl, -CH₂-naphthyl, benzoyl, -C(=O)-C₁₋₄alkyl, or -C(=O)-phenyl; wherein benzyl, -CH₂-naphthyl, and benzoyl, are optionally substituted with one or two substituents each independently selected from -CH₃ and -OCH₃; or
R⁴ and R⁵ taken together are a diol protecting group -C(C₁₋₄alkyl)₂-; or
R⁵ and R⁶ taken together are a diol protecting group -C(C₁₋₄alkyl)₂-;
provided that the compound is not:

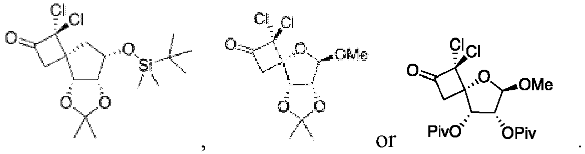

13. The compound of claim 12, wherein the compound is selected from: 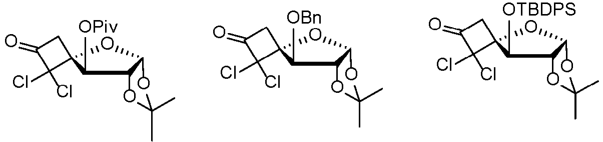 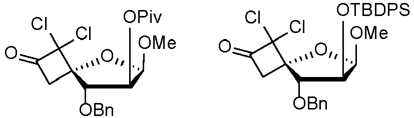 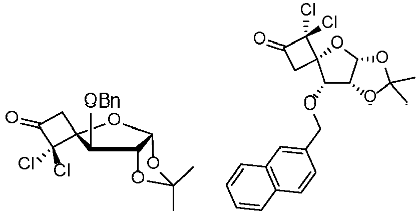 or a salt or solvate thereof.

14. A compound of formula (D): 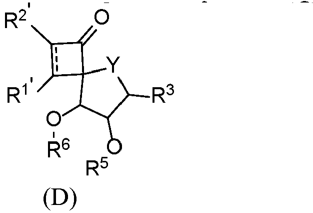 or a salt or solvate thereof, wherein:
Y is -O- or -CH₂-;
wherein - - is a single bond or a double bond; and
when - - is a single bond, one of R¹' and R²' is hydrogen or C₁-₆ alkyl and the other is a silyl group, C₁-₆ alkyl optionally substituted with a silyl group, -O-C₁-₆ alkyl, -B(OR^{b})₂ -S-C₁-₆ alkyl, -S-aryl, -O-aryl or C(O)R^{a};
when - - is a double bond, one of R¹' and R²' is hydrogen or C₁-₆ alkyl and the other is a silyl group, C₁-₆ alkyl optionally substituted with a silyl group, -O-C₁-₆ alkyl, , or C(O)R^{a}; and
R^{a} is hydrogen or C₁-₆ alkyl;
each R^{b} is hydrogen or both R^{b} are taken together to form a boronic acid protecting group that is catechol borane, pinacol borane, N-methyliminodiacetic acid boronate, neopentylglycol borane, pinanediol borane, biscyclohexyldiol borane, or 1-(4-methoxyphenyl)-2-methylpropane-1,2-diol borane;
R³ is OR⁴ or R¹⁰;
R¹⁰ is a nucleobase or nucleobase derivative;
R⁴, R⁵ and R⁶ are each, independently, hydrogen or a hydroxyl protecting group that is a silyl group, C₁₋₄alkyl, C₁₋₄alkyl-O-C₁₋₄alkyl, tetrahydropyranyl, allyl, benzyl, -CH₂-naphthyl, benzoyl, -C(=O)-C₁₋₄alkyl, or -C(=O)-phenyl; wherein benzyl, -CH₂-naphthyl, and benzoyl, are optionally substituted with one or two substituents each independently selected from -CH₃ and -OCH₃; or
R⁴ and R⁵ taken together are a diol protecting group -C(C₁₋₄alkyl)₂-; or
R⁵ and R⁶ taken together are a diol protecting group -C(C₁₋₄alkyl)₂-.

15. The compound of claim 14, wherein the compound is selected from: 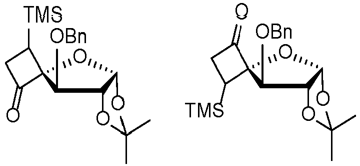 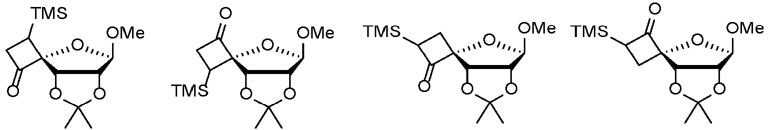 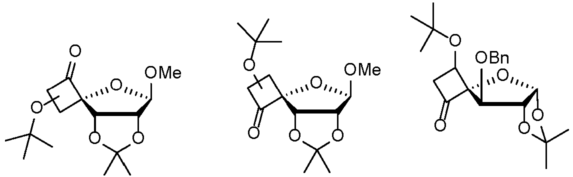 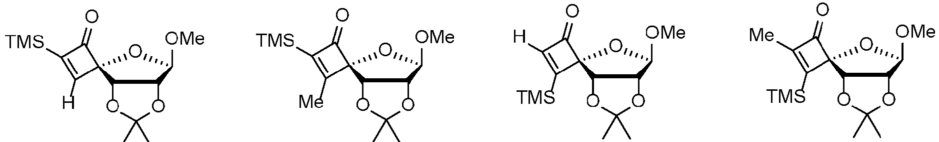 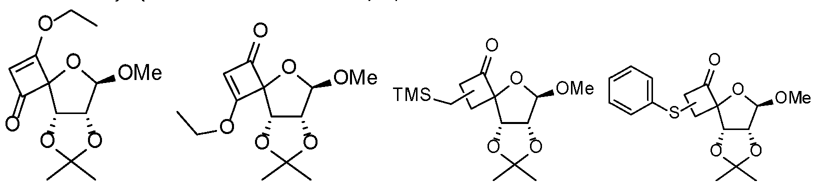 or a salt or solvate thereof

16. A compound of formula (E): 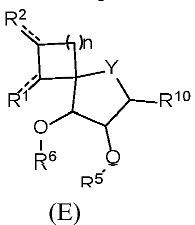 or a salt or solvate thereof, wherein:
Y is -O- or -CH₂-;
n is 0 or 1;
when n is 1, R¹ is hydrogen and R² is -(CH₂)ₘNR^{a}₂, -(CH₂)ₘOR^{a}, -(CH₂)ₘC(O)OR^{a}, - (CH₂)ₘC(O)NR^{a}₂, -(CH₂)ₘC(O)R^{a}, -(CH₂)ₘB(OR^{b})₂, -(CH₂)ₘS(O)ₓR^{c}, a silyl group, C₁-₆ alkyl substituted with a silyl group, =O or =CR^{a}₂; or
when n is 1, R² is hydrogen and R¹ is -(CH₂)ₘNR^{a}₂, -(CH₂)ₘOR^{a}, -(CH₂)ₘOPG, - (CH₂)ₘC(O)OR^{a}, -(CH₂)ₘC(O)NR^{a}₂, -(CH₂)ₘC(O)R^{a}, -(CH₂)ₘB(OR^{b})₂, -(CH₂)ₘS(O)ₓR^{c}, a silyl group, C₁-₆ alkyl substituted with a silyl group, -O-aryl, =O or =CR^{a}₂;
when n is 0, one of R¹ and R² is hydrogen and the other is -(CH₂)ₘNR^{a}₂, -(CH₂)ₘOR^{a}, - (CH₂)ₘOPG, -(CH₂)ₘC(O)OR^{a}, -(CH₂)ₘC(O)NR^{a}₂, -(CH₂)ₘC(O)R^{a}, -(CH₂)ₘB(OR^{b})₂, - (CH₂)ₘS(O)ₓR^{c} or =CH₂;
wherein the bond towards R¹ or R² - - is a single bond when R¹ or R² is hydrogen, - (CH₂)ₘNR^{a}₂, -(CH₂)ₘOR^{a}, -(CH₂)ₘOPG, -(CH₂)ₘC(O)OR^{a}, -(CH₂)ₘC(O)NR^{a}₂, - (CH₂)ₘC(O)R^{a}, -(CH₂)ₘB(OR^{b})₂, -(CH₂)ₘS(O)ₓR^{c}, a silyl group, C₁-₆ alkyl substituted with a silyl group or -O-aryl, or a double bond when R¹ or R² is =O, =CH₂ or =CR^{a}₂;
each R^{a} is, independently, hydrogen or C₁-₆ alkyl;
both R^{b} are hydrogen or both R^{b} are taken together to form a boronic acid protecting group that is catechol borane, pinacol borane, N-methyliminodiacetic acid boronate, neopentylglycol borane, pinanediol borane, biscyclohexyldiol borane, or 1-(4-methoxyphenyl)-2-methylpropane-1,2-diol borane;
R^{c} is C₁-₆ alkyl or aryl;
x is 0, 1 or 2;
m is 0 or 1;
R⁵ and R⁶ are each, independently, hydrogen or a hydroxyl protecting group that is a silyl group, C₁₋₄alkyl, C₁₋₄alkyl-O-C₁₋₄alkyl, tetrahydropyranyl, allyl, benzyl, -CH₂-naphthyl, benzoyl, - C(=O)-C₁₋₄alkyl, or -C(=O)-phenyl; wherein benzyl, -CH₂-naphthyl, and benzoyl, are optionally substituted with one or two substituents each independently selected from -CH₃ and -OCH₃; or R⁵ and R⁶ taken together are a diol protecting group -C(C₁₋₄alkyl)₂-;
R¹⁰ is a nucleobase or nucleobase derivative;
PG is a hydroxyl protecting group that is a silyl group, C₁₋₄alkyl, C₁₋₄alkyl-O-C₁₋₄alkyl, tetrahydropyranyl, allyl, benzyl, -CH₂-naphthyl, benzoyl, -C(=O)-C₁₋₄alkyl, or -C(=O)-phenyl;
wherein benzyl, -CH₂-naphthyl, and benzoyl, are optionally substituted with one or two substituents each independently selected from -CH₃ and -OCH₃;
provided that the compound is not any of: 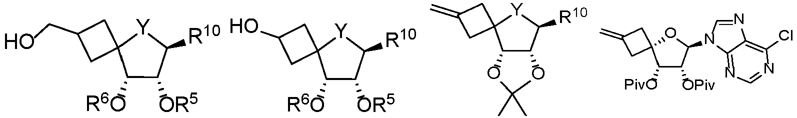 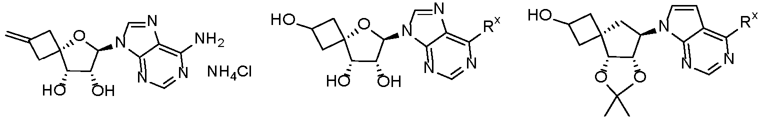
wherein
R⁵ is hydrogen or -C(=O)-C₁₋₄alkyl;
R⁶ is hydrogen or -C(=O)-C₁₋₄alkyl; or R⁵ and R⁶ taken together are -C(CH₃)₂-
Y is -O- or -CH₂-;
R¹⁰ is 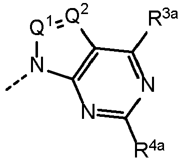
Q¹ is CR^{6a};
Q² is N or CR^{6b};
R^{6a} and R^{6b} each independently represent hydrogen, halogen, C₁₋₄alkyl, -NR^{9a}R^{9b}, or C₁₋₄alkyl substituted with one, two or three halo atoms;
R^{9a} and R^{9b} each independently represent hydrogen or C₁₋₄alkyl;
R^{3a} is hydrogen, halo, -NR^{7a}R^{7b}, C₁₋₄alkyl, C₂₋₄alkenyl, C₃₋₆cycloalkyl, -OH, or
-O-C₁₋₄alkyl;
R^{7a} is hydrogen;
R^{7b} is hydrogen, C₃₋₆cycloalkyl, or C₁₋₄alkyl;
R^{4a} is hydrogen, halo, -NR^{8a}R^{8b}, or C₁₋₄alkyl; and
R^{x} is 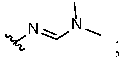
or a salt or solvate thereof.

17. The compound of claim 16, wherein the compound is a compound of formula (G-1a) or (G-1b): 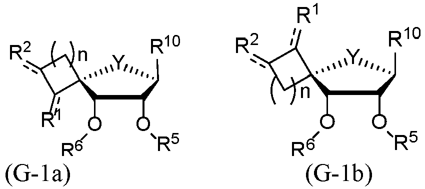 or a salt or solvate thereof.

18. The compound of any one of claims 16 or 17, wherein n is 1.

19. The compound of claim 18, wherein:
R¹ is hydrogen and R² is -(CH₂)ₘNR^{a}₂, -(CH₂)ₘOR^{a}, -(CH₂)ₘC(O)OR^{a}, -(CH₂)ₘC(O)NR^{a}₂, - (CH₂)ₘC(O)R^{a}, -(CH₂)ₘS(O)ₓR^{c}, a silyl group, C₁-₆ alkyl substituted with a silyl group, =O or =CR^{a}₂; or
R² is hydrogen and R¹ is -(CH₂)ₘNR^{a}₂, -(CH₂)ₘOR^{a}, -(CH₂)ₘOPG, -(CH₂)ₘC(O)OR^{a}, - (CH₂)ₘC(O)NR^{a}₂, -(CH₂)ₘC(O)R^{a}, -(CH₂)ₘS(O)ₓR^{c}, a silyl group, C₁-₆ alkyl substituted with a silyl group, -O-aryl, =O or =CR^{a}₂.

20. The compound of any one of claims 16 or 17, wherein n is 0.

21. The compound of claim 18, wherein one of R¹ and R² is hydrogen and the other is - (CH₂)ₘNR^{a}₂, -(CH₂)ₘOR^{a}, -(CH₂)ₘOPG, -(CH₂)ₘC(O)OR^{a}, -(CH₂)ₘC(O)NR^{a}₂, - (CH₂)ₘC(O)R^{a}, -(CH₂)ₘB(OR^{b})₂, or =CH₂.

22. The compound of claim 16, wherein the compound is selected from: 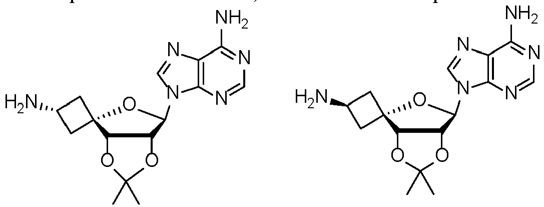 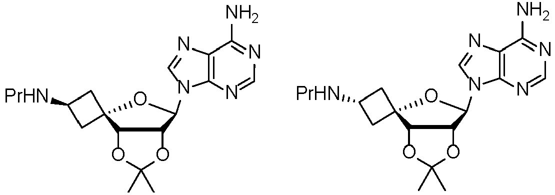 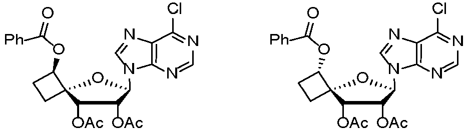 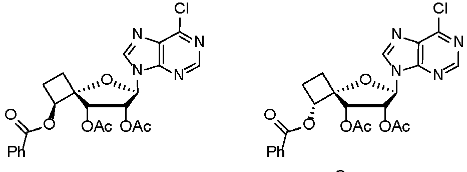 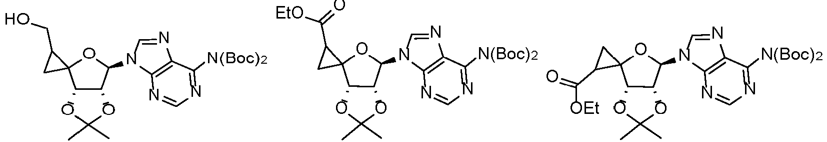 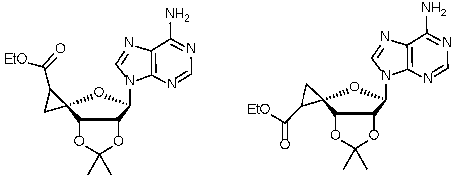 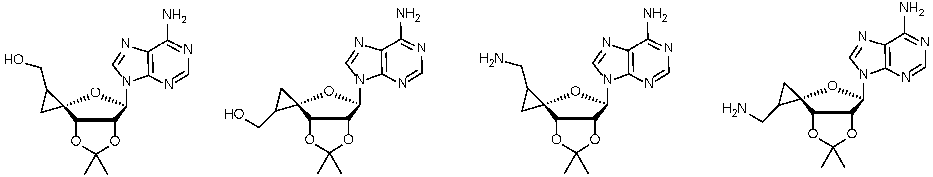 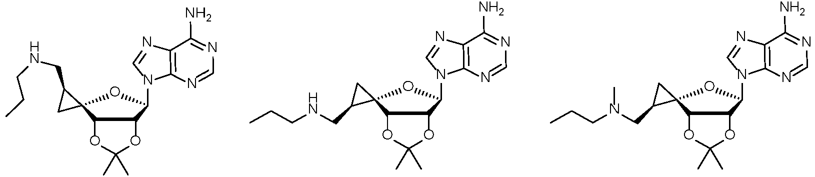 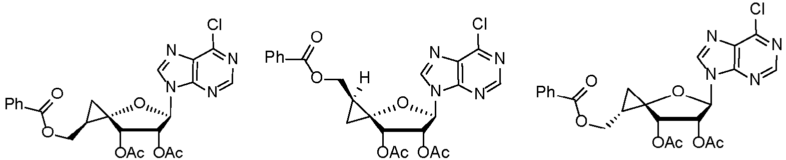 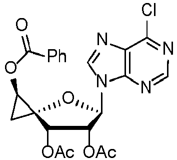 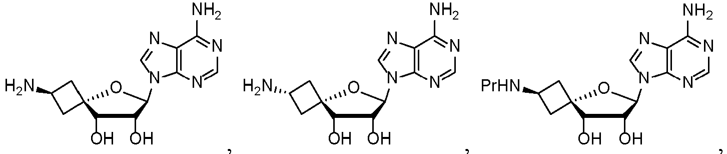 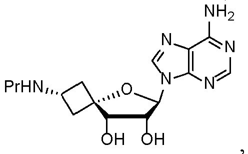 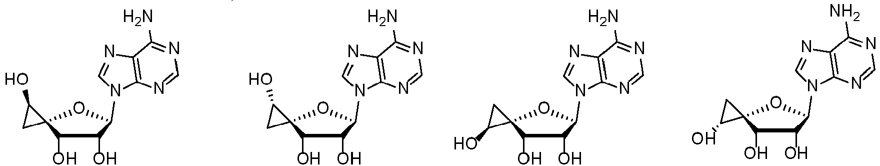 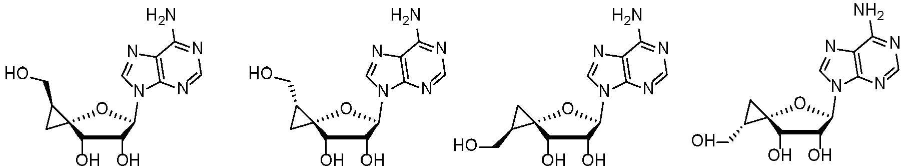 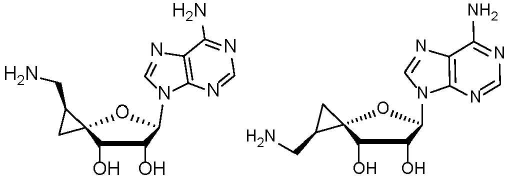 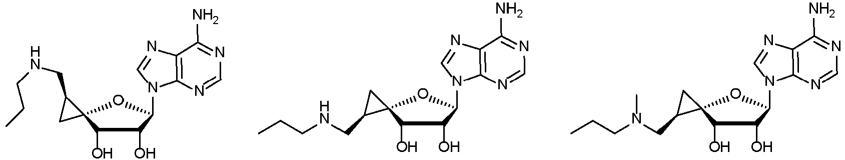 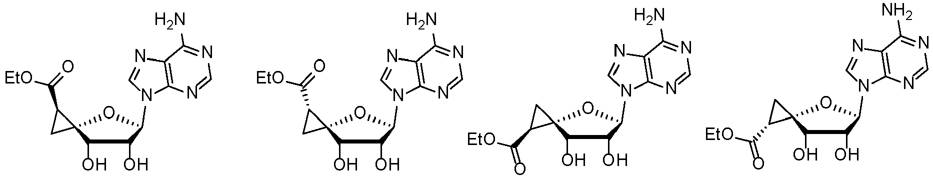 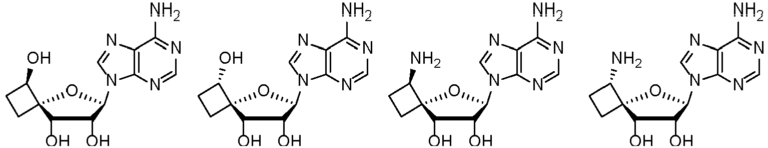 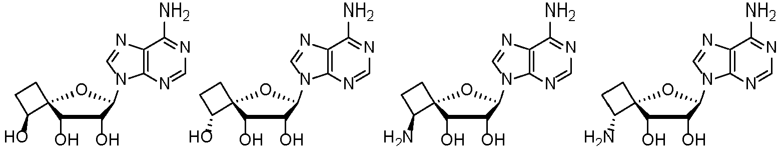 or a salt or solvate thereof.

23. A compound of formula (H): 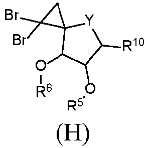 or a salt or solvate thereof, wherein:
Y is -O- or -CH₂-;
R⁵ and R⁶ are each, independently, hydrogen or a hydroxyl protecting group that is a silyl group, C₁₋₄alkyl, C₁₋₄alkyl-O-C₁₋₄alkyl, tetrahydropyranyl, allyl, benzyl, -CH₂-naphthyl, benzoyl, -C(=O)-C₁₋₄alkyl, or -C(=O)-phenyl; wherein benzyl, -CH₂-naphthyl, and benzoyl, are optionally substituted with one or two substituents each independently selected from -CH₃ and -OCH₃; or R⁵ and R⁶ taken together are a diol protecting group -C(C₁₋₄alkyl)₂-; and
R¹⁰ is a nucleobase or nucleobase derivative

24. The compound of any one of claims 14 to 23, wherein R¹⁰, when present, is cytosine, thymine, uracil, or a modified or protected form thereof, or a bicyclic aromatic heterocyclic ring system selected from the group consisting of (a-1), (a-2), (a-3), (a-4) and (a-5): 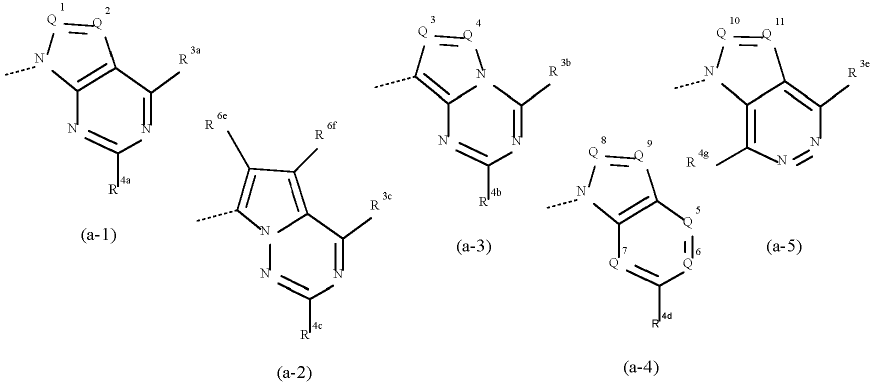
R^{3a}, R^{3b}, R^{3c}, R^{3d} and R^{3e} each independently are hydrogen, halo, -NR^{7a}R^{7b},
C₁₋₄alkyl, C₂₋₄alkenyl, C₃₋₆cycloalkyl, -OH, or -O-C₁₋₄alkyl;
R^{7a} is hydrogen, -C(O)O-C₁₋₄alkyl or 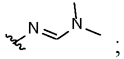
R^{7b} is hydrogen, C₃₋₆cycloalkyl, C₁₋₄alkyl, or -C(O)O-C₁₋₄alkyl;
R^{4a}, R^{4b}, R^{4c}, R^{4d}, R^{4e}, R^{4f} and R^{4g} each independently are hydrogen, halo, -NR^{8a}R^{8b}, or C₁₋₄alkyl;
R^{8a} and R^{8b} each independently are hydrogen or C₁₋₄alkyl;
Q¹ is N or CR^{6a};
Q² is N or CR^{6b};
Q³ is N or CR^{6c};
Q⁴ is N or CR^{6a};
provided that maximum one of Q³ and Q⁴ is N;
Q⁸ is N or CR^{6g};
Q⁹ is N or CR^{6h;}
Q¹⁰ is N or CR⁶ⁱ;
Q¹¹ is N or CR^{6j};
Q⁵ is CR^{3d}; Q⁶ is N; and Q⁷ is CR^{4f}; or
Q⁵ is CR^{3d}; Q⁶ is CR^{4e}; and Q⁷ is N; or
Q⁵ is N; Q⁶ is CR^{4e}; and Q⁷ is CR^{4f}; or
Q⁵ is N; Q⁶ is CR^{4e}; and Q⁷ is N; or
Q⁵ is N; Q⁶ is N; and Q⁷ is CR^{4f}; or
Q⁵ is N; Q⁶ is N; and Q⁷ is N;
R^{6a}, R^{6b}, R^{6c}, R^{6d}, R^{6e}, R^{6f}, R^{6g}, R^{6h}, R⁶ⁱ and R^{6j} each independently are hydrogen, halogen, C₁₋₄alkyl, -NR^{9a}R^{9b}, or C₁₋₄alkyl substituted with one, two or three halo atoms;
R^{9a} and R^{9b} each independently are hydrogen or C₁₋₄alkyl.

## Patentansprüche

1. Verbindung der Formel (A) 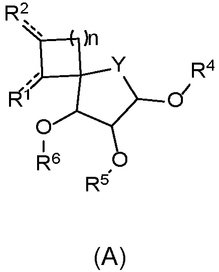 oder ein Salz oder Solvat davon, wobei:
Y -O- oder -CH₂- ist;
n 0 oder 1 ist;
wenn n 1 ist, ist R¹ Wasserstoff und R² ist -(CH₂)ₘNR^{a}₂, -(CH₂)ₘOR^{a}, - (CH₂)ₘC(O)OR^{a}, -(CH₂)ₘC(O)NR^{a}₂, -(CH₂)ₘC(O)R^{a}, -(CH₂)ₘB(OR^{b})₂, -(CH₂)ₘS(O)ₓR^{c}, eine Silyl-Gruppe, C₁-₆-Alkyl substituiert mit einer Silyl-Gruppe, =O oder =CR^{a}₂; oder
wenn n 1 ist, ist R² Wasserstoff und R¹ ist -(CH₂)ₘNR^{a}₂, -(CH₂)ₘOR^{a}, - (CH₂)ₘOPG, -(CH₂)ₘC(O)OR^{a}, -(CH₂)ₘC(O)NR^{a}₂, -(CH₂)ₘC(O)R^{a}, - (CH₂)ₘB(OR^{b})₂, -(CH₂)ₘS(O)ₓR^{c}, eine Silyl-Gruppe, C₁-₆-Alkyl, substituiert mit einer Silyl-Gruppe, -O-Aryl, =O oder =CR^{a}₂;
wenn n 0 ist, dann ist einer von R¹ und R² Wasserstoff und der andere ist - (CH₂)ₘNR^{a}₂,-(CH₂)ₘOR^{a}, -(CH₂)ₘOPG, -(CH₂)ₘC(O)OR^{a}, -(CH₂)ₘC(O)NR^{a}₂, - (CH₂)ₘC(O)R^{a}, -(CH₂)ₘB(OR^{b})₂, -(CH₂)ₘS(O)ₓR^{c} oder =CH₂;
wobei die Bindung zu R¹ oder R² - - eine Einfachbindung ist, wenn R¹ oder R² Wasserstoff, -(CH₂)ₘNR^{a}₂, -(CH₂)ₘOR^{a}, -(CH₂)ₘOPG, -(CH₂)ₘC(O)OR^{a}, - (CH₂)ₘC(O)NR^{a}₂, -(CH₂)ₘC(O)R^{a}, -(CH₂)ₘB(OR^{b})₂, -(CH₂)ₘS(O)_{×}R^{c}, eine Silyl-Gruppe, C₁-₆-Alkyl, substituiert mit einer Silyl-Gruppe oder -O-Aryl ist, oder eine Doppelbindung, wenn R¹ oder R² =O, =CH₂ oder =CR^{a}₂ ist;
jeder R^{a} unabhängig Wasserstoff oder C₁₋₆-Alkyl ist;
jeder R^{b} Wasserstoff ist oder beide R^{b} zusammen eine Boronsäure-Schutzgruppe bilden, nämlich Catecholboran, Pinakolboran, N-Methyliminodiessigsäureboronat, Neopentylglykolboran, Pinandiolboran, Biscyclohexyldiolboran oder 1-(4-Methoxyphenyl)-2-methylpropan-1,2-diolboran;
R^{c} C₁₋₆-Alkyl oder -Aryl ist;
PG eine Hydroxyl-Schutzgruppe ist, die eine Silyl-Gruppe, C₁₋₄-Alkyl, C₁₋₄-Alkyl-O-C₁₋₄-Alkyl, Tetrahydropyranyl, Allyl, Benzyl, -CH₂-Naphthyl,
Benzoyl, -C(=O)-C₁₋₄-Alkyl oder -C(=O)-Phenyl ist; wobei Benzyl, -CH₂-Naphthyl und Benzoyl optional durch einen oder zwei Substituenten substituiert sind, die jeweils unabhängig aus -CH₃ und -OCH₃ ausgewählt sind;
m 0 oder 1 ist;
x 0, 1 oder 2 ist;
R⁴, R⁵ und R⁶ jeweils unabhängig Wasserstoff oder eine Hydroxyl-Schutzgruppe sind, die eine Silyl-Gruppe, C₁₋₄-Alkyl, C₁₋₄-Alkyl-O-C₁₋₄-Alkyl, Tetrahydropyranyl, Allyl, Benzyl, -CH₂-Naphthyl, Benzoyl, -C(=O)-C₁₋₄-Alkyl oder -C(=O)-Phenyl ist; wobei Benzyl, -CH₂-Naphthyl und Benzoyl optional durch einen oder zwei Substituenten substituiert sind, die jeweils unabhängig aus -CH₃ und -OCH₃ ausgewählt sind;
oder R⁵ und R⁶ zusammen eine Diol-Schutzgruppe -C(C₁₋₄-Alkyl)₂- sind;
oder R⁴ und R⁵ zusammen eine Diol-Schutzgruppe -C(C₁₋₄-Alkyl)₂- sind;
unter der Voraussetzung, dass:
(a) die Verbindung keine Verbindung der Formel: 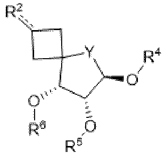 ist, wobei Y -O- oder -CH₂- ist; R⁴ Wasserstoff oder eine Hydroxyl-Schutzgruppe ist, die C₁₋₄-Alkyl, t-Butyldimethylsilyl, C₁₋₄-Alkyl-O-C₁₋₄-Alkyl, Tetrahydropyranyl, Allyl, t-Butyldiphenylsilyl, Benzyl, -C(=O)-C₁₋₄-Alkyl oder -C(=O)-Phenyl ist; R² -OH, =O, -CH₂-OH oder =CH₂ ist; und R⁵ und R⁶ jeweils unabhängig -C(=O)-C₁₋₄-Alkyl, Benzoyl, Benzyl oder -CH₂-Naphthyl sind, wobei Benzyl, -CH₂-Naphthyl und Benzoyl gegebenenfalls mit einem oder zwei Substituenten substituiert sind, die jeweils unabhängig aus -CH₃ und -OCH₃ ausgewählt sind; oder R⁵ und R⁶ zusammen genommen -C(C₁-₄Alkyl)₂- sind; oder wobei Y -O- oder -CH_{2 ist}, R⁴ Methyl ist, R² =O ist oder =CH₂, und R⁵ und R⁶ jeweils unabhängig Wasserstoff oder -C(=O)-C₁₋₄-Alkyl sind; und
(b) die Verbindung keine Verbindung der Formel: 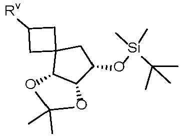 ist, wobei R^{v} =O, -OH oder =CH₂ ist; und
(c) die Verbindung keine Verbindung der Formel: 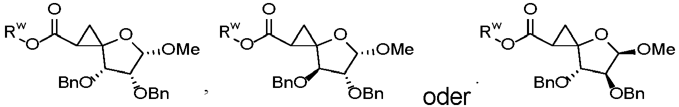 ist, wobei R^{w} Methyl oder H ist; und
(d) die Verbindung keine Verbindung der Formel: 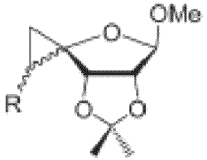 ist, wobei R CO₂Et oder CH₂OH oder ein beliebiges Stereoisomer davon ist.

2. Verbindung nach Anspruch 1, wobei die Verbindung eine Verbindung der Formel (A-1a), (A-3a), (A-1b) oder (A-3b) ist: 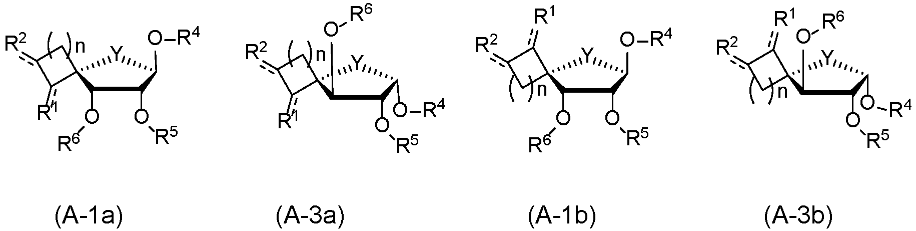 oder ein Salz oder Solvat davon.

3. Verbindung nach einem der Ansprüche 1 bis 2, wobei Y O ist.

4. Verbindung nach einem der Ansprüche 1 bis 2, wobei Y CH₂ ist.

5. Verbindung nach einem der Ansprüche 1 bis 4, wobei n 1 ist.

6. Verbindung nach Anspruch 5, wobei:
R¹ Wasserstoff ist und R² -(CH₂)ₘNR^{a}₂, -(CH₂)ₘOR^{a}, -(CH₂)ₘC(O)OR^{a}, - (CH₂)ₘC(O)NR^{a}₂, -(CH₂)ₘC(O)R^{a}, -(CH₂)ₘS(O)ₓR^{c}, eine Silyl-Gruppe, C₁-₆-Alkyl substituiert mit einer Silyl-Gruppe, =O oder =CR^{a}₂ ist; oder
R² Wasserstoff ist and R¹ -(CH₂)ₘNR^{a}₂, -(CH₂)ₘOR^{a}, -(CH₂)ₘOPG, - (CH₂)ₘC(O)OR^{a}, -(CH₂)ₘC(O)NR^{a}₂, -(CH₂)ₘC(O)R^{a}, -(CH₂)ₘS(O)ₓR^{c}, eine Silyl-Gruppe, C₁-₆-Alkyl substituiert mit einer Silyl-Gruppe, -O-Aryl, =O oder =CR^{a}₂ ist.

7. Verbindung nach einem der Ansprüche 1 bis 4, wobei n 0 ist.

8. Verbindung nach Anspruch 7, wobei einer von R¹ und R² Wasserstoff ist und der andere -(CH₂)ₘNR^{a}₂, -(CH₂)ₘOR^{a}, -(CH₂)ₘOPG, -C(O)OR^{a}, -C(O)NR^{a}₂ oder -B(OR^{b})₂ ist.

9. Verbindung nach Anspruch 1, wobei die Verbindung ausgewählt ist aus: 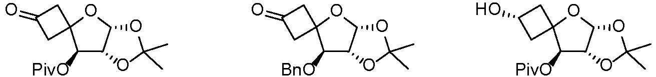 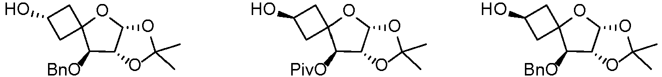 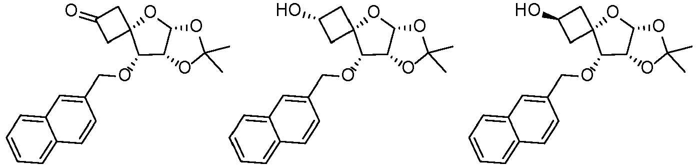 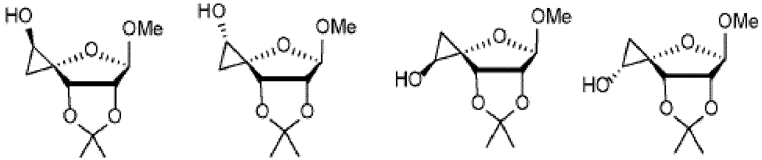 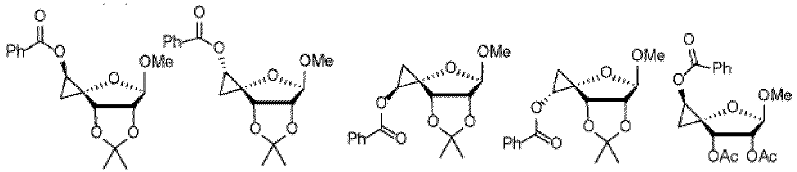 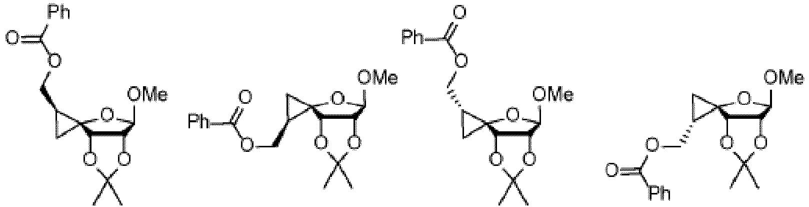 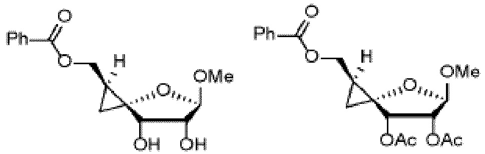 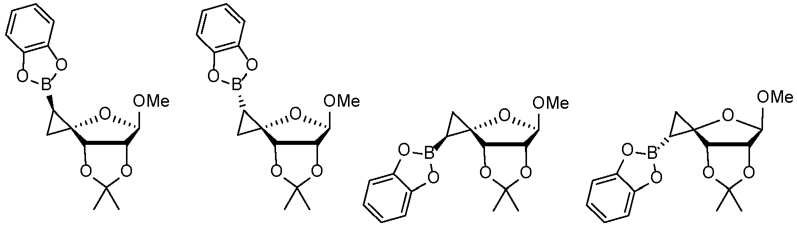 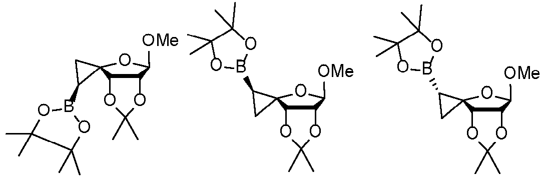 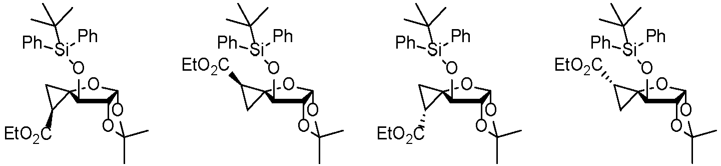 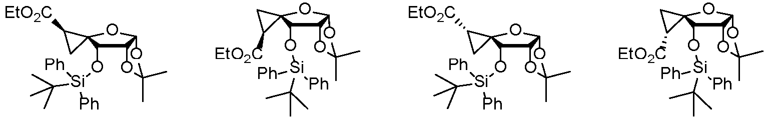 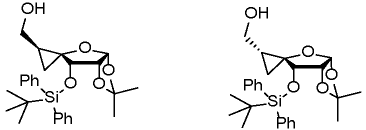 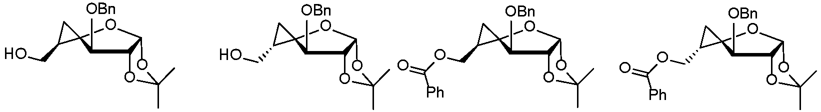 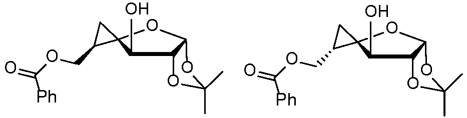 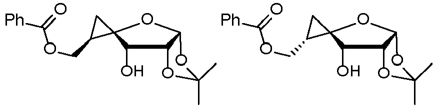 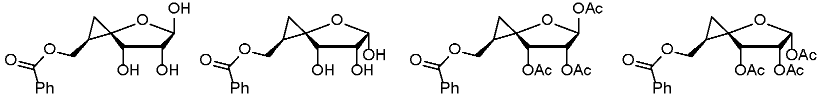 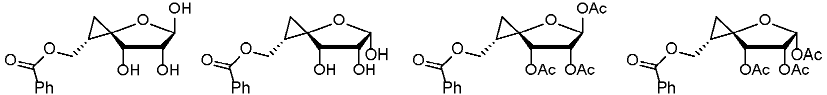 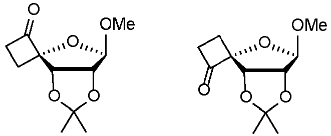 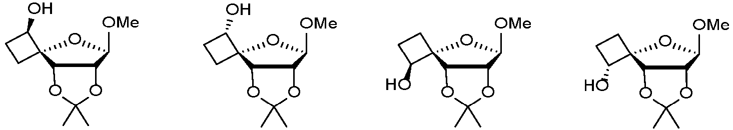 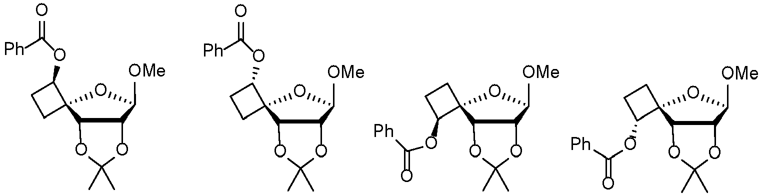 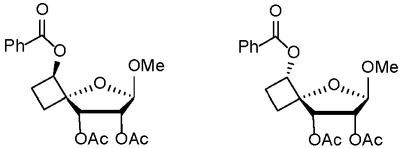 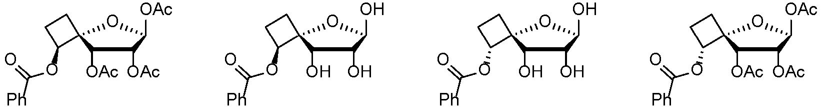 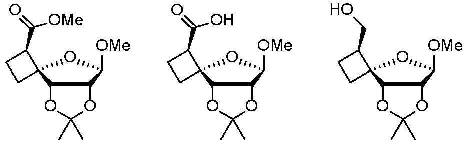 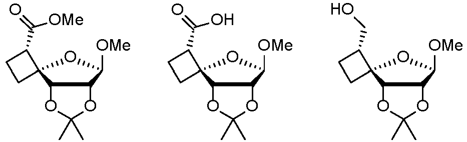 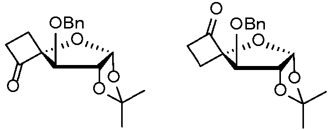 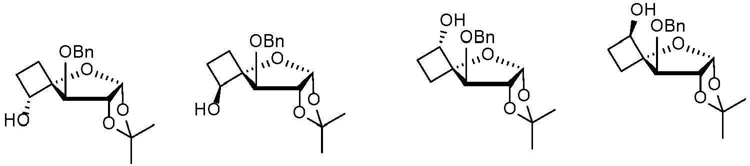 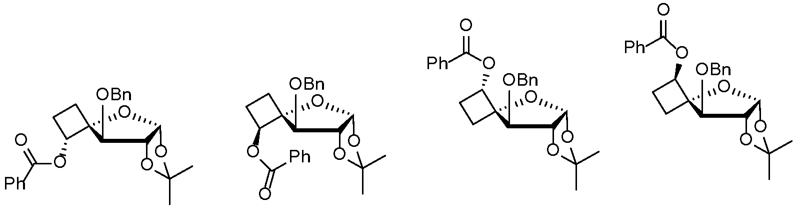 oder einem Salz oder Solvat davon.

10. Verbindung der Formel (B): 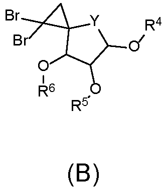 oder ein Salz oder Solvat davon, wobei:
Y -O- oder -CH₂- ist;
R⁴, R⁵ und R⁶ jeweils unabhängig Wasserstoff oder eine Hydroxyl-Schutzgruppe sind, die eine Silyl-Gruppe, C₁₋₄-Alkyl, C₁₋₄-Alkyl-O-C₁₋₄-Alkyl, Tetrahydropyranyl, Allyl, Benzyl, -CH₂ -Naphthyl, Benzoyl, -C(=O)-C₁₋₄-Alkyl oder -C(=O)-Phenyl ist; wobei Benzyl, -CH₂-Naphthyl und Benzoyl optional durch einen oder zwei Substituenten substituiert sind, die jeweils unabhängig aus -CH₃ und -OCH₃ ausgewählt sind;
oder R⁵ und R⁶ zusammen eine Diol-Schutzgruppe -C(C₁₋₄-Alkyl)₂- sind;
oder R⁴ und R⁵ zusammen eine Diol-Schutzgruppe -C(C₁₋₄-Alkyl)₂- sind.

11. Verbindung nach Anspruch 10, wobei die Verbindung ausgewählt ist aus: 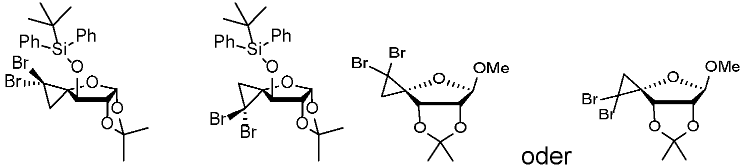 oder einem Salz oder Solvat davon.

12. Verbindung der Formel (C): 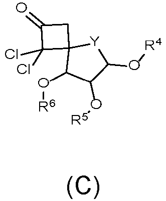 wobei:
Y -O- oder -CH₂- ist;
R⁴, R⁵ und R⁶ jeweils unabhängig Wasserstoff oder eine Hydroxil-Schutzgruppe sind, die eine Silyl-Gruppe, C₁₋₄-Alkyl, C₁₋₄-Alkyl-O-C₁₋₄-Alkyl, Tetrahydropyranyl, Allyl, Benzyl, -CH₂-Naphthyl, Benzoyl, -C(=O)-C₁₋₄-Alkyl, oder -C(=O)-Phenyl ist; wobei Benzyl, -CH₂-Naphthyl und Benzoyl optional durch einen oder zwei Substituenten substituiert sind, die jeweils unabhängig aus -CH₃ und -OCH₃ ausgewählt sind; oder
R⁴ und R⁵ zusammen eine Diol-Schutzgruppe -C(C₁₋₄-Alkyl)₂- sind; oder
R⁵ und R⁶ zusammen eine Diol-Schutzgruppe -C(C₁₋₄-Alkyl)₂- sind;
vorausgesetzt, dass die Verbindung nicht ist:

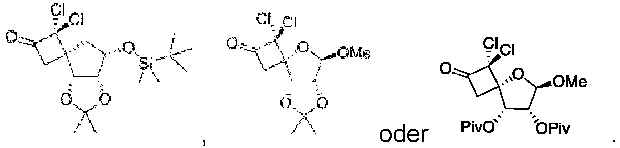

13. Verbindung nach Anspruch 12, wobei die Verbindung ausgewählt ist aus: 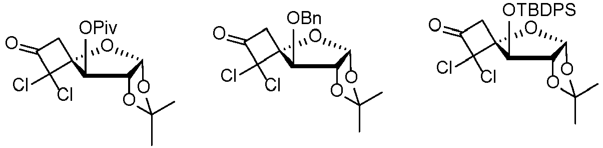 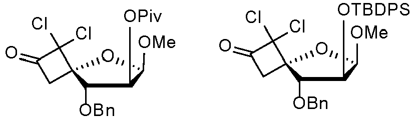 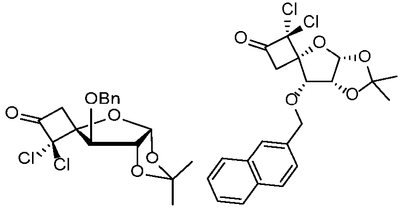 oder einem Salz oder Solvat davon.

14. Verbindung der Formel (D): 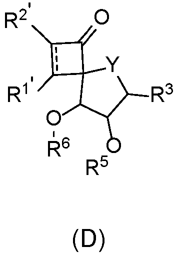 oder ein Salz oder Solvat davon, wobei:
Y -O- oder -CH₂- ist;
wobei - - eine Einfachbindung oder eine Doppelbindung ist; und
wenn - - eine Einfachbindung ist, ist einer von R¹' und R²' Wasserstoff oder C₁-₆-Alkyl und der andere ist eine Silyl-Gruppe, C₁-₆-Alkyl optional substituiert mit einer Silyl-Gruppe, -O-C₁-₆Alkyl, -B(OR^{b})₂-S-C₁-₆-Alkyl, -S-Aryl, -O-Aryl oder C(O)R^{a};
wenn - - eine Doppelbindung ist, ist einer von R¹' und R²' Wasserstoff oder C₁-₆-Alkyl und der andere ist eine Silyl-Gruppe, C₁-₆-Alkyl optional substituiert mit einer Silyl-Gruppe, -O-C₁-₆-Alkyl, oder C(O)R^{a};
und
R^{a} Wasserstoff oder C₁₋₆-Alkyl ist;
jeder R^{b} Wasserstoff ist oder beide R^{b} zusammen eine Boronsäure-Schutzgruppe bilden, nämlich Catecholboran, Pinakolboran, N-Methyliminodiessigsäureboronat, Neopentylglykolboran, Pinandiolboran, Biscyclohexyldiolboran oder 1-(4-Methoxyphenyl)-2-methylpropan-1,2-diolboran;
R³ OR⁴ oder R¹⁰ ist;
R¹⁰ eine Nukleobase oder ein Nukleobase-Derivat ist;
R⁴, R⁵ und R⁶ jeweils unabhängig Wasserstoff oder eine Hydroxil-Schutzgruppe sind, die eine Silyl-Gruppe, C₁₋₄-Alkyl, C₁₋₄-Alkyl-O-C₁₋₄-Alkyl, Tetrahydropyranyl, Allyl, Benzyl, -CH₂-Naphthyl, Benzoyl, -C(=O)-C₁₋₄-Alkyl, oder -C(=O)-Phenyl ist; wobei Benzyl, -CH₂-Naphthyl und Benzoyl optional durch einen oder zwei Substituenten substituiert sind, die jeweils unabhängig aus -CH₃ und -OCH₃ ausgewählt sind; oder
R⁴ und R⁵ zusammen eine Diol-Schutzgruppe -C(C₁₋₄-Alkyl)₂- sind; oder
R⁵ und R⁶ zusammen eine Diol-Schutzgruppe -C(C₁₋₄-Alkyl)₂- sind.

15. Verbindung nach Anspruch 14, wobei die Verbindung ausgewählt ist aus: 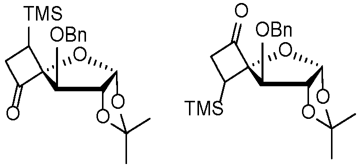 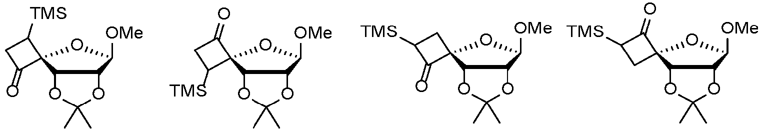 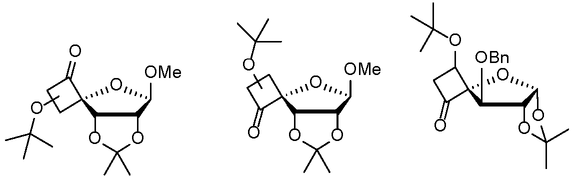 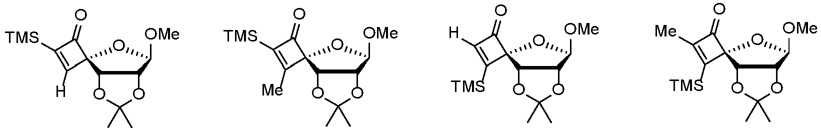 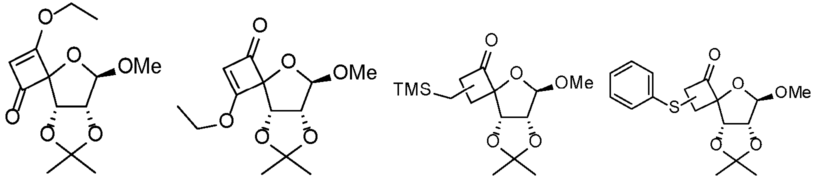 oder einem Salz oder Solvat davon.

16. Verbindung der Formel (E): 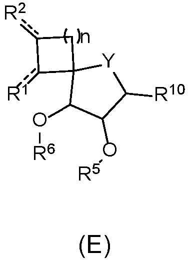 oder ein Salz oder Solvat davon, wobei:
Y -O- oder -CH₂- ist;
n 0 oder 1 ist;
wenn n 1 ist, ist R¹ Wasserstoff und R² ist -(CH₂)ₘNR^{a}₂, -(CH₂)ₘOR^{a}, - (CH₂)ₘC(O)OR^{a}, -(CH₂)ₘC(O)NR^{a}₂, -(CH₂)ₘC(O)R^{a}, -(CH₂)ₘB(OR^{b})₂, -(CH₂)ₘS(O)ₓR^{c}, eine Silyl-Gruppe, C₁-₆-Alkyl substituiert mit einer Silyl-Gruppe, =O oder =CR^{a}₂; oder
wenn n 1 ist, ist R² Wasserstoff und R¹ ist -(CH₂)ₘNR^{a}₂, -(CH₂)ₘOR^{a}, - (CH₂)ₘOPG, -(CH₂)ₘC(O)OR^{a}, -(CH₂)ₘC(O)NR^{a}₂, -(CH₂)ₘC(O)R^{a}, - (CH₂)ₘB(OR^{b})₂, -(CH₂)ₘS(O)ₓR^{c}, eine Silyl-Gruppe, C₁-₆-Alkyl, substituiert mit einer Silyl-Gruppe, -O-Aryl, =O oder =CR^{a}₂;
wenn n 0 ist, ist einer von R¹ und R² Wasserstoff und der andere ist - (CH₂)ₘNR^{a}₂, -(CH₂)ₘOR^{a}, -(CH₂)ₘOPG, -(CH₂)ₘC(O)OR^{a}, -(CH₂)ₘC(O)NR^{a}₂, - (CH2)ₘC(O)R^{a}, -(CH₂)ₘB(OR^{b})₂, -(CH₂)ₘS(O)ₓR^{c} oder =CH₂;
wobei die Bindung zu R¹ oder R² - - eine Einfachbindung ist, wenn R¹ oder R² Wasserstoff, -(CH₂)ₘNR^{a}₂, -(CH₂)ₘOR^{a}, -(CH₂)ₘOPG, -(CH₂)ₘC(O)OR^{a}, - (CH₂)mC(O)NR^{a}₂, -(CH₂)ₘC(O)R^{a}, -(CH₂)ₘB(OR^{b})₂, -(CH₂)ₘS(O)ₓR^{c}, eine Silyl-Gruppe, C₁-₆-Alkyl, substituiert mit einer Silyl-Gruppe oder -O-Aryl ist, oder eine Doppelbindung, wenn R¹ oder R² =O, =CH₂ oder =CR^{a}₂ ist;
jeder R^{a} unabhängig Wasserstoff oder C₁₋₆-Alkyl ist;
beide R^{b} Wasserstoff sind oder beide R^{b} zusammen eine Boronsäure-Schutzgruppe bilden, nämlich Catecholboran, Pinakolboran, N-Methyliminodiessigsäureboronat, Neopentylglykolboran, Pinandiolboran, Biscyclohexyldiolboran oder 1-(4-Methoxyphenyl)-2-methylpropan-1,2-diolboran;
R^{c} C₁-₆-Alkyl oder -Aryl ist;
x 0, 1 oder 2 ist;
m 0 oder 1 ist;
R⁵ und R⁶ jeweils unabhängig Wasserstoff oder eine Hydroxyl-Schutzgruppe sind, die eine Silyl-Gruppe, C₁₋₄-Alkyl, C₁₋₄-Alkyl-O-C₁₋₄-Alkyl, Tetrahydropyranyl, Allyl, Benzyl, -CH₂-Naphthyl, Benzoyl, -C(=O)-C₁₋₄-Alkyl oder -C(=O)-Phenyl ist; wobei Benzyl, -CH₂-Naphthyl und Benzoyl optional durch einen oder zwei Substituenten substituiert sind, die jeweils unabhängig aus -CH₃ und -OCH₃ ausgewählt sind; oder R⁵ und R⁶ zusammen eine Diol-Schutzgruppe -C(C₁₋₄-Alkyl)₂- sind;
R¹⁰ eine Nukleobase oder ein Nukleobase-Derivat ist;
PG eine Hydroxyl-Schutzgruppe ist, die eine Silyl-Gruppe, C₁₋₄-Alkyl, C₁₋₄-Alkyl-O-C₁₋₄-Alkyl, Tetrahydropyranyl, Allyl, Benzyl, -CH₂-Naphthyl,
Benzoyl, -C(=O)-C₁₋₄-Alkyl oder -C(=O)-Phenyl ist; wobei Benzyl, -CH₂-Naphthyl und Benzoyl optional durch einen oder zwei Substituenten substituiert sind, die jeweils unabhängig aus -CH₃ und -OCH₃ ausgewählt sind;
vorausgesetzt, dass die Verbindung keine der folgenden ist: 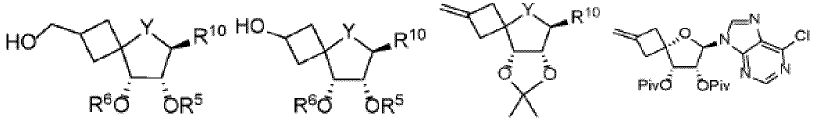 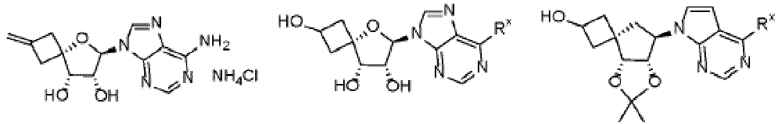
wobei
R⁵ Wasserstoff oder -C(=O)-C₁₋₄-Alkyl ist;
R⁶ Wasserstoff oder -C(=O)-C₁₋₄-Alkyl ist; oder R⁵ und R⁶ zusammen -C(CH₃)₂- sind
Y -O- oder -CH₂- ist;
R¹⁰ ist 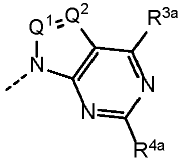
Q¹ CR^{6a} ist;
Q² N oder CR^{6b} ist;
R^{6a} und R^{6b} jeweils unabhängig für Wasserstoff, Halogen, C₁₋₄-Alkyl, - NR^{9a}R^{9b} oder C₁₋₄-Alkyl stehen, das mit einem, zwei oder drei Halogenatomen substituiert ist;
R^{9a} und R^{9b} jeweils unabhängig für Wasserstoff oder C₁₋₄-Alkyl stehen;
R^{3a} Wasserstoff, Halogen, -NR^{7a}R^{7b}, C₁₋₄-Alkyl, C₂₋₄-Alkenyl, C₃₋₆-Cycloalkyl, -OH oder
-O-C₁₋₄-Alkyl ist;
R^{7a} Wasserstoff ist;
R^{7b} Wasserstoff, C₃₋₆-Cycloalkyl oder C₁₋₄-Alkyl ist;
R^{4a} Wasserstoff, Halogen, -NR^{8a}R^{8b} oder C₁₋₄-Alkyl ist; und
R^{x} 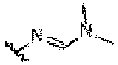 ist;
oder ein Salz oder Solvat davon.

17. Verbindung nach Anspruch 16, wobei die Verbindung eine Verbindung der Formel (G-1a) oder (G-1b) ist: 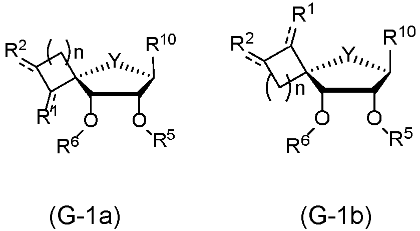 oder ein Salz oder Solvat davon.

18. Verbindung nach einem der Ansprüche 16 oder 17, wobei n 1 ist.

19. Verbindung nach Anspruch 18, wobei:
R¹ Wasserstoff ist und R² -(CH₂)ₘNR^{a}₂, -(CH₂)ₘOR^{a}, -(CH₂)ₘC(O)OR^{a}, - (CH₂)mC(O)NR^{a}₂, -(CH₂)ₘC(O)R^{a}, -(CH₂)ₘS(O)ₓR^{c}, eine Silyl-Gruppe, C₁-₆-Alkyl substituiert mit einer Silyl-Gruppe, =O oder =CR^{a}₂ ist; oder
R² Wasserstoff ist and R¹ -(CH₂)ₘNR^{a}₂, -(CH₂)ₘOR^{a}, -(CH₂)ₘOPG, - (CH₂)ₘC(O)OR^{a}, -(CH₂)ₘC(O)NR^{a}₂, -(CH₂)ₘC(O)R^{a}, -(CH₂)ₘS(O)ₓR^{c}, eine Silyl-Gruppe, C₁-₆-Alkyl substituiert mit einer Silyl-Gruppe, -O-Aryl, =O oder =CR^{a}₂ ist.

20. Verbindung nach einem der Ansprüche 16 oder 17, wobei n 0 ist.

21. Verbindung nach Anspruch 18, wobei einer von R¹ und R² Wasserstoff ist und der andere -(CH₂)ₘNR^{a}₂, -(CH₂)ₘOR^{a}, -(CH₂)ₘOPG, -(CH₂)ₘC(O)OR^{a}, - (CH₂)mC(O)NR^{a}₂, -(CH₂)ₘC(O)R^{a}, -(CH₂)ₘB(OR^{b})₂ oder =CH₂ ist.

22. Verbindung nach Anspruch 16, wobei die Verbindung ausgewählt ist aus: 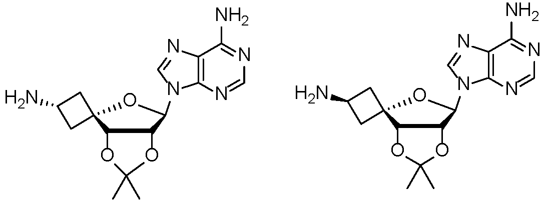 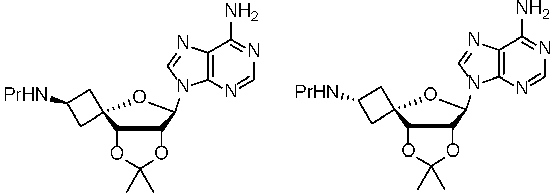 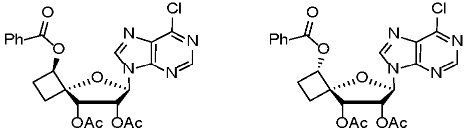 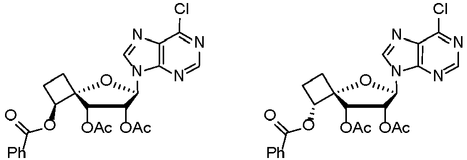 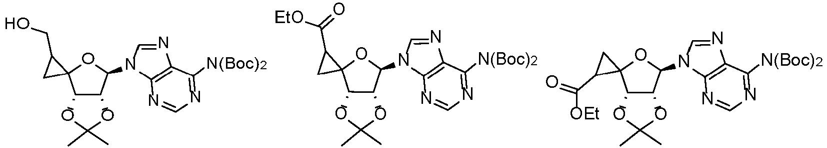 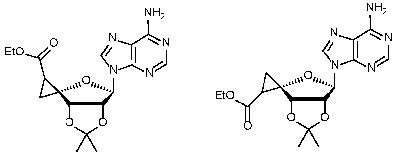 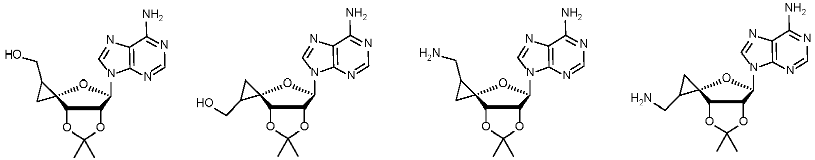 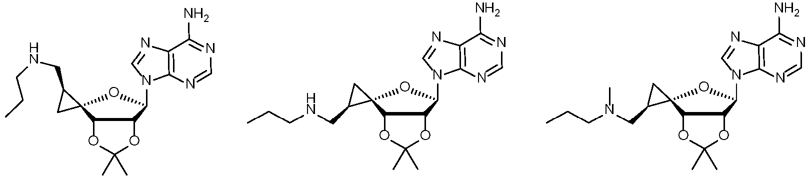 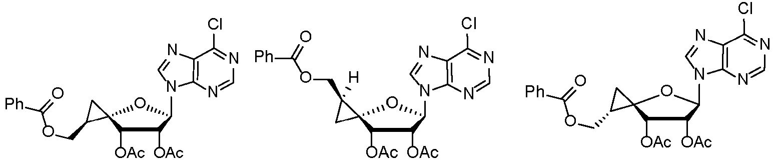 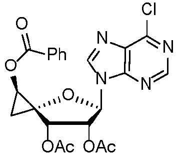 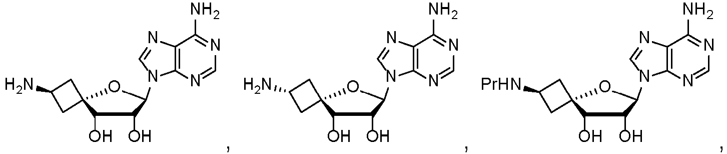 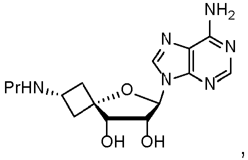 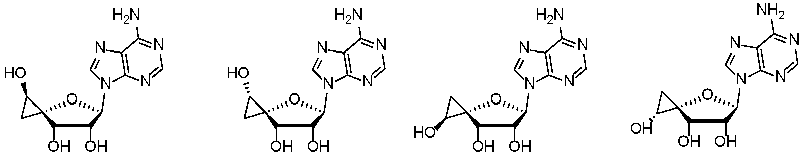 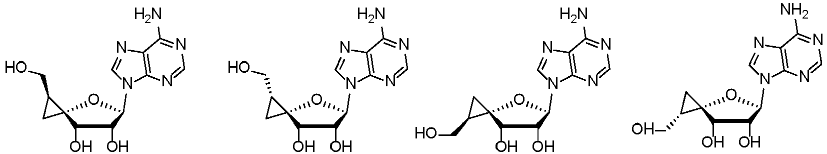 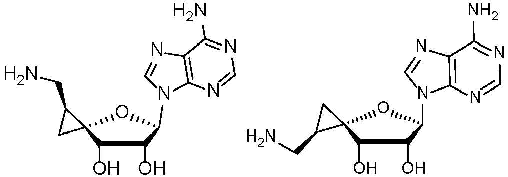 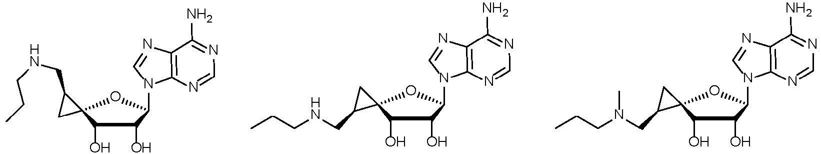 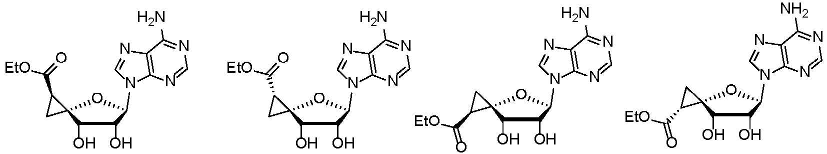 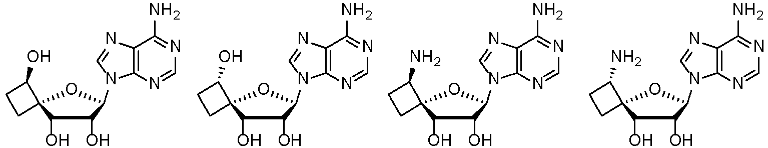 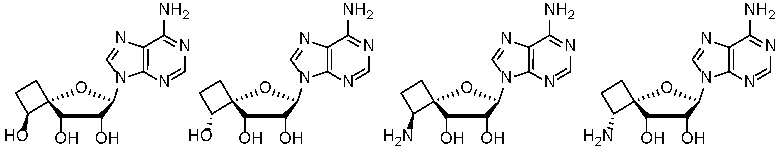 oder einem Salz oder Solvat davon.

23. Verbindung der Formel (H): 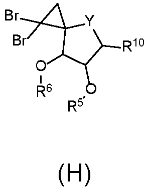 oder ein Salz oder Solvat davon, wobei:
Y -O- oder -CH₂- ist;
R⁵ und R⁶ jeweils unabhängig Wasserstoff oder eine Hydroxyl-Schutzgruppe sind, die eine Silyl-Gruppe, C₁₋₄-Alkyl, C₁₋₄-Alkyl-O-C₁₋₄-Alkyl, Tetrahydropyranyl, Allyl, Benzyl, -CH₂-Naphthyl, Benzoyl, -C(=O)-C₁₋₄-Alkyl oder -C(=O)-Phenyl ist; wobei Benzyl, -CH₂-Naphthyl und Benzoyl optional durch einen oder zwei Substituenten substituiert sind, die jeweils unabhängig aus -CH₃ und -OCH₃ ausgewählt sind; oder R⁵ und R⁶ zusammen eine Diol-Schutzgruppe -C(C₁₋₄-Alkyl)₂- sind; und R¹⁰ eine Nukleobase oder ein Nukleobase-Derivat ist.

24. Verbindung nach einem der Ansprüche 14 bis 23, wobei R¹⁰, sofern vorhanden, Cytosin, Thymin, Uracil oder eine modifizierte oder geschützte Form davon oder ein bicyclisches aromatisches heterocyclisches Ringsystem ist, ausgewählt aus der Gruppe bestehend aus (a-1), (a-2), (a-3), (a-4) und (a-5): 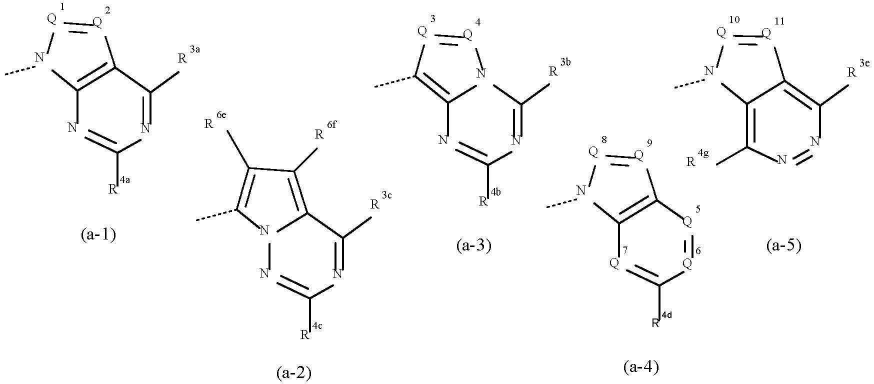
R^{3a}, R^{3b}, R^{3c}, R^{3d} und R^{3e} jeweils unabhängig Wasserstoff, Halogen, -NR^{7a}R^{7b}, C₁₋₄-Alkyl, C₂₋₄-Alkenyl, C₃₋₆-Cycloalkyl, -OH oder -O-C₁₋₄-Alkyl sind;
R^{7a} Wasserstoff, -C(O)O-C₁₋₄-Alkyl oder 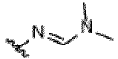 ist;
R^{7b} Wasserstoff, C₃₋₆-Cycloalkyl, C₁₋₄-Alkyl oder -C(O)O-C₁₋₄-Alkyl ist;
R^{4a}, R^{4b}, R^{4c}, R^{4d}, R^{4e}, R^{4f} und R^{4g} jeweils unabhängig Wasserstoff, Halogen, -NR^{8a}R^{8b} oder C₁₋₄-Alkyl sind;
R^{8a} und R^{8b} jeweils unabhängig Wasserstoff oder C₁₋₄-Alkyl sind;
Q¹ N oder CR^{6a} ist;
Q² N oder CR^{6b} ist;
Q³ N oder CR^{6c} ist;
Q⁴ N oder CR^{6d} ist;
mit der Maßgabe, dass maximal eines von Q³ und Q⁴ N ist;
Q⁸ N oder CR^{6g} ist;
Q⁹ N oder CR^{6h} ist;
Q¹⁰ N oder CR⁶ⁱ ist;
Q¹¹ N oder CR^{6j} ist;
Q⁵ CR^{3d} ist; Q⁶ N ist; und Q⁷ CR^{4f} ist; oder
Q⁵ CR^{3d} ist; Q⁶ CR^{4e} ist; und Q⁷ N ist; oder
Q⁵ N ist; Q⁶ CR^{4e} ist; und Q⁷ CR^{4f} ist; oder
Q⁵ N ist; Q⁶ CR^{4e} ist; und Q⁷ N ist; oder
Q⁵ N ist; Q⁶ N ist; und Q⁷ CR^{4f} ist; oder
Q⁵ N ist; Q⁶ N ist; und Q⁷ N ist;
R^{6a}, R^{6b}, R^{6c}, R^{6d}, R^{6e}, R^{6f}, R^{6 g}, R^{6h}, R⁶ⁱ und R^{6j} jeweils unabhängig Wasserstoff, Halogen, C₁₋₄-Alkyl, -NR^{9a}R^{9b} oder C₁₋₄-Alkyl sind, das durch ein, zwei oder drei Halogenatome substituiert ist;
R^{9a} und R^{9b} jeweils unabhängig Wasserstoff oder C₁₋₄-Alkyl sind.

## Revendications

1. Composé de Formule (A) 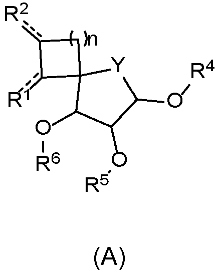 ou un sel ou solvate de celui-ci, dans lequel :
Y est -O- ou -CH₂- ;
n vaut 0 ou 1 ;
lorsque n vaut 1, R¹ est hydrogène et R² est -(CH₂)ₘNR^{a}₂, -(CH₂)ₘOR^{a}, - (CH₂)ₘC(O)OR^{a}, -(CH₂)ₘC(O)NR^{a}₂, -(CH₂)ₘC(O)R^{a}, -(CH₂)ₘB(OR^{b})₂, - (CH₂)ₘS(O)ₓR^{c}, un groupe silyle, C₁-₆ alkyle substitué par un groupe silyle, =O ou =CR^{a}₂ ; ou
lorsque n vaut 1, R² est un hydrogène et R¹ est -(CH₂)ₘNR^{a}₂, -(CH₂)ₘOR^{a}, -(CH₂)ₘOPG, -(CH₂)ₘC(O)OR^{a}, -(CH₂)ₘC(O)NR^{a}₂, -(CH₂)ₘC(O)R^{a}, - (CH₂)ₘB(OR^{b})₂, -(CH₂)ₘS(O)ₓR^{c}, un groupe silyle, C₁₋₆ alkyle substitué par un groupe silyle, -O-aryle, =O ou =CR^{a}₂ ;
lorsque n vaut 0, l'un de R¹ et R² est hydrogène et l'autre est - (CH₂)ₘNR^{a}₂,-(CH₂)ₘOR^{a}, -(CH₂)ₘOPG, -(CH₂)ₘC(O)OR^{a}, -(CH₂)ₘC(O)NR^{a}₂, - (CH2)ₘC(O)R^{a}, -(CH₂)ₘB(OR^{b})₂, -(CH₂)ₘS(O)ₓR^{c} ou =CH₂ ;
dans lequel la liaison vers R¹ ou R² - - est une liaison simple lorsque R¹ ou R² est hydrogène, -(CH₂)ₘNR^{a}₂, -(CH₂)ₘOR^{a}, -(CH₂)ₘOPG, -(CH₂)ₘC(O)OR^{a}, - (CH₂)mC(O)NR^{a}₂, -(CH₂)ₘC(O)R^{a}, -(CH₂)ₘB(OR^{b})₂, -(CH₂)ₘS(O)ₓR^{c}, un groupe silyle, C₁₋₆ alkyle substitué par un groupe silyle ou -O-aryle, ou une double liaison lorsque R¹ ou R² est =O, =CH₂ ou =CR^{a}₂ ;
chaque R^{a} est, indépendamment, hydrogène ou C₁₋₆ alkyle ;
chaque R^{b} est hydrogène ou les deux R^{b} sont pris conjointement pour former un groupe protecteur d'acide boronique qui est catéchol borane, pinacol borane, boronate d'acide N-méthyliminodiacétique, néopentylglycol borane, pinanediol borane, biscyclohexyldiol borane, ou 1-(4-méthoxyphényl)-2-méthylpropane-1,2-diol borane ;
R^{c} est C₁₋₆ alkyle ou aryle ;
PG est un groupe protecteur d'hydroxyle qui est un groupe silyle, C₁₋₄alkyle, C₁₋₄alkyl-O-C₁₋₄alkyle, tétrahydropyranyle, allyle, benzyle, -CH₂-naphtyle, benzoyle, -C(=O)-C₁₋₄alkyle, ou -C(=O)-phényle ; dans lequel benzyle, -CH₂-naphtyle, et benzoyle sont facultativement substitués par un ou deux substituants choisis indépendamment parmi -CH₃ et -OCH₃ ;
m vaut 0 ou 1 ;
x vaut 0, 1 ou 2 ;
R⁴, R⁵ et R⁶ sont chacun indépendamment hydrogène ou un groupe protecteur d'hydroxyle qui est un groupe silyle, C₁₋₄alkyle, C₁₋₄alkyl-O-C₁₋₄alkyle, tétrahydropyranyle, allyle, benzyle, -CH₂-naphtyle, benzoyle, -C(=O)-C₁₋₄alkyle, ou -C(=O)-phényle ; dans lequel benzyle, -CH₂-naphtyle, et benzoyle sont facultativement substitués par un ou deux substituants choisis indépendamment parmi -CH₃ et -OCH₃ ;
ou R⁵ et R⁶ pris conjointement sont un groupe protecteur de diol -C(C₁₋₄alkyl)₂- ;
ou R⁴ et R⁵ pris conjointement sont un groupe protecteur de diol -C(C₁₋₄alkyl)₂- ;
à condition que :
(a) le composé ne soit pas un composé de formule : 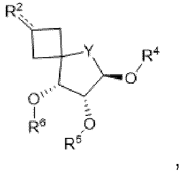 dans lequel Y est -O- ou -CH₂- ; R⁴ est hydrogène ou un groupe protecteur d'hydroxyle qui est C₁₋₄alkyle, t-butyldiméthylsilyle, C₁₋₄alkyl-O-C₁₋₄alkyle, tétrahydropyranyle, allyle, t-butyldiphénylsilyle, benzyle, -C(=O)-C₁₋₄alkyle, ou -C(=O)-phényle ; R² est -OH, =O, -CH₂-OH ou =CH₂ ; et R⁵ et R⁶ sont chacun indépendamment -C(=O)-C₁₋₄alkyle, benzoyle, benzyle, ou -CH₂-naphtyle, dans lequel benzyle, -CH₂-naphtyle, et benzoyle sont facultativement substitués par un ou deux substituants chacun choisis indépendamment parmi -CH₃ et - OCH₃ ; ou R⁵ et R⁶ pris ensemble sont -C(C₁₋₄alkyl)₂- ; ou dans lequel Y est -O- ou -CH₂, R⁴ est méthyle, R² est =O, ou =CH₂, et R⁵ et R⁶ sont chacun indépendamment hydrogène ou -C(=O)-C₁₋₄alkyle ; et
(b) le composé ne soit pas un composé de formule : 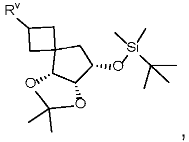 dans lequel R^{v} est =O, -OH ou =CH₂ ; et
(c) le composé ne soit pas un composé de formule : 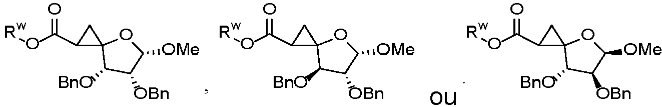 dans lequel R^{w} est méthyle ou H ; et
(d) le composé ne soit pas un composé de formule : 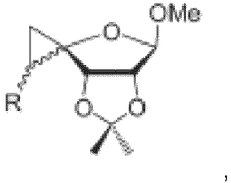 dans lequel R est CO₂Et ou CH₂OH ou tout stéréoisomère de ceux-ci.

2. Composé selon la revendication 1, dans lequel le composé est un composé de formule (A-1a), (A-3a), (A-1b) ou (A-3b) : 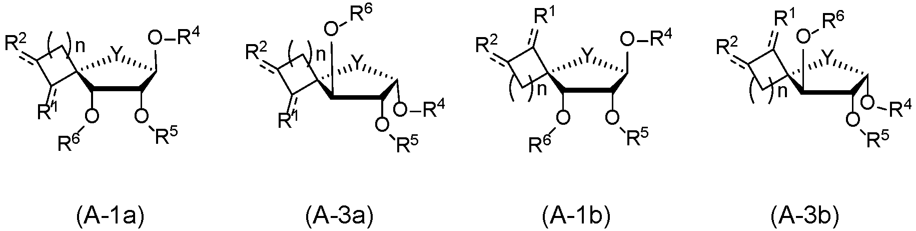 ou un sel ou solvate de celui-ci.

3. Composé selon l'une quelconque des revendications 1 à 2, dans lequel Y est O.

4. Composé selon l'une quelconque des revendications 1 à 2, dans lequel Y est CH₂.

5. Composé selon l'une quelconque des revendications 1 à 4, dans lequel n vaut 1.

6. Composé selon la revendication 5, dans lequel :
R¹ est hydrogène et R² -(CH₂)ₘNR^{a}₂, -(CH₂)ₘOR^{a}, -(CH₂)ₘC(O)OR^{a}, - (CH₂)mC(O)NR^{a}₂, -(CH₂)ₘC(O)R^{a}, -(CH₂)ₘS(O)ₓR^{c}, un groupe silyle, C₁₋₆alkyle substitué par un groupe silyle, =O ou =CR^{a}₂ ; ou
R² est hydrogène et R¹ est -(CH₂)ₘNR^{a}₂, -(CH₂)ₘOR^{a}, -(CH₂)ₘOPG, - (CH₂)ₘC(O)OR^{a}, -(CH₂)ₘC(O)NR^{a}₂, -(CH₂)ₘC(O)R^{a}, -(CH₂)ₘS(O)ₓR^{c}, un groupe silyle, C₁₋₆alkyle substitué par un groupe silyle, -O-aryle, =O ou =CR^{a}₂.

7. Composé selon l'une quelconque des revendications 1 à 4, dans lequel n vaut 0.

8. Composé selon la revendication 7, dans lequel l'un de R¹ et R² est hydrogène et l'autre est -(CH₂)ₘNR^{a}₂, -(CH₂)ₘOR^{a}, -(CH₂)ₘOPG, -C(O)OR^{a}, - C(O)NR^{a}₂ or -B(OR^{b})₂.

9. Composé selon la revendication 1, dans lequel le composé est choisi parmi : 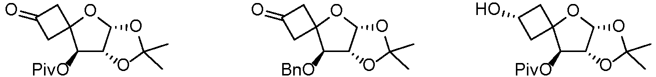 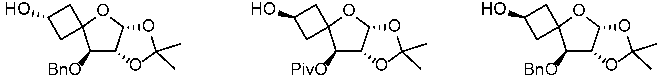 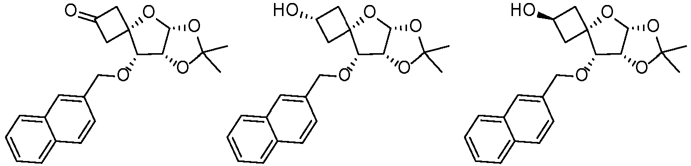 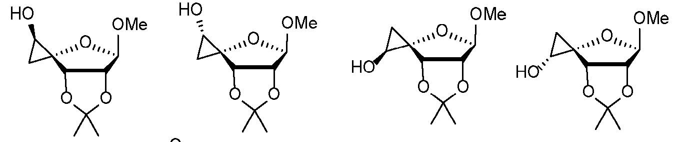 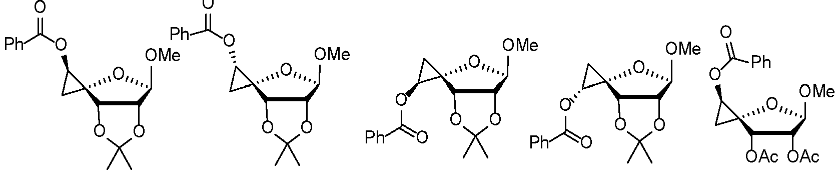 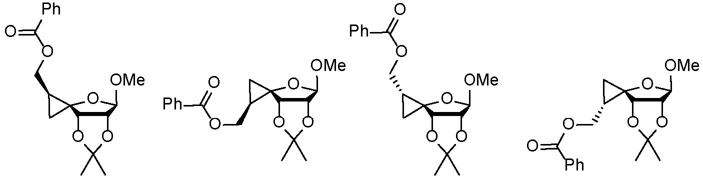 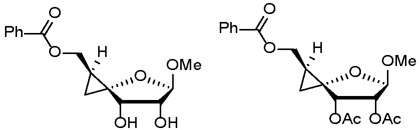 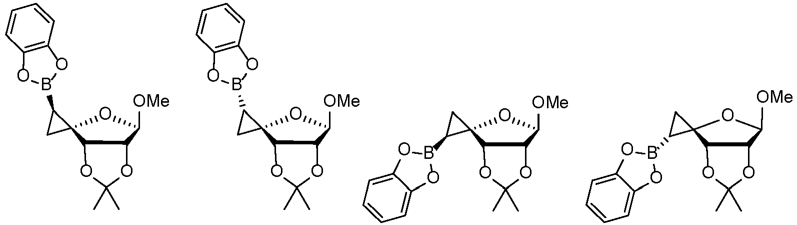 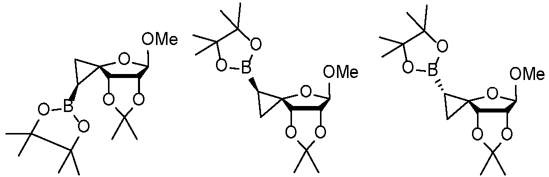 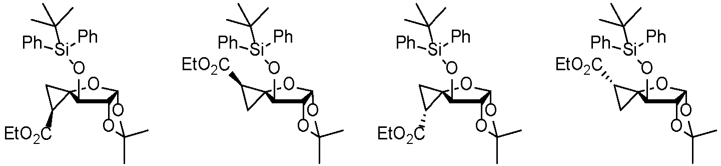 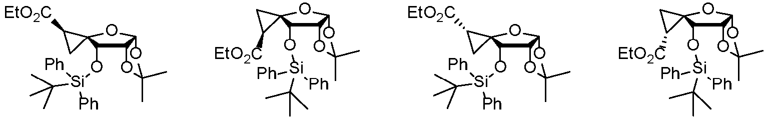 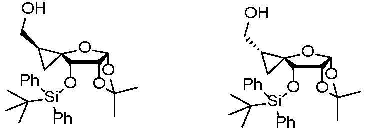 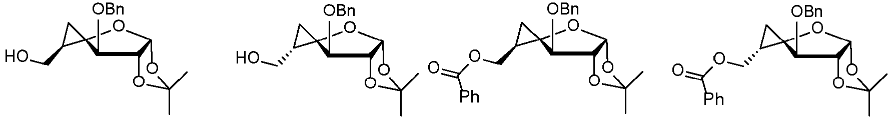 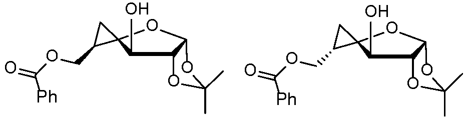 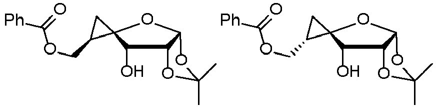 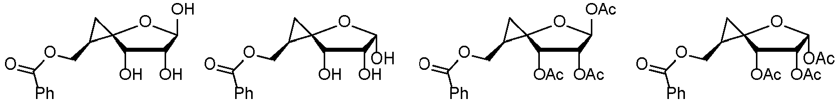 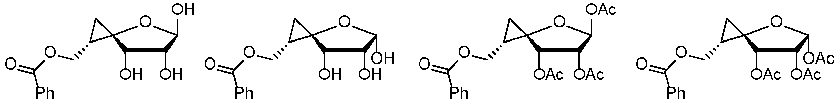 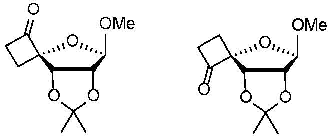 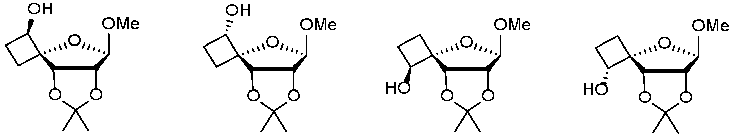 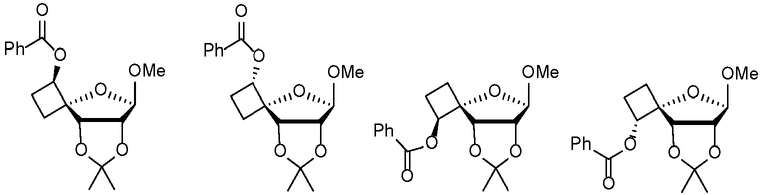 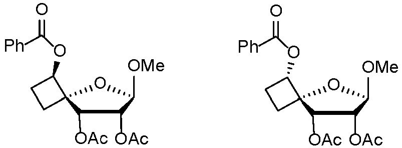 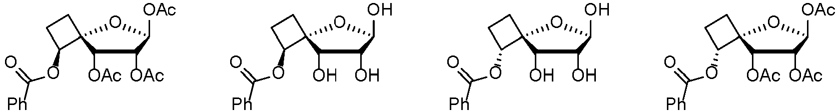 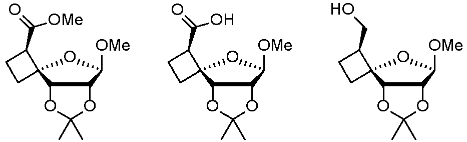 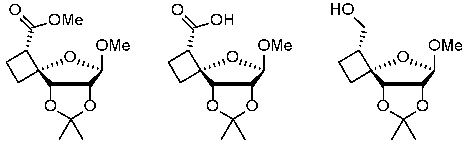 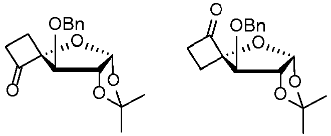 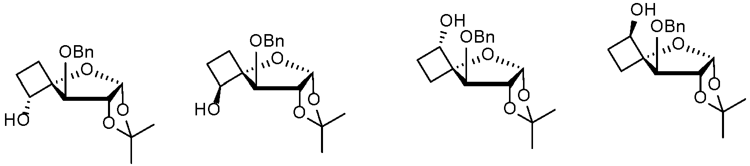 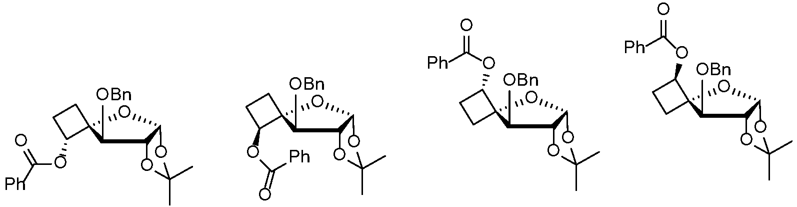 ou un sel ou solvate de celui-ci.

10. Composé de formule (B) : 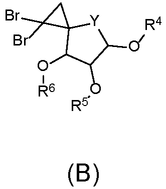 ou un sel ou solvate de celui-ci, dans lequel :
Y est -O- ou -CH₂- ;
R⁴, R⁵ et R⁶ sont chacun, indépendamment, hydrogène ou un groupe protecteur d'hydroxyle qui est un groupe silyle, C₁₋₄alkyle, C₁₋₄alkyl-O-C₁₋₄alkyle, tétrahydropyranyle, allyle, benzyle, -CH₂-naphtyle, benzoyle, -C(=O)-C₁₋₄alkyle, ou -C(=O)-phényle ; dans lequel benzyle, -CH₂-naphtyle, et benzoyle sont facultativement substitués par un ou deux substituants choisis indépendamment parmi -CH₃ et -OCH₃ ;
ou R⁵ et R⁶ pris conjointement sont un groupe protecteur de diol -C(C₁₋₄alkyl)₂- ;
ou R⁴ et R⁵ pris conjointement sont un groupe protecteur de diol -C(C₁₋₄alkyl)₂-.

11. Composé selon la revendication 10, dans lequel le composé est choisi parmi : 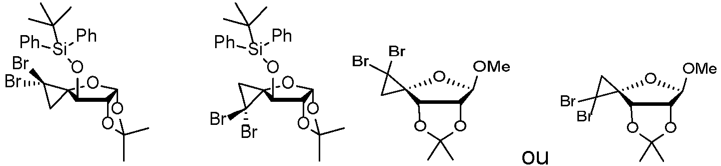 ou un sel ou solvate de celui-ci.

12. Composé de formule (C) : 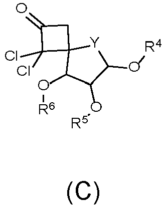 dans lequel :
Y est -O- ou -CH₂- ;
R⁴, R⁵ et R⁶ sont chacun, indépendamment, hydrogène ou un groupe protecteur d'hydroxyle qui est un groupe silyle, C₁₋₄alkyle, C₁₋₄alkyl-O-C₁₋₄alkyle, tétrahydropyranyle, allyle, benzyle, -CH₂-naphtyle, benzoyle, -C(=O)-C₁₋₄alkyle, ou -C(=O)-phényle ; dans lequel benzyle, -CH₂-naphtyle, et benzoyle sont facultativement substitués par un ou deux substituants choisis indépendamment parmi -CH₃ et -OCH₃ ; ou
R⁴ et R⁵ pris conjointement sont un groupe protecteur de diol -C(C₁₋₄alkyl)₂- ; ou
R⁵ et R⁶ pris conjointement sont un groupe protecteur de diol -C(C₁₋₄alkyl)₂- ;
à condition que le composé ne soit pas :

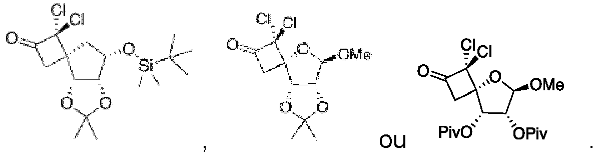

13. Composé selon la revendication 12, dans lequel le composé est choisi parmi : 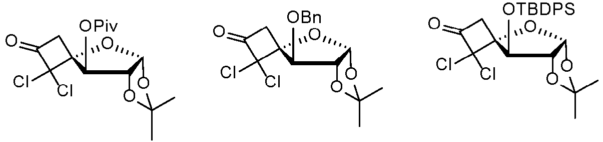 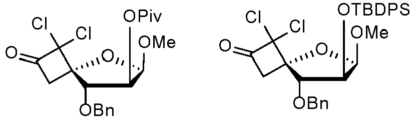 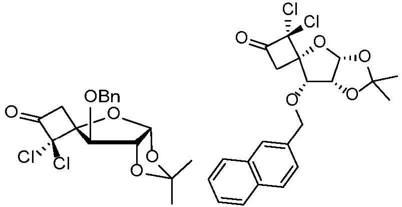 ou un sel ou solvate de celui-ci.

14. Composé de formule (D) : 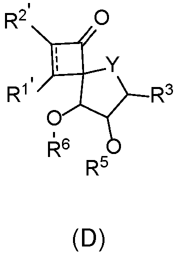 ou un sel ou solvate de celui-ci, dans lequel :
Y est -O- ou -CH₂- ;
dans lequel - - est une liaison simple ou une double liaison ; et
lorsque - - est une liaison simple, l'un de R¹' et R²' est hydrogène ou C₁₋₆ alkyle et l'autre est un groupe silyle, C₁₋₆ alkyle facultativement substitué par un groupe silyle, -O-C₁₋₆ alkyle, -B(OR^{b})₂ -S-C₁₋₆ alkyle, -S-aryle, -O-aryle ou C(O)R^{a} ;
lorsque - - est une double liaison, l'un de R¹' et R²' est hydrogène ou C₁-₆ alkyle et l'autre est un groupe silyle, C₁-₆ alkyle facultativement substitué par un groupe silyle, -O-C₁₋₆ alkyle, , ou C(O)R^{a} ;
et
R^{a} est hydrogène ou C₁₋₆ alkyle ;
chaque R^{b} est hydrogène ou les deux R^{b} sont pris conjointement pour former un groupe protecteur d'acide boronique qui est catéchol borane, pinacol borane, boronate d'acide N-méthyliminodiacétique, néopentylglycol borane, pinanediol borane, biscyclohexyldiol borane, ou 1-(4-méthoxyphényl)-2-méthylpropane-1,2-diol borane ;
R³ est OR⁴ ou R¹⁰ ;
R¹⁰ est une nucléobase ou un dérivé de nucléobase ;
R⁴, R⁵ et R⁶ sont chacun, indépendamment, hydrogène ou un groupe protecteur d'hydroxyle qui est un groupe silyle, C₁₋₄alkyle, C₁₋₄alkyl-O-C₁₋₄alkyle, tétrahydropyranyle, allyle, benzyle, -CH₂-naphtyle, benzoyle, -C(=O)-C₁₋₄alkyle, ou -C(=O)-phényle ; dans lequel benzyle, -CH₂-naphtyle, et benzoyle sont facultativement substitués par un ou deux substituants choisis indépendamment parmi -CH₃ et -OCH₃ ; ou
R⁴ et R⁵ pris conjointement sont un groupe protecteur de diol -C(C₁₋₄alkyl)₂- ; ou
R⁵ et R⁶ pris conjointement sont un groupe protecteur de diol -C(C₁₋₄alkyl)₂-.

15. Composé selon la revendication 14, dans lequel le composé est choisi parmi : 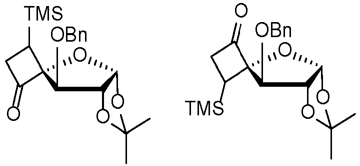 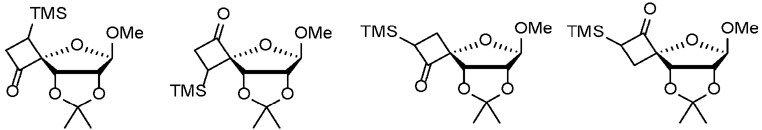 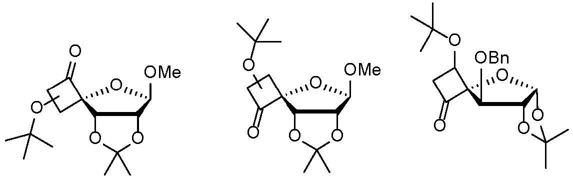 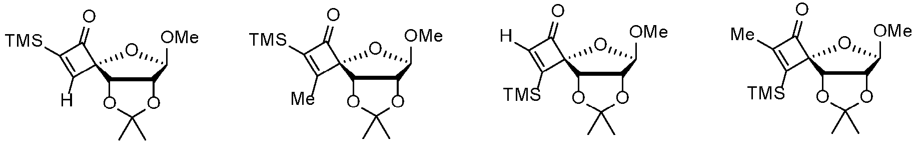 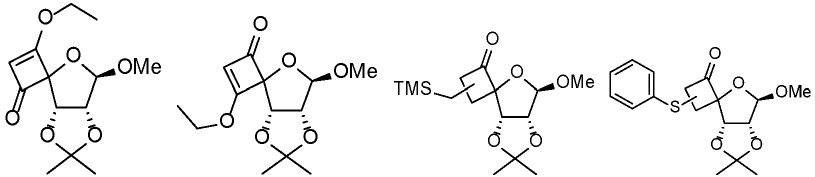 ou un sel ou solvate de celui-ci.

16. Composé de formule (E) : 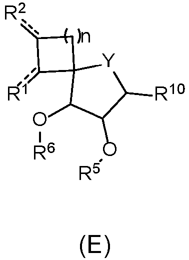 ou un sel ou solvate de celui-ci, dans lequel :
Y est -O- ou -CH₂- ;
n vaut 0 ou 1 ;
lorsque n vaut 1, R¹ est hydrogène et R² est -(CH₂)ₘNR^{a}₂, -(CH₂)ₘOR^{a}, - (CH₂)ₘC(O)OR^{a}, -(CH₂)ₘC(O)NR^{a}₂, -(CH₂)ₘC(O)R^{a}, -(CH₂)ₘB(OR^{b})₂, - (CH₂)ₘS(O)ₓR^{c}, un groupe silyle, C₁-₆ alkyle substitué par un groupe silyle, =O ou =CR^{a}₂ ; ou
lorsque n vaut 1, R² est un hydrogène et R¹ est -(CH₂)ₘNR^{a}₂, -(CH₂)ₘOR^{a}, -(CH₂)ₘOPG, -(CH₂)ₘC(O)OR^{a}, -(CH₂)ₘC(O)NR^{a}₂, -(CH₂)ₘC(O)R^{a}, - (CH₂)ₘB(OR^{b})₂, -(CH₂)ₘS(O)ₓR^{c}, un groupe silyle, C₁₋₆ alkyle substitué par un groupe silyle, -O-aryle, =O ou =CR^{a}₂ ;
lorsque n vaut 0, l'un de R¹ et R² est hydrogène et l'autre est - (CH₂)ₘNR^{a}₂,-(CH₂)ₘOR^{a}, -(CH₂)ₘOPG, -(CH₂)ₘC(O)OR^{a}, -(CH₂)ₘC(O)NR^{a}₂, - (CH2)ₘC(O)R^{a}, -(CH₂)ₘB(OR^{b})₂, -(CH₂)ₘS(O)ₓR^{c} ou =CH₂ ;
dans lequel la liaison vers R¹ ou R² - - est une liaison simple lorsque R¹ ou R² est hydrogène, -(CH₂)ₘNR^{a}₂, -(CH₂)ₘOR^{a}, -(CH₂)ₘOPG, -(CH₂)ₘC(O)OR^{a}, - (CH₂)mC(O)NR^{a}₂, -(CH₂)ₘC(O)R^{a}, -(CH₂)ₘB(OR^{b})₂, -(CH₂)ₘS(O)ₓR^{c}, un groupe silyle, C₁₋₆ alkyle substitué par un groupe silyle ou -O-aryle, ou une double liaison lorsque R¹ ou R² est =O, =CH₂ ou =CR^{a}₂ ;
chaque R^{a} est, indépendamment, hydrogène ou C₁₋₆ alkyle ;
les deux R^{b} sont hydrogène ou les deux R^{b} sont pris conjointement pour former un groupe protecteur d'acide boronique qui est catéchol borane, pinacol borane, boronate d'acide N-méthyliminodiacétique, néopentylglycol borane, pinanediol borane, biscyclohexyldiol borane, ou 1-(4-méthoxyphényl)-2-méthylpropane-1,2-diol borane ;
R^{c} est C₁₋₆ alkyle ou aryle ;
x vaut 0, 1 ou 2 ;
m vaut 0 ou 1 ;
R⁵ et R⁶ sont chacun, indépendamment, hydrogène ou un groupe protecteur d'hydroxyle qui est un groupe silyle, C₁₋₄alkyle, C₁₋₄alkyl-O-C₁₋₄alkyle, tétrahydropyranyle, allyle, benzyle, -CH₂-naphtyle, benzoyle, -C(=O)-C₁₋₄alkyle, ou -C(=O)-phényle ; dans lequel benzyle, -CH₂-naphtyle, et benzoyle sont facultativement substitués par un ou deux substituants choisis indépendamment parmi -CH₃ et -OCH₃ ; ou R⁵ et R⁶ pris conjointement sont un groupe protecteur de diol -C(C₁₋₄alkyl)₂- ;
R¹⁰ est une nucléobase ou un dérivé de nucléobase ;
PG est un groupe protecteur d'hydroxyle qui est un groupe silyle, C₁₋₄alkyle, C₁₋₄alkyl-O-C₁₋₄alkyle, tétrahydropyranyle, allyle, benzyle, -CH₂-naphtyle, benzoyle, -C(=O)-C₁₋₄alkyle, ou -C(=O)-phényle ; dans lequel benzyle, -CH₂-naphtyle, et benzoyle sont facultativement substitués par un ou deux substituants choisis indépendamment parmi -CH₃ et -OCH₃ ;
à condition que le composé ne soit pas l'un quelconque parmi : 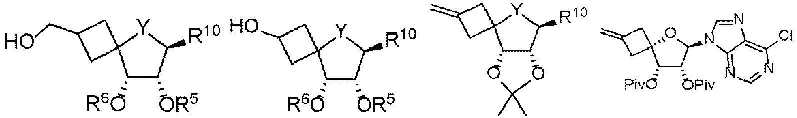 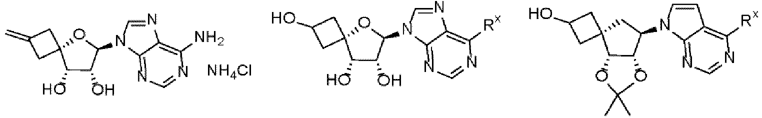
dans lequel
R⁵ est hydrogène ou -C(=O)-C₁₋₄alkyle ;
R⁶ est hydrogène ou -C(=O)-C₁₋₄alkyle ; ou R⁵ et R⁶ pris conjointement sont -C(CH₃)₂-
Y est -O- ou -CH₂- ;
R¹⁰ est 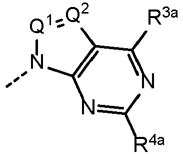
Q¹ est CR^{6a} ;
Q² est N ou CR^{6b} ;
R^{6a} et R^{6b} représentent chacun indépendamment hydrogène, halogène, C₁₋₄alkyle, -NR^{9a}R^{9b}, ou C₁₋₄alkyle substitué par un, deux ou trois atomes halo ;
R^{9a} et R^{9b} représentent chacun indépendamment hydrogène ou C₁₋₄alkyle ;
R^{3a} est hydrogène, halo, -NR^{7a}R^{7b}, C₁₋₄alkyle, C₂₋₄alcényle, C₃₋₆cycloalkyle, -OH, ou
-O-C₁₋₄alkyle ;
R^{7a} est hydrogène ;
R^{7b} est hydrogène, C₃₋₆cycloalkyle, ou C₁₋₄alkyle ;
R^{4a} est hydrogène, halo, -NR^{8a}R^{8b}, ou C₁₋₄alkyle ; et
R^{x} est 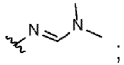 ou un sel ou solvate de celui-ci.

17. Composé selon la revendication 16, dans lequel le composé est un composé de formule (G-1a) ou (G-1b) : 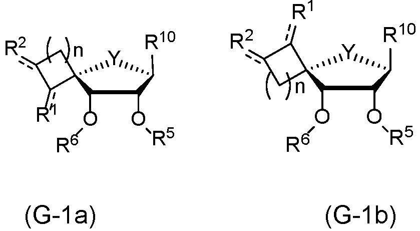 ou un sel ou solvate de celui-ci.

18. Composé selon l'une quelconque des revendications 16 ou 17, dans lequel n vaut 1.

19. Composé selon la revendication 18, dans lequel :
R¹ est hydrogène et R² est -(CH₂)ₘNR^{a}₂, -(CH₂)ₘOR^{a}, -(CH₂)ₘC(O)OR^{a}, - (CH₂)mC(O)NR^{a}₂, -(CH₂)ₘC(O)R^{a}, -(CH₂)ₘS(O)ₓR^{c}, un groupe silyle, C₁₋₆alkyle substitué par un groupe silyle, =O ou =CR^{a}₂ ; ou
R² est hydrogène et R¹ est -(CH₂)ₘNR^{a}₂, -(CH₂)ₘOR^{a}, -(CH₂)ₘOPG, - (CH₂)ₘC(O)OR^{a}, -(CH₂)ₘC(O)NR^{a}₂, -(CH₂)ₘC(O)R^{a}, -(CH₂)ₘS(O)ₓR^{c}, un groupe silyle, C₁₋₆alkyle substitué par un groupe silyle, -O-aryle, =O ou =CR^{a}₂.

20. Composé selon l'une quelconque des revendications 16 ou 17, dans lequel n vaut 0.

21. Composé selon la revendication 18, dans lequel l'un de R¹ et R² est hydrogène et l'autre est -(CH₂)ₘNR^{a}₂, -(CH₂)ₘOR^{a}, -(CH₂)ₘOPG, -(CH₂)ₘC(O)OR^{a}, -(CH₂)ₘC(O)NR^{a}₂, -(CH₂)ₘC(O)R^{a}, -(CH₂)ₘB(OR^{b})₂, ou =CH₂.

22. Composé selon la revendication 16, dans lequel le composé est choisi parmi : 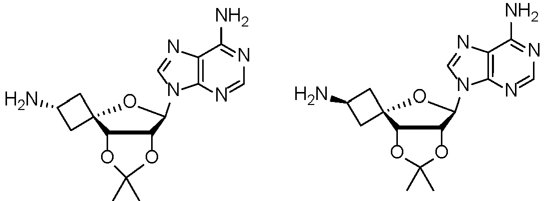 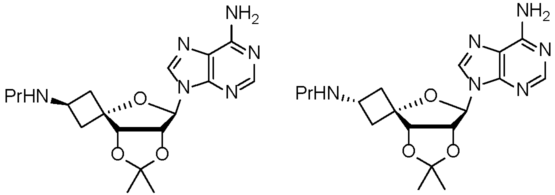 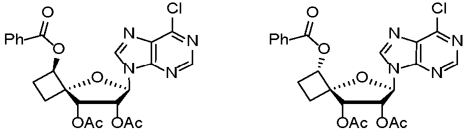 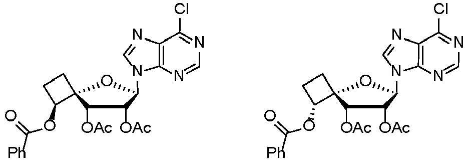 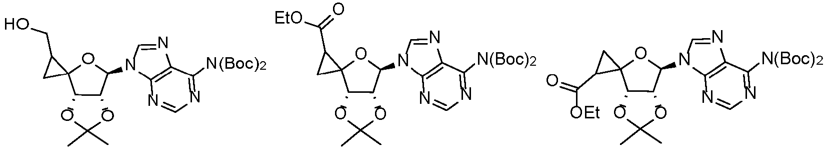 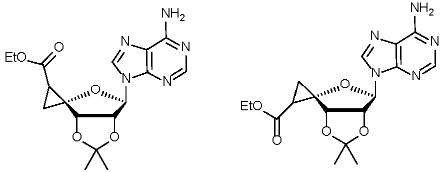 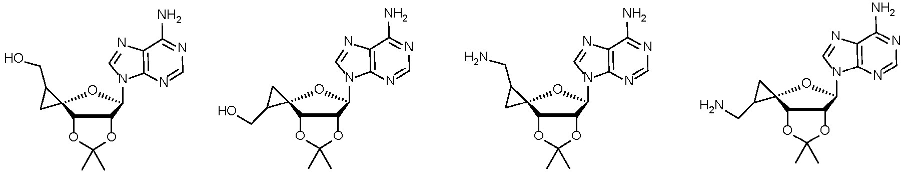 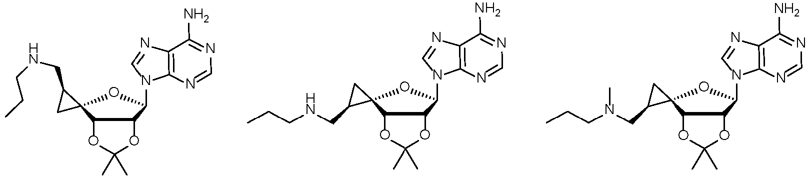 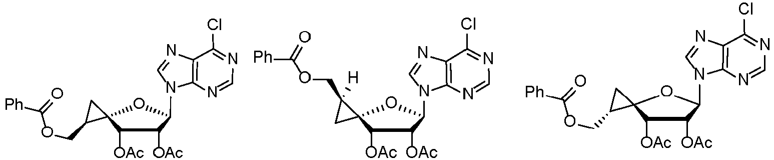 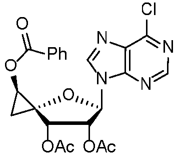 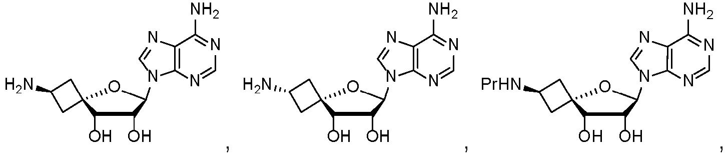 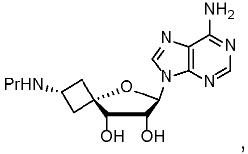 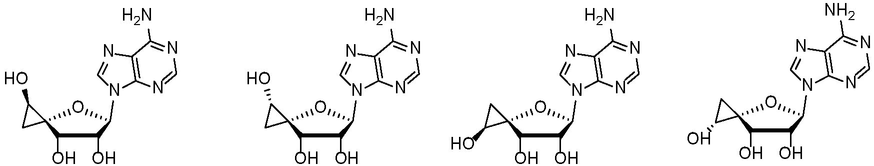 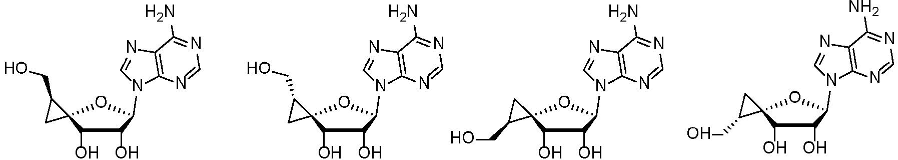 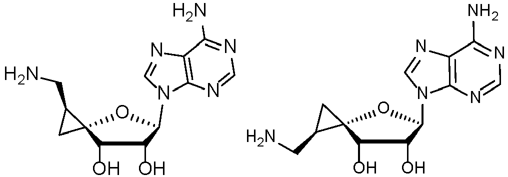 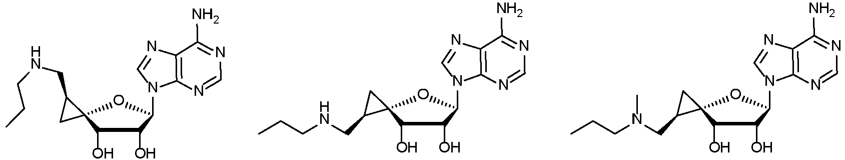 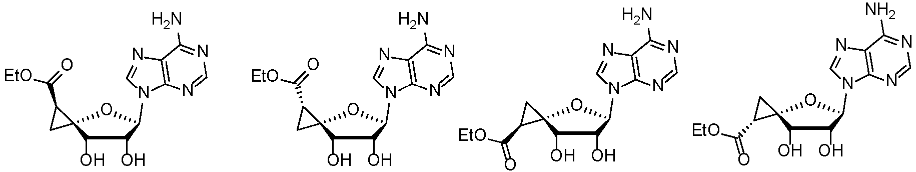 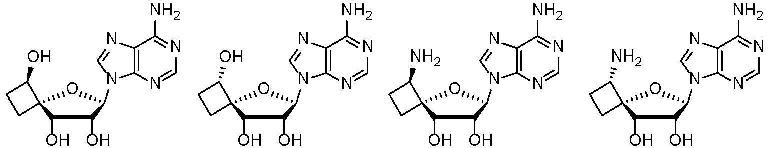 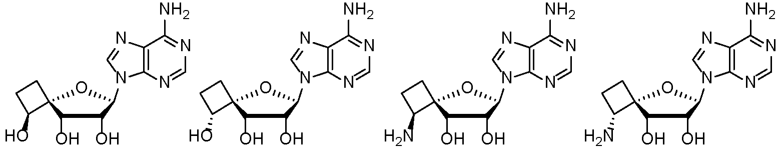 ou un sel ou solvate de celui-ci.

23. Composé de formule (H) : 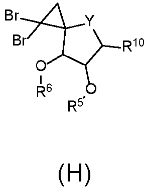 ou un sel ou solvate de celui-ci, dans lequel :
Y est -O- ou -CH₂- ;
R⁵ et R⁶ sont chacun, indépendamment, hydrogène ou un groupe protecteur d'hydroxyle qui est un groupe silyle, C₁₋₄alkyle, C₁₋₄alkyl-O-C₁₋₄alkyle, tétrahydropyranyle, allyle, benzyle, -CH₂-naphtyle, benzoyle, -C(=O)-C₁₋₄alkyle, ou -C(=O)-phényle ; dans lequel benzyle, -CH₂-naphtyle, et benzoyle sont facultativement substitués par un ou deux substituants choisis indépendamment parmi -CH₃ et -OCH₃ ; ou R⁵ et R⁶ pris conjointement sont un groupe protecteur de diol -C(C₁₋₄alkyl)₂- ; et R¹⁰ est une nucléobase ou un dérivé de nucléobase.

24. Composé selon l'une quelconque des revendications 14 à 23, dans lequel R¹⁰, lorsqu'il est présent, est cytosine, thymine, uracile, ou une forme modifiée ou protégée de ceux-ci, ou un système cyclique hétérocyclique aromatique bicyclique choisi dans le groupe constitué de (a-1), (a-2), (a-3), (a-4) et (a-5) : 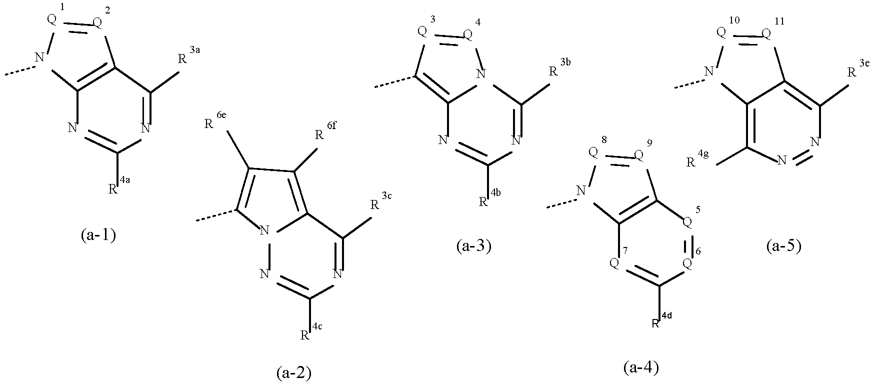
R^{3a}, R^{3b}, R^{3c}, R^{3d} et R^{3e} sont chacun indépendamment hydrogène, halo, - NR^{7a}R^{7b}, C₁₋₄alkyle, C₂₋₄alcényle, C₃₋₆cycloalkyle, -OH, ou -O-C₁₋₄alkyle ;
R^{7a} est hydrogène, -C(O)O-C₁₋₄alkyle ou 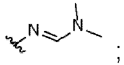
R^{7b} est hydrogène, C₃₋₆cycloalkyle, C₁₋₄alkyle, ou -C(O)O-C₁₋₄alkyle ;
R^{4a}, R^{4b}, R^{4c}, R^{4d}, R^{4e}, R^{4f} et R^{4g} sont chacun indépendamment hydrogène, halo, -NR^{8a}R^{8b}, ou C₁₋₄alkyle ;
R^{6a} et R^{8b} sont chacun indépendamment hydrogène ou C₁₋₄alkyle ;
Q¹ est N ou CR^{6a} ;
Q² est N ou CR^{6b} ;
Q³ est N ou CR^{6c} ;
Q⁴ est N ou CR^{6d} ;
à condition que l'un au maximum de Q³ et Q⁴ soit N ;
Q⁸ est N ou CR^{6g} ;
Q⁹ est N ou CR^{6h} ;
Q¹⁰ est N ou CR⁶ⁱ ;
Q¹¹ est N ou CR^{6j} ;
Q⁵ est CR^{3d} ; Q⁶ est N ; et Q⁷ est CR^{4f} ; ou
Q⁵ est CR^{3d} ; Q⁶ est CR^{4e} ; et Q⁷ est N ; ou
Q⁵ est N ; Q⁶ est CR^{4e} ; et Q⁷ est CR^{4f} ; ou
Q⁵ est N ; Q⁶ est CR^{4e} ; et Q⁷ est N ; ou
Q⁵ est N ; Q⁶ est N ; et Q⁷ est CR^{4f} ; ou
Q⁵ est N ; Q⁶ est N ; et Q⁷ est N ;
R^{6a}, R^{6b}, R^{6c}, R^{6d}, R^{6e}, R^{6f}, R^{6 g}, R^{6h}, R⁶ⁱ et R^{6j} sont chacun indépendamment hydrogène, halogène, C₁₋₄ alkyle, -NR^{9a}R^{9b}, ou C₁₋₄alkyle substitué par un, deux ou trois atomes halo ;
R^{9a} et R^{9b} sont chacun indépendamment hydrogène ou C₁₋₄alkyle.
